# EUROPÄISCHE PATENTSCHRIFT

(11) **EP 2 807 162 B1**
(45) Veröffentlichungstag und Bekanntmachung des Hinweises auf die Patenterteilung: **07.06.2017**
(21) Anmeldenummer: 13700765.4
(22) Anmeldetag: 22.01.2013
(51) Int. Cl.: C07D 487/04, A61K 31/4188, A61P 35/00

(54) **SUBSTITUIERTE PHENYLIMIDAZOPYRAZOLE UND IHRE VERWENDUNG**
SUBSTITUTED PHENYLIMIDAZOPYRAZOLES AND USE THEREOF
PHÉNYLIMIDAZOPYRAZOLES SUBSTITUÉS ET LEUR UTILISATION

(30) Priorität: 25.01.2012 EP 12152515
(43) Veröffentlichungstag der Anmeldung: 03.12.2014
(73) Patentinhaber: Bayer Pharma Aktiengesellschaft, 13353 Berlin (DE); Bayer Intellectual Property GmbH, 40789 Monheim (DE)
(72) Erfinder: SÜßMEIER, Frank, 80992 München (DE); LOBELL, Mario, 42113 Wuppertal (DE); GRÜNEWALD, Sylvia, 10557 Berlin (DE); HÄRTER, Michael, 51375 Leverkusen (DE); BUCHMANN, Bernd, 16540 Hohen Neuendorf (DE); TELSER, Joachim, 42115 Wuppertal (DE); JÖRIßEN, Hannah, 42579 Heiligenhaus (DE); HÉROULT, Mélanie, 40223 Düsseldorf (DE); KAHNERT, Antje, 42113 Wuppertal (DE); LUSTIG, Klemens, 42113 Wuppertal (DE); LINDNER, Niels, 42115 Wuppertal (DE)
(74) Vertreter: BIP Patents
(86) Internationale Anmeldenummer: PCT/EP2013/051106
(87) Internationale Veröffentlichungsnummer: WO 2013/110590

(56) Entgegenhaltungen:
- WO-A1-2004/056830
- HANHUA HUANG ET AL: "Targeting the ANGPT-TIE2 pathway in malignancy", NATURE REVIEWS. CANCER, NATUR PUBLISHING GROUP, LONDON, GB, Bd. 10, Nr. 8, 1. August 2010 (2010-08-01) , Seiten 575-585, XP002663788, ISSN: 1474-175X, DOI: 10.1038/NRC2894

## Beschreibung

Die vorliegende Anmeldung betrifft neue 1-Phenyl-1*H*-imidazo[1,2-b]pyrazol-Derivate, Verfahren zu ihrer Herstellung, ihre Verwendung zur Behandlung und/oder Prävention von Krankheiten sowie ihre Verwendung zur Herstellung von Arzneimitteln zur Behandlung und/oder Prävention von Krankheiten, insbesondere von angiogenen Erkrankungen und hyperproliferativen Erkrankungen, bei denen Neovaskularisierung eine Rolle spielt, wie beispielsweise Krebs- und Tumorerkrankungen. Solche Behandlungen können als Monotherapie oder auch in Kombination mit anderen Arzneimitteln oder weiteren therapeutischen Maßnahmen erfolgen.

Der Prozess der Angiogenese, d.h. die Bildung neuer Blutgefäße aus existierenden Gefäßen [W. Risau, Nature 386, 671 (1997); R.K. Jain, Nat. Med. 9, 685 (2003)], kommt im erwachsenen Organismus relativ selten vor (Wundheilung, ovarieller Zyklus), er spielt jedoch eine wichtige Rolle in pathologischen Prozessen, insbesondere bei Tumorerkrankungen, einschliesslich Hämangiomen und Hämangioblastomen, sowie bei Entzündungskrankheiten und Autoimmunerkrankungen, kardiovaskulären Erkrankungen, Nierenerkrankungen, Augenerkrankungen und der Endometriose mit fehlregulierter Angiogenese.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Entstehende Tumore sind anfänglich nicht vaskularisiert. Voraussetzung für weiteres Wachstum über ein Volumen von wenigen mm³ hinaus ist die Neubildung von Blutgefäßen, um den Tumor mit Sauerstoff und Nährstoffen zu versorgen. Diese Induktion der Angiogenese, auch angiogener Schalter ("angiogenic switch") genannt, gehört zu den charakteristischen Kennzeichen der Krebsentwicklung [Hanahan und Weinberg, Cell 100, 57 (2000)]. Darüber hinaus erhöht die intratumorale Neovaskularisation die Wahrscheinlichkeit, dass Tumorzellen in die systemische Zirkulation gelangen, so dass starke Vaskularisierung zu erhöhtem Metastasierungspotential führt.

Die Abhängigkeit der Tumore von der Neovaskularisierung führte zur Angiogenese-Hemmung als neuartigem Behandlungsprinzip in der Krebstherapie [Ferrara et al., Nature 438, 967 (2005); Carmeliet, Nature 438, 932 (2005)]. Dabei wird die Versorgung des wachsenden Tumors durch eine Hemmung des gleichmäßigen Mitwachsens des Blutgefäßsystems verschlechtert. Dies führt häufig zu verlangsamtem Wachstum, zur Stabilisierung des bestehenden Zustandes oder sogar zur Regression des Tumors. Einer der wichtigsten pro-angiogenen Faktoren ist der vaskuläre endotheliale Wachstumsfaktor VEGF. Mit einem VEGF-neutralisierenden, das Blutgefäßwachstum hemmenden monoklonalen Antikörper (Bevacizumab) ist es gelungen, die Lebenserwartung von Kolorektalkarzinom-Patienten zu verlängern. VEGFR-Kinase-Inhibitoren wie Sorafenib, Sunitinib oder Pazopanib zeigen Erfolge bei der Behandlung von Nierenzellkarzinomen, Leberkarzinomen bzw. fortgeschrittenen Stadien von gastrointestinalen stromalen Tumoren (GIST). Oft bleibt jedoch die Wirksamkeit der derzeit verfügbaren anti-angiogenen Therapien hinter den Erwartungen zurück, wobei zudem erhebliche Nebenwirkungen in Kauf genommen werden müssen. Daher besteht nach wie vor ein großer Bedarf an neuen Verbindungen und Verfahren mit verbesserter therapeutischer Wirksamkeit.

An der Regulation der Angiogenese sind neben der VEGF-vermittelten Signaltransduktion zahlreiche andere Signaltransduktionssysteme beteiligt, jedoch ist das Angiopoietin-Tie2-Signaltransduktionssystem eines der Endothelzell-selektivsten und wichtigsten Signalgeber für die Gefäßstabilisierung und, zusammen mit VEGF, für die Initiation des Gefäßwachstums.

Das humane Angiopoietin-Tie-Signaltransduktionssystem besteht aus den beiden Typ 1-Rezeptor-tyrosinkinasen Tie1 und Tie2 (Tyr kinase with Ig and EGF homology domains) sowie den drei sezernierten Glykoproteinliganden Angiopoietin 1 (Ang1), Angiopoietin 2 (Ang2) und Angiopoietin 4 (Ang4). Diese drei Liganden binden an Tie2, während für Tie1 bislang keine endogenen Liganden identifiziert werden konnten. Tie1 interagiert mit Tie2 und reguliert dessen Aktivität [Huang et al., Nat. Rev. Cancer 10, 575-585 (2010)]. Ang1 wirkt als Tie2-Rezeptor-Agonist und induziert sowohl *in vitro* wie *in vivo* die Multimerisierung und Autophosphorylierung von Tie2 an Tyrosinresten im intrazellulären, C-terminalen Bereich des Rezeptors, was das Andocken verschiedener Effektoren, wie z.B. DOKR (downstream of tyrosine kinase-related protein), GRB2 (growth factor receptorbound protein 2), die p85-Untereinheit der PI3K oder SHP2 (SH2 domain-containing phosphatase), erlaubt und die Aktivierung mehrerer nachgeschalteter Signalkaskaden zur Folge hat. So vermitteln die DOKR- und verschiedene PI3K-Signaltransduktionskaskaden die Ang1-induzierte Migration, Schlauchbildung (tube formation) und Sprossung von Endothelzellen, während die aktivierten MAPK- und PI3K-AKT-Signalwege anti-apoptotisch wirken und zum Überleben der Endothelzellen beitragen [Eklund und Olsen, Exp. Cell Res. 312, 630-641 (2006)]. Ang2 wurde zunächst als Ang1-Antagonist identifiziert [Maisonpierre et al., Science 277, 55-60 (1997)], der die Ang1-stimulierte Tie2-Phosphorylierung hemmt. Ang2 ist jedoch selbst in der Lage, unter bestimmten Bedingungen in Abwesenheit von Ang1 die Tie2-Phosphorylierung zu induzieren, und wirkt daher abhängig von den experimentellen Bedingungen wie ein partieller Agonist.

Die Bedeutung des Angiopoietin-Tie-Systems für die Entwicklung und Aufrechterhaltung des Blutgefäßsystems wird durch knock-out und transgene Tierstudien belegt. Die Phänotypen von Tie2-defizienten und Ang1-defizienten Mäusen sind embryonal lethal und in vergleichbarer Weise gekennzeichnet durch nicht voll ausgebildete, teilweise erweiterte Gefäße, denen die verzweigten Netze und peri-endothelialen Stützzellen fehlen. Die Analyse Tie2-defizienter Mausembryonen ergab ferner eine wichtige Rolle für Tie2 in der Hämatopoese und der Entwicklung des Endokardiums. Tie1-defiziente Mausembryonen sterben an Ödemen und Blutungen, die auf den schlechten strukturellen Zustand der Endothelzellen des Mikrogefäßsystems zurückzuführen sind. Ang2-defiziente Mäuse sind lebensfähig und zeigen keine schwerwiegende Beeinträchtigung der embryonalen vaskulären Entwicklung. Es treten jedoch Defekte dort auf, wo postnatal vaskuläre Restrukturierung und Angiogenese stattfindet, wie z.B. in der Retina. Im Gegensatz dazu führt die transgene Überexpression von Ang2 zu einer Störung der embryonalen Gefäßbildung mit einem dem Tie2- oder Ang1-knock-out ähnlichen, embryonal lethalen Phänotyp. Die konditionale Überexpression von Ang2 in Endothelzellen führt zu einer vollständigen Hemmung der Tie2-Phosphorylierung *in vivo,* was die Betrachtung von Ang2 als Ang1-Antagonisten unterstützt. Überexpression von Ang1 verringert die durch inflammatorische Cytokine hervorgerufene verstärkte Permeabilität der Gefäße, und systemische Behandlung mit Ang1 wirkt der durch VEGF hervorgerufenen Gefäßpermeabilität entgegen [Augustin et al., Nat. Rev. Mol. Cell Biol. 10, 165-177 (2009)].

Die Ergebnisse dieser *loss-of-function-* und *gain-of-function-*Studien sowie von Untersuchungen zur Expression und Funktion der Angiopoietine und Tie-Rezeptoren in der Entwicklung des *Corpus luteum* und der Blutgefäßentwicklung in der Netzhaut deuten auf eine grundlegende Rolle des Angiopoietin-Tie-Rezeptorsystems bei der Vermittlung von Wechselwirkungen zwischen Endothelzellen und umgebenden Perizyten oder glatten Muskelzellen hin, was insbesondere für den Angiogeneseprozess, bestehend aus der Destabilisierung eines bestehenden Gefäßes, Sprossung und Invasion sowie der Stabilisierung der neuen Gefäße, bedeutsam ist.

Entsprechend den in der Literatur publizierten Vorstellungen geht man davon aus, dass in ruhenden, durch murale Zellen (Perizyten oder glatte Muskelzellen) stabilisierten Gefäßen eine konstitutive Ang1-Tie2-Signaltransduktion zur Aufrechterhaltung der Gefäßintegrität und endothelialen Barriere erfolgt. Ang1 wird konstitutiv von Perizyten sezerniert und aktiviert den auf den Endothelzellen lokalisierten Tie2-Rezeptor. Diese konstitutive Aktivierung wird dynamisch durch Tie1 und besonders durch autokrin wirkendes Ang2 kontrolliert. Die Expression von Ang2 in Endothelzellen wird transkriptionell durch Zytokine, insbesondere VEGF, und Hypoxie induziert und ist in Geweben erhöht, in denen Angiogenese bzw. Restrukturierung von Gefäßen stattfindet. In endothelialen Weibel-Palade-Vesikeln gespeichertes Ang2 kann sehr rasch ausgeschüttet werden, was die Verdrängung des an Tie2 gebundenen Ang1 und die Unterdrückung der Ang1-vermittelten Signaltransduktion zur Folge hat. Dies führt zur Dissoziation der Perizyten von den Endothelzellen und verringerten Endothelzell-Zell-Kontakten und somit zur Destabilisierung der Gefäße mit einhergehendem Abbau der Basalmembran. In Abwesenheit von VEGF führt dieser Prozess zur Apoptose der Endothelzellen und Gefäßregression. Bei genügend hohen VEGF-Konzentrationen im Gewebe, z.B. durch hypoxische Induktion der Expression im Tumor, werden die Endothelzellen in Abhängigkeit von ihrer Lokalisation im destabilisierten Gefäß zur Proliferation oder Migration und letztlich zur Bildung neuer Gefäßsprossen stimuliert.

Tie2 ist außerdem auf einer Subpopulation von Tumor-infiltrierenden, CD11b⁺ myeloiden Zellen exprimiert, den Tie2-exprimierenden Monozyten (TEMs). Die zirkulierenden TEMs fördern die Tumorangiogenese mit ihren pro-angiogenen Eigenschaften, welche durch erhöhtes Ang2 verstärkt werden [Coffelt et al., Cancer Res. 70, 5270-5280 (2010)].

In den pathologischen Prozessen, die mit einer anomalen Gefäßneubildung assoziiert sind, werden angiogene Wachstumsfaktoren und ihre Rezeptoren oft verstärkt exprimiert. Eine erhöhte Expression von Tie2-Rezeptoren wurde z.B. im Endothelium von metastasierenden Melanomen [Kaipainen et al., Cancer Res. 54, 6571-6577 (1994)], in Brustkarzinomen [Salven et al., Br. J. Cancer 74, 69-72 (1996)], in wiederkehrendem papillären Schilddrüsenkarzinom [Hsueh et al., J. Surg. Oncol. 103, 395-399 (2011)], in großen Lebertumoren [Dhar et al., Anticancer Res. 22, 379-386 (2002)], in endometrialen Adenokarzinomen [Saito et al., Pathol. Int. 57, 140-147 (2007)] und in Magenkarzinomen [Moon et al., J. Korean Med. Sei. 21, 272-278 (2006)] gefunden.

Entsprechend konnte gezeigt werden, dass eine funktionelle Störung des Tie2-Rezeptors in Xenograft-Modellen von humanen Karposi-Sarkomen und SW1222-Darmkarzinomen durch adenovirale Verabreichung von einkettigen, gegen Tie2 gerichteten Antikörperfragmenten zu einer signifikanten Reduktion der Gefäßdichte und des Tumorwachstums führt [Popkov et al., Cancer Res. 65, 972-981 (2005)]. Ferner wurden zur Blockierung der Angiopoietin-Tie2-Signaltransduktion lösliche Angiopoietin-neutralisierende Tie2-Varianten, bestehend aus der Fc-fusionierten extrazellulären ligandenbindenden Tie2-Domäne, in Xenograft-Tumormodellen eingesetzt und eine Hemmung des Wachstums und der Vaskularisierung der experimentellen Tumore gezeigt [Lin et al., J. Clin. Invest. 103, 159-165 (1999); Siemeister et al., Cancer Res. 59, 3185-3191 (1999)].

Im Bereich der Tie2-Kinasedomäne wurden Mutationen gefunden, die zu Liganden-unabhängiger Tie2-Aktivierung führen und in die Entstehung von venösen Gefäßfehlbildungen involviert sind [Wouters et al., Eur. J. Hum. Genet. 18, 414-420 (2010)].

Prognose korreliert. Dazu gehören verschiedene Krebsindikationen, darunter z.B. Brustkarzinom, Leberkarzinom, Ovarialkarzinom, metastasierendes Kolonkarzinom, Prostatakrebs, Lungenkrebs und multiples Myelom [Huang et al., Nat. Rev. Cancer 10, 575-585 (2010)].

In präklinischen Studien mit Antikörpern, die gegen Ang2 gerichtet sind, oder mit Peptidfusionsproteinen ("Peptibodies"), die entweder nur Ang2 oder sowohl Ang2 als auch Ang1 neutralisieren, konnte das Wachstum verschiedener experimenteller Xenograft-Tumore und deren Vaskularisierung gehemmt werden [Oliner et al., Cancer Cell 6, 507-516 (2004); Brown et al., Mol. Cancer Ther. 9, 145-156 (2010); Huang et al., Clin. Cancer Res. 17, 1001-1011 (2011)]. Ferner wurde in mehreren präklinischen Studien durch Kombination von Angiopoietin-neutralisierenden Proteinen mit VEGF-Signaltransduktion-blockierenden Therapien oder zytotoxischen Verbindungen eine signifikant verbesserte Wirkung gegenüber den Monotherapien erzielt [Brown et al., Mol. Cancer Ther. 9, 145-156 (2010); Coxon et al., Mol. Cancer Ther. 9, 2641-2651(2010)].

Zusammenfassend zeigen *in vitro-* und *in vivo*-Studien die große Bedeutung des Angiopoietin-Tie2-Systems für die Tumorangiogenese und andauerndes Tumorwachstum und auch die Beteiligung bei Lymphangiogenese, Metastasierung und Entzündungsprozessen [Huang et al., Nat. Rev. Cancer 10, 575-585 (2010)]. Das Angiopoietin-Tie2-System ist daher verstärkt Ziel therapeutischer Strategien. Diese umfassen zum einen gegen Angiopoietine gerichtete biologische Moleküle und zum anderen niedermolekulare Verbindungen, die die Tie2-Kinaseaktivität hemmen. AMG-386 (Amgen) und CVX-060 (Pfizer) sind duale Ang1/Ang2- bzw. allein Ang2-neutralisierende Peptidfusionsproteine in Phase III bzw. II der klinischen Entwicklung. Der duale Tie2/VEGFR-Inhibitor CEP-11981 (Cephalon) ist in Phase I der klinischen Entwicklung.

Aufgabe der vorliegenden Erfindung war daher die Bereitstellung neuer Verbindungen, welche die Tie2-Rezeptorkinaseaktivität hemmen und auf diese Weise zur Behandlung und/oder Prävention von Erkrankungen, insbesondere von Krebserkrankungen und anderen angiogenen Erkrankungen, eingesetzt werden können.

In WO 2008/042639-A1 werden *N*-Phenyl-Pyrimidinylpyrazolamine als Multi-Kinase-Inhibitoren für die Behandlung proliferativer Erkrankungen beschrieben. In EP 2 327 704-A1 und WO 2010/ 125799-A1 werden Ureido-substituierte, anellierte Azol-Derivate, unter anderem 1-Phenyl-2,3-dihydro-1*H*-imidazo[1,2-b]pyrazole, als PI3K-Inhibitoren zur Behandlung verschiedenartiger Erkrankungen offenbart.

In WO 2004/056830-A1 (sowie in HANHUA HUANG ET AL: "Targeting the ANGPT-TIE2 pathway inmalignancy",NATURE REVIEWS. CANCER, NATUR PUBLISHING GROUP,LONDON, GB,Bd. 10, Nr. 8, 1. August 2010 (2010-08-01), Seiten 575-585) werden Multikinaserezeptoren offenbart, die auch die Aktivität des Kinaserezeptors TIE2 hemmen - insbesondere die Verbindung CE-245677, welche sich jedoch strukturell von den erfindungsgemäßen Verbindungen deutlich unterscheidet.

Gegenstand der vorliegenden Erfindung sind Verbindungen der allgemeinen Formel (1) 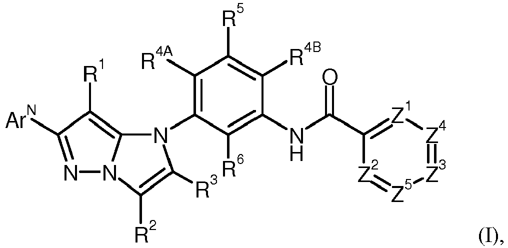 in welcher
- Ar^{N}: für 5- oder 6-gliedriges Aza-Heteroaryl steht, das als kennzeichnendes Strukturmerkmal ein Ring-Stickstoffatom in 3-Position relativ zur Verknüpfungsstelle des Heteroaryl-Rings als Bestandteil einer C=N- oder N=N-Doppelbindung enthält und das ausgewählt ist aus der Gruppe bestehend aus 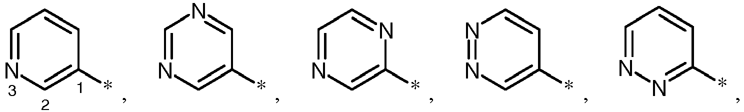 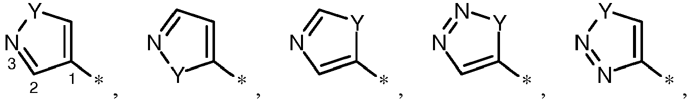 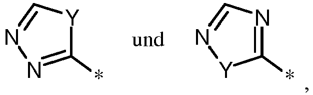
worin * die Verknüpfung zur Imidazopyrazol-Gruppierung markiert und
Y O, S oder NH bedeutet,
- R¹: für Wasserstoff oder Fluor steht,
- R^{4A} und R^{4B}: unabhängig voneinander für Wasserstoff, Fluor, Chlor, Methyl, Fluormethyl, Difluormethyl, Trifluormethyl, Hydroxymethyl, Methoxymethyl, Ethyl, Hydroxy, Methoxy oder Trifluormethoxy stehen,
- R⁵: für Wasserstoff, Fluor, Chlor oder Methyl steht,
- R⁶: für Wasserstoff, Fluor, Methyl oder Hydroxy steht,
- Z¹: für C-R^{7A} oder N steht,
- Z²: für C-R^{7B} oder N steht,
- Z³: für C-R⁸ oder N steht,
- Z⁴: für C-R⁹ oder N steht
und
- Z⁵: für C-R¹⁰ oder N steht,
wobei insgesamt maximal eines der Ringglieder Z¹, Z², Z³, Z⁴ und Z⁵ für N steht
und worin
R^{7A} und R^{7B} unabhängig voneinander Wasserstoff, Fluor, Chlor, Methyl, Hydroxy oder Methoxy bedeuten,
R⁸ Wasserstoff, Fluor, Chlor oder Methyl bedeutet,
R⁹ Wasserstoff, Pentafluorsulfanyl, (Trifluormethyl)sulfanyl, Trimethylsilyl, (C₁-C₆)-Alkyl, (C₁-C₆)-Alkoxy, (C₃-C₆)-Cycloalkyl, Oxetanyl oder Tetrahydropyranyl bedeutet,
wobei (C₁-C₆)-Alkyl und (C₁-C₆)-Alkoxy bis zu sechsfach mit Fluor substituiert sein können
und
(C₃-C₆)-Cycloalkyl, Oxetanyl und Tetrahydropyranyl bis zu zweifach, gleich oder verschieden, mit einem Rest ausgewählt aus der Reihe Fluor, Methyl, Trifluormethyl und Hydroxy substituiert sein können,
und
R¹⁰ Wasserstoff, Fluor, Chlor, Brom, Cyano, (C₁-C₆)-Alkyl, Hydroxy, (C₁-C₆)-Alkoxy, (C₁-C₄)-Alkylsulfonyl, (C₃-C₆)-Cycloalkyl, Phenyl, 5- oder 6-gliedriges Heteroaryl oder eine Gruppe der Formel -L¹-C(=O)-OR¹¹, -L¹-NR^{12A}R^{12B}, -L¹-C(=O)-NR¹³R^{13B}, -L²-S(=O)₂-NR^{13A}R^{13B} oder -L³-R¹⁴ bedeutet,
wobei (C₁-C₆)-Alkyl und (C₁-C₆)-Alkoxy mit einem Rest ausgewählt aus der Reihe Hydroxy, Methoxy, Ethoxy, 2-Hydroxyethoxy, 2-Methoxyethoxy, 2-Ethoxyethoxy, Amino, Methylamino und Dimethylamino oder bis zu sechsfach mit Fluor substituiert sein können
und
(C₃-C₆)-Cycloalkyl bis zu zweifach, gleich oder verschieden, mit einem Rest ausgewählt aus der Reihe Methyl, Hydroxy, Methoxy, Ethoxy, Amino, Methylamino und Dimethylamino substituiert sein kann
und
Phenyl und 5- oder 6-gliedriges Heteroaryl bis zu zweifach, gleich oder verschieden, mit einem Rest ausgewählt aus der Reihe Fluor, Chlor, Cyano, Methyl und Trifluormethyl substituiert sein können,
und worin
L¹ eine Bindung oder -CH₂- darstellt,
L² eine Bindung oder -CH₂- darstellt,
L³ eine Bindung oder -O- darstellt,
R¹¹ Wasserstoff oder (C₁-C₄)-Alkyl darstellt,
R^{12A} , R^{12B}, R^{13A} und R^{13B} unabhängig voneinander Wasserstoff oder (C₁-C₄)-Alkyl darstellen,
wobei (C₁-C₄)-Alkyl jeweils mit einem Rest ausgewählt aus der Reihe Hydroxy, Methoxy, Ethoxy, Amino, Methylamino und Dimethylamino substituiert sein kann,
oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N, O oder S enthalten kann und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann,
und
R¹⁴ einen über ein Ring-Kohlenstoffatom gebundenen 4- bis 6-gliedrigen Heterocyclus darstellt, der ein Ring-Heteroatom aus der Reihe N, O oder S enthält und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann, wobei R¹⁰ nicht Wasserstoff, Fluor, Chlor oder Brom bedeutet, wenn Z⁴ für CH oder N steht, und Z⁵ nicht für N steht, wenn Z⁴ für CH steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindunggemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor Brom und Iod wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N*,*N*-Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylmorpholin, *N*-Methylpiperidin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die *N*-Oxide von in erfindungsgemäßen Verbindungen enthaltenen Pyridyl-Ringen und tertiären cyclischen Amin-Gruppierungen sind gleichfalls von der vorliegenden Erfindung umfasst.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl, (C₁-C₄)-Alkyl und (C₂-C₄)-Alkyl stehen im Rahmen der Erfindung für einen geradkettigen oder verzweigten Alkylrest mit 1 bis 6, 1 bis 4 bzw. 2 bis 4 Kohlenstoffatomen. Beispielhaft und vorzugsweise seien genannt: Methyl, Ethyl, *n*-Propyl, Isopropyl, *n*-Butyl, Isobutyl, *sec*.-Butyl, *tert.-Butyl, n*-Pentyl, 2-Pentyl, 3-Pentyl, Neopentyl, *n*-Hexyl, 2-Hexyl und 3-Hexyl.

(C₁-C₄)-Alkylsulfonyl steht im Rahmen der Erfindung für einen geradkettigen oder verzweigten Alkylrest mit 1 bis 4 Kohlenstoffatomen, der über eine Sulfonyl-Gruppe [-S(=O)₂-] mit dem Rest des Moleküls verknüpft ist. Beispielhaft und vorzugsweise seien genannt: Methylsulfonyl, Ethylsulfonyl, *n*-Propylsulfonyl, Isopropylsulfonyl, *n*-Butylsulfonyl und *tert*.-Butylsulfonyl.

(C₁-C₆)-Alkoxy, (C₁-C₄)-Alkoxy und (C₂-C₄)-Alkoxy stehen im Rahmen der Erfindung für einen geradkettigen oder verzweigten Alkoxyrest mit 1 bis 6, 1 bis 4 bzw. 2 bis 4 Kohlenstoffatomen. Beispielhaft und vorzugsweise seien genannt: Methoxy, Ethoxy, *n*-Propoxy, Isopropoxy, *n*-Butoxy, Isobutoxy, *sec*.-Butoxy, *tert*.-Butoxy, *n*-Pentoxy, 2-Pentoxy, 3-Pentoxy, Neopentoxy, *n*-Hexoxy, 2-Hexoxy und 3-Hexoxy.

(C₃-C₆)-Cycloalkyl steht im Rahmen der Erfindung für eine monocyclische, gesättigte Cycloalkylgruppe mit 3 bis 6 Ring-Kohlenstoffatomen. Beispielhaft und vorzugsweise seien genannt: Cyclopropyl, Cyclobutyl, Cyclopentyl und Cyclohexyl.

Ein 4- bis 6-gliedriger Heterocyclus steht im Rahmen der Erfindung für einen monocyclischen gesättigten Heterocyclus mit insgesamt 4 bis 6 Ringatomen, welcher ein oder zwei gleiche oder verschiedene Ring-Heteroatome aus der Reihe N, O und S enthält und über ein Ring-Kohlenstoffatom oder ein Ring-Stickstoffatom verknüpft ist. Bevorzugt ist ein 4- bis 6-gliedriger Heterocyclus mit einem oder zwei Ring-Heteroatomen aus der Reihe N und/oder O. Beispielhaft seien genannt: Azetidinyl, Oxetanyl, Thietanyl, Pyrrolidinyl, Pyrazolidinyl, Tetrahydrofuranyl, Thiolanyl, 1,2-Oxazolidinyl, 1,3-Oxazolidinyl, 1,3-Thiazolidinyl, Piperidinyl, Piperazinyl, Tetrahydropyranyl, Tetrahydrothiopyranyl, 1,3-Dioxanyl, 1,4-Dioxanyl, 1,2-Oxazinanyl, Morpholinyl und Thiomorpholinyl. Bevorzugt sind Azetidinyl, Oxetanyl, Pyrrolidinyl, Tetrahydrofuranyl, Piperidinyl, Piperazinyl, Tetrahydropyranyl und Morpholinyl. Besonders bevorzugt sind Azetidinyl, Pyrrolidinyl, Piperidinyl, Piperazinyl und Morpholinyl.

5- oder 6-gliedriges Heteroaryl in der Definition des Restes R¹⁰ steht für einen monocyclischen aromatischen Heterocyclus (Heteroaromaten) mit insgesamt 5 bzw. 6 Ringatomen, welcher bis zu drei gleiche oder verschiedene Ring-Heteroatome aus der Reihe N, O und S enthält und über ein Ring-Kohlenstoffatom oder ein Ring-Stickstoffatom verknüpft ist. Beispielhaft seien genannt: Furyl, Pyrrolyl, Thienyl, Pyrazolyl, Imidazolyl, 1,2-Oxazolyl (Isoxazolyl), 1,3-Oxazolyl, 1,2-Thiazolyl (Isothiazolyl), 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl, 1,3,4-Thiadiazolyl, Pyridyl, Pyrimidinyl, Pyridazinyl, Pyrazinyl, 1,2,4-Triazinyl und 1,3,5-Triazinyl. Bevorzugt ist 5-gliedriges Heteroaryl, das ein Ring-Stickstoffatom enthält ("Aza-Heteroaryl") und darüber hinaus ein weiteres Ring-Heteroatom aus der Reihe N, O oder S enthalten kann, wie Pyrrolyl, Pyrazolyl, Imidazolyl, 1,2-Oxazolyl, 1,3-Oxazolyl, 1,2-Thiazolyl und 1,3-Thiazolyl.

Ein Oxo-Substituent steht im Rahmen der Erfindung für ein Sauerstoffatom, das über eine Doppelbindung an ein Kohlenstoff- oder Schwefelatom gebunden ist.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei oder drei gleichen oder verschiedenen Substituenten ist bevorzugt. Besonders bevorzugt ist die Substitution mit einem oder mit zwei gleichen oder verschiedenen Substituenten. Ganz besonders bevorzugt ist die Substitution mit einem Substituenten.

Eine bestimmte Ausführungsform der vorliegenden Erfindung umfasst Verbindungen der Formel (I), in welcher
- Ar^{N}: für 5- oder 6-gliedriges Aza-Heteroaryl steht, das als kennzeichnendes Strukturmerkmal ein Ring-Stickstoffatom in 3-Position relativ zur Verknüpfungsstelle des Heteroaryl-Rings als Bestandteil einer C=N- oder N=N-Doppelbindung enthält und das ausgewählt ist aus der Gruppe bestehend aus 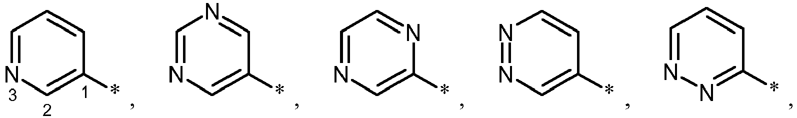 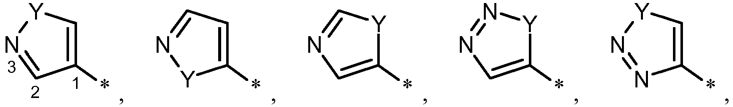 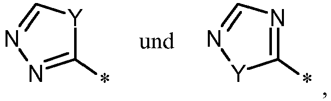 worin * die Verknüpfung zur Imidazopyrazol-Gruppierung markiert
und
Y O, S oder NH bedeutet,
- R¹: für Wasserstoff oder Fluor steht,
- R²: für Wasserstoff oder (C₁-C₄)-Alkyl steht,
- R³: für Wasserstoff steht,
- R^{4A} und R^{4B}: unabhängig voneinander für Wasserstoff, Fluor, Chlor, Methyl, Fluormethyl, Difluormethyl, Trifluormethyl, Hydroxymethyl, Methoxymethyl, Ethyl, Hydroxy, Methoxy oder Trifluormethoxy stehen,
- R⁵: für Wasserstoff, Fluor, Chlor oder Methyl steht,
- R⁶: für Wasserstoff, Fluor, Methyl oder Hydroxy steht,
- Z¹: für C-R^{7A} oder N steht,
- Z²: für C-R^{7B} oder N steht,
- Z³: für C-R⁸ oder N steht,
- Z⁴: für C-R⁹ oder N steht
und
- Z⁵: für C-R¹⁰ oder N steht,
wobei insgesamt maximal eines der Ringglieder Z¹, Z², Z³, Z⁴ und Z⁵ für N steht
und worin
R^{7A} und R^{7B} unabhängig voneinander Wasserstoff, Fluor, Chlor, Methyl, Hydroxy oder Methoxy bedeuten,
R⁸ Wasserstoff, Fluor, Chlor oder Methyl bedeutet,
R⁹ Wasserstoff, Pentafluorsulfanyl, (Trifluormethyl)sulfanyl, Trimethylsilyl, (C₁-C₆)-Alkyl, (C₁-C₆)-Alkoxy, (C₃-C₆)-Cycloalkyl, Oxetanyl oder Tetrahydropyranyl bedeutet,
wobei (C₁-C₆)-Alkyl und (C₁-C₆)-Alkoxy bis zu dreifach mit Fluor substituiert sein können
und
(C₃-C₆)-Cycloalkyl, Oxetanyl und Tetrahydropyranyl bis zu zweifach, gleich oder verschieden, mit einem Rest ausgewählt aus der Reihe Fluor, Methyl, Trifluormethyl und Hydroxy substituiert sein können,
und
R¹⁰ Wasserstoff, Fluor, Chlor, Brom, Cyano, (C₁-C₆)-Alkyl, Hydroxy, (C₁-C₆)-Alkoxy, (C₁-C₄)-Alkylsulfonyl, (C₃-C₆)-Cycloalkyl, Phenyl, 5- oder 6-gliedriges Heteroaryl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₆)-Alkyl und (C₁-C₆)-Alkoxy mit einem Rest ausgewählt aus der Reihe Hydroxy, Methoxy, Ethoxy, 2-Hydroxyethoxy, 2-Methoxyethoxy, 2-Ethoxyethoxy, Amino, Methylamino und Dimethylamino oder bis zu dreifach mit Fluor substituiert sein können
und
(C₃-C₆)-Cycloalkyl bis zu zweifach, gleich oder verschieden, mit einem Rest ausgewählt aus der Reihe Methyl, Hydroxy, Methoxy, Ethoxy, Amino, Methylamino und Dimethylamino substituiert sein kann
und
Phenyl und 5- oder 6-gliedriges Heteroaryl bis zu zweifach, gleich oder verschieden, mit einem Rest ausgewählt aus der Reihe Fluor, Chlor, Cyano und Methyl substituiert sein können,
und worin
L¹ eine Bindung oder -CH₂- darstellt,
L² eine Bindung oder -CH₂- darstellt,
L³ eine Bindung oder -O- darstellt,
R¹¹ Wasserstoff oder (C₁-C₄)-Alkyl darstellt,
R^{12A} , R^{12B} , R^{13A} und R^{13B} unabhängig voneinander Wasserstoff oder (C₁-C₄)-Alkyl darstellen,
wobei (C₁-C₄)-Alkyl jeweils mit einem Rest ausgewählt aus der Reihe Hydroxy, Methoxy, Ethoxy, Amino, Methylamino und Dimethylamino substituiert sein kann, oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N, O oder S enthalten kann und der bis zu zweifach, gleich oder verschieden, mit einem Rest ausgewählt aus der Reihe Methyl, Hydroxy und Oxo substituiert sein kann, und
R¹⁴ einen über ein Ring-Kohlenstoffatom gebundenen 4- bis 6-gliedrigen Heterocyclus darstellt, der ein Ring-Heteroatom aus der Reihe N, O oder S enthält und der bis zu zweifach, gleich oder verschieden, mit einem Rest ausgewählt aus der Reihe Methyl, Hydroxy und Oxo substituiert sein kann, wobei R¹⁰ nicht Wasserstoff, Fluor, Chlor oder Brom bedeutet, wenn Z⁴ für CH oder N steht, und Z⁵ nicht für N steht, wenn Z⁴ für CH steht,
sowie ihre Salze, Solvate und Solvate der Salze.

Bevorzugt im Rahmen der vorliegenden Erfindung sind Verbindungen der Formel (I), in welcher
- Ar^{N}: für 5- oder 6-gliedriges Aza-Heteroaryl steht, das ausgewählt ist aus der Gruppe bestehend aus 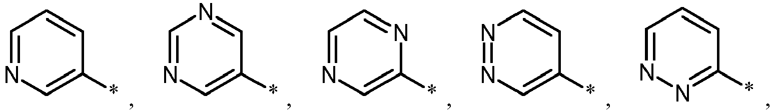 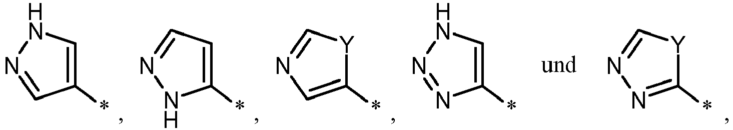
worin * die Verknüpfung zur Imidazopyrazol-Gruppierung markiert und
- Y S: oder NH bedeutet,
- R¹: für Wasserstoff oder Fluor steht,
- R²: für Wasserstoff oder (C₁-C₄)-Alkyl steht,
- R³: für Wasserstoff steht,
- R^{4A} und R^{4B}: unabhängig voneinander für Wasserstoff, Fluor, Chlor, Methyl, Fluormethyl, Difluormethyl, Trifluormethyl, Ethyl oder Methoxy stehen,
- R⁵: für Wasserstoff, Fluor, Chlor oder Methyl steht,
- R⁶: für Wasserstoff, Fluor, Methyl oder Hydroxy steht,
- Z¹: für C-R^{7A} oder N steht,
- Z²: für C-R^{7B} oder N steht,
- Z³: für C-R⁸ oder N steht,
- Z⁴: für C-R⁹ steht
und
- Z⁵: für C-R¹⁰ oder N steht,
wobei insgesamt maximal eines der Ringglieder Z¹, Z², Z³ und Z⁵ für N steht
und worin
R^{7A} und R^{7B} unabhängig voneinander Wasserstoff oder Fluor bedeuten,
R⁸ Wasserstoff oder Fluor bedeutet,
R⁹ Wasserstoff, Pentafluorsulfanyl, (Trifluormethyl)sulfanyl, (C₁-C₄)-Alkyl, (C₁-C₄)-Alkoxy, Cyclopropyl, Cyclobutyl oder Oxetanyl bedeutet,
wobei (C₁-C₄)-Alkyl und (C₁-C₄)-Alkoxy bis zu sechsfach mit Fluor substituiert sein können
und
Cyclopropyl, Cyclobutyl und Oxetanyl bis zu zweifach, gleich oder verschieden, mit einem Rest ausgewählt aus der Reihe Fluor, Methyl, Trifluormethyl und Hydroxy substituiert sein können, und
- R¹⁰: Wasserstoff, Fluor, Chlor, Brom, Cyano, (C₁-C₄)-Alkyl, Hydroxy, (C₁-C₄)-Alkoxy, (C₁-C₄)-Alkylsulfonyl, 5-gliedriges Aza-Heteroaryl oder eine Gruppe der Formel -L¹-C(=O)-OR¹¹, -L¹-NR^{12A}R^{12B} -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
5-gliedriges Aza-Heteroaryl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A}, R^{12B} , R^{13A} und R^{13B} unabhängig voneinander Wasserstoff oder (C₁-C₄)-Alkyl darstellen oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N oder O enthalten kann und der bis zu zweifach, gleich oder verschieden, mit einem Rest ausgewählt aus der Reihe Fluor, Cyano, Methyl, Ethyl, Hydroxy, Methoxy und Ethoxy substituiert sein kann,
und
R¹⁴ einen über ein Ring-Kohlenstoffatom gebundenen 4- bis 6-gliedrigen Heterocyclus darstellt, der ein Ring-Heteroatom aus der Reihe N oder O enthält und der bis zu zweifach, gleich oder verschieden, mit einem Rest ausgewählt aus der Reihe Fluor, Cyano, Methyl, Ethyl, Hydroxy, Methoxy und Ethoxy substituiert sein kann, wobei R¹⁰ nicht Wasserstoff, Fluor, Chlor oder Brom bedeutet, wenn Z⁴ für CH steht, und Z⁵ nicht für N steht, wenn Z⁴ für CH steht,
sowie ihre Salze, Solvate und Solvate der Salze.

Eine weitere bevorzugte Ausführungsform der vorliegenden Erfindung umfasst Verbindungen der Formel (I), in welcher
- Ar^{N}: für 5- oder 6-gliedriges Aza-Heteroaryl steht, das ausgewählt ist aus der Gruppe bestehend aus 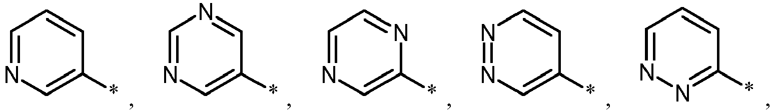 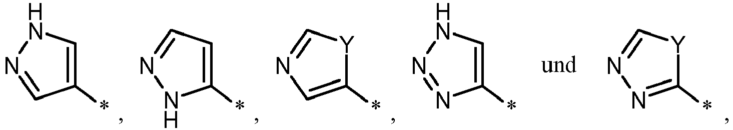 worin * die Verknüpfung zur Imidazopyrazol-Gruppierung markiert
und
Y S oder NH bedeutet,
- R¹: für Wasserstoff oder Fluor steht,
- R²: für Wasserstoff oder (C₁-C₄)-Akl steht,
- R³: für Wasserstoff steht,
- R^{4A} und R^{4B}: unabhängig voneinander für Wasserstoff, Fluor, Chlor, Methyl, Fluormethyl, Difluormethyl, Trifluormethyl, Ethyl oder Methoxy stehen,
- R⁵: für Wasserstoff, Fluor, Chlor oder Methyl steht,
- R⁶: für Wasserstoff, Fluor, Methyl oder Hydroxy steht,
- Z¹: für C-R^{7A} oder N steht,
- Z²: für C-R^{7B} oder N steht,
- Z³: für C-R⁸ oder N steht,
- Z⁴: für C-R⁹ steht
und
- Z⁵: für C-R¹⁰ oder N steht,
wobei insgesamt maximal eines der Ringglieder Z¹, Z², Z³ und Z⁵ für N steht
und worin
R^{7A} und R^{7B} unabhängig voneinander Wasserstoff oder Fluor bedeuten,
R⁸ Wasserstoff oder Fluor bedeutet,
R⁹ Wasserstoff, Pentafluorsulfanyl, (Trifluormethyl)sulfanyl, (C₁-C₄)-Alkyl, (C₁-C₄)-Alkoxy, Cyclopropyl, Cyclobutyl oder Oxetanyl bedeutet,
wobei (C₁-C₄)-Alkyl und (C₁-C₄)-Alkoxy bis zu dreifach mit Fluor substituiert sein können
und
Cyclopropyl, Cyclobutyl und Oxetanyl bis zu zweifach, gleich oder verschieden, mit einem Rest ausgewählt aus der Reihe Fluor, Methyl, Trifluormethyl und Hydroxy substituiert sein können, und
R¹⁰ Wasserstoff, Fluor, Chlor, Brom, Cyano, (C₁-C₄)-Alkyl, Hydroxy, (C₁-C₄)-Alkoxy, (C₁-C₄)-Alkylsulfonyl, 5-gliedriges Aza-Heteroaryl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
5-gliedriges Aza-Heteroaryl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A} , R^{12B} , R^{13A} und R^{13B} unabhängig voneinander Wasserstoff oder (C₁-C₄)-Alkyl darstellen oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N oder O enthalten kann und der mit Methyl oder Hydroxy substituiert sein kann, und
R¹⁴ einen über ein Ring-Kohlenstoffatom gebundenen 4- bis 6-gliedrigen Heterocyclus darstellt, der ein Ring-Heteroatom aus der Reihe N oder O enthält und der mit Methyl oder Hydroxy substituiert sein kann, wobei R¹⁰ nicht Wasserstoff, Fluor, Chlor oder Brom bedeutet, wenn Z⁴ für CH steht, und Z⁵ nicht für N steht, wenn Z⁴ für CH steht,
sowie ihre Salze, Solvate und Solvate der Salze.

Eine besondere Ausführungsform der vorliegenden Erfindung betrifft Verbindungen der Formel (I), in welcher
- R¹: für Wasserstoff oder Fluor steht,
- R²: für Wasserstoff oder Methyl steht,
und
- R³: für Wasserstoff steht,
sowie ihre Salze, Solvate und Solvate der Salze.

Eine weitere besondere Ausführungsform der vorliegenden Erfindung betrifft Verbindungen der Formel (I), in welcher
- R^{4A}: für Fluor, Chlor, Methyl oder Trifluormethyl steht,
sowie ihre Salze, Solvate und Solvate der Salze.

Eine weitere besondere Ausführungsform der vorliegenden Erfindung betrifft Verbindungen der Formel (I), in welcher
- R^{4B}: für Wasserstoff, Fluor, Chlor oder Methyl steht,
sowie ihre Salze, Solvate und Solvate der Salze.

Eine weitere besondere Ausführungsform der vorliegenden Erfindung betrifft Verbindungen der Formel (I), in welcher
R⁵ und R⁶ jeweils für Wasserstoff stehen,
sowie ihre Salze, Solvate und Solvate der Salze.

Eine weitere besondere Ausführungsform der vorliegenden Erfindung betrifft Verbindungen der Formel (I), in welcher
Z¹ und Z² jeweils für CH stehen,
sowie ihre Salze, Solvate und Solvate der Salze.

Eine weitere besondere Ausführungsform der vorliegenden Erfindung betrifft Verbindungen der Formel (I), in welcher
- Z³: für CH oder N steht,
sowie ihre Salze, Solvate und Solvate der Salze.

Eine weitere besondere Ausführungsform der vorliegenden Erfindung betrifft Verbindungen der Formel (I), in welcher
- Z⁴: für C-R⁹ steht, worin
R⁹ Pentafluorsulfanyl, (Trifluormethyl)sulfanyl, Trimethylsilyl, (C₁-C₄)-Alkyl, (C₁-C₄)-Alkoxy, Cyclopropyl, Cyclobutyl oder Oxetanyl bedeutet,
wobei (C₁-C₄)-Alkyl und (C₁-C₄)-Alkoxy bis zu sechsfach mit Fluor substituiert sein können
und
Cyclopropyl, Cyclobutyl und Oxetanyl bis zu zweifach, gleich oder verschieden, mit einem Rest ausgewählt aus der Reihe Fluor, Methyl, Trifluormethyl und Hydroxy substituiert sein können,
sowie ihre Salze, Solvate und Solvate der Salze.

Besonders bevorzugt im Rahmen der vorliegenden Erfindung sind Verbindungen der Formel (I), in welcher
- Ar^{N}: für 5- oder 6-gliedriges Aza-Heteroaryl der Formel 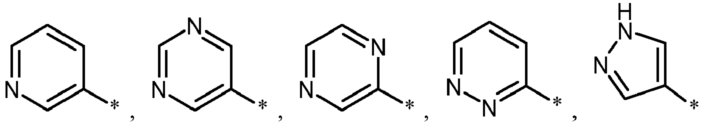 oder 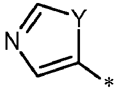 steht, worin * die Verknüpfung zur Imidazopyrazol-Gruppierung markiert
und
Y S oder NH bedeutet,
- R¹: für Wasserstoff oder Fluor steht,
- R²: für Wasserstoff oder Methyl steht,
- R³: für Wasserstoff steht,
- R^{4A}: für Chlor, Methyl oder Trifluormethyl steht,
- R^{4B}: für Wasserstoff, Fluor, Chlor oder Methyl steht,
- R⁵: für Wasserstoff, Fluor, Chlor oder Methyl steht,
- R⁶: für Wasserstoff, Fluor, Methyl oder Hydroxy steht,
- Z¹: für CH steht,
- Z²: für CH steht,
- Z³: für CH oder N steht,
- Z⁴: für C-R⁹ steht, worin
R⁹ Pentafluorsulfanyl, (Trifluormethyl)sulfanyl, Trifluormethyl, Trifluormethoxy, (C₂-C₄)-Alkyl, (C₂-C₄)-Alkoxy, Cyclopropyl, Cyclobutyl oder Oxetan-3-yl bedeutet,
wobei (C₂-C₄)-Alkyl und (C₂-C₄)-Alkoxy bis zu fünffach mit Fluor substituiert sein können
und
Cyclopropyl, Cyclobutyl und Oxetan-3-yl mit einem Rest ausgewählt aus der Reihe Fluor, Methyl, Trifluormethyl und Hydroxy substituiert sein können, und
- Z⁵: für C-R¹⁰ steht, worin
R¹⁰ Wasserstoff, Fluor, Chlor, Brom, Cyano, (C₁-C₄)-Alkyl, Hydroxy, (C₁-C₄)-Alkoxy, Methylsulfonyl, 1*H*-Imidazol-1-yl oder eine Gruppe der Formel -L¹-C(=O)-OR¹¹, -L¹₋NR^{12A}R^{12B}, -L¹-C(=O)-NR^{13A}R^{13b}, -L²-S(=O)₂-NR^{13A}R^{13B} oder -L³-R¹⁴ bedeutet, wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
1*H*-Imidazol-1-yl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A} und R^{12B} unabhängig voneinander Wasserstoff oder (C₁-C₄)-Alkyl darstellen oder
R^{12A} und R^{12B} miteinander verknüpft sind und gemeinsam mit dem Stickstoffatom, an das sie gebunden sind, einen 4- bis 6-gliedrigen Heterocyclus bilden, der ein weiteres Ring-Heteroatom aus der Reihe N oder O enthalten kann und der mit einem Rest ausgewählt aus der Reihe Cyano, Methyl, Hydroxy und Methoxy oder bis zu zweifach mit Fluor substituiert sein kann,
R^{13A} und R^{13B} unabhängig voneinander Wasserstoff oder (C₁-C₄)-Alkyl darstellen, und
R¹⁴ einen über ein Ring-Kohlenstoffatom gebundenen 4- bis 6-gliedrigen Heterocyclus darstellt, der als Ring-Heteroatom ein Stickstoffatom enthält und der mit einem Rest ausgewählt aus der Reihe Cyano, Methyl, Hydroxy und Methoxy oder bis zu zweifach mit Fluor substituiert sein kann,
sowie ihre Salze, Solvate und Solvate der Salze.

Eine weitere besonders bevorzugte Ausführungsform der vorliegenden Erfindung umfasst Verbindungen der Formel (I), in welcher
- Ar^{N}: für 5- oder 6-gliedriges Aza-Heteroaryl der Formel 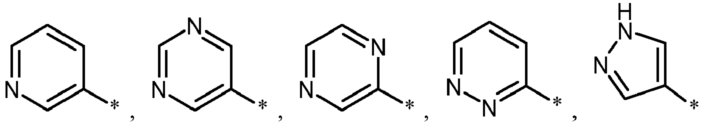 oder 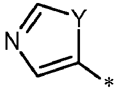 steht, worin * die Verknüpfung zur Imidazopyrazol-Gruppierung markiert
und
Y S oder NH bedeutet,
- R¹: für Wasserstoff steht,
- R²: für Wasserstoff oder Methyl steht,
- R³: für Wasserstoff steht,
- R^{4A}: für Chlor, Methyl oder Trifluormethyl steht,
- R^{4B}: für Wasserstoff, Fluor, Chlor oder Methyl steht,
- R⁵: für Wasserstoff, Fluor, Chlor oder Methyl steht,
- R⁶: für Wasserstoff, Fluor, Methyl oder Hydroxy steht,
- Z¹: für CH steht,
- Z²: für CH steht,
- Z³: für CH oder N steht,
- Z⁴: für C-R⁹ steht, worin
R⁹ Pentafluorsulfanyl, Trifluormethyl, Trifluormethoxy, (C₂-C₄)-Alkyl, (C₂-C₄)-Alkoxy, Cyclopropyl, Cyclobutyl oder Oxetan-3-yl bedeutet,
wobei (C₂-C₄)-Alkyl und (C₂-C₄)-Alkoxy bis zu dreifach mit Fluor substituiert sein können
und
Cyclopropyl, Cyclobutyl und Oxetan-3-yl mit einem Rest ausgewählt aus der Reihe Fluor, Methyl, Trifluormethyl und Hydroxy substituiert sein können, und

- Z⁵: für C-R¹⁰ steht, worin
R¹⁰ Wasserstoff, Fluor, Chlor, Brom, Cyano, (C₁-C₄)-Alkyl, Hydroxy, (C₁-C₄)-Alkoxy, Methylsulfonyl, 1*H*-Imidazol-1-yl oder eine Gruppe der Formel -L¹-C(=O)-OR¹¹, -L¹-NR^{12A}R^{12B}, -L¹-C(=O)-NR^{13A}R^{13b}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
1*H*-Imidazol-1-yl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A} und R^{12B} unabhängig voneinander Wasserstoff oder (C₁-C₄)-Alkyl darstellen oder
R^{12A} und R^{12B} miteinander verknüpft sind und gemeinsam mit dem Stickstoffatom, an das sie gebunden sind, einen 4- bis 6-gliedrigen Heterocyclus bilden, der ein weiteres Ring-Heteroatom aus der Reihe N oder O enthalten kann und der mit Methyl oder Hydroxy substituiert sein kann,
R^{13A} und R^{13B} unabhängig voneinander Wasserstoff oder (C₁-C₄)-Alkyl darstellen, und
R¹⁴ einen über ein Ring-Kohlenstoffatom gebundenen 4- bis 6-gliedrigen Heterocyclus darstellt, der als Ring-Heteroatom ein Stickstoffatom enthält und der mit Methyl oder Hydroxy substituiert sein kann,

sowie ihre Salze, Solvate und Solvate der Salze.

Ganz besonders bevorzugt im Rahmen der vorliegenden Erfindung sind Verbindungen der Formel (I), in welcher
- Ar^{N}: für 5- oder 6-gliedriges Aza-Heteroaryl der Formel 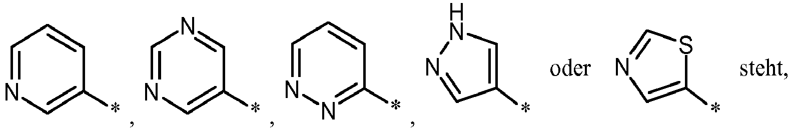 worin * die Verknüpfung zur Imidazopyrazol-Gruppierung markiert,
- R¹: für Wasserstoff steht,
- R²: für Wasserstoff oder Methyl steht,
- R³: für Wasserstoff steht,
- R^{4A}: für Chlor oder Methyl steht,
- R^{4B}: für Wasserstoff, Fluor, Chlor oder Methyl steht,
- R⁵: für Wasserstoff steht,
- R⁶: für Wasserstoff steht,
- Z¹: für CH steht,
- Z²: für CH steht,
- Z³: für CH oder N steht,
- Z⁴: für C-R⁹ steht, worin
R⁹ Pentafluorsulfanyl, (Trifluormethyl)sulfanyl, Trifluormethyl, 2-Fluorpropan-2-yl, tert.-Butyl, 1,1,1-Trifluor-2-methylpropan-2-yl, Trifluormethoxy, 1,1,2,2-Tetrafluorethoxy oder 3-Methyloxetan-3-yl bedeutet, und
Z⁵ für C-R¹⁰ steht, worin
R¹⁰ Wasserstoff, Fluor, Chlor, Cyano, (C₁-C₄)-Alkyl, Hydroxy, (C₁-C₄)-Alkoxy, Methylsulfonyl, 2-Methyl-1*H*-imidazol-1-yl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können,
und worin
L¹ eine Bindung oder -CH₂- darstellt,
L² eine Bindung darstellt,
L³ eine Bindung oder -O- darstellt,
R¹ Wasserstoff darstellt,
R^{12A} und R^{12B} unabhängig voneinander Wasserstoff oder Methyl darstellen oder
R^{12A} und R^{12B} miteinander verknüpft sind und gemeinsam mit dem Stickstoffatom, an das sie gebunden sind, einen Azetidin-1-yl-, Pyrrolidin-1-yl- oder Piperidin-1-yl-Ring, der jeweils mit einem Rest ausgewählt aus der Reihe Cyano, Hydroxy und Methoxy substituiert sein kann, oder einen Piperazin-1-yl-, 4-Methylpiperazin-1-yl- oder Morpholin-4-yl-Ring bilden,
R^{13A} und R^{13B} unabhängig voneinander Wasserstoff oder Methyl darstellen, und
R¹⁴ einen Azetidin-3-yl-, Pyrrolidin-3-yl-, Piperidin-3-yl- oder Piperidin-4-yl-Ring, der jeweils mit Hydroxy substituiert sein kann, darstellt,
sowie ihre Salze, Solvate und Solvate der Salze.

Eine weitere ganz besonders bevorzugte Ausführungsform der vorliegenden Erfindung umfasst Verbindungen der Formel (I), in welcher
- Ar^{N}: für 5- oder 6-gliedriges Aza-Heteroaryl der Formel 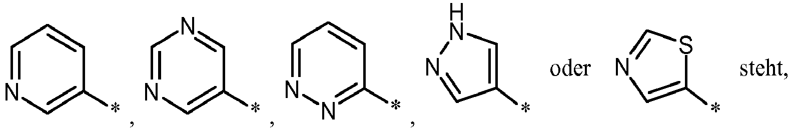 worin * die Verknüpfung zur Imidazopyrazol-Gruppierung markiert,
- R¹: für Wasserstoff steht,
- R²: für Wasserstoff oder Methyl steht,
- R³: für Wasserstoff steht,
- R^{4A}: für Chlor oder Methyl steht,
- R^{4B}: für Wasserstoff, Fluor, Chlor oder Methyl steht,
- R⁵: für Wasserstoff steht,
- R⁶: für Wasserstoff steht,
- Z¹: für CH steht,
- Z²: für CH steht,
- Z³: für CH oder N steht,
- Z⁴: für C-R⁹ steht, worin
R⁹ Pentafluorsulfanyl, Trifluormethyl, *tert*.-Butyl oder Trifluormethoxy bedeutet,
und
- Z⁵: für C-R¹⁰ steht, worin
R¹⁰ Fluor, Chlor, Cyano, (C₁-C₄)-Alkyl, Hydroxy, (C₁-C₄)-Alkoxy, Methylsulfonyl, 2-Methyl-1*H*-imidazol-1-yl oder eine Gruppe der Formel -L¹-C(=O)-OR¹¹, -L¹-NR^{12A}R^{12B}, -L¹-C(=O)-NR^{13A}R^{13b}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können,
und worin
L¹ eine Bindung oder -CH₂- darstellt,
L² eine Bindung darstellt,
L³ eine Bindung oder -O- darstellt,
R¹ Wasserstoff darstellt,
R^{12A} und R^{12B} unabhängig voneinander Wasserstoff oder Methyl darstellen oder
R^{12A} und R^{12B} miteinander verknüpft sind und gemeinsam mit dem Stickstoffatom, an das sie gebunden sind, einen Azetidin-1-yl-, Pyrrolidin-1-yl- oder Piperidin-1-yl-Ring, der jeweils mit Hydroxy substituiert sein kann, oder einen Piperazin-1-yl-, 4-Methylpiperazin-1-yl- oder Morpholin-4-yl-Ring bilden,
R^{13A} und R^{13B} unabhängig voneinander Wasserstoff oder Methyl darstellen, und
R¹⁴ einen Azetidin-3-yl-, Pyrrolidin-3-yl-, Piperidin-3-yl- oder Piperidin-4-yl-Ring, der jeweils mit Hydroxy substituiert sein kann, darstellt,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zweien oder mehreren der oben genannten Vorzugsbereiche.

Weiterer Gegenstand der vorliegenden Erfindung ist ein Verfahren zur Herstellung der erfindungsgemäßen Verbindungen der Formel (I), dadurch gekennzeichnet, dass man entweder

### [A] ein Anilin-Derivat der Formel (II)

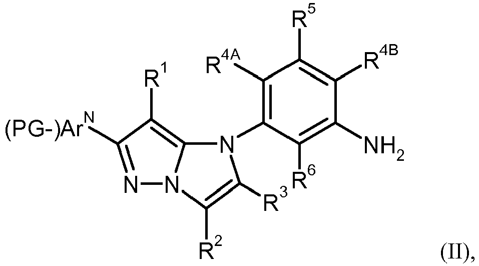

in welcher Ar^{N}, R¹, R², R³, R^{4A}, R^{4B}, R⁵ und R⁶ die oben angegebenen Bedeutungen haben
und
(PG-) eine optionale Stickstoff-Schutzgruppe im Fall, dass Y in Ar^{N} für NH steht, bedeutet,

in einem inerten Lösungsmittel in Gegenwart eines Kondensationsmittels mit einer Carbonsäure der Formel (III) 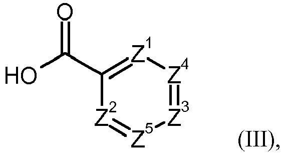 in welcher Z¹, Z², Z³, Z⁴ und Z⁵ die oben angegebenen Bedeutungen haben,
zum Carbonsäureamid der Formel (IV) 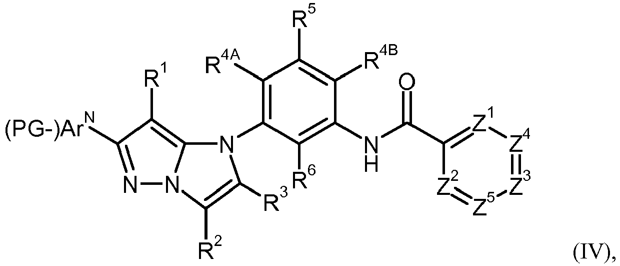 in welcher Ar^{N}, (PG-), R¹, R², R³, R^{4A},R^{4B}, R⁵, R⁶, Z¹, Z², Z³, Z⁴ und Z⁵ die oben angegebenen Bedeutungen haben,
kuppelt und anschließend die Schutzgruppe PG, falls vorhanden, abspaltet,
oder

### [B] ein 1H-Imidazo[1,2-b]pyrazol-Derivat der Formel (V)

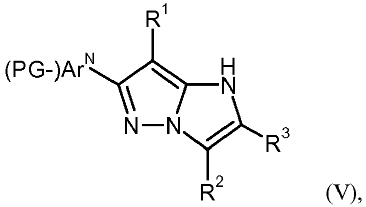

in welcher Ar^{N}, R¹, R² und R³ die oben angegebenen Bedeutungen haben
und
(PG-) eine optionale Stickstoff-Schutzgruppe im Fall, dass Y in Ar^{N} für NH steht, bedeutet,
in einem inerten Lösungsmittel unter Kupfer(I)-Katalyse mit einem Phenylbromid der Formel (VI) 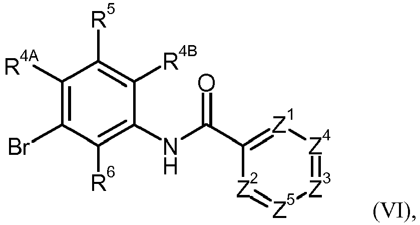 in welcher R^{4A}, R^{4B}, R⁵, R⁶, Z¹, Z², Z³, Z⁴ und Z⁵ die oben angegebenen Bedeutungen haben,
zum 1-Phenyl-1*H*-imidazo[1,2-b]pyrazol-Derivat der Formel (IV) 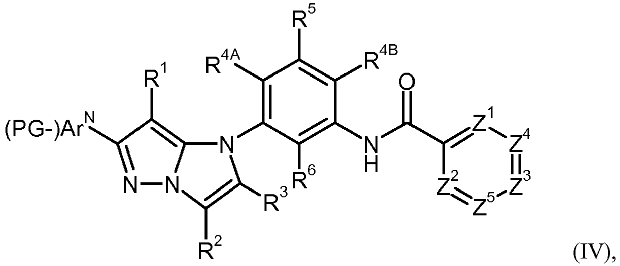 in welcher Ar^{N} (PG-), R¹, R², R³, R^{4A}, R^{4B}, R⁵, R⁶, Z¹, Z², Z³, Z⁴ und Z⁵ die oben angegebenen Bedeutungen haben,
kuppelt und anschließend die Schutzgruppe PG, falls vorhanden, abspaltet,
oder

### [C] ein Aminopyrazol-Derivat der Formel (VII)

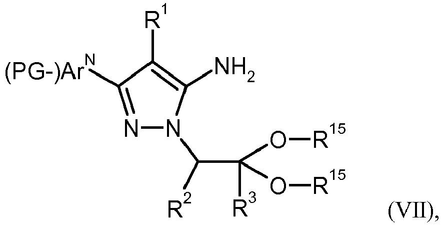

in welcher Ar^{N}, R¹, R² und R³ die oben angegebenen Bedeutungen haben,
R¹⁵ für Methyl oder Ethyl steht,
und
(PG-) eine optionale Stickstoff-Schutzgruppe im Fall, dass Y in Ar^{N} für NH steht, bedeutet,
in einem inerten Lösungsmittel unter Palladium-Katalyse mit einem Phenylbromid der Formel (VI) 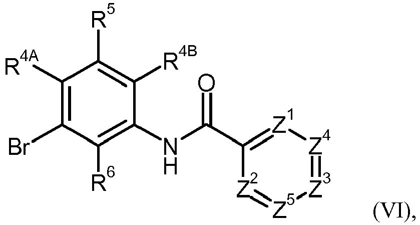 in welcher R^{4A}, R^{4B}, R⁵, R⁶, Z¹, Z², Z³, Z⁴ und Z⁵ die oben angegebenen Bedeutungen haben,
zu einer Verbindung der Formel (VIII) 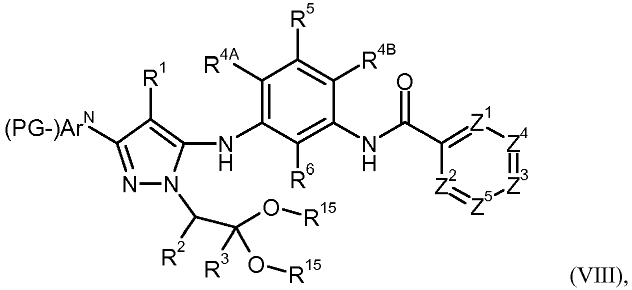 in welcher Ar^{N}, (PG-), R¹ R², R³, R^{4A}, R^{4B}, R⁵, R⁶, R¹⁵, Z¹, Z², Z³, Z⁴ und Z⁵ die oben angegebenen Bedeutungen haben,
kuppelt, die Verbindung der Formel (VIII) dann durch Säure-Behandlung zum 1-Phenyl-1*H* imidazo[1,2-b]pyrazol-Derivat der Formel (IV) 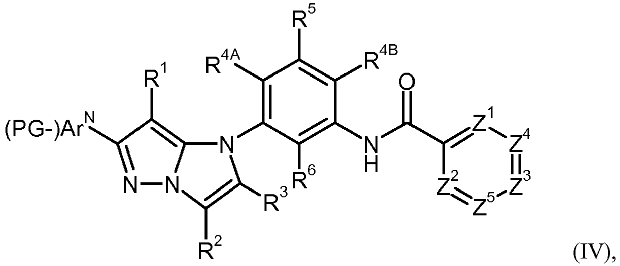 in welcher Ar^{N}, (PG-), R¹, R², R³, R^{4A}, R^{4B}, R⁵, R⁶, Z¹, Z², Z³, Z⁴ und Z⁵ die oben angegebenen Bedeutungen haben,
cyclisiert und anschließend die Schutzgruppe PG, falls vorhanden, abspaltet,
und gegebenenfalls die so erhaltenen Verbindungen der Formel (I) mit den entsprechenden (i) Lösungsmitteln und/oder (*ii*) Säuren oder Basen in ihre Solvate, Salze und/oder Solvate der Salze überführt.

Im Fall, dass die Gruppe Ar^{N} in Formel (I) für 5-gliedriges Aza-Heteroaryl der oben dargestellten Strukturen steht und das Ringglied Y darin für NH steht, kann es bei den zuvor beschriebenen Verfahrensschritten zweckmäßig oder erforderlich sein, dieses Ring-Stickstoffatom vorübergehend mit einer Schutzgruppe PG zu blockieren. Hierfür kommen bekannte Amino-Schutzgruppen wie insbesondere Benzyl, 4-Methoxybenzyl, 2,4-Dimethoxybenzyl oder Tetrahydro-2*H*-pyran-2-yl (THP) in Betracht. Einführung und Entfernung solcher Schutzgruppen erfolgen nach allgemein üblichen Methoden [siehe z.B. T.W. Greene und P.G.M. Wuts, Protective Groups in Organic Synthesis, Wiley, New York, 1999]. Bevorzugt wird die 4-Methoxybenzyl-Gruppe verwendet. Die Abspaltung dieser Schutzgruppe im Verfahrensschritt (IV) → (I) wird vorzugsweise mit Hilfe einer starken, wasserfreien Säure, wie Trifluoressigsäure, Chlorwasserstoff oder Bromwasserstoff, gegebenenfalls unter Zusatz eines inerten Lösungsmittels, wie Dichlormethan, 1,4-Dioxan beziehungsweise Eisessig, durchgeführt.

Inerte Lösungsmittel für den Verfahrensschritt [A] (II) + (III) → (IV) [Amid-Kupplung] sind beispielsweise Ether wie Diethylether, Diisopropylether, *tert.*-Butyl-methylether, Tetrahydrofuran, 1,4-Dioxan, 1,2-Dimethoxyethan oder Bis(2-methoxyethyl)ether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Acetonitril, Ethylacetat, Pyridin, Dimethylsulfoxid (DMSO), *N*,*N*-Dimethylformamid (DMF), *N*,*N*-Dimethylacetamid (DMA), *N*,*N'*-Dimethylpropylenharnstoff (DMPU) oder *N*-Methylpyrrolidinon (NMP). Ebenso ist es möglich, Gemische solcher Lösungsmittel einzusetzen. Bevorzugt werden Dichlormethan, Tetrahydrofuran, Dimethylformamid oder Gemische hiervon verwendet.

Als Kondensationsmittel für diese Kupplungsreaktion eignen sich beispielsweise Carbodiimide wie *N*,*N'*-Diethyl-, *N*,*N'*-Dipropyl-, *N*,*N'*-Diisopropyl-, *N*,*N'*-Dicyclohexylcarbodiimid (DCC) oder *N*-(3-Dimethylaminopropyl)-*N'*-ethylcarbodiimid-Hydrochlorid (EDC), Phosgen-Derivate wie *N*,*N'*-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat (BOP) oder Benzotriazol-1-yl-oxy-tris(pyrrolidino)phosphonium-hexafluorophosphat (PyBOP), oder Uronium-Verbindungen wie *O*-(Benzotriazol-1-yl)-*N*,*N*,*N'*,*N'*-tetramethyluronium-tetrafluoroborat (TBTU), *O*-(Benzotriazol-1-yl)-*N*,*N*,*N'*,*N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N*,*N*,*N'*,*N'*-tetramethyl-uronium-hexafluorophosphat (HATU) oder *O-*(1*H-*6-Chlorbenzotriazol-1-yl)-1,1,3,3-tetramethyl-uronium-tetrafluoroborat (TCTU), gegebenenfalls in Kombination mit einer Aktivester-Komponente wie 1-Hydroxybenzotriazol (HOBt), *N*-Hydroxysuccinimid (HOSu), 4-Nitrophenol oder Pentafluorphenol, sowie als Base einem Alkalicarbonat, z.B. Natrium- oder Kaliumcarbonat, oder tertiären Amin wie Triethylamin, *N*-Methylmorpholin, *N*-Methylpiperidin, *N*,*N*-Diisopropylethyl-amin, Pyridin oder 4-*N*,*N*-Dimethylaminopyridin. Bevorzugt eingesetzt werden *O*-(7-Azabenzotriazol-1-yl)-*N*,*N*,*N*',*N'*-tetramethyluronium-hexafluorophosphat (HATU) oder *O*-(Benzotriazol-1-yl)-*N*,*N*,*N'*,*N'*-tetramethyluronium-tetrafluoroborat (TBTU) in Kombination mit *N*-Methylmorpholin oder *N*,*N-*Diisopropylethylamin als Base.

Die Umsetzung [A] (II) + (III) —> (IV) erfolgt in der Regel in einem Temperaturbereich von -20°C bis +60°C, bevorzugt bei 0°C bis +40°C. Die Reaktion kann bei normalem, bei erhöhtem oder bei erniedrigtem Druck durchgeführt werden (z.B. von 0.5 bis 5 bar); im Allgemeinen arbeitet man bei Normaldruck.

Die Kupplungsreaktion [B] (V) + (VI) → (IV) wird mit Hilfe eines Kupfer(I)-Katalysators, wie Kupfer(I)oxid, -bromid oder -iodid, in Gegenwart eines Kupfer-Liganden, wie 8-Hydroxychinolin oder 1,10-Phenanthrolin, und einer anorganischen oder organischen Carbonat-Base, wie Kalium-, Cäsium- oder Bis(tetraethylammonium)carbonat, durchgeführt. Als inerte Lösungsmittel für diese Umsetzung eignen sich insbesondere Toluol, Xylol, 1,4-Dioxan, Acetonitril, Dimethylsulfoxid (DMSO), *N*,*N*-Dimethylformamid (DMF) oder Gemische hiervon, gegebenenfalls unter Zusatz von Wasser. Bevorzugt wird ein System bestehend aus Kupfer(I)iodid, 8-Hydroxychinolin und Bis(tetraethylammonium)carbonat in Dimethylformamid mit etwa 10% Wasser-Zusatz eingesetzt [vgl. L. Liu et al., J. Org. Chem. 70 (24), 10135-10138 (2005) und dort zitierte weitere Literatur]. Die Reaktion erfolgt im Allgemeinen in einem Temperaturbereich von +100°C bis +200°C, vorteilhafterweise unter Verwendung eines Mikrowellenofens.

Geeignete Palladium-Katalysatoren für die Kupplungsreaktion [C] (VII) + (VI) → (VIII) sind beispielsweise Palladium(II)acetat, Palladium(II)chlorid, Bis(triphenylphosphin)palladium(II)chlorid, Bis(acetonitril)palladium(II)chlorid, Tetrakis(triphenylphosphin)palladium(0), Bis(dibenzylidenaceton)palladium(0), Tris(dibenzylidenaceton)dipalladium(0) oder [1,1'-Bis(diphenylphosphino)-ferrocen]palladium(II)chlorid, jeweils in Kombination mit einem geeigneten Phosphin-Liganden wie zum Beispiel 2-Dicyclohexylphosphino-2',4',6'-triisopropylbiphenyl (X-Phos), 2-Dicyclohexylphosphino-2',6'-dimethoxybiphenyl (S-Phos), 1,2,3,4,5-Pentaphenyl-1'-(di-*tert*.-butylphosphino)ferrocen (Q-Phos), 4,5-Bis(diphenylphosphino)-9,9-dimethylxanthen (Xantphos), 2,2'-Bis(diphenyl-phosphino)-1,1'-binaphthyl (BINAP), 2-Dicyclohexylphosphino-2'-(*N*,*N*-dimethylamino)biphenyl oder 2-Di-*tert*.-butylphosphino-2'-(*N*,*N-*dimethylamino)biphenyl.

Die Kupplungsreaktion [C] (VII) + (VI) → (VIII) wird in der Regel in Gegenwart einer Base durchgeführt. Als solche eignen sich insbesondere Alkalicarbonate wie Natrium-, Kalium- oder Cäsiumcarbonat, Alkaliphosphate wie Natrium- oder Kaliumphosphat, Alkalifluoride wie Kalium-oder Cäsiumfluorid, oder Alkali-*tert*.-butylate wie Natrium- oder Kalium-tert.-butylat. Die Umsetzung erfolgt in einem inerten Lösungsmittel wie beispielsweise Toluol, 1,2-Dimethoxyethan, Tetrahydrofuran, 1,4-Dioxan, Dimethylsulfoxid (DMSO), *N*,*N*-Dimethylformamid (DMF), *N*,*N-*Dimethylacetamid (DMA) oder Mischungen hiervon in einem Temperaturbereich von +80°C bis +200°C, wobei ein Erhitzen mittels Mikrowellenapparatur auch hier von Vorteil sein kann.

Bevorzugt wird für diese Kupplungsreaktion ein Katalysator/Ligand/Base-System bestehend aus Palladium(II)acetat, 4,5-Bis(diphenylphosphino)-9,9-dimethylxanthen (Xantphos) und Cäsiumcarbonat eingesetzt und 1,4-Dioxan als Lösungsmittel verwendet.

Die Cyclisierung [C] (VIII) → (IV) wird vorzugsweise durch Erhitzen des Acetals (VIII) mit einer wässrigen Säure, wie beispielsweise Schwefelsäure, in einem alkoholischen Lösungsmittel wie Methanol oder Ethanol bewerkstelligt, wobei eine Reaktionsführung unter Mikrowellen-Einstrahlung wiederum von Nutzen sein kann.

Im Fall, dass die Aza-Heteroaryl-Gruppe Ar^{N} in (VIII) in THP-geschützter Form vorliegt (PG = Tetrahydro-2*H*-pyran-2-yl), erfolgt unter den genannten Reaktionsbedingungen der Cyclisierung auch eine Abspaltung dieser Schutzgruppe, so dass hier direkt die entsprechende erfindungsgemäße Verbindung der Formel (I) als Reaktionsprodukt erhalten wird. Bei Anwesenheit einer Benzyl-, 4-Methoxybenzyl- oder 2,4-Dimethoxybenzyl-Schutzgruppe in Ar^{N} wird diese in einem nachfolgenden, separaten Reaktionsschritt (IV) → (I), wie oben beschrieben, entfernt.

Weitere erfindungsgemäße Verbindungen der Formel (I) können, falls zweckmäßig, auch durch Umwandlungen von funktionellen Gruppen einzelner Reste und Substituenten, insbesondere den unter R⁹ und R¹⁰ aufgeführten, hergestellt werden, wobei von anderen, nach obigen Verfahren erhaltenen Verbindungen der Formel (I) oder deren Vorstufen ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen (z.B. Ullmann-Reaktion, Buchwald-Hartwig-Reaktion, Suzuki-Kupplung, Negishi-Kupplung), Additionsreaktionen von Metallorganylen (z.B. Grignard-Verbindungen oder Lithiumorganylen) an Carbonylverbindungen, Oxidations- und Reduktionsreaktionen, Hydrierung, Alkylierung, Acylierung, Sulfonylierung, Aminierung, Hydroxylierung, die Bildung von Nitrilen, Carbonsäureestern, Carbonsäureamiden und Sulfonsäureamiden, die Esterspaltung und -hydrolyse sowie die Einführung und Entfernung temporärer Schutzgruppen.

Ebenso können, falls zweckmäßig, erfindungsgemäße Verbindungen der Formel (I) auch dadurch hergestellt werden, dass man bei den Ausgangsverbindungen der zuvor beschriebenen Verfahrensvarianten anstelle der Substituenten R⁹ und/oder R¹⁰ zunächst andere funktionelle Gruppen außerhalb des Bedeutungsumfangs von R⁹ bzw. R¹⁰ einsetzt, die dann durch nachfolgende, dem Fachmann geläufige Transformationen (wie oben beispielhaft aufgeführt) in die jeweiligen Substituenten R⁹ bzw. R¹⁰ umgewandelt werden. Beispiele für solche als "Vorstufe" zu R⁹ und/oder R¹⁰ dienende funktionelle Gruppen sind Reste wie Chlor, Brom, Iod, Nitro, Hydroxy, Methansulfonat (Mesylat), Trifluormethansulfonat (Triflat), Formyl und Alkylcarbonyl [vgl. auch die im nachfolgenden Experimentellen Teil detailliert beschriebene Herstellung der Ausführungsbeispiele und ihrer Vorstufen].

Das Aminopyrazol-Intermediat der Formel (VII) aus Verfahrensweg [C] kann durch säurekatalysierte Kondensation eines Cyano-enamins oder -enols der Formel (IX) 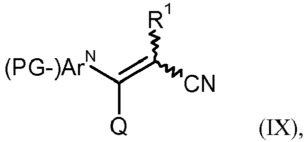 in welcher Ar^{N}, (PG-) und R¹ die oben angegebenen Bedeutungen haben
und
Q für NH₂ oder OH steht,
mit einem Hydrazino-acetal der Formel (X) 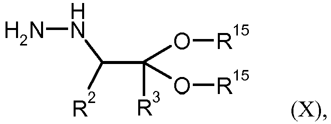 in welcher R², R³ und R¹⁵ die oben angegebenen Bedeutungen haben,
hergestellt werden. Die Umsetzung wird vorzugsweise in einem alkoholischen Lösungsmittel wie Methanol oder Ethanol in einem Temperaturbereich von +60°C bis +120°C durchgeführt, wobei die Verwendung eines Mikrowellenofens von Vorteil ist. Als Säurekatalysator ist insbesondere wässrige Salzsäure geeignet. Anstelle der Enole der Formel (IX) [Q = OH] können auch entsprechenden Enolat-Salze, wie beispielsweise Lithium-, Natrium- oder Kaliumenolate, in der Reaktion eingesetzt werden.

Das 1*H*-Imidazo[1,2-b]pyrazol-Intermediat der Formel (V) aus Verfahrensweg [B] ist analog zur Umsetzung [C] (VIII) → (IV) durch säurekatalysierte Cyclisierung des Aminopyrazol-Acetals (VII) zugänglich.

Ebenfalls ausgehend vom Aminopyrazol (VII) kann das Anilin-Intermediat der Formel (II) aus Verfahrensweg [A] durch (*i*) Palladium-katalysierte Kupplung von (VII) mit einem *meta*-Nitrophenylbromid der Formel (XI) 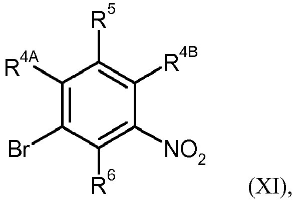 in welcher R^{4A}, R^{4B}, R⁵ und R⁶ die oben angegebenen Bedeutungen haben,
zur *N*-arylierten Verbindung der Formel (XII) 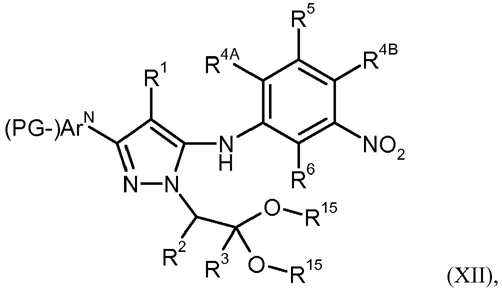 in welcher Ar^{N}, (PG-), R¹, R², R³, R^{4A}, R^{4B}, R⁵, R⁶ und R¹⁵ die oben angegebenen Bedeutungen haben,
(*ii*) nachfolgende säurekatalysierte Cyclisierung zum 1-Phenyl-1*H*-imidazo[1,2-b]pyrazol der Formel (XIII) 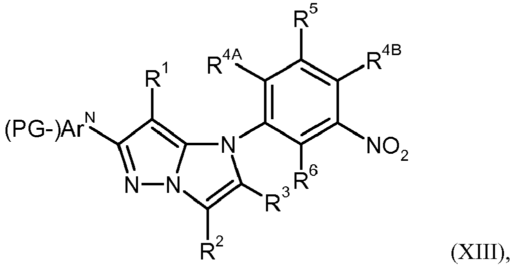 in welcher Ar^{N}, (PG-), R¹, R², R³, R^{4A}, R^{4B}, R⁵ und R⁶ die oben angegebenen Bedeutungen haben,
und (*iii*) anschließende Reduktion der Nitro-Gruppe in (XIII) erhalten werden.

Die Kupplungsreaktion (VII) + (XI) → (XII) und die Cyclisierung (XII) → (XIII) werden auf analoge Weise durchgeführt wie zuvor im Rahmen des Verfahrenswegs [C] für die Umsetzungen (VII) + (VI) → (VIII) bzw. (VIII) → (IV) beschrieben.

Die Reduktion der Nitro-Gruppe zum Amin im Verfahrensschritt (XIII) → (II) kann beispielsweise mit Hilfe von Zinn(II)chlorid oder durch katalytische Hydrierung mit gasförmigem Wasserstoff oder, im Sinne einer Transfer-Hydrierung, in Gegenwart von Wasserstoff-Donatoren wie Ammoniumformiat, Cyclohexen oder Cyclohexadien erfolgen. Bevorzugte Methode ist die Palladium(0)-katalysierte Hydrierung mit Ammoniumformiat. Die Reaktion wird vorzugsweise in einem alkoholischen Lösungsmittel wie Methanol oder Ethanol, gegebenenfalls unter Zusatz von Wasser, in einem Temperaturbereich von +20°C bis +100°C durchgeführt.

Das *meta*-Amidophenylbromid-Intermediat der Formel (VI) aus den Verfahrenswegen [B] und [C] ist auf einfache Weise durch Kupplung eines Anilins der Formel (XIV) 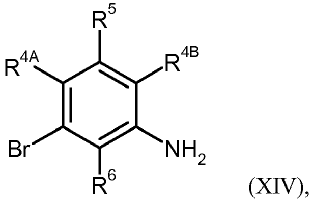 in welcher R^{4A}, R^{4B}, R⁵ und R⁶ die oben angegebenen Bedeutungen haben,
mit einer Carbonsäure der Formel (III) oder einem entsprechenden Carbonsäurechlorid der Formel (XV) 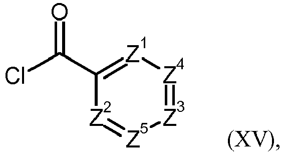 in welcher Z¹, Z², Z³, Z⁴ und Z⁵ die oben angegebenen Bedeutungen haben,
zugänglich.

Die Carbonsäure-Amidierung (XIV) + (III) → (VI) erfolgt nach üblichen Methoden mit Hilfe eines Kondensationsmittels unter ähnlichen Reaktionsbedingungen, wie sie zuvor schon für die analoge Umsetzung [A] (II) + (III) → (IV) beschrieben wurden. Bevorzugt wird als Kondensationsmittel *O*-(7-Azabenzotriazol-1-yl)-*N,N,N',N'*-tetramethyluronium-hexafluorophosphat (HATU) und als Base *N,N*-Diisopropylethylamin eingesetzt.

Bei Verwendung des entsprechenden Carbonsäurechlorids (XV) erfolgt die Kupplung mit der AminKomponente (XIV) in Gegenwart einer üblichen organischen Hilfsbase wie Triethylamin, *N,N*-Diisopropylethylamin, *N*-Methylmorpholin, *N*-Methylpiperidin, Pyridin, 2,6-Lutidin, 4-*N,N*-Dimethylaminopyridin, 1,8-Diazabicyclo[5.4.0]undec-7-en (DBU) oder 1,5-Diazabicyclo[4.3.0]non-5-en (DBN). Bevorzugt wird Triethylamin oder *N,N*-Diisopropylethylamin verwendet. Die Reaktion wird im Allgemeinen in einem inerten Lösungsmittel wie Dichlormethan in einem Temperaturbereich von-20°C bis +60°C, bevorzugt bei 0°C bis +40°C, durchgeführt.

Die Verbindungen der Formeln (III), (IX), (X), (XI), (XIV) und (XV) sind kommerziell erhältlich oder als solche in der Literatur beschrieben, oder sie können auf für den Fachmann offenkundigem Wege in Analogie zu in der Literatur publizierten Methoden hergestellt werden. Zahlreiche ausführliche Vorschriften sowie Literaturangaben zur Herstellung der jeweiligen Ausgangsmaterialien befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden: 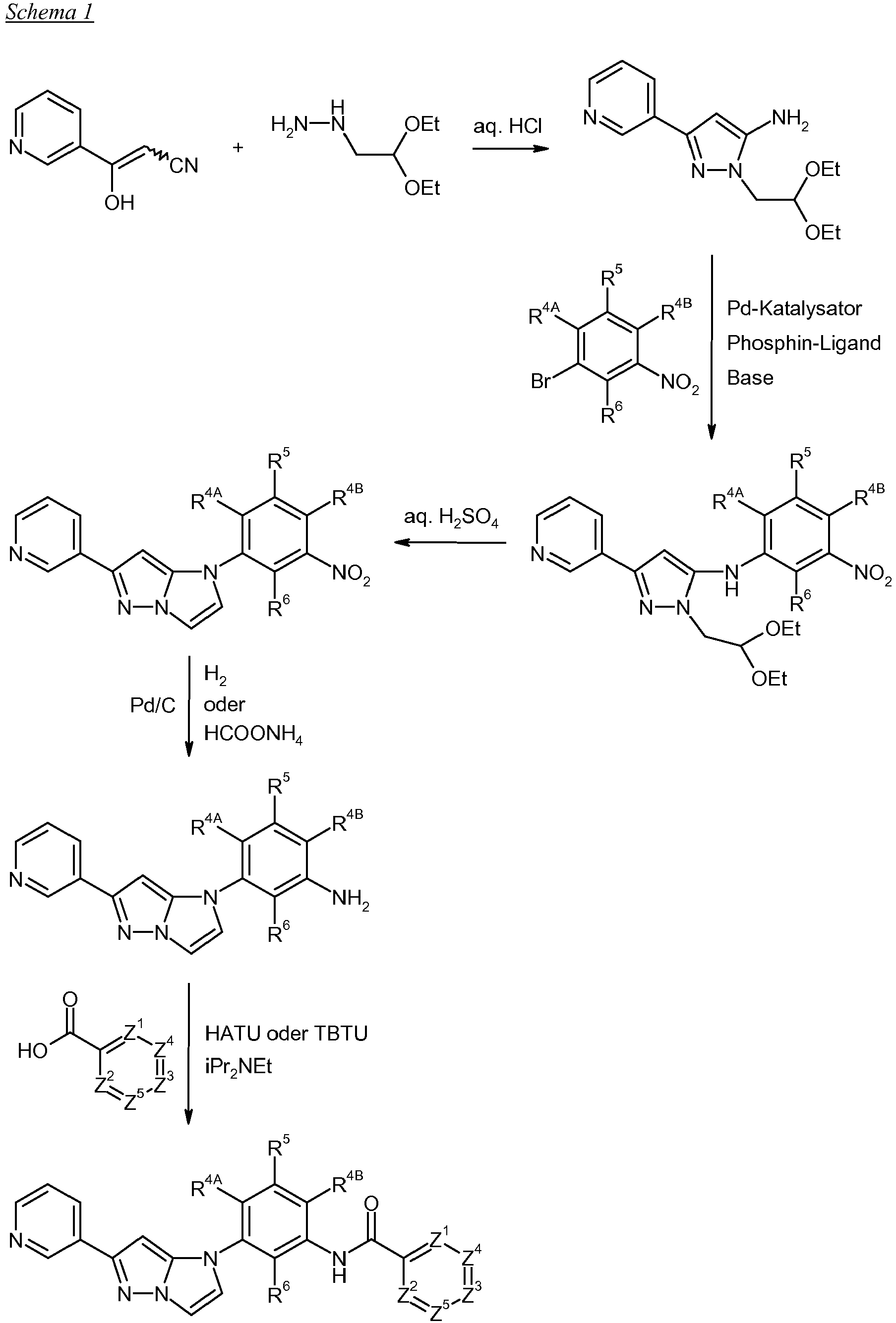 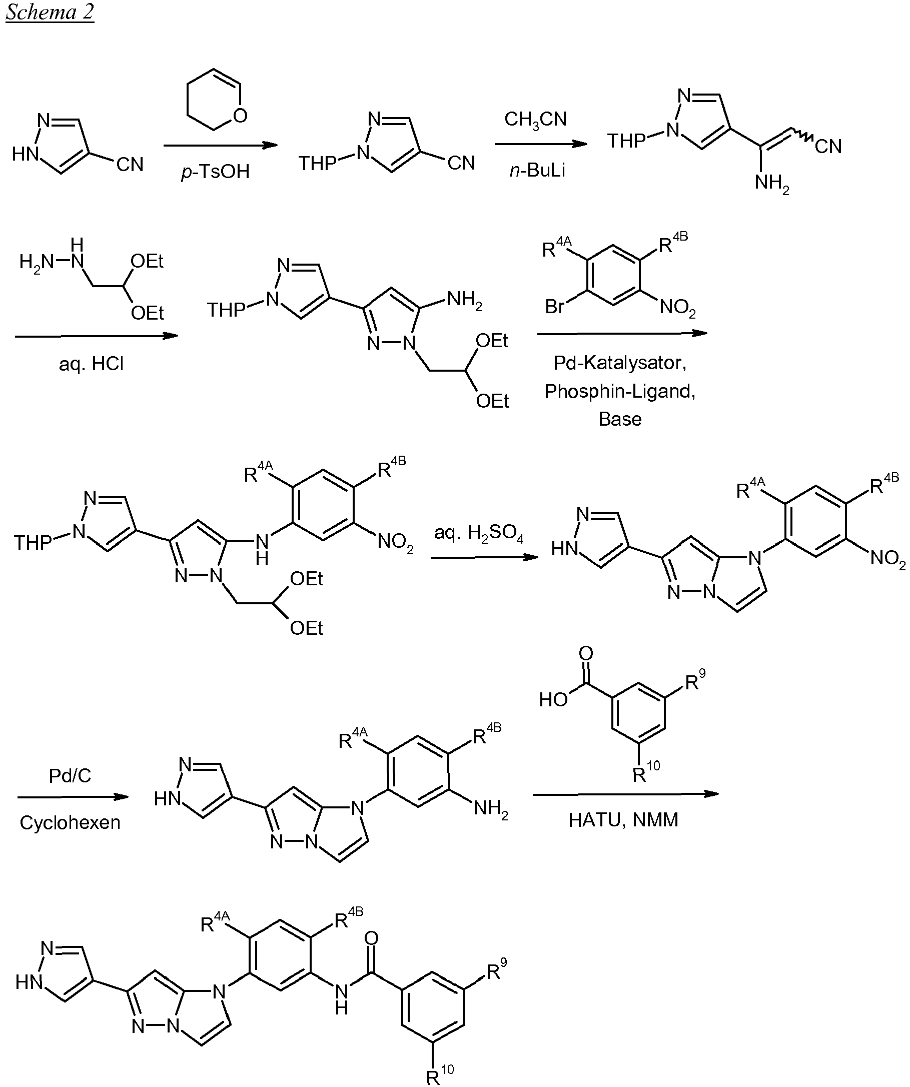 [THP = Tetrahydro-2*H*-pyran-2-yl]. 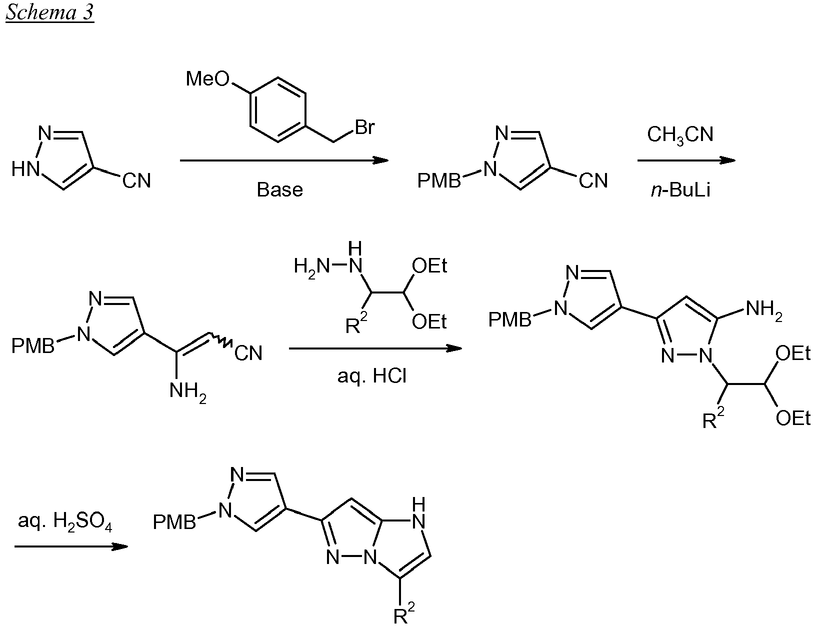 [PMB = *para*-Methoxybenzyl].

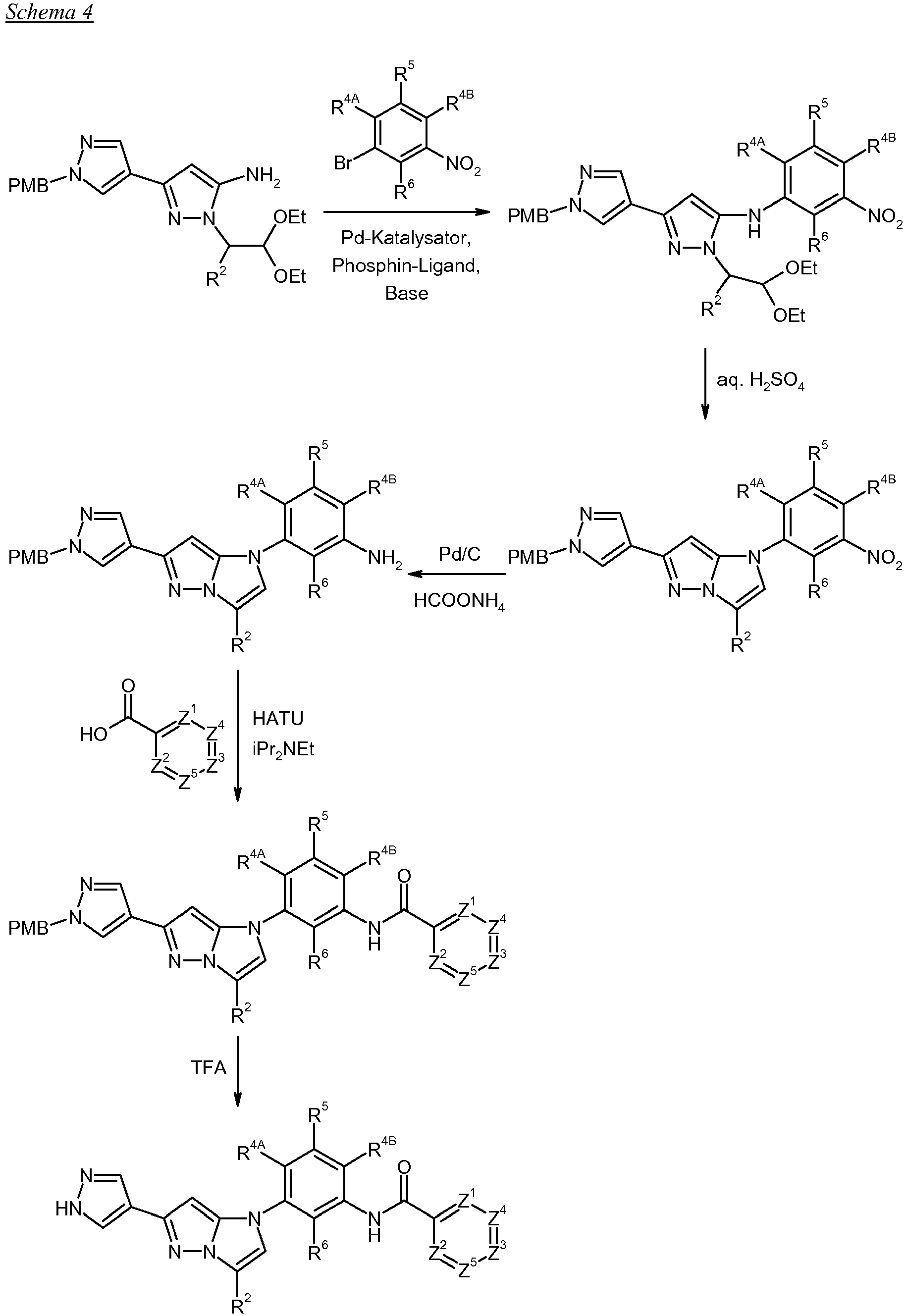

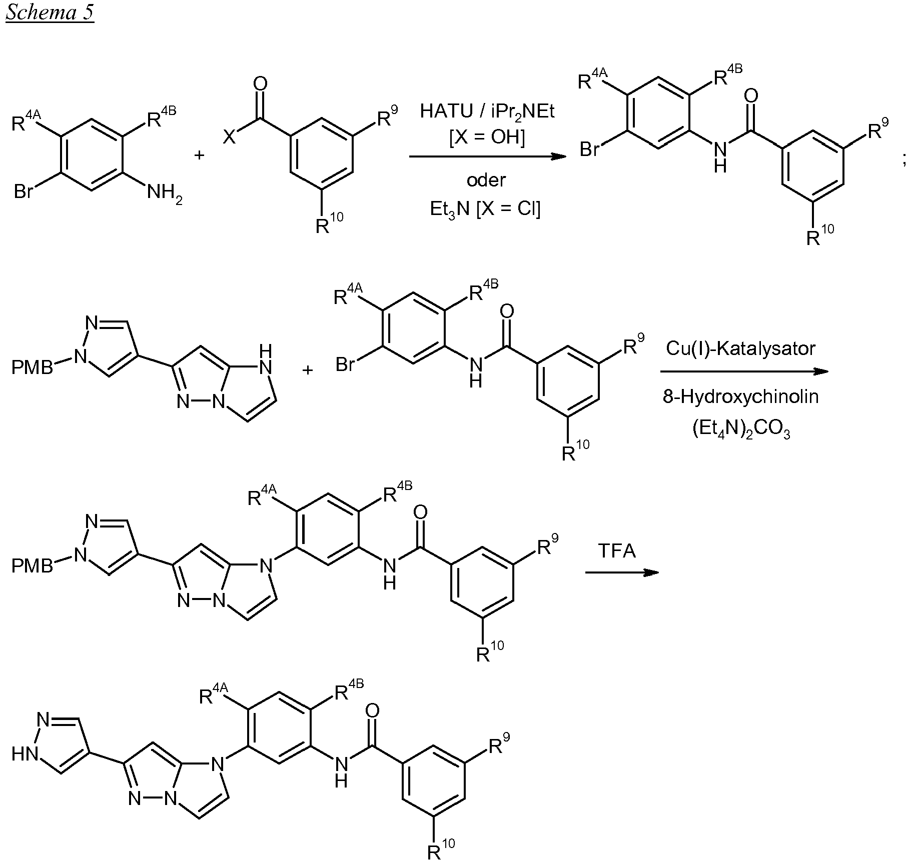

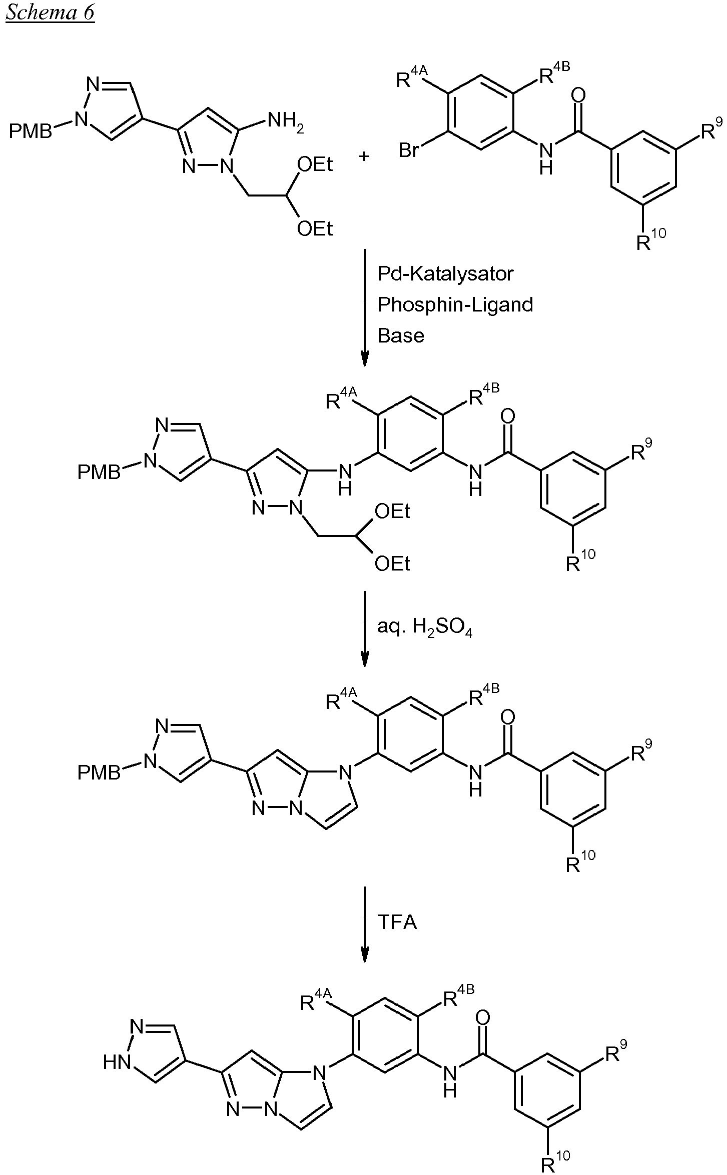

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen hochpotente Inhibitoren der Tie2-Rezeptorkinase dar und können oral appliziert werden. Aufgrund dieses Wirkprofils eignen sich die erfindungsgemäßen Verbindungen insbesondere zur Behandlung von angiogenen Erkrankungen beim Menschen und bei Säugetieren allgemein.

Zu diesen angiogenen Erkrankungen gehören insbesondere Krebs- und Tumorerkrankungen, worunter im Rahmen der vorliegenden Erfindung insbesondere die folgenden Erkrankungen verstanden werden, ohne jedoch auf sie beschränkt zu sein: Brustkarzinome und Brusttumore (Mammakarzinome einschließlich ductaler und lobulärer Formen, auch *in situ*), Atemwegstumore (kleinzelliges und nicht-kleinzelliges Lungenkarzinom, Bronchialkarzinome), Hirntumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischt-hepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloische, akute lymphoblastische, chronische myeloische, chronische lymphozytische und Haarzell-Leukämie, multiples Myelom (Plasmozytom) sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Die Behandlung der zuvor genannten Krebserkrankungen kann im Sinne der vorliegenden Erfindung sowohl eine Behandlung der soliden Tumore als auch eine Behandlung metastasierter oder zirkulierender Formen hiervon umfassen.

Aufgrund ihres Wirkprofils eignen sich die erfindungsgemäßen Verbindungen insbesondere zur Behandlung von Brust-, Kolorektal-, Leber-, Nieren- und Ovarialkarzinomen, Glioblastomen, akuter myeloischer Leukämie (AML), chronischer myeloischer Leukämie (CML) und multiplem Myelom. akuter myeloischer Leukämie (AML), chronischer myeloischer Leukämie (CML) und multiplem Myelom.

Die Verbindungen der vorliegenden Erfindung können ferner zur Behandlung von Gefäßfehlbildungen, wie Hämangiomen, Hämangioblastomen, Kavernomen und Lymphangiomen, sowie weiterer Erkrankungen, die mit exzessiver oder anormaler Angiogenese verbunden sind, verwendet werden. Hierzu gehören unter anderem diabetische Retinopathie, ischämische Retinalvenenokklusion und Retinopathie bei Frühgeburt, altersabhängige Makuladegeneration, neovaskuläres Glaukom, Psoriasis, retrolentale Fibroplasie, Angiofibrom, Entzündung, rheumatische Arthritis, Restenose, in-stent-Restenose sowie Restenose nach Gefäßimplantation, Endometriose, Nierenerkrankungen (z.B. Glomerulonephritis, diabetische Nephropathie, maligne Nephrosklerose) und fibrotische Erkrankungen (z.B. Leberzirrhose, Mesangiose, Arteriosklerose). Darüber hinaus eignen sich die erfindungsgemäßen Verbindungen auch zur Behandlung der pulmonalen Hypertonie.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Der Begriff "Behandlung" oder "behandeln" umfasst im Sinne der vorliegenden Erfindung ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-angiogenen, anti-hyperproliferativen, zytostatischen oder zytotoxischen Substanzen zur Behandlung von Krebserkrankungen kombiniert werden. Als geeignete Kombinationswirkstoffe seien beispielhaft genannt:
Abarelix, Abirateron, Aclarubicin, Afatinib, Aflibercept, Aldesleukin, Alemtuzumab, Alitretinoin, Altretamin, AMG-386, Aminoglutethimid, Amonafid, Amrubicin, Amsacrin, Anastrozol, Andromustin, Arglabin, Asparaginase, Axitinib, 5-Azacitidin, Basiliximab, Belotecan, Bendamustin, Bevacizumab, Bexaroten, Bicalutamid, Bisantren, Bleomycin, Bortezomib, Bosutinib, Brivanib-Alaninat, Buserelin, Busulfan, Cabazitaxel, Calciumfolinat, Calciumlevofolinat, Camptothecin, Capecitabin, Carboplatin, Carmofur, Carmustin, Catumaxomab, Cediranib, Celmoleukin, Cetuximab, Chlorambucil, Chlormadinon, Chlormethin, Cidofovir, Cisplatin, Cladribin, Clodronsäure, Clofarabin, Combretastatin, Crisantaspase, Crizotinib, CVX-060, Cyclophosphamid, Cyproteron, Cytarabin, Dacarbazin, Dactinomycin, Darbepoetin-alfa, Darinaparsin, Dasatinib, Daunorubicin, Decitabin, Degarelix, Denileukin-Diftitox, Denosumab, Deslorelin, Dibrospidiumchlorid, Docetaxel, Dovitinib, Doxifluridin, Doxorubicin, Dutasterid, Eculizumab, Edrecolomab, Eflornithin, Elliptiniumacetat, Eltrombopag, Endostatin, Enocitabin, Epimbicin, Epirubicin, Epitiostanol, Epoetin-alfa, Epoetin-beta, Epothilon, Eptaplatin, Eribulin, Erlotinib, Estradiol, Estramustin, Etoposid, Everolimus, Exatecan, Exemestan, Exisulind, Fadrozol, Fenretinid, Filgrastim, Finasterid, Flavopiridol, Fludarabin, 5-Fluoruracil, Fluoxymesteron, Flutamid, Foretinib, Formestan, Fotemustin, Fulvestrant, Ganirelix, Gefitinib, Gemcitabin, Gemtuzumab, Gimatecan, Gimeracil, Glufosfamid, Glutoxim, Goserelin, Histrelin, Hydroxyharnstoff, Ibandronsäure, Ibritumomab-Tiuxetan, Idarubicin, Ifosfamid, Imatinib, Imiquimod, Improsulfan, Intedanib, Interferon-alpha, Interferon-alpha-2a, Interferon-alpha-2b, Interferon-beta, Interferon-gamma, Interleukin-2, Ipilimumab, Irinotecan, Ixabepilon, Lanreotid, Lapatinib, Lasofoxifen, Lenalidomid, Lenograstim, Lentinan, Lestaurtinib, Letrozol, Leuprorelin, Levamisol, Linifanib, Linsitinib, Lisurid, Lobaplatin, Lomustin, Lonidamin, Lurtotecan, Mafosfamid, Mapatumumab, Masitinib, Masoprocol, Medroxyprogesteron, Megestrol, Melarsoprol, Melphalan, Mepitiostan, Mercaptopurin, Methotrexat, Methyl-Aminolevulinat, Methyltestosteron, Mifamurtid, Mifepriston, Miltefosin, Miriplatin, Mitobronitol, Mitoguazon, Mitolactol, Mitomycin, Mitotan, Mitoxantron, Molgramostim, Motesanib, Nandrolon, Nedaplatin, Nelarabin, Neratinib, Nilotinib, Nilutamid, Nimotuzumab, Nimustin, Nitracrin, Nolatrexed, Ofatumumab, Oprelvekin, Oxaliplatin, Paclitaxel, Palifermin, Pamidronsäure, Panitumumab, Pazopanib, Pegaspargase, Peg-epoetin-beta, Pegfilgrastim, Peg-interferon-alpha-2b, Pelitrexol, Pemetrexed, Pemtumomab, Pentostatin, Peplomycin, Perfosfamid, Idarubicin, Ifosfamid, Imatinib, Imiquimod, Improsulfan, Intedanib, Interferon-alpha, Interferon-alpha-2a, Interferon-alpha-2b, Interferon-beta, Interferon-gamma, Interleukin-2, Ipilimumab, Irinotecan, Ixabepilon, Lanreotid, Lapatinib, Lasofoxifen, Lenalidomid, Lenograstim, Lentinan, Lestaurtinib, Letrozol, Leuprorelin, Levamisol, Linifanib, Linsitinib, Lisurid, Lobaplatin, Lomustin, Lonidamin, Lurtotecan, Mafosfamid, Mapatumumab, Masitinib, Masoprocol, Medroxyprogesteron, Megestrol, Melarsoprol, Melphalan, Mepitiostan, Mercaptopurin, Methotrexat, Methyl-Aminolevulinat, Methyltestosteron, Mifamurtid, Mifepriston, Miltefosin, Miriplatin, Mitobronitol, Mitoguazon, Mitolactol, Mitomycin, Mitotan, Mitoxantron, Molgramostim, Motesanib, Nandrolon, Nedaplatin, Nelarabin, Neratinib, Nilotinib, Nilutamid, Nimotuzumab, Nimustin, Nitracrin, Nolatrexed, Ofatumumab, Oprelvekin, Oxaliplatin, Paclitaxel, Palifermin, Pamidronsäure, Panitumumab, Pazopanib, Pegaspargase, Peg-epoetin-beta, Pegfilgrastim, Peg-interferon-alpha-2b, Pelitrexol, Pemetrexed, Pemtumomab, Pentostatin, Peplomycin, Perfosfamid, Pertuzumab, Picibanil, Pirambicin, Pirarubicin, Plerixafor, Plicamycin, Poliglusam, Polyestradiol-Phosphat, Porfimer-Natrium, Pralatrexat, Prednimustin, Procarbazin, Procodazol, Quinagolid, Raloxifen, Raltitrexed, Ranibizumab, Ranimustin, Razoxan, Regorafenib, Risedronsäure, Rituximab, Romidepsin, Romiplostim, Rubitecan, Saracatinib, Sargramostim, Satraplatin, Selumetinib, Sipuleucel-T, Sirolimus, Sizofiran, Sobuzoxan, Sorafenib, Streptozocin, Sunitinib, Talaporfin, Tamibaroten, Tamoxifen, Tandutinib, Tasonermin, Teceleukin, Tegafur, Telatinib, Temoporfin, Temozolomid, Temsirolimus, Teniposid, Testolacton, Testosteron, Tetrofosmin, Thalidomid, Thiotepa, Thymalfasin, Tioguanin, Tipifarnib, Tivozanib, Toceranib, Tocilizumab, Topotecan, Toremifen, Tositumomab, Trabectedin, Trastuzumab, Treosulfan, Tretinoin, Triapin, Trilostan, Trimetrexat, Triptorelin, Trofosfamid, Ubenimex, Valrubicin, Vandetanib, Vapreotid, Varlitinib, Vatalanib, Vemurafenib, Vidarabin, Vinblastin, Vincristin, Vindesin, Vinflunin, Vinorelbin, Volociximab, Vorinostat, Zinostatin, Zoledronsäure, Zorubicin.

Generell können mit der Kombination von Verbindungen der vorliegenden Erfindung mit anderen, anti-angiogen, anti-hyperproliferativ, zytostatisch oder zytotoxisch wirkenden Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur derzeitigen Standardtherapie.

Darüber hinaus können die erfindungsgemäßen Verbindungen auch in Verbindung mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen, enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat oder Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und besonders bevorzugt etwa 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- aq.: wässrig, wässrige Lösung
- br.: breit (bei NMR)
- Bsp.: Beispiel
- Bu: Butyl
- ca.: *circa,* ungefähr
- CI: chemische Ionisation (bei MS)
- d: Dublett (bei NMR)
- d: Tag(e)
- DAST: Diethylaminoschwefeltrifluorid
- DBU: 1,8-Diazabicyclo[5.4.0]undec-7-en
- DC: Dünnschichtchromatographie
- DCI: direkte chemische Ionisation (bei MS)
- dd: Dublett von Dublett (bei NMR)
- DMAP: 4-*N,N*-Dimethylaminopyridin
- DME: 1,2-Dimethoxyethan
- DMF: *N,N*-Dimethylformamid
- DMSO: Dimethylsulfoxid
- dq: Dublett von Quartett (bei NMR)
- dt: Dublett von Triplett (bei NMR)
- d. Th.: der Theorie (bei chemischer Ausbeute)
- EI: Elektronenstoß-Ionisation (bei MS)
- eq.: Äquivalent(e)
- ESI: Elektrospray-Ionisation (bei MS)
- Et: Ethyl
- GC: Gaschromatographie
- GC/MS: Gaschromatographie-gekoppelte Massenspektrometrie
- h: Stunde(n)
- HATU: *O*-(7-Azabenzotriazol-1-yl)-*N,N,N',N'*-tetramethyluronium-hexafluorophosphat
- HOBt: 1-Hydroxy-1*H*-benzotriazol-Hydrat
- HPLC: Hochdruck- / Hochleistungsflüssigchromatographie
- ⁱPr: Isopropyl
- konz.: konzentriert
- LC/MS: Flüssigchromatographie-gekoppelte Massenspektrometrie
- Lit.: Literatur(stelle)
- m: Multiplett (bei NMR)
- Me: Methyl
- min: Minute(n)
- MPLC: Mitteldruckflüssigchromatographie (über Kieselgel; auch "flash-Chromatographie" genannt)
- MS: Massenspektrometrie
- NBS: *N*-Bromsuccinimid
- *n*-Bu: *n*-Butyl
- NMM: *N*-Methylmorpholin
- NMP: *N*-Methyl-2-pyrrolidinon
- NMR: Kernresonanzspektrometrie
- Pd/C: Palladium auf Aktivkohle
- PEG: Polyethylenglykol
- PMB: *para*-Methoxybenzyl
- Pr: Propyl
- *p*-TsOH: *para*-Toluolsulfonsäure
- Q-Phos: 1,2,3,4,5-Pentaphenyl-1'-(di-*tert*.-butylphosphino)ferrocen
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- sept: Septett (bei NMR)
- t: Triplett (bei NMR)
- TBTU: *N*-[(1*H*-Benzotriazol-1-yloxy)(dimethylamino)methylen]-*N*-methyl-methanaminium-Tetrafluoroborat
- ^{t}Bu: *tert.*-Butyl
- TFA: Trifluoressigsäure
- THF: Tetrahydrofuran
- THP: Tetrahydro-2*H*-pyran-2-yl
- UV: Ultraviolett-Spektrometrie
- v/v: Volumen zu Volumen-Verhältnis (einer Lösung)
- Xantphos: 4,5-Bis-(diphenylphosphino)-9,9-dimethylxanthen
- zus.: zusammen

### HPLC-, LC/MS- und GC/MS-Methoden:

### Methode 1 (LC/MS):

Instrument: Micromass Quattro Premier mit Waters UPLC Acquity; Säule: Thermo Hypersil GOLD 1.9 µm, 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Temperatur: 50°C; UV-Detektion: 210 nm.

### Methode 2 (LC/MS):

Instrument: Micromass Quattro Premier mit Waters UPLC Acquity; Säule: Thermo Hypersil GOLD 1.9 µm, 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 97% A → 0.5 min 97% A → 3.2 min 5% A → 4.0 min 5% A; Fluss: 0.3 ml/min; Temperatur: 50°C; UV-Detektion: 210 nm.

### Methode 3 (LC/MS):

Instrument: Waters Acquity SQD UPLC System; Säule: Waters Acquity UPLC HSS T3 1.8 µm, 50 mm x 1 mm; Eluent A: 1 l Wasser + 0.25 ml 99%-ige Ameisensäure, Eluent B: 1 l Acetonitril + 0.25 ml 99%-ige Ameisensäure; Gradient: 0.0 min 90% A → 1.2 min 5% A → 2.0 min 5% A; Fluss: 0.40 ml/min; Temperatur: 50°C; UV-Detektion: 210-400 nm.

### Methode 4 (LC/MS):

Instrument: Waters Acquity SQD UPLC System; Säule: Waters Acquity UPLC HSS T3 1.8 µm, 30 mm x 2 mm; Eluent A: 1 l Wasser + 0.25 ml 99%-ige Ameisensäure, Eluent B: 1 l Acetonitril + 0.25 ml 99%-ige Ameisensäure; Gradient: 0.0 min 90% A → 1.2 min 5% A → 2.0 min 5% A; Fluss: 0.60 ml/min; Temperatur: 50°C; UV-Detektion: 208-400 nm.

### Methode 5 (LC/MS):

Instrument: Waters Acquity UPLC/MS 100-800 Dalton, 20 V (Waters ZQ 4000); Säule: BEH C18 (Waters), 2.1 mm x 50 mm, 1.7 µm; Eluent A: Wasser / 0.05% Ameisensäure, Eluent B: Acetonitril / 0.05% Ameisensäure; Gradient: 10% B → 98% B in 1.7 min, 90% B für 0.2 min, 98% B → 2% B in 0.6 min; Fluss: 1.3 ml/min; UV-Detektion: 200-400 nm.

### Methode 6 (LC/MS):

Instrument: Waters Acquity UPLC/MS 100-800 Dalton, 20 V (Waters SQD); Säule: BEH C18 (Waters), 2.1 mm x 50 mm, 1.7 µm; Eluent A: Wasser / 0.05% Ameisensäure, Eluent B: Acetonitril / 0.05% Ameisensäure; Gradient: 10% B → 98% B in 1.7 min, 90% B für 0.2 min, 98% B → 2% B in 0.6 min; Fluss: 0.8 ml/min; UV-Detektion: 200-400 nm.

### Methode 7 (LC/MS):

Instrument: Waters Acquity UPLC/MS SQD; Säule: Acquity UPLC BEH C18, 1.7 µm, 50 mm x 2.1 mm; Eluent A: Wasser / 0.1% Ameisensäure, Eluent B: Acetonitril; Gradient: 0-1.6 min 1% B → 99% B, 1.6-2.0 min 99% B; Fluss 0.8 ml/min; Temperatur: 60°C; UV-Detektion (DAD): 210-400 nm.

### Methode 8 (GC/MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Einlass: 250°C; Gradient: 70°C, 30°C/min → 310°C (3 min halten).

### Methode 9 (präparative HPLC):

Säule: Reprosil-Pur C18, 10 µm, 250 mm x 30 mm; Eluent: Acetonitril / Wasser mit 0.1% Ameisensäure; Gradient: 10:90 → 90:10.

### Methode 10 (präparative HPLC):

Anlage: SFC Prep 200; Säule: 2-Ethylpyridin 12 µ, 300 mm x 100 mm; Eluent: Kohlendioxid / Methanol; Gradient: 85:15 → 70:30; Gesamtlaufzeit 5 min.

### Methode 11 (präparative HPLC):

Säule: Reprosil-Pur C18, 10 µm, 250 mm x 30 mm; Eluent: Acetonitril / Wasser mit 0.05% Trifluoressigsäure; Gradient: 30:70 → 100:0.

### Methode 12 (präparative HPLC):

Säule: Reprosil-Pur C18, 10 µm, 250 mm x 40 mm; Eluent: Acetonitril / Wasser mit 0.05% Trifluoressigsäure; Gradient: 30:70 → 100:0.

### Methode 13 (präparative HPLC):

Säule: Reprosil-Pur C18, 10 µm, 250 mm x 40 mm; Eluent: Acetonitril / Wasser mit 0.05% Trifluoressigsäure; Gradient: 15:85 → 100:0.

### Methode 14 (präparative HPLC):

Säule: Reprosil-Pur C18, 10 µm, 250 mm x 40 mm; Eluent: Methanol / Wasser mit 0.05% Trifluoressigsäure; Gradient: 30:70 → 100:0.

### Methode 15 (präparative HPLC):

Säule: Reprosil-Pur C18, 10 µm, 250 mm x 30 mm; Eluent: Methanol / Wasser mit 0.05% Trifluoressigsäure; Gradient: 30:70 → 100:0.

### Methode 16 (präparative HPLC):

Säule: XBridge C18, 5 µm, 100 mm x 30 mm; Eluent: Wasser mit 0.1% Ameisensäure / Acetonitril; Gradient: 99:1 → 1:99.

### Methode 17 (präparative HPLC):

Säule: Reprosil-Pur C18, 10 µm, 250 mm x 40 mm; Eluent: Methanol / Wasser mit 0.05% Trifluoressigsäure; Gradient: 15:85 → 100:0.

### Methode 18 (präparative HPLC):

Säule: Reprosil-Pur C18, 10 µm, 250 mm x 30 mm; Eluent: Acetonitril / Wasser mit 0.05% Trifluoressigsäure; Gradient: 20:80 → 100:0.

### Methode 19 (präparative HPLC):

Säule: Reprosil-Pur C18, 10 µm, 250 mm x 30 mm; Eluent: Methanol / Wasser mit 0.05% Trifluoressigsäure; Gradient: 40:60 → 100:0.

### Methode 20 (präparative HPLC):

Säule: Reprosil-Pur C18, 10 µm, 250 mm x 40 mm; Eluent: Acetonitril / Wasser mit 0.05% Trifluoressigsäure; Gradient: 20:80 → 100:0.

### Methode 21 (präparative HPLC):

Säule: Reprosil-Pur C18, 10 µm, 250 mm x 30 mm; Eluent: Methanol / Wasser mit 0.05% Trifluoressigsäure; Gradient: 50:50 → 100:0.

### Methode 22 (präparative HPLC):

Säule: Sunfire C18, 5 µm, 75 mm x 30 mm; Eluent: Acetonitril / Wasser / Trifluoressigsäure 45:50:5 (isokratisch).

### Methode 23 (präparative HPLC):

Säule: Sunfire C18, 5 µm, 75 mm x 30 mm; Eluent: Acetonitril / Wasser; Gradient: 20:80 → 95:5.

### Methode 24 (präparative HPLC):

Säule: XBridge C18, 5 µm, 150 mm x 19 mm; Eluent: Acetonitril / Wasser / 0.2% aq. Diethylamin; Gradient: 45:50:5 → 60:35:5.

### Methode 25 (präparative HPLC):

Säule: GROM-SiL 120 ODS-4HE, 10 µm, 40 mm x 250 mm; Eluent: Methanol / Wasser + 0.05% Trifluoressigsäure; Gradient: 0-8 min 35% Methanol, 8-20 min Rampe auf 55% Methanol, 20-32 min Rampe auf 70% Methanol, dann isokratisch 70% Methanol; Fluss: 50 ml/min.

### Methode 26 (präparative HPLC):

Säule: Interchim Puriflash-SiHC, 120 g-Kartusche; Eluent: Cyclohexan / Ethylacetat; Gradient: 0-8 min isokratisch 80:20, 8-25 min Rampe auf 40:60, 25-55 min isokratisch 40:60; Fluss: 25 ml/ min.

### Methode 27 (präparative HPLC):

Säule: GROM-SiL 120 ODS-4HE, 10 µm, 30 mm x 250 mm; Eluent: Methanol / Wasser + 0.05% Trifluoressigsäure; Gradient: 0-3 min 20% Methanol, 3-20 min Rampe auf 50% Methanol, 20-45 min isokratisch 50% Methanol; Fluss: 25 ml/min.

### Methode 28 (präparative HPLC):

Säule: Kromasil C18 5 µm 100A, 30 mm x 250 mm; Eluent: Acetonitril / Wasser + 0.05% Trifluoressigsäure; Gradient: 0-15 min 50% Acetonitril, 15-32 min Rampe auf 70% Acetonitril, 32-50 min isokratisch 70% Acetonitril; Fluss: 25 ml/min.

### Methode 29 (präparative HPLC):

Säule: Kromasil C18 5 µm 100A, 20 mm x 250 mm; Eluent: Acetonitril / Wasser + 0.05% Trifluoressigsäure; Gradient: 0-5 min 30% Acetonitril, 5-20 min Rampe auf 70% Acetonitril, 20-30 min Rampe auf 80% Acetonitril, 30-45 min isokratisch 80% Acetonitril; Fluss: 12 ml/min.

### Methode 30 (präparative HPLC):

Säule: Kromasil C18 5 µm 100A, 20 mm x 250 mm; Eluent: Acetonitril / Wasser + 0.05% Trifluoressigsäure; Gradient: 0-8 min 10% Acetonitril, 8-15 min Rampe auf 25% Acetonitril, 15-25 min Rampe auf 30% Acetonitril, 25-50 min isokratisch 30% Acetonitril; Fluss: 15 ml/min.

### Methode 31 (LC/MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98% A → 0.9 min 25% A → 1.0 min 5% A → 1.4 min 5% A → 1.41 min 98% A → 1.5 min 98% A; Ofen: 40°C; Fluss: 0.60 ml/min; UV-Detektion (DAD): 210 nm.

### Methode 32 (präparative HPLC):

Säule: Reprosil-Pur C18, 10 µm, 250 mm x 30 mm; Eluent: Acetonitril / Wasser mit 0.1% Ameisensäure; Gradient: 40:60 → 100:0 innerhalb von 20 min.

### Methode 33 (präparative HPLC):

Säule: Reprosil-Pur C18, 10 µm, 250 mm x 30 mm; Eluent: Acetonitril / Wasser mit 0.1% Ameisensäure; Gradient: 30:70 → 95:5 innerhalb von 38 min.

### Methode 34 (präparative HPLC):

Säule: Daicel Chiralpak AZ-H, 5 µm, 250 mm x 4.6 mm; Eluent: Isohexan / Ethanol mit 0.2% Diethylamin 50:50 (isokratisch).

### Methode 35 (präparative HPLC):

Säule: Sunfire C18, 5 µm, 75 mm x 30 mm; Eluent: Acetonitril / Wasser 55:45 (isokratisch).

### Methode 36 (präparative HPLC):

Säule: Chromatorex C18, 10 µm, 250 mm x 30 mm; Eluent: Acetonitril / Wasser mit 0.1% Ameisensäure; Gradient: 30:70 → 95:5 innerhalb von 20 min.

### Methode 37 (präparative HPLC):

Säule: Chromatorex C18, 10 µm, 250 mm x 30 mm; Eluent: Acetonitril / Wasser mit 0.1% Ameisensäure; Gradient: 20:80 → 95:5 innerhalb von 20 min.

### Methode 38 (präparative HPLC):

Säule: Chromatorex C18, 10 µm, 250 mm x 30 mm; Eluent: Acetonitril / Wasser mit 0.1% Ameisensäure; Gradient: 10:90 → 95:5 innerhalb von 20 min.

Die nachfolgenden Beschreibungen der Kopplungsmuster von ¹H-NMR-Signalen orientieren sich an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals; bei breiten Multipletts erfolgt die Angabe eines Intervalls.

Schmelzpunkte und Schmelzbereiche, soweit angegeben, sind nicht korrigiert.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### 1-(2,2-Diethoxyethyl)-1'-(tetrahydro-2H-pyran-2-yl)-1H,1'H-3,4'-bipyrazol-5-amin

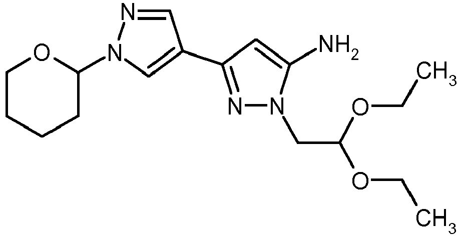

### Schritt 1: 1-(Tetrahydro-2H-pyran-2-yl)-1H-pyrazol-4-carbonitril

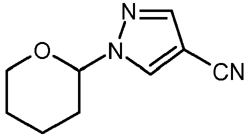

Die Reaktion wurde in einem 3 Liter-Dreihalskolben mit Innenthermometer unter Argon durchgeführt. 50 g (537 mmol) 1*H*-Pyrazol-4-carbonitril wurden in 1.66 Liter Methylenchlorid suspendiert und nach 3 h mit einem Eisbad auf 3°C abgekühlt. Bei dieser Temperatur wurden 10.2 g (53.7 mmol) 4-Toluolsulfonsäure-Monohydrat hinzugegeben. Man tropfte dann 58.8 ml (54.2 g, 644 mmol) 3,4 Dihydro-2*H*-pyran bei 3° bis 6°C innerhalb von 30 min zu. Der Ansatz wurde anschließend über Nacht bei RT gerührt. Dann wurde die klare Reaktionslösung mit 2 M wässriger Natriumcarbonat-Lösung und mit Wasser gewaschen, über Magnesiumsulfat getrocknet, filtriert und eingeengt. Der Rückstand wurde mit Cyclohexan verrührt, und der erhaltene Feststoff wurde abgesaugt und 4 h bei 30°C im Vakuumtrockenschrank getrocknet. Man erhielt 91.8 g (96% d. Th.) der Titelverbindung als hellgelbes Pulver.

¹H-NMR (400 MHz, DMSO-d₆, δ/ppm): 8.76 (s, 1H), 8.11 (s, 1H), 5.50 (dd, *J¹* = 9.66 Hz, *J²* = 2.57 Hz, 1H), 4.02-3.82 (m, 1H), 3.72-3.56 (m, 1H), 2.15-1.82 (m, 3H), 1.76-1.40 (m, 4H).

LC/MS (Methode 3, ESIpos): Rₜ = 0.70 min, m/z = 178 [M+H]⁺.

### Schritt 2: (2E)-3-Amino-3-[1-(tetrahydro-2H-pyran-2-yl)-1H-pyrazol-4-yl]acrylonitril

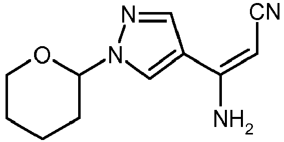

2.8 ml (53.6 mmol) Acetonitril wurden unter Argon in 150 ml THF vorgelegt und bei -70°C mit 21.4 ml (53.6 mmol) einer 2.5 M Lösung von *n*-Buthyllithium in *n*-Hexan versetzt. Nach 15 min bei - 70°C wurde dann eine Lösung von 9.5 g (53.6 mmol) der Verbindung aus Beispiel 1A / Schritt 1 in 20 ml THF portionsweise zugegeben. Der Ansatz wurde 1 h bei -70°C und anschließend 1 h bei RT nachgerührt und dann mit 1 ml Wasser versetzt und mit Ethylacetat verdünnt. Die organische Phase wurde abgetrennt, mit gesättigter Natriumchlorid-Lösung gewaschen, über Natriumsulfat getrocknet, filtriert und eingeengt. Der Rückstand wurde mit *tert.*-Butylmethylether verrührt, und die Kristalle wurden abgesaugt und im Vakuum getrocknet. Man erhielt 6.46 g (54% d. Th.) der Titelverbindung.

¹H-NMR (400 MHz, DMSO-d₆, δ/ppm): 8.36 (s, 1H), 7.89 (s, 1H), 6.64 (s, 2H), 5.39 (dd, 1H), 4.29 (s, 1H), 3.91 (d, 1H), 3.70-3.46 (m, 1H), 2.16-1.3 (m, 6H).

LC/MS (Methode 1, ESIpos): Rₜ = 0.78 min, m/z = 219 [M+H]⁺.

### Schritt 3: 1-(2,2-Diethoxyethyl)-1'-(tetrahydro-2H-pyran-2-yl)-1H,1'H-3,4'-bipyrazol-5-amin

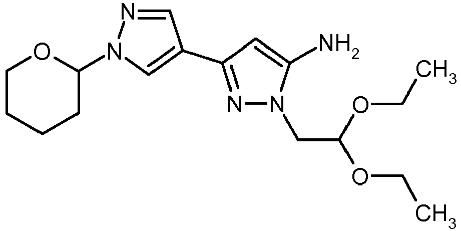

15.2 g (69.6 mmol) der Verbindung aus Beispiel 1A / Schritt 2 und 11.4 g (76.6 mmol) (2,2-Diethoxyethyl)hydrazin wurden in 150 ml Ethanol gelöst. Es wurden 0.7 ml 1 M Salzsäure zugegeben, und die Lösung wurde auf mehrere Mikrowellenreaktorgefäße (20 ml) verteilt. Jeder Teilansatz wurde 1 h lang in einer single-mode-Mikrowelle (Biotage Emrys Optimizer) auf 120°C erhitzt. Nach beendeter Reaktion wurden die Teilansätze vereinigt, das Lösungsmittel wurde im Vakuum abdestilliert und der Rückstand dreimal mit Ethylacetat extrahiert. Die vereinigten organischen Phasen wurden mit verdünnter Natriumhydrogencarbonat-Lösung gewaschen, über Natriumsulfat getrocknet, filtriert und im Vakuum eingeengt. Das Rohprodukt wurde chromatographisch gereinigt (Kieselgel, Laufmittelgradient Cyclohexan/Ethylacetat 1:1 → 100% Ethylacetat, Nachspülen der Säule mit Ethylacetat/Methanol 20:1). Es wurden 17.4 g (67% d. Th.) der Titelverbindung erhalten.

¹H-NMR (400 MHz, DMSO-d₆, δ/ppm): 8.02 (s, 1H), 7.64 (s, 1H), 5.47 (s, 2H), 5.10 (s, 2H), 4.78 (s, 1H), 3.92 (d, 3H), 3.73-3.37 (m, 5H), 1.91 (br. s, 3H), 1.53 (d, 3H), 1.06 (t, 6H).

LC/MS (Methode 3, ESIpos): Rₜ = 0.79 min, m/z = 350 [M+H]⁺.

### Beispiel 2A

### 1-(2,2-Diethoxyethyl)-1'-(4-methoxybenzyl)-1H,1'H-3,4'-bipyrazol-5-amin

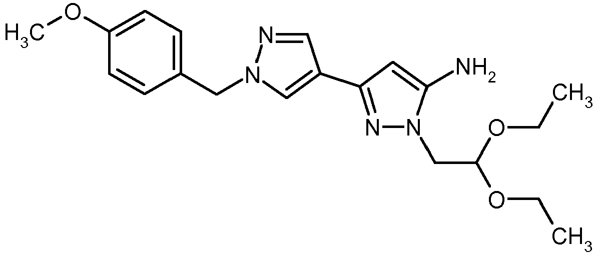

### Schritt 1: 1-(4-Methoxybenzyl)-1H-pyrazol-4-carbonitril

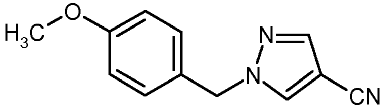

1 g (10.7 mmol) 1*H*-Pyrazol-4-carbonitril und 2.4 g (11.8 mmol) 4-Methoxybenzylbromid wurden bei 0°C in 22 ml THF vorgelegt. 1.3 g (11.8 mmol) Kalium-*tert*.-butylat wurden portionsweise zugesetzt, und der Ansatz wurde über Nacht bei RT gerührt. Danach wurde der ausgefallene Feststoff abfiltriert, und die Mutterlauge wurde am Rotationsverdampfer vom Lösungsmittel befreit. Der Rückstand wurde in Ethylacetat gelöst und die Lösung mit Wasser und gesättigter Natriumchlorid-Lösung gewaschen, über Natriumsulfat getrocknet, filtriert und eingeengt. Man erhielt so 2 g (87% d. Th.) der Titelverbindung, welche ohne weitere Reinigung in der Folgestufe eingesetzt wurde.

GC/MS (Methode 8, EIpos): Rₜ = 6.73 min, m/z = 213 [M]⁺.

### Schritt 2: (2E)-3-Amino-3-[1-(4-methoxybenzyl)-1H-pyrazol-4-yl]acrylonitril

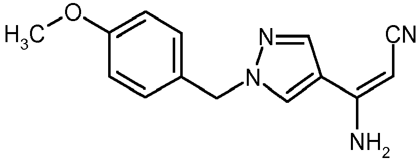

105 ml THF wurden vorgelegt und auf -70°C gekühlt. Dann gab man 25.8 ml (41.3 mmol) einer 1.6 M Lösung von *n*-Butyllithium in *n*-Hexan hinzu. Unter starkem Rühren wurden 2.2 ml (41.3 mmol) Acetonitril innerhalb von 5 min zugetropft, und der Ansatz wurde 10 min nachgerührt. Dann wurde eine Lösung von 8.0 g (37.5 mmol) der Verbindung aus Beispiel 2A / Schritt 1 in 4 ml THF bei -70°C zugetropft. Das Reaktionsgemisch wurde zunächst 30 min bei -70°C und dann 1 h bei RT nachgerührt. Anschließend wurde der Ansatz unter Eisbadkühlung mit 12 ml Wasser versetzt und am Rotationsverdampfer eingeengt. Der Rückstand wurde in 100 ml Ethylacetat aufgenommen, und die Lösung wurde mit je 50 ml Wasser und gesättigter Natriumchlorid-Lösung gewaschen, über Natriumsulfat getrocknet, filtriert und eingeengt. Der ölige Rückstand wurde in 20 ml Methylenchlorid gelöst und mit etwas Cyclohexan versetzt. Durch langsames Eindampfen bei RT unter Normaldruck bildete sich ein Niederschlag, der abfiltriert wurde. Der Feststoff wurde mit *n-*Pentan gewaschen und im Vakuum getrocknet. Es wurden 4.26 g (44.6% d. Th.) der Titelverbindung erhalten.

¹H-NMR (400 MHz, DMSO-d₆, δ/ppm): 8.20 (s, 1H), 7.85 (s, 1H), 7.21 (d, 2H), 6.91 (d, 2H), 6.61 (s, 2H), 5.23 (s, 2H), 4.20 (s, 1H), 3.73 (s, 3H).

LC/MS (Methode 3, ESIpos): Rₜ = 0.81 min, m/z = 255 [M+H]⁺.

### Schritt 3: 1-(2,2-Diethoxyethyl)-1'-(4-methoxybenzyl)-1H,1'H-3,4'-bipyrazol-5-amin

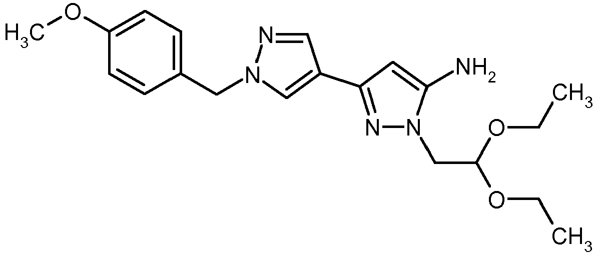

1.27 g (4.99 mmol) der Verbindung aus Beispiel 2A / Schritt 2 und 0.77 g (5.2 mmol) (2,2-Diethoxyethyl)hydrazin wurden in 10.6 ml Ethanol vorgelegt und mit 50 µl 1 M Salzsäure versetzt. Der Ansatz wurde 45 min bei 120°C in einem Mikrowellenofen (Biotage Initiator, mit dynamischer Steuerung der Einstrahlleistung) gerührt. Dann wurde das Lösungsmittel im Vakuum entfernt. Das so erhaltene Rohprodukt (1.87 g, 97% d. Th.) wurde ohne weitere Aufreinigung in Folgereaktionen eingesetzt.

LC/MS (Methode 4, ESIpos): Rₜ = 0.76 min, m/z = 372 [M+H]⁺.

### Beispiel 3A

### 1-(1,1-Dimethoxypropan-2-yl)-1'-(4-methoxybenzyl)-1H,1'H-3,4'-bipyrazol-5-amin

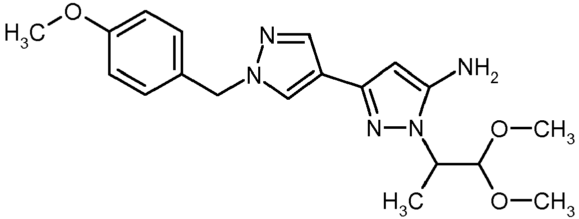

### Schritte 1: (1,1-Dimethoxypropan-2-yl)hydrazin

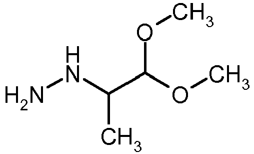

6.25 g (34.1 mmol) 2-Brom-1,1-dimethoxypropan wurden mit 6.65 ml (137 mmol) Hydrazinhydrat im Ölbad bei 140°C 6 h unter Rückfluss gerührt. Nach dem Abkühlen wurde das zweiphasige Reaktionsgemisch mit 50 ml *tert*.-Butylmethylether extrahiert. Die organische Phase wurde filtriert, das Filtrat am Rotationsverdampfer eingeengt und der Rückstand im Hochvakuum getrocknet. Man erhielt 1.89 g (21% d. Th.) eines fahlgelben Öls, welches direkt, ohne weitere Reinigung, in der Folgestufe umgesetzt wurde.

### Schritt 2: 1-(1,1-Dimethoxypropan-2-yl)-1'-(4-methoxybenzyl)-1H,1'H-3,4'-bipyrazol-5-amin

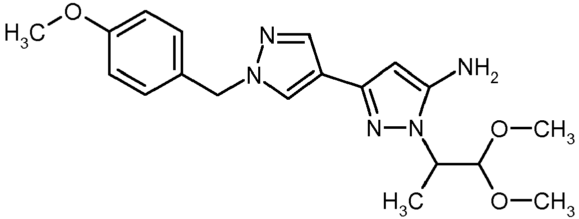

1.35 g (5.31 mmol) der Verbindung aus Beispiel 2A / Schritt 2 wurden zusammen mit 1.78 g der Verbindung aus Beispiel 3A / Schritt 1 in 32 ml Methanol gelöst und mit 53 µl (53 µmol) 1 M Salzsäure versetzt. Die Lösung wurde auf zwei Ansätze aufgeteilt und in einem Mikrowellenofen (Biotage Initiator, mit dynamischer Steuerung der Einstrahlleistung) 15 min lang bei 120°C gerührt. Die beiden Ansätze wurden danach wieder vereinigt und am Rotationsverdampfer eingeengt. Der Rückstand wurde durch präparative HPLC (Methode 10) gereinigt. Man erhielt 990 mg (50% d. Th.) der Titelverbindung.

¹H-NMR (400 MHz, DMSO-d₆, δ/ppm): 7.90 (s, 1H), 7.58 (s, 1H), 7.22 (d, 2H), 6.90 (d, 2H), 5.39 (s, 1H), 5.21 (s, 1H), 5.10 (s, 2H), 4.53 (d, 1H), 4.24 (quin, 1H), 3.73 (s, 3H), 3.10 (s, 3H), 1.29 (d, 3H).

LC/MS (Methode 3, ESIpos): Rₜ = 0.79 min, m/z = 372 [M+H]⁺.

### Beispiel 4A

### 1-(2,2-Diethoxyethyl)-3-(pyridin-3-yl)-1H-pyrazol-5-amin

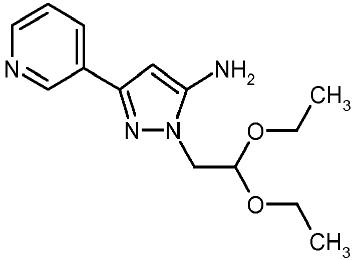

125 g (812 mmol, Reinheit 95%) 3-Oxo-3-(pyridin-3-yl)propanonitril [Lit. z.B.: P. Seneci et al., Synth. Commun. 1999, 29 (2), 311-341; auch kommerziell erhältlich] wurden in 1.25 Liter Ethanol unter leichtem Erwärmen gelöst. Anschließend wurden 126 g (853 mmol) (2,2-Diethoxyethyl)-hydrazin und 4.1 ml (4.06 mmol) 1 M Salzsäure hinzugefügt. Nachdem das Reaktionsgemisch 4 h unter Rückfluss erhitzt worden war, wurden alle flüchtigen Bestandteile am Rotationsverdampfer weitgehend entfernt. Der erhaltene Rückstand wurde in Ethylacetat aufgenommen, und die Lösung wurde mit Wasser gewaschen und über wasserfreiem Magnesiumsulfat getrocknet. Nach Filtration wurde zur Trockene eingedampft. Der verbliebene Rückstand wurde mit Diisopropylether bei RT verrührt. Anschließend wurde der resultierende Feststoff abgesaugt und im Hochvakuum getrocknet. Es wurden 153 g (65% d. Th., Reinheit 96%) der Titelverbindung erhalten.

¹H-NMR (400 MHz, DMSO-d₆, δ/ppm): 8.88 (d, 1H), 8.44 (dd, 1H), 8.02 (dt, 1H), 7.37 (dd, 1H), 5.79 (s, 1H), 5.29 (s, 2H), 4.85 (t, 1H), 4.02 (d, 2H), 3.70-3.62 (m, 2H), 3.48-3.40 (m, 2H), 1.07 (t, 6H).

LC/MS (Methode 3, ESIpos): Rₜ = 0.52 min, m/z = 277 [M+H]⁺.

### Beispiel 5A

### 6-[1-(4-Methoxybenzyl)-1H-pyrazol-4-yl]-1H-imidazo[1,2-b]pyrazol

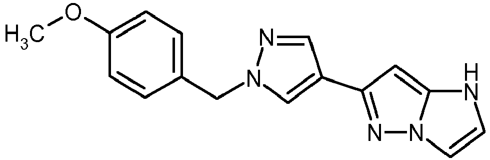

155 mg (0.40 mmol) der Verbindung aus Beispiel 2A wurden in 0.4 ml Ethanol und 0.2 ml 2 M Schwefelsäure in einem Mikrowellenofen (Biotage Initiator, mit dynamischer Steuerung der Einstrahlleistung) 15 min lang auf 120°C erhitzt. Der Ansatz wurde anschließend direkt mittels präparativer HPLC (Methode 11) gereinigt. Die Produktfraktionen wurden eingeengt und der Rückstand im Hochvakuum getrocknet. Es wurden 54 mg (46% d. Th.) der Titelverbindung erhalten.

¹H-NMR (400 MHz, DMSO-d₆, δ/ppm): 11.44 (br, 1H), 8.08 (s, 1H), 7.79 (s, 1H), 7.58 (s, 1H), 7.25 (d, 2H), 7.22 (s, 1H), 6.91 (d, 2H), 6.03 (s, 1H), 5.27 (s, 2H), 3.73 (s, 3H).

LC/MS (Methode 3, ESIpos): Rₜ = 0.72 min, m/z = 294 [M+H]⁺.

### Beispiel 6A

### 4-Methyl-3-[6-(pyridin-3-yl)-1H-imidazo[1,2-b]pyrazol-1-yl]anilin

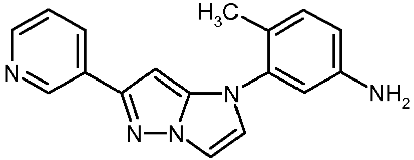

### Schritt 1: 1-(2,2-Diethoxyethyl)-N-(2-methyl-5-nitrophenyl)-3-(pyridin-3-yl)-1H-pyrazol-5-amin

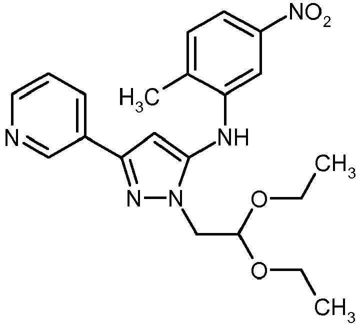

155 g (561 mmol) der Verbindung aus Beispiel 4A wurden zusammen mit 133 g (617 mmol) 2-Brom-4-nitrotoluol, 12.6 g (56.1 mmol) Palladium(II)acetat, 48.7 g (84.1 mmol) Xantphos [4,5-Bis-(diphenylphosphino)-9,9-dimethylxanthen] und 548 g (1683 mmol) Cäsiumcarbonat in 3.1 Liter 1,4-Dioxan unter Argon zum Rückfluss erhitzt. Nach 4 h wurde auf RT abgekühlt, über Kieselgur filtriert und das Filtrat am Rotationsverdampfer eingedampft. Das so erhaltene Rohprodukt wurde mittels Saugfiltration über Kieselgel gereinigt (Laufmittelgradient Ethylacetat/Petrolether 4:1 → 100% Ethylacetat). Die Produktfraktionen wurden vereinigt und am Rotationsverdampfer vom Lösungsmittel befreit. Nach Verrühren mit Diisopropylether bei RT, Absaugen des Feststoffs und Trocknen im Hochvakuum wurden 195 g (84% d. Th.) der Titelverbindung erhalten.

¹H-NMR (400 MHz, DMSO-d₆, δ/ppm): 9.04 (d, 1H), 8.52 (dd, 1H), 8.18 (dt, 1H), 7.64-7.61 (m, 2H), 7.50 (d, 1H), 7.46-7.42 (m, 2H), 6.80 (s, 1H), 4.90 (t, 1H), 4.18 (d, 2H), 3.64-3.56 (m, 2H), 3.47-3.40 (m, 2H), 2.38 (s, 3H), 1.00 (t, 6H).

LC/MS (Methode 3, ESIpos): Rₜ = 1.03 min, m/z = 412 [M+H]⁺, 823 [2M+H]⁺.

### Schritt 2: 1-(2-Methyl-5-nitrophenyl)-6-(pyridin-3-yl)-1H-imidazo[1,2-b]pyrazol

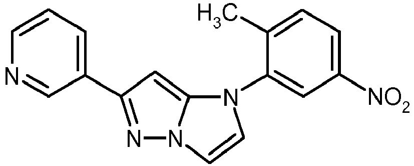

Eine Lösung von 170 g (413 mmol) der Verbindung aus Beispiel 6A / Schritt 1 in 1.7 Liter Ethanol wurde mit 496 ml (992 mmol) 2 M Schwefelsäure versetzt und 4 h unter Rückfluss erhitzt. Dabei fiel ein weißer Feststoff aus. Nach dem Abkühlen auf RT wurde der Feststoff abgesaugt und mit wenig Ethanol nachgewaschen. Dann wurde der Feststoff zwischen Wasser und Ethylacetat verteilt und das heterogene Gemisch durch Zusatz von verdünnter Natronlauge neutral bis schwach alkalisch gestellt. Die organische Phase wurde abgetrennt und am Rotationsverdampfer vom Lösungsmittel befreit. Der erhaltene Rückstand wurde mit wenig Acetonitril bei RT verrührt, und der Feststoff wurde abgesaugt und im Hochvakuum getrocknet. Es wurden 127 g (93% d. Th., 97% Reinheit) der Titelverbindung erhalten.

¹H-NMR (400 MHz, DMSO-d₆, δ/ppm): 9.06 (d, 1H), 8.49 (dd, 1H), 8.31 (d, 1H), 8.26 (dd, 1H), 8.19 (dt, 1H), 7.98 (d, 1H), 7.79 (d, 1H), 7.66 (d, 1H), 7.43 (dd, 1H), 6.46 (s, 1H), 2.43 (s, 3H).

LC/MS (Methode 3, ESIpos): Rₜ = 0.97 min, m/z = 320 [M+H]⁺.

### Schritt 3: 4-Methyl-3-[6-(pyridin-3-yl)-1H-imidazo[1,2-b]pyrazol-1-yl]anilin

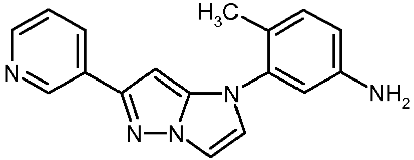

In zwei Teilansätzen wurden jeweils 63.5 g (398 mmol) der Verbindung aus Beispiel 6A / Schritt 2 in 1.25 Liter Ethanol gelöst und mit 6.35 g Palladium (10% auf Aktivkohle) versetzt. Es wurde 48 h bei RT unter einer Wasserstoffatmosphäre bei Normaldruck hydriert. Anschließend wurde über wenig Kieselgur filtriert, um den Katalysator abzutrennen. Das Filtrat wurde am Rotationsverdampfer bis zur Trockene eingedampft. Die Rohprodukte aus den beiden Ansätzen wurden vereinigt und bei RT mit Diisopropylether, dem wenig Ethylacetat beigefügt war, verrührt. Nach Absaugen des Feststoffs und Trocknen im Hochvakuum wurden 108 g (94% d. Th.) der Titelverbindung erhalten.

¹H-NMR (400 MHz, DMSO-d₆, δ/ppm): 9.05 (d, 1H), 8.47 (dd, 1H), 8.18 (dt, 1H), 7.83 (d, 1H), 7.43 (d, 1H), 7.41 (dd, 1H), 7.06 (d, 1H), 6.63 (d, 1H), 6.59 (dd, 1H), 6.29 (s, 1H), 2.08 (s, 3H).

LC/MS (Methode 3, ESIpos): Rₜ = 0.59 min, m/z = 290 [M+H]⁺.

### Beispiel 7A

### 4-Methyl-3-[6-(1H-pyrazol-4-yl)-1H-imidazo[1,2-b]pyrazol-1-yl]anilin

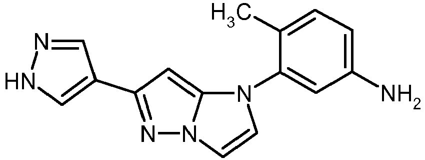

### Schritt 1: 1-(2,2-Diethoxyethyl)-N(2-methyl-5-nitrophenyl)-1'-(tetrahydro-2H-pyran-2-yl)-1H,1'H 3,4'-bipyrazol-5-amin

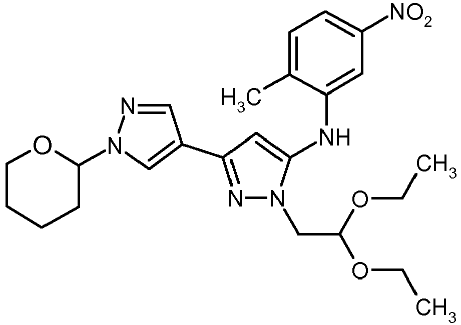

12.3 g (35.2 mmol) der Verbindung aus Beispiel 1A, 9.9 g (45.8 mmol) 2-Brom-4-nitrotoluol, 0.79 g (3.52 mmol) Palladium(II)acetat, 22.9 g (70.4 mmol) Cäsiumcarbonat und 2.04 g (3.52 mmol) Xantphos [4,5-Bis-(diphenylphosphino)-9,9-dimethylxanthen] wurden in 170 ml 1,4-Dioxan unter Argon 4 h zum Rückfluss erhitzt. Nach dem Abkühlen auf RT wurde über Kieselgur filtriert und das Filtrat am Rotationsverdampfer eingedampft. Der Rückstand wurde in Ethylacetat aufgenommen und das Gemisch mit halbkonzentrierter Natriumhydrogencarbonat-Lösung und anschließend mit konzentrierter Natriumchlorid-Lösung gewaschen. Die organische Phase wurde über wasserfreiem Natriumsulfat getrocknet und eingeengt. Das Rohprodukt wurde an Kieselgel chromatographisch gereinigt (Laufmittelgradient Dichlormethan/Ethylacetat 2:1 → 1:3). Man erhielt 15.9 g (92% d. Th.) der Titelverbindung.

¹H-NMR (400 MHz, DMSO-d₆, δ/ppm): 8.20 (s, 1H), 7.80 (s, 1H), 7.60 (dd, 1H), 7.52 (s, 1H), 7.48 (d, 1H), 7.41 (d, 1H), 6.40 (d, 1H), 5.41 (dd, 1H), 4.83 (t, 1H), 4.09 (d, 2H), 3.93 (m, 1H), 3.67-3.54 (m, 3H), 3.41 (m, 2H), 2.36 (s, 3H), 2.11 (m, 1H), 1.94 (m, 2H), 1.68 (m, 1H), 1.55 (m, 2H), 0.99 (t, 6H).

LC/MS (Methode 4, ESIpos): Rₜ = 1.20 min, m/z = 485 [M+H]⁺.

### Schritt 2: 1-(2-Methyl-5-nitrophenyl)-6-(1H-pyrazol-4-yl)-1H-imidazo[1,2-b]pyrazol

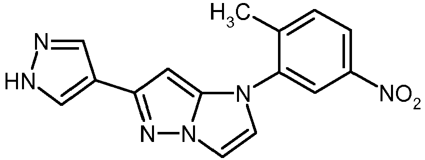

Die Verbindung aus Beispiel 7A / Schritt 1 (5.5 g, 11.3 mmol) wurde in 80 ml Ethanol gelöst und mit 17 ml 2 M Schwefelsäure versetzt. Der Ansatz wurde dann auf fünf 20 ml-Mikrowellenreaktorgefäße verteilt, und jeder Teilansatz wurde 15 min lang in einer single-mode-Mikrowelle (Biotage Emrys Optimizer) auf 120°C erhitzt. Die Teilansätze wurden danach wieder vereinigt, auf verdünnte wässrige Natriumhydrogencarbonat-Lösung gegossen und zweimal mit Methylenchlorid extrahiert. Die vereinigten organischen Phasen wurden über Natriumsulfat getrocknet, filtriert und eingedampft. Der Rückstand wurde in vier Portionen über präparative HPLC gereinigt (Methode 25). Es wurden 2.1 g (86% Reinheit, 52% d. Th.) der Titelverbindung erhalten.

¹H-NMR (400 MHz, DMSO-d₆, δ/ppm): 8.26-8.22 (m, 2H), 7.90 (s, 2H), 7.83 (d, 1H), 7.78 (d, 1H), 7.51 (d, 1H), 6.00 (s, 1H), 2.43 (s, 3H).

LC/MS (Methode 3, ESIpos): Rₜ = 0.75 min, m/z = 309 [M+H]⁺.

### Schritt 3: 4-Methyl-3-[6-(1H-pyrazol-4-yl)-1H-imidazo[1,2-b]pyrazol-1-yl]anilin

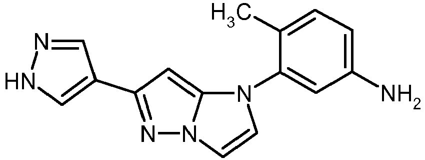

Die Verbindung aus Beispiel 7A / Schritt 2 (906 mg, 2.94 mmol) wurde in 45 ml Ethanol gelöst und unter Argon mit 10% Pd/C (272 mg) und Cyclohexen (3.30 ml, 32.3 mmol) versetzt. Der Ansatz wurde 16 h unter Rückfluss erhitzt, dann über Kieselgur abgesaugt und eingeengt. Die Titelverbindung wurde in einer Ausbeute von 810 mg (99% d. Th.) erhalten.

¹H-NMR (400 MHz, DMSO-d₆, δ/ppm): 7.80 (s, 2H), 7.71 (d, 1H), 7.32 (d, 1H), 7.12 (d, 1H), 6.74 (s, 1H), 6.69 (d, 1H), 5.86 (s, 1H), 2.11 (s, 3H).

LC/MS (Methode 4, ESIpos): Rₜ = 0.58 min, m/z = 278 [M+H]⁺.

### Beispiel 8A

### 3-{6-[1-(4-Methoxybenzyl)-1H-pyrazol-4-yl]-1H-imidazo[1,2-b]pyrazol-1-yl}-4-methylanilin-Trifluoracetat

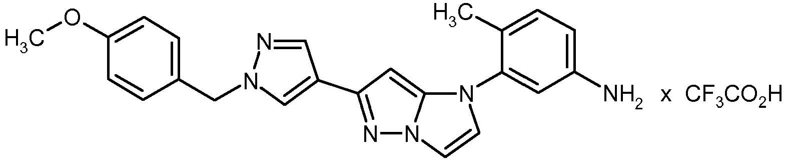

### Schritt 1: 1-(2,2-Diethoxyethyl)-1'-(4-methoxybenzyl)-N-(2-methyl-5-nitrophenyl)-1H,1'H-3,4'-bipyrazol-5-amin

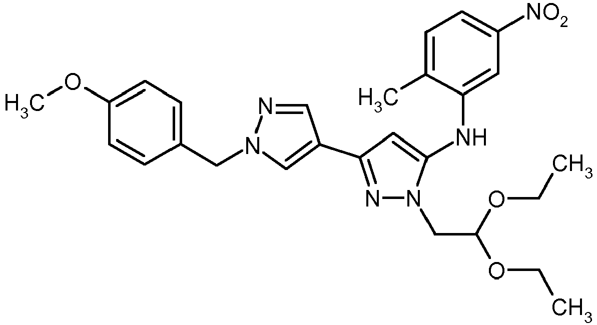

Ein Gemisch aus der Verbindung aus Beispiel 2A (2.9 g, 7.5 mmol), 2-Brom-4-nitrotoluol (2.11 g, 9.78 mmol), Palladium(II)acetat (0.17 g, 0.75 mmol), Cäsiumcarbonat (4.90 g, 15.1 mmol) und Xantphos (4,5-Bis-(diphenylphosphino)-9,9-dimethylxanthen; 0.44 g, 0.75 mmol) in 37 ml 1,4-Dioxan wurde unter Argon auf mehrere 20 ml-Mikrowellenreaktorgefäße verteilt. Jeder Teilansatz wurde in einer single-mode-Mikrowelle (Biotage Emrys Optimizer) 30 min lang auf 150°C erhitzt. Die Teilansätze wurden danach vereinigt, über Kieselgur filtriert und das Filtrat am Rotationsverdampfer eingeengt. Der Rückstand wurde in Ethylacetat aufgenommen und mit halbkonzentrierter Natriumhydrogencarbonat-Lösung und anschließend mit konzentrierter Natriumchlorid-Lösung gewaschen. Die organische Phase wurde über wasserfreiem Natriumsulfat getrocknet und eingeengt. Das Rohprodukt wurde chromatographisch gereinigt (Methode 26). Man erhielt 2.74 g (70% d. Th.) der Titelverbindung.

¹H-NMR (400 MHz, DMSO-d₆, δ/ppm): 8.08 (s, 1H), 7.75 (s, 1H), 7.60 (dd, 1H), 7.49 (s, 1H), 7.46 (d, 1H), 7.40 (d, 1H), 7.26 (d, 2H), 6.34 (s, 1H), 5.25 (s, 2H), 4.80 (t, 1H), 4.07 (d, 2H), 3.73 (s, 3H), 3.60-3.52 (m, 2H), 3.44-3.36 (m, 2H), 2.35 (s, 3H), 0.98 (t, 3H).

LC/MS (Methode 4, ESIpos): Rₜ = 1.25 min, m/z = 521 [M+H]⁺.

### Schritt 2: 6-[1-(4-Methoxybenzyl)-1H-pyrazol-4-yl]-1-(2-methyl-5-nitrophenyl)-1H-imidazo-[1,2-b]pyrazol

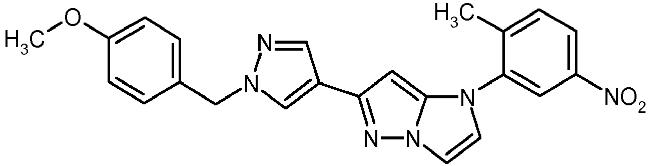

1.70 g (3.27 mmol) der Verbindung aus Beispiel 8A / Schritt 1 wurden in 12 ml Ethanol gelöst. Es wurden 1.63 ml 2 M Schwefelsäure hinzugegeben, und die Mischung wurde in einem single-mode-Mikrowellengerät (Biotage Emrys Optimizer) 35 min lang auf 120°C erhitzt. Nach Reaktionsende wurde der Ansatz auf verdünnte wässrige Natriumhydrogencarbonat-Lösung gegossen und zweimal mit Dichlormethan extrahiert. Die vereinigten organischen Phasen wurden über Natriumsulfat getrocknet, filtriert und im Vakuum eingeengt. Die Titelverbindung wurde in einer Ausbeute von 1.32 g (80% d. Th., 85% Reinheit) erhalten.

¹H-NMR (400 MHz, DMSO-d₆, δ/ppm): 8.24-8.21 (m, 2H), 8.04 (s, 1H), 7.82 (s, 1H), 7.75 (s, 2H), 7.53 (s, 1H), 7.22 (d, 2H), 6.90 (d, 2H), 5.98 (s, 1H), 5.23 (s, 2H), 3.71 (s, 1H), 2.42 (s, 3H).

LC/MS (Methode 3, ESIpos): Rₜ = 1.00 min, m/z = 429 [M+H]⁺.

### Schritt 3: 3-{6-[1-(4-Methoxybenzyl)-1H-pyrazol-4-yl]-1H-imidazo[1,2-b]pyrazol-1-yl}-4-methylanilin-Trifluoracetat

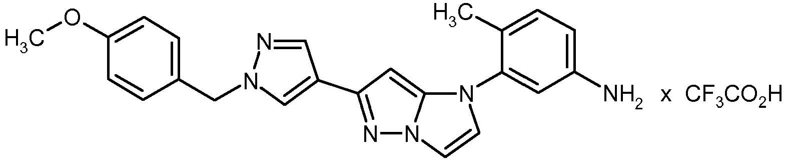

789 mg (1.25 mmol; Reinheit 67%) der Verbindung aus Beispiel 8A / Schritt 2 wurden zusammen mit 393 mg (6.23 mmol) Ammoniumformiat und 16.0 mg (0.02 mmol) 10% Palladium auf Aktivkohle in 11 ml Ethanol und 0.55 ml Wasser vorgelegt. Der Ansatz wurde in einem single-mode-Mikrowellengerät (Biotage Emrys Optimizer) 1 h lang bei 90°C gerührt. Nach dem Erkalten wurde von unlöslichen Bestandteilen abfiltriert, das Filtrat im Vakuum eingeengt und der Rückstand über präparative HPLC gereinigt (Methode 27). Es wurden 510 mg (80% d. Th.) der Titelverbindung erhalten.

¹H-NMR (400 MHz, DMSO-d₆, δ/ppm): 8.04 (s, 1H), 7.74 (s, 1H), 7.73 (d, 1H), 7.35 (d, 1H), 7.25-7.20 (m, 3H), 6.92-6.81 (m, 4H), 5.85 (s, 1H), 5.25 (s, 2H), 3.73 (s, 3H), 2.14 (s, 3H).

LC/MS (Methode 3, ESIpos): Rₜ = 0.86 min, m/z = 399 [M+H]⁺.

### Beispiel 9A

### 3-{6-[1-(4-Methoxybenzyl)-1H-pyrazol-4-yl]-3-methyl-1H-imidazo[1,2-b]pyrazol-1-yl}-4-methylanilin

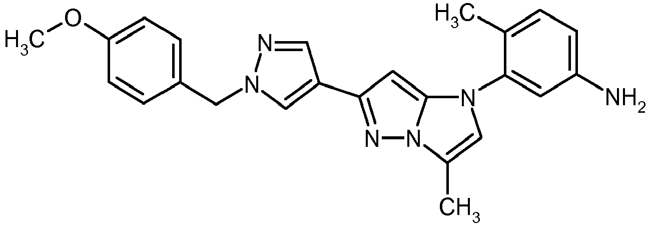

### Schritte 1: 1-(1,1-Dimethoxypropan-2-yl)-1'-(4-methoxybenzyl)-N-(2-methyl-5-nitrophenyl)-1H,1'H-3,4'-bipyrazol-5-amin

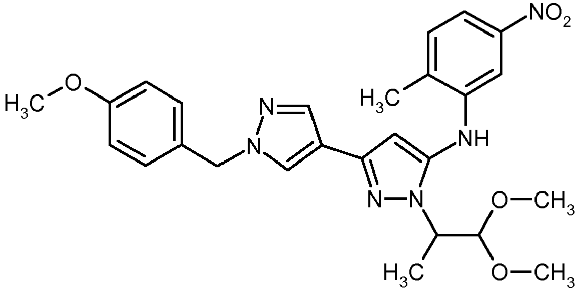

980 mg (2.64 mmol) der Verbindung aus Beispiel 3A und 627 mg (2.90 mmol) 2-Brom-4-nitrotoluol wurden in 13 ml Dioxan gelöst, mit Argon entgast und mit 59 mg (0.26 mmol) Palladium(II)acetat, 229 mg (0.40 mmol) Xantphos [4,5-Bis-(diphenylphosphino)-9,9-dimethylxanthen] sowie 2.57 g (7.91 mmol) Cäsiumcarbonat versetzt. Das Gemisch wurde in einem Mikrowellenofen (Biotage Initiator, mit dynamischer Steuerung der Einstrahlleistung) für 30 min auf 150°C erhitzt. Der Ansatz wurde danach über Kieselgur filtriert, das Filtrat im Vakuum eingeengt und der Rückstand über Kieselgel flash-chromatographiert (Laufmittel Dichlormethan/Ethylacetat 2:1). Man erhielt 980 mg (73% d. Th.) der Titelverbindung.

¹H-NMR (400 MHz, DMSO-d₆, δ/ppm): 8.07 (s, 1H), 7.75 (s, 1H), 7.57-7.56 (m, 2H), 7.37 (d, 1H), 7.34 (d, 1H), 7.26 (d, 2H), 6.91 (d, 2H), 6.27 (s, 1H), 5.25 (s, 2H), 4.53 (d, 1H), 4.30 (m, 1H), 3.73 (s, 3H), 3.30 (s, 3H), 3.07 (s, 3H), 2.36 (s, 3H), 1.39 (d, 3H).

LC/MS (Methode 4, ESIpos): Rₜ = 1.19 min, m/z = 507 [M+H]⁺.

### Schritt 2: 6-[1-(4-Methoxybenzyl)-1H-pyrazol-4-yl]-3-methyl-1-(2-methyl-5-nitrophenyl)-1H-imidazo[1,2-b]pyrazol

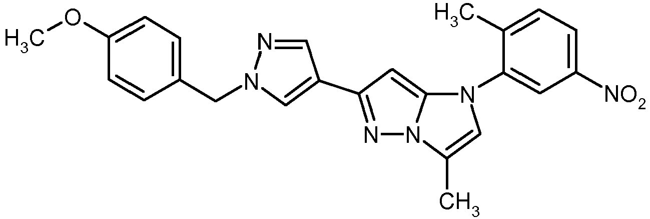

980 mg (385 mmol) der Verbindung aus Beispiel 9A / Schritt 1 wurden in 9.8 ml Methanol unter Zusatz von 1.16 ml (2.32 mmol) 2 M Schwefelsäure in einem Mikrowellenofen (Biotage Initiator, mit dynamischer Steuerung der Einstrahlleistung) für 60 min auf 120°C erhitzt. Der Ansatz wurde danach im Vakuum eingeengt und der Rückstand in Ethylacetat aufgenommen. Die organische Phase wurde mit gesättigter Natriumhydrogencarbonat-Lösung und gesättigter Natriumchlorid-Lösung gewaschen, über Natriumsulfat getrocknet, filtriert und am Rotationsverdampfer eingeengt. Der Rückstand wurde im Hochvakuum getrocknet. Es wurden 792 mg (93% d. Th.) der Titelverbindung erhalten.

¹H-NMR (400 MHz, DMSO-d₆, δ/ppm): 8.21-8.18 (m, 2H), 8.09 (s, 1H), 7.76 (s, 1H), 7.74 (d, 1H), 7.28 (d, 1H), 7.25 (d, 2H), 6.91 (d, 2H), 5.99 (s, 1H), 5.24 (s, 2H), 3.73 (s, 3H), 2.42 (s, 3H), 2.40 (s, 3H).

LC/MS (Methode 3, ESIpos): Rₜ = 1.05 min, m/z = 443 [M+H]⁺.

### Schritt 3: 3-{6-[1-(4-Methoxybenzyl)-1H-pyrazol-4-yl]-3-methyl-1H-imidazo[1,2-b]pyrazol-1-yl}-4-methylanilin

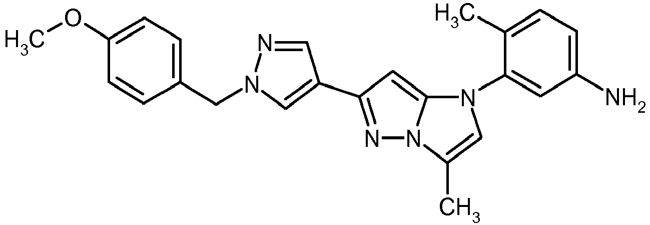

790 mg (1.78 mmol) der Verbindung aus Beispiel 9A / Schritt 2, 563 mg (8.93 mmol) Ammoniumformiat und 23 mg (0.02 mmol) Palladium (10% auf Kohle) wurden in 9.6 ml Ethanol und 0.5 ml Wasser 1.5 h lang unter Rückfluss erhitzt. Anschließend wurde der Ansatz im Vakuum eingeengt, der Rückstand in Dichlormethan suspendiert, mit Natriumsulfat versetzt und filtriert. Das Filtrat wurde im Vakuum eingeengt und der Rückstand im Hochvakuum getrocknet. Man erhielt 740 mg (96% d. Th.) der Titelverbindung.

¹H-NMR (400 MHz, DMSO-d₆, δ/ppm): 8.07 (s, 1H), 7.75 (s, 1H), 7.25 (d, 2H), 7.01 (m, 2H), 6.91 (d, 2H), 6.57 (d, 1H), 6.53 (dd, 1H), 5.82 (s, 1H), 5.23 (s, 2H), 5.17 (br, 2H), 3.73 (s, 3H), 2.37 (s, 3H), 2.07 (s, 3H).

LC/MS (Methode 3, ESIpos): Rₜ = 0.95 min, m/z = 413 [M+H]⁺.

### Beispiel 10A

### 4-Methoxy-3-[6-(pyridin-3-yl)-1H-imidazo[1,2-b]pyrazol-1-yl]anilin

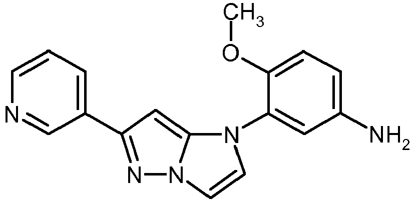

### Schritt 1: 1-(2,2-Diethoxyethyl)-N-(2-methoxy-5-nitrophenyl)-3-(pyridin-3-yl)-1H-pyrazol-5-amin

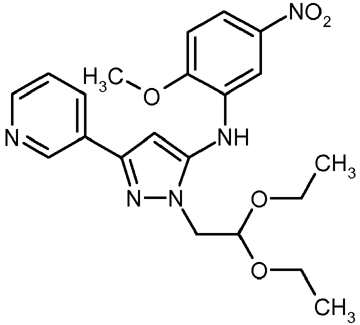

200 mg (0.72 mmol) der Verbindung aus Beispiel 4A wurden zusammen mit 201 mg (0.87 mmol) 2-Brom-4-nitroanisol in 3.6 ml Dioxan gelöst, mit Argon entgast und mit 16 mg (0.07 mmol) Palladium(II)acetat, 63 mg (0.11 mmol) Xantphos [4,5-Bis-(diphenylphosphino)-9,9-dimethylxanthen] sowie 589 mg (1.81 mmol) Cäsiumcarbonat versetzt. Das Gemisch wurde in einem Mikrowellenofen (Biotage Initiator, mit dynamischer Steuerung der Einstrahlleistung) für 30 min auf 150°C erhitzt. Der Ansatz wurde anschließend über Kieselgur filtriert, das Filtrat im Vakuum eingeengt und der Rückstand mittels präparativer HPLC (Methode 12) in seine Komponenten aufgetrennt. Die produkthaltigen Fraktionen wurden vereinigt und im Vakuum fast vollständig eingeengt. Der Rückstand wurde mit etwas gesättigter wässriger Natriumhydrogencarbonat-Lösung alkalisch gestellt. Der entstandene Niederschlag wurde abfiltriert, mit Wasser gewaschen und getrocknet. Man erhielt 210 mg (92% Reinheit, 62% d. Th.) der Titelverbindung.

LC/MS (Methode 2, ESIpos): Rₜ = 2.15 min, m/z = 428 [M+H]⁺.

### Schritt 2: 1-(2-Methoxy-5-nitrophenyl)-6-(pyridin-3-yl)-1H-imidazo[1,2-b]pyrazol

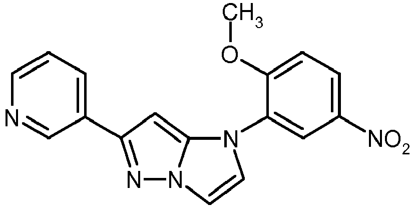

Eine Lösung von 210 mg (0.49 mmol) der Verbindung aus Beispiel 10A / Schritt 1 in 4.8 ml Ethanol und 0.49 ml (0.98 mmol) 2 M Schwefelsäure wurde in einem Mikrowellenofen (Biotage Initiator, mit dynamischer Steuerung der Einstrahlleistung) für 15 min auf 120°C erhitzt. Anschließend wurde der Ansatz direkt mittels präparativer HPLC (Methode 12) aufgereinigt. Die produkthaltigen Fraktionen wurden vereinigt und im Vakuum fast vollständig eingeengt. Der Rückstand wurde mit etwas gesättigter wässriger Natriumhydrogencarbonat-Lösung alkalisch gestellt, und der entstandene Niederschlag wurde ab filtriert, mit Wasser gewaschen und getrocknet. Man erhielt 112 mg (89% Reinheit, 61% d. Th.) der Titelverbindung.

LC/MS (Methode 4, ESIpos): Rₜ = 0.73 min, m/z = 336 [M+H]⁺.

### Schritt 3: 4-Methoxyl-3-[6-(pyridin-3-yl)-1H-imidazo[1,2-b]pyrazol-1-yl]anilin

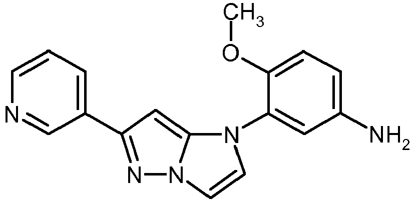

110 mg (89% Reinheit, 0.29 mmol) der Verbindung aus Beispiel 10A / Schritt 2, 92 mg (1.46 mmol) Ammoniumformiat und 31 mg (0.1 mmol) Palladium (10% auf Kohle) wurden in 9.7 ml Ethanol und 0.97 ml Wasser 15 min unter Rückfluss erhitzt. Anschließend wurde der Ansatz im Vakuum eingeengt, der Rückstand in Dichlormethan suspendiert, mit Natriumsulfat versetzt und filtriert. Das Filtrat wurde im Vakuum eingeengt und der Rückstand im Hochvakuum getrocknet. Man erhielt 46 mg (50% d. Th.) der Titelverbindung.

LC/MS (Methode 3, ESIpos): Rₜ = 0.52 min, m/z = 306 [M+H]⁺.

### Beispiel 11A

### 2,4-Dimethyl-3-[6-(pyridin-3-yl)-1H-imidazo[1,2-b]pyrazol-1-yl]anilin

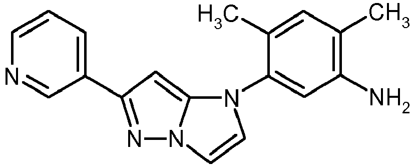

### Schritt 1: 1-(2,2-Diethoxyethyl)-N-(2,4-dimethyl-5-nitrophenyl)-3-(pyridin-3-yl)-1H-pyrazol-5-amin

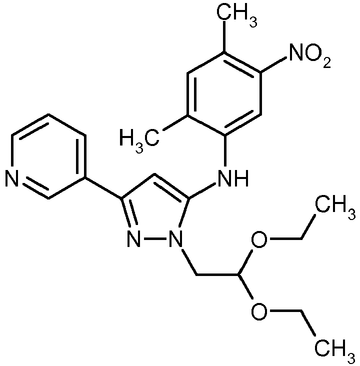

### Variante A:

200 mg (0.72 mmol) der Verbindung aus Beispiel 4A wurden zusammen mit 200 mg (0.87 mmol) 1-Brom-2,4-dimethyl-5-nitrobenzol analog der Vorschrift zu Beispiel 10A / Schritt 1 umgesetzt und aufgearbeitet. Man erhielt 200 mg (86% Reinheit, 56% d. Th.) der Titelverbindung.

LC/MS (Methode 3, ESIpos): Rₜ = 1.06 min, m/z = 426 [M+H]⁺.

### Variante B:

1.02 g (3.68 mmol) der Verbindung aus Beispiel 4A wurden zusammen mit 931 mg (4.05 mmol) 1-Brom-2,4-dimethyl-5-nitrobenzol in 15 ml Dioxan gelöst, mit Argon entgast und mit 83 mg (0.37 mmol) Palladium(II)acetat, 319 mg (0.55 mmol) Xantphos [4,5-Bis-(diphenylphosphino)-9,9-di-methylxanthen] sowie 3.60 g (11.0 mmol) Cäsiumcarbonat versetzt. Das Gemisch wurde in einem Mikrowellenofen (Biotage Initiator, mit dynamischer Steuerung der Einstrahlleistung) für 60 min auf 140°C erhitzt. Die Rohprodukte von drei identischen solchen Ansätzen wurden vereinigt und anschließend über Kieselgur filtriert. Das Filtrat wurde im Vakuum eingeengt und der Rückstand mittels MPLC in seine Komponenten aufgetrennt (Kieselgel, Laufmittel Cyclohexan/Ethylacetat 1:1 → 1:2). Die produkthaltigen Fraktionen wurden vereinigt und im Vakuum zur Trockene eingeengt. Es wurden insgesamt 4.18 g (89% d. Th.) der Titelverbindung erhalten.

¹H-NMR (400 MHz, CDCl₃, δ/ppm): 9.02 (m, 1H), 8.55 (dd, 1H), 8.07 (dt, 1H), 7.76 (s, 1H), 7.33 (dd, 1H), 7.11 (s, 1H), 6.66 (s, 1H), 6.41 (s, 1H), 4.81 (t, 1H), 4.27 (d, 2H), 3.88-3.80 (m, 2H), 3.66-3.58 (m, 2H), 2.52 (s, 3H), 2.32 (s, 3H), 1.26 (t, 6H).

LC/MS (Methode 3, ESIpos): Rₜ = 1.07 min, m/z = 426 [M+H]⁺.

### Schritt 2: 1-(2,4-Dimethyl-5-nitrophenyl)-6-(pyridin-3-yl)-1H-imidazo[1,2-b]pyrazol

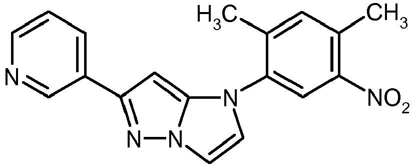

### Variante A:

Eine Lösung von 195 mg (86% Reinheit, 0.40 mmol) der Verbindung aus Beispiel 11A / Schritt 1 in 3.9 ml Ethanol und 0.39 ml (0.78 mmol) 2 M Schwefelsäure wurde in einem Mikrowellenofen (Biotage Initiator, mit dynamischer Steuerung der Einstrahlleistung) für 15 min auf 120°C erhitzt. Der Ansatz wurde danach in 50 ml Ethylacetat eingerührt. Es wurde mit gesättigter wässriger Kaliumcarbonat-Lösung und gesättigter wässriger Natriumchlorid-Lösung gewaschen, über Natriumsulfat getrocknet, filtriert und im Vakuum eingeengt. Nach Trocknen des Rückstands im Vakuum erhielt man 136 mg (85% Reinheit, 88% d. Th.) der Titelverbindung.

LC/MS (Methode 2, ESIpos): Rₜ = 1.89 min, m/z = 334 [M+H]⁺.

### Variante B:

Eine Lösung von 4.17 g (9.81 mmol) der Verbindung aus Beispiel 11A / Schritt 1 in 42 ml Ethanol und 9.8 ml (19.6 mmol) 2 M Schwefelsäure wurde auf vier Mikrowellen-Reaktionsgefäße verteilt, die anschließend in einem Mikrowellenofen (Biotage Initiator, mit dynamischer Steuerung der Einstrahlleistung) jeweils für 15 min auf 130°C erhitzt wurden. Der Inhalt der vier Reaktionsgefäße wurde danach in ca. 100 ml Wasser eingerührt. Durch Zusatz von gesättigter wässriger Natriumhydrogencarbonat-Lösung wurde die wässrige Phase auf einen neutralen pH-Wert eingestellt. Es wurde dann mit Ethylacetat wiederholt extrahiert. Die vereinigten organischen Extrakte wurden mit gesättigter wässriger Natriumchlorid-Lösung gewaschen, über Magnesiumsulfat getrocknet, filtriert und im Vakuum eingeengt. Der erhaltene Rückstand wurde mit wenig Acetonitril verrührt, abermals filtriert und der Feststoff im Hochvakuum getrocknet. Es wurden 2.8 g (85% d. Th.) der Titelverbindung erhalten.

¹H-NMR (400 MHz, DMSO-d₆, δ/ppm): 9.05 (m, 1H), 8.49 (dd, 1H), 8.18 (dt, 1H), 8.12 (s, 1H), 7.94 (d, 1H), 7.65 (s, 1H), 7.60 (d, 1H), 7.42 (dd, 1H), 6.42 (s, 1H), 2.59 (s, 3H), 2.35 (s, 3H).

LC/MS (Methode 4, ESIpos): Rₜ = 0.84 min, m/z = 334 [M+H]⁺.

### Schritt 3: 2,4-Dimethyl-3-[6-(pyridin-3-yl)-1H-imidazo[1,2-b]pyrazol-1-yl]anilin

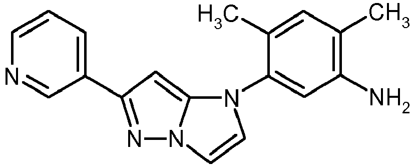

### Variante A:

135 mg (85% Reinheit, 0.34 mmol) der Verbindung aus Beispiel 11A / Schritt 2 wurden analog der Vorschrift zu Beispiel 10A / Schritt 3 zu 110 mg (87% Reinheit, 92% d. Th.) der Titelverbindung umgesetzt.

¹H-NMR (400 MHz, DMSO-d₆, δ/ppm): 9.04 (d, 1H), 8.47 (dd, 1H), 8.17 (dt, 1H), 7.82 (d, 1H), 7.42-7.37 (m, 2H), 6.97 (s, 1H), 6.67 (s, 1H), 6.25 (s, 1H), 4.99 (s, 2H), 2.09 (s, 3H), 2.05 (s, 3H).

LC/MS (Methode 2, ESIpos): Rₜ = 1.63 min, m/z = 304 [M+H]⁺.

### Variante B:

2.78 g (8.34 mmol) der Verbindung aus Beispiel 11A / Schritt 2, 2.63 g (41.7 mmol) Ammoniumformiat und 107 mg (0.10 mmol) Palladium (10% auf Kohle) wurden in 55 ml Ethanol und 5.5 ml Wasser 2.5 h unter Rückfluss erhitzt. Anschließend wurde der Katalysator über Kieselgur abfiltriert und das Filtrat im Vakuum eingedampft. Der Rückstand wurde in Dichlormethan aufgenommen und nacheinander mit gesättigter wässriger Natriumhydrogencarbonat-Lösung, Wasser und gesättigter wässriger Natriumchlorid-Lösung gewaschen. Nach Trocknen mit wasserfreiem Magnesiumsulfat wurde filtriert und das Filtrat im Vakuum eingeengt. Der Rückstand wurde im Hochvakuum getrocknet. Man erhielt 2.18 g (86% d. Th.) der Titelverbindung.

¹H-NMR (400 MHz, CDCl₃, δ/ppm): 9.04 (d, 1H), 8.52 (dd, 1H), 8.14 (dt, 1H), 7.46 (d, 1H), 7.32 (dd, 1H), 7.04 (s, 1H), 6.87 (d, 1H), 6.69 (s, 1H), 5.97 (s, 1H), 3.67 (br. s, 2H), 2.21 (s, 3H), 2.14 (s, 3H).

LC/MS (Methode 4, ESIpos): Rₜ = 0.68 min, m/z = 304 [M+H]⁺.

### Beispiel 12A

### 2-Fluor-4-methyl-3-[6-(pyridin-3-yl)-1H-imidazo[1,2-b]pyrazol-1-yl]anilin

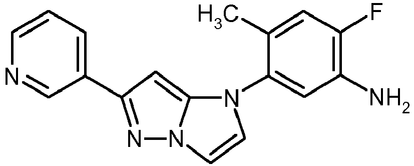

### Schritt 1: 1-Brom-4-fluor-2-methyl-5-nitrobenzol

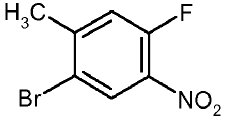

2.21 g (16.7 mmol) Nitroniumtetrafluoroborat wurden in 30 ml Dichlormethan suspendiert. Unter Rückfluss wurde eine Lösung von 3.0 g (15.9 mmol) 2-Brom-5-fluortoluol in 30 ml Dichlormethan zugetropft. Anschließend wurde das Gemisch noch 6 h unter Rückfluss nachgerührt. Man goss dann auf Eis, extrahierte mit Dichlormethan, wusch die organische Phase mit Wasser, trocknete sie über Natriumsulfat und engte am Rotationsverdampfer ein. Man erhielt ein gelbes Öl, aus dem sich über Nacht Kristalle bildeten. Diese wurden abgetrennt, mit 2 ml Pentan gewaschen und getrocknet. Man erhielt so 265 mg (6.9% d. Th.) der Titelverbindung.

GC/MS (Methode 8, EIpos): Rₜ = 4.49 min, m/z = 232/234 [M]⁺.

### Schritt 2: 1-(2,2-Diethoxyethyl)-N-(4-fluor-2-methyl-5-nitrophenyl)-3-(pyridin-3-yl)-1H-pyrazol-5-amin

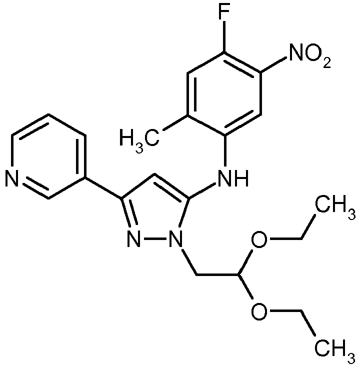

250 mg (0.91 mmol) der Verbindung aus Beispiel 4A wurden zusammen mit 254 mg (1.09 mmol) der Verbindung aus Beispiel 12A / Schritt 1 analog der Vorschrift zu Beispiel 10A / Schritt 1 umgesetzt und aufgearbeitet. Abweichend hiervon wurden die nach der präparativen HPLC bis auf ein Restvolumen eingeengten, mit etwas gesättigter Natriumhydrogencarbonat-Lösung alkalisch gestellten produkthaltigen Fraktionen mit Ethylacetat extrahiert. Die organische Phase wurde mit gesättigter wässriger Natriumchlorid-Lösung gewaschen, über Natriumsulfat getrocknet, filtriert und im Vakuum eingeengt. Nach Trocknen des Rückstands erhielt man 120 mg (85% Reinheit, 26% d. Th.) der Titelverbindung.

LC/MS (Methode 3, ESIpos): Rₜ = 1.03 min, m/z = 430 [M+H]⁺.

### Schritt 3: 1-(4-Fluor-2-methyl-5-nitrophenyl)-6-(pyridin-3-yl)-1H-imidazo[1,2-b]pyrazol

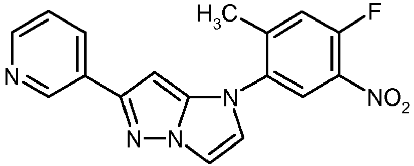

Eine Lösung von 120 mg (85% Reinheit, 0.28 mmol) der Verbindung aus Beispiel 12A / Schritt 2 in 2.8 ml Ethanol und 0.28 ml (0.56 mmol) 2 M Schwefelsäure wurde in einem Mikrowellenofen (Biotage Initiator, mit dynamischer Steuerung der Einstrahlleistung) für 30 min auf 120°C erhitzt. Der Ansatz wurde danach im Vakuum eingeengt und der Rückstand in Ethylacetat suspendiert. Es wurde mit gesättigter wässriger Natriumhydrogencarbonat-Lösung gewaschen, über Natriumsulfat getrocknet, filtriert und im Vakuum eingeengt. Nach Trocknen des Rückstands erhielt man 85 mg (90% Reinheit, 81% d. Th.) der Titelverbindung.

¹H-NMR (400 MHz, DMSO-d₆, δ/ppm): 9.04 (d, 1H), 8.49 (dd, 1H), 8.28 (d, 1H), 8.17 (dt, 1H), 7.95 (d, 1H), 7.82 (d, 1H), 7.60 (d, 1H), 7.42 (dd, 1H), 6.44 (s, 1H), 2.39 (s, 3H).

LC/MS (Methode 2, ESIpos): Rₜ = 1.81 min, m/z = 338 [M+H]⁺.

### Schritt 4: 2-Fluor-4-methyl-5-[6-(pyridin-3-yl)-1H-imidazo[1,2-b]pyrazol-1-yl]anilin

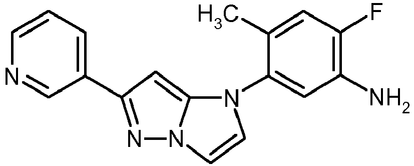

80 mg (0.23 mmol) der Verbindung aus Beispiel 12A / Schritt 3 wurden analog der Vorschrift zu Beispiel 10A / Schritt 3 zu 65 mg (94% Reinheit, 84% d. Th.) der Titelverbindung umgesetzt. Abweichend zu der Vorschrift wurde hier 30 min (statt 15 min) unter Rückfluss gerührt.

¹H-NMR (400 MHz, DMSO-d₆, δ/ppm): 9.04 (d, 1H), 8.47 (dd, 1H), 8.18 (dt, 1H), 7.84 (d, 1H), 7.43-7.38 (m, 2H), 7.09 (d, 1H), 6.82 (d, 1H), 6.28 (s, 1H), 5.29 (br, 2H), 2.07 (s, 3H).

LC/MS (Methode 3, ESIpos): Rₜ = 0.67 min, m/z = 308 [M+H]⁺.

### Beispiel 13A

### 4-Fluor-3-[6-(pyridin-3-yl)-1H-imidazo[1,2-b]pyrazol-1-yl]anilin

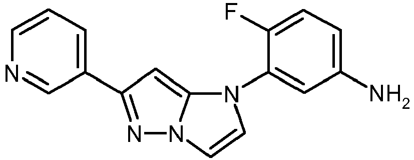

### Schritt 1: 1-(2,2-Diethoxyethyl)-N-(2-fluor-5-nitrophenyl)-3-(pyridin-3-yl)-1H-pyrazol-5-amin

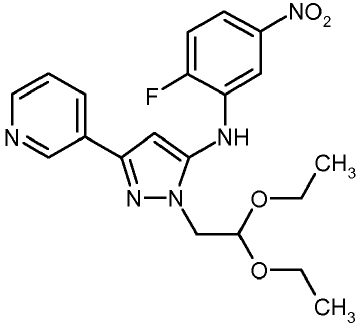

576 mg (2.08 mmol) der Verbindung aus Beispiel 4A wurden zusammen mit 504 mg (2.29 mmol) 2-Brom-1-fluor-4-nitrobenzol, 49.8 mg (0.21 mmol) Palladium(II)acetat, 181 mg (0.31 mmol) Xantphos [4,5-Bis-(diphenylphosphino)-9,9-dimethylxanthen] und 2.04 g (6.25 mmol) Cäsiumcarbonat in 8.6 ml 1,4-Dioxan unter Stickstoff 1 h unter Rückfluss erhitzt. Nach dem Abkühlen wurde über Celite filtriert, mit Dioxan nachgewaschen und das Filtrat im Vakuum eingeengt. Das so erhaltene Rohprodukt wurde mit dem Rohprodukt aus einem analog durchgeführten Probeansatz, ausgehend von 100 mg (0.36 mmol) der Verbindung aus Beispiel 4A, vereinigt und zusammen mittels einer Biotage-Anlage gereinigt (100 g Snap-Säule; Laufmittelgradient Hexan/Ethylacetat, von 0% Ethylacetat bis 100% Ethylacetat langsam ansteigend, dann Ethylacetat/Methanol, langsam auf 80% Methanol ansteigend). Auf diese Weise wurden 342 mg (38% d. Th.) der Titelverbindung erhalten.

¹H-NMR (400 MHz, DMSO-d₆, δ/ppm): 9.00 (d, 1H), 8.49 (dd, 1H), 8.36 (s, 1H), 8.14 (dt, 1H), 7.62-7.73 (m, 2H), 7.38-7.53 (m, 2H), 6.85 (s, 1H), 4.84 (t, 1H), 4.17 (d, 2H), 3.56 (dq, 2H), 3.39 (dq, 2H), 0.95 (t, 6H).

LC/MS (Methode 6, ESIpos): Rₜ = 1.18 min, m/z = 416 [M+H]⁺.

### Schritt 2: 1-(2-Fluor-5-nitrophenyl)-6-(pyridin-3-yl)-1H-imidazo[1,2-b]pyrazol

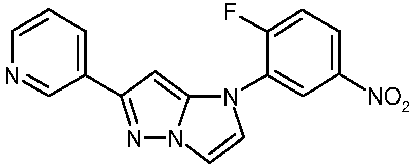

Eine Lösung von 341 mg (0.82 mmol) der Verbindung aus Beispiel 13A / Schritt 1 in 10 ml Ethanol wurde mit 0.99 ml (1.97 mmol) 2 M Schwefelsäure versetzt und anschließend für 30 min in der Mikrowelle auf 130°C erhitzt. Nach dem Abkühlen wurde das Reaktionsgemisch in 15 ml gesättigte wässrige Kaliumcarbonat-Lösung gegeben und gerührt. Die Mischung wurde zweimal mit je 50 ml Ethylacetat extrahiert, und die vereinigten organischen Phasen wurden mit gesättigter Natriumchlorid-Lösung gewaschen. Nach dem Trocknen über Natriumsulfat und Filtration wurde im Vakuum eingeengt. Das so erhaltene Rohprodukt wurde mittels einer Biotage-Anlage gereinigt (50 g Snap-Säule; Laufmittelgradient Hexan/Ethylacetat, von 0% Ethylacetat bis 100% Ethylacetat stetig ansteigend, dann Ethylacetat/Methanol, langsam auf 80% Methanol ansteigend). Auf diese Weise wurden 198 mg (67% d. Th.) der Titelverbindung erhalten.

¹H-NMR (400 MHz, DMSO-d₆, δ/ppm): 9.04 (d, 1H), 8.52 (dd, 1H), 8.48 (dd, 1H), 8.29 (ddd, 1H), 8.18 (dt, 1H), 8.00 (d, 1H), 7.82 (dd, 1H), 7.76 (dd, 1H), 7.41 (dd, 1H), 6.61 (d, 1H).

LC/MS (Methode 6, ESIpos): Rₜ = 0.87 min, m/z = 324 [M+H]⁺.

### Schritt 3: 4-Fluor-3-[6-(pyridin-3-yl)-1H-imidazo[1,2-b]pyrazol-1-yl]anilin

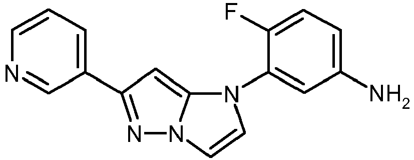

Zu einer Lösung von 198 mg (0.61 mmol) der Verbindung aus Beispiel 13A / Schritt 2 in einem Gemisch aus 7.55 ml Ethanol und 0.38 ml Wasser wurden 13 mg Palladium (10% auf Aktivkohle) gegeben. Nach Zugabe von 193 mg (3.1 mmol) Ammoniumformiat wurde die Reaktionsmischung 1 h unter Rückfluss erhitzt. Nach dem Abkühlen wurde abfiltriert, das Filtrat mit Ethylacetat verdünnt und die organische Phase mit gesättigter wässriger Natriumchlorid-Lösung gewaschen. Nach dem Trocknen über Natriumsulfat und Filtration wurde im Vakuum eingeengt. Das so erhaltene Rohprodukt der Titelverbindung (149 mg, 70% d. Th.) wurde ohne zusätzliche Reinigung weiter umgesetzt.

¹H-NMR (400 MHz, DMSO-d₆, δ/ppm): 9.02 (d, 1H), 8.46 (dd, 1H), 8.16 (dt, 1H), 7.87 (d, 1H), 7.51 (t, 1H), 7.40 (dd, 1H), 7.11 (dd, 1H), 6.82 (dd, 1H), 6.46-6.54 (m, 2H), 5.28 (br. s, 2H).

LC/MS (Methode 6, ESIpos): Rₜ = 0.74 min, m/z = 294 [M+H]⁺.

### Beispiel 14A

### 2-Methyl-5-[6-(pyridin-3-yl)-1H-imidazo[1,2-b]pyrazol-1-yl]anilin

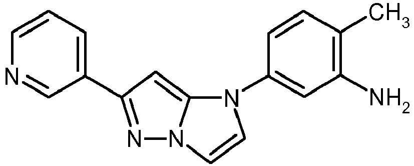

### Schritt 1: 1-(2,2-Diethoxyethyl)-N-(4-methyl-3-nitrophenyl)-3-(pyridin-3-yl)-1H-pyrazol-5-amin

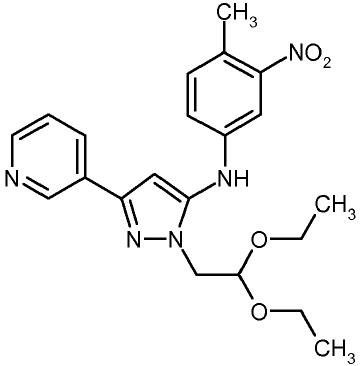

In Analogie zu Beispiel 13A / Schritt 1 wurden aus einem Probeansatz mit 200 mg (0.72 mmol) und dem Hauptansatz mit 1.76 g (6.37 mmol) der Verbindung aus Beispiel 4A und 172 mg (0.80 mmol) bzw. 1.51 g (7.01 mmol) 4-Brom-2-nitrotoluol 1.11 g (40% d. Th.) der Titelverbindung sowie 842 mg (29% d. Th.) einer leicht verunreinigten Charge der Titelverbindung erhalten.

¹H-NMR (400 MHz, DMSO-d₆, δ/ppm): 8.99 (d, 1H), 8.47 (dd, 1H), 8.40 (s, 1H), 8.12 (dt, 1H), 7.48 (d, 1H), 7.39 (dd, 1H), 7.30 (d, 1H), 7.17 (dd, 1H), 6.70 (s, 1H), 4.85 (t, 1H), 4.14 (d, 2H), 3.57 (dq, 2H), 3.36 (dq, 2H), 2.38 (s, 3H), 0.96 (t, 6H).

LC/MS (Methode 6, ESIpos): Rₜ = 1.19 min, m/z = 412 [M+H]⁺.

### Schritt 2: 1-(4-Methyl-3-nitrophenyl)-6-(pyridin-3-yl)-1H-imidazo[1,2-b]pyrazol

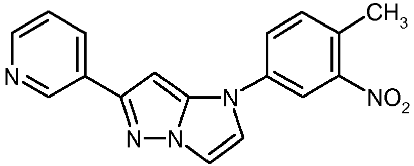

In Analogie zu Beispiel 13A / Schritt 2 wurden aus einem Probeansatz mit 841 mg (2.04 mmol) und dem Hauptansatz mit 1.1 g (2.67 mmol) der Verbindung aus Beispiel 14A / Schritt 1 ein Rohprodukt erhalten, welches zunächst über eine Biotage-Anlage gereinigt wurde (100 g Snap-Säule; Laufmittelgradient Hexan/Ethylacetat, von 0% Ethylacetat bis 100% Ethylacetat stetig ansteigend, danach Ethylacetat/Methanol, von 0% Methanol bis 80% Methanol langsam ansteigend). Auf diese Weise wurden 623 mg (69% d. Th.) der Titelverbindung sowie noch verunreinigtes Material erhalten. Das verunreinigte Material wurde durch nochmalige Chromatographie mittels Biotage-Anlage gereinigt (25 g Snap-Säule; Laufmittelgradient Ethylacetat/Methanol, von 0% Methanol bis 80% Methanol langsam ansteigend). Dies lieferte weitere 225 mg (25% d. Th.) der Titelverbindung.

¹H-NMR (400 MHz, DMSO-d₆, δ/ppm): 9.08 (d, 1H), 8.49 (dd, 1H), 8.18-8.24 (m, 2H), 8.07 (d, 1H), 7.99 (d, 1H), 7.96 (dd, 1H), 7.65 (d, 1H), 7.43 (ddd, 1H), 7.02 (s, 1H), 2.49 (s, 2H).

LC/MS (Methode 5, ESIpos): Rₜ = 0.93 min, m/z = 320 [M+H]⁺.

### Schritt 3: 2-Methyl-5-[6-(pyridin-3-yl)-1H-imidazo[1,2-b]pyrazol-1-yl]anilin

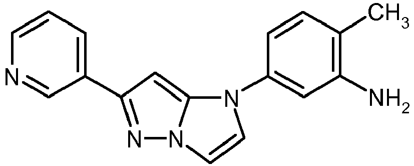

In Analogie zu Beispiel 13A / Schritt 3 wurde aus einem Probeansatz mit 225 mg (0.71 mmol) und dem Hauptansatz mit 622 mg (1.95 mmol) der Verbindung aus Beispiel 14A / Schritt 2 ein Rohprodukt der Titelverbindung erhalten (730 mg, 95% d. Th.), welches ohne zusätzliche Reinigung weiter umgesetzt wurde.

¹H-NMR (400 MHz, DMSO-d₆, δ/ppm): 9.04 (d, 1H), 8.47 (dd, 1H), 8.17 (dt, 1H), 7.85 (d, 1H), 7.72 (d, 1H), 7.42 (dd, 1H), 7.02 (d, 1H), 6.94 (d, 1H), 6.73-6.78 (m, 2H), 5.13 (br. s, 2H), 2.05 (s, 3H).

LC/MS (Methode 7, ESIpos): Rₜ = 0.78 min, m/z = 290 [M+H]⁺.

### Beispiel 15A

### 3-Brom-5-(pentafluor-λ⁶-sulfanyl)benzoesäure

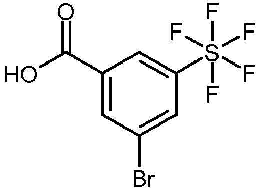

150 g (604 mmol) 3-(Pentafluor-λ⁶-sulfanyl)benzoesäure [Lit. z.B.: C. Zarantonello et al., J. Fluorine Chem. 2007, 128 (12), 1449-1453; WO 2005/047240-A1; auch kommerziell erhältlich] wurden in 300 ml Trifluoressigsäure vorgelegt und mit 90 ml konzentrierter Schwefelsäure versetzt. Zu der erhaltenen klaren Lösung wurden 161.4 g (907 mmol) *N*-Bromsuccinimid (NBS) hinzugefügt. Anschließend wurde das Reaktionsgemisch über Nacht (ca. 18 h) bei einer Temperatur von 50°C gerührt. Nach dem Abkühlen auf RT wurde das Gemisch vorsichtig in ca. 2.25 Liter Eiswasser eingerührt. Das ausgefallene Produkt wurde abgesaugt, mit Wasser gewaschen und im Hochvakuum getrocknet. Es wurden 193 g (96% d. Th., 98% Reinheit) der Titelverbindung erhalten.

¹H-NMR (400 MHz, DMSO-d₆, δ/ppm): 14.03 (br, 1H), 8.47 (t, 1H), 8.32 (t, 1H), 8.26 (dd, 1H).

LC/MS (Methode 3, ESIneg): Rₜ = 0.99 min, m/z = 325/327 [M-H]⁻.

### Beispiel 16A

### 3-[4-(tert.-Butoxycarbonyl)piperazin-1-yl]-5-(pentafluor-λ⁶-sulfanyl)benzoesäure

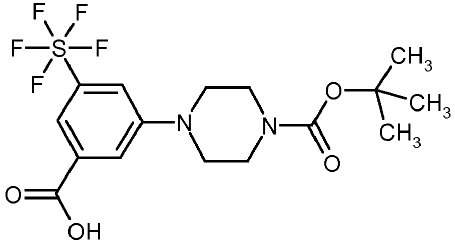

3.0 g (9.2 mmol) der Verbindung aus Beispiel 15A und 2.05 g (11.0 mmol) *tert.*-Butyl-piperazin-1-carboxylat wurden in 80 ml Toluol vorgelegt und mit Argon entgast. Zu dieser Lösung gab man nacheinander 135 mg (0.18 mmol) [2-(2-Aminoethyl)phenyl](chlor)palladium-dicyclohexyl-(2',4',6'-triisopropylbiphenyl-2-yl)phosphan *tert*.-Butyhnethylether-Addukt, 87.5 mg (0.18 mmol) Dicyclohexyl(2',4',6'-triisopropylbiphenyl-2-yl)phosphan sowie 2.1 g (22.0 mmol) Natrium-tert.-butylat. Das Gemisch wurde 3 h bei 110°C gerührt. Nach dieser Zeit wurden nochmals 67.8 mg (0.09 mmol) [2-(2-Aminoethyl)phenyl](chlor)palladium-dicyclohexyl(2',4',6'-triisopropylbiphenyl-2-yl)phosphan *tert*.-Butylmethylether-Addukt sowie 43.8 mg (0.09 mmol) Dicyclohexyl(2',4',6'-tri-isopropylbiphenyl-2-yl)phosphan hinzugefügt und die Mischung weitere 3 h bei 110°C nachgerührt. Danach wurde der Ansatz heiß über Kieselgur filtriert, und die Mutterlauge wurde am Rotationsverdampfer eingeengt. Der Rückstand wurde in 50 ml pH 7-Pufferlösung suspendiert und der ausgefallene Feststoff abfiltriert. Der Filterkuchen wurde mit Wasser gewaschen und im Vakuum getrocknet. Der Rückstand wurde mit 20 ml Methylenchlorid verrührt, die Suspension erneut filtriert und der Filterkuchen wieder im Vakuum getrocknet. Es wurden so 2.98 g (72% d. Th.) der Titelverbindung erhalten.

¹H-NMR (400 MHz, DMSO-d₆, δ/ppm): 7.71 (s, 1H), 7.66 (s, 1H), 7.22 (s, 1H), 3.47 (m, 4H), 3.15 (m, 4H), 1.42 (s, 9H).

LC/MS (Methode 4, ESIpos): Rₜ = 1.19 min, m/z = 433 [M+H]⁺.

### Beispiel 17A

### 3-(4-Methylpiperazin-1-yl)-5-(pentafluor-λ⁶-sulfanyl)benzoesäure-Trifluoracetat

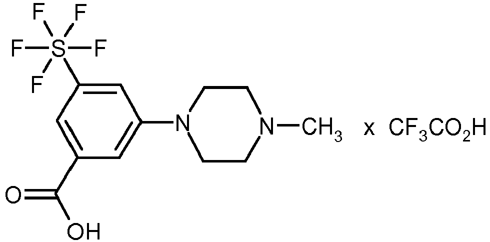

2.5 g (7.6 mmol) der Verbindung aus Beispiel 15A und 0.92 g (9.17 mmol) 1-Methylpiperazin wurden in 50 ml Toluol gelöst und mit Argon entgast. Zu dieser Lösung gab man nacheinander 126 mg (0.15 mmol) [2-(2-Aminoethyl)phenyl](chlor)palladium-dicyclohexyl(2',4',6'-triisopropyl-biphenyl-2-yl)phosphan *tert*.-Butylmethylether-Addukt, 73 mg (0.15 mmol) Dicyclohexyl(2',4',6'-triisopropylbiphenyl-2-yl)phosphan sowie 1.76 g (18.3 mmol) Natrium-*tert*.-butylat. Der Ansatz wurde 4 h unter Rückfluss gerührt, anschließend heiß über Kieselgur filtriert und das Filtrat am Rotationsverdampfer eingeengt. Der Rückstand wurde in 10 ml *tert*.-Butylmethylether suspendiert und zweimal mit je 15 ml 1 M Natronlauge extrahiert. Die wässrige Phase wurde mit konzentrierter Salzsäure neutral gestellt, am Rotationsverdampfer eingeengt und der Rückstand durch präparative HPLC gereinigt (Methode 13). Man erhielt 1.06 g (30% d. Th.) der Titelverbindung.

¹H-NMR (400 MHz, DMSO-d₆, δ/ppm): 7.73 (s, 2H), 7.69 (s, 1H), 3.60-3.20 (br, 8H), 2.85 (s, 3H).

LC/MS (Methode 3, ESIpos): Rₜ = 0.57 min, m/z = 347 [M+H]⁺.

### Beispiel 18A

### 3-(Morpholin-4-yl)-5-(pentafluor-λ⁶-sulfanyl)benzoesäure

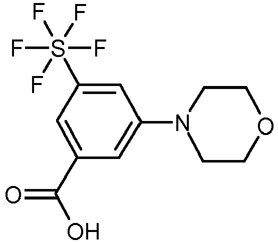

1.5 g (4.6 mmol) der Verbindung aus Beispiel 15A und 0.48 g (5.5 mmol) Morpholin wurden in 15 ml Toluol gelöst und mit Argon entgast. Zu dieser Lösung gab man nacheinander 75 mg (0.09 mmol) [2-(2-Aminoethyl)phenyl](chlor)palladium-dicyclohexyl(2',4',6'-triisopropylbiphenyl-2-yl)-phosphan *tert*.-Butylmethylether-Addukt, 44 mg (0.09 mmol) Dicyclohexyl(2',4',6'-triisopropyl-biphenyl-2-yl)phosphan sowie 1.06 g (11.0 mmol) Natrium-tert.-butylat. Der Ansatz wurde 30 min in einem Mikrowellenofen (Biotage Initiator, mit dynamischer Steuerung der Einstrahlleistung) bei 130°C gerührt. Der Ansatz wurde anschließend in 10 ml gesättigte wässrige Natriumchlorid-Lösung eingerührt und zweimal mit je 30 ml Ethylacetat extrahiert. Die organische Phase wurde mit pH 4-Pufferlösung gewaschen, über Natriumsulfat getrocknet und am Rotationsverdampfer eingeengt. Der Rückstand wurde in 5 ml *tert*.-Butylmethylether gelöst und mit 10 ml Pentan versetzt. Der entstandene Niederschlag wurde abfiltriert und getrocknet. Man erhielt 1.04 g (68% d. Th.) der Titelverbindung.

¹H-NMR (400 MHz, DMSO-d₆, δ/ppm): 7.73 (s, 1H), 7.69 (s, 1H), 7.35 (s, 1H), 3.75 (m, 4H), 3.20 (m, 4H).

LC/MS (Methode 3): Rₜ = 0.91 min; MS (ESIpos): m/z = 334 [M+H]⁺.

### Beispiel 19A

### 3-(2-Hydroxypropan-2-yl)-5-(pentafluor-λ⁶-sulfanyl)benzoesäure

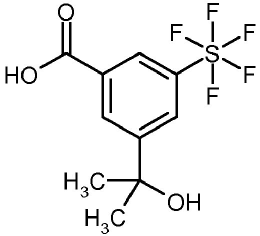

7.6 g (23.3 mmol) der Verbindung aus Beispiel 15A wurden unter Argon in 76 ml THF gelöst und mit einigen Körnchen 4Å-Molekularsieb versetzt. Dann wurde der Ansatz auf 0°C abgekühlt, und 53.7 ml (69.8 mmol) einer 1.3 M Lösung von 2-Propylmagnesiumchlorid-Lithiumchlorid-Komplex in THF wurden langsam zugetropft. Nach beendeter Zugabe wurde das Reaktionsgemisch 30 min bei 0°C nachgerührt. Dann wurden 2.6 ml (34.9 mmol) wasserfreies Aceton zugegeben und der Ansatz 1 h bei 0°C weitergerührt. Nun setzte man 90 ml 1 M Salzsäure zu und ließ den Ansatz über 40 min langsam auf RT kommen. Dann extrahierte man dreimal mit Ethylacetat. Die vereinigten organischen Phasen wurden mit gesättigter Natriumchlorid-Lösung gewaschen, über Natriumsulfat getrocknet, filtriert und eingeengt. Der Rückstand wurde mit Petrolether unter Zusatz von etwas Diethylether verrieben, abfiltriert und getrocknet. Es wurden 5.8 g (80% d. Th.) der Titelverbindung als weißer Feststoff erhalten.

¹H-NMR (400 MHz, DMSO-d₆, δ/ppm): 8.27 (s, 1H), 8.19 (s, 1H), 8.15 (s, 1H), 5.54 (s, 1H), 1.48 (s, 6 H).

LC/MS (Methode 3): Rₜ = 0.89 min; MS (ESIneg): m/z = 305 [M-H]⁻.

### Beispiel 20A

### 3-[1-(tert.-Butoxycarbonyl)-3-hydroxyazetidin-3-yl]-5-(pentafluor-λ⁶-sulfanyl)benzoesäure

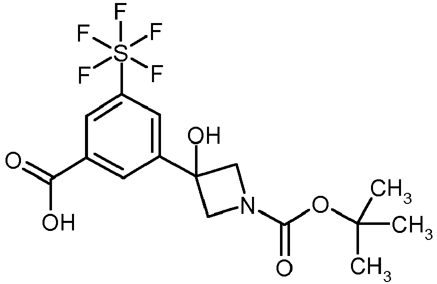

Analog der Vorschrift zu Beispiel 19A erhielt man aus 250 mg (0.76 mmol) der Verbindung aus Beispiel 15A und 0.25 ml (1.15 mmol) *tert*.-Butyl-3-oxoazetidin-1-carboxylat nach Aufreinigung mittels präparativer HPLC (Methode 27) 65 mg (20% d. Th.) der Titelverbindung.

¹H-NMR (400 MHz, DMSO-d₆, δ/ppm): 13.82 (br, 1H), 8.29 (s, 1H), 8.22 (s, 1H), 8.15 (s, 1H), 6.89 (s, 1H), 4.09 (quart, 4H), 1.43 (s, 9H).

LC/MS (Methode 3): Rₜ = 1.01 min; MS (ESIneg): m/z = 418 [M-H]⁻.

### Beispiel 21A

### 3-tert.-Butyl-5-(4-methylpiperazin-1-yl)benzoesäure-Trifluoracetat

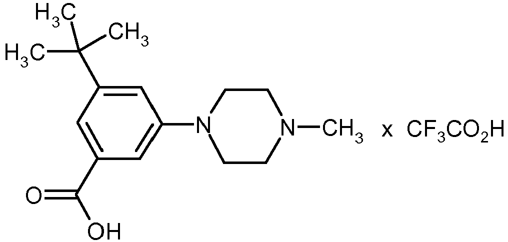

500 mg (1.9 mmol) 3-Brom-5-*tert*.-butylbenzoesäure und 233 mg (2.3 mmol) 1-Methylpiperazin wurden in 12.7 ml Toluol vorgelegt und mit Argon entgast. Zu dieser Lösung gab man nacheinander 32.2 mg (0.04 mmol) [2-(2-Aminoethyl)phenyl](chlor)palladium-dicyclohexyl(2',4',6'-tri-isopropylbiphenyl-2-yl)phosphan *tert*.-Butylmethylether-Addukt, 18.5 mg (0.04 mmol) Dicyclohexyl(2',4',6'-triisopropylbiphenyl-2-yl)phosphan und 448.5 mg (4.7 mmol) Natrium-*tert*.-butylat. Die Reaktionsmischung wurde 30 min in einem Mikrowellenofen (Biotage Initiator, mit dynamischer Steuerung der Einstrahlleistung) bei 130°C gerührt. Der Ansatz wurde danach heiß filtriert und der Rückstand gut mit Toluol nachgewaschen. Die vereinigten Filtrate wurden im Vakuum eingeengt und der Rückstand mittels präparativer HPLC gereinigt (Methode 13). Es wurden so 255 mg (34% d. Th.) der Titelverbindung erhalten.

¹H-NMR (400 MHz, DMSO-d₆, δ/ppm): 9.94 (br, 1H), 7.49 (s, 1H), 7.34 (s, 1H), 7.26 (s, 1H), 3.91 (br, 2H), 3.53 (br, 2H), 3.17 (br, 2H), 2.99 (br, 2H), 2.88 (s, 3H), 1.29 (s, 9H).

LC/MS (Methode 3): Rₜ = 0.64 min; MS (ESIpos): m/z = 277 (M+H)⁺.

### Beispiel 22A

### 3-tert.-Butyl-5-(pyrrolidin-1-ylmethyl)benzoesäure-Trifluoracetat

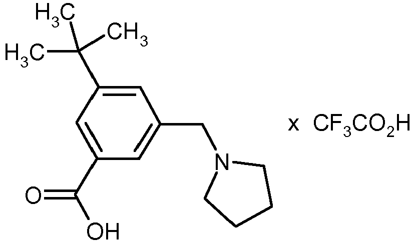

### Schritt 1: 3-tert.-Butyl-5-formylbenzoesäure

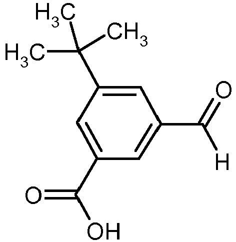

0.38 g (1.7 mmol) Methyl-3-*tert*.-butyl-5-formylbenzoat [zur Herstellung siehe z.B. WO 2008/ 089034-A2, Intermediat K / Stufe 1] wurden in 20 ml Wasser vorgelegt, mit 0.5 g Lithiumhydroxid-Monohydrat versetzt und 1 h bei RT gerührt. Danach wurde die Reaktionslösung mit 1 M Salzsäure auf pH 2 gestellt und mit Ethylacetat extrahiert. Die organische Phase wurde über Natriumsulfat getrocknet, filtriert und am Rotationsverdampfer eingeengt. Es wurden 0.31 g (87% d. Th.) der Titelverbindung erhalten.

¹H-NMR (400 MHz, DMSO-d₆, δ/ppm): 13.46-13.20 (m, 1H), 10.10 (s, 1H), 8.28 (s, 1H), 8.25 (s, 1H), 8.19 (s, 1H), 1.36 (s, 9H).

LC/MS (Methode 3): Rₜ = 0.92 min; MS (ESIpos): m/z = 207 (M+H)⁺.

### Schritt 2: 3-tert.-Butyl-5-(pyrrolidin-1-ylmethyl)benzoesäure-Trifluoracetat

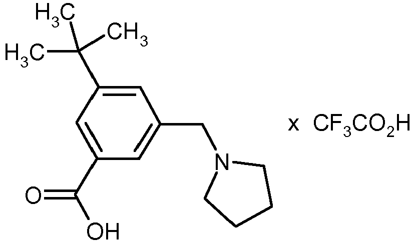

500 mg (2.4 mmol) der Verbindung aus Beispiel 22A / Schritt 1 wurden in 24 ml Methylenchlorid vorgelegt und nacheinander mit 258 mg (3.6 mmol) Pyrrolidin, 719 mg (3.4 mmol) Natriumtriacetoxyborhydrid sowie 1 ml (17.5 mmol) Essigsäure versetzt. Der Ansatz wurde 2 h bei RT gerührt. Danach wurde das Gemisch mit etwas Wasser versetzt und eingeengt. Der Rückstand wurde mittels präparativer HPLC gereinigt (Methode 13). Es wurden 560 mg (62% d. Th.) der Titelverbindung erhalten.

¹H-NMR (400 MHz, DMSO-d₆, δ/ppm): 8.00 (s, 1H), 7.96 (s, 1H), 7.83 (s, 1H), 4.43 (s, 2H), 3.11 (br, 2H), 2.02 (br, 2H), 1.89 (br, 2H), 1.33 (s, 9H) [weitere Signale unter Lösungsmittel-Peaks verborgen].

LC/MS (Methode 3): Rₜ = 0.64 min; MS (ESIpos): m/z = 262 (M+H)⁺.

### Beispiel 23A

### 3-Cyano-5-(pentafluor-λ⁶-sulfanyl)benzoesäure

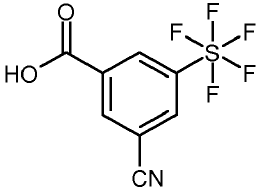

200 mg (0.61 mmol) der Verbindung aus Beispiel 15A wurden in 2.0 ml DMF gelöst und mit Argon entgast. Nach Zugabe von 79 mg (0.67 mmol) Zinkcyanid und 42 mg (0.04 mmol) Tetrakis-(triphenylphosphin)palladium(0) wurde der Ansatz 30 min in einem Mikrowellenofen (Biotage Initiator, mit dynamischer Steuerung der Einstrahlleistung) bei 120°C gerührt. Nach Abkühlen wurde von festen Bestandteilen abfiltriert, und das Filtrat wurde mittels präparativer HPLC (Methode 15) in seine Komponenten aufgetrennt. Nach Eindampfen der Produktfraktionen und Trocknen des Rückstands im Hochvakuum wurden 115 mg (88% Reinheit, 61% d. Th.) der Titelverbindung erhalten.

¹H-NMR (400 MHz, DMSO-d₆, δ/ppm): 14.18 (br, 1H), 8.89 (t, 1H), 8.60 (t, 1H), 8.50 (t, 1H).

LC/MS (Methode 3, ESIneg): Rₜ = 0.83 min, m/z = 272 [M-H]⁻.

### Beispiel 24A

### 3-Hydroxy-5-(pentafluor-λ⁶-sulfanyl)benzoesäure

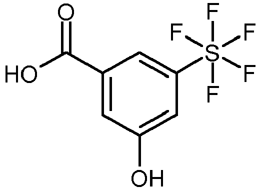

2.0 g (6.11 mmol) der Verbindung aus Beispiel 15A wurden in 20 ml Dioxan und 2 ml Wasser gelöst, mit Argon entgast, mit 280 mg (0.31 mmol) Tris(dibenzylidenaceton)dipalladium, 325 mg (0.76 mmol) 2-Di-*tert*.-butylphosphino-2',4',6'-triisopropyl-1,1'-biphenyl sowie 1.37 g (24.5 mmol) gepulvertem Kaliumhydroxid versetzt und 2 h bei 100°C gerührt. Der Ansatz wurde danach in 50 ml 1 M Salzsäure eingerührt und zweimal mit je 50 ml Ethylacetat extrahiert. Die vereinigten organischen Phasen wurden mit gesättigter Natriumchlorid-Lösung gewaschen, über Natriumsulfat getrocknet und im Vakuum eingeengt. Der Rückstand wurde in Dichlormethan/Pentan (9:1) suspendiert, der Feststoff abfiltriert und der Filterkuchen im Hochvakuum getrocknet. Man erhielt so 980 mg (59% d. Th.) der Titelverbindung.

¹H-NMR (400 MHz, DMSO-d₆, δ/ppm): 13.58 (br, 1H), 10.74 (s, 1H), 7.74 (s, 1H), 7.58 (s, 1H), 7.46 (t, 1H).

LC/MS (Methode 3, ESIneg): Rₜ = 0.78 min, m/z = 263 [M-H]⁻.

### Beispiel 25A

### 3-Methoxy-5-(pentafluor-λ⁶-sulfanyl)benzoesäure

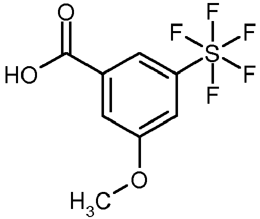

150 mg (0.57 mmol) der Verbindung aus Beispiel 24A wurden in 1.5 ml Methanol gelöst, mit 0.25 ml (1.14 mmol) einer 25%-igen Lösung von Natriummethylat in Methanol sowie mit 0.04 ml (0.62 mmol) Methyliodid versetzt und in einem Mikrowellenofen (Biotage Initiator, mit dynamischer Steuerung der Einstrahlleistung) für 30 min auf 120°C erhitzt. Nach dem Abkühlen wurde der Ansatz mit 1 M Salzsäure sauer gestellt und am Rotationsverdampfer etwas eingeengt. Der entstandene Niederschlag wurde abfiltriert, mit Wasser gewaschen und im Hochvakuum getrocknet. Man erhielt 96 mg (94% Reinheit, 57% d. Th.) der Titelverbindung.

LC/MS (Methode 4, ESIneg): Rₜ = 0.98 min, m/z = 277 [M-H]⁻.

### Beispiel 26A

### 3-(2-Methyl-1H-imidazol-1-yl)-5-(pentafluor-λ⁶-sulfanyl)benzoesäure

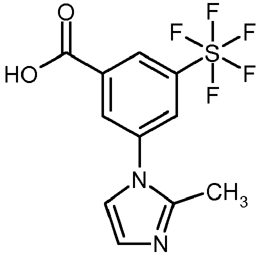

1.0 g (3.06 mmol) der Verbindung aus Beispiel 15A und 276 mg (3.36 mmol) 2-Methylimidazol wurden in 20 ml DMF und 2 ml Wasser gelöst und mit Argon entgast. Vorsichtig versetzte man mit 2.06 g (6.42 mmol) Tetraethylammoniumbicarbonat und anschließend mit 232 mg (1.22 mmol) Kupfer(I)iodid sowie 177 mg (1.22 mmol) 8-Hydroxychinolin. Das Gemisch wurde in einem Mikrowellenofen (Biotage Initiator, mit dynamischer Steuerung der Einstrahlleistung) für 60 min auf 160°C erhitzt. Der Ansatz wurde danach filtriert, das Filtrat mit konzentrierter Salzsäure auf pH 1 eingestellt und im Vakuum eingeengt. Der Rückstand wurde mittels präparativer HPLC (Methode 17) in seine Komponenten aufgetrennt. Nach Eindampfen der Produktfraktionen und Trocknen des Rückstands im Hochvakuum wurden 238 mg (94% Reinheit, 22% d. Th.) der Titelverbindung erhalten.

¹H-NMR (400 MHz, DMSO-d₆, δ/ppm): 8.61 (t, 1H), 8.48 (t, 1H), 8.45 (t, 1H), 7.97 (d, 1H), 7.75 (d, 1H), 2.54 (s, 3H).

LC/MS (Methode 3, ESIpos): Rₜ = 0.57 min, m/z = 329 [M+H]⁺.

### Beispiel 27A

### 3-tert.-Butyl-5-(2-methyl-1H-imidazol-1-yl)benzoesäure

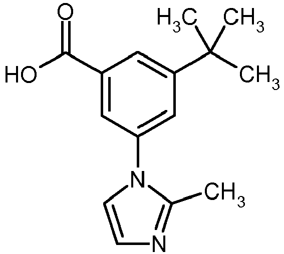

Analog der Vorschrift zu Beispiel 26A erhielt man aus 500 mg (1.95 mmol) 3-Brom-5-tert.-butylbenzoesäure und 175 mg (2.14 mmol) 2-Methylimidazol 405 mg (56% d. Th.) der Titelverbindung.

¹H-NMR (400 MHz, DMSO-d₆, δ/ppm): 8.15 (t, 1H), 7.97-7.93 (m, 3H), 7.78 (d, 1H) [weitere Signale unter Lösungsmittel-Peaks verborgen].

LC/MS (Methode 3, ESIpos): Rₜ = 0.56 min, m/z = 259 [M+H]⁺.

### Beispiel 28A

### 3-tert.-Butyl-5-(2-hydroxypropan-2-yl)benzoesäure

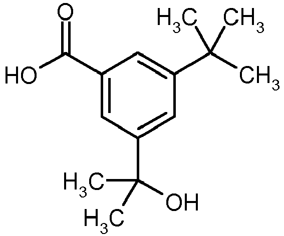

3.0 g (12.7 mmol) 3-*tert*.-Butyl-5-(methoxycarbonyl)benzoesäure wurden in 40 ml abs. THF gelöst. Unter Eisbadkühlung wurden 12.7 ml einer 3 M Lösung von Methylmagnesiumbromid in THF zugetropft und das Gemisch 1 h ohne Kühlung nachgerührt. Anschließend wurden weitere 12.7 ml Methylmagnesiumbromid-Lösung zugegeben und erneut 1 h bei RT nachgerührt. Danach wurden abermals 12.7 ml Methylmagnesiumbromid-Lösung zugesetzt und 72 h bei RT nachgerührt. Der Ansatz wurde dann mit gesättigter wässriger Ammoniumchlorid-Lösung versetzt und mit konzentrierter Salzsäure auf pH 1 gestellt. Das Gemisch wurde mit Ethylacetat extrahiert und die organische Phase im Vakuum eingeengt. Der Rückstand wurde mittels präparativer HPLC (Methode 15) in seine Komponenten aufgetrennt. Die Produktfraktionen wurden am Rotationsverdampfer vom Lösungsmittel befreit und der Rückstand im Hochvakuum getrocknet. Man erhielt 1.40 g (47% d.Th.) der Titelverbindung.

¹H-NMR (400 MHz, DMSO-d₆, δ/ppm): 12.83 (br, 1H), 7.87 (t, 1H), 7.80 (t, 1H), 7.76 (t, 1H), 5.14 (br, 1H), 1.44 (s, 6H), 1.31 (s, 9H).

LC/MS (Methode 3, ESIneg): Rₜ = 0.84 min, m/z = 235 [M-H]⁻.

### Beispiel 29A

### 3-{2-[(tert.-Butoxycarbonyl)amino]ethoxy}-5-(pentafluor-λ⁶-sulfanyl)benzoesäure

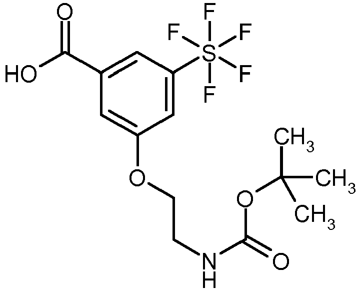

150 mg (0.57 mmol) der Verbindung aus Beispiel 24A, 140 mg (0.63 mmol) tert.-Butyl-(2-bromethyl)carbamat und 388 mg (1.2 mmol) Cäsiumcarbonat wurden in 1.5 ml DMF vorgelegt und über Nacht bei RT gerührt. Danach wurden 1.7 ml 1 M Natronlauge zugegeben und der Ansatz 1 h bei RT nachgerührt. Anschließend wurde das Reaktionsgemisch mit konzentrierter Essigsäure leicht sauer gestellt und direkt mittels präparativer HPLC aufgereinigt (Methode 18). Es wurden 63 mg (26% d. Th.) der Titelverbindung erhalten.

LC/MS (Methode 3, ESIpos): Rₜ = 1.06 min, m/z = 408 [M+H]⁺.

### Beispiel 30A

### 3-{3-[(tert.-Butoxycarbonyl)amino]propoxy}-5-(pentafluor-λ⁶-sulfanyl)benzoesäure

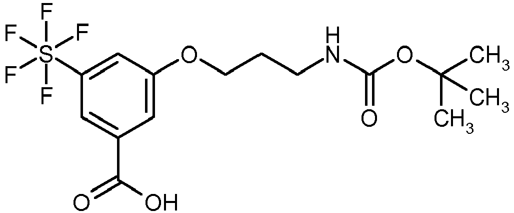

1.25 g (4.73 mmol) der Verbindung aus Beispiel 24A wurden unter Argon mit 1.46 g (6.2 mmol) *tert*.-Butyl-(3-brompropyl)carbamat und 4.62 g (14.2 mmol) Cäsiumcarbonat in 25 ml DMF zusammengegeben und bis zur vollständigen Umsetzung bei RT gerührt. Danach wurde der Ansatz mit 4 M Salzsäure sauer gestellt und mit Ethylacetat extrahiert. Die organische Phase wurde über Magnesiumsulfat getrocknet, filtriert und im Vakuum eingeengt. Der Rückstand wurde in 25 ml THF und 12 ml Wasser aufgenommen und nach Zugabe von 596 mg (14.2 mmol) Lithiumhydroxid-Monohydrat 4 h bei 40°C gerührt. Nach beendeter Reaktion wurde Wasser und Ethylacetat zugesetzt und die Mischung mit 1 M Salzsäure auf pH 2 gestellt. Die organische Phase wurde abgetrennt, über Magnesiumsulfat getrocknet, filtriert und am Rotationsverdampfer eingeengt. Das Rohprodukt wurde über präparative HPLC gereinigt (Methode 19). Es wurden 1.64 g (92% Reinheit, 76% d. Th.) der Titelverbindung erhalten.

¹H-NMR (400 MHz, DMSO-d₆, δ/ppm): 13.73 (br, 1H), 7.86 (s, 1H), 7.68 (s, 1H), 7.64 (t, 1H), 6.92 (t, 1H), 4.13 (t, 2H), 3.10 (quart, 2H), 1.85 (quint, 2H), 1.36 (s, 9H).

LC/MS (Methode 3, ESIpos): Rₜ = 1.10 min, m/z = 422 [M+H]⁺.

### Beispiel 31A

### 3-{[1-(tert.-Butoxycarbonyl)azetidin-3-yl]oxy}-5-(pentafluor-λ⁶-sulfanyl)benzoesäure

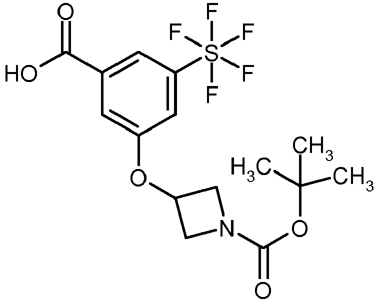

100 mg (0.38 mmol) der Verbindung aus Beispiel 24A und 104 mg (0.42 mmol) *tert.*-Butyl-3-[(methylsulfonyl)oxy]azetidin-1-carboxylat wurden zusammen mit 259 mg (0.80 mmol) Cäsiumcarbonat in 1 ml DMF vorgelegt und bei 90°C über Nacht gerührt. Danach wurde der Ansatz in 10 ml 0.1 M Salzsäure eingerührt und mit Ethylacetat extrahiert. Die organische Phase wurde mit gesättigter Natriumchlorid-Lösung gewaschen, über Natriumsulfat getrocknet, filtriert und im Vakuum eingeengt. Den Rückstand reinigte man mittels präparativer HPLC (Methode 18). Es wurden 60 mg (88% Reinheit, 33% d. Th.) der Titelverbindung erhalten.

¹H-NMR (400 MHz, DMSO-d₆, δ/ppm): 13.80 (br, 1H), 7.91 (s, 1H), 7.63 (t, 1H), 7.53 (s, 1H), 5.23 (m, 1H), 4.32 (m, 2H), 3.85 (m, 2H), 1.39 (s, 9H).

LC/MS (Methode 3, ESIneg): Rₜ = 1.10 min, m/z = 418 [M-H]⁻.

### Beispiel 32A

### 3-(Methylsulfonyl)-5-(pentafluor-λ⁶-sulfanyl)benzoesäure

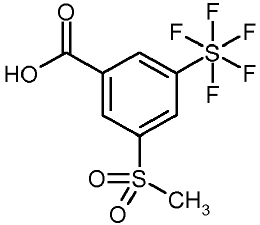

Ein Gemisch von 250 mg (0.764 mmol) der Verbindung aus Beispiel 15A, 86 mg (0.841 mmol) Natriummethansulfinat, 19 mg (0.168 mmol) (*S*)-Prolin und 23 mg (0.168 mmol) Kaliumcarbonat in 3 ml DMSO/Wasser (4:1) wurde zunächst entgast, dann mit 16 mg (0.084 mmol) Kupfer(I)iodid versetzt und schließlich unter einer Argonatmosphäre in einem Mikrowellenofen (Biotage Initiator, mit dynamischer Steuerung der Einstrahlleistung) für 30 min auf 150°C erhitzt. Nach dem Abkühlen auf RT wurde das Reaktionsgemisch über wenig Celite filtriert und anschließend mittels präparativer HPLC (Methode 9) in seine Komponenten aufgetrennt. Nach Eindampfen der Produktfraktionen und Trocknen des Rückstands im Hochvakuum wurden 83 mg (33% d. Th.) der Titelverbindung erhalten.

¹H-NMR (400 MHz, DMSO-d₆, δ/ppm): 14.27 (br, 1H), 8.65 (t, 1H), 8.62 (t, 1H), 8.56 (dd, 1H), 3.46 (s, 3H).

LC/MS (Methode 3, ESIneg): Rₜ = 0.75 min, m/z = 325 [M-H]⁻.

### Beispiel 33A

### 3-Chlor-5-(pentafluor-λ⁶-sulfanyl)benzoesäure

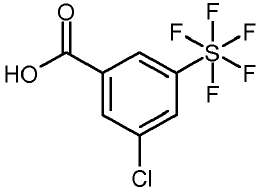

Eine Lösung von 250 mg (0.764 mmol) der Verbindung aus Beispiel 15A in 3 ml wasserfreiem DMF wurde zunächst entgast, dann mit 378 mg (3.82 mmol) Kupfer(I)chlorid und 73 mg (0.382 mmol) Kupfer(I)iodid versetzt und schließlich unter einer Argonatmosphäre in einem Mikrowellenofen (Biotage Initiator, mit dynamischer Steuerung der Einstrahlleistung) für 60 min auf 150°C erhitzt. Nach dem Abkühlen auf RT wurde das Reaktionsgemisch über wenig Celite filtriert und anschließend mittels präparativer HPLC (Methode 9) in seine Komponenten aufgetrennt. Nach Eindampfen der Produktfraktionen und Trocknen des Rückstands im Hochvakuum wurden 127 mg (59% d. Th.) der Titelverbindung erhalten.

¹H-NMR (400 MHz, DMSO-d₆, δ/ppm): 14.04 (br, 1H), 8.40 (t, 1H), 8.22 (dd, 1H), 8.20 (t, 1H).

LC/MS (Methode 3, ESIneg): Rₜ = 1.02 min, m/z = 281/283 [M-H]⁻.

### Beispiel 34A

### 3-Methyl-5-(pentafluor-λ⁶-sulfanyl)benzoesäure

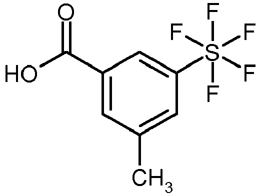

Eine Lösung von 250 mg (0.764 mmol) der Verbindung aus Beispiel 15A und 27 mg (0.023 mmol) Tetrakis(triphenylphosphin)palladium(0) in 2.5 ml wasserfreiem THF wurde mit 1.15 ml (2.29 mmol) einer 2 M Lösung von Trimethylaluminium in Hexanfraktion versetzt und anschließend unter einer Argonatmosphäre in einem Mikrowellenofen (Biotage Initiator, mit dynamischer Steuerung der Einstrahlleistung) 120 min auf 150°C erhitzt. Nach dem Abkühlen auf RT wurde das Reaktionsgemisch mit ca. 10 ml Wasser versetzt und dreimal mit je ca. 10 ml Ethylacetat extrahiert. Die vereinigten organischen Extrakte wurden mit gesättigter Natriumchlorid-Lösung gewaschen, über wasserfreiem Magnesiumsulfat getrocknet, filtriert und anschließend am Rotationsverdampfer zur Trockene eingedampft. Das Produkt wurde mittels präparativer HPLC (Methode 9) isoliert. Nach Eindampfen der Produktfraktionen und Trocknen des Rückstands im Hochvakuum wurden 98 mg (47% d. Th., 95% Reinheit) der Titelverbindung erhalten.

¹H-NMR (400 MHz, DMSO-d₆, δ/ppm): 13.64 (sehr breit, 1H), 8.09 (br, 1H), 8.04 (br, 2H), 2.48 (s, 3H).

LC/MS (Methode 3, ESIneg): Rₜ = 0.97 min, m/z = 261 [M-H]⁻.

### Beispiel 35A

### 3-{[3-(Dimethylamino)propyl](methyl)amino}-5-(trifluormethyl)benzoesäure-Dihydrochlorid

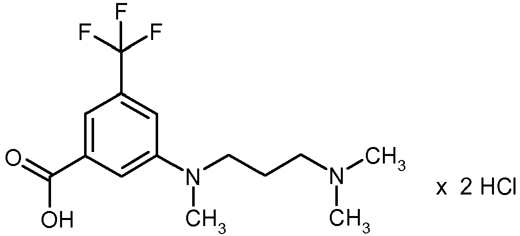

### Schritt 1: 3-{[3-(Dimethylamino)propyl](methyl)amino}-5-(trifluormethyl)benzonitril

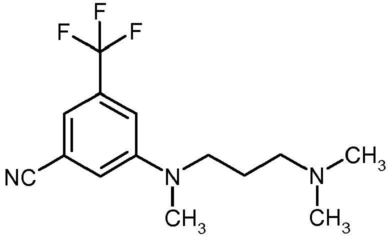

500 mg (2.64 mmol) 3-Fluor-5-(trifluormethyl)benzonitril, 338 mg (2.91 mmol) *N,N,N'*-Trimethyl-propan-1,3-diamin und 767 mg (5.52 mmol) Kaliumcarbonat in 5.0 ml DMSO wurden 8 h bei 110°C gerührt. Das Reaktionsgemisch wurde anschließend direkt mittels präparativer HPLC (Methode 12) in seine Komponenten aufgetrennt. Die Produktfraktionen wurden vom Lösungsmittel befreit, der Rückstand in Ethylacetat suspendiert und nacheinander mit gesättigter wässriger Kaliumcarbonat-Lösung und gesättigter wässriger Natriumchlorid-Lösung gewaschen. Die organische Phase wurde über Natriumsulfat getrocknet und eingeengt. Man erhielt 290 mg (38% d. Th.) der Titelverbindung.

¹H-NMR (400 MHz, DMSO-d₆, δ/ppm): 7.38 (s, 1H), 7.31 (s, 1H), 7.21 (s, 1H), 3.44 (t, 2H), 2.97 (s, 3H), 2.18 (t, 3H), 2.12 (s, 6H), 1.62 (quint, 2H).

LC/MS (Methode 3, ESIpos): Rₜ = 0.72 min, m/z = 286 [M+H]⁺.

### Schritt 2: 3-{[3-(Dimethylamino)propyl](methyl)amino}-5-(trifluormethyl)benzoesäure-Dihydrochlorid

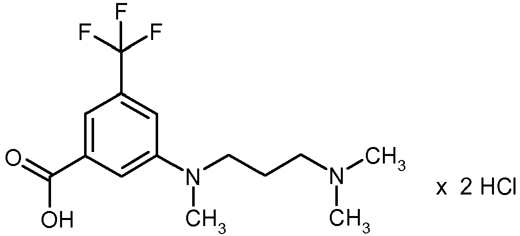

280 mg (0.98 mmol) der Verbindung aus Beispiel 35A / Schritt 1 wurden in 2.5 ml halbkonzentrierter Salzsäure vorgelegt und in einem Mikrowellenofen (Biotage Initiator, mit dynamischer Steuerung der Einstrahlleistung) für 60 min auf 140°C erhitzt. Nach Beendigung der Reaktion wurde der Ansatz eingedampft und der Rückstand im Vakuum getrocknet. Es wurden 370 mg (99% d. Th.) der Titelverbindung erhalten.

### Beispiel 36A

### 3-Formyl-5-(pentafluor-λ⁶-sulfanyl)benzoesäure

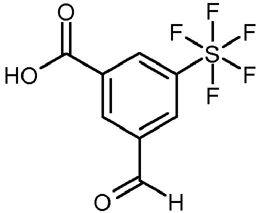

Zu einer Lösung von 3.0 g (9.17 mmol) der Verbindung aus Beispiel 15A in 1.6 ml abs. THF wurden unter Eisbadkühlung 11.5 ml (23 mmol) einer 2 M Lösung von 2-Propylmagnesiumchlorid in Diethylether getropft. Nach Beendigung der Zugabe wurde noch 30 min ohne Kühlung nachgerührt. Anschließend wurde unter Eisbadkühlung mit 1.76 ml (22.9 mmol) DMF versetzt und weitere 30 min ohne Kühlung nachgerührt. Der Ansatz wurde dann mit 20 ml 1 M Salzsäure versetzt und mit 100 ml Ethylacetat extrahiert. Die organische Phase wurde mit 1 M Salzsäure und gesättigter Natriumchlorid-Lösung gewaschen, über Natriumsulfat getrocknet, filtriert und am Rotationsverdampfer zur Trockene eingeengt. Der Rückstand wurde in 50 ml Dichlormethan/ Pentan (1:1) suspendiert und der Feststoff abfiltriert und getrocknet. Noch vorhandene Verunreinigungen wurden mittels präparativer HPLC (Methode 22) entfernt. Man erhielt so 780 mg (31% d. Th.) der Titelverbindung.

¹H-NMR (400 MHz, DMSO-d₆, δ/ppm): 14.06 (br, 1H), 10.17 (s, 1H), 8.66 (s, 1H), 8.64 (s, 1H), 8.52 (s, 1H).

LC/MS (Methode 3, ESIneg): Rₜ = 0.87 min, m/z = 275 [M-H]⁻.

### Beispiel 37A

### 3-(Hydroxymethyl)-5-(pentafluor-λ⁶-sulfanyl)benzoesäure

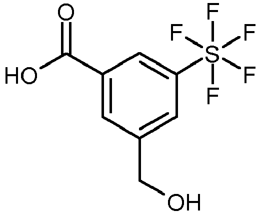

Zu einer Lösung von 90 mg (0.32 mmol) der Verbindung aus Beispiel 36A in 3.2 ml Dichlormethan gab man nacheinander 54 mg (0.49 mmol) 3-Hydroxyazetidin-Hydrochlorid, 97 mg (0.46 mmol) Natriumtriacetoxyborhydrid und 0.13 ml (2.35 mmol) Essigäure hinzu und ließ 1 h bei RT rühren. Der Ansatz wurde dann mit etwas Wasser versetzt und am Rotationsverdampfer eingeengt. Der Rückstand wurde mittels präparativer HPLC (Methode 18) gereinigt. Nach Eindampfen der entsprechenden Fraktionen und Trocknen des Rückstands im Hochvakuum wurden 60 mg (66% d. Th.) der Titelverbindung als Produkt der Umsetzung erhalten.

¹H-NMR (400 MHz, DMSO-d₆, δ/ppm): 13.68 (br, 1H), 8.17 (s, 1H), 8.16 (s, 1H), 8.08 (s, 1H), 5.62 (br, 1H), 4.67 (s, 2H).

LC/MS (Methode 4, ESIneg): Rₜ = 0.78 min, m/z = 277 [M-H]⁻.

### Beispiel 38A

### 3-(Pentafluor-λ⁶-sulfanyl)-5-(pyrrolidin-1-ylmethyl)benzoesäure-Trifluoracetat

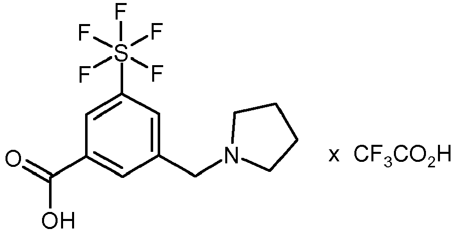

Zu einer Lösung von 50 mg (0.18 mmol) der Verbindung aus Beispiel 36A in 1.8 ml abs. THF gab man 0.03 ml (0.36 mmol) Pyrrolidin und 0.001 ml (0.018 mmol) Essigäure hinzu und ließ 1 h bei RT rühren. Anschließend versetzte man mit 54 mg (0.25 mmol) Natriumtriacetoxyborhydrid und rührte 16 h bei RT nach. Der Ansatz wurde dann mit etwas Wasser versetzt und am Rotationsverdampfer eingeengt. Der Rückstand wurde mittels präparativer HPLC (Methode 18) gereinigt. Nach Eindampfen der Produktfraktionen und Trocknen des Rückstands im Hochvakuum wurden 70 mg (87% d. Th.) der Titelverbindung erhalten.

LC/MS (Methode 4, ESIpos): Rₜ = 0.53 min, m/z = 332 [M+H]⁺.

### Beispiel 39A

### 3-{[(3S)-3-Hydroxypyrrolidin-1-yl]methyl}-5-(pentafluor-λ⁶-sulfanyl)benzoesäure-Trifluoracetat

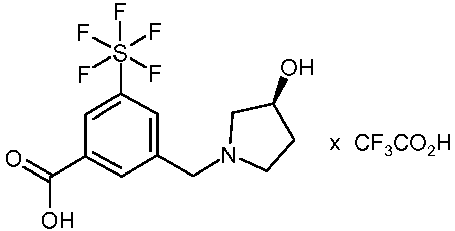

Analog der Vorschrift zu Beispiel 38A wurden aus 100 mg (0.36 mmol) der Verbindung aus Beispiel 36A und 63 mg (0.72 mmol) (*S*)-3-Hydroxypyrrolidin 140 mg (84% d. Th.) der Titelverbindung erhalten.

LC/MS (Methode 4, ESIpos): Rₜ = 0.48 min, m/z = 348 [M+H]⁺.

### Beispiel 40A

### 3-[(4-Methylpiperazin-1-yl)methyl]-5-(pentafluor-λ⁶-sulfanyl)benzoesäure-Trifluoracetat

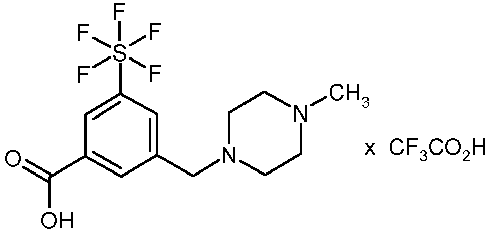

Analog der Vorschrift zu Beispiel 38A wurden aus 150 mg (0.54 mmol) der Verbindung aus Beispiel 36A und 109 mg (1.09 mmol) *N*-Methylpiperazin 205 mg (80% d. Th.) der Titelverbindung erhalten.

¹H-NMR (400 MHz, DMSO-d₆, δ/ppm): 13.80 (br, 1H), 9.52 (br, 1H), 8.21 (s, 1H), 8.20 (s, 1H), 8.11 (s, 1H), 3.79 (s, 2H), 3.38 (m, 2H), 3.06 (m, 2H), 2.94 (m, 2H), 2.79 (m, 3H), 2.37 (m, 2H).

LC/MS (Methode 3, ESIpos): Rₜ = 0.60 min, m/z = 361 [M+H]⁺.

### Beispiel 41A

### 3-Cyano-5-(trifluormethoxy)benzoesäure

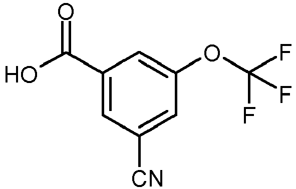

Ein Gemisch von 2.0 g (7.02 mmol) 3-Brom-5-(trifluormethoxy)benzoesäure, 906 mg (7.72 mmol) Zinkcyanid und 486 mg (0.42 mmol) Tetrakis(triphenylphosphin)palladium(0) in 23 ml DMF wurde zunächst entgast und dann unter einer Argonatmosphäre 4 h unter Rückfluss erhitzt. Danach wurden weitere 243 mg (0.21 mmol) Tetrakis(triphenylphosphin)palladium(0) und 453 mg (3.86 mmol) Zinkcyanid zugegeben und das Gemisch 16 h unter Rückfluss nachgerührt. Der Ansatz wurde dann im Vakuum auf ein Volumen von ca. 5 ml eingeengt und in 100 ml 0.1 M Natronlauge eingerührt. Der entstandene Feststoff wurde abfiltriert, das Filtrat mit konzentrierter Salzsäure auf pH 1 gestellt und der entstandene Niederschlag erneut ab filtriert. Das Filtrat wurde zweimal mit je 50 ml *tert.-*Butylmethylether extrahiert, und die vereinigten organischen Phasen wurden mit Wasser und gesättigter Natriumchlorid-Lösung gewaschen, über Natriumsulfat getrocknet, filtriert und im Vakuum eingeengt. Das Produkt wurde mittels präparativer HPLC (Methode 20) isoliert. Nach Eindampfen der Produktfraktionen und Trocknen des Rückstands im Hochvakuum wurden 75 mg (92% Reinheit, 4% d. Th.) der Titelverbindung erhalten.

¹H-NMR (400 MHz, DMSO-d₆, δ/ppm): 13.99 (br, 1H), 8.35 (t, 1H), 8.33 (br, 1H), 8.11 (br, 1H).

LC/MS (Methode 4, ESIneg): Rₜ = 0.85 min, m/z = 230 [M-H]⁻.

### Beispiel 42A

### 3-Cyano-5-(trifluormethyl)benzoesäure

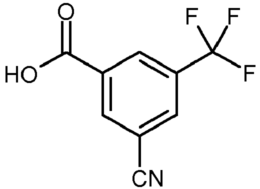

Ein Gemisch von 2.0 g (7.43 mmol) 3-Brom-5-(trifluormethyl)benzoesäure [US 2006/0069261-A1, Verbindung 1.2], 995 mg (8.48 mmol) Zinkcyanid und 859 mg (0.743 mmol) Tetrakis(triphenylphosphin)palladium(0) in 75 ml DMF/Wasser (99:1) wurde zunächst entgast und dann unter einer Argonatmosphäre 3 h auf 120°C erwärmt. Nach dem Abkühlen auf RT wurde mit ca. 300 ml Wasser verdünnt und dreimal mit je ca. 150 ml Ethylacetat extrahiert. Die vereinigten organischen Extrakte wurden mit gesättigter Natriumchlorid-Lösung gewaschen, über wasserfreiem Magnesiumsulfat getrocknet, filtriert und anschließend am Rotationsverdampfer zur Trockene eingeengt. Das Produkt wurde mittels präparativer HPLC (Methode 9) isoliert. Nach Eindampfen der Produktfraktionen und Trocknen des Rückstands im Hochvakuum wurden 421 mg (26% d. Th.) der Titelverbindung erhalten.

¹H-NMR (400 MHz, DMSO-d₆, δ/ppm): 14.03 (br, 1H), 8.66 (t, 1H), 8.60 (t, 1H), 8.42 (t, 1H).

LC/MS (Methode 3, ESIneg): Rₜ = 0.82 min, m/z = 214 [M-H]⁻.

### Beispiel 43A

### 2-tert.-Butyl-6-chlorisonicotinsäure

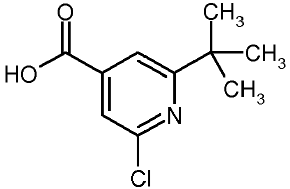

1.0 g (4.39 mmol) 2-*tert*.-Butyl-6-chlorisonicotinsäuremethylester [Lit.: O. Isler *et al.*, *Helvetica Chimica Acta* 1955, *38 (4)*, 1033-1046] wurden in 17 ml THF und 8.8 ml (8.8 mmol) 1 M Natronlauge 30 min unter Rückfluss erhitzt. Nach Erkalten des Ansatzes wurde mit konzentrierter Salzsäure auf pH 1 gestellt und am Rotationsverdampfer fast vollständig eingeengt. Der entstandene Feststoff wurde abfiltriert, mit etwas Wasser gewaschen und getrocknet. Man erhielt 870 mg (93% d. Th.) der Titelverbindung.

LC/MS (Methode 3, ESIpos): Rₜ = 1.00 min, m/z = 214 [M+H]⁺.

### Beispiel 44A

### 2-tert.-Butyl-6-(methylamino)isonicotinsäure

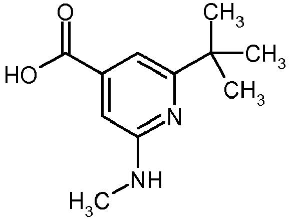

1.0 g (4.39 mmol) 2-*tert*.-Butyl-6-chlorisonicotinsäuremethylester [Lit.: O. Isler et al., Helvetica Chimica Acta 1955, 38 (4), 1033-1046] wurden mit 3.8 ml (43.9 mmol) einer 40%-igen wässrigen Methylamin-Lösung in einem Mikrowellenofen (Biotage Initiator, mit dynamischer Steuerung der Einstrahlleistung) 6 h lang auf 160°C erhitzt. Nach dem Abkühlen wurden die flüchtigen Bestandteile am Rotationsverdampfer abgetrennt. Der Rückstand wurde in 4 ml halbkonzentrierter Salzsäure aufgenommen und erneut in der Mikrowelle für 1 h auf 130°C erhitzt. Das Produkt wurde dann direkt mittels präparativer HPLC (Methode 12) isoliert. Nach Eindampfen der Produktfraktionen und Trocknen des Rückstands im Hochvakuum wurden 180 mg (92% Reinheit, 18% d. Th.) der Titelverbindung erhalten.

LC/MS (Methode 4, ESIpos): Rₜ = 0.39 min, m/z = 209 [M+H]⁺.

### Beispiel 45A

### N-(3-Brom-4-methylphenyl)-3-(trifluormethyl)benzamid

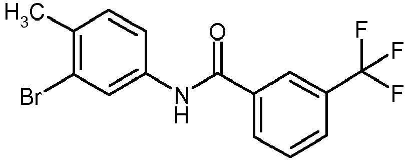

5.51 g (29.6 mmol) 3-Brom-4-methylanilin wurden in 127 ml Dichlormethan gelöst, nacheinander mit 6.18 g (29.6 mmol) 3-(Trifluormethyl)benzoesäurechlorid und 4.54 ml (32.6 mmol) Triethylamin versetzt und 30 min bei RT gerührt. Im Vakuum wurden alle flüchtigen Bestandteile entfernt und der Rückstand in 50 ml Methanol suspendiert. Der Festkörper wurde abfiltriert und das Filtrat in 100 ml 1 M Salzsäure eingerührt. Der entstandene Niederschlag wurde abfiltriert, mit Wasser gewaschen und getrocknet. Man erhielt 7.05 g (90% Reinheit, 60% d. Th.) der Titelverbindung.

¹H-NMR (400 MHz, DMSO-d₆, δ/ppm): 10.52 (s, 1H), 8.29 (s, 1H), 8.26 (d, 1H), 8.10 (d, 1H), 7.98 (d, 1H), 7.80 (t, 1H), 7.68 (dd, 1H), 7.36 (d, 1H), 2.33 (s, 3H).

LC/MS (Methode 1, ESIpos): Rₜ = 1.44 min, m/z = 360 [M+H]⁺.

### Beispiel 46A

### N-(3-Brom-4-methylphenyl)-3-tert.-butylbenzamid

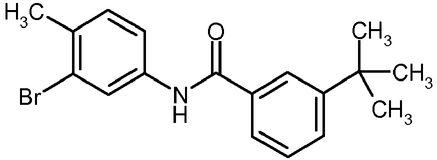

285 mg (1.53 mmol) 3-Brom-4-methylanilin, 300 mg (1.68 mmol) 3-tert.-Butylbenzoesäure und 698 mg (1.83 mmol) HATU wurden in 3 ml DMF gelöst, mit 0.32 ml (1.83 mmol) *N,N-*Diisopropylethylamin versetzt und 16 h bei RT gerührt. Der Ansatz wurde danach in 20 ml 0.1 M Natronlauge eingerührt und mit Ethylacetat extrahiert. Die organische Phase wurde mit Wasser und gesättigter Natriumchlorid-Lösung gewaschen, über Natriumsulfat getrocknet, filtriert und im Vakuum eingeengt. Nach Trocknen des Rückstands wurden 490 mg (88% d. Th.) der Titelverbindung erhalten.

¹H-NMR (400 MHz, DMSO-d₆, δ/ppm): 10.26 (s, 1H), 8.10 (d, 1H), 7.76 (d, 1H), 7.67 (dd, 1H), 7.63 (d, 1H), 7.46 (t, 1H), 7.33 (d, 1H), 2.33 (s, 3H), 1.34 (s, 9H).

LC/MS (Methode 3, ESIpos): Rₜ = 1.35 min, m/z = 347 [M+H]⁺.

### Beispiel 47A

### tert.-Butyl-4-[3-({4-methyl-3-[6-(1H-pyrazol-4-yl)-1H-imidazo[1,2-b]pyrazol-1-yl]phenyl}-carbamoyl)-5-(pentafluor-λ⁶-sulfanyl)phenyl]piperazin-1-carboxylat

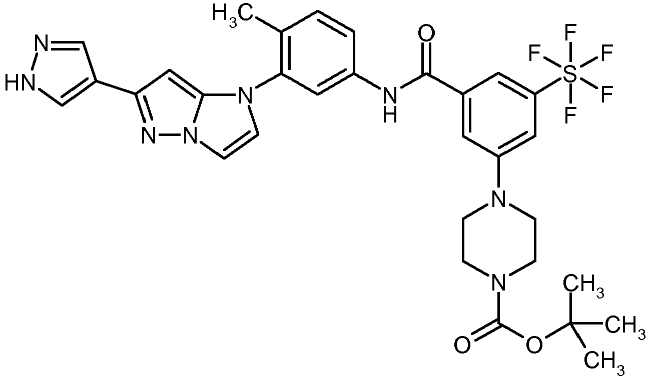

Die Verbindung aus Beispiel 16A (320 mg, 0.74 mmol) wurde in 2.3 ml DMF gelöst, HATU (309 mg, 0.81 mmol) und anschließend 4-Methylmorpholin (0.32 ml) wurden hinzugegeben, und das Gemisch wurde 30 min bei RT gerührt. Anschließend gab man bei -5°C die Verbindung aus Beispiel 7A (103 mg, 0.37 mmol) hinzu und rührte 16 h bei RT nach. Danach wurden einige Milliliter konzentrierte wässrige Ammoniak-Lösung zugesetzt. Das Gemisch wurde 15 min bei RT gerührt und dann mit Ethylacetat extrahiert. Die organische Phase wurde mit konz. Natriumchlorid-Lösung gewaschen, über Natriumsulfat getrocknet, filtriert und eingeengt. Das Rohprodukt wurde über präparative HPLC gereinigt (Methode 28). Es wurden 210 mg (Reinheit 61%, 50% d. Th.) der Titelverbindung erhalten, welche ohne weitere Aufreinigung im nächsten Syntheseschritt eingesetzt wurde.

LC/MS (Methode 3, ESIpos): Rₜ = 1.19 min, m/z = 692 [M+H]⁺.

### Beispiel 48A

### N-(3-{6-[1-(4-Methoxybenzyl)-1H-pyrazol-4-yl]-1H-imidazo[1,2-b]pyrazol-1-yl}-4-methylphenyl)-3-(4-methylpiperazin-1-yl)-5-(pentafluor-λ⁶-sulfanyl)benzamid

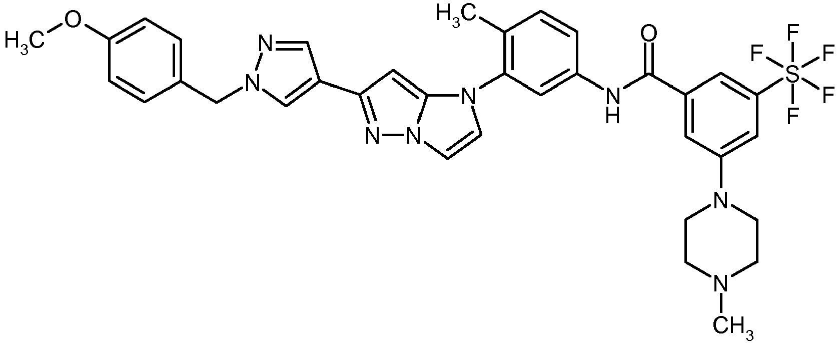

70 mg (0.14 mmol) der Verbindung aus Beispiel 8A, 62.9 mg (0.14 mmol) der Verbindung aus Beispiel 17A und 62 mg (0.16 mmol) HATU wurden in 0.79 ml DMF gelöst, mit 0.07 ml (0.41 mmol) *N,N*-Diisopropylethylamin versetzt und 1 h bei RT gerührt. Anschließend rührte man den Ansatz in 10 ml 0.1 M Natronlauge ein, rührte 10 min bei RT nach und filtrierte den entstandenen Niederschlag ab. Der Feststoff wurde mit Wasser gewaschen und im Hochvakuum getrocknet. Es wurden 94 mg (94% Reinheit, 89% d. Th.) der Titelverbindung erhalten.

¹H-NMR (400 MHz, DMSO-d₆, δ/ppm): 10.52 (s, 1H), 8.05 (s, 1H), 7.90 (d, 1H), 7.77-7.71 (m, 5H), 7.50 (s, 1H), 7.44 (d, 1H), 7.41 (d, 1H), 7.23 (d, 2H), 6.90 (d, 2H), 5.90 (s, 1H), 5.24 (s, 2H), 2.26 (s, 3H), 2.23 (s, 3H) [weitere Signale unter Lösungsmittel-Peaks verborgen].

LC/MS (Methode 4, ESIpos): Rₜ = 0.86 min, m/z = 727 [M+H]⁺.

### Beispiel 49A

### N-(3-{6-[1-(4-Methoxybenzyl)-1H-pyrazol-4-yl]-1H-imidazo[1,2-b]pyrazol-1-yl}-4-methylphenyl)-3-(morpholin-4-yl)-5-(pentafluor-λ⁶-sulfanyl)benzamid

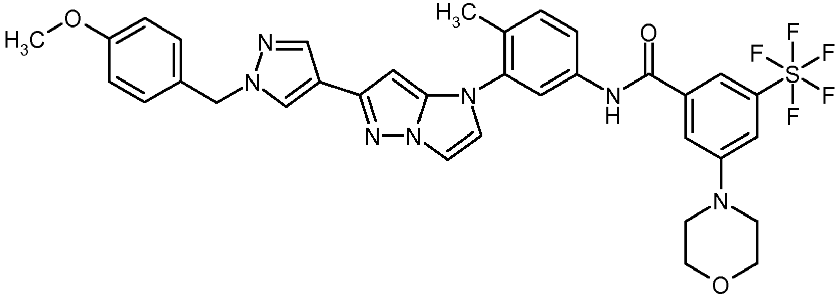

82 mg (0.16 mmol) der Verbindung aus Beispiel 8A, 59 mg (0.18 mmol) der Verbindung aus Beispiel 18A und 73 mg (0.192 mmol) HATU wurden in 1 ml DMF gelöst. Anschließend wurden 53 µl (0.48 mmol) 4-Methylmorpholin hinzugegeben. Nachdem das Reaktionsgemisch 16 h bei RT gerührt worden war, wurde es mit 10 ml 0.1 M Natronlauge versetzt, wobei das Produkt als Feststoff ausfiel. Es wurde noch 10 min nachgerührt, dann wurde das Produkt abgesaugt, mit Wasser gewaschen und im Hochvakuum getrocknet. Es wurden 104 mg (70% d. Th., 77% Reinheit) der Titelverbindung als graubrauner Feststoff erhalten.

LC/MS (Methode 3, ESIpos): Rₜ = 1.21 min, m/z = 714 [M+H]⁺.

### Beispiel 50A

### N-(3-{6-[1-(4-Methoxybenzyl)-1H-pyrazol-4-yl]-1H-imidazo[1,2-b]pyrazol-1-yl}-4-methylphenyl)-3-(trifluormethyl)benzamid

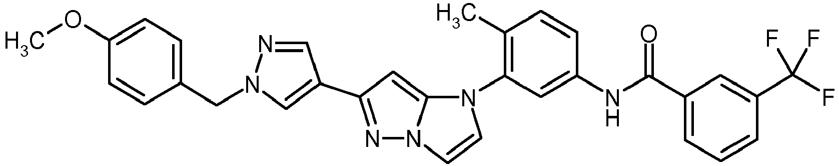

55 mg (0.19 mmol) der Verbindung aus Beispiel 5A und 74 mg (0.21 mmol) der Verbindung aus Beispiel 45A wurden in 1.24 ml DMF und 0.12 ml Wasser gelöst und vorsichtig mit 126 mg (0.39 mmol) Bis(tetraethylammonium)carbonat versetzt. Das Gemisch wurde mit Argon entgast und anschließend mit 14.3 mg (0.08 mmol) Kupfer(I)iodid und 10.9 mg (0.08 mmol) 8-Hydroxychinolin versetzt. Der Ansatz wurde dann in einem Mikrowellenofen (Biotage Initiator, mit dynamischer Steuerung der Einstrahlleistung) 1 h lang auf 160°C erhitzt. Nach dem Abkühlen wurde das Gemisch in 10 ml Wasser gegossen und mit Ethylacetat extrahiert. Die organische Phase wurde mit gesättigter Natriumchlorid-Lösung gewaschen und im Vakuum eingeengt. Der Rückstand wurde mittels präparativer HPLC (Methode 11) gereinigt. Die Produktfraktionen wurden eingeengt und der Rückstand im Hochvakuum getrocknet. Es wurden 26 mg (57% Reinheit, 14% d. Th.) der Titelverbindung erhalten, welche in dieser Form weiter umgesetzt wurde.

LC/MS (Methode 3, ESIpos): Rₜ = 1.14 min, m/z = 571 [M+H]⁺.

### Beispiel 51A

### N-(3-{6-[1-(4-Methoxybenzyl)-1H-pyrazol-4-yl]-1H-imidazo[1,2-b]pyrazol-1-yl}-4-methylphenyl)-3-(2-methyl-1H-imidazol-1-yl)-5-(trifluormethyl)benzamid

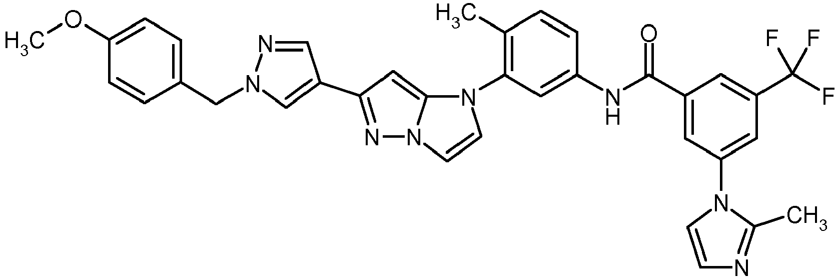

60 mg (0.12 mmol) der Verbindung aus Beispiel 8A und 31.6 mg (0.12 mmol) 3-(2-Methyl-1*H-*imidazol-1-yl)-5-(trifluormethyl)benzoesäure [Lit.: WO 2004/005281-A1, Beispiel 91b] wurden analog der Vorschrift zu Beispiel 48A umgesetzt und aufgearbeitet. Es wurden 61 mg (77% d. Th.) der Titelverbindung erhalten.

LC/MS (Methode 4, ESIpos): Rₜ = 0.83 min, m/z = 651 [M+H]⁺.

### Beispiel 52A

### 3-tert.-Butyl-N-(3-{6-[1-(4-methoxybenzyl)-1H-pyrazol-4-yl]-1H-imidazo[1,2-b]pyrazol-1-yl}-4-methylphenyl)benzamid

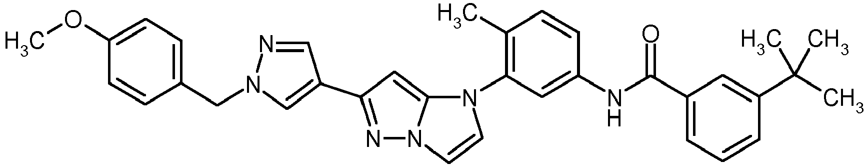

### Schritt 1: 3-tert.-Butyl-N-(3-{[1-(2,2-diethoxyethyl)-1'-(4-methoxybenzyl)-1H,1'H-3,4'-bipyrazol-5-yl]amino}-4-methylphenyl)benzamid

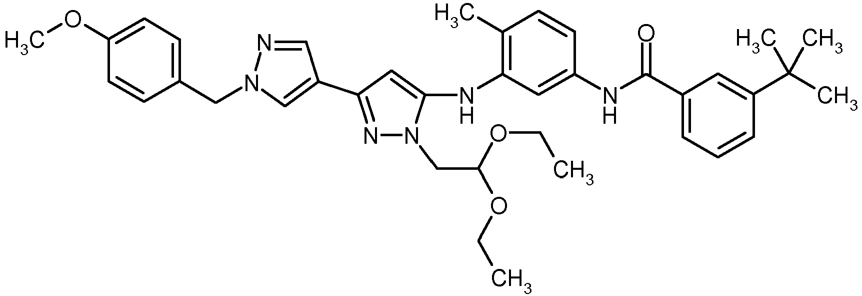

200 mg (0.44 mmol, Reinheit 85%) der Verbindung aus Beispiel 2A und 199 mg (0.57 mmol) der Verbindung aus Beispiel 46A in 2.18 ml 1,4-Dioxan wurden mit Argon entgast. Man fügte 9.9 mg (0.044 mmol) Palladium(II)acetat, 38.3 mg (0.066 mmol) Xantphos und 431 mg (1.32 mmol) Cäsiumcarbonat hinzu und erhitzte das Gemisch in einem Mikrowellenofen (Biotage Initiator, mit dynamischer Steuerung der Einstrahlleistung) für 30 min auf 150°C. Der Ansatz wurde danach über Kieselgur filtriert und das Filtrat eingeengt. Der Rückstand wurde über Kieselgel flashchromatographiert (Laufmittel Dichlormethan/Ethylacetat 1:1). Die Produktfraktionen wurden im Vakuum eingeengt und der Rückstand getrocknet. Es wurden 180 mg (63% d. Th.) der Titelverbindung erhalten.

¹H-NMR (400 MHz, DMSO-d₆, δ/ppm): 10.04 (s, 1H), 8.03 (s, 1H), 7.84 (s, 1H), 7.71 (s, 1H), 7.69 (d, 1H), 7.58 (d, 1H), 7.40 (t, 1H), 7.28-7.22 (m, 4H), 7.09 (d, 1H), 6.96 (s, 1H), 6.89 (d, 2H), 6.11 (s, 1H), 5.23 (s, 2H), 4.82 (t, 1H), 4.06 (d, 2H), 3.63 (m, 2H), 3.45 (m, 2H), 2.21 (s, 3H), 1.31 (s, 9H), 1.06 (t, 6H).

LC/MS (Methode 3, ESIpos): Rₜ = 1.38 min, m/z = 651 [M+H]⁺.

### Schritt 2: 3-tert.-Butyl-N-(3-{6-[1-(4-methoxybenzyl)-1H-pyrazol-4-yl]-1H-imidazo[1,2-b]-pyrazol-1-yl}-4-methylphenyl)benzamid

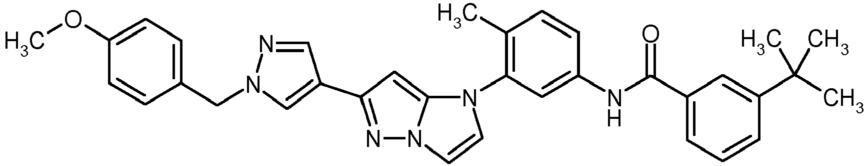

150 mg (0.23 mmol) der Verbindung aus Beispiel 52A / Schritt 1 in 1.2 ml Ethanol und 58 µl (0.12 mmol) 2 M Schwefelsäure wurden in einem Mikrowellenofen (Biotage Initiator, mit dynamischer Steuerung der Einstrahlleistung) für 30 min auf 120°C erhitzt. Der Ansatz wurde danach im Vakuum eingeengt und der Rückstand getrocknet. Man erhielt so 140 mg (78% Reinheit, 85% d. Th.) der Titelverbindung, welche ohne weitere Reinigung in Folgereaktionen eingesetzt wurde.

LC/MS (Methode 4, ESIpos): Rₜ = 1.27 min, m/z = 559 [M+H]⁺.

### Beispiel 53A

### 3-tert.-Butyl-N-(3-{6-[1-(4-methoxybenzyl)-1H-pyrazol-4-yl]-1H-imidazo[1,2-b]pyrazol-1-yl}-4-methylphenyl)-5-(pyrrolidin-1-ylmethyl)benzamid

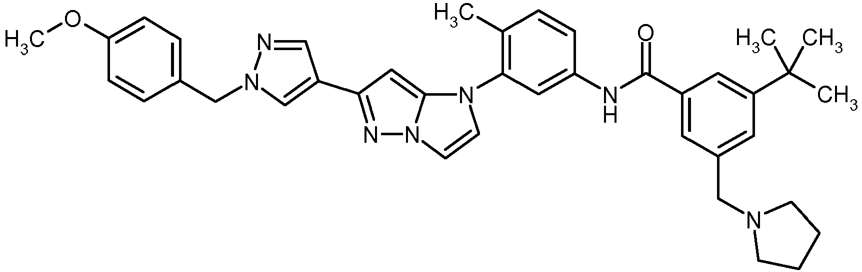

70 mg (0.14 mmol) der Verbindung aus Beispiel 8A und 51 mg (0.14 mmol) der Verbindung aus Beispiel 22A wurden analog der Vorschrift zu Beispiel 48A umgesetzt. Es wurden 83 mg (88% Reinheit, 83% d. Th.) der Titelverbindung erhalten.

LC/MS (Methode 3, ESIpos): Rₜ = 0.91 min, m/z = 642 [M+H]⁺.

### Beispiel 54A

### N-(3-{6-[1-(4-Methoxybenzyl)-1H-pyrazol-4-yl]-3-methyl-1H-imidazo[1,2-b]pyrazol-1-yl}-4-methylphenyl)-3-(4-methylpiperazin-1-yl)-5-(pentafluor-λ⁶-sulfanyl)benzamid

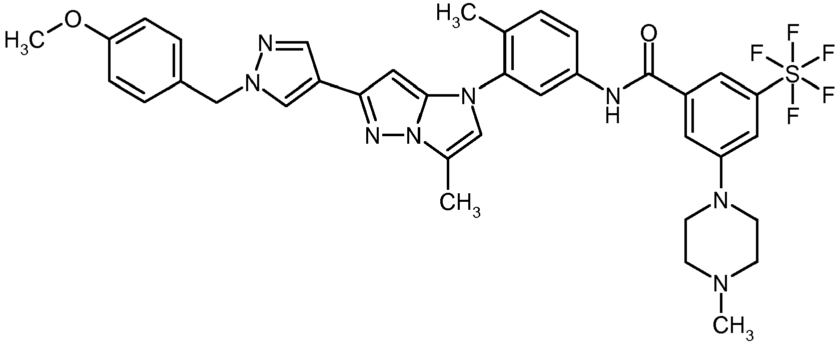

70 mg (0.17 mmol) der Verbindung aus Beispiel 9A und 78 mg (0.17 mmol) der Verbindung aus Beispiel 17A wurden analog der Vorschrift zu Beispiel 48A umgesetzt und aufgearbeitet. Es wurden 125 mg (95% d. Th.) der Titelverbindung erhalten.

¹H-NMR (400 MHz, DMSO-d₆, δ/ppm): 10.50 (s, 1H), 8.08 (s, 1H), 7.87 (d, 1H), 7.76 (s, 1H), 7.71 (m, 3H), 7.50 (s, 1H), 7.42 (d, 1H), 7.25 (d, 2H), 7.15 (d, 1H), 6.91 (d, 2H), 5.91 (s, 1H), 5.23 (s, 2H), 3.73 (s, 3H), 2.34 (s, 3H), 2.27 (s, 3H), 2.23 (s, 3H) [weitere Signale unter Lösungsmittel-Peaks verborgen].

LC/MS (Methode 3, ESIpos): Rₜ = 0.97 min, m/z = 741 [M+H]⁺.

### Beispiel 55A

### 3-Formyl-N-{4-methyl-3-[6-(pyridin-3-yl)-1H-imidazo[1,2-b]pyrazol-1-yl]phenyl}-5-(pentafluor-λ⁶-sulfanyl)benzamid

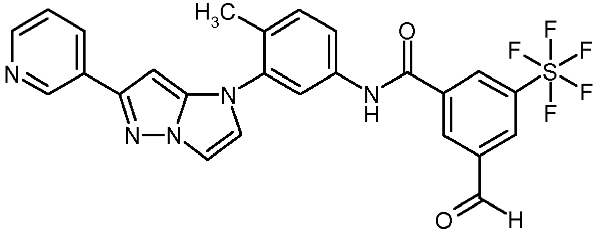

320 mg (1.11 mmol) der Verbindung aus Beispiel 6A, 336 mg (1.22 mmol) der Verbindung aus Beispiel 36A und 504 mg (1.33 mmol) HATU wurden in 3.3 ml DMF gelöst, mit 0.23 ml (1.33 mmol) *N*,*N*-Diisopropylethylamin versetzt und 3 h bei RT gerührt. Anschließend wurde der Ansatz in 30 ml halbkonzentrierte wässrige Natriumhydrogencarboant-Lösung eingerührt. Nach 10 min Nachrühren bei RT wurde der entstandene Niederschlag abfiltriert, mit Wasser gewaschen und getrocknet. Das so erhaltene Rohprodukt wurde mittels präparativer HPLC (Methode 12) gereinigt.

Die Produktfraktionen wurden eingeengt, der Rückstand in Ethylacetat gelöst und nacheinander mit gesättigter Natriumhydrogencarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden 100 mg (16% d. Th.) der Titelverbindung erhalten.

¹H-NMR (400 MHz, DMSO-d₆, δ/ppm): 10.88 (s, 1H), 10.18 (s, 1H), 9.05 (d, 1H), 8.77 (s, 1H), 8.71 (s, 1H), 8.66 (s, 1H), 8.48 (dd, 1H), 8.19 (dt, 1H), 7.96 (d, 1H), 7.93 (d, 1H), 7.82 (m, 1H), 7.56 (d, 1H), 7.50 (d, 1H), 7.42 (dd, 1H), 6.38 (s, 1H), 2.29 (s, 3H).

LC/MS (Methode 3, ESIpos): Rₜ = 0.98 min, m/z = 548 [M+H]⁺.

### Beispiel 56A

### 3-Cyano-N-(3-{6-[1-(4-methoxybenzyl)-1H-pyrazol-4-yl]-3-methyl-1H-imidazo[1,2-b]pyrazol-1-yl}-4-methylphenyl)-5-(pentafluor-λ⁶-sulfanyl)benzamid

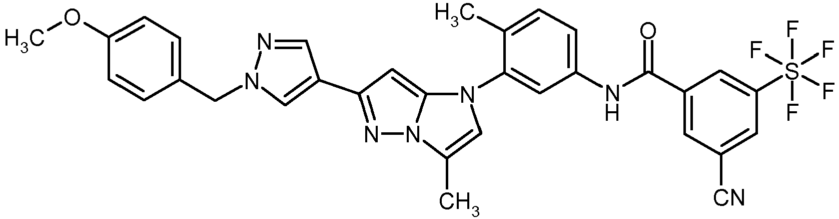

100 mg (0.24 mmol) der Verbindung aus Beispiel 9A und 66 mg (0.24 mmol) der Verbindung aus Beispiel 23A wurden analog der Vorschrift zu Beispiel 48A umgesetzt und aufgearbeitet. Es wurden 153 mg (77% Reinheit, 73% d. Th.) der Titelverbindung erhalten.

LC/MS (Methode 1, ESIpos): Rₜ = 2.66 min, m/z = 576 [M+H]⁺.

### Beispiel 57A

### 3-tert.-Butyl-N-(3-{6-[1-(4-methoxybenzyl)-1H-pyrazol-4-yl]-1H-imidazo[1,2-b]pyrazol-1-yl}-4-methylphenyl)-5-(4-methylpiperazin-1-yl)benzamid

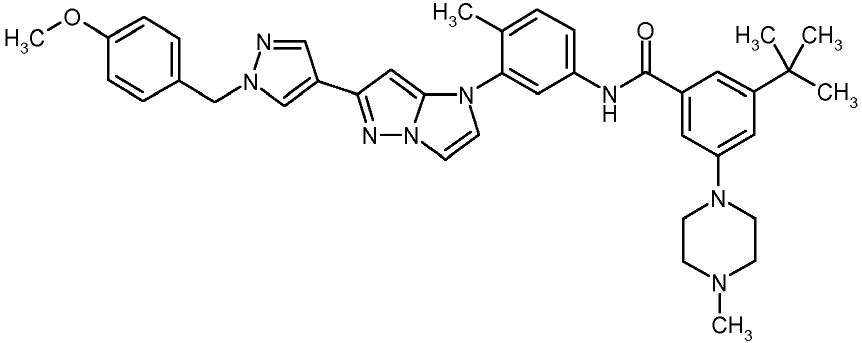

65 mg (0.13 mmol) der Verbindung aus Beispiel 8A, 50 mg (0.13 mmol) der Verbindung aus Beispiel 21A und 58 mg (0.15 mmol) HATU wurden in 0.73 ml DMF gelöst, mit 0.07 ml (0.63 mmol) 4-Methylmorpholin versetzt und 16 h bei RT gerührt. Man versetzte dann mit 10 ml 0.1 M Natronlauge und rührte noch 10 min bei RT nach. Der entstandene Niederschlag wurde abfiltriert, mit Wasser gewaschen und im Hochvakuum getrocknet. Es wurden 70 mg (76% Reinheit, 64% d. Th.) der Titelverbindung erhalten.

LC/MS (Methode 1, ESIpos): Rₜ = 1.16 min, m/z = 657 [M+H]⁺.

### Beispiel 58A

### tert.-Butyl-3-hydroxy-3-[3-({4-methyl-3-[6-(pyridin-3-yl)-1H-imidazo[1,2-b]pyrazol-1-yl]phenyl}-carbamoyl)-5-(pentafluor-λ⁶-sulfanyl)phenyl]azetidin-1-carboxylat

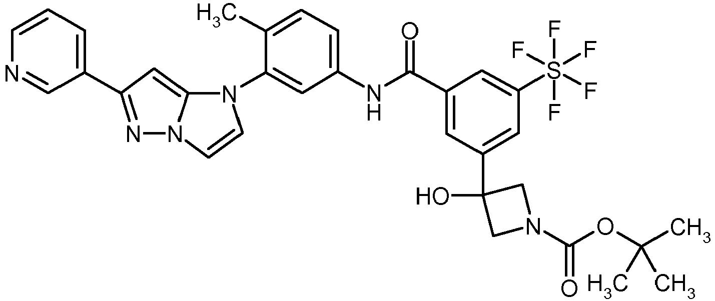

64 mg (0.15 mmol) der Verbindung aus Beispiel 20A und 70 mg (0.18 mmol) HATU wurden in 0.88 ml DMF vorgelegt, mit 0.13 ml (1.22 mmol) 4-Methylmorpholin versetzt und 30 min bei RT gerührt. Bei -5°C wurden 44 mg (0.15 mmol) der Verbindung aus Beispiel 6A zugegeben und das Gemisch dann 16 h bei RT gerührt. Der Ansatz wurde danach mit Wasser und 2 M Natronlauge versetzt und 15 min bei RT gerührt. Der entstandene Niederschlag wurde abfiltriert, mit Wasser gewaschen und getrocknet. Es wurden 67 mg (93% Reinheit, 60% d. Th.) der Titelverbindung erhalten.

LC/MS (Methode 3, ESIpos): Rₜ = 1.07 min, m/z = 691 [M+H]⁺.

### Beispiel 59A

### 1-(2,2-Diethoxyethyl)-3-(pyrazin-2-yl)-1H-pyrazol-5-amin

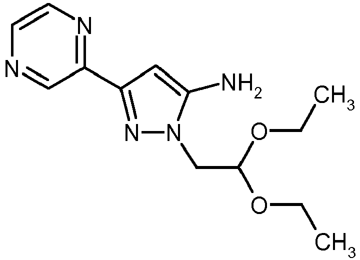

### Schritt 1: Natrium-2-cyano-1-(pyrazin-2-yl)ethenolat

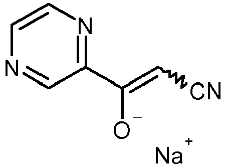

Zu einer Suspension von 434 mg Natriumhydrid (60%-ige Suspension in Mineralöl) in 10 ml THF, die unter Rückfluss erhitzt wurde, wurde eine Lösung von 1.5 g (10.9 mmol) Pyrazin-2-carbonsäuremethylester und 446 mg (10.9 mmol) Acetonitril in 16.5 ml THF getropft. Das Reaktionsgemisch wurde 20 h unter Rückfluss erhitzt. Nach dem Abkühlen wurden 50 ml Methyl-*tert.*-butylether hinzugegeben und das Gemisch für 30 Minuten gerührt. Der dabei entstandene Niederschlag wurde über eine Fritte abgesaugt und im Ölpumpenvakuum getrocknet. Man erhielt auf diese Weise 1.77 g (96% d. Th.) der Titelverbindung, welche ohne weitere Charakterisierung in der Folgestufe eingesetzt wurde.

### Schritt 2: 1-(2,2-Diethoxyethyl)-3-(pyrazin-2-yl)-1H-pyrazol-5-amin

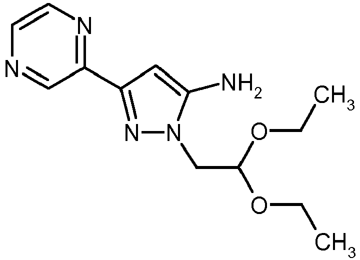

1.76 g (10.4 mmol) des Natrium-Salzes aus Beispiel 59A / Schritt 1 wurden in 10.5 ml Ethanol suspendiert und nacheinander mit 1.62 g (10.9 mmol) (2,2-Diethoxyethyl)hydrazin, 0.6 ml (10.4 mmol) Essigsäure und 52 µl 1 M Salzsäure versetzt. Nach zweistündigem Erhitzen unter Rückfluss wurde die Reaktionsmischung auf RT abgekühlt und mit 200 ml Ethylacetat verdünnt. Die organische Phase wurde jeweils einmal mit je 30 ml gesättigter Natriumhydrogencarbonat-Lösung, Wasser und gesättigter Natriumchlorid-Lösung gewaschen, über Natriumsulfat getrocknet und nach Filtration im Vakuum eingeengt. Das so erhaltene Rohprodukt wurde über eine Biotage-Anlage gereinigt (50 g Snap-Säule; Laufmittelgradient Ethylacetat/0-10% Methanol). Man erhielt auf diese Weise 1.17 g (40% d. Th.) der Titelverbindung.

¹H-NMR (400 MHz, DMSO-d₆, δ/ppm): 9.00 (d, 1H), 8.53 (dd, 1H), 8.43 (d, 1H), 5.87 (s, 1H), 5.31 (s, 2H), 4.84 (t, 1H), 4.02 (d, 2H), 3.63 (dq, 2H), 3.41 (dq, 2H), 1.04 (t, 6H).

LC/MS (Methode 7, ESIpos): Rₜ = 0.79 min, m/z = 278 [M+H]⁺.

### Beispiel 60A

### 3-{6-[1-(4-Methoxybenzyl)-1H-pyrazol-4-yl]-1H-imidazo[1,2-b]pyrazol-1-yl}-4-methylanilin

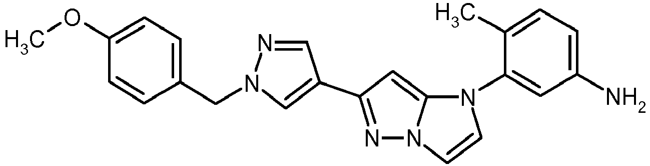

490 mg (1.14 mmol) der Verbindung aus Beispiel 8A / Schritt 2 wurden in Analogie zu Beispiel 9A / Schritt 3 umgesetzt und aufgearbeitet. Der Ansatz wurde in diesem Fall 30 min (statt 1.5 h) unter Rückfluss erhitzt. Es wurden 436 mg (77% d. Th., 80% Reinheit) der Titelverbindung erhalten.

LC/MS (Methode 4, ESIpos): Rₜ = 0.89 min, m/z = 399 [M+H]⁺.

### Beispiel 61A

### 5-{6-[1-(4-Methoxybenzyl)-1H-pyrazol-4-yl]-1H-imidazo[1,2-b]pyrazol-1-yl}-2,4-dimethylanilin

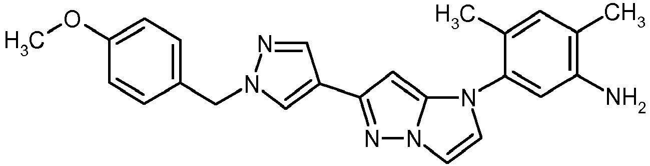

### Schritt 1: 1-(2,2-Diethoxyethyl)-N-(2,4-dimethyl-5-nitrophenyl)-1'-(4-methoxybenzyl)-1H,1'H-3,4'-bipyrazol-5-amin

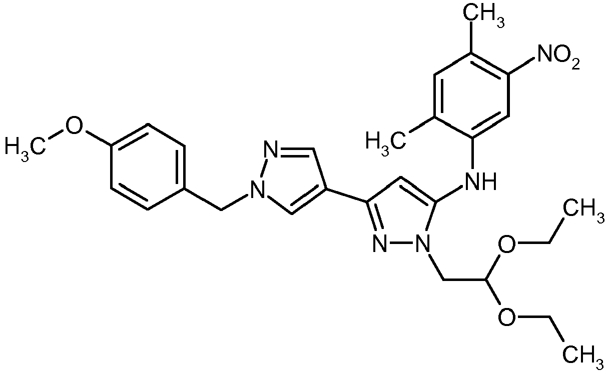

980 mg (2.64 mmol) der Verbindung aus Beispiel 2A wurden in Analogie zu Beispiel 9A umgesetzt. Abweichend von der dort beschriebenen Aufarbeitung wurde der Ansatz hier über Kieselgur filtriert, das Filtrat im Vakuum eingeengt und der Rückstand mittels präparativer HPLC (Methode 35) in seine Bestandteile aufgetrennt. Man erhielt 1.12 g (66% d. Th.) der Titelverbindung.

¹H-NMR (400 MHz, DMSO-d₆, δ/ppm): 8.06 (s, 1H), 7.73 (s, 1H), 7.33 (d, 2H), 7.25 (m, 3H), 6.91 (d, 2H), 6.24 (s, 1H), 5.24 (s, 2H), 4.81 (t, 1H), 4.06 (d, 2H), 3.73 (s, 3H), 3.58 (m, 2H), 3.41 (m, 2H), 2.42 (s, 3H), 2.29 (s, 3H), 1.00 (t, 6H).

LC/MS (Methode 2, ESIpos): Rₜ = 2.68 min, m/z = 535 [M+H]⁺.

### Schritt 2: 1-(2,4-Dimethyl-5-nitrophenyl)-6-[1-(4-methoxybenzyl)-1H-pyrazol-4-yl]-1H-imidazo-[1,2-b]pyrazol

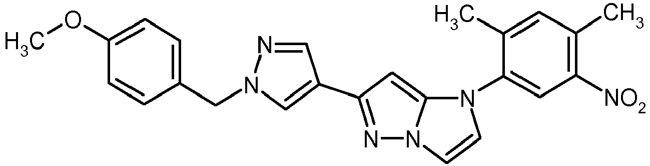

1.12 g (2.10 mmol) der Verbindung aus Beispiel 61A / Schritt 1 wurden in 21 ml Ethanol unter Zusatz von 2.5 ml (5.03 mmol) 2 M Schwefelsäure 16 h unter Rückfluss erhitzt. Nach Erkalten des Ansatzes wurde der entstandene Niederschlag abfiltriert, mit Ethanol gewaschen und getrocknet. Der Filterkuchen wurde in 20 ml Ethylacetat aufgenommen, und die Lösung wurde mit je 10 ml gesättigter wässriger Kaliumcarbonat-Lösung und gesättigter wässriger Natriumchlorid-Lösung gewaschen und über Natriumsulfat getrocknet. Nach Filtration wurde das Filtrat am Rotationsverdampfer vom Lösungsmittel befreit und der Rückstand im Hochvakuum getrocknet. Es wurden 410 mg (44% d. Th.) der Titelverbindung erhalten.

¹H-NMR (400 MHz, DMSO-d₆, δ/ppm): 8.05 (d, 2H), 7.79 (d, 1H), 7.74 (s, 1H), 7.62 (s, 1H), 7.47 (d, 1H), 7.23 (d, 2H), 6.90 (d, 2H), 5.96 (s, 1H), 5.25 (s, 2H), 3.73 (s, 3H), 2.58 (s, 3H), 2.33 (s, 3H).

LC/MS (Methode 3, ESIpos): Rₜ = 1.06 min, m/z = 443 [M+H]⁺.

### Schritt 3: 5-{6-[1-(4-Methoxybenzyl)-1H-pyrazol-4-yl]-1H-imidazo[1,2-b]pyrazol-1-yl}-2,4-dimethylanilin

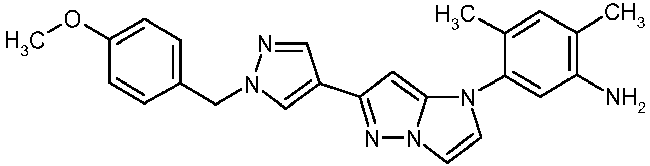

200 mg (0.45 mmol) der Verbindung aus Beispiel 61A / Schritt 2 wurden in Analogie zu Beispiel 61A umgesetzt. Nach Reaktionsende wurde der Ansatz filtriert, das Filtrat im Vakuum eingeengt und der Rückstand mittels präparativer HPLC (Methode 21) in seine Bestandteile aufgetrennt. Die produktenthaltenden Fraktionen wurden vereinigt, im Vakuum fast vollständig eingeengt und mit etwas gesättigter wässriger Natriumhydrogencarbonat-Lösung alkalisch gestellt. Der dabei entstandene Niederschlag wurde abfiltriert, mit Wasser gewaschen und im Hochvakuum getrocknet. Es wurden 120 mg (64% d. Th.) der Titelverbindung erhalten.

¹H-NMR (400 MHz, DMSO-d₆, δ/ppm): 8.03 (s, 1H), 7.73 (s, 1H), 7.66 (d, 1H), 7.26-7.20 (m, 3H), 6.94-6.88 (m, 3H), 6.63 (s, 1H), 5.78 (s, 1H), 5.24 (s, 2H), 4.96 (s, 2H), 3.72 (s, 3H), 2.07 (s, 3H), 2.03 (s, 3H).

LC/MS (Methode 3, ESIpos): Rₜ = 0.94 min, m/z = 413 [M+H]⁺.

### Beispiel 62A

### 3-[6-(Pyridin-3-yl)-1H-imidazo[1,2-b]pyrazol-1-yl]anilin

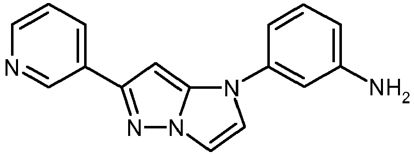

### Schritt 1: 1-(2,2-Diethoxyethyl)-N-(3-nitrophenyl)-3-(pyridin-3-yl)-1H-pyrazol-5-amin

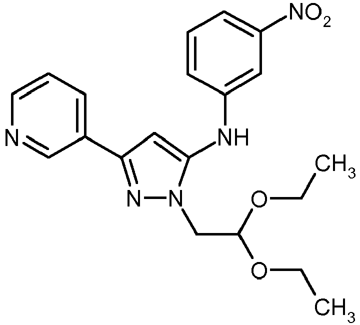

Analog zu dem unter Beispiel 6A / Schritt 1 beschriebenen Verfahren wurden aus 1.0 g (3.62 mmol) der Verbindung aus Beispiel 4A und 804 mg (3.98 mmol) 1-Brom-3-nitrobenzol 1.26 g (87% d. Th.) der Titelverbindung erhalten. Die chromatographische Isolierung des Produktes erfolgte in diesem Fall mittels MPLC (Laufmittel Cyclohexan/Ethylacetat 1:2); auf ein anschließendes Verrühren mit Diisopropylether wurde verzichtet.

¹H-NMR (400 MHz, CDCl₃, δ/ppm): 9.02 (d, 1H), 8.57 (dd, 1H), 8.08 (dt, 1H), 7.80 (t, 1H), 7.74 (dd, 1H), 7.42 (t, 1H), 7.34 (dd, 1H), 7.28-7.25 (m, 2H, teilweise überdeckt vom CHCl₃-Signal), 6.47 (s, 1H), 4.82 (t, 1H), 4.30 (d, 2H), 3.90-3.83 (m, 2H), 3.64-3.56 (m, 2H), 1.28 (t, 6H).

LC/MS (Methode 3, ESIpos): Rₜ = 0.93 min, m/z = 398 [M+H]⁺.

### Schritt 2: 1-(3-Nitrophenyl)-6-(pyridin-3-yl)-1H-imidazo[1,2-b]pyrazol

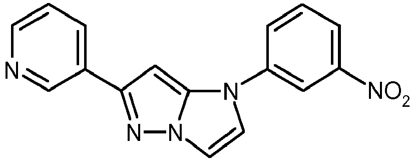

Eine Lösung von 1.25 g (3.14 mmol) der Verbindung aus Beispiel 62A / Schritt 1 in 12.5 ml Ethanol wurde mit 3.8 ml (7.55 mmol) 2 M Schwefelsäure versetzt und anschließend 15 Minuten in einem Mikrowellenofen (Biotage Initiator, mit dynamischer Steuerung der Einstrahlleistung) auf 130°C erhitzt. Nach dem Abkühlen auf RT wurde das Reaktionsgemisch unter Rühren in ca. 25 ml gesättigte wässrige Kaliumcarbonat-Lösung gegeben. Diese Mischung wurde zweimal mit je ca. 50 ml Ethylacetat extrahiert und die vereinigten organischen Phasen mit gesättigter Natriumchlorid-Lösung gewaschen. Nach Trocknen über wasserfreiem Magnesiumsulfat und Filtration wurde im Vakuum eingeengt und der Rückstand anschließend im Hochvakuum getrocknet. Es wurden 874 mg (82% d. Th., 91% Reinheit) der Titelverbindung erhalten.

¹H-NMR (400 MHz, DMSO-d₆, δ/ppm): 9.13 (d, 1H), 8.53 (dd, 1H), 8.50 (t, 1H), 8.26 (dt, 1H), 8.22-8.19 (m, 2H), 8.12 (dd, 1H), 8.06 (d, 1H), 7.86 (t, 1H), 7.48 (dd, 1H), 7.07 (s, 1H).

LC/MS (Methode 2, ESIpos): Rₜ = 1.72 min, m/z = 306 [M+H]⁺.

### Schritt 3: 3-[6-(Pyridin-3-yl)-1H-imidazo[1,2-b]pyrazol-1-yl]anilin

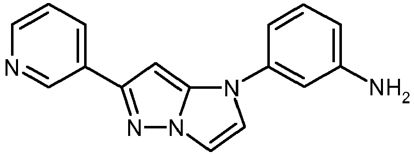

Analog zu dem unter Beispiel 9A / Schritt 3 beschriebenen Verfahren wurden aus 855 mg (2.80 mmol) der Verbindung aus Beispiel 62A / Schritt 2 760 mg (98% d. Th.) der Titelverbindung erhalten. Die Reaktionszeit betrug in diesem Fall 30 Minuten.

¹H-NMR (400 MHz, DMSO-d₆, δ/ppm): 9.09 (d, 1H), 8.51 (dd, 1H), 8.21 (dt, 1H), 7.91 (d, 1H), 7.80 (d, 1H), 7.46 (dd, 1H), 7.17 (t, 1H), 6.92 (dd, 1H), 6.83 (dd, 1H), 6.80 (s, 1H), 6.49 (dd, 1H), 5.41 (s, breit, 2H).

LC/MS (Methode 3, ESIpos): Rₜ = 0.57 min, m/z = 276 [M+H]⁺.

### Beispiel 63A

### 3-[6-(Pyridin-3-yl)-1H-imidazo[1,2-b]pyrazol-1-yl]-4-(trifluormethyl)anilin

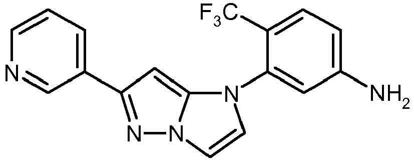

### Schritt 1: 1-(2,2-Diethoxyethyl)-N-[5-nitro-2-(trifluormethyl)phenyl]-3-(pyridin-3-yl)-1H-pyrazol-5-amin

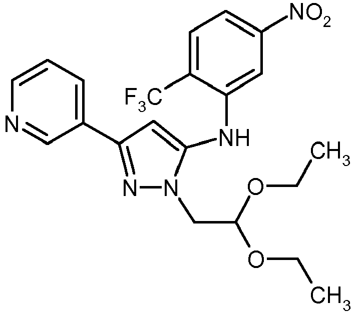

In Analogie zu Beispiel 13A / Schritt 1 wurden aus 1.50 g (5.43 mmol) der Verbindung aus Beispiel 4A und 1.47 g (5.43 mmol) 2-Brom-4-nitro-1-(trifluormethyl)benzol 2.03 g (75% d. Th.) der Titelverbindung erhalten.

¹H-NMR (400 MHz, DMSO-d₆, δ/ppm): 9.11 (s, 1H), 9.00 (d, 1H), 8.49 (dd, 1H), 8.14 (dt, 1H), 7.77 (d, 1H), 7.39-7.45 (m, 2H), 7.24 (dd, 1H), 6.86 (s, 1H), 4.84 (t, 1H), 4.15 (d, 2H), 3.56 (dq, 2H), 3.36 (dq, 2H), 0.94 (t, 6H).

LC/MS (Methode 7, ESIpos): Rₜ = 1.24 min, m/z = 466 [M+H]⁺.

### Schritt 2: 1-[5-Nitro-2-(trifluormethyl)phenyl]-6-(pyridin-3-yl)-1H-imidazo[1,2-b]pyrazol

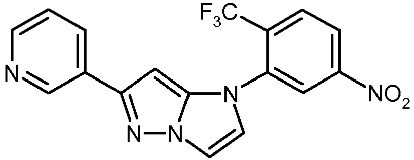

In Analogie zu Beispiel 13A / Schritt 2 wurde aus 2.03 g (4.36 mmol) der Verbindung aus Beispiel 63A / Schritt 1 ein Rohprodukt erhalten, welches durch einmalige Chromatographie über eine Biotage-Anlage gereinigt wurde (50 g Snap-Säule; Laufmittelgradient Dichlormethan/Methanol, von 2% Methanol bis 8% Methanol stetig ansteigend). Man erhielt auf diese Weise 1.17 g (65% d. Th.) der Titelverbindung.

¹H-NMR (400 MHz, DMSO-d₆, δ/ppm): 9.08 (dd, 1H), 8.51 (dd, 1H), 8.38 (d, 1H), 8.20 (dt, 1H), 8.14-8.18 (m, 2H), 8.11 (d, 1H), 8.08 (d, 1H), 7.45 (ddd, 1H), 7.20 (s, 1H).

LC/MS (Methode 7, ESIpos): Rₜ = 1.05 min, m/z = 374 [M+H]⁺.

### Schritt 3: 3-[6-(Pyridin-3-yl)-1H-imidazo[1,2-b]pyrazol-1-yl]-4-(trifluormethyl)anilin

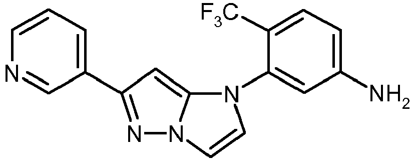

In Analogie zu Beispiel 13A / Schritt 3 wurden aus 1.17 g (3.13 mmol) der Verbindung aus Beispiel 63A / Schritt 2 866 mg (78% d. Th.) der Titelverbindung erhalten.

¹H-NMR (400 MHz, DMSO-d₆, δ/ppm): 9.05 (dd, 1H), 8.50 (dd, 1H), 8.17 (dt, 1H), 7.95 (d, 1H), 7.87 (d, 1H), 7.41-7.48 (m, 2H), 7.16 (d, 1H), 6.96 (dd, 1H), 6.89 (d, 1H), 5.84 (s, 2H).

LC/MS (Methode 7, ESIpos): Rₜ = 0.93 min, m/z = 344 [M+H]⁺.

### Beispiel 64A

### 4-Chlor-3-[6-(pyridin-3-yl)-1H-imidazo[1,2-b]pyrazol-1-yl]anilin

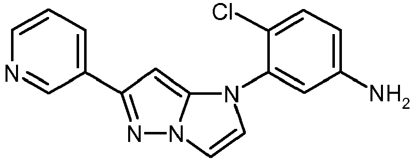

### Schritt 1: N-(2-Chlor-5-nitrophenyl)-1-(2,2-diethoxyethyl)-3-(pyridin-3-yl)-1H-pyrazol-5-amin

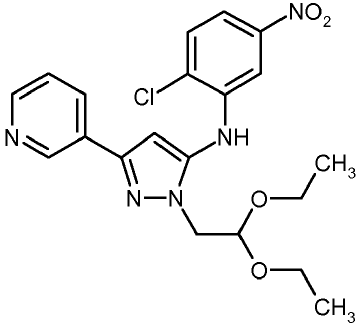

Analog zu dem unter Beispiel 9A / Schritt 1 beschriebenen Verfahren wurden aus 1.0 g (3.62 mmol) der Verbindung aus Beispiel 4A und 941 mg (3.98 mmol) 1-Brom-2-chlor-5-nitrobenzol 1.26 g (80% d. Th.) der Titelverbindung erhalten. Die Reaktionszeit in der Mikrowelle betrug in diesem Fall 1 h bei einer Temperatur von 140°C. Die chromatographische Isolierung des Produktes erfolgte mittels MPLC (Laufmittel Cyclohexan/Ethylacetat 1:2).

¹H-NMR (400 MHz, CDCl₃, δ/ppm): 9.04 (d, 1H), 8.58 (dd, 1H), 8.09 (dt, 1H), 8.04 (d, 1H), 7.68 (dd, 1H), 7.53-7.50 (m, 2H), 7.36 (dd, 1H), 6.55 (s, 1H), 4.81 (t, 1H), 4.30 (d, 2H), 3.88-3.80 (m, 2H), 3.66-3.58 (m, 2H), 1.26 (t, 6H).

LC/MS (Methode 3, ESIpos): Rₜ = 1.08 min, m/z = 432/434 [M+H]⁺.

### Schritt 2: 1-(2-Chlor-5-nitrophenyl)-6-(pyridin-3-yl)-1H-imidazo[1,2-b]pyrazol

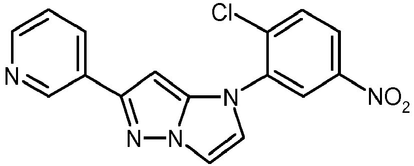

Eine Lösung von 1.44 g (3.33 mmol) der Verbindung aus Beispiel 64A / Schritt 1 in 14 ml Ethanol wurde mit 4 ml (8 mmol) 2 M Schwefelsäure versetzt und anschließend 60 Minuten in einem Mikrowellenofen (Biotage Initiator, mit dynamischer Steuerung der Einstrahlleistung) auf 130°C erhitzt. Nach dem Abkühlen auf RT wurde das Reaktionsgemisch unter Rühren in ca. 50 ml gesättigte wässrige Natriumhydrogencarbonat-Lösung gegeben. Diese Mischung wurde zweimal mit je ca. 50 ml Ethylacetat extrahiert und die vereinigten organischen Phasen mit gesättigter Natriumchlorid-Lösung gewaschen. Nach Trocknen über wasserfreiem Magnesiumsulfat und Filtration wurde im Vakuum eingeengt. Aus dem so erhaltenen Rückstand wurde das Produkt mittels MPLC isoliert (Kieselgel, Dichlormethan/Methanol 50:1). Es wurden 160 mg (14% d. Th.) der Titelverbindung erhalten.

¹H-NMR (400 MHz, DMSO-d₆, δ/ppm): 9.05 (d, 1H), 8.53 (d, 1H), 8.50 (dd, 1H), 8.34 (dd, 1H), 8.19 (dt, 1H), 8.08 (d, 1H), 8.01 (d, 1H), 7.69 (d, 1H), 7.43 (dd, 1H), 6.47 (s, 1H).

LC/MS (Methode 4, ESIpos): Rₜ = 0.77 min, m/z = 340/342 [M+H]⁺.

### Schritt 3: 4-Chlor-3-[6-(pyridin-3-yl)-1H-imidazo[1,2-b]pyrazol-1-yl]anilin

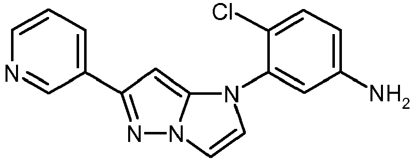

Analog zu dem unter Beispiel 9A / Schritt 3 beschriebenen Verfahren wurden aus 310 mg (0.912 mmol) der Verbindung aus Beispiel 64A / Schritt 2 nach einer Reaktionszeit von 60 Minuten 267 mg (73% Reinheit, 68% d. Th.) eines Gemisches erhalten, welches aus der Titelverbindung und der Verbindung aus Beispiel 62A im Verhältnis 73:27 bestand. Dieses Gemisch wurde ohne weitere Aufreinigung für Folgereaktionen eingesetzt.

¹H-NMR (400 MHz, CDCl₃, δ/ppm, zur Titelverbindung): 9.05 (d, 1H), 8.53 (dd, 1H), 8.16 (dt, 1H), 7.48 (d, 1H), 7.34-7.29 (m, 2H), 7.08 (d, 1H), 6.82 (d, 1H), 6.67 (dd, 1H), 6.12 (s, 1H), 3.91 (s, breit, 2H).

LC/MS (Methode 3, ESIpos): Rₜ = 0.66 min, m/z = 310/312 [M+H]⁺.

### Beispiel 65A

### 3,4-Dimethyl-5-[6-(pyridin-3-yl)-1H-imidazo[1,2-b]pyrazol-1-yl]anilin

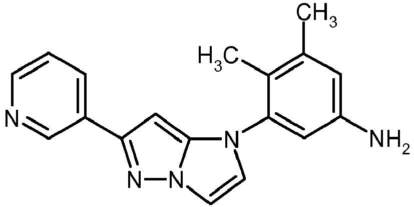

### Schritt 1: 1-(2,2-Diethoxyethyl)-N-(2,3-dimethyl-5-nitrophenyl)-3-(pyridin-3-yl)-1H-pyrazol-5-amin

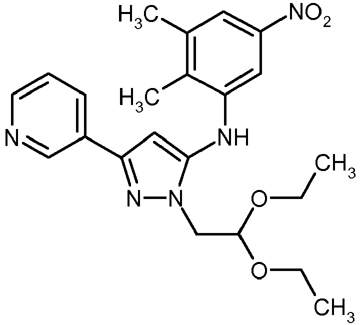

In Analogie zu Beispiel 13A / Schritt 1 wurden aus zwei Ansätzen mit 100 mg (0.36 mmol) bzw. 1.50 g (5.43 mmol) der Verbindung aus Beispiel 4A und 92 mg (0.40 mmol) bzw. 1.37 g (5.97 mmol) 1-Brom-2,3-dimethyl-5-nitrobenzol insgesamt 1.31 g (23% d. Th.) der Titelverbindung erhalten.

¹H-NMR (400 MHz, DMSO-d₆, δ/ppm): 8.99 (d, 1H), 8.47 (dd, 1H), 8.13 (dt, 1H), 7.54-7.60 (m, 2H), 7.36-7.42 (m, 2H), 6.62 (s, 1H), 4.87 (t, 1H), 4.13 (d, 2H), 3.58 (dq, 2H), 3.41 (dq, 2H), 2.35 (s, 3H), 2.23 (s, 3H), 0.98 (t, 6H).

LC/MS (Methode 7, ESIpos): Rₜ = 1.34 min, m/z = 426 [M+H]⁺.

### Schritt 2: 1-(2,3-Dimethyl-5-nitrophenyl)-6-(pyridin-3-yl)-1H-imidazo[1,2-b]pyrazol

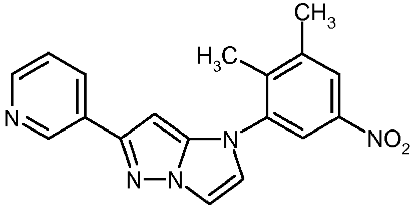

In Analogie zu Beispiel 13A / Schritt 2 wurde aus 1.31 g (3.08 mmol) der Verbindung aus Beispiel 65A / Schritt 1 ein Rohprodukt erhalten, welches durch einmalige Chromatographie über eine Biotage-Anlage gereinigt wurde (50 g Snap-Säule; Laufmittelgradient Dichlormethan/Methanol, von 0% Methanol bis 10% Methanol stetig ansteigend). Man erhielt auf diese Weise 670 mg (63% d. Th.) der Titelverbindung.

¹H-NMR (400 MHz, DMSO-d₆, δ/ppm): 9.00 (dd, 1H), 8.45 (dd, 1H), 8.19 (d, 1H), 8.14 (dt, 1H), 8.10 (d, 1H), 7.92 (dd, 1H), 7.55 (d, 1H), 7.38 (ddd, 1H), 6.35 (s, 1H), 2.23 (s, 3H).

LC/MS (Methode 7, ESIpos): Rₜ = 0.93 min, m/z = 334 [M+H]⁺.

### Schritt 3: 3,4-Dimethyl-5-[6-(pyridin-3-yl)-1H-imidazo[1,2-b]pyrazol-1-yl]anilin

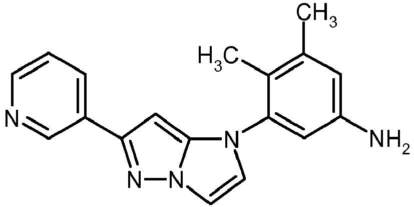

In Analogie zu Beispiel 13A / Schritt 3 wurden aus 670 mg (2.01 mmol) der Verbindung aus Beispiel 65A / Schritt 2 524 mg (86% d. Th.) der Titelverbindung erhalten.

¹H-NMR (400 MHz, DMSO-d₆, δ/ppm): 9.00 (dd, 1H), 8.43 (dd, 1H), 8.14 (dt, 1H), 7.78 (dd, 1H), 7.36 (ddd, 1H), 7.32 (d, 1H), 6.50 (d, 1H), 6.42 (d, 1H), 6.19 (d, 1H), 5.08 (s, 2H), 2.17 (s, 3H), 1.90 (s, 3H).

LC/MS (Methode 7, ESIpos): Rₜ = 0.73 min, m/z = 304 [M+H]⁺.

### Beispiel 66A

### 3-Fluor-4-methyl-5-[6-(pyridin-3-yl)-1H-imidazo[1,2-b]pyrazol-1-yl]anilin

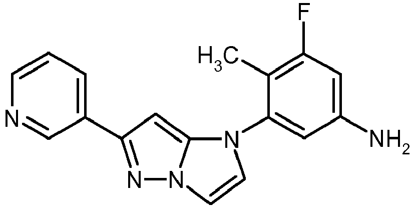

### Schritt 1: N-(3-Fluor-2-methyl-5-nitrophenyl)-1-(2,2-diethoxyethyl)-3-(pyridin-3-yl)-1H-pyrazol-5-amin

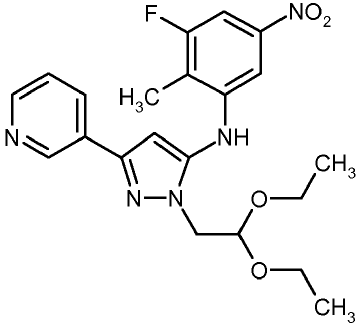

In Analogie zu Beispiel 13A / Schritt 1 wurden aus 1.5 g (5.43 mmol) der Verbindung aus Beispiel 4A und 1.4 g (5.97 mmol) 1-Brom-3-fluor-2-methyl-5-nitrobenzol 1.19 g (50% d. Th.) der Titelverbindung erhalten.

¹H-NMR (400 MHz, DMSO-d₆, δ/ppm): 9.00 (d, 1H), 8.49 (dd, 1H), 8.14 (dt, 1H), 7.89 (s, 1H), 7.50 (dd, 1H), 7.41 (ddd, 1H), 7.28-7.31 (m, 1H), 6.78 (s, 1H), 4.87 (t, 1H), 4.14 (d, 2H), 3.51-3.61 (m, 2H), 3.35-3.45 (m, 2H), 2.24 (d, 3H), 0.96 (t, 6H).

LC/MS (Methode 7, ESIpos): Rₜ = 1.25 min, m/z = 430 [M+H]⁺.

### Schritt 2: 1-(3-Fluor-2-methyl-5-nitrophenyl)-6-(pyridin-3-yl)-1H-imidazo[1,2-b]pyrazol

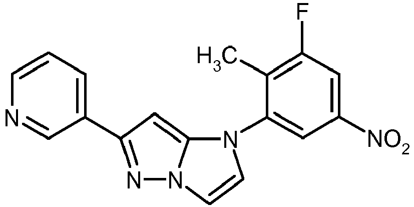

In Analogie zu Beispiel 13A / Schritt 2 wurde aus 1.19 g (2.77 mmol) der Verbindung aus Beispiel 66A / Schritt 1 ein Rohprodukt erhalten, welches durch einmalige Chromatographie über eine Biotage-Anlage gereinigt wurde (25 g Snap-Säule; Laufmittelgradient Hexan/Ethylacetat, von 0% Ethylacetat bis 100% Ethylacetat stetig ansteigend, danach Ethylacetat/Methanol, Methanol-Anteil von 0-80% langsam ansteigend). Man erhielt auf diese Weise 587 mg (60% d. Th.) der Titelverbindung.

¹H-NMR (400 MHz, DMSO-d₆, δ/ppm): 9.01 (d, 1H), 8.46 (dd, 1H), 8.12-8.24 (m, 3H), 7.97 (dd, 1H), 7.64 (d, 1H), 7.39 (ddd, 1H), 6.48 (s, 1H), 2.30 (d, 3H).

LC/MS (Methode 7, ESIpos): Rₜ = 0.92 min, m/z = 338 [M+H]⁺.

### Schritt 3: 3-Fluor-4-methyl-5-[6-(pyridin-3-yl)-1H-imidazo[1,2-b]pyrazol-1-yl]anilin

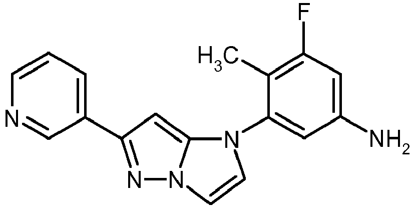

In Analogie zu Beispiel 13A / Schritt 3 wurden aus 570 mg (1.69 mmol) der Verbindung aus Beispiel 66A / Schritt 2 520 mg (100% d. Th.) der Titelverbindung erhalten.

¹H-NMR (400 MHz, DMSO-d₆, δ/ppm): 9.01 (d, 1H), 8.44 (dd, 1H), 8.15 (dt, 1H), 7.83 (d, 1H), 7.43 (d, 1H), 7.37 (dd, 1H), 6.46 (s, 1H), 6.41 (dd, 1H), 6.31 (s, 1H), 5.52 (s, 2H), 1.95 (d, 3H).

LC/MS (Methode 7, ESIpos): Rₜ = 0.79 min, m/z = 308 [M+H]⁺.

### Beispiel 67A

### 3-Chlor-4-methyl-5-[6-(pyridin-3-yl)-1H-imidazo[1,2-b]pyrazol-1-yl]anilin

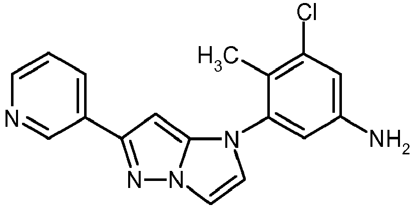

### Schritt 1: N-(3-Chlor-2-methyl-5-nitrophenyl)-1-(2,2-diethoxyethyl)-3-(pyridin-3-yl)-1H-pyrazol-5-amin

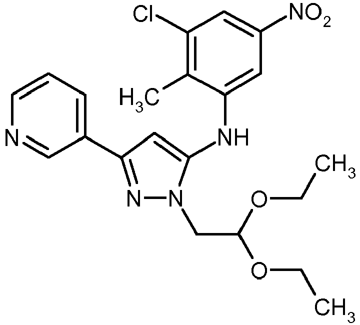

In Analogie zu Beispiel 13A / Schritt 1 wurden aus 1.0 g (3.62 mmol) der Verbindung aus Beispiel 4A und 1.0 g (3.98 mmol) 1-Brom-3-chlor-2-methyl-5-nitrobenzol 670 mg (42% d. Th.) der Titelverbindung erhalten. Die Ausgangsverbindung 1-Brom-3-chlor-2-methyl-5-nitrobenzol ist aus 2-Chlor-1-methyl-4-nitrobenzol gemäß einem Verfahren herstellbar, das in US-Patent 5 877 191 zur Herstellung von 3-Brom-5-fluor-4-methyl-1-nitrobenzol beschrieben ist.

¹H-NMR (400 MHz, DMSO-d₆, δ/ppm): 9.00 (d, 1H), 8.49 (dd, 1H), 8.14 (dt, 1H), 7.88 (s, 1H), 7.71 (d, 1H), 7.35-7.45 (m, 2H), 6.76 (s, 1H), 4.87 (t, 1H), 4.13 (d, 2H), 3.50-3.63 (m, 2H), 3.33-3.46 (m, 2H), 2.39 (s, 3H), 0.96 (t, 6H).

LC/MS (Methode 7, ESIpos): Rₜ = 1.40 min, m/z = 446/448 [M+H]⁺.

### Schritt 2: 1-(3-Chlor-2-methyl-5-nitrophenyl)-6-(pyridin-3-yl)-1H-imidazo[1,2-b]pyrazol

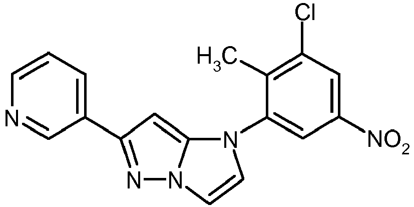

In Analogie zu Beispiel 13A / Schritt 2 wurde aus 670 mg (1.50 mmol) der Verbindung aus Beispiel 67A / Schritt 1 ein Rohprodukt erhalten, welches durch einmalige Chromatographie über eine Biotage-Anlage gereinigt wurde (25 g Snap-Säule; Laufmittelgradient Hexan/Ethylacetat, von 0% Ethylacetat bis 100% Ethylacetat stetig ansteigend, danach Ethylacetat/Methanol, Methanol-Anteil von 0-80% langsam ansteigend). Man erhielt auf diese Weise 309 mg (52% d. Th.) der Titelverbindung.

¹H-NMR (400 MHz, DMSO-d₆, δ/ppm): 9.01 (d, 1H), 8.45 (dd, 1H), 8.40 (d, 1H), 8.27 (d, 1H), 8.15 (dt, 1H), 7.96 (d, 1H), 7.62 (d, 1H), 7.39 (dd, 1H), 6.46 (s, 1H), 2.39 (s, 3H).

LC/MS (Methode 7, ESIpos): Rₜ = 1.00 min, m/z = 354/356 [M+H]⁺.

### Schritt 3: 3-Chlor-4-methyl-5-[6-(pyridin-3-yl)-1H-imidazo[1,2-b]pyrazol-1-yl]anilin

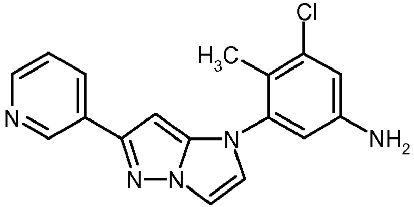

In Analogie zu Beispiel 13A / Schritt 3 wurden aus 253 mg (0.72 mmol) der Verbindung aus Beispiel 67A / Schritt 2 249 mg (97% d. Th.) der Titelverbindung erhalten.

¹H-NMR (400 MHz, DMSO-d₆, δ/ppm): 9.01 (d, 1H), 8.44 (dd, 1H), 8.15 (dt, 1H), 7.83 (d, 1H), 7.42 (d, 1H), 7.37 (dd, 1H), 6.73 (d, 1H), 6.58 (d, 1H), 6.30 (s, 1H), 5.51 (s, 2H), 2.04 (s, 3H).

LC/MS (Methode 7, ESIpos): Rₜ = 0.86 min, m/z = 324/326 [M+H]⁺.

### Beispiel 68A

### 2,4-Dimethyl-3-[6-(pyridin-3-yl)-1H-imidazo[1,2-b]pyrazol-1-yl]anilin

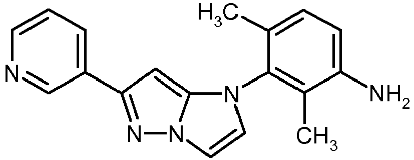

### Schritt 1: 1-(2,2-Diethoxyethyl)-N-(2,6-dimethyl-3-nitrophenyl)-3-(pyridin-3-yl)-1H-pyrazol-5-amin

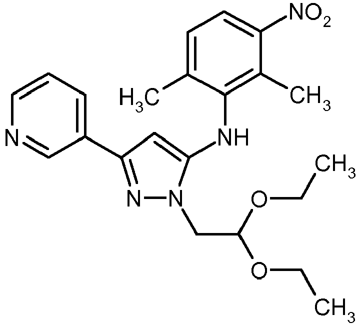

In Analogie zu Beispiel 13A / Schritt 1 wurden aus einem Probeansatz mit 1.0 g (3.62 mmol) bzw. dem Hauptansatz mit 2.0 g (7.24 mmol) der Verbindung aus Beispiel 4A und 916 mg (3.98 mmol) bzw. 1.83 g (7.96 mmol) 4-Brom-1,3-dimethyl-2-nitrobenzol insgesamt 3.15 g (75% d. Th.) der Titelverbindung erhalten.

¹H-NMR (400 MHz, DMSO-d₆, δ/ppm): 8.83 (d, 1H), 8.39 (dd, 1H), 7.95-8.00 (m, 1H), 7.70 (d, 1H), 7.40 (s, 1H), 7.34 (d, 1H), 7.29 (dd, 1H), 5.46 (s, 1H), 4.97 (t, 1H), 4.24 (d, 2H), 3.62-3.73 (m, 2H), 3.44-3.54 (m, 2H), 2.27 (s, 3H), 2.25 (s, 3H), 1.06 (t, 6H).

LC/MS (Methode 6, ESIpos): Rₜ = 1.15 min, m/z = 426 [M+H]⁺.

### Schritt 2: 1-(2,6-Dimethyl-3-nitrophenyl)-6-(pyridin-3-yl)-1H-imidazo[1,2-b]pyrazol

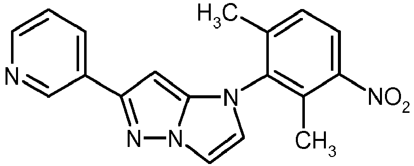

In Analogie zu Beispiel 13A / Schritt 2 wurde aus zwei Ansätzen mit je 1.58 g (3.70 mmol) der Verbindung aus Beispiel 68A / Schritt 1 ein Rohprodukt erhalten, welches zunächst über eine Biotage-Anlage aufgereinigt wurde (100 g Snap-Säule; Laufmittelgradient Hexan/Ethylacetat, von 0% Ethylacetat bis 100% Ethylacetat stetig ansteigend, danach Ethylacetat/Methanol, Methanol-Anteil von 0-80% langsam ansteigend). Eine zweite Reinigung erfolgte dann mittels präparativer HPLC (Methode 16). Es wurden 534 mg (22% d. Th.) der Titelverbindung erhalten.

¹H-NMR (400 MHz, DMSO-d₆, δ/ppm): 8.99 (dd, 1H), 8.44 (dd, 1H), 8.13 (dt, 1H), 8.03 (d, 1H), 7.92 (dd, 1H), 7.55 (d, 1H), 7.44 (d, 1H), 7.37 (ddd, 1H), 6.23 (d, 1H), 2.14 (s, 3H), 2.13 (s, 3H).

LC/MS (Methode 6, ESIpos): Rₜ = 0.93 min, m/z = 334 [M+H]⁺.

### Schritt 3: 2,4-Dimethyl-3-[6-(pyridin-3-yl)-1H-imidazo[1,2-b]pyrazol-1-yl]anilin

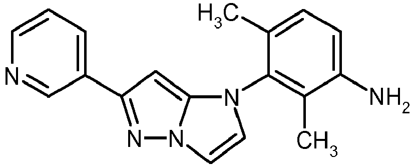

In Analogie zu Beispiel 13A / Schritt 3 wurden aus 530 mg (1.59 mmol) der Verbindung aus Beispiel 68A / Schritt 2 507 mg (99% d. Th.) der Titelverbindung erhalten.

¹H-NMR (400 MHz, DMSO-d₆, δ/ppm): 8.99 (dd, 1H), 8.42 (dd, 1H), 8.13 (dt, 1H), 7.80 (dd, 1H), 7.35 (ddd, 1H), 7.25 (d, 1H), 6.91 (d, 1H), 6.69 (d, 1H), 6.08 (d, 1H), 4.96 (s, 2H), 1.86 (s, 3H), 1.72 (s, 3H).

LC/MS (Methode 6, ESIpos): Rₜ = 0.74 min, m/z = 304 [M+H]⁺.

### Beispiel 69A

### 2-Methyl-3-[6-(pyridin-3-yl)-1H-imidazo[1,2-b]pyrazol-1-yl]anilin

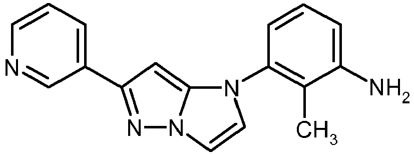

### Schritt 1: 1-(2,2-Diethoxyethyl)-N-(2-methyl-3-nitrophenyl)-3-(pyridin-3-yl)-1H-pyrazol-5-amin

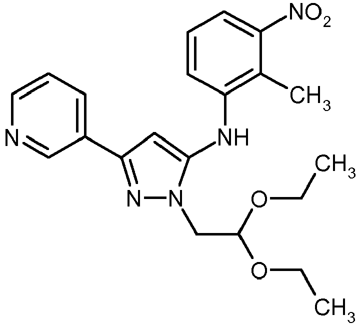

Analog zu dem unter Beispiel 64A / Schritt 1 beschriebenen Verfahren wurden aus 1.0 g (3.62 mmol) der Verbindung aus Beispiel 4A und 860 mg (3.98 mmol) 2-Brom-6-nitrotoluol 1.45 g (97% d. Th.) der Titelverbindung erhalten.

¹H-NMR (400 MHz, CDCl₃, δ/ppm): 9.00 (d, 1H), 8.56 (dd, 1H), 8.08 (dt, 1H), 7.35-7.31 (m, 3H), 7.22 (t, 1H), 6.80 (s, 1H), 6.37 (s, 1H), 4.82 (t, 1H), 4.27 (d, 2H), 3.88-3.80 (m, 2H), 3.66-3.58 (m, 2H), 2.38 (s, 3H), 1.26 (t, 6H).

LC/MS (Methode 3, ESIpos): Rₜ = 1.02 min, m/z = 412 [M+H]⁺.

### Schritt 2: 1-(2-Methyl-3-nitrophenyl)-6-(pyridin-3-yl)-1H-imidazo[1,2-b]pyrazol

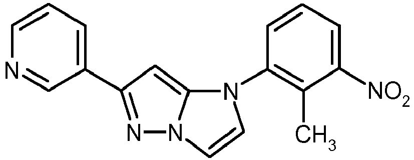

Analog zu dem unter Beispiel 64A / Schritt 2 beschriebenen Verfahren wurden aus 1.43 g (3.47 mmol) der Verbindung aus Beispiel 69A / Schritt 1 1.09 g (95% d. Th.) der Titelverbindung erhalten. Die Reaktionszeit in der Mikrowelle betrug in diesem Fall 15 min bei 130°C. Auf eine chromatographische Reinigung des Produktes konnte hier verzichtet werden.

¹H-NMR (400 MHz, DMSO-d₆, δ/ppm): 9.06 (d, 1H), 8.49 (dd, 1H), 8.19 (dt, 1H), 8.06 (d, 1H), 7.97 (d, 1H), 7.88 (d, 1H), 7.66 (t, 1H), 7.61 (d, 1H), 7.42 (dd, 1H), 6.44 (s, 1H), 2.35 (s, 3H).

LC/MS (Methode 4, ESIpos): Rₜ = 0.76 min, m/z = 320 [M+H]⁺.

### Schritt 3: 2-Methyl-3-[6-(pyridin-3-yl)-1H-imidazo[1,2-b]pyrazol-1-yl]anilin

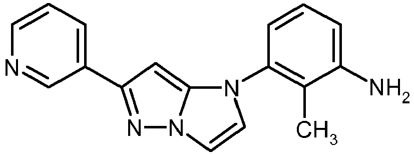

Analog zu dem unter Beispiel 9A / Schritt 3 beschriebenen Verfahren wurden aus 1.08 g (3.38 mmol) der Verbindung aus Beispiel 69A / Schritt 2 940 mg (93% d. Th., 97% Reinheit) der Titelverbindung erhalten. Die Reaktionszeit betrug in diesem Fall 30 Minuten.

¹H-NMR (400 MHz, CDCl₃, δ/ppm): 9.04 (d, 1H), 8.52 (dd, 1H), 8.15 (dt, 1H), 7.49 (d, 1H), 7.32 (dd, 1H), 7.14 (t, 1H), 6.90 (d, 1H), 6.80 (d, 1H), 6.77 (d, 1H), 5.96 (s, 1H), 3.86 (breit, 2H), 2.06 (s, 3H).

LC/MS (Methode 3, ESIpos): Rₜ = 0.63 min, m/z = 290 [M+H]⁺.

### Beispiel 70A

### 2-Fluor-3-[6-(pyridin-3-yl)-1H-imidazo[1,2-b]pyrazol-1-yl]anilin

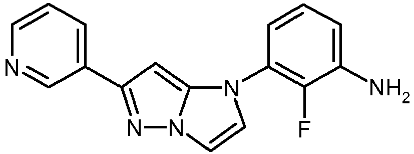

### Schritt 1: 1-(2,2-Diethoxyethyl)-N-(2-fluor-3-nitrophenyl)-3-(pyridin-3-yl)-1H-pyrazol-5-amin

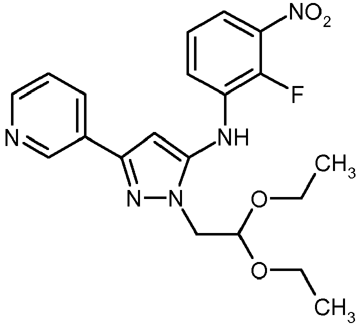

Analog zu dem unter Beispiel 64A / Schritt 1 beschriebenen Verfahren wurden aus 1.0 g (3.62 mmol) der Verbindung aus Beispiel 4A und 876 mg (3.98 mmol) 1-Brom-2-fluor-3-nitrobenzol 1.11 g (73% d. Th.) der Titelverbindung erhalten.

¹H-NMR (400 MHz, CDCl₃, δ/ppm): 9.00 (d, 1H), 8.56 (dd, 1H), 8.09 (dt, 1H), 7.54-7.48 (m, 3H), 7.34 (t, 1H), 7.17 (t, 1H), 6.44 (s, 1H), 4.81 (t, 1H), 4.31 (d, 2H), 3.89-3.81 (m, 2H), 3.65-3.57 (m, 2H), 1.28 (t, 6H).

LC/MS (Methode 3, ESIpos): Rₜ = 1.00 min, m/z = 416 [M+H]⁺.

### Schritt 2: 1-(2-Fluor-3-nitrophenyl)-6-(pyridin-3-yl)-1H-imidazo[1,2-b]pyrazol

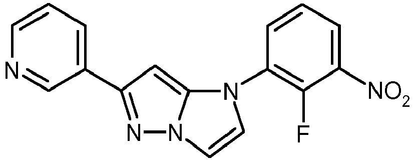

Analog zu dem unter Beispiel 69A / Schritt 2 beschriebenen Verfahren wurden aus 1.10 g (2.65 mmol) der Verbindung aus Beispiel 70A / Schritt 1 570 mg (63% d. Th.) der Titelverbindung erhalten. Das Produkt wurde abschließend zur Reinigung mit wenig Acetonitril bei RT verrührt.

¹H-NMR (400 MHz, DMSO-d₆, δ/ppm): 9.07 (d, 1H), 8.51 (dd, 1H), 8.21 (dt, 1H), 8.18 (d, 1H), 8.15 (d, 1H), 8.04 (d, 1H), 7.74 (dd, 1H), 7.63 (td, 1H), 7.45 (dd, 1H), 6.66 (d, 1H).

LC/MS (Methode 4, ESIpos): Rₜ = 0.73 min, m/z = 324 [M+H]⁺.

### Schritt 3: 2-Fluor-3-[6-(pyridin-3-yl)-1H-imidazo[1,2-b]pyrazol-1-yl]anilin

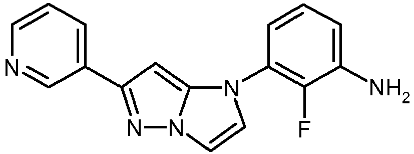

Analog zu dem unter Beispiel 69A / Schritt 3 beschriebenen Verfahren wurden aus 560 mg (1.73 mmol) der Verbindung aus Beispiel 70A / Schritt 2 490 mg (96% d. Th.) der Titelverbindung erhalten.

¹H-NMR (400 MHz, DMSO-d₆, δ/ppm): 9.07 (d, 1H), 8.49 (dd, 1H), 8.20 (dt, 1H), 7.92 (d, 1H), 7.57 (dd, 1H), 7.43 (dd, 1H), 7.03 (td, 1H), 6.81 (dd, 1H), 6.77 (dd, 1H), 6.49 (s, 1H), 5.56 (s, breit, 2H).

LC/MS (Methode 3, ESIpos): Rₜ = 0.60 min, m/z = 294 [M+H]⁺.

### Beispiel 71A

### 2-Amino-6-[6-(pyridin-3-yl)-1H-imidazo[1,2-b]pyrazol-1-yl]phenol

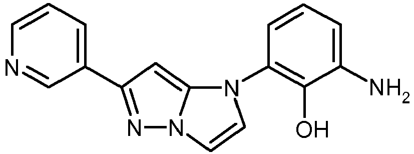

### Schritt 1: 2-(Benzyloxy)-1-brom-3-nitrobenzol

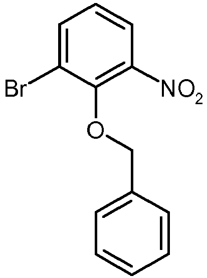

Ein Gemisch aus 5.22 g (23.9 mmol) 2-Brom-6-nitrophenol, 3.0 ml (25.1 mmol) Benzylbromid und 3.64 g (26.3 mmol) Kaliumcarbonat in 50 ml Acetonitril wurde 2 h unter Rückfluss erhitzt. Nach dem Abkühlen auf RT wurde vom Feststoff abfiltriert und das Filtrat am Rotationsverdampfer vom Lösungsmittel befreit. Der erhaltene Rückstand wurde in Ethylacetat gelöst und nacheinander mit Wasser und gesättigter Natriumchlorid-Lösung gewaschen. Nach Trocknen über wasserfreiem Magnesiumsulfat wurde filtriert und das Filtrat am Rotationsverdampfer eingedampft. Der verbliebene Rückstand wurde im Hochvakuum getrocknet. Es wurden 7.53 g (99% d. Th.) der Titelverbindung erhalten.

¹H-NMR (400 MHz, CDCl₃, δ/ppm): 7.84 (dd, 1H), 7.79 (dd, 1H), 7.57-7.53 (m, 2H), 7.44-7.36 (m, 3H), 7.16 (t, 1H), 5.20 (s, 2H).

LC/MS (Methode 4, ESIpos): Rₜ = 1.26 min, keine Ionisation.

### Schritt 2: N-[2-(Benzyloxy)-3-nitrophenyl]-1-(2,2-diethoxyethyl)-3-(pyridin-3-yl)-1H-pyrazol-5-amin

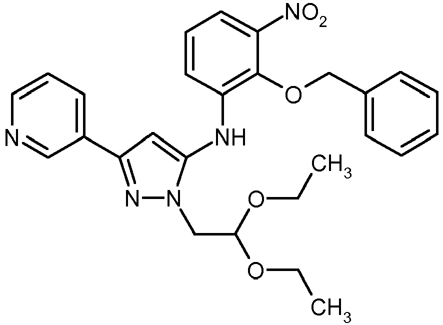

Analog zu dem unter Beispiel 64A / Schritt 1 beschriebenen Verfahren wurden aus 1.0 g (3.62 mmol) der Verbindung aus Beispiel 4A und 1.23 g (3.98 mmol) der Verbindung aus Beispiel 71A / Schritt 1 1.44 g der Titelverbindung erhalten (63% d. Th., Gehalt 80%, Verunreinigung: debenzyliertes Produkt). Die chromatographische Reinigung erfolgte hier mit Cyclohexan/Ethylacetat 1:1 als Laufmittel.

LC/MS (Methode 2, ESIpos): Rₜ = 2.54 min, m/z = 504 [M+H]⁺.

### Schritt 3: 2-Nitro-6-[6-(pyridin-3-yl)-1H-imidazo[1,2-b]pyrazol-1-yl]phenol

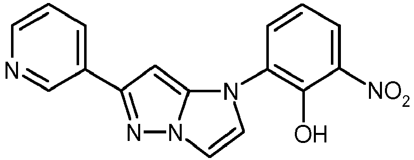

Eine Lösung von 1.43 g (2.27 mmol, Gehalt 80%) der Verbindung aus Beispiel 71A / Schritt 2 in 10 ml Ethanol wurde mit 2.7 ml (5.45 mmol) 2 M Schwefelsäure versetzt und anschließend 20 min in einem Mikrowellenofen (Biotage Initiator, mit dynamischer Steuerung der Einstrahlleistung) auf 130°C erhitzt. Nach dem Abkühlen auf RT wurde das Reaktionsgemisch unter Rühren in ca. 50 ml gesättigte wässrige Natriumhydrogencarbonat-Lösung gegeben. Diese Mischung wurde zweimal mit je ca. 50 ml Ethylacetat extrahiert und die vereinigten organischen Phasen mit gesättigter Natriumchlorid-Lösung gewaschen. Nach Trocknen über wasserfreiem Magnesiumsulfat und Filtration wurde im Vakuum eingeengt. Der verbliebene Rückstand wurde mit Acetonitril verrührt. Das Ungelöste wurde abgesaugt und im Hochvakuum getrocknet. Es wurden so 75 mg (10% d. Th.) der Titelverbindung gewonnen. Das erhaltene Filtrat wurde weiter bearbeitet, siehe nachfolgenden Schritt 4.

¹H-NMR (400 MHz, CDCl₃, δ/ppm): 9.06 (s, 1H), 8.55 (d, 1H), 8.19-8.15 (m, 2H), 7.89 (d, 1H), 7.54 (d, 1H), 7.36-7.33 (m, 2H), 7.18 (t, 1H), 6.22 (s, 1H).

LC/MS (Methode 3, ESIpos): Rₜ = 0.68 min, m/z = 322 [M+H]⁺.

### Schritt 4: 1-[2-(Benzyloxy)-3-nitrophenyl]-6-(pyridin-3-yl)-1H-imidazo[1,2-b]pyrazol

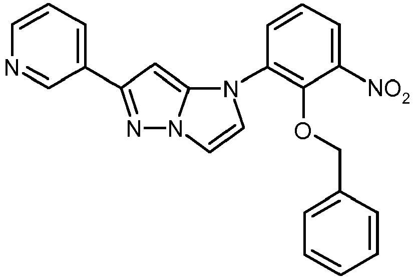

Das aus dem vorangegangenen Schritt 3 erhaltene Filtrat wurde am Rotationsverdampfer vom Lösungsmittel befreit, und der verbliebene Rückstand wurde mittels präparativer HPLC (Methode 9) gereinigt. Nach Eindampfen der Produktfraktionen und Trocknen des Rückstands im Hochvakuum wurden 263 mg (28% d. Th.) der Titelverbindung erhalten.

¹H-NMR (400 MHz, CDCl₃, δ/ppm): 9.05 (d, 1H), 8.57 (dd, 1H), 8.15 (dt, 1H), 7.82-7.78 (m, 2H), 7.52 (d, 1H), 7.40 (t, 1H), 7.36 (dd, 1H), 7.25-7.21 (m, 3H, teilweise überdeckt vom CHCl₃-Signal), 7.15-7.12 (m, 2H), 6.19 (s, 1H), 4.83 (s, 2H).

LC/MS (Methode 3, ESIpos): Rₜ = 0.92 min, m/z = 412 [M+H]⁺.

### Schritt 5: 2-Amino-6-[6-(pyridin-3-yl)-1H-imidazo[1,2-b]pyrazol-1-yl]phenol

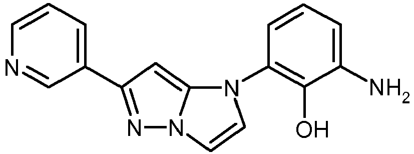

### Variante A: Herstellung ausgehend von der Verbindung aus Beispiel 71A / Schritt 3:

Analog zu dem unter Beispiel 9A / Schritt 3 beschriebenen Verfahren wurden aus 72 mg (0.224 mmol) der Verbindung aus Beispiel 71A / Schritt 3 57 mg (87% d. Th.) der Titelverbindung erhalten. Die Reaktionszeit betrug in diesem Fall 1 h.

### Variante B: Herstellung ausgehend von der Verbindung aus Beispiel 71A / Schritt 4:

Analog zu dem unter Beispiel 9A / Schritt 3 beschriebenen Verfahren wurden aus 260 mg (0.632 mmol) der Verbindung aus Beispiel 71A / Schritt 4 200 mg (86% d. Th., 80% Reinheit) der Titelverbindung erhalten. Die Reaktionszeit betrug in diesem Fall 1 h.

¹H-NMR (400 MHz, DMSO-d₆, δ/ppm): 9.04 (d, 1H), 8.47 (dd, 1H), 8.18 (dt, 1H), 7.80 (d, 1H), 7.46 (d, 1H), 7.41 (dd, 1H), 6.76 (t, 1H), 6.69-6.64 (m, 2H), 6.32 (s, 1H), 3.50 (breit, 1H).

LC/MS (Methode 3, ESIpos): Rₜ = 0.56 min, m/z = 292 [M+H]⁺.

### Beispiel 72A

### 4-Methyl-3-[6-(pyrazin-2-yl)-1H-imidazo[1,2-b]pyrazol-1-yl]anilin

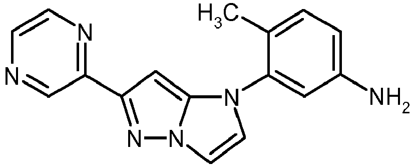

### Schritt 1: 1-(2,2-Diethoxyethyl)-N-(2-methyl-5-nitrophenyl)-3-(pyrazin-2-yl)-1H-pyrazol-5-amin

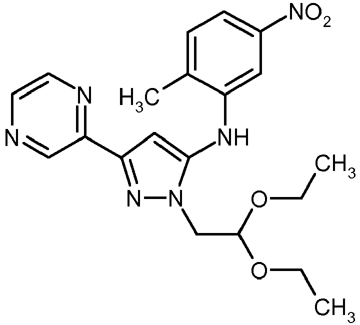

In Analogie zu Beispiel 13A / Schritt 1 wurden aus 1.16 g (4.18 mmol) der Verbindung aus Beispiel 59A und 994 mg (4.60 mmol) 2-Brom-4-nitrotoluol 1.64 g (86% d. Th.) der Titelverbindung erhalten.

¹H-NMR (400 MHz, DMSO-d₆, δ/ppm): 9.14 (d, 1H), 8.61 (dd, 1H), 8.54 (d, 1H), 7.63 (s, 1H), 7.60 (dd, 1H), 7.48 (d, 1H), 7.40 (d, 1H), 6.71 (s, 1H), 4.88 (t, 1H), 4.20 (d, 2H), 3.53-3.62 (m, 2H), 3.36-3.46 (m, 2H), 2.35 (s, 3H), 0.97 (t, 6H).

LC/MS (Methode 7, ESIpos): Rₜ = 1.29 min, m/z = 413 [M+H]⁺.

### Schritt 2: 1-(2-Methyl-5-nitrophenyl)-6-(pyrazin-2-yl)-1H-imidazo[1,2-b]pyrazol

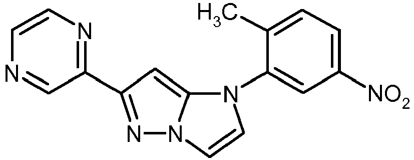

In Analogie zu Beispiel 13A / Schritt 2 wurde aus 1.63 g (3.95 mmol) der Verbindung aus Beispiel 72A / Schritt 1 ein Rohprodukt erhalten, welches durch einmalige Chromatographie über eine Biotage-Anlage gereinigt wurde (25 g Snap-Säule; Laufmittelgradient Dichlormethan/Methanol, von 2% Methanol bis 8% Methanol stetig ansteigend). Man erhielt auf diese Weise 346 mg (25% d. Th.) der Titelverbindung.

¹H-NMR (400 MHz, DMSO-d₆, δ/ppm): 9.16 (d, 1H), 8.58 (dd, 1H), 8.51 (d, 1H), 8.28 (d, 1H), 8.21 (dd, 1H), 7.99 (dd, 1H), 7.73 (d, 1H), 7.70 (d, 1H), 6.41 (d, 1H), 5.72 (s, 2H), 2.39 (s, 3H).

LC/MS (Methode 7, ESIpos): Rₜ = 1.02 min, m/z = 321 [M+H]⁺.

### Schritt 3: 4-Methyl-3-[6-(pyrazin-2-yl)-1H-imidazo[1,2-b]pyrazol-1-yl]anilin

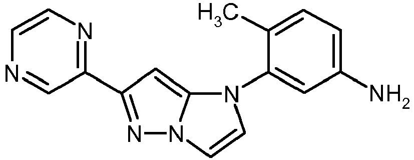

In Analogie zu Beispiel 13A / Schritt 3 wurden aus 343 mg (1.07 mmol) der Verbindung aus Beispiel 72A / Schritt 2 255 mg (65% d. Th.) der Titelverbindung erhalten.

¹H-NMR (400 MHz, DMSO-d₆, δ/ppm): 9.14 (d, 1H), 8.57 (dd, 1H), 8.49 (d, 1H), 7.85 (dd, 1H), 7.48 (d, 1H), 7.02 (d, 1H), 6.62 (d, 1H), 6.55 (dd, 1H), 6.24 (d, 1H), 5.19 (br. s, 2H), 2.05 (s, 3H).

LC/MS (Methode 7, ESIpos): Rₜ = 0.81 min, m/z = 291 [M+H]⁺.

### Beispiel 73A

### 3-Cyano-5-(1-hydroxycyclobutyl)benzoesäure

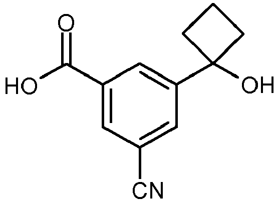

### Schritt 1: Methyl-3-carbamoyl-5-iodbenzoat

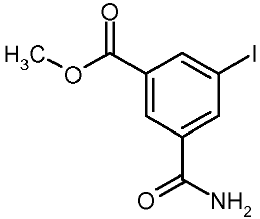

Eine Lösung von 3.88 g (11.8 mmol) Natrium-3-iod-5-(methoxycarbonyl)benzoat [J. H. Ackermann et al., J. Med. Chem. 1966, 9 (1), 165-168] in 100 ml Wasser wurde mit 20 ml 1 M Salzsäure versetzt. Nach 10 min wurde dreimal mit je ca. 100 ml Ethylacetat extrahiert. Die vereinigten organischen Extrakte wurden mit gesättigter Natriumchlorid-Lösung gewaschen und über wasserfreiem Magnesiumsulfat getrocknet. Nach Filtration wurde das Lösungsmittel am Rotationsverdampfer entfernt. Der verbliebene Rückstand wurde in 75 ml Dichlormethan gelöst und bei RT mit 5.2 ml (59.1 mmol) Oxalylchlorid und einem kleinen Tropfen DMF versetzt. Nach 1 h Rühren bei RT wurden alle flüchtigen Bestandteile am Rotationsverdampfer entfernt. Dann wurde der Rückstand in 50 ml Dioxan gelöst und bei einer Temperatur von ca. 0°C langsam zu 44 ml einer 25%-igen wässrigen Ammoniak-Lösung getropft. Nach beendetem Zutropfen wurde das Kältebad entfernt und das Rühren 30 min bei RT fortgesetzt. Anschließend wurde das ausgefallene Produkt abgesaugt, mit kaltem Wasser gewaschen und im Hochvakuum getrocknet. Es wurden 3.02 g (81% d. Th., 97% Reinheit) der Titelverbindung erhalten.

¹H-NMR (400 MHz, DMSO-d₆, δ/ppm): 8.47 (dd, 1H), 8.44 (dd, 1H), 8.35 (dd, 1H), 8.25 (s, breit, 1H), 7.63 (s, breit, 1H), 3.89 (s, 3H).

LC/MS (Methode 4, ESIpos): Rₜ = 0.75 min, m/z = 306 [M+H]⁺.

### Schritt 2: Methyl-3-cyano-5-iodbenzoat

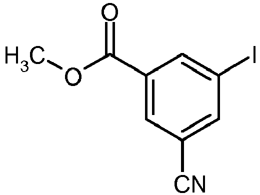

Eine Lösung von 2.80 g (9.18 mmol) der Verbindung aus Beispiel 73A / Schritt 1 und 8 ml (45.9 mmol) *N,N*-Diisopropylethylamin in 150 ml Dichlormethan wurde bei einer Temperatur von ca. 0°C tropfenweise mit einer Lösung von 2.8 ml (16.5 mmol) Trifluormethansulfonsäureanhydrid in 50 ml Dichlormethan versetzt. Nach einer Reaktionszeit von 30 min bei 0°C wurde das Gemisch mit 50 ml gesättigter wässriger Natriumhydrogencarbonat-Lösung versetzt und 10 min intensiv bei RT gerührt. Die organische Phase wurde abgetrennt, über wasserfreiem Magnesiumsulfat getrocknet, filtriert und am Rotationsverdampfer vom Lösungsmittel befreit. Das so erhaltene Rohprodukt wurde mittels Saugfiltration gereinigt (ca. 200 g Kieselgel, Laufmittel Cyclohexan/Ethylacetat 3:1). Es wurden 2.24 g (85% d. Th.) der Titelverbindung erhalten.

¹H-NMR (400 MHz, CDCl₃, δ/ppm): 8.59 (dd, 1H), 8.28 (dd, 1H), 8.15 (dd, 1H), 3.97 (s, 3H).

GC/MS (Methode 8, EIpos): Rₜ = 5.62 min, m/z = 287 [M]⁺.

### Schritt 3: Methyl-3-cyano-5-(1-hydroxycyclobutyl)benzoat

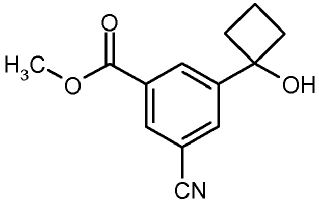

Eine Lösung von 400 mg (1.39 mmol) der Verbindung aus Beispiel 73A / Schritt 2 in 10 ml wasserfreiem THF wurde bei einer Temperatur von -40°C tropfenweise mit 1.1 ml (1.46 mmol) einer 1.3 M Lösung von Isopropylmagnesiumchlorid-Lithiumchlorid-Komplex in THF versetzt. Nach beendetem Zutropfen wurde das Gemisch noch 1.5 h bei -40°C bis -30°C nachgerührt, bevor die Temperatur auf -78°C gesenkt wurde. Dann wurde diese Lösung langsam bei -78°C zu einer Lösung von 209 µl (2.79 mmol) Cyclobutanon in 4 ml wasserfreiem THF getropft. Zehn Minuten nach beendeter Zugabe und weiterem Rühren bei -78°C wurde das Reaktionsgemisch zunächst auf 0°C und dann nach weiteren 45 min auf RT erwärmt. Nach 1 h Rühren bei RT wurde bei ca. -20°C mit 250 ml Wasser versetzt. Es wurde dreimal mit jeweils 100 ml Ethylacetat extrahiert. Die vereinigten organischen Extrakte wurden mit gesättigter Natriumchlorid-Lösung gewaschen, über wasserfreiem Magnesiumsulfat getrocknet, filtriert und am Rotationsverdampfer vom Lösungsmittel befreit. Das Produkt wurde aus dem so erhaltenen Rückstand mittels MPLC isoliert (ca. 50 g Kieselgel, Laufmittelgradient Cyclohexan/Ethylacetat 10:1 → 4:1). Es wurden 94 mg (29% d. Th.) der Titelverbindung erhalten.

¹H-NMR (400 MHz, CDCl₃, δ/ppm): 8.41 (dd, 1H), 8.23 (dd, 1H), 8.01 (dd, 1H), 3.97 (s, 3H), 2.60-2.52 (m, 2H), 2.47-2.39 (m, 3H), 2.18-2.08 (m, 1H), 1.87-1.76 (m, 1H).

GC/MS (Methode 8, EIpos): Rₜ = 6.60 min, m/z = 213 [M-H₂O]⁺.

### Schritt 4: 3-Cyano-5-(1-hydroxycyclobutyl)benzoesäure

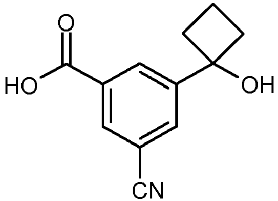

85 mg (0.368 mmol) der Verbindung aus Beispiel 73A / Schritt 3 wurden in einem Gemisch aus 1 ml Methanol und 1 ml THF gelöst und bei RT mit einer Lösung von 19 mg (0.441 mmol) Lithiumhydroxid-Monohydrat in 1 ml Wasser versetzt. Nachdem das Reaktionsgemisch 1 h gerührt worden war, wurde es mit je 50 ml Wasser und Ethylacetat sowie 2 ml 1 M Salzsäure versetzt und intensiv gerührt. Nach Phasentrennung wurde die wässrige Phase noch einmal mit 50 ml Ethylacetat extrahiert. Die vereinigten organischen Extrakte wurden mit gesättigter Natriumchlorid-Lösung gewaschen, über wasserfreiem Magnesiumsulfat getrocknet, filtriert und am Rotationsverdampfer vom Lösungsmittel befreit. Auf diese Weise wurden 78 mg (95% d. Th., 97% Reinheit) der Titelverbindung erhalten.

¹H-NMR (400 MHz, DMSO-d₆, δ/ppm): 13.53 (breit, 1H), 8.33 (dd, 1H), 8.17 (dd, 1H), 8.14 (dd, 1H), 5.90 (s, breit, 1H), 2.45-2.39 (m, 2H), 2.34-2.27 (m, 2H), 2.02-1.93 (m, 1H), 1.77-1.69 (m, 1H).

LC/MS (Methode 3, ESIneg): Rₜ = 0.68 min, m/z = 216 [M-H]⁻, 433 [2M-H]⁻.

### Beispiel 74A

### 3-(Methoxymethyl)-5-(pentafluor-λ⁶-sulfanyl)benzoesäure

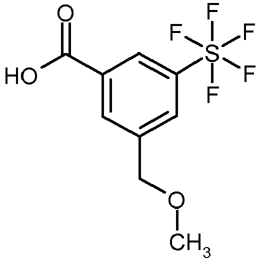

108 mg (2.70 mmol) Natriumhydrid (als 60%-ige Suspension in Mineralöl) wurden mit Pentan entölt und anschließend in 5 ml wasserfreiem THF suspendiert. Zu dieser Suspension wurde bei 0°C eine Lösung von 250 mg (0.899 mmol) der Verbindung aus Beispiel 37A in 2.5 ml wasserfreiem THF langsam hinzugetropft. Nach 30 min Rühren bei 0°C wurden 170 µl (2.70 mmol) Iodmethan hinzugefügt und das Kältebad entfernt. Da nach 6 h bei RT der Umsatz laut analytischer HPLC noch nicht vollständig war, wurde erneut auf 0°C abgekühlt und nacheinander mit weiteren 560 µl (9.0 mmol) Iodmethan und 36 mg (0.897 mmol) Natriumhydrid (60%-ige Suspension in Mineralöl) versetzt. Nach 2 h Rühren bei RT wurde das Reaktionsgemisch vorsichtig mit ca. 75 ml Wasser versetzt und durch Zugabe von 1 M Salzsäure sauer gestellt. Es wurde dreimal mit je ca. 25 ml Ethylacetat extrahiert. Die vereinigten organischen Extrakte wurden nacheinander mit je ca. 30 ml Wasser und gesättigter Natriumchlorid-Lösung gewaschen. Nach Trocknen über wasserfreiem Magnesiumsulfat, Filtration und Entfernen des Lösungsmittels am Rotationsverdampfer wurde der erhaltene Rückstand mit Pentan verrührt. Das so gewonnene Rohprodukt wurde mittels präparativer HPLC (Methode 32) gereinigt. Nach Vereinigung der Produktfraktionen, Entfernen des Lösungsmittels am Rotationsverdampfer und Trocknen im Hochvakuum wurden 153 mg (58% d. Th.) der Titelverbindung erhalten.

¹H-NMR (400 MHz, DMSO-d₆, δ/ppm): 13.75 (breit, 1H), 8.20 (s, 1H), 8.16 (s, 1H), 8.09 (s, 1H), 4.60 (s, 1H), 3.36 (s, 3H).

LC/MS (Methode 3, ESIneg): Rₜ = 0.94 min, m/z = 291 [M-H]⁻.

### Beispiel 75A

### 3-Cyano-N-(3-{6-[1-(4-methoxybenzyl)-1H-pyrazol-4-yl]-1H-imidazo[1,2-b]pyrazol-1-yl}-4-methylphenyl)-5-(pentafluor-λ⁶-sulfanyl)benzamid

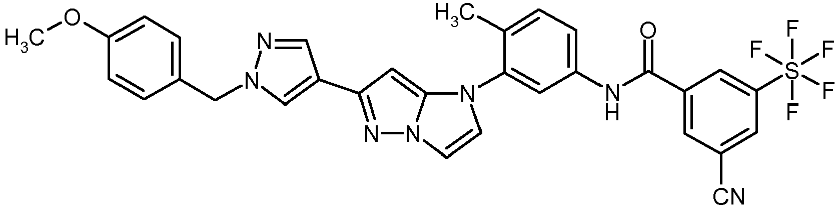

80 mg (0.16 mmol) der Verbindung aus Beispiel 60A und 66 mg (0.24 mmol) der Verbindung aus Beispiel 23A wurden in Analogie zu Beispiel 48A miteinander umgesetzt. Abweichend von der dort beschriebenen Aufarbeitung wurde der Ansatz hier direkt mittels präparativer HPLC (Methode 21) in seine Bestandteile aufgetrennt. Die produkthaltigen Fraktionen wurden im Vakuum eingeengt und der Rückstand im Hochvakuum getrocknet. Es wurden 84 mg (64% d. Th., 80% Reinheit) der Titelverbindung erhalten.

LC/MS (Methode 4, ESIpos): Rₜ = 1.21 min, m/z = 654 [M+H]⁺.

### Beispiel 76A

### 3-Cyano-N-(5-{6-[1-(4-methoxybenzyl)-1H-pyrazol-4-yl]-1H-imidazo[1,2-b]pyrazol-1-yl}-2,4-dimethylphenyl)-5-(pentafluor-λ⁶-sulfanyl)benzamid

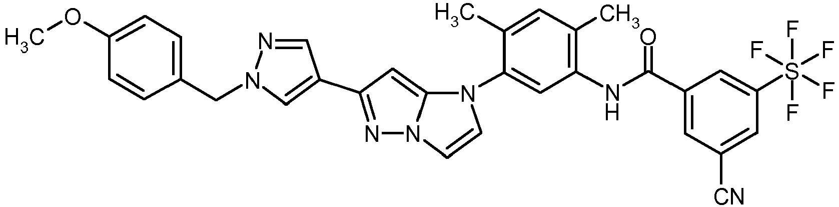

105 mg (0.26 mmol) der Verbindung aus Beispiel 61A und 82 mg (0,24 mmol) der Verbindung aus Beispiel 23A wurden in Analogie zu Beispiel 48A miteinander umgesetzt. Abweichend von der dort beschriebenen Aufarbeitung wurde der Ansatz hier direkt mittels präparativer HPLC (Methode 21) in seine Bestandteile aufgetrennt. Die produkthaltigen Fraktionen wurden im Vakuum eingeengt und der Rückstand im Hochvakuum getrocknet. Es wurden 125 mg (71% d. Th.) der Titelverbindung erhalten.

¹H-NMR (400 MHz, DMSO-d₆, δ/ppm): 10.40 (s, 1H), 8.86 (s, 1H), 8.73 (s, 1H), 8.66 (s, 1H), 8.04 (s, 1H), 7.75 (d, 1H), 7.74 (s, 1H), 7.48 (s, 1H), 7.37 (d, 2H), 7.23 (d, 2H), 6.90 (d, 2H), 5.85 (s, 1H), 5.24 (s, 2H), 3.72 (s, 3H), 2.30 (s, 3H), 2.24 (s, 3H).

LC/MS (Methode 4, ESIpos): Rₜ = 1.20 min, m/z = 668 [M+H]⁺.

### Beispiel 77A

### N-{2,4-Dimethyl-5-[6-(pyridin-3-yl)-1H-imidazo[1,2-b]pyrazol-1-yl]phenyl}-3-formyl-5-(pentafluor-λ⁶-sulfanyl)benzamid

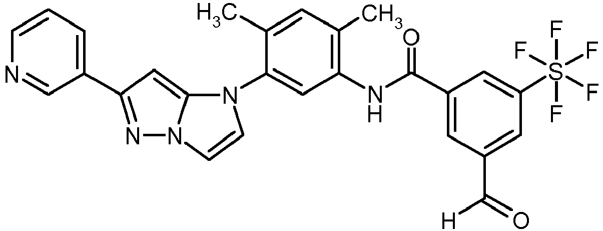

214 mg (0.659 mmol, 85% Reinheit) der Verbindung aus Beispiel 36A und 301 mg (0.791 mmol) HATU wurden in 2.3 ml wasserfreiem DMF gelöst und mit 140 µl (0.791 mmol) *N,N*-Diisopropylethylamin versetzt. Nach 30 min wurden 200 mg (0.659 mmol) der Verbindung aus Beispiel 11A hinzugefügt. Nachdem das Reaktionsgemisch 1.5 h bei RT gerührt worden war, wurden ca. 50 ml Wasser zugesetzt, und es wurde dreimal mit je ca. 50 ml Ethylacetat extrahiert. Die vereinigten organischen Extrakte wurden mit gesättigter Natriumchlorid-Lösung gewaschen, über wasserfreiem Magnesiumsulfat getrocknet, filtriert und am Rotationsverdampfer vom Lösungsmittel befreit. Das so erhaltene Rohprodukt wurde mittels MPLC gereinigt (30 g Kieselgel, Laufmittelgradient Cyclohexan/Ethylacetat 1:1 → 1:5). Nach Eindampfen der Produktfraktionen und Trocknen des Rückstands im Hochvakuum wurden 160 mg (36% d. Th., 85% Reinheit) der Titelverbindung erhalten.

LC/MS (Methode 3, ESIpos): Rₜ = 0.98 min, m/z = 562 [M+H]⁺.

### Beispiel 78A

### 1-(2,2-Dicthoxyethyl)-3-(pyrimidin-5-yl)-1H-pyrazol-5-amin

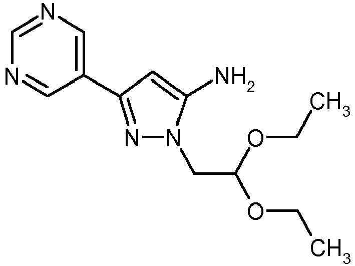

In Analogie zu Beispiel 59A / Schritte 1 und 2 wurden aus 5.0 g (32.9 mmol) 5-Pyrimidincarbon-säureethylester 3.01 g der Titelverbindung erhalten (27% d. Th. über beide Stufen).

¹H-NMR (400 MHz, DMSO-d₆, δ/ppm): 9.01 (s, 2H), 5.85 (s, 1H), 5.71 (s, 1H), 5.33 (s, 2H), 4.82 (t, 1H), 4.00 (d, 2H), 3.53-3.67 (m, 2H), 3.41 (dd, 2H), 1.04 (t, 6H).

LC/MS (Methode 7, ESIpos): Rₜ = 0.75 min, m/z = 278 [M+H]⁺.

### Beispiel 79A

### 1-(2,2-Diethoxyethyl)-3-(pyridazin-4-yl)-1H-pyrazol-5-amin

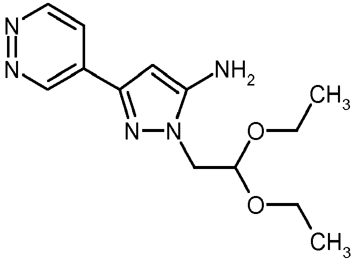

In Analogie zu Beispiel 59A / Schritte 1 und 2 wurden aus 5.0 g (32.9 mmol) Pyridazin-4-carbon-säureethylester 4.5 g der Titelverbindung erhalten (49% d. Th. über beide Stufen).

¹H-NMR (400 MHz, DMSO-d₆, δ/ppm): 9.46 (dd, 1H), 9.10 (dd, 1H), 7.79 (dd, 1H), 5.94 (s, 1H), 5.42 (s, 2H), 4.83 (t, 1H), 4.02 (d, 2H), 3.55-3.68 (m, 2H), 3.33-3.46 (m, 3H), 1.03 (t, 6H).

LC/MS (Methode 7, ESIpos): Rₜ = 0.75 min, m/z = 278 [M+H]⁺.

### Beispiel 80A

### 1-(2,2-Diethoxyethyl)-3-(pyridazin-3-yl)-1H-pyrazol-5-amin

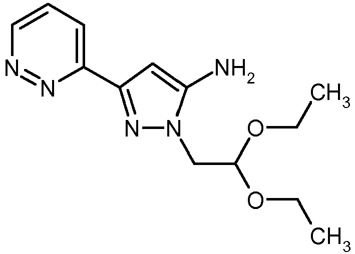

In Analogie zu Beispiel 59A / Schritte 1 und 2 wurden aus 2.5 g (16.4 mmol) Pyridazin-3-carbon-säureethylester 1.09 g der Titelverbindung erhalten (24% d. Th. über beide Stufen).

¹H-NMR (400 MHz, DMSO-d₆, δ/ppm): 9.05 (dd, 1H), 7.97 (dd, 1H), 7.60 (dd, 1H), 6.00 (s, 1H), 5.32 (s, 2H), 4.83 (t, 1H), 4.02 (d, 2H), 3.57-3.68 (m, 2H), 3.36-3.46 (m, 2H), 1.04 (t, 6H).

LC/MS (Methode 7, ESIpos): Rₜ = 0.76 min, m/z = 278 [M+H]⁺.

### Beispiel 81A

### 1-(2,2-Diethoxyethyl)-3-(1,3-thiazol-5-yl)-1H-pyrazol-5-amin

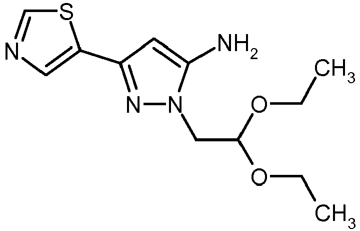

### Schritt 1: Natrium-2-cyano-1-(1,3-thiazol-5-yl)ethenolat

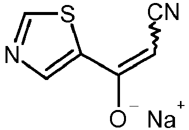

Zu einer Suspension von 419 mg Natriumhydrid (60%-ige Suspension in Mineralöl) in 16 ml THF, die unter Rückfluss erhitzt wurde, wurde eine Lösung von 1.5 g (10.5 mmol) Methyl-1,3-thiazol-5-carboxylat und 430 mg (10.8 mmol) Acetonitril in 15 ml THF getropft. Das Reaktionsgemisch wurde 20 h unter Rückfluss erhitzt. Nach dem Abkühlen wurden 50 ml Methyl-tert.-butylether hinzugegeben und das Gemisch für 30 Minuten gerührt. Der dabei entstandene Niederschlag wurde über eine Fritte abgesaugt und im Ölpumpenvakuum getrocknet. Man erhielt auf diese Weise 1.71 g (94% d. Th.) der Titelverbindung, welche mit dem Produkt (3.48 g, 95% d. Th.) aus einem zweiten Ansatz, ausgehend von 3.0 g (21 mmol) Methyl-1,3-thiazol-5-carboxylat, vereinigt wurde. Dieses Material wurde ohne weitere Charakterisierung in der nächsten Stufe eingesetzt.

### Schritt 2: 1-(2,2-Dicthoxyethyl)-3-(1,3-thiazol-5-yl)-1H-pyrazol-5-amin

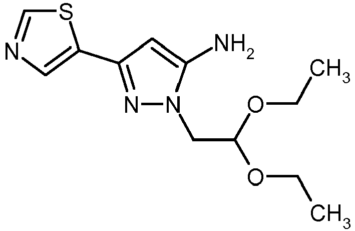

5.19 g (29.8 mmol) des Natrium-Salzes aus Beispiel 81A / Schritt 1 wurden in 30 ml Ethanol suspendiert und nacheinander mit 4.64 g (31.3 mmol) (2,2-Diethoxyethyl)hydrazin, 1.7 ml (29.8 mmol) Essigsäure und 149 µl 1 M Salzsäure versetzt. Nach zweistündigem Erhitzen unter Rückfluss wurde die Reaktionsmischung auf RT abgekühlt und mit 400 ml Ethylacetat verdünnt. Die organische Phase wurde jeweils einmal mit je 50 ml gesättigter Natriumhydrogencarbonat-Lösung, Wasser und gesättigter Natriumchlorid-Lösung gewaschen, über Natriumsulfat getrocknet und nach Filtration im Vakuum eingeengt. Das so erhaltene Rohprodukt wurde über eine Biotage-Anlage gereinigt (100 g Snap-Säule; Laufmittelgradient Ethylacetat/0-8% Methanol). Man erhielt auf diese Weise 2.93 g (34% d. Th.) der Titelverbindung.

¹H-NMR (400 MHz, DMSO-d₆, δ/ppm): 8.88 (s, 1H), 8.02 (s, 1H), 5.65 (s, 1H), 5.28 (s, 2H), 4.76 (t, 1H), 3.93 (d, 2H), 3.53-3.68 (m, 2H), 3.31-3.46 (m, 2H), 1.03 (t, 6H).

LC/MS (Methode 7, ESIpos): Rₜ = 0.85 min, m/z = 283 [M+H]⁺.

### Beispiel 82A

### 6-Amino-3-methyl-2-[6-(pyridin-3-yl)-1H-imidazo[1,2-b]pyrazol-1-yl]phenol

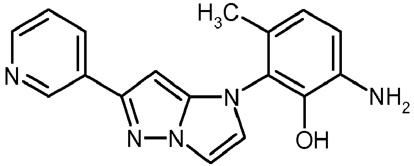

### Schritt 1: Methyl-2-brom-3-hydroxy-4-nitrobenzoat

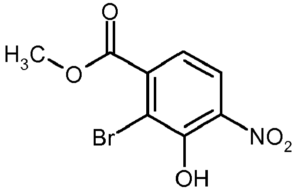

Eine Lösung von 1 ml (20.3 mmol) Brom in 5 ml Dichlormethan wurde bei -78°C langsam zu einer Lösung von 4.3 ml (40.6 mmol) *tert.-Butylamin* in 35 ml Dichlormethan getropft. Nach beendeter Brom-Zugabe wurde das Gemisch noch eine weitere Stunde bei -78°C gerührt, bevor eine Lösung von 4.0 g (20.3 mmol) Methyl-3-hydroxy-4-nitrobenzoat in 5 ml Dichlormethan hinzugefügt wurde. Das Reaktionsgemisch wurde danach im Verlaufe von ca. 16 h langsam auf RT erwärmt. Dabei fiel ein Feststoff aus, der abgesaugt und mit je ca. 25 ml Dichlormethan und 2 M Salzsäure verrührt wurde. Die Dichlormethan-Phase wurde abgetrennt, über Magnesiumsulfat getrocknet, filtriert und eingedampft ("Rückstand 1"). Ebenso wurde das zuvor erhaltene Filtrat des Reaktionsgemisches am Rotationsverdampfer eingedampft ("Rückstand 2"). Rückstand 1 wurde in 5 Portionen und Rückstand 2 in 2 Portionen mittels präparativer HPLC gereinigt (Methode 33). Die Produktfraktionen wurden, getrennt nach Rückstand 1 und 2, vereinigt, eingedampft und im Hochvakuum getrocknet. Aus Rückstand 1 wurden 1.79 g (31% d. Th.) eines Gemisches aus der Titelverbindung und dem isomeren Methyl-2-brom-5-hydroxy-4-nitrobenzoat im Verhältnis 85:15 erhalten; aus Rückstand 2 wurden 0.77 g (13% d. Th.) des gleichen Gemisches, jedoch im Verhältnis 73:27, gewonnen.

¹H-NMR (400 MHz, DMSO-d₆, δ/ppm): 8.03 (d, 1H), 7.28 (d, 1H), 3.89 (s, 3H).

LC/MS (Methode 3, ESIneg): Rₜ = 0.90 min, m/z = 274/276 [M-H]⁻ (⁷⁹Br/⁸¹Br).

### Schritt 2: Methyl-3-(benzyloxy)-2-brom-4-nitrobenzoat

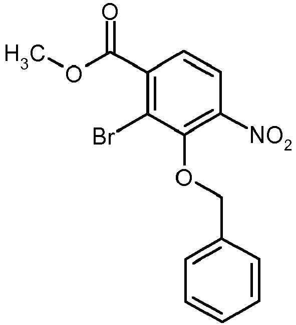

1.79 g (6.48 mmol) der Verbindung aus Beispiel 82A / Schritt 1 (Produktgemisch aus "Rückstand 1") wurden zusammen mit 0.81 ml (6.80 mmol) Benzylbromid und 0.99 g (7.12 mmol) Kaliumcarbonat in 30 ml Acetonitril 2 h unter Rückfluss erhitzt. Nach dem Abkühlen auf RT wurde vom Feststoff abfiltriert und das Filtrat im Vakuum eingedampft. Der verbliebene Rückstand wurde in ca. 75 ml Ethylacetat aufgenommen und nacheinander mit Wasser und gesättigter wässriger Natriumchlorid-Lösung gewaschen. Nach Trocknen über Magnesiumsulfat wurde filtriert und das Filtrat zur Trockene eingeengt. Die Titelverbindung wurde mittels MPLC isoliert (Kieselgel, Laufmittel Cyclohexan/Ethylacetat 5:1). Es wurden 1.95 g (82% d. Th.) der Titelverbindung erhalten.

¹H-NMR (400 MHz, CDCl₃, δ/ppm): 7.79 (d, 1H), 7.57-7.53 (m, 3H), 7.43-7.37 (m, 3H), 5.21 (s, 2H), 3.99 (s, 3H).

LC/MS (Methode 3, ESIneg): Rₜ = 1.20 min, m/z = 364/366 [M-H]⁻ (⁷⁹Br/⁸¹Br).

### Schritt 3: [3-(Benzyloxy)-2-brom-4-nitrophenyl]methanol

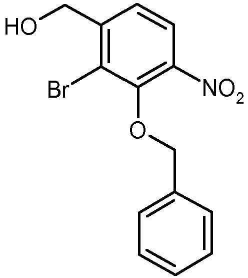

Eine Lösung von 1.95 g (5.32 mmol) der Verbindung aus Beispiel 82A / Schritt 2 in 40 ml wasserfreiem THF wurde bei -78°C tropfenweise mit 2.9 ml (2.93 mmol) einer 1 M Lösung von Lithiumaluminiumhydrid in THF versetzt. Nach beendeter Zugabe wurde das Kältebad entfernt, das Reaktionsgemisch auf RT erwärmt und das Rühren noch 1 h bei RT fortgesetzt. Dann wurden vorsichtig ca. 5 ml gesättigte wässrige Ammoniumchlorid-Lösung zugesetzt. Es wurde mit Ethylacetat verdünnt und über Magnesiumsulfat getrocknet. Nach Filtration und Abdampfen des Lösungsmittels wurde der erhaltene Rückstand mittels MPLC gereinigt (Kieselgel, Laufmittel Cyclohexan/Ethyl-acetat 5:1). Es wurden 1.12 g (62% d. Th.) der Titelverbindung erhalten.

¹H-NMR (400 MHz, DMSO-d₆, δ/ppm): 8.04 (d, 1H), 7.56 (d, 1H), 7.50 (d, 2H), 7.44-7.37 (m, 3H), 5.75 (t, 1H), 5.11 (s, 2H), 4.59 (d, 2H).

### Schritt 4: 2-(Benzyloxy)-3-brom-4-(brommethyl)-1-nitrobenzol

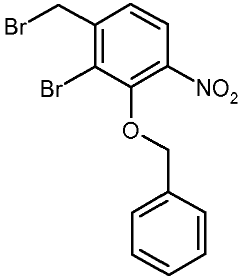

1.02 g (3.02 mmol) der Verbindung aus Beispiel 82A / Schritt 3 wurden 18 ml THF gelöst und mit 0.95 g (3.62 mmol) Triphenylphosphin versetzt. Nachdem dieses in Lösung gegangen war, wurden 1.20 g (3.62 mmol) Tetrabrommethan hinzugefügt und das Reaktionsgemisch ca. 20 h bei RT gerührt. Dabei fiel ein feiner weißer Niederschlag aus. Die Fällung wurde durch Zugabe von 50 ml Cyclohexan vervollständigt. Anschließend wurde filtriert und das Filtrat am Rotationsverdampfer bis zur Trockene eingedampft. Das Produkt wurde aus diesem Rückstand mittels MPLC isoliert (Kieselgel, Laufmittel Cyclohexan/Ethylacetat 10:1). Es wurden 1.01 g (83% d. Th.) der Titelverbindung erhalten.

¹H-NMR (400 MHz, DMSO-d₆, δ/ppm): 8.01 (d, 1H), 7.68 (d, 1H), 7.49 (d, 2H), 7.45-7.39 (m, 3H), 5.12 (s, 2H), 4.83 (s, 2H).

### Schritt 5: 2-(Benzyloxy)-3-brom-4-methyl-1-nitrobenzol

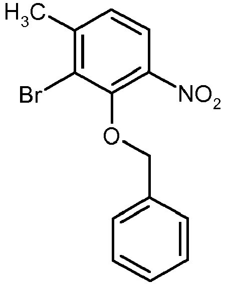

Eine Lösung von 1.0 g (2.50 mmol) der Verbindung aus Beispiel 82A / Schritt 4 in 25 ml wasserfreiem THF wurde bei -78°C tropfenweise mit 0.75 ml (0.75 mmol) einer 1 M Lösung von Lithiumaluminiumhydrid in THF versetzt. Nach beendeter Zugabe wurde das Kältebad entfernt, das Reaktionsgemisch auf RT erwärmt und das Rühren noch 1 h bei RT fortgesetzt. Da die Umsetzung laut DC-Kontrolle nicht vollständig war, wurde erneut auf -78°C abgekühlt und mit weiteren 0.75 ml (0.75 mmol) der 1 M Lösung von Lithiumaluminiumhydrid in THF versetzt. Nach Erwärmen auf RT wurde wiederum 1 h bei RT nachgerührt, bevor vorsichtig ca. 5 ml gesättigte wässrige Ammoniumchlorid-Lösung zugesetzt wurden. Es wurde mit Ethylacetat verdünnt und über Magnesiumsulfat getrocknet. Nach Filtration und Abdampfen des Lösungsmittels wurde der erhaltene Rückstand mittels MPLC gereinigt (Kieselgel, Laufmittel Cyclohexan/Ethylacetat 10:1). Nach Einengen der Produktfraktionen und Trocknen des Rückstands im Hochvakuum wurden 622 mg (77% d. Th.) der Titelverbindung erhalten.

¹H-NMR (400 MHz, DMSO-d₆, δ/ppm): 7.93 (d, 1H), 7.50 (d, 2H), 7.45-7.39 (m, 4H), 5.10 (s, 2H), 2.54 (s, 3H).

MS (DCI, NH₃): m/z = 339/341 [M-H]⁻ (⁷⁹Br/⁸¹Br).

### Schritt 6: N-[2-(Benzyloxy)-6-methyl-3-nitrophenyl]-1-(2,2-diethoxyethyl)-3-(pyridin-3-yl)-1H-pyrazol-5-amin und

### 2-{[1-(2,2-Diethoxyethyl)-3-(pyridin-3-yl)-1H-pyrazol-5-yl]amino}-3-methyl-6-nitrophenol

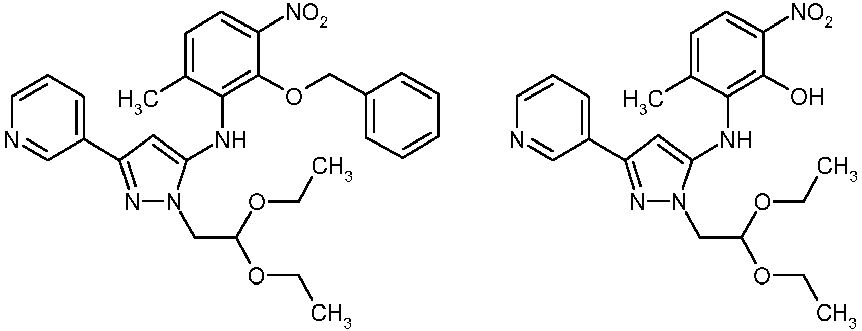

452 mg (1.64 mmol) der Verbindung aus Beispiel 82A / Schritt 5 wurden zusammen mit 580 mg (1.80 mmol) der Verbindung aus Beispiel 4A, 37 mg (0.164 mmol) Palladium(II)acetat, 142 mg (0.245 mmol) Xantphos [4,5-Bis-(diphenylphosphino)-9,9-dimethylxanthen] und 1.6 g (4.91 mmol) Cäsiumcarbonat in 10 ml 1,4-Dioxan in einem Mikrowellenofen (Biotage Initiator, mit dynamischer Steuerung der Einstrahlleistung) für 1 h auf 140°C erhitzt. Anschließend wurde über Kieselgur filtriert und das Filtrat am Rotationsverdampfer eingedampft. Das so erhaltene Rohprodukt wurde mittels MPLC über Kieselgel gereinigt (Laufmittelgradient Cyclohexan/Ethylacetat 10:1 → 1:1). Die Produktfraktionen wurden vereinigt und am Rotationsverdampfer vom Lösungsmittel befreit. Es wurden 230 mg (27% d. Th.) eines Gemisches der beiden Titelverbindungen erhalten, das als solches in der Folgereaktion eingesetzt wurde.

LC/MS (Methode 3, ESIpos): Rₜ = 1.13 min, m/z = 518 [M+H]⁺ (Benzylether);

LC/MS (Methode 3, ESIpos): Rₜ = 0.97 min, m/z = 428 [M+H]⁺ (Phenol).

### Schritt 7: 1-[2-(Benzyloxy)-6-methyl-3-nitrophenyl]-6-(pyridin-3-yl)-1H-imidazo[1,2-b]pyrazol und

### 3-Methyl-6-nitro-2-[6-(pyridin-3-yl)-1H-imidazo[1,2-b]pyrazol-1-yl]phenol

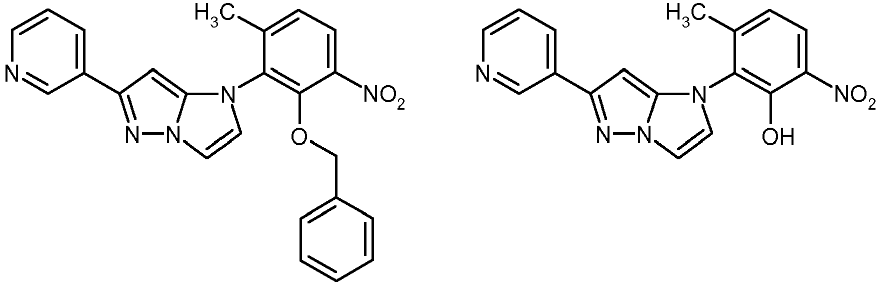

Eine Lösung von 230 mg (0.444 mmol) des Produktgemisches aus Beispiel 82A / Schritt 6 in 2 ml Ethanol und 533 µl (1.07 mmol) 2 M Schwefelsäure wurde in einem Mikrowellenofen (Biotage Initiator, mit dynamischer Steuerung der Einstrahlleistung) für 30 min auf 130°C erhitzt. Der Ansatz wurde danach mit 4 ml DMF verdünnt und in 2 Portionen mittels präparativer HPLC (Methode 36) in seine Komponenten aufgetrennt. Dabei wurden auch das Benzylether- und das Phenol-Produkt voneinander getrennt. Nach Vereinigen der jeweiligen Produktfraktionen wurde das Lösungsmittel am Rotationsverdampfer entfernt. Abschließendes Trocknen im Hochvakuum ergab 23 mg (12% d. Th.) des Benzylethers sowie 63 mg (42% d. Th.) des Phenols.

1-[2-(Benzyloxy)-6-methyl-3-nitrophenyl]-6-(pyridin-3-yl)-1*H*-imidazo[1,2-b]pyrazol:

LC/MS (Methode 3, ESIpos): Rₜ = 0.95 min, m/z = 426 [M+H]⁺.

### 3-Methyl-6-nitro-2-[6-(pyridin-3-yl)-1H-imidazo[1,2-b]pyrazol-1-yl]phenol:

LC/MS (Methode 3, ESIpos): Rₜ = 0.73 min, m/z = 336 [M+H]⁺.

### Schritt 8: 6-Amino-3-methyl-2-[6-(pyridin-3-yl)-1H-imidazo[1,2-b]pyrazol-1-yl]phenol

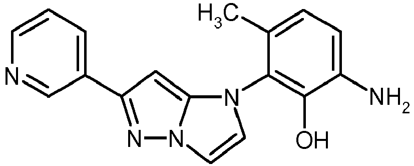

Das Benzylether- und das Phenol-Produkt aus Beispiel 82A / Schritt 7 wurden wieder vereinigt. 85 mg (0.253 mmol) dieses Gemisches wurden in 1.7 ml Ethanol und 0.8 ml Wasser gelöst. Es wurden 80 mg (1.27 mmol) Ammoniumformiat und 3.3 mg Palladium (10% auf Aktivkohle, 0.003 mmol) hinzugefügt. Das Gemisch wurde danach ca. 20 h unter Rückfluss erhitzt. Da die Umsetzung nach dieser Zeit noch nicht vollständig war, wurde filtriert und 10 mg frisches Palladium (10% auf Aktivkohle, 0.01 mmol) zum Filtrat hinzugefügt. Es wurde anschließend ca. 40 h bei RT unter einer Atmosphäre von 1 bar Wasserstoff hydriert. Danach wurde das Reaktionsgemisch über wenig Kieselgur filtriert und das Filtrat zur Trockene eingedampft. Der Rückstand wurde mittels präparativer HPLC (Methode 37) gereinigt. Nach Eindampfen der Produktfraktionen und Trocknen im Hochvakuum wurden 40 mg (51% d. Th.) der Titelverbindung erhalten.

LC/MS (Methode 3, ESIpos): Rₜ = 0.54 min, m/z = 306 [M+H]⁺.

### Beispiel 83A

### 3-[7-Fluor-6-(pyridin-3-yl)-1H-imidazo[1,2-b]pyrazol-1-yl]-4-methylanilin

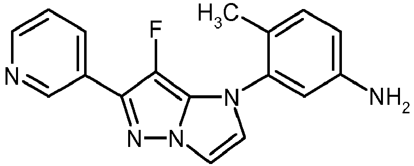

### Schritt 1: 7-Fluor-1-(2-methyl-5-nitrophenyl)-6-(pyridin-3-yl)-1H imidazo[1,2-b]pyrazol

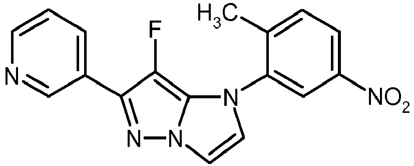

Eine Suspension von 1.15 g (3.60 mmol) der Verbindung aus Beispiel 6A / Schritt 2 in 34.5 ml Acetonitril wurde bei 0°C mit 1.91 g (5.40 mmol) 1-Chlormethyl-4-fluor-1,4-diazabicyclo[2.2.2]-octan-bis(tetrafluoroborat) versetzt und 45 min bei dieser Temperatur gerührt. Dabei entstand eine klare Lösung. Anschließend wurde das Reaktionsgemisch mit ca. 100 ml Wasser und 100 ml Ethylacetat versetzt und mit gesättigter wässriger Natriumhydrogencarbonat-Lösung schwach basisch gestellt. Nach Phasentrennung wurde die wässrige Phase noch zweimal mit Ethylacetat extrahiert. Die vereinigten organischen Extrakte wurden nacheinander mit Wasser und gesättigter Natriumchlorid-Lösung gewaschen. Nach Trocknen über Magnesiumsulfat wurde filtriert und das Filtrat am Rotationsverdampfer zur Trockene eingedampft. Der verbliebene Rückstand wurde mittels MPLC über ca. 70 g Kieselgel mit einem Laufmittelgradienten Dichlormethan/Ethylacetat/ Methanol 1:1:0 → 1:2:0 → 10:0:1 gereinigt. Nach Vereinigen der Produktfraktionen, Abdampfen des Lösungsmittels und Trocknen des Rückstands im Hochvakuum wurden 400 mg (32% d. Th.) der Titelverbindung erhalten.

¹H-NMR (400 MHz, DMSO-d₆, δ/ppm): 8.98 (dd, 1H), 8.56 (dd, 1H), 8.37 (d, 1H), 8.27 (dd, 1H), 8.14 (dt, 1H), 7.98 (dd, 1H), 7.79 (d, 1H), 7.70 (d, 1H), 7.50 (dd, 1H), 2.47 (s, 3H).

LC/MS (Methode 3, ESIpos): Rₜ = 0.90 min, m/z = 338 [M+H]⁺.

### Schritt 2: 3-[7-Fluor-6-(pyridin-3-yl)-1H-imidazo[1,2-b]pyrazol-1-yl]-4-methylanilin

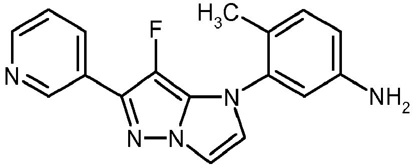

400 mg (1.18 mmol) der Verbindung aus Beispiel 83A / Schritt 1 wurden in 8 ml Ethanol und 0.8 ml Wasser gelöst. Es wurden 374 mg (5.93 mmol) Ammoniumformiat und 15 mg (0.014 mmol) Palladium (10% auf Aktivkohle) hinzugefügt. Das Gemisch wurde 2.5 h unter Rückfluss erhitzt. Nach dem Abkühlen auf RT wurde über wenig Celite filtriert und der größte Teil des Lösungsmittels am Rotationsverdampfer entfernt. Der verbliebene Rückstand wurde in Dichlormethan aufgenommen, mit festem Magnesiumsulfat versetzt, gerührt und nach einer Weile filtriert. Das Filtrat wurde eingeengt und in 2 Portionen mittels präparativer HPLC in seine Komponenten aufgetrennt (Methode 37). Die Produktfraktionen wurden vereinigt und eingedampft. Der Rückstand wurde in wenig Methanol gelöst und über eine Hydrogencarbonat-Kartusche gegeben (Fa. Polymerlabs, Stratospheres SPE, PL-HCO₃ MP SPE, Kapazität 0.9 mmol). Nach erneutem Eindampfen und Trocknen im Hochvakuum wurden 191 mg (52% d. Th.) der Titelverbindung erhalten.

¹H-NMR (400 MHz, CDCl₃, δ/ppm: 9.15 (m, 1H), 8.55 (dd, 1H), 8.19 (dt, 1H), 7.38 (m, 1H), 7.36 (dd, 1H), 7.13 (d, 1H), 6.89 (d, 1H), 6.70-6.68 (m, 2H), 2.22 (s, 3H).

LC/MS (Methode 3, ESIpos): Rₜ = 0.68 min, m/z = 308 [M+H]⁺.

### Beispiel 84A

### 4-Chlor-2-methyl-5-[6-(pyridin-3-yl)-1H-imidazo[1,2-b]pyrazol-1-yl]anilin

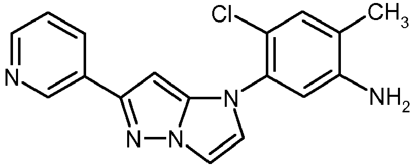

### Schritt 1: N-(2-Chlor-4-methyl-5-nitrophenyl)-1-(2,2-diethoxyethyl)-3-(pyridin-3-yl)-1H-pyrazol-5-amin

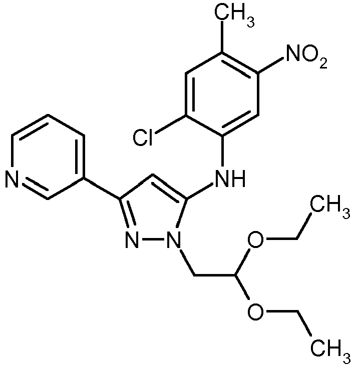

In Analogie zu Beispiel 13A / Schritt 1 wurde aus zwei Ansätzen (273 mg und 1.58 g; insgesamt 6.72 mmol) der Verbindung aus Beispiel 4A sowie 250 mg und 1.45 g (insgesamt 7.4 mmol) 1-Brom-2-chlor-4-methyl-5-nitrobenzol nach 2 h Erhitzen unter Rückfluss ein Rohprodukt erhalten, welches durch zweimalige Chromatographie mittels einer Biotage-Anlage gereinigt wurde (jeweils 50 g Snap-Säule; Laufmittelgradient Hexan/Ethylacetat, von 20% Ethylacetat bis 100% Ethylacetat schnell ansteigend, dann Ethylacetat/Methanol, langsam auf 100% Methanol ansteigend). Auf diese Weise wurden 1.96 g (69% d. Th.) der Titelverbindung erhalten.

¹H-NMR (400 MHz, DMSO-d₆, δ/ppm): 8.99 (d, 1H), 8.48 (dd, 1H), 8.13 (dt, 1H), 7.86 (s, 1H), 7.61 (s, 1H), 7.45 (s, 1H), 7.41 (ddd, 1H), 6.84 (s, 1H), 4.83 (t, 1H), 4.15 (d, 2H), 3.60 (dq, 2H), 3.43 (dq, 2H), 2.39 (s, 3H), 0.99 (t, 6H).

LC/MS (Methode 7, ESIpos): Rₜ = 1.31 min, m/z = 446/448 [M+H]⁺ (³⁵Cl/³⁷Cl).

### Schritt 2: 1-(2-Chlor-4-methyl-5-nitrophenyl)-6-(pyridin-3-yl)-1H-imidazo[1,2-b]pyrazol

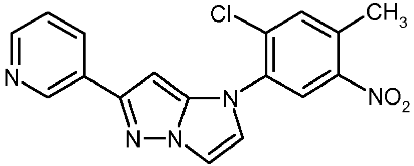

In Analogie zu Beispiel 13A / Schritt 2 wurden aus 2.32 g (5.2 mmol) der unter Beispiel 84A / Schritt 1 hergestellten Verbindung nach zweimaliger Chromatographie mittels einer Biotage-Anlage (jeweils 50 g Snap-Säule; Laufmittelgradient Hexan/Ethylacetat, von 20% Ethylacetat bis 100% Ethylacetat schnell ansteigend, dann Ethylacetat/Methanol, langsam auf 100% Methanol ansteigend) 420 mg (10% d. Th.) der Titelverbindung erhalten.

¹H-NMR (400 MHz, DMSO-d₆, δ/ppm): 9.00 (d, 1H), 8.46 (dd, 1H), 8.30 (s, 1H), 8.14 (dt, 1H), 7.97 (s, 1H), 7.94 (d, 1H), 7.59 (d, 1H), 7.39 (dd, 1H), 6.40 (s, 1H), 2.57 (s, 3H).

LC/MS (Methode 7, ESIpos): Rₜ = 0.96 min, m/z = 354/356 [M+H]⁺ (³⁵Cl/³⁷Cl).

### Schritt 3: 4-Chlor-2-methyl-5-[6-(pyridin-3-yl)-1H-imidazo[1,2-b]pyrazol-1-yl]anilin

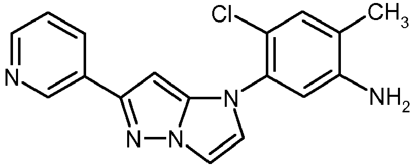

In Analogie zu Beispiel 13A / Schritt 3 wurden aus 420 mg (1.2 mmol) der unter Beispiel 84A / Schritt 2 hergestellten Verbindung 395 mg (102% d. Th.) der Titelverbindung als noch stark verunreinigtes Rohprodukt erhalten, welches ohne weitere Reinigung in Folgereaktionen eingesetzt wurde.

LC/MS (Methode 7, ESIpos): Rₜ = 0.85 min, m/z = 324/326 [M+H]⁺ (³⁵Cl/³⁷Cl).

### Beispiel 85A

### 4-Methyl-3-[6-(pyrimidin-5-yl)-1H-imidazo[1,2-b]pyrazol-1-yl]anilin

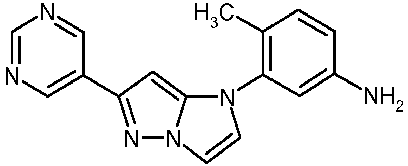

### Schritt 1: 1-(2,2-Diethoxyethyl)-N-(2-methyl-5-nitrophenyl)-3-(pyrimidin-5-yl)-1H-pyrazol-5-amin

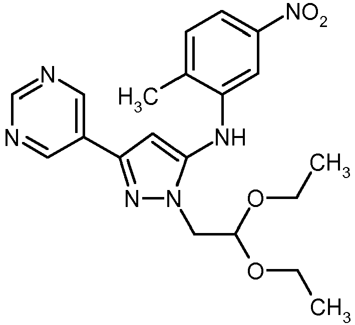

In Analogie zu Beispiel 13A / Schritt 1 wurde aus 2.22 g (8.01 mmol) der Verbindung aus Beispiel 78A und 1.90 g (8.81 mmol) 2-Brom-4-nitrotoluol nach 3 h Erhitzen unter Rückfluss ein Rohprodukt erhalten, welches durch Chromatographie mittels einer Biotage-Anlage gereinigt wurde (100 g Snap-Säule; Laufmittelgradient Ethylacetat/0-8% Methanol). Auf diese Weise wurden 1.93 g (49% d. Th.) der Titelverbindung erhalten.

¹H-NMR (400 MHz, DMSO-d₆, δ/ppm): 9.18 (s, 2H), 9.10 (s, 1H), 7.58-7.64 (m, 2H), 7.48 (d, 1H), 7.40 (d, 1H), 6.84 (s, 1H), 4.87 (t, 1H), 4.17 (d, 2H), 3.52-3.63 (m, 2H), 3.35-3.45 (m, 2H), 2.35 (s, 3H), 0.97 (t, 6H).

LC/MS (Methode 7, ESIpos): Rₜ = 1.22 min, m/z = 413 [M+H]⁺.

### Schritt 2: 1-(2-Methyl-5-nitrophenyl)-6-(pyrimidin-5-yl)-1H-imidazo[1,2-b]pyrazol

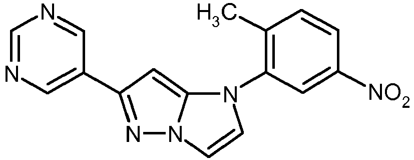

In Analogie zu Beispiel 13A / Schritt 2 wurden aus 1.93 g (4.7 mmol) der unter Beispiel 85A / Schritt 1 hergestellten Verbindung nach Chromatographie mittels einer Biotage-Anlage (50 g Snap-Säule; Laufmittelgradient Methylenchlorid/0-7% Methanol) 180 mg (11% d. Th.) der Titelverbindung erhalten.

¹H-NMR (400 MHz, DMSO-d₆, δ/ppm): 9.18 (s, 2H), 9.07 (s, 1H), 8.28 (d, 1H), 8.24 (dd, 1H), 7.98 (d, 1H), 7.76 (d, 1H), 7.66 (d, 1H), 6.53 (s, 1H), 2.39 (s, 3H).

LC/MS (Methode 7, ESIpos): Rₜ = 0.95 min, m/z = 321 [M+H]⁺.

### Schritt 3: 4-Methyl-3-[6-(pyrimidin-5-yl)-1H-imidazo[1,2-b]pyrazol-1-yl]anilin

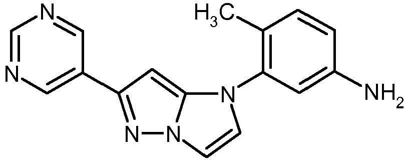

In Analogie zu Beispiel 13A / Schritt 3 wurden aus 169 mg (0.53 mmol) der unter Beispiel 85A / Schritt 2 hergestellten Verbindung 113 mg (63% d. Th., Reinheit ca. 85%) der Titelverbindung als Rohprodukt erhalten, welches nicht weiter aufgereinigt wurde.

¹H-NMR (400 MHz, DMSO-d₆, δ/ppm): 9.17 (s, 2H), 9.05 (s, 1H), 7.83 (dd, 1H), 7.43 (d, 1H), 7.03 (d, 1H), 6.54-6.61 (m, 2H), 6.38 (d, 1H), 5.19 (s, 2H), 2.03 (s, 3H).

LC/MS (Methode 7, ESIpos): Rₜ = 0.77 min, m/z = 291 [M+H]⁺.

### Beispiel 86A

### 2,4-Dimethyl-5-[6-(pyrimidin-5-yl)-1H-imidazo[1,2-b]pyrazol-1-yl]anilin

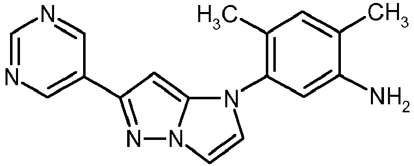

### Schritt 1: 1-(2,2-Diethoxyethyl)-N-(2,4-dimethyl-5-nitrophenyl)-3-(pyrimidin-5-yl)-1H-pyrazol-5-amin

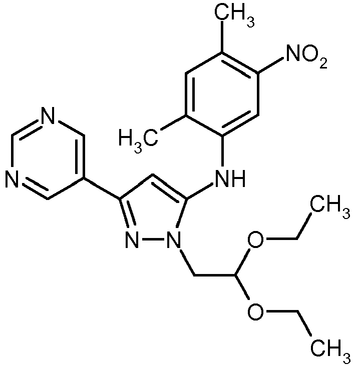

In Analogie zu Beispiel 13A / Schritt 1 wurde aus 3.01 g (10.8 mmol) der Verbindung aus Beispiel 78A und 2.75 g (11.9 mmol) 5-Brom-2,4-dimethylnitrobenzol nach 3 h Erhitzen unter Rückfluss ein Rohprodukt erhalten, welches durch Chromatographie mittels Biotage-Anlage gereinigt wurde (100 g Snap-Säule; Laufmittelgradient Ethylacetat/0-8% Methanol). Auf diese Weise wurden 3.70 g (65% d. Th.) der Titelverbindung erhalten.

¹H-NMR (400 MHz, DMSO-d₆, δ/ppm): 9.16 (s, 2H), 9.09 (s, 1H), 7.49 (s, 1H), 7.35 (s, 1H), 7.26 (s, 1H), 6.73 (s, 1H), 4.87 (t, 1H), 4.16 (d, 2H), 3.51-3.65 (m, 2H), 3.34-3.47 (m, 2H), 2.39 (s, 3H), 2.28 (s, 3H), 0.98 (t, 6H).

LC/MS (Methode 7, ESIpos): Rₜ = 1.31 min, m/z = 427 [M+H]⁺.

### Schritt 2: 1-(2,4-Dimethyl-5-nitrophenyl)-6-(pyrimidin-5-yl)-1H-imidazo[1,2-b]pyrazol

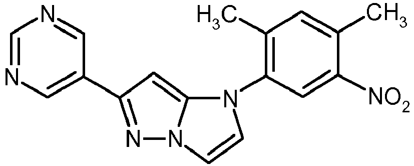

In Analogie zu Beispiel 13A / Schritt 2 wurde aus zwei Ansätzen zu je 1.85 g (4.3 mmol) der in Beispiel 86A / Schritt 1 hergestellten Verbindung ein vereinigtes Rohprodukt erhalten, das durch Chromatographie mittels einer Biotage-Anlage (100 g Snap-Säule; Laufmittelgradient Methylen-chlorid/0-7% Methanol) gereinigt wurde. Man erhielt auf diese Weise 502 mg (17% d. Th.) der Titelverbindung.

¹H-NMR (400 MHz, DMSO-d₆, δ/ppm): 9.17 (s, 2H), 9.07 (s, 1H), 8.09 (s, 1H), 7.95 (d, 1H), 7.59-7.63 (m, 2H), 6.50 (s, 1H), 2.55 (s, 3H), 2.31 (s, 3H).

LC/MS (Methode 7, ESIpos): Rₜ = 1.06 min, m/z = 335 [M+H]⁺.

### Schritt 3: 2,4-Dimethyl-5-[6-(pyrimidin-5-yl)-1H-imidazo[1,2-b]pyrazol-1-yl]anilin

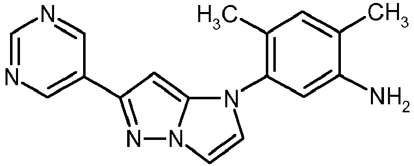

In Analogie zu Beispiel 13A / Schritt 3 wurden aus 498 mg (1.49 mmol) der in Beispiel 86A / Schritt 2 hergestellten Verbindung 331 mg (62% d. Th., Reinheit ca. 85%) der Titelverbindung als Rohprodukt erhalten, welches ohne weitere Aufreinigung eingesetzt wurde.

¹H-NMR (400 MHz, DMSO-d₆, δ/ppm): 9.17 (s, 2H), 9.05 (s, 1H), 7.82 (d, 1H), 7.40 (d, 1H), 6.93 (s, 1H), 6.63 (s, 1H), 6.35 (s, 1H), 4.96 (s, 2H), 2.05 (s, 3H), 2.00 (s, 3H).

LC/MS (Methode 7, ESIpos): Rₜ = 0.90 min, m/z = 305 [M+H]⁺.

### Beispiel 87A

### 4-Methyl-3-[6-(pyridazin-4-yl)-1H-imidazo[1,2-b]pyrazol-1-yl]anilin

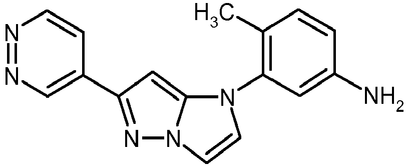

### Schritt 1: 1-(2,2-Diethoxyethyl)-N-(2-methyl-5-nitrophenyl)-3-(pyridazin-4-yl)-1H-pyrazol-5-amin

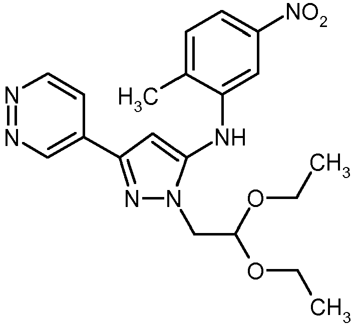

In Analogie zu Beispiel 13A / Schritt 1 wurde aus 4.5 g (16.2 mmol) der Verbindung aus Beispiel 79A und 3.86 g (17.8 mmol) 2-Brom-4-nitrotoluol nach 3 h Erhitzen unter Rückfluss ein Rohprodukt erhalten, welches durch Chromatographie mittels Biotage-Anlage gereinigt wurde (100 g Snap-Säule; Laufmittelgradient Ethylacetat/0-8% Methanol). Auf diese Weise wurden 5.52 g (82% d. Th.) der Titelverbindung erhalten.

¹H-NMR (400 MHz, DMSO-d₆, δ/ppm): 9.62 (dd, 1H), 9.20 (dd, 1H), 7.96 (dd, 1H), 7.65 (s, 1H), 7.62 (dd, 1H), 7.47 (d, 1H), 7.41 (d, 1H), 6.95 (s, 1H), 4.88 (t, 1H), 4.20 (d, 2H), 3.52-3.62 (m, 2H), 3.35-3.46 (m, 2H), 2.35 (s, 3H), 0.97 (t, 6H).

LC/MS (Methode 7, ESIpos): Rₜ = 1.19 min, m/z = 413 [M+H]⁺.

### Schritt 2: 1-(2-Methyl-5-nitrophenyl)-6-(pyridazin-4-yl)-1H-imidazo[1,2-b]pyrazol

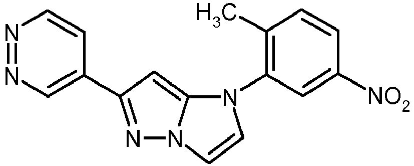

In Analogie zu Beispiel 13A / Schritt 2 wurde aus zwei Ansätzen zu je 2.76 g (6.7 mmol) der in Beispiel 87A / Schritt 1 hergestellten Verbindung ein vereinigtes Rohprodukt erhalten, das durch Chromatographie mittels einer Biotage-Anlage (100 g Snap-Säule; Laufmittelgradient Methylen-chlorid/0-7% Methanol) gereinigt wurde. Man erhielt auf diese Weise 1.56 g (36% d. Th.) der Titelverbindung.

¹H-NMR (400 MHz, DMSO-d₆, δ/ppm): 9.63 (dd, 1H), 9.17 (dd, 1H), 8.29 (d, 1H), 8.24 (dd, 1H), 8.01 (d, 1H), 7.96 (dd, 1H), 7.76 (d, 1H), 7.71 (d, 1H), 6.68 (s, 1H), 2.38 (s, 3H).

LC/MS (Methode 7, ESIpos): Rₜ = 0.91 min, m/z = 321 [M+H]⁺.

### Schritt 3: 4-Methyl-3-[6-(pyridazin-4-yl)-1H-imidazo[1,2-b]pyrazol-1-yl]anilin

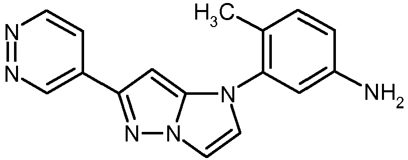

In Analogie zu Beispiel 13A / Schritt 3 wurden aus zwei Ansätzen zu 500 mg (1.56 mmol) bzw. 1.06 g (3.31 mmol) der in Beispiel 87A / Schritt 2 hergestellten Verbindung insgesamt 1.14 g (80% d. Th.) der Titelverbindung als Rohprodukt erhalten, welches nicht weiter gereinigt wurde.

¹H-NMR (400 MHz, DMSO-d₆, δ/ppm): 9.63 (dd, 1H), 9.15 (dd, 1H), 7.95-7.98 (m, 1H), 7.87 (dd, 1H), 7.49 (d, 1H), 7.04 (d, 1H), 6.56-6.61 (m, 2H), 6.53 (s, 1H), 5.20 (s, 2H), 2.03 (s, 3H).

LC/MS (Methode 7, ESIpos): Rₜ = 0.81 min, m/z = 291 [M+H]⁺.

### Beispiel 88A

### 2,4-Dimethyl-5-[6-(pyridazin-3-yl)-1H-imidazo[1,2-b]pyrazol-1-yl]anilin

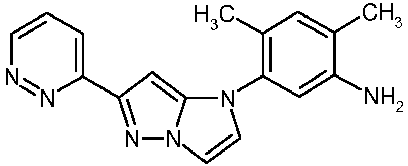

### Schritt 1: 1-(2,2-Diethoxyethyl)-N-(2,4-dimethyl-5-nitrophenyl)-3-(pyridazin-3-yl)-1H-pyrazol-5-amin

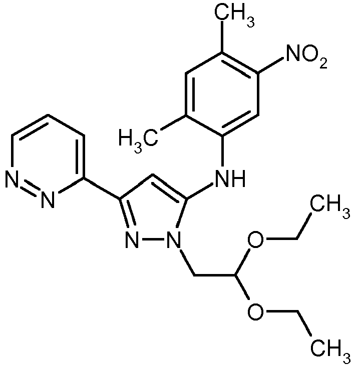

In Analogie zu Beispiel 13A / Schritt 1 wurde aus 1.09 g (3.93 mmol) der Verbindung aus Beispiel 80A und 996 mg (4.33 mmol) 5-Brom-2,4-dimethylnitrobenzol nach 3 h Erhitzen unter Rückfluss ein Rohprodukt erhalten, welches durch Chromatographie mittels einer Biotage-Anlage gereinigt wurde (100 g Snap-Säule; Laufmittelgradient Ethylacetat/0-8% Methanol). Auf diese Weise wurden 1.38 g (77% d. Th.) der Titelverbindung erhalten.

¹H-NMR (400 MHz, DMSO-d₆, δ/ppm): 9.13 (dd, 1H), 8.11 (dd, 1H), 7.70 (dd, 1H), 7.52 (s, 1H), 7.37 (s, 1H), 7.27 (s, 1H), 6.72 (s, 1H), 4.87 (t, 1H), 4.19 (d, 2H), 3.53-3.64 (m, 2H), 3.36-3.46 (m, 2H), 2.41 (s, 3H), 2.30 (s, 3H), 0.98 (t, 6H).

LC/MS (Methode 7, ESIpos): Rₜ = 1.28 min, m/z = 427 [M+H]⁺.

### Schritt 2: 1-(2,4-Dimethyl-5-nitrophenyl)-6-(pyridazin-3-yl)-1H-imidazo[1,2-b]pyrazol

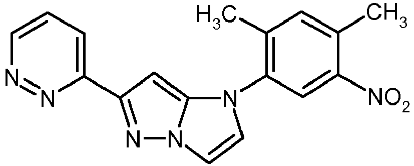

In Analogie zu Beispiel 13A / Schritt 2 wurden aus 1.38 g (3.24 mmol) der in Beispiel 88A / Schritt 1 hergestellten Verbindung nach Chromatographie mittels Biotage-Anlage (100 g Snap-Säule; Laufmittelgradient Methylenchlorid/0-7% Methanol) 277 mg (23% d. Th.) der Titelverbindung erhalten.

¹H-NMR (400 MHz, DMSO-d₆, δ/ppm): 9.11 (dd, 1H), 8.09-8.16 (m, 2H), 7.97 (d, 1H), 7.69 (dd, 1H), 7.65 (d, 1H), 7.60 (s, 1H), 6.52 (s, 1H), 2.55 (s, 3H), 2.33 (s, 3H).

LC/MS (Methode 7, ESIpos): Rₜ = 1.01 min, m/z = 335 [M+H]⁺.

### Schritt 3: 2,4-Dimethyl-5-[6-(pyridazin-3-yl)-1H-imidazo[1,2-b]pyrazol-1-yl]anilin

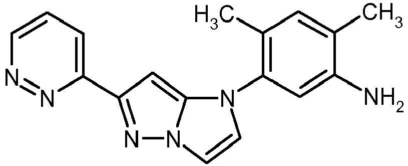

In Analogie zu Beispiel 13A / Schritt 3 wurden aus 272 mg (0.81 mmol) der in Beispiel 88A / Schritt 2 hergestellten Verbindung 182 mg (63% d. Th., ca. 85% Reinheit) der Titelverbindung als Rohprodukt erhalten, welches nicht weiter aufgereinigt wurde.

¹H-NMR (400 MHz, DMSO-d₆, δ/ppm): 9.09 (dd, 1H), 8.11 (dd, 1H), 7.84 (d, 1H), 7.68 (dd, 1H), 7.45 (d, 1H), 6.94 (s, 1H), 6.67 (s, 1H), 6.34 (s, 1H), 4.97 (s, 2H), 2.06 (s, 3H), 2.05 (s, 3H).

LC/MS (Methode 7, ESIpos): Rₜ = 0.85 min, m/z = 305 [M+H]⁺.

### Beispiel 89A

### 4-Methyl-3-[6-(1,3-thiazol-5-yl)-1H-imidazo[1,2-b]pyrazol-1-yl]anilin

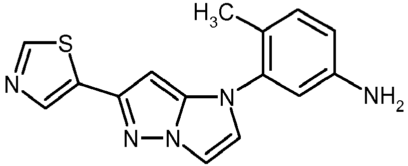

### Schritt 1: 1-(2,2-Diethoxyethyl)-N-(2-methyl-5-nitrophenyl)-3-(1,3-thiazol-5-yl)-1H-pyrazol-5-amin

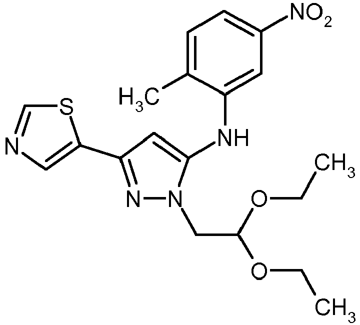

2.93 g (10.4 mmol) der Verbindung aus Beispiel 81A wurden unter Stickstoff zusammen mit 2.47 g (11.4 mmol) 2-Brom-4-nitrotoluol, 233 mg (1.04 mmol) Palladium(II)acetat, 901 mg (1.56 mmol) Xantphos [4,5-Bis-(diphenylphosphino)-9,9-dimethylxanthen] und 10.2 g (31.1 mmol) Cäsiumcarbonat in 43 ml 1,4-Dioxan für 3 h unter Rückfluss erhitzt. Nach dem Abkühlen wurde über Celite filtriert, mit Ethylacetat nachgewaschen und das Filtrat im Vakuum eingeengt. Das so erhaltene Rohprodukt wurde mittels einer Biotage-Anlage gereinigt (100 g Snap-Säule; Laufmittelgradient Ethylacetat/0-8% Methanol). Auf diese Weise wurden 3.26 g (75% d. Th.) der Titelverbindung erhalten.

¹H-NMR (400 MHz, DMSO-d₆, δ/ppm): 8.99 (s, 1H), 8.21 (s, 1H), 7.60 (dd, 1H), 7.57 (s, 1H), 7.47 (d, 1H), 7.39 (d, 1H), 6.61 (s, 1H), 4.80 (t, 1H), 4.11 (d, 2H), 3.51-3.61 (m, 2H), 3.35-3.44 (m, 2H), 2.33 (s, 3H), 0.96 (t, 6H).

LC/MS (Methode 7, ESIpos): Rₜ = 1.31 min, m/z = 418 [M+H]⁺.

### Schritt 2: 1-(2-Methyl-5-nitrophenyl)-6-(1,3-thiazol-5-yl)-1H-imidazo[1,2-b]pyrazol

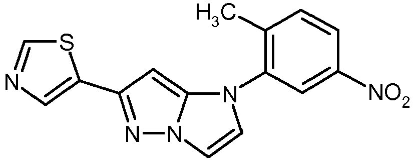

Eine Lösung von 308 mg (0.82 mmol) der Verbindung aus Beispiel 89A / Schritt 1 in 2.6 ml Ethanol wurde mit 0.9 ml (1.77 mmol) 2 M Schwefelsäure versetzt und anschließend für 20 min in der Mikrowelle auf 125°C erhitzt. Nach dem Abkühlen wurde das Reaktionsgemisch mit zwei weiteren Ansätzen zu je 1.63 g (3.9 mmol) der Verbindung aus Beispiel 89A / Schritt 1 zusammengefasst und mit 350 ml Ethylacetat verdünnt. Die organische Phase wurde einmal mit 30 ml gesättigter Natriumhydrogencarbonat-Lösung und einmal mit gesättigter Natriumchlorid-Lösung gewaschen. Nach dem Trocknen über Natriumsulfat und Filtration wurde im Vakuum eingeengt. Das so erhaltene Rohprodukt wurde mittels einer Biotage-Anlage gereinigt (100 g Snap-Säule; Laufmittelgradient Methylenchlorid/0-7% Methanol). Auf diese Weise wurden 1.94 g (69% d. Th.) der Titelverbindung erhalten.

¹H-NMR (400 MHz, DMSO-d₆, δ/ppm): 8.97 (s, 1H), 8.26 (d, 1H), 8.22 (dd, 1H), 8.20 (d, 1H), 7.89 (d, 1H), 7.75 (d, 1H), 7.60 (d, 1H), 6.31 (s, 1H), 2.38 (s, 3H).

LC/MS (Methode 7, ESIpos): Rₜ = 1.04 min, m/z = 326 [M+H]⁺.

### Schritt 3: 4-Methyl-3-[6-(1,3-thiazol-5-yl)-1H-imidazo[1,2-b]pyrazol-1-yl]anilin

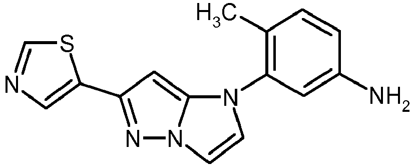

Zu einer Lösung von 132 mg (0.41 mmol) der Verbindung aus Beispiel 89A / Schritt 2 in einem Gemisch aus 3.2 ml Ethanol und 0.01 ml Wasser wurden 13 mg Palladium (10% auf Aktivkohle) gegeben. Nach Zugabe von 128 mg (2.3 mmol) Ammoniumformiat wurde die Reaktionsmischung 1 h unter Rückfluss erhitzt. Nach dem Abkühlen wurde über Celite abfiltriert, mit Ethylacetat nachgewaschen und das Filtrat mit 200 ml Ethylacetat verdünnt. Die organische Phase wurde einmal mit 30 ml Wasser und einmal mit 30 ml gesättigter Natriumchlorid-Lösung gewaschen. Nach dem Trocknen über Natriumsulfat und Filtration wurde im Vakuum eingeengt. Das so erhaltene Rohprodukt enthielt noch Startmaterial und wurde daher zusammen mit weiteren 1.81 g (5.56 mmol) der Verbindung aus Beispiel 89A / Schritt 2 unter analogen Bedingungen nochmals zur Reaktion gebracht. Da auch das hieraus resultierende Rohprodukt noch Startmaterial enthielt, wurde dieses Material noch einmal zur Reaktion gebracht, wobei in diesem Falle nur die halbe Menge an Palladium-Katalysator eingesetzt wurde. Das so erhaltene Rohprodukt wurde schließlich mittels einer Biotage-Anlage gereinigt (500 g Snap-Säule; Laufmittelgradient Hexan/60-100% Ethylacetat, dann Ethylacetat/0-15% Methanol). Auf diese Weise wurden 904 mg (49% d. Th.) der Titelverbindung erhalten.

¹H-NMR (400 MHz, DMSO-d₆, δ/ppm): 8.94 (s, 1H), 8.20 (s, 1H), 7.75 (d, 1H), 7.38 (d, 1H), 7.02 (d, 1H), 6.53-6.60 (m, 2H), 6.14 (s, 1H), 5.18 (s, 2H), 2.03 (s, 3H).

LC/MS (Methode 7, ESIpos): Rₜ = 0.85 min, m/z = 296 [M+H]⁺.

### Beispiel 90A

### 3-(4,4-Difluorpiperidin-1-yl)benzoesäure

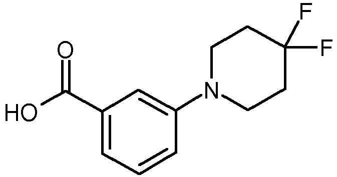

### Schritt 1: Methyl-3-(4,4-difluorpiperidin-1-yl)benzoat

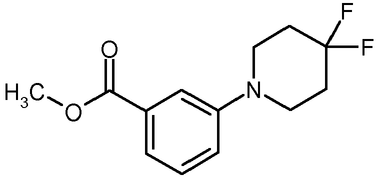

In einem Mikrowellenreaktionsgefäß wurde eine Lösung von 500 mg (2.33 mmol) Methyl-3-brombenzoat und 366 mg (2.33 mmol) 4,4-Difluorpiperidin-Hydrochlorid in 15 ml Dioxan durch Hindurchleiten von Argon sauerstofffrei gemacht. Dann wurden 52 mg (0.233 mmol) Palladium(II)-acetat, 202 mg (0.349 mmol) 4,5-Bis-(diphenylphosphino)-9,9-dimethylxanthen (Xantphos) und 2.27 g (6.98 mmol) Cäsiumcarbonat hinzugefügt. Das Mikrowellengefäß wurde mit einem Krampverschluss verschlossen und unter Magnetrührung 1.5 Stunden in einem Mikrowellenofen auf 120-140°C erhitzt (Biotage Initiator, mit dynamischer Steuerung der Einstrahlleistung). Nach beendeter Reaktion wurde das Reaktionsgemisch über wenig Kieselgur filtriert und anschließend zur Trockene eingedampft. Der Rückstand wurde mittels präparativer HPLC (Methode 33) in seine Komponenten aufgetrennt. Nach Eindampfen der Produktfraktionen und Trocknen des Rückstands im Hochvakuum wurden 342 mg (57% d. Th.) der Titelverbindung erhalten.

¹H-NMR (400 MHz, DMSO-d₆, δ/ppm): 7.52-7.50 (m, 1H), 7.40-7.35 (m, 2H), 7.32-7.28 (m, 1H), 3.83 (s, 3H), 3.38 (m, 4H), 2.06 (m, 4H).

LC/MS (Methode 3, ESIpos): Rₜ = 1.09 min, m/z = 256 [M+H]⁺.

### Schritt 2: 3-(4,4-Difluorpiperidin-1-yl)benzoesäure

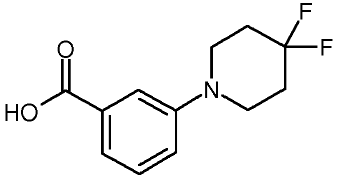

335 mg (1.31 mmol) der Verbindung aus Beispiel 90A / Schritt 1 wurden in 13 ml Methanol gelöst und mit 3.9 ml (3.94 mmol) 1 M Natronlauge versetzt. Nachdem das Reaktionsgemisch 7 h bei RT gerührt worden war, wurde das Methanol am Rotationsverdampfer entfernt und der verbliebene wässrige Rückstand unter Eiskühlung mit 1 M Salzsäure sauer gestellt. Dabei fiel ein Teil des Produktes aus, der abgesaugt, mit Wasser neutral gewaschen und im Hochvakuum getrocknet wurde (280 mg, 88% d. Th.). Eine zweite Fraktion des Produktes wurde durch Extraktion der wässrigen Mutterlauge mit Ethylacetat und Eindampfen und Trocknen des organischen Extraktes gewonnen (32 mg, 10% d. Th.). Insgesamt wurden somit 312 mg (98% d. Th.) der Titelverbindung erhalten.

¹H-NMR (400 MHz, DMSO-d₆, δ/ppm): 7.51 (m, 1H), 7.39 (dt, 1H), 7.35 (t, 1H), 7.27 (ddd, 1H), 3.37 (m, 4H), 2.06 (m, 4H).

LC/MS (Methode 3, ESIpos): Rₜ = 0.90 min, m/z = 242 [M+H]⁺.

### Beispiel 91A

### 3-(1,1,1-Trifluor-2-methylpropan-2-yl)benzoesäure

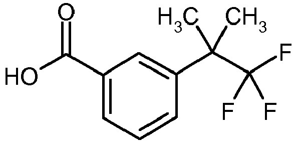

### Schritt 1: 2-(3-Bromphenyl)-1,1,1-trifluorpropan-2-ol

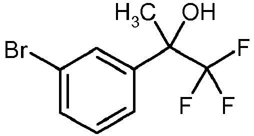

Unter Argon und unter Rühren wurde bei 0°C eine Lösung von 5.22 g (20.6 mmol) 1-(3-Bromphenyl)-2,2,2-trifluorethanon in 40 ml wasserfreiem THF tropfenweise mit 22.7 ml (22.7 mmol) einer 1 M Lösung von Methylmagnesiumbromid in Diethylether versetzt. Nach beendeter Zugabe wurde das Reaktionsgemisch noch 30 min bei 0°C nachgerührt. Dann wurde - weiterhin bei 0°C - überschüssiges Grignard-Reagenz durch vorsichtige Zugabe von Wasser hydrolysiert. Anschließend wurde das Gemisch durch Zugabe von 2 M Salzsäure auf einem pH-Wert von ca. 5 eingestellt. Die Phasen wurden getrennt, und die wässrige Phase wurde zweimal mit je ca. 20 ml Diethylether extrahiert. Die vereinigten organischen Phasen wurden über Magnesiumsulfat getrocknet, filtriert und am Rotationsverdampfer zur Trockene eingedampft. Auf diese Weise wurden 5.32 g (95% d. Th.) der Titelverbindung erhalten.

¹H-NMR (400 MHz, DMSO-d₆, δ/ppm): 7.76 (s, 1H), 7.58 (d, 2H), 7.38 (t, 1H), 6.77 (s, 1H), 1.69 (s, 3H).

GC/MS (Methode 8, EIpos): Rₜ = 3.62 min, m/z = 268/270 [M]⁺, 199/201 [M-CF₃]⁺ (⁷⁹Br/⁸¹Br).

### Schritt 2: 2-(3-Bromphenyl)-1,1,1-trifluorpropan-2-yl-methansulfonat

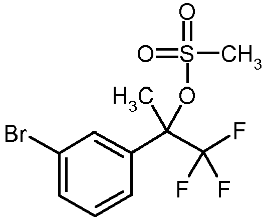

Unter Argon und unter Rühren wurde bei RT eine Lösung von 3.15 g (11.7 mmol) der Verbindung aus Beispiel 91A / Schritt 1 in 20 ml wasserfreiem THF tropfenweise zu einer Suspension von 937 mg (23.4 mmol) Natriumhydrid (60% in Mineralöl) in 30 ml wasserfreiem THF hinzugefügt. Nach beendetem Zutropfen wurde noch 1 h bei RT nachgerührt. Dann wurde das Gemisch auf 40°C erwärmt. Nach 30 min - weiterhin bei 40°C - wurde eine Lösung von 1.8 ml (23.4 mmol) Methansulfonsäurechlorid in 5 ml wasserfreiem THF zugetropft. Das Reaktionsgemisch wurde danach 1 h bei dieser Temperatur gerührt, bevor es auf RT abgekühlt wurde. Es wurden vorsichtig und tropfenweise 50 ml Wasser und dann 50 ml gesättigte wässrige Natriumhydrogencarbonat-Lösung zugesetzt. Die Phasen wurden getrennt, und die wässrige Phase wurde zweimal mit je ca. 100 ml Ethylacetat extrahiert. Die vereinigten organischen Phasen wurden über Magnesiumsulfat getrocknet, filtriert und am Rotationsverdampfer zur Trockene eingedampft. Das auf diese Weise erhaltene Rohprodukt wurde durch Verrühren mit wenig Hexan bei RT gereinigt. Nach Filtration und Trocknen im Vakuum wurden 3.70 g (91% d. Th.) der Titelverbindung erhalten.

¹H-NMR (400 MHz, DMSO-d₆, δ/ppm): 7.81 (s, 1H), 7.71 (d, 1H), 7.69 (d, 1H), 7.47 (t, 1H), 3.44 (s, 3H), 2.27 (s, 3H).

GC/MS (Methode 8, EIpos): Rₜ = 5.34 min, m/z = 346/348 [M]⁺, 250/252 [M-CH₃SO₃H]⁺ (⁷⁹Br/ ⁸¹Br).

### Schritt 3: 1-Brom-3-(1,1,1-trifluor-2-methylpropan-2-yl)benzol

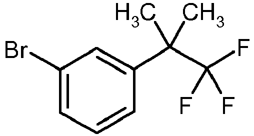

Unter Argon und unter Rühren wurde bei 0°C eine Lösung von 4.0 g (11.5 mmol) der Verbindung aus Beispiel 91A / Schritt 2 in 20 ml Dichlormethan tropfenweise mit 11.5 ml (23.0 mmol) einer 2 M Lösung von Trimethylaluminium in Toluol versetzt. Nach beendetem Zutropfen wurde das Eis/Wasser-Bad entfernt und das Rühren noch 1.5 h bei RT fortgesetzt. Nach dieser Zeit wurden vorsichtig und tropfenweise 40 ml gesättigte wässrige Natriumhydrogencarbonat-Lösung und dann 12 ml gesättigte wässrige Natriumchlorid-Lösung hinzugefügt. Die dabei ausgefallenen organischen Salze wurden über wenig Kieselgur abfiltriert. Es wurde zweimal mit Dichlormethan nachgewaschen. Die vereinigten Filtrate wurden mit gesättigter Kochsalz-Lösung gewaschen und über Magnesiumsulfat getrocknet. Nach Abdampfen des Lösungsmittels am Rotationsverdampfer wurden 2.9 g (84% d. Th.) der Titelverbindung erhalten.

¹H-NMR (400 MHz, DMSO-d₆, δ/ppm): 7.69 (s, 1H), 7.57 (d, 2H), 7.38 (t, 1H), 1.55 (s, 6H).

GC/MS (Methode 8, EIpos): Rₜ = 3.14 min, m/z = 266/268 [M]⁺, 197/199 [M-CF₃]⁺ (⁷⁹Br/⁸¹Br).

### Schritt 4: 3-(1,1,1-Trifluor-2-methylpropan-2-yl)benzoesäure

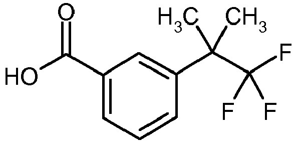

Unter Argon und unter Rühren wurde bei 0°C eine Lösung von 500 mg (1.87 mmol) der Verbindung aus Beispiel 91A / Schritt 3 in 15 ml wasserfreiem Diethylether tropfenweise mit 824 µl (2.06 mmol) einer 2.5 M Lösung von *n*-Butyllithium in Hexanfraktion versetzt. Nachdem das Zutropfen beendet war, wurde noch 1 h bei 0°C nachgerührt, bevor trockenes Kohlendioxid-Gas in die Apparatur eingeleitet wurde. Nach einer weiteren Stunde bei 0°C wurde das Reaktionsgemisch auf RT erwärmt und mit ca. 50 ml Wasser versetzt. Die Phasen wurden getrennt, und die Etherphase wurde einmal mit Wasser extrahiert. Die vereinigten wässrigen Phasen wurden dann durch Zusatz von 1 M Salzsäure angesäuert und anschließend dreimal mit je ca. 50 ml Ethylacetat extrahiert. Die vereinigten organischen Extrakte wurden über Magnesiumsulfat getrocknet und am Rotationsverdampfer zur Trockene eingeengt. Nach Trocknen des Rückstands im Hochvakuum wurden 95 mg (21% d. Th.) der Titelverbindung erhalten.

¹H-NMR (400 MHz, DMSO-d₆, δ/ppm): 13.07 (breit, 1H), 8.08 (s, 1H), 7.93 (d, 1H), 7.82 (d, 1H), 7.55 (t, 1H), 1.59 (s, 6H).

LC/MS (Methode 3, ESIneg): Rₜ = 0.96 min, m/z = 231 [M-H]⁻.

### Beispiel 92A

### 3-(2-Hydroxypropan-2-yl)benzoesäure

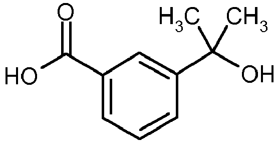

Unter Argon wurde bei -78°C eine Lösung von 1.0 g (4.97 mmol) 3-Brombenzoesäure in 20 ml wasserfreiem THF tropfenweise mit 4 ml (9.95 mmol) einer 2.5 M Lösung von *n*-Butyllithium in Hexanfraktion versetzt. Nach 20 min wurden bei der gleichen Temperatur 730 µl (9.95 mmol) Aceton hinzugetropft. Das Reaktionsgemisch wurde eine weitere Stunde bei -78°C nachgerührt, bevor man es im Verlauf von ca. 1 h auf RT aufwärmen ließ. Dann wurde das Reaktionsgemisch durch vorsichtige Zugabe von einigen Tropfen gesättigter wässriger Ammoniumchlorid-Lösung hydrolysiert. Es wurde mit 200 ml Wasser verdünnt und fünfmal mit je ca. 25 ml Ethylacetat extrahiert. Die vereinigten organischen Extrakte wurden mit gesättigter wässriger Natriumchlorid-Lösung gewaschen, über Magnesiumsulfat getrocknet, filtriert und am Rotationsverdampfer vom Lösungsmittel befreit. Der erhaltene Rückstand wurde in vier Portionen mittels präparativer HPLC gereinigt (Methode 36). Nach Vereinigen der Produktfraktionen, Eindampfen und Trocknen im Hochvakuum wurden 356 mg (39% d. Th.) der Titelverbindung erhalten.

¹H-NMR (400 MHz, DMSO-d₆, δ/ppm): 12.85 (breit, 1H), 8.08 (t, 1H), 7.78 (dt, 1H), 7.69 (dt, 1H), 7.42 (t, 1H), 5.14 (s, 1H), 1.44 (s, 6H).

LC/MS (Methode 3, ESIneg): Rₜ = 0.62 min, m/z = 179 [M-H]⁻.

### Beispiel 93A

### 3-(Pentafluor-λ⁶-sulfanyl)-5-(piperidin-1-yl)benzoesäure

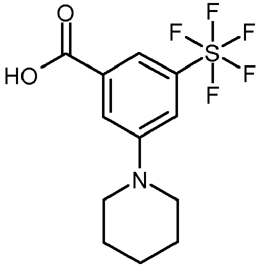

### Schritt 1: Methyl-3-brom-5-(pentafluor-λ⁶-sulfanyl)benzoat

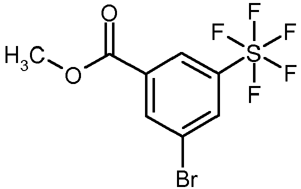

5.0 g (15.3 mmol) der Verbindung aus Beispiel 15A wurden in 150 ml Methanol gelöst und tropfenweise bei RT mit 2.2 ml (30.6 mmol) Thionylchlorid versetzt. Das Reaktionsgemisch wurde anschließend 4 h unter Rückfluss erhitzt. Nach dem Abkühlen auf RT wurde der Großteil des Lösungsmittels bis auf ein Restvolumen von ca. 50 ml am Rotationsverdampfer entfernt. Der Rest wurde mit Ethylacetat verdünnt und nacheinander mit Wasser, gesättigter Natriumhydrogencarbonat-Lösung und gesättigter Natriumchlorid-Lösung gewaschen. Nach Trocknen über Magnesiumsulfat wurde filtriert und eingedampft. Das so erhaltene Rohprodukt wurde mittels Saugfiltration über ca. 50 g Kieselgel mit Dichlormethan als Laufmittel gereinigt. Nach neuerlichem Eindampfen wurden 5.06 g (97% d. Th.) der Titelverbindung erhalten.

¹H-NMR (400 MHz, DMSO-d₆, δ/ppm): 8.51 (t, 1H), 8.35 (s, 1H), 8.27 (dd, 1H), 3.92 (s, 3H).

LC/MS (Methode 3, ESIpos): Rₜ = 1.27 min, keine Ionisierung.

### Schritt 2: 3-(Pentafluor-λ⁶-sulfanyl)-5-(piperidin-1-yl)benzoesäure

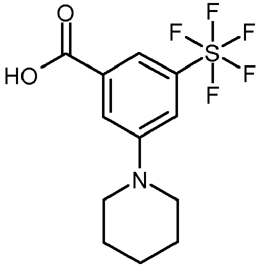

Eine Mischung aus 200 mg (0.586 mmol) der Verbindung aus Beispiel 93A / Schritt 1, 70 µl (0.704 mmol) Piperidin, 27 mg (0.029 mmol) Tris(dibenzylidenaceton)dipalladium, 42 mg (0.088 mmol) 2-Dicyclohexylphosphino-2',4',6'-triisopropylbiphenyl und 141 mg (1.47 mmol) Natrium-tert.-butylat in 6 ml Toluol wurde in einem Mikrowellenofen für 90 min auf 80°C erwärmt (Biotage Initiator, mit dynamischer Steuerung der Einstrahlleistung). Nach dem Abkühlen auf RT wurde mit ca. 20 ml Wasser versetzt und dreimal mit je ca. 20 ml Ethylacetat extrahiert. Die vereinigten organischen Extrakte wurden mit gesättigter wässriger Natriumchlorid-Lösung gewaschen, über Magnesiumsulfat getrocknet, filtriert und am Rotationsverdampfer vom Lösungsmittel befreit. Der erhaltene Rückstand wurde mittels präparativer HPLC in seine Komponenten aufgetrennt (Methode 33). Es wurden zwei Fraktionen erhalten: 41 mg (21% d. Th.) der Titelverbindung sowie 27 mg (13% d. Th.) des korrespondierenden Methylesters.

LC/MS (Methode 3, ESIpos): Rₜ = 1.28 min, m/z = 332 [M+H]⁺.

### Beispiel 94A

### 3-(4-Cyanopiperidin-1-yl)-5-(pentafluor-λ⁶-sulfanyl)benzoesäure

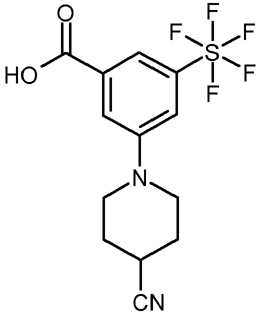

Analog zu dem unter Beispiel 93A / Schritt 2 beschriebenen Verfahren wurden aus 400 mg (1.17 mmol) der Verbindung aus Beispiel 93A / Schritt 1 und 155 mg (1.41 mmol) 4-Cyanopiperidin 158 mg (37% d. Th.) der Titelverbindung sowie 104 mg (23% d. Th.) des korrespondierenden Methylesters erhalten.

¹H-NMR (400 MHz, DMSO-d₆, δ/ppm): 13.61 (breit, 1H), 7.67-7.65 (m, 2H), 7.59 (t, 1H), 3.54-3.48 (m, 2H), 3.24-3.17 (m, 2H), 3.12-3.05 (m, 1H), 2.04-1.96 (m, 2H), 1.87-1.78 (m, 2H).

LC/MS (Methode 3, ESIpos): Rₜ = 0.99 min, m/z = 357 [M+H]⁺.

### Beispiel 95A

### 3-(4-Methoxypiperidin-1-yl)-5-(pentafluor-λ⁶-sulfanyl)benzoesäure

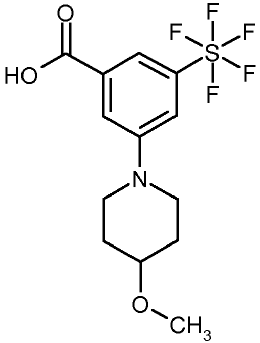

Analog zu dem unter Beispiel 93A / Schritt 2 beschriebenen Verfahren wurden aus 200 mg (0.586 mmol) der Verbindung aus Beispiel 93A / Schritt 1 und 81 mg (0.704 mmol) 4-Methoxypiperidin 49 mg (23% d. Th.) der Titelverbindung sowie 23 mg (10% d. Th.) des korrespondierenden Methylesters erhalten.

LC/MS (Methode 3, ESIpos): Rₜ = 1.06 min, m/z = 362 [M+H]⁺.

### Beispiel 96A

### 3-(3-Methoxyazetidin-1-yl)-5-(pentafluor-λ⁶-sulfanyl)benzoesäure

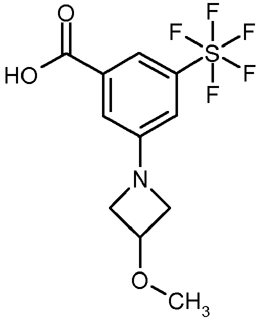

### Schritt 1: Methyl-3-(3-methoxyazetidin-1-yl)-5-(pentafluor-λ⁶-sulfanyl)benzoat

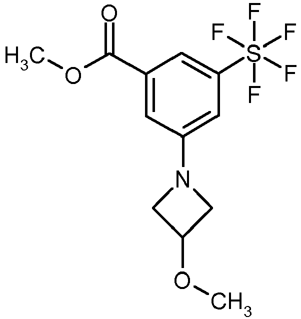

Analog zu dem unter Beispiel 93A / Schritt 2 beschriebenen Verfahren wurden aus 220 mg (0.645 mmol) der Verbindung aus Beispiel 93A / Schritt 1 und 120 mg (0.967 mmol) 3-Methoxyazetidin-Hydrochlorid 88 mg (37% d. Th.) der Titelverbindung sowie 35 mg (16% d. Th.) der korrespondierenden Benzoesäure erhalten.

¹H-NMR (400 MHz, DMSO-d₆, δ/ppm): 7.54 (m, 1H), 7.14 (m, 1H), 7.04 (t, 1H), 4.37-4.32 (m, 1H), 4.18 (dd, 2H), 3.88 (s, 3H), 3.78 (dd, 2H), 3.25 (s, 3H).

LC/MS (Methode 3, ESIpos): Rₜ = 1.21 min, m/z = 348 [M+H]⁺.

### Schritt 2: 3-(3-Methoxyazetidin-1-yl)-5-(pentafluor-λ⁶-sulfanyl)benzoesäure

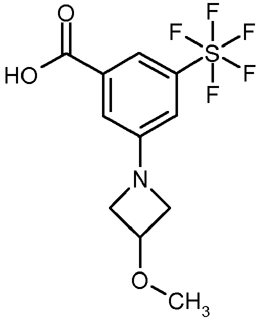

80 mg (0.230 mmol) der Verbindung aus Beispiel 96A / Schritt 1 wurden in 3 ml Methanol gelöst und mit 691 µl (0.691 mmol) 1 M Natronlauge versetzt. Das Reaktionsgemisch wurde zunächst ca. 18 h bei RT und dann 10 h bei 50°C gerührt. Anschließend wurde das Reaktionsgemisch durch Zugabe von 1 M Salzsäure sauer gestellt. Es wurde dreimal mit je ca. 10 ml Ethylacetat extrahiert. Die vereinigten organischen Extrakte wurden mit gesättigter wässriger Natriumchlorid-Lösung gewaschen, über Magnesiumsulfat getrocknet, filtriert und am Rotationsverdampfer vom Lösungsmittel befreit. Nach Trocknen des Rückstands im Hochvakuum wurden 78 mg (96% d. Th.) der Titelverbindung erhalten.

¹H-NMR (400 MHz, DMSO-d₆, δ/ppm): 13.49 (breit, 1H), 7.54 (m, 1H), 7.15 (m, 1H), 7.00 (t, 1H), 4.37-4.32 (m, 1H), 4.17 (dd, 2H), 3.77 (dd, 2H), 3.25 (s, 3H).

LC/MS (Methode 3, ESIpos): Rₜ = 1.02 min, m/z = 334 [M+H]⁺.

### Beispiel 97A

### 3-(2-tert.-Butoxy-2-oxoethyl)-5-(pentafluor-λ⁶-sulfanyl)benzoesäure

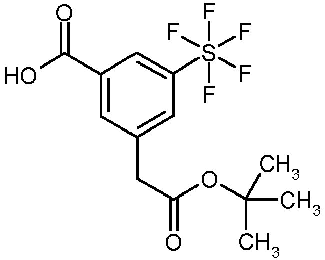

### Schritt 1: Methyl-3-(2-tert.-butoxy-2-oxoethyl)-5-(pentafluor-λ⁶-sulfanyl)benzoat

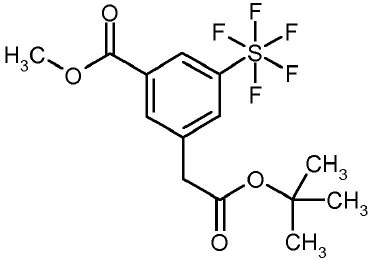

Unter Argon wurden 2.40 g (36.6 mmol) Zinkstaub in 20 ml wasserfreiem Diethylether vorgelegt und mit 265 µl (2.09 mmol) Chlortrimethylsilan versetzt. Nach 15 min Rühren bei RT wurde das Gemisch unter Rückfluss erhitzt. Dann wurde das Heizbad entfernt, und 5.4 ml (36.6 mmol) *tert.-*Butyl-bromacetat wurden so zugetropft, dass die Mischung am Sieden blieb. Nach beendetem Zutropfen wurde mit Hilfe des Heizbades noch 1 h weiter unter Rückfluss erhitzt. Dann ließ man die so erhaltene Zinkorganyl-Lösung auf RT abkühlen.

Unter Argon wurden 9 ml der oben hergestellten Zinkorganyl-Lösung [entsprechend ca. 14.7 mmol Brom(2-*tert.*-butoxy-2-oxoethyl)zink] mit einer Lösung von 2.5 g (7.33 mmol) der Verbindung aus Beispiel 93A / Schritt 1 in 15 ml wasserfreiem THF versetzt. Dann wurden 104 mg (0.147 mmol) 1,2,3,4,5-Pentaphenyl-1'-(di-*tert*.-butylphosphino)ferrocen (Q-Phos) und 84 mg (0.147 mmol) Bis-(dibenzylidenaceton)palladium hinzugefügt, und das Gemisch wurde 16 h bei RT gerührt. Da nach dieser Zeit der Umsatz noch nicht vollständig war, wurde die restliche Zinkorganyl-Lösung hinzugefügt und das resultierende Gemisch für 20 h auf 60°C erwärmt. Nach dem Abkühlen auf RT wurde mit 400 ml Ethylacetat verdünnt. Es wurde nacheinander mit je ca. 200 ml Wasser und gesättigter wässriger Natriumchlorid-Lösung gewaschen. Es wurde über Magnesiumsulfat getrocknet, filtriert und das Lösungsmittel am Rotationsverdampfer entfernt. Das so erhaltene Rohprodukt wurde mittels Saugfiltration über ca. 120 g Kieselgel mit Petrolether/Dichlormethan 2:1 → 1:3 als Laufmittel gereinigt. Nach Eindampfen der Produktfraktionen und Trocknen des Rückstands im Hochvakuum wurden 1.21 g (43% d. Th.) der Titelverbindung erhalten.

¹H-NMR (400 MHz, CDCl₃, δ/ppm): 8.33 (t, 1H), 8.10 (s, 1H), 7.88 (t, 1H), 3.96 (s, 3H), 3.65 (s, 2H), 1.45 (s, 9H).

GC/MS (Methode 8, EIpos): Rₜ = 5.37 min, m/z = 289 [M-87]⁺.

### Schritt 2: 3-(2-tert.-Butoxy-2-oxoethyl)-5-(pentafluor-λ⁶-sulfanyl)benzoesäure

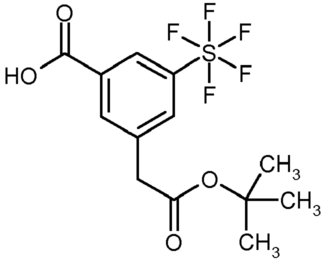

Eine Lösung von 1.21 g (3.22 mmol) der Verbindung aus Beispiel 97A / Schritt 1 in 28 ml THF wurde bei RT mit 25.7 ml (6.43 mmol) 0.25 M wässriger Lithiumhydroxid-Lösung versetzt. Nach 2 h bei RT wurde das Reaktionsgemisch in 250 ml Wasser gegossen und mit Essigsäure auf einen schwach sauren pH-Wert eingestellt. Es wurde zügig dreimal mit je ca. 75 ml Ethylacetat extrahiert. Die vereinigten organischen Extrakte wurden einmal mit gesättigter wässriger Natriumchlorid-Lösung gewaschen, über Magnesiumsulfat getrocknet, filtriert und am Rotationsverdampfer vom Lösungsmittel befreit. Das so erhaltene Rohprodukt wurde 20 min bei RT in 20 ml Pentan/Diisopropylether 20:1 verrührt. Der Feststoff wurde abgesaugt, mit Pentan gewaschen und im Hochvakuum getrocknet. Es wurden 845 mg (72% d. Th.) der Titelverbindung erhalten.

¹H-NMR (400 MHz, DMSO-d₆, δ/ppm): 13.68 (breit, 1H), 8.18 (dd, 1H), 8.13 (s, 1H), 8.11 (dd, 1H), 3.87 (s, 2H), 1.41 (s, 9H).

LC/MS (Methode 3, ESIneg): Rₜ = 1.12 min, m/z = 361 [M-H]⁻.

### Beispiel 98A

### 3-[(2-Methoxyethoxy)methyl]-5-(pentafluor-λ⁶-sulfanyl)benzoesäure

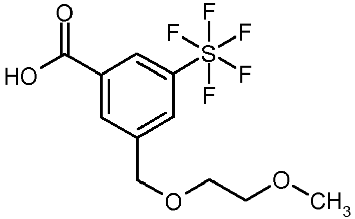

### Schritt 1: 2-Methoxyethyl-3-[(2-methoxyethoxy)methyl]-5-(pentafluor-λ⁶-sulfanyl)benzoat

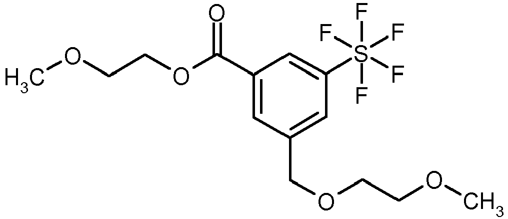

Eine Lösung von 200 mg (0.719 mmol) der Verbindung aus Beispiel 37A in 5.7 ml wasserfreiem DMF wurde bei 0°C mit 86 mg (2.16 mmol) Natriumhydrid (60%-ige Suspension in Mineralöl) versetzt und anschließend auf RT erwärmt. Dann wurden 300 mg (2.16 mmol) 2-Bromethyl-methylether hinzugefügt, und das Reaktionsgemisch wurde ca. 16 h bei 60°C gerührt. Nach dem Abkühlen auf RT wurde 1 ml Methanol hinzugesetzt. Das Reaktionsgemisch wurde dann in zwei Portionen mittels präparativer HPLC in seine Komponenten aufgetrennt (Methode 33). Die Produktfraktionen wurden vereinigt, am Rotationsverdampfer eingedampft und der Rückstand im Hochvakuum getrocknet. Es wurden so 58 mg (20% d. Th.) der Titelverbindung erhalten. Daneben wurden 138 mg (57% d. Th.) 2-Methoxyethyl-3-(hydroxymethyl)-5-(pentafluor-λ⁶-sulfanyl)benzoat isoliert, die nochmals in der zuvor beschriebenen Weise mit 2-Bromethyl-methylether umgesetzt wurden und weitere 30 mg der Titelverbindung lieferten.

¹H-NMR (400 MHz, CDCl₃, δ/ppm): 8.34 (t, 1H), 8.17 (s, 1H), 7.97 (s, 1H), 4.67 (s, 2H), 4.52 (m, 2H), 3.74 (m, 2H), 3.69 (m, 2H), 3.61 (m, 2H), 3.43 (s, 3H), 3.41 (s, 3H).

LC/MS (Methode 3, ESIpos): Rₜ = 1.12 min, m/z = 395 [M+H]⁺.

### Schritt 2: 3-[(2-Methoxyethoxy)methyl]-5-(pentafluor-λ⁶-sulfanyl)benzoesäure

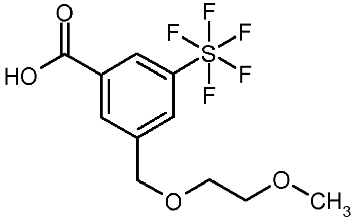

85 mg (0.194 mmol) der Verbindung aus Beispiel 98A / Schritt 1 wurden in 2 ml THF gelöst und mit 204 µl (0.204 mmol) einer 1 M Lösung von Lithiumhydroxid in Wasser versetzt. Das Reaktionsgemisch wurde zunächst 1 h bei RT und dann ca. 16 h bei 5-8°C gerührt. Nach dem Aufwärmen auf RT wurde das Reaktionsgemisch mit 2 ml Wasser und 14 µl (0.242 mmol) Eisessig versetzt und anschließend mittels präparativer HPLC in seine Komponenten aufgetrennt (Methode 36). Die Produktfraktionen wurden vereinigt, am Rotationsverdampfer eingedampft und der Rückstand im Hochvakuum getrocknet. Es wurden 60 mg (92% d. Th.) der Titelverbindung erhalten.

¹H-NMR (400 MHz, DMSO-d₆, δ/ppm): 13.70 (breit, 1H), 8.20 (t, 1H), 8.17 (s, 1H), 7.11 (t, 1H), 4.68 (s, 2H), 4.63 (m, 2H), 3.51 (m, 2H), 3.26 (s, 3H).

LC/MS (Methode 3, ESIneg): Rₜ = 0.96 min, m/z = 335 [M-H]⁻.

### Beispiel 99A

### 3-(3-Bromphenyl)-3-methyloxetan

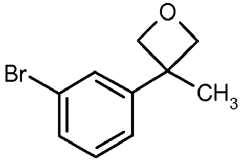

### Schritt 1: Diethyl-(3-bromphenyl)malonat

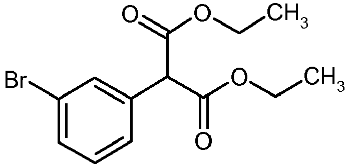

*Darstellung von Ethyl-3-bromphenylacetat:* Zu einer Lösung von 25 g (116 mmol) 3-Bromphenylessigsäure in 170 ml Ethanol wurden 2.5 ml konz. Schwefelsäure gegeben. Die Mischung wurde 20 h unter Rückfluss erhitzt. Nach dem Abkühlen wurde im Vakuum eingeengt und der Rückstand in 800 ml Ethylacetat gelöst. Die organische Phase wurde dreimal mit je 50 ml gesättigter wässriger Natriumhydrogencarbonat-Lösung und zweimal mit je 50 ml gesättigter wässriger Natriumchlorid-Lösung gewaschen, über Natriumsulfat getrocknet und nach Filtration im Vakuum eingeengt. Der so erhaltene Rückstand wurde mittels Säulenchromatographie an Kieselgel mit Hexan/ 0-20% Ethylacetat als Laufmittel gereinigt. Man erhielt auf diese Weise 27.8 g (98% d. Th.) 3-Bromphenylessigsäureethylester.

*Darstellung der Titelverbindung:* Zu einer Suspension von 13.7 g (343 mmol) Natriumhydrid (60%-ige Suspension in Mineralöl) in 299 ml Toluol und 54 g (457 mmol) Diethylcarbonat wurde bei 150°C eine Lösung von 27.8 g (114 mmol) 3-Bromphenylessigsäureethylester in 100 ml Toluol innerhalb von 30 Minuten zugetropft und die Mischung dann für 3 h unter Rückfluss erhitzt. Nach dem Abkühlen wurde die Reaktionsmischung auf Eiswasser gegossen und dreimal mit je 250 ml Ethylacetat extrahiert. Die vereinigten organischen Phasen wurden einmal mit 100 ml gesättigter Natriumchlorid-Lösung gewaschen, über Natriumsulfat getrocknet und nach Filtration im Vakuum eingeengt. Der so erhaltene Rückstand wurde mittels Säulenchromatographie an Kieselgel mit Hexan/0-20% Ethylacetat als Laufmittel gereinigt. Man erhielt auf diese Weise 31.8 g (88% d. Th.) der Titelverbindung.

¹H-NMR (400 MHz, DMSO-d₆, δ/ppm): 7.56 (t, 1H), 7.52 (dt, 1H), 7.34-7.39 (m, 1H), 7.32 (d, 1H), 5.00 (s, 1H), 4.06-4.18 (m, 4H), 1.14 (t, 6H).

LC/MS (Methode 7, ESIneg): Rₜ = 1.32 min, m/z = 315 [M-H]⁻.

### Schritt 2: 3-(3-Bromphenyl)-3-methyloxetan

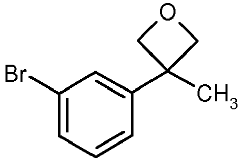

*Methylierung:* Zu einer Lösung von 31.8 g (101 mmol) der Verbindung aus Beispiel 99A / Schritt 1 in 220 ml Ethanol wurden bei RT 6.55 g (121 mmol) Natriummethylat gegeben. Nach dem vollständigen Auflösen wurden 7.54 ml (121 mmol) Iodmethan zugetropft und die Mischung 24 h bei RT gerührt. Dann wurde die Reaktionsmischung im Vakuum eingeengt und der Rückstand in 800 ml Ethylacetat aufgenommen. Die organische Phase wurde zweimal mit je 50 ml Wasser und einmal mit gesättigter Natriumchlorid-Lösung gewaschen, über Natriumsulfat getrocknet und nach Filtration im Vakuum eingeengt. Da das so erhaltene Rohprodukt noch Startmaterial enthielt, wurde das Rohprodukt noch drei weitere Male in oben beschriebenen Art umgesetzt, wobei jeweils nur 1.5 g (24.1 mmol) Natriummethylat und 1.5 g (27.8 mmol) Iodmethan eingesetzt wurden. Das schließlich erhaltene Rohprodukt wurde durch Säulenchromatographie an Kieselgel mit Hexan/ 0-20% Ethylacetat als Laufmittel gereinigt. Man erhielt auf diese Weise 25.7 g einer Mischung von Methyl- und Ethylestern mit Diethyl-(3-bromphenyl)(methyl)malonat als Hauptkomponente.

*Reduktion:* Zu einer Lösung von 171 mg (4.5 mmol) Lithiumaluminiumhydrid in 200 ml THF wurden bei 0°C 988 mg (3.0 mmol) des zuvor hergestellten Diesters, gelöst in 100 ml THF, getropft. Die Mischung wurde zunächst 30 min bei RT und anschließend 4 h bei 40°C gerührt. Dann wurde auf 0°C abgekühlt und vorsichtig 20 ml gesättigte wässrige Natriumhydrogencarbonat-Lösung zugegeben. Das Gemisch wurde über Celite filtriert und anschließend mit Ethylacetat extrahiert. Die organische Phase wurde über Natriumsulfat getrocknet und nach Filtration im Vakuum eingeengt. Die Reinigung des Rohprodukts erfolgte zusammen mit dem eines zweiten analogen Ansatzes mit 21 g (44.8 mmol) des zuvor hergestellten Diesters durch Säulenchromatographie an Kieselgel mit Hexan/Ethylacetat 8:1 → 1:2 als Laufmittel. Auf diese Weise wurden 9.0 g (76% d. Th.) 2-(3-Bromphenyl)-2-methylpropan-1,3-diol erhalten.

*Darstellung der Titelverbindung:* Zu einer Lösung von 200 mg (0.82 mmol) des zuvor hergestellten Diols in 5.0 ml Toluol wurden 428 mg (1.63 mmol) Triphenylphosphin gegeben. Nach 10 min Rühren bei RT wurden 374 mg (1.22 mmol) Ziram (Zinkdimethyldithiocarbamat) zugegeben und danach 284 mg (1.63 mmol) Diethylazodicarboxylat als 40%-ige Lösung in Toluol zugetropft. Die Reaktionsmischung wurde anschließend 18 h bei RT gerührt. Nach Filtration über Celite wurde das Filtrat im Vakuum eingeengt. Dieses Rohprodukt wurde mit dem aus zwei weiteren Ansätzen (200 mg bzw. 1.1 g Diol) vereinigt und durch Säulenchromatographie an Kieselgel mit Hexan/ 0-10% Ethylacetat als Laufmittel gereinigt. Die so erhaltenen 700 mg noch verunreinigten Materials wurden zusammen mit dem aus vier weiteren Ansätzen (einmal 1.1 g Diol und dreimal jeweils 2.33 g Diol) gewonnenen Material in gleicher Weise nochmals gereinigt. Man erhielt so 5.0 g (69% d. Th.) der Titelverbindung.

¹H-NMR (400 MHz, DMSO-d₆, δ/ppm): 7.37-7.43 (m, 2H), 7.29 (t, 1H), 7.19-7.24 (m, 1H), 4.74 (d, 2H), 4.48 (d, 2H), 1.57 (s, 3H).

### Ausführungsbeispiele:

### Beispiel 1

### N-{4-Methyl-3-[6-(1H-pyrazol-4-yl)-1H-imidazo[1,2-b]pyrazol-1-yl]phenyl}-3-(pentafluor-λ⁶-sulfanyl)-5-(piperazin-1-yl)benzamid

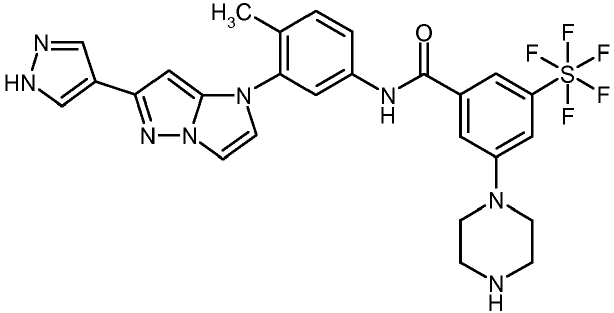

210 mg (0.19 mmol, Reinheit 61%) der Verbindung aus Beispiel 47A wurden in 4.4 ml einer 25%-igen Lösung von Trifluoressigsäure in Methylenchlorid 30 min bei RT gerührt. Danach wurde der Ansatz im Vakuum eingeengt. Der Rückstand wurde in wenig Methanol gelöst und auf halbkonzentrierte wässrige Natriumhydrogencarbonat-Lösung gegeben. Nach 15 min Rühren bei RT wurden 15 ml Ethylacetat zugesetzt. Der entstandene Niederschlag wurde ab filtriert, und die organische Phase des Filtrats wurde abgetrennt, mit Wasser gewaschen, über Natriumsulfat getrocknet, filtriert und eingeengt. Nach Trocknen des Rückstands im Vakuum wurden 41 mg (37% d. Th.) der Titelverbindung erhalten.

¹H-NMR (400 MHz, DMSO-d₆, δ/ppm): 12.85 (br. s, 1H), 10.55 (s, 1H), 8.01 (br. s, 1H), 7.90 (d, 1H), 7.78 (m, 3H), 7.75 (d, 1H), 7.72 (s, 1H), 7.55 (s, 1H), 7.45 (d, 1H), 7.41 (d, 1H), 5.92 (s, 1H), 3.38 (m, 4H), 3.06 (m, 4H), 2.27 (s, 3H).

LC/MS (Methode 3, ESIpos): Rₜ = 0.75 min, m/z = 593 [M+H]⁺.

### Beispiel 2

### 3-(4-Methylpiperazin-1-yl)-N-{4-methyl-3-[6-(1H-pyrazol-4-yl)-1H-imidazo[1,2-b]pyrazol-1-yl]-phenyl}-5-(pentafluor-λ⁶-sulfanyl)benzamid

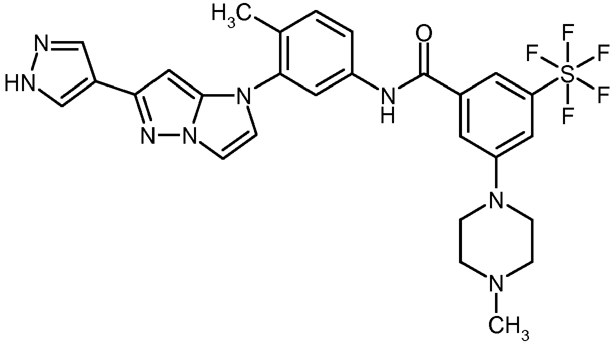

90 mg (0.12 mmol) der Verbindung aus Beispiel 48A wurden in 0.61 ml Trifluoressigsäure 3 h bei 80°C gerührt. Der Ansatz wurde danach eingeengt und der Rückstand mittels präparativer HPLC gereinigt (Methode 18). Die Produktfraktionen wurden eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands im Hochvakuum wurden 40 mg (53% d. Th.) der Titelverbindung erhalten.

¹H-NMR (400 MHz, DMSO-d₆, δ/ppm): 12.85 (br, 1H), 10.53 (s, 1H), 8.01 (br, 1H), 7.90 (d, 1H), 7.81-7.71 (m, 5H), 7.51 (s, 1H), 7.45 (d, 1H), 7.41 (d, 1H), 5.92 (s, 1H), 2.27 (s, 3H) [weitere Signale unter Lösungsmittel-Peaks verborgen].

LC/MS (Methode 3, ESIpos): Rₜ = 0.75 min, m/z = 607 [M+H]⁺.

### Beispiel 3

### N-{4-Methyl-3-[6-(1H-pyrazol-4-yl)-1H-imidazo[1,2-b]pyrazol-1-yl]phenyl}-3-(morpholin-4-yl)-5-(pentafluor-λ⁶-sulfanyl)benzamid

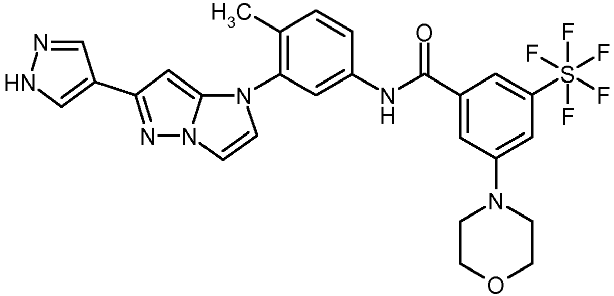

98 mg (0.14 mmol) der Verbindung aus Beispiel 49A wurden in 0.56 ml Trifluoressigsäure 3 h bei 80°C gerührt. Der Ansatz wurde danach eingeengt, der Rückstand in Ethylacetat gelöst und die Lösung mit gesättigter Natriumhydrogencarbonat-Lösung gewaschen. Die organische Phase wurde über Natriumsulfat getrocknet, filtriert und im Vakuum eingeengt. Nach Trocknen des Rückstands im Hochvakuum wurde das Rohprodukt mittels präparativer HPLC (Methode 29) gereinigt. Die Produktfraktionen wurden vereinigt und eingeengt, und der Rückstand wurde in Ethylacetat aufgenommen und mit gesättigter Natriumhydrogencarbonat-Lösung gewaschen. Die organische Phase wurde über Natriumsulfat getrocknet, filtriert und im Vakuum eingeengt. Nach Trocknen im Hochvakuum wurde eine erste Produktfraktion (39 mg) erhalten. Eine zweite Produktfraktion von weiteren 19 mg wurde aus den vereinigten Mischfraktionen der HPLC-Trennung gewonnen, indem diese eingedampft und nochmals nach gleicher Methode mittels präparativer HPLC gereinigt wurden. Insgesamt wurden somit 58 mg (69% d. Th.) der Titelverbindung erhalten.

¹H-NMR (400 MHz, DMSO-d₆, δ/ppm): 12.85 (br. s, 1H), 10.53 (s, 1H), 8.01 (br. s, 1H), 7.90 (d, 1H), 7.81-7.75 (m, 4H), 7.71 (s, 1H), 7.52 (t, 1H), 7.45 (d, 1H), 7.41 (d, 1H), 5.92 (s, 1H), 3.76 (m, 4H), 2.27 (s, 3H) [weitere Signale unter Lösungsmittel-Peaks verborgen].

LC/MS (Methode 4, ESIpos): Rₜ = 1.04 min, m/z = 594 [M+H]⁺.

### Beispiel 4

### 3-(2-Hydroxypropan-2-yl)-N-{4-methyl-3-[6-(1H-pyrazol-4-yl)-1H-imidazo[1,2-b]pyrazol-1-yl]-phenyl}-5-(pentafluor-λ⁶-sulfanyl)benzamid

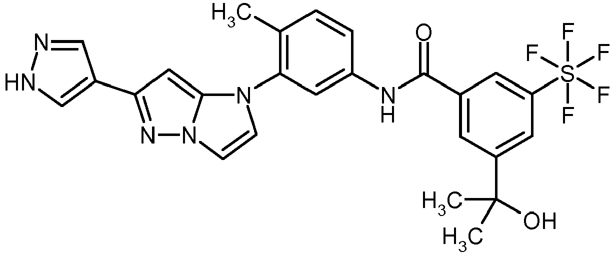

Die Verbindung aus Beispiel 7A (2.63 g, 9.45 mmol) wurde in 55 ml DMF gelöst. Es wurden HATU (7.91 g, 20.8 mmol) und *N*-Methylmorpholin (8.31 ml, 75.6 mmol) hinzugegeben, und die Mischung wurde 30 min bei RT gerührt. Anschließend wurde der Ansatz auf -5°C abgekühlt und die Verbindung aus Beispiel 19A (5.79 g, 18.9 mmol) hinzugefügt. Es wurde 45 min weitergerührt, dann mit konzentrierter wässriger Ammoniak-Lösung versetzt und noch 15 min nachgerührt. Darauf wurde mit Ethylacetat extrahiert, und der organische Extrakt wurde mit konz. Natriumchlorid-Lösung gewaschen, über Natriumsulfat getrocknet und eingeengt. Das Rohprodukt wurde durch präparative HPLC gereinigt [Säule: Waters Sunfire C-18 5 µm, 250 mm x 20 mm; ternärer Gradient Wasser/Acetonitril/1% TFA in Wasser: 0-6.7 min 45:50:5, Rampe, 6.9-9.0 min 0:95:5]. Es wurden so 3.86 g (72% d. Th.) der Titelverbindung erhalten.

¹H-NMR (400 MHz, DMSO-d₆, δ/ppm): 10.64 (s, 1H), 8.28 (d, 2H), 8.17 (s, 1H), 7.92 (m, 3H), 7.79 (d, 1H), 7.76 (d, 1H), 7.46 (d, 1H), 7.43 (d, 1H), 5.95 (s, 1H), 2.27 (s, 3H), 1.51 (s, 6H).

LC/MS (Methode 4, ESIpos): Rₜ = 1.04 min, m/z = 594 [M+H]⁺.

### Beispiel 5

### N-{4-Methyl-3-[6-(1H-pyrazol-4-yl)-1H-imidazo[1,2-b]pyrazol-1-yl]phenyl}-3-(trifluormethyl)-benzamid

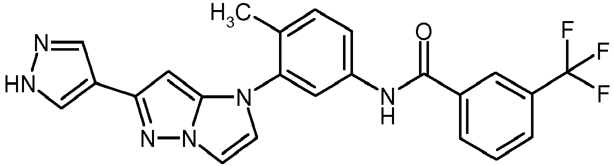

25 mg (0.025 mmol, 57% Reinheit) der Verbindung aus Beispiel 50A wurden in 0.5 ml Trifluoressigsäure 6 h bei 80°C gerührt. Nach Einengen des Ansatzes im Vakuum wurde der Rückstand mittels präparativer HPLC gereinigt (Methode 11). Es wurden 6.0 mg (53% d. Th.) der Titelverbindung erhalten.

¹H-NMR (400 MHz, DMSO-d₆, δ/ppm): 10.62 (s, 1H), 8.30 (s, 1H), 8.27 (d, 1H), 7.98 (d, 1H), 7.95 (d, 1H), 7.91 (s, 2H), 7.82-7.77 (m, 3H), 7.45 (d, 1H), 7.43 (d, 1H), 5.94 (s, 1H), 2.27 (s, 3H).

LC/MS (Methode 3, ESIpos): Rₜ = 0.94 min, m/z = 451 [M+H]⁺.

### Beispiel 6

### 3-(2-Methyl-1H-imidazol-1-yl)-N-{4-methyl-3-[6-(1H-pyrazol-4-yl)-1H-imidazo[1,2-b]pyrazol-1-yl]phenyl}-5-(trifluormethyl)benzamid

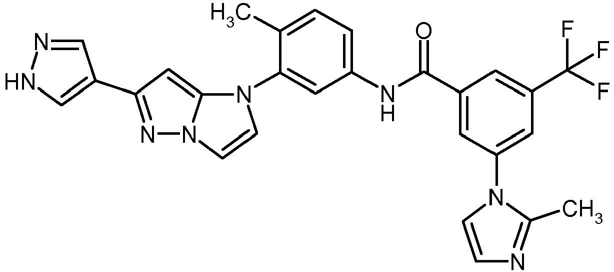

60 mg (0.09 mmol) der Verbindung aus Beispiel 51A wurden in Analogie zur Vorschrift von Beispiel 2 umgesetzt und aufgearbeitet. Es wurden 20 mg (40% d. Th.) der Titelverbindung erhalten.

¹H-NMR (400 MHz, DMSO-d₆, δ/ppm): 12.85 (s, 1H), 10.66 (s, 1H), 8.35 (s, 1H), 8.32 (s, 1H), 8.16 (s, 1H), 8.02 (s, 1H), 7.93 (d, 1H), 7.79 (m, 3H), 7.50 (d, 1H), 7.47 (d, 1H), 7.42 (d, 1H), 6.99 (d, 1H), 5.92 (s, 1H), 2.36 (s, 3H), 2.27 (s, 3H).

LC/MS (Methode 1, ESIpos): Rₜ = 0.91 min, m/z = 531 [M+H]⁺.

### Beispiel 7

### 3-tert.-Butyl-N-{4-methyl-3-[6-(1H-pyrazol-4-yl)-1H-imidazo[1,2-b]pyrazol-1-yl]phenyl}benzamid

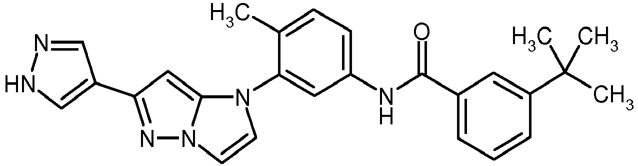

140 mg (0.2 mmol) der Verbindung aus Beispiel 52A wurden in Analogie zur Vorschrift von Beispiel 2 umgesetzt. Die Reinigung des Rohprodukts erfolgte hier durch präparative HPLC nach Methode 11. Es wurden 38 mg (44% d. Th.) der Titelverbindung erhalten.

¹H-NMR (400 MHz, DMSO-d₆, δ/ppm): 10.36 (s, 1H), 7.95 (d, 1H), 7.91 (m, 3H), 7.80-7.77 (m, 3H), 7.63 (d, 1H), 7.48-7.41 (m, 3H), 5.94 (s, 1H), 2.26 (s, 3H), 1.33 (s, 9H).

LC/MS (Methode 3, ESIpos): Rₜ = 1.02 min, m/z = 439 [M+H]⁺.

### Beispiel 8

### 3-tert.-Butyl-5-(4-methylpiperazin-1-yl)-N-{4-methyl-3-[6-(1H-pyrazol-4-yl)-1H-imidazo[1,2-b]-pyrazol-1-yl]phenyl}benzamid

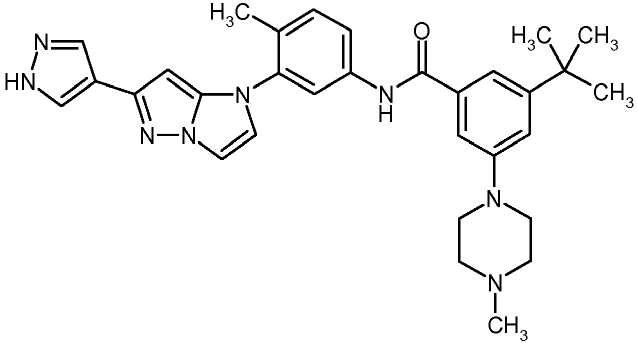

68 mg (76% Reinheit, 79 µmol) der Verbindung aus Beispiel 57A wurden in 0.32 ml Trifluoressigsäure vorgelegt und 3 h bei 80°C gerührt. Anschließend wurde direkt über präparative HPLC gereinigt (Methode 29). Die Produktfraktionen wurden im Vakuum eingeengt, der Rückstand in Ethylacetat aufgenommen und mit gesättigter wässriger Natriumhydrogencarbonat-Lösung gewaschen. Die organische Phase wurde über Natriumsulfat getrocknet, filtriert und im Vakuum eingeengt. Es wurden 41 mg (97% d. Th.) der Titelverbindung erhalten.

¹H-NMR (400 MHz, DMSO-d₆, δ/ppm): 12.84 (br. s, 1H), 10.25 (s, 1H), 8.01 (br. s, 1H), 7.92 (d, 1H), 7.84-7.74 (m, 3H), 7.44-7.39 (m, 2H), 7.37 (s, 1H), 7.27 (s, 1H), 7.14 (s, 1H), 5.92 (s, 1H), 3.23 (m, 4H), 2.31 (m, 2H), 2.25 (s, 3H), 1.31 (s, 9H) [weitere Signale unter Lösungsmittel-Peaks verborgen].

LC/MS (Methode 3, ESIpos): Rₜ = 0.76 min, m/z = 537 [M+H]⁺.

### Beispiel 9

### 3-tert.-Butyl-N-{4-methyl-3-[6-(1H-pyrazol-4-yl)-1H-imidazo[1,2-b]pyrazol-1-yl]phenyl}-5-(pyrrolidin-1-yhnethyl)benzamid

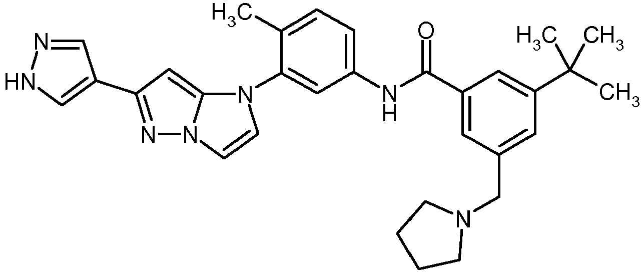

80 mg (0.13 mmol) der Verbindung aus Beispiel 53A wurden analog der Vorschrift zu Beispiel 2 umgesetzt. Es wurden 27 mg (39% d. Th.) der Titelverbindung erhalten.

¹H-NMR (400 MHz, DMSO-d₆, δ/ppm): 12.84 (br, 1H), 10.36 (s, 1H), 8.02 (s, 1H), 7.94 (d, 1H), 7.81-7.77 (m, 3H), 7.71 (s, 1H), 7.54 (s, 1H), 7.42 (m, 2H), 5.93 (s, 1H), 3.65 (br, 2H), 2.46 (br, 2H), 2.26 (s, 3H), 1.71 (br, 4H), 1.33 (s, 9H) [weitere Signale unter Lösungsmittel-Peaks verborgen].

LC/MS (Methode 3, ESIpos): Rₜ = 0.76 min, m/z = 522 [M+H]⁺.

### Beispiel 10

### N-{4-Methyl-3-[3-methyl-6-(1H-pyrazol-4-yl)-1H-imidazo[1,2-b]pyrazol-1-yl]phenyl}-3-(pentafluor-λ⁶-sulfanyl)-5-(piperazin-1-yl)benzamid

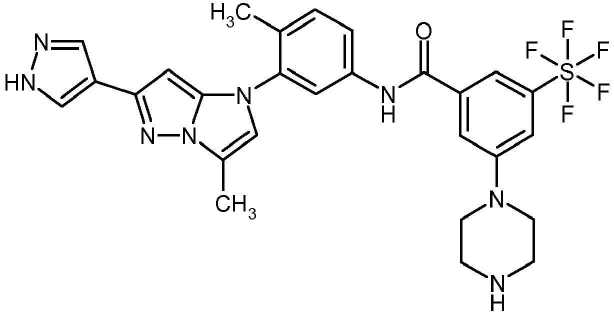

65 mg (0.16 mmol) der Verbindung aus Beispiel 9A, 68 mg (0.158 mmol) der Verbindung aus Beispiel 16A und 72 mg (0.19 mmol) HATU wurden in 0.9 ml DMF vorgelegt, mit 0.033 ml (0.19 mmol) *N*,*N-*Diisopropylethylamin versetzt und 1 h bei RT gerührt. Der Ansatz wurde danach in 10 ml 0.1 M Natronlauge eingerührt. Nach 10 min weiterem Rühren bei RT wurde der entstandene Niederschlag abfiltriert, mit Wasser gewaschen und getrocknet. Die so erhaltene Zwischenverbindung wurde in 0.5 ml Trifluoressigsäure gelöst und 6 h bei 80°C gerührt. Dann wurde der Ansatz im Vakuum eingeengt und der Rückstand durch zweimalige präparative HPLC gereinigt (Methode 18). Es wurden 18 mg (19% d. Th.) der Titelverbindung erhalten.

¹H-NMR (400 MHz, DMSO-d₆, δ/ppm): 12.84 (br, 1H), 10.51 (s, 1H), 8.10-7.80 (br, 2H), 7.88 (d, 1H), 7.74-7.69 (m, 3H), 7.46 (t, 1H), 7.43 (d, 1H), 7.15 (d, 1H), 5.93 (s, 1H), 3.22 (m, 4H), 2.84 (m, 4H), 2.41 (s, 3H), 2.27 (s, 3H).

LC/MS (Methode 3, ESIpos): Rₜ = 0.77 min, m/z = 607 [M+H]⁺.

### Beispiel 11

### N-{4-Methyl-3-[3-methyl-6-(1H-pyrazol-4-yl)-1H-imidazo[1,2-b]pyrazol-1-yl]phenyl}-3-(4-methylpiperazin-1-yl)-5-(pentafluor-λ⁶-sulfanyl)benzamid

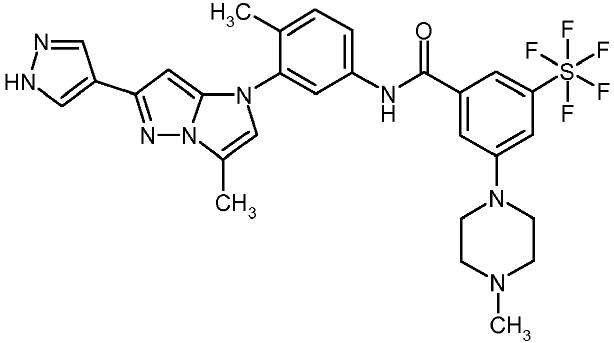

115 mg (0.16 mmol) der Verbindung aus Beispiel 54A wurden in Analogie zur Vorschrift von Beispiel 2 umgesetzt und aufgearbeitet. Es wurden 64 mg (65% d. Th.) der Titelverbindung erhalten.

¹H-NMR (400 MHz, DMSO-d₆, δ/ppm): 12.85 (br, 1H), 10.51 (s, 1H), 8.05-7.78 (br, 2H), 7.89 (d, 1H), 7.74-7.71 (m, 3H), 7.50 (s, 1H), 7.43 (d, 1H), 7.14 (d, 1H), 5.93 (s, 1H), 2.41 (s, 3H), 2.27 (s, 3H), 2.23 (s, 3H) [weitere Signale unter Lösungsmittel-Peaks verborgen].

LC/MS (Methode 3, ESIpos): Rₜ = 0.82 min, m/z = 621 [M+H]⁺.

### Beispiel 12

### 3-Cyano-N-{4-methyl-3-[3-methyl-6-(1H-pyrazol-4-yl)-1H-imidazo[1,2-b]pyrazol-1-yl]phenyl}-5-(pentafluor-λ⁶-sulfanyl)benzamid

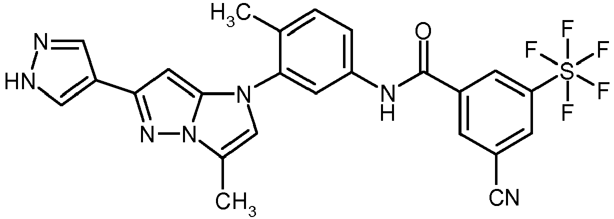

115 mg (0.23 mmol) der Verbindung aus Beispiel 56A wurden in 1.5 ml Trifluoressigsäure 60 min bei 90°C gerührt. Der Ansatz wurde danach im Vakuum eingeengt und der Rückstand mittels präparativer HPLC (Methode 21) gereinigt. Die Produktfraktionen wurden vereinigt, im Vakuum fast vollständig eingeengt und mit etwas gesättigter wässriger Natriumhydrogencarbonat-Lösung alkalisch gestellt. Der dabei entstandene Niederschlag wurde abfiltriert, mit Wasser gewaschen und im Hochvakuum getrocknet. Es wurden 60 mg (46% d. Th.) der Titelverbindung erhalten.

¹H-NMR (400 MHz, DMSO-d₆, δ/ppm): 12.85 (br, 1H), 10.76 (s, 1H), 8.85 (s, 1H), 8.74 (s, 1H), 8.66 (s, 1H), 8.05-7.78 (br, 2H), 7.90 (d, 1H), 7.73 (dd, 1H), 7.46 (d, 1H), 7.16 (d, 1H), 5.93 (s, 1H), 2.41 (s, 3H), 2.29 (s, 3H).

LC/MS (Methode 3, ESIpos): Rₜ = 1.07 min, m/z = 548 [M+H]⁺.

### Beispiel 13

### 3-Methoxy-N-{4-methyl-6-[3-methyl-6-(1H-pyrazol-4-yl)-1H-imidazo[1,2-b]pyrazol-1-yl]phenyl}-5-(pentafluor-λ⁶-sulfanyl)benzamid

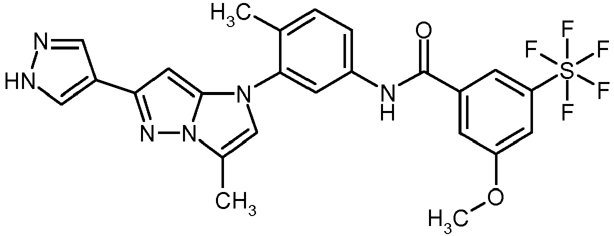

60 mg (0.15 mmol) der Verbindung aus Beispiel 9A und 40 mg (0.15 mmol) der Verbindung aus Beispiel 25A wurden analog der Vorschrift zu Beispiel 10 umgesetzt, mit dem Unterschied, dass hier nur 3 h (statt 6 h) mit Trifluoressigsäure bei 80°C gerührt wurde. Es wurden 36 mg (43% d. Th.) der Titelverbindung erhalten.

¹H-NMR (400 MHz, DMSO-d₆, δ/ppm): 12.84 (br, 1H), 10.59 (s, 1H), 8.04 (s, 1H), 7.99 (s, 1H), 7.90 (d, 1H), 7.83 (s, 1H), 7.80 (s, 1H), 7.73 (dd, 1H), 7.63 (s, 1H), 7.43 (d, 1H), 7.15 (s, 1H), 5.93 (s, 1H), 3.94 (s, 3H), 2.41 (s, 3H), 2.28 (s, 3H).

LC/MS (Methode 4, ESIpos): Rₜ = 1.10 min, m/z = 553 [M+H]⁺.

### Beispiel 14

### 3-(2-Methyl-1H-imidazol-1-yl)-N-{4-methyl-3-[3-methyl-6-(1H-pyrazol-4-yl)-1H-imidazo[1,2-b]-pyrazol-1-yl]phenyl}-5-(trifluormethyl)benzamid

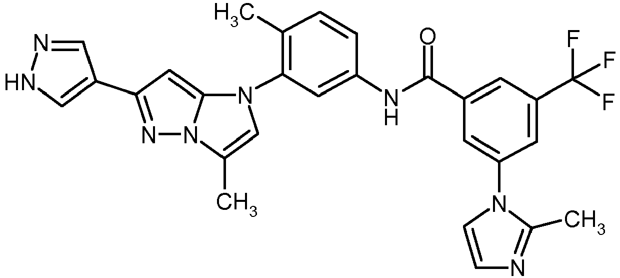

70 mg (0.17 mmol) der Verbindung aus Beispiel 9A und 50 mg (0.19 mmol) 3-(2-Methyl-1*H-*imidazol-1-yl)-5-(trifluormethyl)benzoesäure [Lit.: WO 2004/005281-A1, Beispiel 91b] wurden analog der Vorschrift zu Beispiel 13 umgesetzt. Es wurden 58 mg (71% d. Th.) der Titelverbindung erhalten.

¹H-NMR (400 MHz, DMSO-d₆, δ/ppm): 12.84 (br, 1H), 10.63 (s, 1H), 8.35 (s, 1H), 8.32 (s, 1H), 8.16 (s, 1H), 8.04 (s, 1H), 7.92 (d, 1H), 7.80 (s, 1H), 7.75 (dd, 1H), 7.50 (d, 1H), 7.45 (d, 1H), 7.15 (d, 1H), 6.99 (d, 1H), 5.93 (s, 1H), 2.41 (s, 3H), 2.35 (s, 3H), 2.28 (s, 3H).

LC/MS (Methode 4, ESIpos): Rₜ = 0.73 min, m/z = 545 [M+H]⁺.

### Beispiel 15

### N-{4-Methyl-3-[6-(pyridin-3-yl)-1H-imidazo[1,2-b]pyrazol-1-yl]phenyl}-3-(pentafluor-λ⁶-sulfanyl)-benzamid

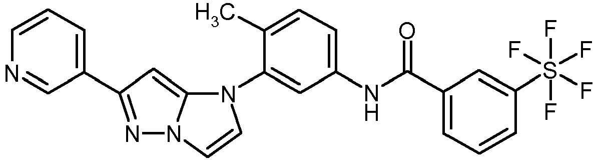

150 mg (0.52 mmol) der Verbindung aus Beispiel 6A, 128 mg (0.52 mmol) 3-(Pentafluor-λ⁶-sulfanyl)benzoesäure und 237 mg (0.62 mmol) HATU wurden in 1.8 ml DMF vorgelegt, mit 0.11 ml (0.62 mmol) *N*,*N-*Diisopropylethylamin versetzt und 1 h bei RT gerührt. Der Ansatz wurde danach in 15 ml 0.1 M Natronlauge eingerührt. Nach 10 min weiterem Rühren bei RT wurde der entstandene Niederschlag abfiltriert, mit Wasser gewaschen und getrocknet. Es wurden 235 mg (83% d. Th.) der Titelverbindung erhalten.

¹H-NMR (400 MHz, DMSO-d₆, δ/ppm) 10.69 (s, 1H), 9.06 (s, 1H), 8.48 (d, 1H), 8.41 (s, 1H), 8.28 (s, 1H), 8.20-8.16 (m, 2H), 7.96 (d, 1H), 7.92 (d, 1H), 7.84-7.79 (m, 2H), 7.55 (d, 1H), 7.48 (d, 1H), 7.41 (dd, 1H), 6.37 (s, 1H), 2.28 (s, 3H).

LC/MS (Methode 2, ESIpos): Rₜ = 2.24 min, m/z = 520 [M+H]⁺.

### Beispiel 16

### 3-(2-Methyl-1H-imidazol-1-yl)-N-{4-methyl-3-[6-(pyridin-3-yl)-1H-imidazo[1,2-b]pyrazol-1-yl]-phenyl}-5-(pentafluor-λ⁶-sulfanyl)benzamid

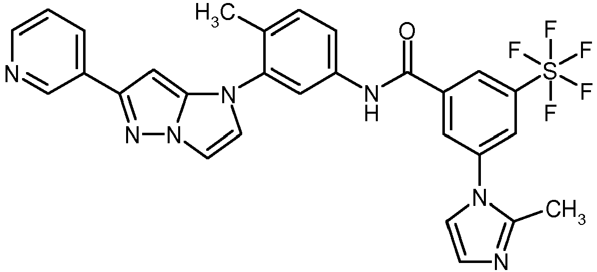

50 mg (0.17 mmol) der Verbindung aus Beispiel 6A, 57 mg (0.17 mmol) der Verbindung aus Beispiel 26A und 79 mg (0.207 mmol) HATU wurden in 0.6 ml DMF vorgelegt, mit 36 µl (0.207 mmol) *N*,*N*-Diisopropylethylamin versetzt und 16 h bei RT gerührt. Der Ansatz wurde danach in 10 ml 0.1 M Natronlauge eingerührt. Nach 10 min weiterem Rühren bei RT wurde der entstandene Niederschlag abfiltriert, mit Wasser gewaschen und getrocknet. Es wurden 66 mg (62% d. Th.) der Titelverbindung erhalten.

¹H-NMR (400 MHz, DMSO-d₆, δ/ppm): 10.70 (s, 1H), 9.05 (s, 1H), 8.47 (m, 2H), 8.36 (s, 2H), 8.19 (d, 1H), 7.94 (m, 2H), 7.80 (d, 1H), 7.55 (d, 1H), 7.49 (m, 2H), 7.42 (m, 2H), 6.99 (s, 1H), 6.36 (s, 1H), 2.35 (s, 3H), 2.28 (s, 3H).

LC/MS (Methode 1, ESIpos): Rₜ = 1.86 min, m/z = 600 [M+H]⁺.

### Beispiel 17

### N-{4-Methyl-3-[6-(pyridin-3-yl)-1H-imidazo[1,2-b]pyrazol-1-yl]phenyl}-3-(pentafluor-λ⁶-sulfanyl)-5-(piperazin-1-yl)benzamid

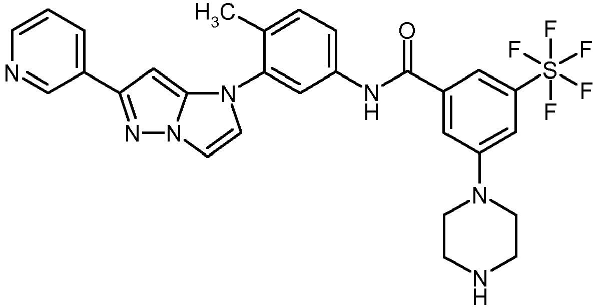

120 mg (0.42 mmol) der Verbindung aus Beispiel 6A und 179 mg (0.42 mmol) der Verbindung aus Beispiel 16A wurden in Analogie zur Vorschrift von Beispiel 16 umgesetzt und aufgearbeitet. Es wurden auf diese Weise 260 mg (89% d. Th.) des Boc-geschützten Zwischenprodukts *tert.*-Butyl-4-[3-({4-methyl-3-[6-(pyridin-3-yl)-1*H*-imidazo[1,2-b]pyrazol-1-yl]phenyl}carbamoyl)-5-(pentafluor-λ⁶-sulfanyl)phenyl]piperazin-1-carboxylat erhalten. Diese Verbindung wurde in 3 ml 1,4-Dioxan und 1 ml Methanol gelöst, mit 0.31 ml (1.24 mmol) einer 4 M Lösung von Chlorwasserstoff in 1,4-Dioxan versetzt und 1 h bei 80°C gerührt. Anschließend wurde der Ansatz im Vakuum eingeengt und der Rückstand über präparative HPLC gereinigt (Methode 18). Die Produktfraktionen wurden eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden so 155 mg (62% d. Th.) der Titelverbindung erhalten.

¹H-NMR (400 MHz, DMSO-d₆, δ/ppm): 10.54 (s, 1H), 9.06 (d, 1H), 8.48 (dd, 1H), 8.19 (m, 1H), 7.92 (m, 2H), 7.78 (dd, 1H), 7.72 (s, 1H), 7.69 (s, 1H), 7.55 (d, 1H), 7.47 (m, 2H), 7.41 (dd, 1H), 6.37 (s, 1H), 3.22 (m, 4H), 2.85 (m, 4H), 2.27 (s, 3H).

LC/MS (Methode 1, ESIpos): Rₜ = 0.96 min, m/z = 604 [M+H]⁺.

### Beispiel 18

### 3-(4-Methylpiperazin-1-yl)-N-{4-methyl-3-[6-(pyridin-3-yl)-1H-imidazo[1,2-b]pyrazol-1-yl]-phenyl}-5-(pentafluor-λ⁶-sulfanyl)benzamid

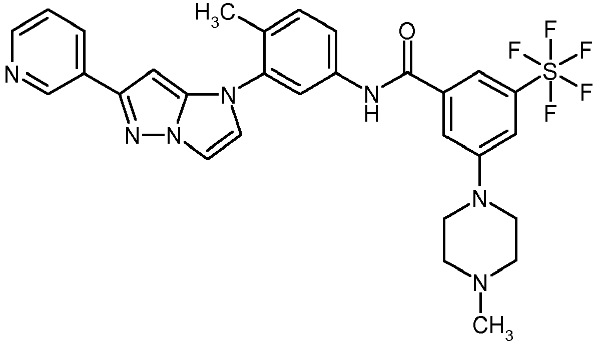

50 mg (0.17 mmol) der Verbindung aus Beispiel 6A und 79.6 mg (0.17 mmol) der Verbindung aus Beispiel 17A wurden in Analogie zur Vorschrift von Beispiel 16 umgesetzt und aufgearbeitet. Es wurden 82 mg (77% d. Th.) der Titelverbindung erhalten.

¹H-NMR (400 MHz, DMSO-d₆, δ/ppm): 10.55 (s, 1H), 9.06 (d, 1H), 8.48 (d, 1H), 8.19 (m, 1H), 7.93 (m, 2H), 7.79 (m, 1H), 7.73 (s, 1H), 7.71 (s, 1H), 7.50-7.47 (m, 2H), 7.41 (dd, 1H), 6.37 (s, 1H), 3.22 (m, 4H), 2.85 (m, 4H), 2.28 (s, 3H), 2.23 (s, 3H) [weitere Signale unter Lösungsmittel-Peaks verborgen].

LC/MS (Methode 3, ESIpos): Rₜ = 0.76 min, m/z = 618 [M+H]⁺.

### Beispiel 19

### N-{4-Methyl-3-[6-(pyridin-3-yl)-1H-imidazo[1,2-b]pyrazol-1-yl]phenyl}-3-(morpholin-4-yl)-5-(pentafluor-λ⁶-sulfanyl)benzamid

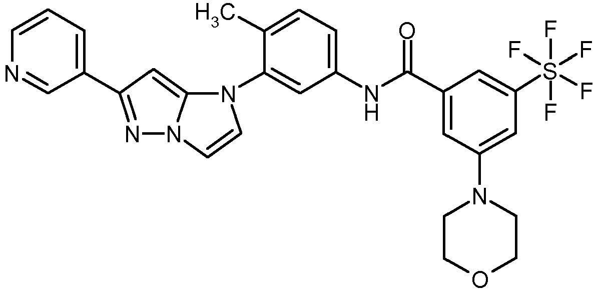

50 mg (0.17 mmol) der Verbindung aus Beispiel 6A, 58 mg (0.17 mmol) der Verbindung aus Beispiel 18A und 79 mg (0.21 mmol) HATU wurden in 1.0 ml DMF gelöst, mit 38 µl (0.35 mmol) 4-Methylmorpholin versetzt und 16 h bei RT gerührt. Man versetzte danach mit 10 ml 0.1 M Natronlauge und rührte noch 10 min bei RT nach. Der entstandene Niederschlag wurde ab filtriert, mit Wasser gewaschen und im Hochvakuum getrocknet. Dieses Rohprodukt wurde anschließend mittels präparativer HPLC (Methode 29) gereinigt. Die Produktfraktionen wurden im Vakuum eingeengt, der Rückstand in Ethylacetat aufgenommen und mit gesättigter Natriumhydrogencarbonat-Lösung gewaschen. Die organische Phase wurde über Natriumsulfat getrocknet, filtriert und im Vakuum eingeengt. Es wurden 55 mg (53% d. Th.) der Titelverbindung erhalten.

¹H-NMR (400 MHz, DMSO-d₆, δ/ppm): 10.56 (s, 1H), 9.05 (d, 1H), 8.48 (dd, 1H), 8.19 (dt, 1H), 7.93 (t, 2H), 7.81-7.76 (m, 2H), 7.72 (s, 1H), 7.55 (d, 1H), 7.53 (s, 1H), 7.48 (d, 1H), 7.42 (dd, 1H), 6.37 (s, 1H), 3.77 (t, 4H), 2.28 (s, 3H).

LC/MS (Methode 3, ESIpos): Rₜ = 1.03 min, m/z = 605 [M+H]⁺.

### Beispiel 20

### 3-Hydroxy-N-{4-methyl-3-[6-(pyridin-3-yl)-1H-imidazo[1,2-b]pyrazol-1-yl]phenyl}-5-(pentafluor-λ⁶-sulfanyl)benzamid

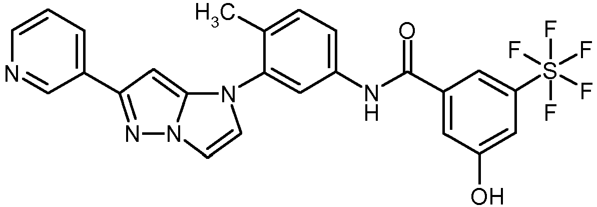

300 mg (1.04 mmol) der Verbindung aus Beispiel 6A, 301 mg (1.14 mmol) der Verbindung aus Beispiel 24A und 473 mg (1.24 mmol) HATU wurden in 3.6 ml DMF vorgelegt, mit 0.36 ml (2.07 mmol) *N*,*N*-Diisopropylethylamin versetzt und zwei Tage bei RT gerührt. Der Ansatz wurde danach direkt mittels präparativer HPLC (Methode 18) in seine Komponenten aufgetrennt. Es wurden 180 mg (31% d. Th.) der Titelverbindung erhalten.

¹H-NMR (400 MHz, DMSO-d₆, δ/ppm): 10.73 (s, 1H), 10.60 (s, 1H), 9.05 (d, 1H), 8.48 (dd, 1H), 8.19 (m, 1H), 7.95 (d, 1H), 7.92 (d, 1H), 7.86 (s, 1H), 7.79 (dd, 1H), 7.65 (s, 1H), 7.55 (d, 1H), 7.48-7.40 (m, 3H), 6.37 (s, 1H), 2.28 (s, 3H).

LC/MS (Methode 3, ESIpos): Rₜ = 0.92 min, m/z = 536 [M+H]⁺.

### Beispiel 21

### 3-Methoxy-N-{4-methyl-3-[6-(pyridin-3-yl)-1H-imidazo[1,2-b]pyrazol-1-yl]phenyl}-5-(pentafluoro-λ⁶-sulfanyl)benzamid

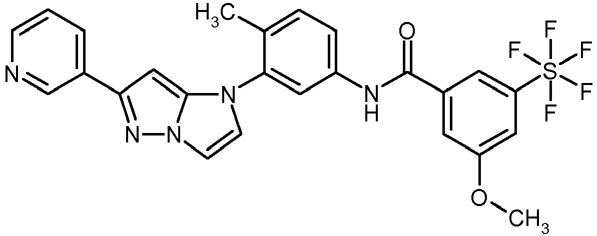

30 mg (0.10 mmol) der Verbindung aus Beispiel 6A und 29 mg (0.10 mmol) der Verbindung aus Beispiel 25A wurden in Analogie zur Vorschrift von Beispiel 16 umgesetzt und aufgearbeitet. Es wurden 32 mg (55% d. Th.) der Titelverbindung erhalten.

¹H-NMR (400 MHz, DMSO-d₆, δ/ppm): 10.63 (s, 1H), 9.06 (d, 1H), 8.48 (d, 1H), 8.19 (m, 1H), 8.00 (s, 1H), 7.94 (d, 1H), 7.92 (d, 1H), 7.84 (s, 1H), 7.79 (dd, 1H), 7.63 (s, 1H), 7.55 (d, 1H), 7.48 (d, 1H), 7.42 (dd, 1H), 6.37 (s, 1H), 3.94 (s, 3H), 2.28 (s, 3H).

LC/MS (Methode 3, ESIpos): Rₜ = 1.08 min, m/z = 550 [M+H]⁺.

### Beispiel 22

### 3-(2-Aminoethoxy)-N-{4-methyl-3-[6-(pyridin-3-yl)-1H-imidazo[1,2-b]pyrazol-1-yl]phenyl}-5-(pentafluor-λ⁶-sulfanyl)benzamid

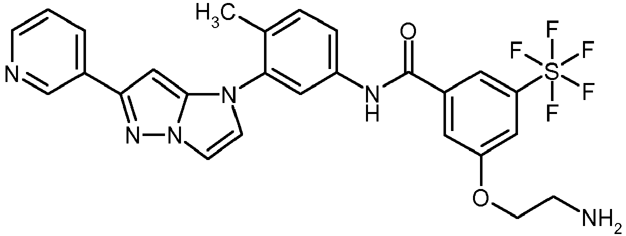

40 mg (0.14 mmol) der Verbindung aus Beispiel 6A und 62 mg (0.15 mmol) der Verbindung aus Beispiel 29A wurden in Analogie zur Vorschrift von Beispiel 16 umgesetzt und aufgearbeitet. Es wurden auf diese Weise 81 mg (86% d. Th.) des Boc-geschützten Zwischenprodukts *tert.*-Butyl-{2-[3-({4-methyl-3-[6-(pyridin-3-yl)-1*H*-imidazo[1,2-b]pyrazol-1-yl]phenyl}carbamoyl)-5-(pentafluor-λ⁶-sulfanyl)phenoxy]ethyl}carbamat erhalten. Diese Verbindung wurde in 1 ml Dichlormethan und 0.5 ml Trifluoressigsäure 2 h bei RT gerührt. Anschließend wurde im Vakuum eingeengt und der Rückstand mittels präparativer HPLC gereinigt (Methode 18). Die produkthaltigen Fraktionen wurden vereinigt und im Vakuum eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden so 61 mg (76% d. Th.) der Titelverbindung erhalten.

¹H-NMR (400 MHz, DMSO-d₆, δ/ppm): 10.63 (s, 1H), 9.05 (d, 1H), 8.48 (dd, 1H), 8.19 (m, 1H), 7.99 (s, 1H), 7.95 (d, 1H), 7.92 (d, 1H), 7.84 (s, 1H), 7.79 (dd, 1H), 7.63 (t, 1H), 7.55 (d, 1H), 7.48 (d, 1H), 7.41 (dd, 1H), 6.37 (s, 1H), 4.11 (t, 2H), 2.91 (t, 2H), 2.28 (s, 3H).

LC/MS (Methode 4, ESIpos): Rₜ = 0.71 min, m/z = 579 [M+H]⁺.

### Beispiel 23

### 3-(3-Aminopropoxy)-N-{4-methyl-3-[6-(pyridin-3-yl)-1H-imidazo[1,2-b]pyrazol-1-yl]phenyl}-5-(pentafluor-λ⁶-sulfanyl)benzamid

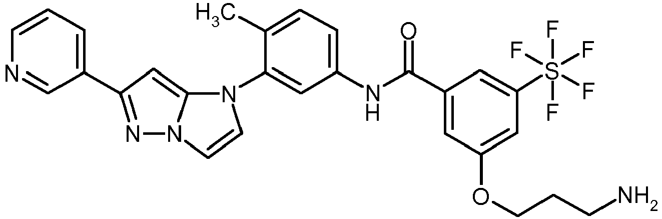

50 mg (0,17 mmol) der Verbindung aus Beispiel 6A und 73 mg (0.17 mmol) der Verbindung aus Beispiel 30A wurden in Analogie zur Vorschrift von Beispiel 16 umgesetzt und aufgearbeitet. Es wurden auf diese Weise 118 mg (98% d. Th.) des Boc-geschützten Zwischenprodukts *tert*.-Butyl-{3-[3-({4-methyl-3-[6-(pyridin-3-yl)-1*H*-imidazo[1,2-b]pyrazol-1-yl]phenyl}carbamoyl)-5-(pentafluor-λ⁶-sulfanyl)phenoxy]propyl}carbamat erhalten. Diese Verbindung wurde in 3 ml 1,4-Dioxan und 1 ml Methanol gelöst, mit 0.13 ml (0.52 mmol) einer 4 M Lösung von Chlorwasserstoff in 1,4-Dioxan versetzt und 1 h bei 80°C gerührt. Anschließend wurde im Vakuum eingeengt und der Rückstand mittels präparativer HPLC gereinigt (Methode 18). Die produkthaltigen Fraktionen wurden vereinigt und im Vakuum eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden so 41 mg (38% d. Th.) der Titelverbindung erhalten.

¹H-NMR (400 MHz, DMSO-d₆, δ/ppm): 10.61 (br, 1H), 9.05 (d, 1H), 8.48 (dd, 1H), 8.19 (m, 1H), 7.98 (s, 1H), 7.95 (d, 1H), 7.92 (d, 1H), 7.84 (s, 1H), 7.80 (dd, 1H), 7.63 (t, 1H), 7.55 (d, 1H), 7.48 (d, 1H), 7.42 (dd, 1H), 6.37 (s, 1H), 4.22 (t, 2H), 2.71 (t, 2H), 2.28 (s, 3H), 1.83 (m, 2H).

LC/MS (Methode 3, ESIpos): Rₜ = 0.76 min, m/z = 593 [M+H]⁺.

### Beispiel 24

### 3-(Azetidin-3-yloxy)-N-{4-methyl-3-[6-(pyridin-3-yl)-1H-imidazo[1,2-b]pyrazol-1-yl]phenyl}-5-(pentafluor-λ⁶-sulfanyl)benzamid

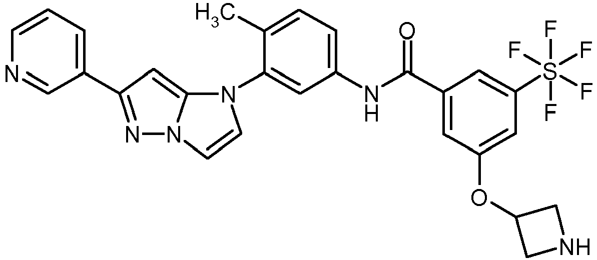

37.6 mg (0.13 mmol) der Verbindung aus Beispiel 6A und 60 mg (0.14 mmol) der Verbindung aus Beispiel 31A wurden in Analogie zur Vorschrift von Beispiel 16 umgesetzt, mit dem Unterschied, dass die Isolierung des Zwischenprodukts, der Boc-geschützten Verbindung *tert*.-Butyl-3-[3-({4-methyl-3-[6-(pyridin-3-yl)-1*H*-imidazo[1,2-b]pyrazol-1-yl]phenyl}carbamoyl)-5-(pentafluor-λ⁶-sulfanyl)phenoxy]azetidin-1-carboxylat, hier mittels präparativer HPLC (Methode 11) erfolgte. Es wurden auf diese Weise 42 mg (47% d. Th.) des Boc-geschützten Zwischenprodukts erhalten. Diese Verbindung wurde in 1 ml Methylenchlorid gelöst, mit 0.5 ml Trifluoressigsäure versetzt und 2 h bei RT gerührt. Anschließend wurde der Ansatz im Vakuum eingeengt und der Rückstand mittels präparativer HPLC gereinigt (Methode 18). Nach Einengen der produkthaltigen Fraktionen wurde der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden so 24 mg (90% Reinheit, 28% d. Th.) der Titelverbindung erhalten.

¹H-NMR (400 MHz, DMSO-d₆, δ/ppm): 10.64 (br, 1H), 9.05 (d, 1H), 8.48 (dd, 1H), 8.19 (m, 1H), 8.01 (s, 1H), 7.94 (d, 1H), 7.92 (d, 1H), 7.79 (d, 1H), 7.67 (s, 1H), 7.55 (d, 1H), 7.47 (m, 2H), 7.42 (dd, 1H), 6.37 (s, 1H), 5.22 (quint, 1H), 3.80 (t, 2H), 3.52 (t, 1H), 2.28 (s, 3H), 1.83 (m, 1H).

LC/MS (Methode 4, ESIpos): Rₜ = 0.73 min, m/z = 591 [M+H]⁺.

### Beispiel 25

### N-{4-Methyl-3-[6-(pyridin-3-yl)-1H-imidazo[1,2-b]pyrazol-1-yl]phenyl}-3-(pentafluor-λ⁶-sulfanyl)-5-(pyrrolidin-3-yloxy)benzamid

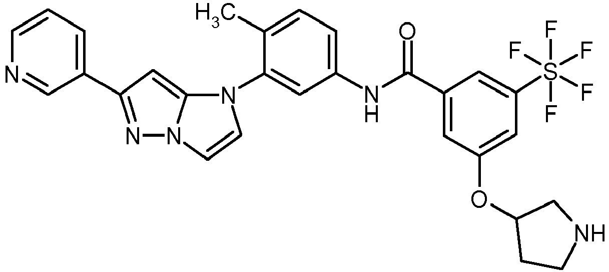

150 mg (0.28 mmol) der Verbindung aus Beispiel 20, 89 mg (0.34 mmol) *tert.*-Butyl-3-[(methyl-sulfonyl)oxy]pyrrolidin-1-carboxylat [Lit. z.B.: P. Kocalka et al., Tetrahedron 2006, 62 (24), 5763-5774] und 201 mg (0.62 mmol) Cäsiumcarbonat wurden in 3 ml DMF 6 h lang auf 90°C erhitzt. Der Ansatz wurde danach in 15 ml 0.1 M Natronlauge eingerührt und das Gemisch noch 10 min bei RT nachgerührt. Der entstandene Niederschlag wurde abfiltriert, mit Wasser gewaschen und im Vakuum getrocknet. Das so erhaltene Zwischenprodukt wurde in 3 ml Dichlormethan und 1 ml Trifluoressigsäure 1 h bei RT gerührt. Anschließend wurde im Vakuum eingeengt und der Rückstand mittels präparativer HPLC gereinigt (Methode 18). Nach Einengen der produkthaltigen Fraktionen wurde der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden so 101 mg (90% Reinheit, 60% d. Th.) der Titelverbindung erhalten.

¹H-NMR (400 MHz, DMSO-d₆, δ/ppm): 10.70 (br, 1H), 9.05 (s, 1H), 8.48 (d, 1H), 8.19 (d, 1H), 7.99 (s, 1H), 7.94 (s, 1H), 7.92 (d, 1H), 7.81 (s, 1H), 7.55 (m, 2H), 7.47 (s, 1H), 7.41 (dd, 1H), 6.37 (s, 1H), 5.09 (br, 1H), 3.07 (dd, 1H), 2.89 (m, 2H), 2.79 (m, 1H), 2.28 (s, 3H), 2.05 (m, 1H), 1.78 (m, 1H).

LC/MS (Methode 3, ESIpos): Rₜ = 0.76 min, m/z = 605 [M+H]⁺.

### Beispiel 26

### N-{4-Methyl-3-[6-(pyridin-3-yl)-1H-imidazo[1,2-b]pyrazol-1-yl]phenyl}-3-(methylsulfonyl)-5-(pentafluor-λ⁶-sulfanyl)benzamid

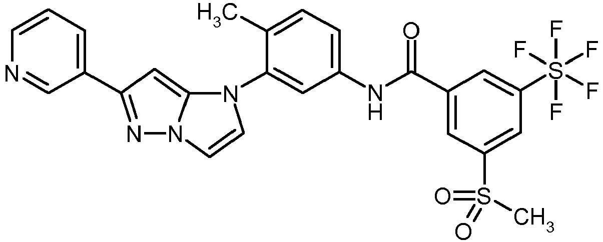

66 mg (0.228 mmol) der Verbindung aus Beispiel 6A und 74 mg (0.228 mmol) der Verbindung aus Beispiel 32A wurden in 1.5 ml wasserfreiem DMF gelöst und nacheinander mit 104 mg (0.273 mmol) HATU und 48 µl (0.273 mmol) *N*,*N*-Diisopropylethylamin versetzt. Nachdem das Reaktionsgemisch 4 h bei RT gerührt worden war, wurde es komplett mittels präparativer HPLC (Methode 9) in seine Komponenten aufgetrennt. Die Produktfraktionen wurden vereinigt und am Rotationsverdampfer bis zur Trockene eingeengt. Hierdurch wurde die Titelverbindung in Form ihres Ameisensäure-Salzes erhalten. Zur Überführung in die salzfreie Form wurde das Formiat in ca. 5 ml Methanol gelöst und über eine Hydrogencarbonat-Kartusche gegeben (Fa. Polymerlabs, Stratospheres SPE, PL-HCO₃ MP SPE, Kapazität 0.9 mmol). Nach neuerlichem Eindampfen wurde der Rückstand mit wenig Diisopropylether bei RT verrührt. Nach Absaugen und Trocknen des Feststoffs im Hochvakuum wurden 72.6 mg (53% d. Th.) der Titelverbindung erhalten.

¹H-NMR (400 MHz, CDCl₃, δ/ppm): 9.73 (br. s, 1H), 8.97 (s, 1H), 8.69-8.68 (m, 2H), 8.49 (d, 1H), 8.44 (s, 1H), 8.08 (d, 1H), 7.80 (s, 1H), 7.73 (dd, 1H), 7.47 (d, 1H), 7.38 (d, 1H), 7.31 (dd, 1H), 6.95 (d, 1H), 5.94 (s, 1H), 3.12 (s, 3H), 2.31 (s, 3H).

LC/MS (Methode 4, ESIpos): Rₜ = 0.97 min, m/z = 598 [M+H]⁺.

### Beispiel 27

### 3-Chlor-N-{4-methyl-3-[6-(pyridin-3-yl)-1H-imidazo[1,2-b]pyrazol-1-yl]phenyl}-5-(pentafluor-λ⁶-sulfanyl)benzamid

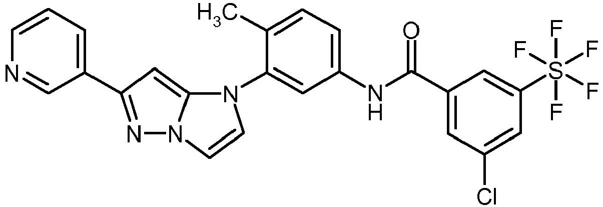

Analog zu dem unter Beispiel 26 beschriebenen Verfahren wurden aus 80 mg (0.276 mmol) der Verbindung aus Beispiel 6A und 78 mg (0.276 mmol) der Verbindung aus Beispiel 33A 101 mg (66% d. Th.) der Titelverbindung erhalten.

¹H-NMR (400 MHz, CDCl₃, δ/ppm): 9.00 (d, 1H), 8.85 (br. s, 1H), 8.50 (dd, 1H), 8.22 (s, 1H), 8.11 (dt, 1H), 8.08 (s, 1H), 7.91 (t, 1H), 7.81 (s, 1H), 7.63 (dd, 1H), 7.48 (d, 1H), 7.39 (d, 1H), 7.32 (dd, 1H), 6.95 (d, 1H), 5.96 (s, 1H), 2.31 (s, 3H).

LC/MS (Methode 4, ESIpos): Rₜ = 1.15 min, m/z = 554/556 [M+H]⁺.

### Beispiel 28

### 3-Cyano-N-{4-methyl-3-[6-(pyridin-3-yl)-1H-imidazo[1,2-b]pyrazol-1-yl]phenyl}-5-(pentafluor-λ⁶-sulfanyl)benzamid

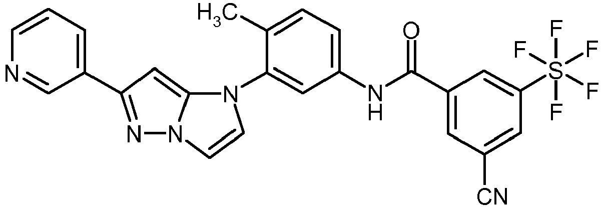

1.00 g (3.46 mmol) der Verbindung aus Beispiel 6A, 944 mg (3.46 mmol) der Verbindung aus Beispiel 23A und 1.58 g (4.15 mmol) HATU wurden in 12.1 ml wasserfreiem DMF gelöst und mit 0.72 ml (4.15 mmol) *N*,*N*-Diisopropylethylamin versetzt. Nachdem das Reaktionsgemisch 1 h bei RT gerührt worden war, wurde es in 120 ml 0.1 M Natronlauge eingerührt. Nach 10 min weiterem Rühren bei RT wurde der entstandene Niederschlag abfiltriert und getrocknet. Dieses Rohprodukt wurde anschließend mittels präparativer HPLC (Methode 23) in seine Komponenten aufgetrennt. Die Produktfraktionen wurden vereinigt und am Rotationsverdampfer bis zur Trockene eingeengt. Das erhaltene Produkt wurde in einem Gemisch aus 10 ml Acetonitril und 10 ml Wasser suspendiert, mit etwas gesättigter Natriumhydrogencarbonat-Lösung alkalisch gestellt und 10 min bei RT gerührt. Der Feststoff wurde abfiltriert, mit Wasser gewaschen und getrocknet. Es wurden 1.12 g (69% d. Th.) der Titelverbindung erhalten.

¹H-NMR (400 MHz, DMSO-d₆, δ/ppm): 10.80 (s, 1H), 9.05 (d, 1H), 8.86 (s, 1H), 8.75 (s, 1H), 8.66 (s, 1H), 8.49 (d, 1H), 8.19 (dt, 1H), 7.94 (dd, 2H), 7.79 (dd, 1H), 7.56 (d, 1H), 7.50 (d, 1H), 7.42 (dd, 1H), 6.37 (s, 1H), 2.29 (s, 3H).

LC/MS (Methode 3, ESIpos): Rₜ = 1.01 min, m/z = 545 [M+H]⁺.

### Beispiel 29

### N-{4-Methyl-3-[6-(pyridin-3-yl)-1H-imidazo[1,2-b]pyrazol-1-yl]phenyl}-5-(pentafluor-λ⁶-sulfanyl)-isophthalamid

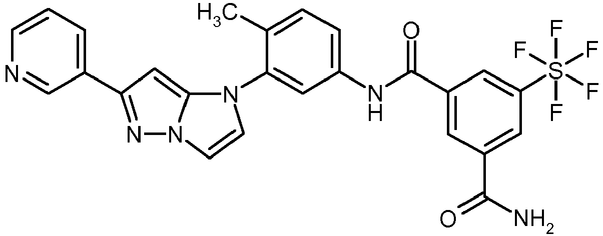

Die Titelverbindung wurde bei der Herstellung und Aufarbeitung von Beispiel 28 als Nebenprodukt isoliert. Die entsprechenden Fraktionen aus der HPLC-Trennung (nach Methode 23) wurden vereinigt und am Rotationsverdampfer bis zur Trockene eingeengt. Das erhaltene Produkt wurde anschließend in einem Gemisch aus 2 ml Acetonitril und 2 ml Wasser suspendiert, mit etwas gesättigter Natriumhydrogencarbonat-Lösung alkalisch gestellt und 10 min bei RT gerührt. Der Feststoff wurde abfiltriert, mit Wasser gewaschen und getrocknet. Es wurden so 154 mg der Titelverbindung erhalten.

¹H-NMR (400 MHz, DMSO-d₆, δ/ppm): 10.80 (s, 1H), 9.05 (d, 1H), 8.77 (s, 1H), 8.55 (d, 2H), 8.48 (dd, 1H), 8.46 (br, 1H), 8.19 (dt, 1H), 7.96 (d, 1H), 7.93 (d, 1H), 7.86 (br, 1H), 7.81 (dd, 1H), 7.56 (d, 1H), 7.49 (d, 1H), 7.42 (dd, 1H), 6.37 (s, 1H), 2.28 (s, 3H).

LC/MS (Methode 4, ESIpos): Rₜ = 0.87 min, m/z = 563 [M+H]⁺.

### Beispiel 30

### 3-Methyl-N-{4-methyl-3-[6-(pyridin-3-yl)-1H-imidazo[1,2-b]pyrazol-1-yl]phenyl}-5-(pentafluor-λ⁶-sulfanyl)benzamid

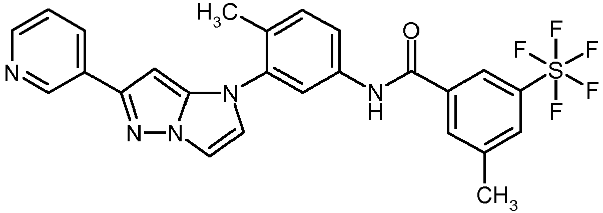

Analog zu dem unter Beispiel 26 beschriebenen Verfahren wurden aus 80 mg (0.276 mmol) der Verbindung aus Beispiel 6A und 73 mg (0.276 mmol) der Verbindung aus Beispiel 34A 101 mg (61% d. Th., 90% Reinheit) der Titelverbindung erhalten.

¹H-NMR (400 MHz, CDCl₃, δ/ppm): 9.02 (d, 1H), 8.51 (br. s, 1H), 8.51 (dd, 1H), 8.13 (dt, 1H), 8.09 (s, 1H), 7.86-7.84 (m, 2H), 7.73 (s, 1H), 7.60 (dd, 1H), 7.49 (d, 1H), 7.38 (d, 1H), 7.31 (dd, 1H), 6.95 (d, 1H), 5.99 (s, 1H), 2.49 (s, 3H), 2.30 (s, 3H).

LC/MS (Methode 4, ESIpos): Rₜ = 1.10 min, m/z = 534 [M+H]⁺.

### Beispiel 31

### 3-(Hydroxymethyl)-N-{4-methyl-3-[6-(pyridin-3-yl)-1H-imidazo[1,2-b]pyrazol-1-yl]phenyl}-5-(pentafluor-λ⁶-sulfanyl)benzamid

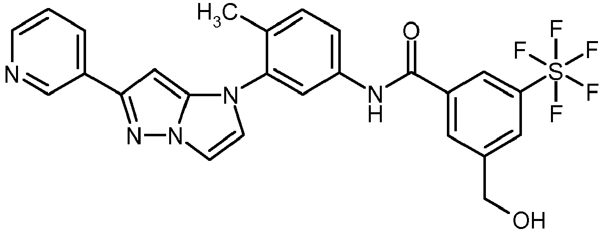

62 mg (0.21 mmol) der Verbindung aus Beispiel 6A und 60 mg (0.21 mmol) der Verbindung aus Beispiel 37A wurden in Analogie zur Vorschrift von Beispiel 16 umgesetzt und aufgearbeitet. Das so erhaltene Produkt wurde mittels präparativer HPLC (Methode 15) nachgereinigt. Die produkthaltigen Fraktionen wurden vereinigt und im Vakuum eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden 40 mg (48% d. Th.) der Titelverbindung erhalten.

¹H-NMR (400 MHz, DMSO-d₆, δ/ppm): 10.70 (s, 1H), 9.05 (d, 1H), 8.48 (dd, 1H), 8.29 (s, 1H), 8.22 (s, 1H), 8.19 (dt, 1H), 8.05 (s, 1H), 7.95 (d, 1H), 7.92 (d, 1H), 7.81 (dd, 1H), 7.56 (d, 1H), 7.48 (d, 1H), 7.42 (dd, 1H), 6.37 (s, 1H), 5.65 (t, 1H), 4.69 (d, 2H), 2.28 (s, 3H).

LC/MS (Methode 3, ESIpos): Rₜ = 0.95 min, m/z = 550 [M+H]⁺.

### Beispiel 32

### 3-(2-Hydroxypropan-2-yl)-N-{4-methyl-3-[6-(pyridin-3-yl)-1H-imidazo[1,2-b]pyrazol-1-yl]phenyl}-5-(pentafluor-λ⁶-sulfanyl)benzamid

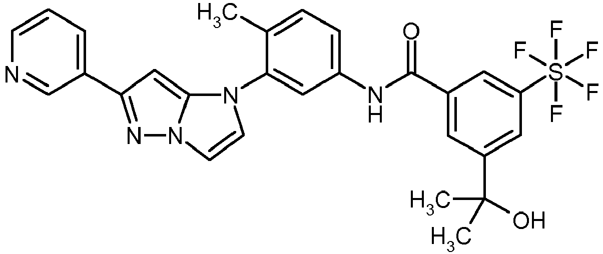

50 mg (0.17 mmol) der Verbindung aus Beispiel 6A und 52.9 mg (0.17 mmol) der Verbindung aus Beispiel 19A wurden in Analogie zur Vorschrift von Beispiel 19 umgesetzt und aufgearbeitet. Es wurden 49 mg (49% d. Th.) der Titelverbindung erhalten.

¹H-NMR (400 MHz, DMSO-d₆, δ/ppm): 10.65 (s, 1H), 9.05 (d, 1H), 8.48 (dd, 1H), 8.29 (s, 1H), 8.27 (s, 1H), 8.21-8.14 (m, 2H), 7.94 (d, 1H), 7.92 (d, 1H), 7.79 (dd, 1H), 7.55 (d, 1H), 7.48 (d, 1H), 7.42 (dd, 1H), 6.37 (s, 1H), 5.54 (s, 1H), 2.28 (s, 3H), 1.51 (s, 6H).

LC/MS (Methode 3, ESIpos): Rₜ = 0.97 min, m/z = 578 [M+H]⁺.

### Beispiel 33

### N-{4-Methyl-3-[6-(pyridin-3-yl)-1H-imidazo[1,2-b]pyrazol-1-yl]phenyl}-3-(pentafluor-λ⁶-sulfanyl)-5-(pyrrolidin-1-ymethyl)benzamid

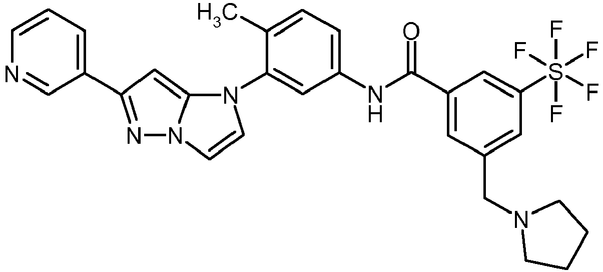

45 mg (0.16 mmol) der Verbindung aus Beispiel 6A und 70 mg (0.16 mmol) der Verbindung aus Beispiel 38A wurden in Analogie zur Vorschrift von Beispiel 16 umgesetzt, mit dem Unterschied, dass der Ansatz hier nach Reaktionsende direkt mittels präparativer HPLC (Methode 15) in seine Komponenten aufgetrennt wurde. Die produkthaltigen Fraktionen wurden vereinigt und im Vakuum eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden 48 mg (51% d. Th.) der Titelverbindung erhalten.

¹H-NMR (400 MHz, DMSO-d₆, δ/ppm): 10.68 (s, 1H), 9.05 (d, 1H), 8.48 (dd, 1H), 8.32 (s, 1H), 8.21 (s, 1H), 8.19 (m, 1H), 8.03 (s, 1H), 7.95 (d, 1H), 7.93 (d, 1H), 7.80 (dd, 1H), 7.56 (d, 1H), 7.48 (d, 1H), 7.41 (dd, 1H), 6.37 (s, 1H), 3.79 (br, 2H), 2.28 (s, 3H), 1.73 (br, 4H) [weitere Signale unter Lösungsmittel-Peaks verborgen].

LC/MS (Methode 4, ESIpos): Rₜ = 0.80 min, m/z = 603 [M+H]⁺.

### Beispiel 34

### 3-{[(3S)-3-Hydroxypyrrolidin-1-yl]methyl}-N-{4-methyl-3-[6-(pyridin-3-yl)-1H-imidazo[1,2-b]-pyrazol-1-yl]phenyl}-5-(pentafluor-λ⁶-sulfanyl)benzamid

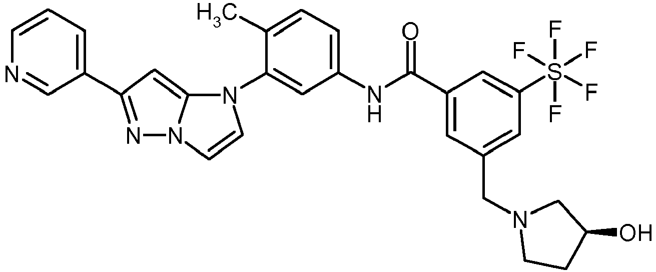

Analog der Vorschrift zu Beispiel 33 wurden aus 44 mg (0.15 mmol) der Verbindung aus Beispiel 6A und 70 mg (0.15 mmol) der Verbindung aus Beispiel 39A 38 mg (39% d. Th.) der Titelverbindung erhalten.

¹H-NMR (400 MHz, DMSO-d₆, δ/ppm): 10.69 (br, 1H), 9.05 (d, 1H), 8.48 (d, 1H), 8.32 (s, 1H), 8.19 (m, 2H), 8.00 (s, 1H), 7.94 (d, 1H), 7.92 (d, 1H), 7.78 (d, 1H), 7.55 (d, 1H), 7.46 (d, 1H), 7.41 (dd, 1H), 6.37 (s, 1H), 4.75 (br, 1H), 4.21 (m, 1H), 3.75 (quart, 2H), 2.70 (quart, 1H), 2.63 (quart, 1H), 2.45 (m, 1H), 2.35 (dd, 1H), 2.27 (s, 3H), 2.01 (m, 1H), 1.57 (m, 1H).

LC/MS (Methode 4, ESIpos): Rₜ = 0.75 min, m/z = 619 [M+H]⁺.

### Beispiel 35

### N-{4-Methyl-3-[6-(pyridin-3-yl)-1H-imidazo[1,2-b]pyrazol-1-yl]phenyl}-3-(pentafluor-λ⁶-sulfanyl)-5-(piperazin-1-ylmethyl)benzamid

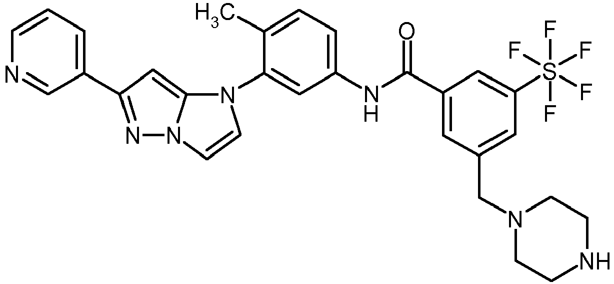

90 mg (0.16 mmol) der Verbindung aus Beispiel 55A und 34 mg (0.18 mmol) tert.-Butyl-piperazin-1-carboxylat wurden in 3.2 ml Dichlormethan gelöst, mit 52 mg (0.25 mmol) Natriumtriacetoxyborhydrid versetzt und 3 h bei RT gerührt. Danach wurde mit 3 ml Wasser versetzt und mit 6 ml Ethylacetat extrahiert. Die organische Phase wurde mit gesättigter Natriumchlorid-Lösung gewaschen, über Natriumsulfat getrocknet, filtriert und im Vakuum eingeengt. Der Rückstand wurde anschließend in 2 ml Dichlormethan und 1 ml Trifluoressigsäure 2 h bei RT gerührt. Der Ansatz wurde dann am Rotationsverdampfer eingeengt und der Rückstand mittels präparativer HPLC (Methode 24) in seine Komponenten aufgetrennt. Die produkthaltigen Fraktionen wurden vereinigt und im Vakuum eingeengt und der Rückstand im Hochvakuum getrocknet. Man erhielt 11 mg (11% d. Th.) der Titelverbindung.

¹H-NMR (400 MHz, DMSO-d₆, δ/ppm): 10.67 (s, 1H), 9.05 (d, 1H), 8.48 (dd, 1H), 8.32 (s, 1H), 8.21-8.17 (m, 2H), 8.03 (s, 1H), 7.95 (d, 1H), 7.92 (d, 1H), 7.80 (dd, 1H), 7.55 (d, 1H), 7.48 (d, 1H), 7.41 (dd, 1H), 6.37 (s, 1H), 3.63 (s, 2H), 2.71 (br, 4H), 2.34 (br, 4H), 2.28 (s, 3H).

LC/MS (Methode 3, ESIneg): Rₜ = 0.77 min, m/z = 616 [M-H]⁻.

### Beispiel 36

### 3-[(4-Methylpiperazin-1-yl)methyl]-N-{4-methyl-3-[6-(pyridin-3-yl)-1H-imidazo[1,2-b]pyrazol-1-yl]phenyl}-5-(pentafluor-λ⁶-sulfanyl)benzamid

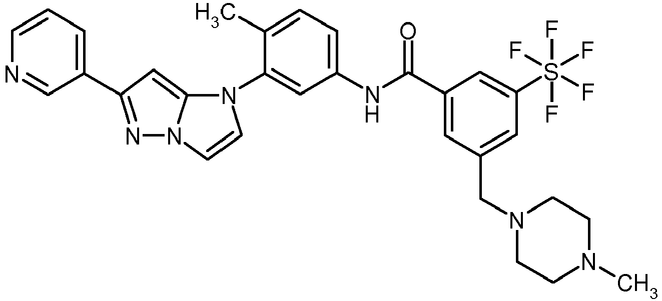

Analog der Vorschrift zu Beispiel 33 wurden aus 60 mg (0.18 mmol) der Verbindung aus Beispiel 6A und 85 mg (0.18 mmol) der Verbindung aus Beispiel 40A 73 mg (65% d. Th.) der Titelverbindung erhalten.

¹H-NMR (400 MHz, DMSO-d₆, δ/ppm): 10.68 (s, 1H), 9.06 (s, 1H), 8.48 (d, 1H), 8.32 (s, 1H), 8.19 (m, 2H), 8.03 (s, 1H), 7.95 (d, 1H), 7.93 (d, 1H), 7.80 (d, 1H), 7.56 (d, 1H), 7.48 (d, 1H), 7.41 (dd, 1H), 6.38 (s, 1H), 3.66 (s, 2H), 2.50-2.20 (br, 8H), 2.28 (s, 3H), 2.15 (s, 3H).

LC/MS (Methode 3, ESIpos): Rₜ = 0.74 min, m/z = 632 [M+H]⁺.

### Beispiel 37

### 3-(3-Hydroxyazetidin-3-yl)-N-{4-methyl-3-[6-(pyridin-3-yl)-1H-imidazo[1,2-b]pyrazol-1-yl]-phenyl}-5-(pentafluor-λ⁶-sulfanyl)benzamid-Bis(trifluoracetat)

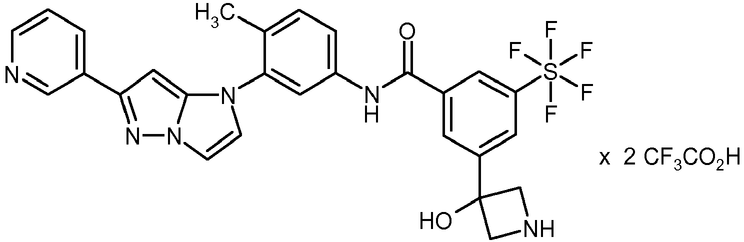

65 mg (0.09 mmol) der Verbindung aus Beispiel 58A wurden in 0.5 ml 1,4-Dioxan gelöst, mit 2.0 ml einer 4 M Lösung von Chlorwasserstoff in Dioxan versetzt und 30 min bei RT gerührt. Anschließend wurde im Vakuum eingeengt, der Rückstand in etwas Methanol gelöst, in halbkonzentrierte wässrige Natriumhydrogencarbonat-Lösung eingerührt und 15 min bei RT nachgerührt. Der entstandene Feststoff wurde abfiltriert, mit Wasser gewaschen und getrocknet. Das so erhaltene Produkt wurde mittels präparativer HPLC (Methode 30) nachgereinigt. Es wurden so 33 mg (43% d. Th.) der Titelverbindung erhalten.

¹H-NMR (400 MHz, DMSO-d₆, δ/ppm): 10.77 (s, 1H), 9.29 (br. s, 1H), 9.12 (s, 1H), 8.82 (br, 1H), 8.58 (d, 1H), 8.45 (m, 2H), 8.40 (d, 1H), 8.25 (t, 1H), 7.97 (m, 2H), 7.79 (dd, 1H), 7.63-7.57 (m, 2H), 7.51 (d, 1H), 7.15 (s, 1H), 6.44 (s, 1H), 4.48 (m, 2H), 4.13 (m, 2H), 2.29 (s, 3H).

LC/MS (Methode 3, ESIpos): Rₜ = 0.74 min, m/z = 591 [M+H]⁺.

### Beispiel 38

### 3-Cyano-N-{4-methyl-3-[6-(pyridin-3-yl)-1H-imidazo[1,2-b]pyrazol-1-yl]phenyl}-5-(trifluormethoxy)benzamid

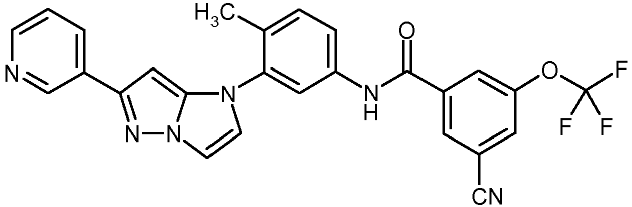

Analog der Vorschrift zu Beispiel 33 wurden 50 mg (0.17 mmol) der Verbindung aus Beispiel 6A und 40 mg (0.18 mmol) der Verbindung aus Beispiel 41A miteinander umgesetzt. Nach der Reinigung des Rohprodukts durch präparative HPLC wurden die produkthaltigen Fraktionen bis auf ein kleines Restvolumen eingeengt und mit etwas gesättigter wässriger Natriumhydrogencarbonat-Lösung alkalisch gestellt. Der entstandene Niederschlag wurde abfiltriert, mit Wasser gewaschen und getrocknet. Man erhielt 56 mg (65% d. Th.) der Titelverbindung.

¹H-NMR (400 MHz, DMSO-d₆, δ/ppm): 10.69 (s, 1H), 9.05 (d, 1H), 8.49 (m, 2H), 8.31 (s, 1H), 8.22 (s, 1H), 8.19 (dt, 1H), 7.95 (d, 1H), 7.93 (d, 1H), 7.79 (dd, 1H), 7.56 (d, 1H), 7.49 (d, 1H), 7.42 (dd, 1H), 6.37 (s, 1H), 2.28 (s, 3H).

LC/MS (Methode 3, ESIpos): Rₜ = 0.98 min, m/z = 503 [M+H]⁺.

### Beispiel 39

### N-{4-Methyl-3-[6-(pyridin-3-yl)-1H-imidazo[1,2-b]pyrazol-1-yl]phenyl}-3-(trifluormethyl)benzamid

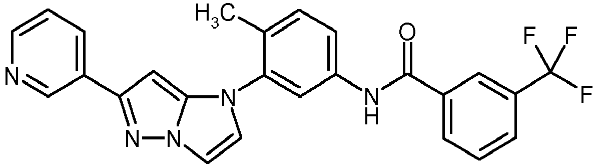

19 mg (0.1 mmol) 3-(Trifluormethyl)benzoesäure wurden in einer Vertiefung einer 96er-Multititerplatte vorgelegt. Dazu wurden 27.1 mg (0.1 mmol) der Verbindung aus Beispiel 6A und 41.7 mg (0.13 mmol) *N*-[(1*H*-Benzotriazol-1-yloxy)(dimethylamino)methylen]-*N*-methylmethanaminium-Tetrafluoroborat (TBTU), jeweils gelöst in 0.3 ml DMF, sowie 26 mg (0.2 mmol) *N*,*N*-Diisopropylethylamin gegeben. Die Multititerplatte wurde abgedeckt und 18 h bei RT geschüttelt. Danach wurde filtriert und das Filtrat direkt mittels präparativer LC/MS nach einer der folgenden Methoden aufgereinigt:

### Methode A:

Instrument MS: Waters, Instrument HPLC: Waters; Säule: Phenomenex Luna 5µ C18(2) 100A, AXIA Tech. 50 mm x 21.2 mm; Eluent A: Wasser + 0.05% Ameisensäure, Eluent B: Methanol + 0.05% Ameisensäure, mit Gradient; Fluss: 40 ml/min; UV-Detektion (DAD): 210-400 nm.

### Methode B:

Instrument MS: Waters, Instrument HPLC: Waters; Säule: Phenomenex Luna 5µ C18(2) 100A, AXIA Tech. 50 mm x 21.2 mm; Eluent A: Wasser + 0.05% Triethylamin, Eluent B: Methanol + 0.05% Triethylamin, mit Gradient; Fluss: 40 ml/min; UV-Detektion (DAD): 210-400 nm.

Die produkthaltigen Fraktionen wurden mittels Zentrifugaltrockner im Vakuum eingeengt. Der Rückstand der einzelnen Fraktionen wurde in jeweils 0.6 ml DMSO gelöst und die Lösungen dann vereinigt. Anschließend wurde im Zentrifugaltrockner das Lösungsmittel vollständig abgedampft. Es wurden so 28.5 mg (62% d. Th.) der Titelverbindung erhalten.

LC/MS (Methode 31, ESIpos): Rₜ = 1.25 min, m/z = 462 [M+H]⁺, Reinheit 100%.

### Beispiel 40

### 3-(2-Methyl-1H-imidazol-1-yl)-N-{4-methyl-3-[6-(pyridin-3-yl)-1H-imidazo[1,2-b]pyrazol-1-yl]-phenyl} -5-(trifluormethyl)benzamid

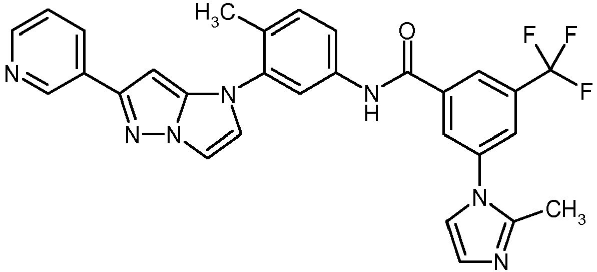

35 mg (0.12 mmol) der Verbindung aus Beispiel 6A und 33 mg (0.12 mmol) 3-(2-Methyl-1*H-*imidazol-1-yl)-5-(trifluormethyl)benzoesäure [Lit.: WO 2004/005281-A1, Beispiel 91b] wurden in Analogie zur Vorschrift von Beispiel 16 umgesetzt und aufgearbeitet. Es wurden 53 mg (81% d. Th.) der Titelverbindung erhalten.

¹H-NMR (400 MHz, DMSO-d₆, δ/ppm): 10.67 (s, 1H), 9.05 (d, 1H), 8.48 (d, 1H), 8.36 (s, 1H), 8.32 (s, 1H), 8.18 (m, 1H), 8.16 (s, 1H), 7.96 (d, 1H), 7.92 (d, 1H), 7.81 (d, 1H), 7.55 (d, 1H), 7.49 (m, 2H), 7.41 (dd, 1H), 6.99 (s, 1H), 6.37 (s, 1H), 2.35 (s, 3H), 2.28 (s, 3H).

LC/MS (Methode 3, ESIpos): Rₜ = 0.69 min, m/z = 542 [M+H]⁺.

### Beispiel 41

### 3-(4-Methylpiperazin-1-yl)-N-{4-methyl-3-[6-(pyridin-3-yl)-1H-imidazo[1,2-b]pyrazol-1-yl]-phenyl} -5-(trifluormethyl)benzamid

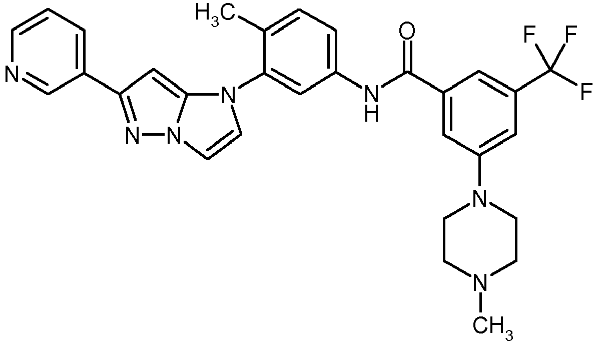

40 mg (0.14 mmol) der Verbindung aus Beispiel 6A und 44 mg (0.14 mmol) 3-(4-Methylpiperazin-1-yl)-5-(trifluormethyl)benzoesäure [Lit.: WO 2004/029038-A1, Beispiel 14.2] wurden in Analogie zur Vorschrift von Beispiel 33 umgesetzt und aufgearbeitet. Es wurden 43 mg (53% d. Th.) der Titelverbindung erhalten.

¹H-NMR (400 MHz, DMSO-d₆, δ/ppm): 10.50 (s, 1H), 9.05 (d, 1H), 8.48 (dd, 1H), 8.19 (dt, 1H), 7.94 (d, 1H), 7.92 (d, 1H), 7.80 (dd, 1H), 7.70 (s, 1H), 7.61 (s, 1H), 7.55 (d, 1H), 7.46 (d, 1H), 7.41 (dd, 1H), 7.38 (s, 1H), 6.37 (s, 1H), 2.47 (m, 4H), 2.27 (s, 3H), 2.23 (s, 3H) [weitere Signale unter Lösungsmittel-Peaks verborgen].

LC/MS (Methode 4, ESIpos): Rₜ = 0.70 min, m/z = 560 [M+H]⁺.

### Beispiel 42

3-{[3-(Dimethylamino)propyl](methyl)amino}-*N*-{4-methyl-3-[6-(pyridin-3-yl)-1*H*-imidazo[1,2-b]-pyrazol-1-yl]phenyl}-5-(trifluormethyl)benzamid

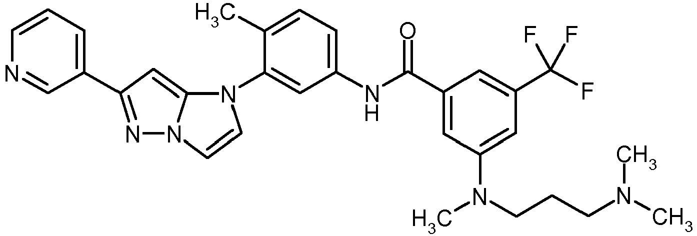

40 mg (0.14 mmol) der Verbindung aus Beispiel 6A und 57 mg (0.15 mmol) der Verbindung aus Beispiel 35A wurden in Analogie zur Vorschrift von Beispiel 33 umgesetzt und aufgearbeitet, mit dem Unterschied, dass hier 3 Äquivalente N,N-Diisopropylethylamin bei der Reaktion verwendet wurden. Es wurden 22 mg (90% Reinheit, 25% d. Th.) der Titelverbindung erhalten.

¹H-NMR (400 MHz, DMSO-d₆, δ/ppm): 10.47 (s, 1H), 9.05 (d, 1H), 8.48 (dd, 1H), 8.19 (dt, 1H), 7.95 (d, 1H), 7.92 (d, 1H), 7.80 (dd, 1H), 7.55 (d, 1H), 7.47-7.40 (m, 4H), 7.13 (s, 1H), 6.37 (s, 1H), 3.47 (t, 2H), 3.00 (s, 3H), 2.27 (s, 3H), 2.21 (t, 2H), 2.11 (s, 6H), 1.65 (quint, 2H).

LC/MS (Methode 3, ESIpos): Rₜ = 0.79 min, m/z = 576 [M+H]⁺.

### Beispiel 43

### N-{4-Methyl-3-[6-(pyridin-3-yl)-1H-imidazo[1,2-b]pyrazol-1-yl]phenyl}-3,5-bis(trifluormethyl)-benzamid

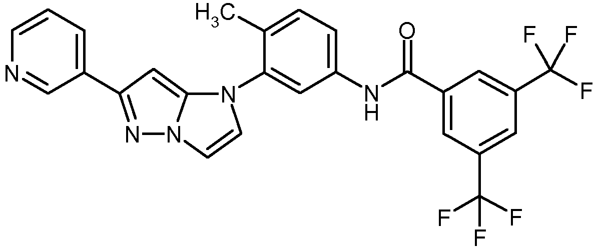

Nach dem unter Beispiel 39 beschriebenen Verfahren wurden aus der Verbindung aus Beispiel 6A und 3,5-Bis(trifluormethyl)benzoesäure 17.8 mg (34% d. Th.) der Titelverbindung erhalten.

LC/MS (Methode 31, ESIpos): Rₜ = 1.36 min, m/z = 530 [M+H]⁺, Reinheit 100%.

### Beispiel 44

### 3-Cyano-N-{4-methyl-3-[6-(pyridin-3-yl)-1H-imidazo[1,2-b]pyrazol-1-yl]phenyl}-5-(trifluormethyl)benzamid

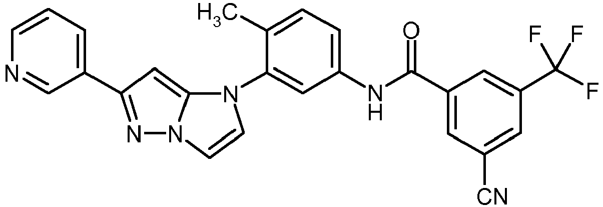

Analog zu dem unter Beispiel 26 beschriebenen Verfahren wurden aus 80 mg (0.276 mmol) der Verbindung aus Beispiel 6A und 60 mg (0.276 mmol) der Verbindung aus Beispiel 42A 72 mg (54% d. Th.) der Titelverbindung erhalten. Die Reaktionszeit betrug in diesem Falle 18 h, und auf ein abschließendes Verrühren des Produkts mit Diisopropylether konnte hier verzichtet werden.

¹H-NMR (400 MHz, CDCl₃, δ/ppm): 9.38 (br. s, 1H), 8.98 (s, 1H), 8.50-8.46 (m, 3H), 8.11 (d, 1H), 8.05 (s, 1H), 7.84 (s, 1H), 7.70 (dd, 1H), 7.48 (d, 1H), 7.40 (d, 1H), 7.32 (dd, 1H), 6.96 (d, 1H), 5.97 (s, 1H), 2.31 (s, 3H).

LC/MS (Methode 3, ESIpos): Rₜ = 0.99 min, m/z = 487 [M+H]⁺.

### Beispiel 45

### 4-Fluor-N-{4-methyl-3-[6-(pyridin-3-yl)-1H-imidazo[1,2-b]pyrazol-1-yl]phenyl}-3-(trifluormethyl)-benzamid

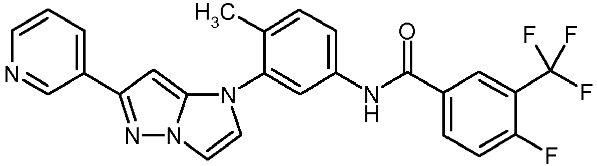

Nach dem unter Beispiel 39 beschriebenen Verfahren wurden aus der Verbindung aus Beispiel 6A und 4-Fluor-3-(trifluormethyl)benzoesäure 32.2 mg (67% d. Th.) der Titelverbindung erhalten.

LC/MS (Methode 31, ESIpos): Rₜ = 1.27 min, m/z = 480 [M+H]⁺, Reinheit 100%.

### Beispiel 46

### 2-Fluor-N-{4-methyl-3-[6-(pyridin-3-yl)-1H-imidazo[1,2-b]pyrazol-1-yl]phenyl}-3-(trifluormethyl)-benzamid

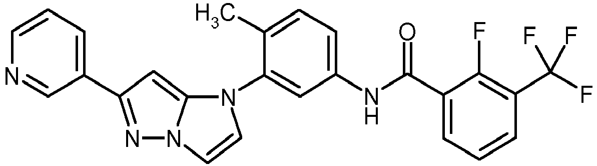

Nach dem unter Beispiel 39 beschriebenen Verfahren wurden aus der Verbindung aus Beispiel 6A und 2-Fluor-3-(trifluormethyl)benzoesäure 20.6 mg (43% d. Th.) der Titelverbindung erhalten.

LC/MS (Methode 31, ESIpos): Rₜ = 1.24 min, m/z = 480 [M+H]⁺, Reinheit 100%.

### Beispiel 47

### 3-tert.-Butyl-5-(2-methyl-1H-imidazol-1-yl)-N-{4-methyl-3-[6-(pyridin-3-yl)-1H-imidazo[1,2-b]-pyrazol-1-yl]phenyl}benzamid

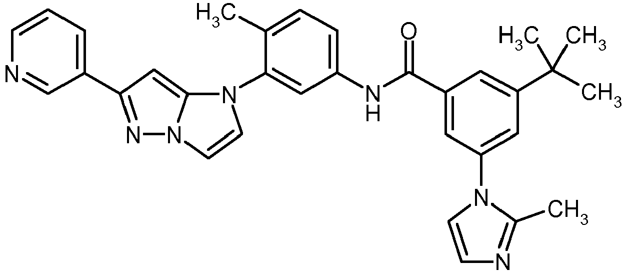

50 mg (0.17 mmol) der Verbindung aus Beispiel 6A und 64 mg (0.17 mmol) der Verbindung aus Beispiel 27A wurden in Analogie zur Vorschrift von Beispiel 16 umgesetzt und aufgearbeitet. Es wurden 65 mg (97% Reinheit, 69% d. Th.) der Titelverbindung erhalten.

¹H-NMR (400 MHz, DMSO-d₆, δ/ppm): 10.48 (s, 1H), 9.05 (d, 1H), 8.48 (d, 1H), 8.19 (d, 1H), 8.00 (s, 1H), 7.96 (d, 1H), 7.92 (d, 1H), 7.84 (s, 1H), 7.80 (d, 1H), 7.69 (s, 1H), 7.55 (d, 1H), 7.47 (d, 1H), 7.41 (m, 1H), 7.40 (s, 1H), 6.95 (s, 1H), 6.37 (s, 1H), 2.32 (s, 3H), 2.27 (s, 3H), 1.37 (s, 9H).

LC/MS (Methode 3, ESIpos): Rₜ = 0.84 min, m/z = 530 [M+H]⁺.

### Beispiel 48

### 3-tert.-Butyl-5-(4-methylpiperazin-1-yl)-N-{4-methyl-3-[6-(pyridin-3-yl)-1H-imidazo[1,2-b]pyrazol-1-yl]phenyl}benzamid

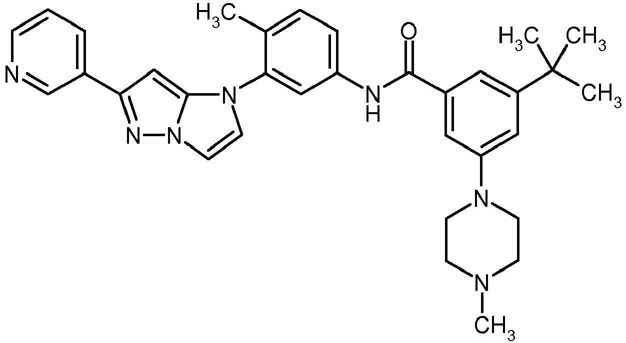

35 mg (0.12 mmol) der Verbindung aus Beispiel 6A und 47 mg (0.12 mmol) der Verbindung aus Beispiel 21A wurden in Analogie zur Vorschrift von Beispiel 16 umgesetzt und aufgearbeitet. Es wurden 25 mg (95% Reinheit, 38% d. Th.) der Titelverbindung erhalten.

¹H-NMR (400 MHz, DMSO-d₆, δ/ppm): 10.26 (s, 1H), 9.05 (d, 1H), 8.48 (dd, 1H), 8.19 (dt, 1H), 7.94 (d, 1H), 7.91 (d, 1H), 7.79 (dd, 1H), 7.54 (d, 1H), 7.44 (d, 1H), 7.41 (m, 1H), 7.36 (s, 1H), 7.27 (s, 1H), 7.13 (s, 1H), 6.37 (s, 1H), 3.21 (t, 4H), 2.47 (t, 4H), 2.26 (s, 3H), 2.23 (s, 3H), 1.31 (s, 9H).

LC/MS (Methode 4, ESIneg): Rₜ = 0.72 min, m/z = 546 [M-H]⁻.

### Beispiel 49

### 3-tert.-Butyl-5-(2-hydroxypropan-2-yl)-N-{4-methyl-3-[6-(pyridin-3-yl)-1H-imidazo[1,2-b]pyrazol-1-yl]phenyl}benzamid

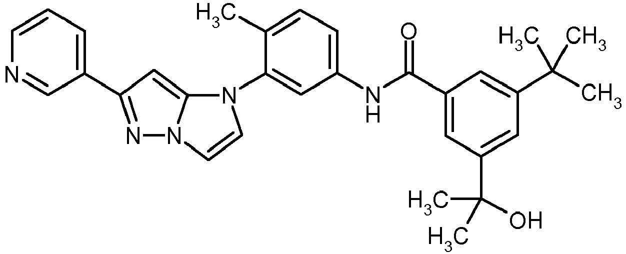

40 mg (0.14 mmol) der Verbindung aus Beispiel 6A und 33 mg (0.14 mmol) der Verbindung aus Beispiel 28A wurden in Analogie zur Vorschrift von Beispiel 33 umgesetzt und aufgearbeitet. Es wurden 31 mg (44% d. Th.) der Titelverbindung erhalten.

¹H-NMR (400 MHz, DMSO-d₆, δ/ppm): 10.33 (s, 1H), 9.06 (d, 1H), 8.48 (dd, 1H), 8.19 (dt, 1H), 7.95 (d, 1H), 7.92 (d, 1H), 7.83-7.78 (m, 2H), 7.76 (s, 1H), 7.73 (s, 1H), 7.55 (d, 1H), 7.45 (d, 1H), 7.41 (dd, 1H), 6.37 (s, 1H), 5.14 (s, 1H), 2.27 (s, 3H), 1.47 (s, 6H), 1.34 (s, 9H).

LC/MS (Methode 3, ESIpos): Rₜ = 0.95 min, m/z = 508 [M+H]⁺.

### Beispiel 50

### 3-tert.-Butyl-5-cyano-N-{4-methyl-3-[6-(pyridin-3-yl)-1H-imidazo[1,2-b]pyrazol-1-yl]phenyl}-benzamid

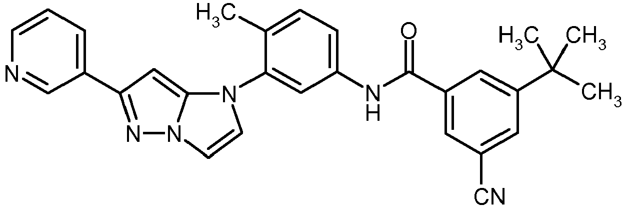

Zu einer Lösung von 91 mg (0.31 mmol) der Verbindung aus Beispiel 6A und 173 mg (Reinheit 37%, 0.31 mmol) 3-*tert*.-Butyl-5-cyanobenzoesäure [Lit.: WO 2008/021388-A1, Intermediat E, Seite 164] in 2.0 ml DMSO wurden 144 mg (0.38 mmol) HATU und 81 mg (0.63 mmol) *N*,*N-*Diisopropylethylamin gegeben. Der Ansatz wurde 16 h bei 25°C gerührt. Das Reaktionsgemisch wurde danach mit Ethylacetat verdünnt und die Phasen getrennt. Die organische Phase wurde zweimal mit je 50 ml gesättigte Natriumhydrogencarbonat-Lösung gewaschen und anschließend über Natriumsulfat getrocknet. Nach Filtration wurde im Vakuum eingeengt und der so erhaltene Rückstand durch zweimalige Chromatographie an einer Biotage-Anlage gereinigt (1. Lauf: 25 g Snap-Säule, Laufmittelgradient Ethylacetat/Hexan, beginnend mit 20% Ethylacetat, dann schnell auf 100% Ethylacetat ansteigend, danach Ethylacetat/Methanol, von 0% Methanol bis 50% Methanol stetig ansteigend; 2. Lauf: 10 g Snap-Säule, Laufmittelgradient Ethylacetat/Hexan, beginnend mit 20% Ethylacetat, dann schnell auf 100% Ethylacetat ansteigend, danach Ethylacetat/ Methanol, von 0% Methanol bis 50% Methanol stetig ansteigend). Auf diese Weise wurden 17.8 mg (11% d. Th.) der Titelverbindung erhalten.

¹H-NMR (300 MHz, CDCl₃, δ/ppm): 9.03 (s, 1H), 8.51 (d, 1H), 8.09-8.18 (m, 3H), 7.93 (s, 1H), 7.88 (s, 1H), 7.84 (s, 1H), 7.56 (d, 1H), 7.50 (d, 1H), 7.40 (d, 1H), 7.32 (dd, 1H), 6.96 (d, 1H), 6.02 (s, 1H), 2.32 (s, 3H), 1.37 (s, 9H).

LC/MS (Methode 6, ESIpos): Rₜ = 1.23 min, m/z = 475 [M+H]⁺.

### Beispiel 51

### 3-tert.-Butyl-N-{4-methyl-3-[6-(pyridin-3-yl)-1H-imidazo[1,2-b]pyrazol-1-yl]phenyl}-5-(pyrrolidin-1-ylmethyl)benzamid

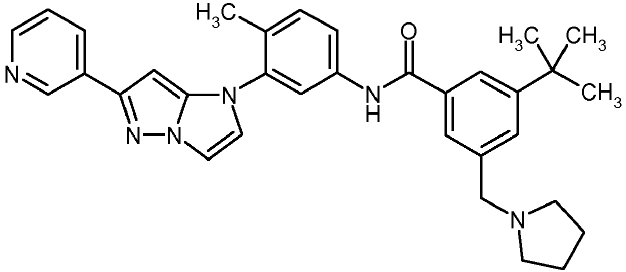

35 mg (0.12 mmol) der Verbindung aus Beispiel 6A und 47 mg (0.12 mmol) der Verbindung aus Beispiel 22A wurden in Analogie zur Vorschrift von Beispiel 16 umgesetzt und aufgearbeitet. Es wurden 55 mg (95% Reinheit, 81% d. Th.) der Titelverbindung erhalten.

¹H-NMR (400 MHz, DMSO-d₆, δ/ppm): 10.36 (s, 1H), 9.06 (d, 1H), 8.48 (dd, 1H), 8.19 (dt, 1H), 7.96 (d, 1H), 7.91 (d, 1H), 7.83-7.78 (m, 2H), 7.71 (s, 1H), 7.55 (d, 1H), 7.53 (s, 1H), 7.45 (d, 1H), 7.41 (dd, 1H), 6.37 (s, 1H), 3.63 (s, 2H), 2.45 (br, 4H), 2.27 (s, 3H), 1.70 (br, 4H), 1.33 (s, 9H).

LC/MS (Methode 3, ESIneg): Rₜ = 0.76 min, m/z = 531 [M-H]⁻.

### Beispiel 52

### 3,5-Dimethyl-N-{4-methyl-3-[6-(pyridin-3-yl)-1H-imidazo[1,2-b]pyrazol-1-yl]phenyl}benzamid

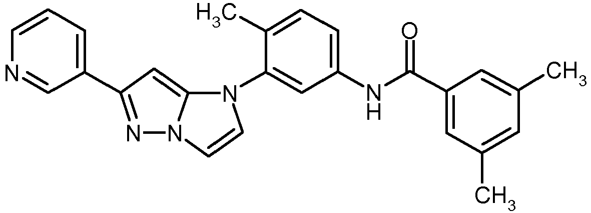

Nach dem unter Beispiel 39 beschriebenen Verfahren wurden aus der Verbindung aus Beispiel 6A und 3,5-Dimethylbenzoesäure 9.6 mg (22% d. Th.) der Titelverbindung erhalten.

LC/MS (Methode 31, ESIpos): Rₜ = 1.23 min, m/z = 422 [M+H]⁺, Reinheit 95%.

### Beispiel 53

### 2-Hydroxy-N-{4-methyl-3-[6-(pyridin-3-yl)-1H-imidazo[1,2-b]pyrazol-1-yl]phenyl}-5-(propan-2-yl)benzamid

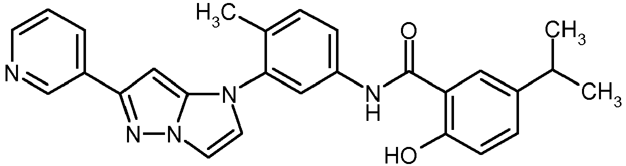

Nach dem unter Beispiel 39 beschriebenen Verfahren wurden aus der Verbindung aus Beispiel 6A und 2-Hydroxy-5-isopropylbenzoesäure 9.2 mg (18% d. Th.) der Titelverbindung erhalten.

LC/MS (Methode 31, ESIpos): Rₜ = 1.32 min, m/z = 452 [M+H]⁺, Reinheit 89%.

### Beispiel 54

### 3'-Cyano-N-{4-methyl-3-[6-(pyridin-3-yl)-1H-imidazo[1,2-b]pyrazol-1-yl]phenyl}biphenyl-3-carboxamid

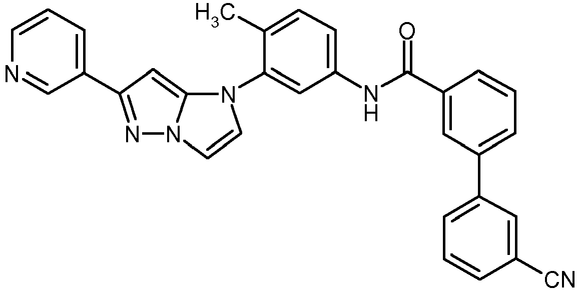

Nach dem unter Beispiel 39 beschriebenen Verfahren wurden aus der Verbindung aus Beispiel 6A und 3'-Cyanobiphenyl-3-carbonsäure 9.1 mg (18% d. Th.) der Titelverbindung erhalten.

LC/MS (Methode 31, ESIpos): Rₜ = 1.28 min, m/z = 495 [M+H]⁺, Reinheit 100%.

### Beispiel 55

### N-{4-Methyl-3-[6-(pyridin-3-yl)-1H-imidazo[1,2-b]pyrazol-1-yl]phenyl}-3-(1H-pyrazol-1-yl)benzamid

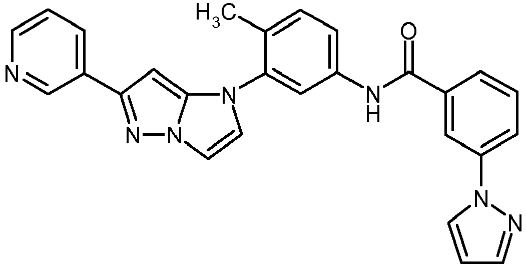

Nach dem unter Beispiel 39 beschriebenen Verfahren wurden aus der Verbindung aus Beispiel 6A und 3-(1*H*-Pyrazol-1-yl)benzoesäure 31.7 mg (69% d. Th.) der Titelverbindung erhalten.

LC/MS (Methode 31, ESIpos): Rₜ = 1.14 min, m/z = 460 [M+H]⁺, Reinheit 100%.

### Beispiel 56

### N-{4-Methyl-3-[6-(pyridin-3-yl)-1H-imidazo[1,2-b]pyrazol-1-yl]phenyl}-3-(pyrrolidin-1-yl)benzamid

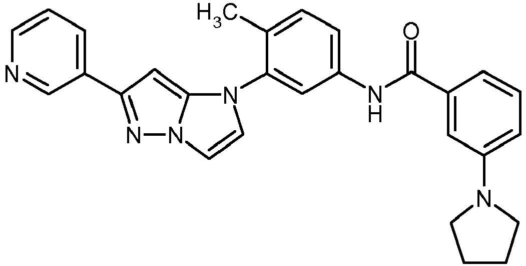

Nach dem unter Beispiel 39 beschriebenen Verfahren wurden aus der Verbindung aus Beispiel 6A und 3-(Pyrrolidin-1-yl)benzoesäure 26.3 mg (57% d. Th.) der Titelverbindung erhalten.

LC/MS (Methode 31, ESIpos): Rₜ = 1.26 min, m/z = 463 [M+H]⁺, Reinheit 100%.

### Beispiel 57

### 2-Chlor-N-{4-methyl-3-[6-(pyridin-3-yl)-1H-imidazo[1,2-b]pyrazol-1-yl]phenyl}-5-(pyrrolidin-1-yl)benzamid

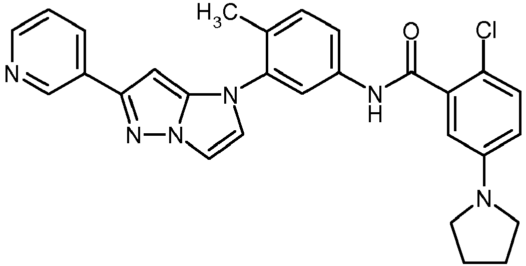

Nach dem unter Beispiel 39 beschriebenen Verfahren wurden aus der Verbindung aus Beispiel 6A und 2-Chlor-5-(pyrrolidin-1-yl)benzoesäure 15.2 mg (31% d. Th.) der Titelverbindung erhalten.

LC/MS (Methode 31, ESIpos): Rₜ = 1.28 min, m/z = 497 [M+H]⁺, Reinheit 100%.

### Beispiel 58

### 4-Chlor-N-{4-methyl-3-[6-(pyridin-3-yl)-1H-imidazo[1,2-b]pyrazol-1-yl]phenyl}-3-(piperidin-1-yl)-benzamid

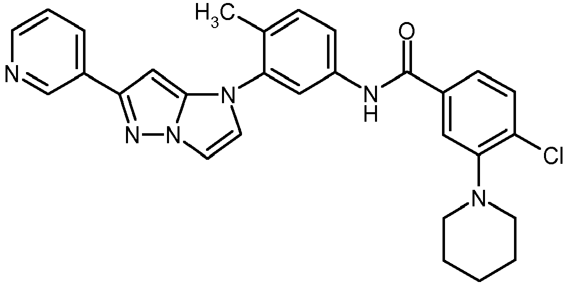

Nach dem unter Beispiel 39 beschriebenen Verfahren wurden aus der Verbindung aus Beispiel 6A und 4-Chlor-3-(piperidin-1-yl)benzoesäure 23.2 mg (45% d. Th.) der Titelverbindung erhalten.

LC/MS (Methode 31, ESIpos): Rₜ = 1.39 min, m/z = 511 [M+H]⁺, Reinheit 100%.

### Beispiel 59

### 3-(Dimethylamino)-N-{4-methyl-3-[6-(pyridin-3-yl)-1H-imidazo[1,2-b]pyrazol-1-yl]phenyl}benzamid

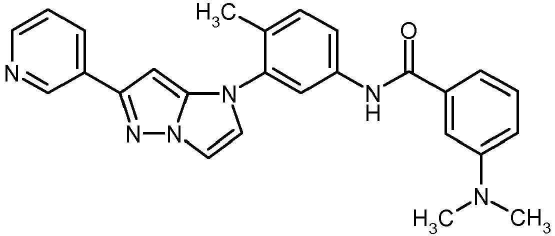

Nach dem unter Beispiel 39 beschriebenen Verfahren wurden aus der Verbindung aus Beispiel 6A und 3-(Dimethylamino)benzoesäure 26.1 mg (60% d. Th.) der Titelverbindung erhalten.

LC/MS (Methode 31, ESIpos): Rₜ = 1.14 min, m/z = 437 [M+H]+, Reinheit 100%.

### Beispiel 60

### N-{4-Methyl-3-[6-(pyridin-3-yl)-1H-imidazo[1,2-b]pyrazol-1-yl]phenyl}-3-(propan-2-yloxy)benzamid

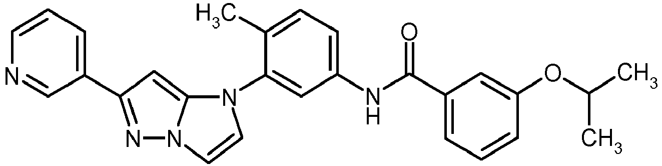

Nach dem unter Beispiel 39 beschriebenen Verfahren wurden aus der Verbindung aus Beispiel 6A und 3-Isopropoxybenzoesäure 41.2 mg (69% d. Th.) der Titelverbindung erhalten.

LC/MS (Methode 31, ESIpos): Rₜ = 1.23 min, m/z = 452 [M+H]⁺, Reinheit 76%.

### Beispiel 61

### N-{4-Methyl-3-[6-(pyridin-3-yl)-1H-imidazo[1,2-b]pyrazol-1-yl]phenyl}-3-propoxybenzamid

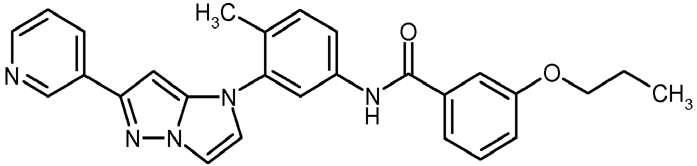

Nach dem unter Beispiel 39 beschriebenen Verfahren wurden aus der Verbindung aus Beispiel 6A und 3-Propoxybenzoesäure 40.3 mg (67% d. Th.) der Titelverbindung erhalten.

LC/MS (Methode 31, ESIpos): Rₜ = 1.26 min, m/z = 452 [M+H]⁺, Reinheit 75%.

### Beispiel 62

### 3-(2-Methylpropoxy)-N-{4-methyl-3-[6-(pyridin-3-yl)-1H-imidazo[1,2-b]pyrazol-1-yl]phenyl}benzamid

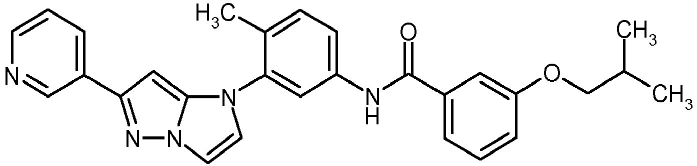

Nach dem unter Beispiel 39 beschriebenen Verfahren wurden aus der Verbindung aus Beispiel 6A und 3-Isobutoxybenzoesäure 26.9 mg (58% d. Th.) der Titelverbindung erhalten.

LC/MS (Methode 31, ESIpos): Rₜ = 1.32 min, m/z = 466 [M+H]⁺, Reinheit 100%.

### Beispiel 63

### 3,5-Dimethoxy-N-{4-methyl-3-[6-(pyridin-3-yl)-1H-imidazo[1,2-b]pyrazol-1-yl]phenyl}benzamid

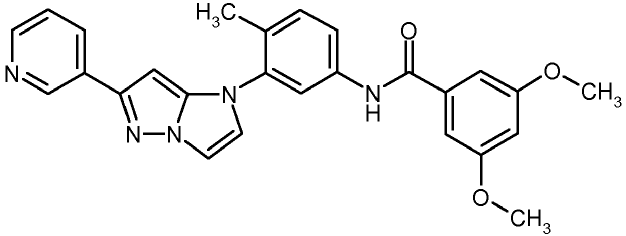

Nach dem unter Beispiel 39 beschriebenen Verfahren wurden aus der Verbindung aus Beispiel 6A und 3,5-Dimethoxybenzoesäure 18.3 mg (40% d. Th.) der Titelverbindung erhalten.

LC/MS (Methode 31, ESIpos): Rₜ = 1.17 min, m/z = 454 [M+H]⁺, Reinheit 100%.

### Beispiel 64

### 2-tert.-Butyl-N-{4-methyl-3-[6-(pyridin-3-yl)-1H-imidazo[1,2-b]pyrazol-1-yl]phenyl}isonicotinamid

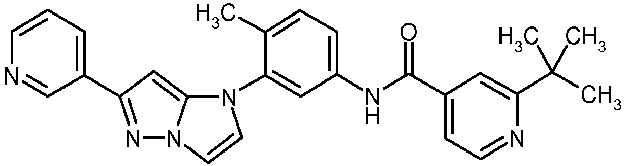

60 mg (0.21 mmol) der Verbindung aus Beispiel 6A und 37 mg (0.21 mmol) 2-tert.-Butylisonicotin-säure wurden in Analogie zur Vorschrift von Beispiel 33 umgesetzt und aufgearbeitet, mit dem Unterschied, dass die Reaktionsdauer in diesem Fall 16 h betrug. Es wurden 13 mg (96% Reinheit, 13% d. Th.) der Titelverbindung erhalten.

¹H-NMR (400 MHz, DMSO-d₆, δ/ppm): 10.63 (br, 1H), 9.05 (d, 1H), 8.69 (d, 1H), 8.48 (d, 1H), 8.19 (d, 1H), 7.95 (s, 1H), 7.91 (d, 1H), 7.86 (s, 1H), 7.76 (d, 1H), 7.68 (d, 1H), 7.54 (d, 1H), 7.44 (d, 1H), 7.41 (m, 1H), 6.36 (s, 1H), 2.27 (s, 3H), 1.36 (s, 9H).

LC/MS (Methode 4, ESIneg): Rₜ = 0.92 min, m/z = 449 [M-H]⁻.

### Beispiel 65

### 2-tert.-Butyl-6-chlor-N-{4-methyl-3-[6-(pyridin-3-yl)-1H-imidazo[1,2-b]pyrazol-1-yl]phenyl}isonicotinamid

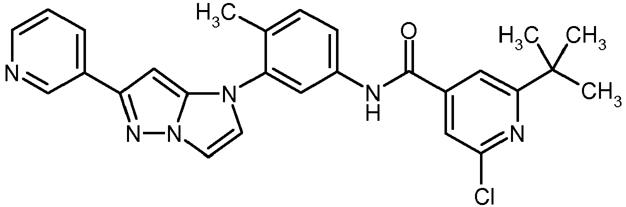

50 mg (0.17 mmol) der Verbindung aus Beispiel 6A und 37 mg (0.17 mmol) der Verbindung aus Beispiel 43A wurden in Analogie zur Vorschrift von Beispiel 15 umgesetzt, mit dem Unterschied, dass hier zur Aufarbeitung nur 6 ml 0.1 M Natronlauge (statt 15 ml) verwendet wurden. Das erhaltene Produkt wurde nochmals mittels präparativer HPLC nachgereinigt (Säule: Reprosil-Pur C18, 10 µm, 250 mm x 30 mm; Eluent: Methanol/Wasser mit 0.05% TFA, mit Gradient). Die produkthaltigen Fraktionen wurden vereinigt und im Vakuum eingeengt, der Rückstand in Ethylacetat gelöst und nacheinander mit gesättigter Kaliumcarbonat-Lösung und gesättigter Natriumchlorid-Lösung gewaschen. Die organische Phase wurde über Natriumsulfat getrocknet, filtriert und im Vakuum eingeengt. Nach Trocknen des Rückstands wurden 44 mg (97% Reinheit, 51% d. Th.) der Titelverbindung erhalten.

¹H-NMR (400 MHz, DMSO-d₆, δ/ppm): 10.68 (br, 1H), 9.05 (d, 1H), 8.48 (d, 1H), 8.19 (d, 1H), 7.93 (m, 2H), 7.84 (s, 1H), 7.82 (s, 1H), 7.78 (dd, 1H), 7.55 (d, 1H), 7.49 (d, 1H), 7.42 (dd, 1H), 6.37 (s, 1H), 2.28 (s, 3H), 1.35 (s, 9H).

LC/MS (Methode 4, ESIpos): Rₜ = 1.15 min, m/z = 485 [M+H]⁺.

### Beispiel 66

### 2-tert.-Butyl-6-(methylamino)-N-{4-methyl-3-[6-(pyridin-3-yl)-1H-imidazo[1,2-b]pyrazol-1-yl]-phenyl} isonicotinamid

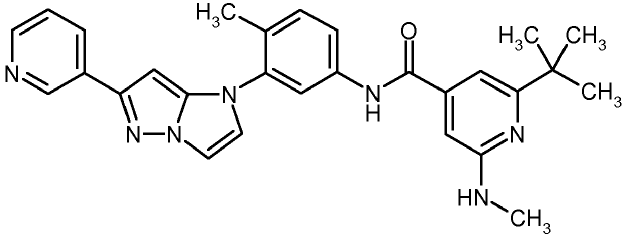

60 mg (0.21 mmol) der Verbindung aus Beispiel 6A und 43 mg (0.21 mmol) der Verbindung aus Beispiel 44A wurden in Analogie zur Vorschrift von Beispiel 33 umgesetzt, mit dem Unterschied, dass die Reaktionszeit in diesem Fall 16 h betrug. Es wurden 31 mg (31% d. Th.) der Titelverbindung erhalten.

¹H-NMR (400 MHz, DMSO-d₆, δ/ppm): 10.40 (s, 1H), 9.05 (d, 1H), 8.48 (dd, 1H), 8.19 (dt, 1H), 7.93 (d, 1H), 7.91 (d, 1H), 7.77 (dd, 1H), 7.54 (d, 1H), 7.45 (d, 1H), 7.41 (dd, 1H), 6.89 (s, 1H), 6.67 (s, 1H), 6.60 (quart, 1H), 6.37 (s, 1H), 2.82 (d, 3H), 2.27 (s, 3H), 1.30 (s, 9H).

LC/MS (Methode 3, ESIneg): Rₜ = 0.78 min, m/z = 478 [M-H]⁻.

### Beispiel 67

### N-{4-Methyl-3-[6-(pyridin-3-yl)-1H-imidazo[1,2-b]pyrazol-1-yl]phenyl}-2-(pyrrolidin-1-yl)pyridin-4-carboxamid

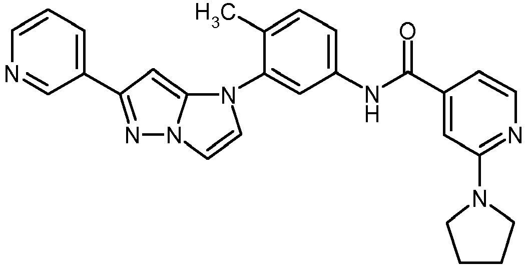

Nach dem unter Beispiel 39 beschriebenen Verfahren wurden aus der Verbindung aus Beispiel 6A und 2-(Pyrrolidin-1-yl)pyridin-4-carbonsäure 8.6 mg (19% d. Th.) der Titelverbindung erhalten.

LC/MS (Methode 31, ESIpos): Rₜ = 0.82 min, m/z = 462 [M+H]⁺, Reinheit 100%.

### Beispiel 68

### N-{4-Methyl-3-[6-(pyridin-3-yl)-1H-imidazo[1,2-b]pyrazol-1-yl]phenyl}-2-(piperidin-1-yl)pyridin-4-carboxamid

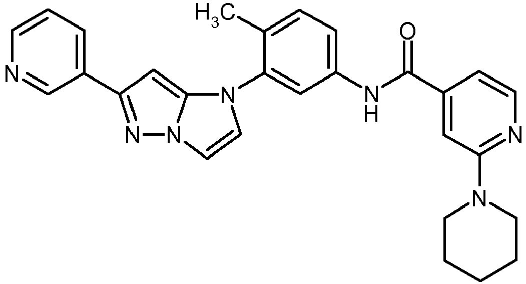

Nach dem unter Beispiel 39 beschriebenen Verfahren wurden aus der Verbindung aus Beispiel 6A und 2-(Piperidin-1-yl)pyridin-4-carbonsäure 30.0 mg (63% d. Th.) der Titelverbindung erhalten.

LC/MS (Methode 31, ESIpos): Rₜ = 0.98 min, m/z = 478 [M+H]⁺, Reinheit 100%.

### Beispiel 69

### N-{4-Methyl-3-[6-(pyridin-3-yl)-1H-imidazo[1,2-b]pyrazol-1-yl]phenyl}-2-(morpholin-4-yl)pyridin-4-carboxamid

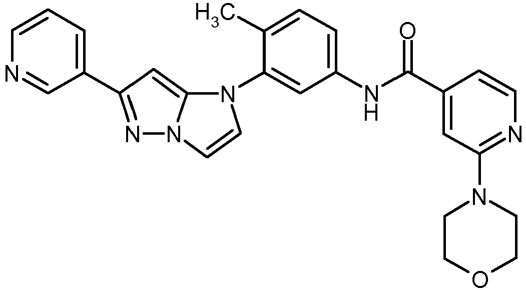

Nach dem unter Beispiel 39 beschriebenen Verfahren wurden aus der Verbindung aus Beispiel 6A und 2-(Morpholin-4-yl)pyridin-4-carbonsäure 10.9 mg (23% d. Th.) der Titelverbindung erhalten.

LC/MS (Methode 31, ESIneg): Rₜ = 1.01 min, m/z = 478 [M-H]⁻, Reinheit 100%.

### Beispiel 70

### N-{4-Fluor-5-[6-(pyridin-3-yl)-1H-imidazo[1,2-b]pyrazol-1-yl]phenyl}-3-(pentafluor-λ⁶-sulfanyl)-benzamid

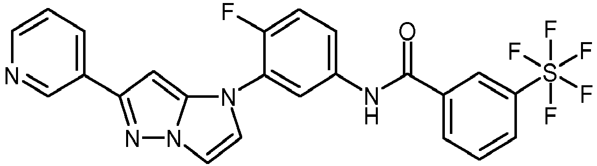

In Analogie zur Vorschrift von Beispiel 50 wurde aus 70 mg (0.24 mmol) der Verbindung aus Beispiel 13A und 59 mg (0.24 mmol) 3-(Pentafluor-λ⁶-sulfanyl)benzoesäure ein Rohprodukt erhalten, welches, abweichend zu Beispiel 50, nach dem ersten Reinigungslauf auf einer Biotage-Anlage durch präparative Dickschicht-Chromatographie (Laufmittel Ethylacetat/Methanol 9:1) weiter gereinigt wurde. Auf diese Weise wurden 39 mg (27% d. Th.) der Titelverbindung erhalten.

¹H-NMR (300 MHz, CDCl₃, δ/ppm): 9.09 (d, 1H), 8.53 (dd, 1H), 8.37 (s, 1H), 8.32 (t, 1H), 8.24 (dd, 1H), 8.15 (dt, 1H), 8.07 (d, 1H), 7.97 (dd, 1H), 7.64 (t, 1H), 7.51 (d, 1H), 7.40-7.46 (m, 1H), 7.30-7.36 (m, 2H), 7.28 (t, 1H), 6.34 (s, 1H).

LC/MS (Methode 5, ESIpos): Rₜ = 1.17 min, m/z = 524 [M+H]⁺.

### Beispiel 71

### 3-Cyano-N-{4-fluor-5-[6-(pyridin-3-yl)-1H-imidazo[1,2-b]pyrazol-1-yl]phenyl}-3-(pentafluor-λ⁶-sulfanyl)benzamid

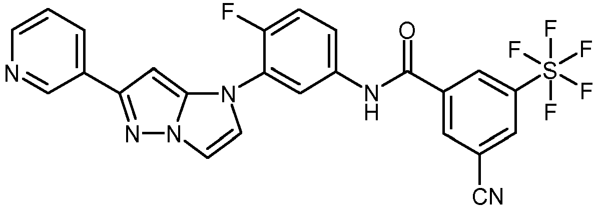

In Analogie zur Vorschrift von Beispiel 50 wurde aus 80 mg (0.27 mmol) der Verbindung aus Beispiel 13A und 74 mg (0.27 mmol) der Verbindung aus Beispiel 23A ein Rohprodukt erhalten, welches, abweichend zu Beispiel 50, auch im zweiten Lauf auf der Biotage-Anlage mit einer 25 g Snap-Säule (statt 10 g) gereinigt wurde. Auf diese Weise wurden 38 mg (25% d. Th.) der Titelverbindung erhalten.

¹H-NMR (300 MHz, DMSO-d₆, δ/ppm): 10.85 (s, 1H), 9.03 (d, 1H), 8.84 (t, 1H), 8.72 (s, 1H), 8.64 (t, 1H), 8.48 (dd, 1H), 8.13-8.21 (m, 2H), 7.96 (d, 1H), 7.74 (ddd, 1H), 7.63 (t, 1H), 7.56 (dd, 1H), 7.41 (dd, 1H), 6.54 (s, 1H).

LC/MS (Methode 5, ESIpos): Rₜ = 1.17 min, m/z = 549 [M+H]⁺.

### Beispiel 72

### N-{4-Methoxy-3-[6-(pyridin-3-yl)-1H-imidazo[1,2-b]pyrazol-1-yl]phenyl}-3-(pentafluor-λ⁶-sulfanyl)benzamid

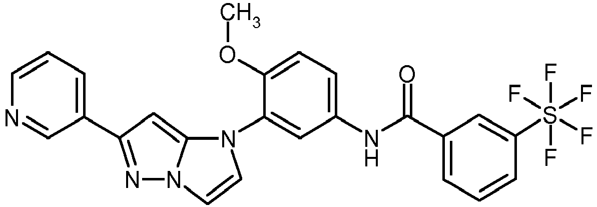

45 mg (0.15 mmol) der Verbindung aus Beispiel 10A und 37 mg (0.15 mmol) 3-(Pentafluor-λ⁶-sulfanyl)benzoesäure wurden in Analogie zur Vorschrift von Beispiel 16 umgesetzt und aufgearbeitet. Es wurden 72 mg (91% d. Th.) der Titelverbindung erhalten.

¹H-NMR (400 MHz, DMSO-d₆, δ/ppm): 10.63 (s, 1H), 9.05 (d, 1H), 8.49 (dd, 1H), 8.42 (s, 1H), 8.29 (d, 1H), 8.21-8.14 (m, 2H), 8.03 (d, 1H), 7.88 (d, 1H), 7.85-7.77 (m, 2H), 7.57 (d, 1H), 7.43 (dd, 1H), 7.34 (d, 1H), 6.43 (s, 1H), 3.91 (s, 3H).

LC/MS (Methode 3, ESIpos): Rₜ = 0.98 min, m/z = 536 [M+H]⁺.

### Beispiel 73

### N-{2,4-Dimethyl-5-[6-(pyridin-3-yl)-1H-imidazo[1,2-b]pyrazol-1-yl]phenyl}-3-(pentafluor-λ⁶-sulfanyl)benzamid

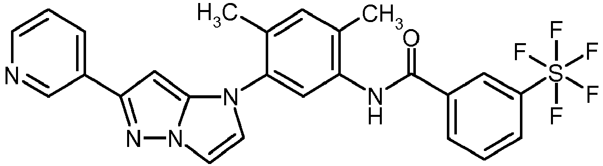

45 mg (87% Reinheit, 0.13 mmol) der Verbindung aus Beispiel 11A und 32 mg (0.153 mmol) 3-(Pentafluor-λ⁶-sulfanyl)benzoesäure wurden in Analogie zur Vorschrift von Beispiel 38 umgesetzt und aufgearbeitet. Es wurden 36 mg (96% Reinheit, 50% d. Th.) der Titelverbindung erhalten.

¹H-NMR (400 MHz, DMSO-d₆, δ/ppm): 10.32 (s, 1H), 9.04 (s, 1H), 8.48 (d, 1H), 8.42 (s, 1H), 8.29 (d, 1H), 8.17 (m, 2H), 7.90 (d, 1H), 7.81 (t, 1H), 7.51 (s, 1H), 7.49 (d, 1H), 7.43-7.3 (m, 2H), 6.31 (s, 1H), 2.30 (s, 3H), 2.26 (s, 3H).

LC/MS (Methode 3, ESIpos): Rₜ = 1.03 min, m/z = 534 [M+H]⁺.

### Beispiel 74

### 3-Cyano-N-{2,4-dimethyl-5-[6-(pyridin-3-yl)-1H-imidazo[1,2-b]pyrazol-1-yl]phenyl}-5-(pentafluor-λ⁶-sulfanyl)benzamid

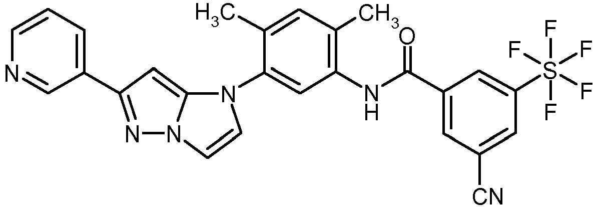

### Varianten A:

60 mg (87% Reinheit, 0.17 mmol) der Verbindung aus Beispiel 11A und 55 mg (85% Reinheit, 0.17 mmol) der Verbindung aus Beispiel 23A wurden in Analogie zur Vorschrift von Beispiel 38 umgesetzt und aufgearbeitet. Es wurden 38 mg (40% d. Th.) der Titelverbindung erhalten.

¹H-NMR (400 MHz, DMSO-d₆, δ/ppm): 10.42 (s, 1H), 9.04 (d, 1H), 8.86 (s, 1H), 8.74 (s, 1H), 8.66 (s, 1H), 8.50 (dd, 1H), 8.17 (dt, 1H), 7.90 (d, 1H), 7.53 (s, 1H), 7.49 (d, 1H), 7.43-7.38 (m, 2H), 6.31 (s, 1H), 2.31 (s, 3H), 2.26 (s, 3H).

LC/MS (Methode 3, ESIpos): Rₜ = 1.00 min, m/z = 559 [M+H]⁺.

### Variante B:

5.75 g (18.9 mmol) der Verbindung aus Beispiel 11A und 5.18 g (18.9 mmol) der Verbindung aus Beispiel 23A wurden in 30 ml DMF gelöst und nacheinander mit 8.65 g (22.7 mmol) HATU und 6.6 ml (37.9 mmol) *N*,*N*-Diisopropylethylamin versetzt. Nach 4 h Rühren bei RT wurde das Reaktionsgemisch in ca. 120 ml kalte, gesättigte wässrige Natriumhydrogencarbonat-Lösung eingerührt. Dann wurde dreimal mit je ca. 100 ml Ethylacetat extrahiert. Die vereinigten organischen Extrakte wurden über Magnesiumsulfat getrocknet, filtriert und im Vakuum eingeengt. Der erhaltene Rückstand wurde mittels MPLC gereinigt (350 g Kieselgel, Laufmittel Dichlormethan/Methanol 100:1 → 50:1). Nach Eindampfen der Produktfraktionen wurde ein Produkt von ca. 95% Reinheit erhalten. Eine weitergehende Aufreinigung wurde durch Umkristallisation aus einem Lösungsmittelgemisch bestehend aus 100 ml Isopropanol und 80 ml Wasser erreicht. Nach Filtration bei RT und Trocknen des Feststoffs im Hochvakuum wurden so 9.01 g (84% d. Th.) der Titelverbindung erhalten.

¹H-NMR (400 MHz, DMSO-d₆, δ/ppm): 10.42 (s, 1H), 9.04 (d, 1H), 8.86 (s, 1H), 8.74 (s, 1H), 8.66 (s, 1H), 8.48 (dd, 1H), 8.17 (dt, 1H), 7.90 (d, 1H), 7.53 (s, 1H), 7.49 (d, 1H), 7.42 (dd, 1H), 7.40 (s, 1H), 6.31 (s, 1H), 2.31 (s, 3H), 2.26 (s, 3H).

LC/MS (Methode 3, ESIpos): Rₜ = 1.06 min, m/z = 559 [M+H]⁺.

### Beispiel 75

### 3-Cyano-N-{2-fluor-4-methyl-5-[6-(pyridin-3-yl)-1H-imidazo[1,2-b]pyrazol-1-yl]phenyl}-5-(pentafluor-λ⁶-sulfanyl)benzamid

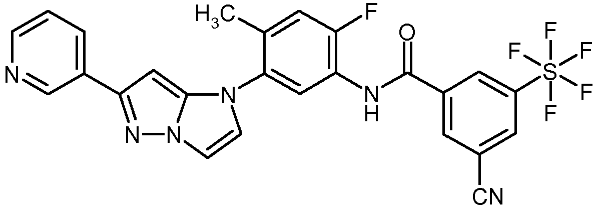

55 mg (0.18 mmol) der Verbindung aus Beispiel 12A und 58 mg (85% Reinheit, 0.18 mmol) der Verbindung aus Beispiel 23A wurden in Analogie zur Vorschrift von Beispiel 38 umgesetzt und aufgearbeitet. Es wurden 43 mg (42% d. Th.) der Titelverbindung erhalten.

¹H-NMR (400 MHz, DMSO-d₆, δ/ppm): 10.77 (s, 1H), 9.04 (d, 1H), 8.87 (s, 1H), 8.72 (s, 1H), 8.67 (s, 1H), 8.48 (dd, 1H), 8.18 (dt, 1H), 7.92 (d, 1H), 7.84 (d, 1H), 7.54-7.50 (m, 2H), 7.42 (d, 1H), 6.35 (s, 1H), 2.29 (s, 3H).

LC/MS (Methode 3, ESIpos): Rₜ = 0.98 min, m/z = 563 [M+H]⁺.

### Beispiel 76

### N-{2-Methyl-5-[6-(pyridin-3-yl)-1H-imidazo[1,2-b]pyrazol-1-yl]phenyl}-3-(pentafluor-λ⁶-sulfanyl)-benzamid

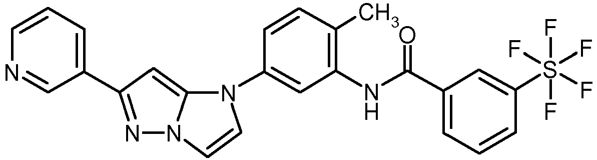

In Analogie zur Vorschrift von Beispiel 50 wurde aus 100 mg (0.35 mmol) der Verbindung aus Beispiel 14A und 86 mg (0.35 mmol) 3-(Pentafluor-λ⁶-sulfanyl)benzoesäure ein Rohprodukt erhalten, welches, abweichend zu Beispiel 50, nach dem ersten Reinigungslauf auf der Biotage-Anlage zunächst durch präparative Dickschicht-Chromatographie (Laufmittel Ethylacetat/Methanol 95:5) und anschließend durch präparative HPLC (Methode 15) weiter gereinigt wurde. Die so erhaltenen 7 mg an Produkt wurden in wenig Ethylacetat gelöst und nacheinander mit 15 ml gesättigter Natriumhydrogencarbonat-Lösung und gesättigter Natriumchlorid-Lösung gewaschen. Die organische Phase wurde über Natriumsulfat getrocknet und nach Filtration im Vakuum eingeengt. Auf diese Weise wurden 5.6 mg (2.6% d. Th.) der Titelverbindung erhalten.

¹H-NMR (300 MHz, CDCl₃, δ/ppm): 9.11 (br. s, 1H), 8.54 (d, 1H), 8.40 (d, 1H), 8.35 (s, 1H), 8.19 (d, 1H), 8.04 (d, 1H), 7.99 (dd, 1H), 7.85 (s, 1H), 7.67 (t, 1H), 7.51 (d, 1H), 7.30-7.43 (m, 4H), 6.47 (s, 1H), 2.41 (s, 3H).

LC/MS (Methode 5, ESIpos): Rₜ = 1.15 min, m/z = 520 [M+H]⁺.

### Beispiel 77

### 3-Cyano-N-{2-methyl-5-[6-(pyridin-3-yl)-1H-imidazo[1,2-b]pyrazol-1-yl]phenyl}-3-(pentafluor-λ⁶-sulfanyl)benzamid

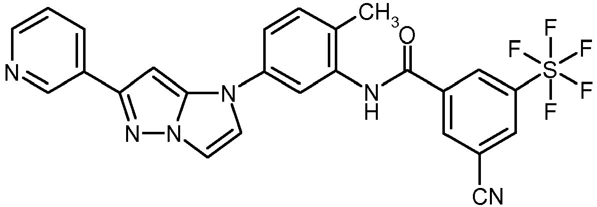

Zu einer Lösung von 189 mg (0.69 mmol) der Verbindung aus Beispiel 14A und 200 mg (0.69 mmol) 3-Cyano-5-(pentafluor-λ⁶-sulfanyl)benzoesäure aus Beispiel 23A in 4.4 ml DMSO wurden 315 mg (0.83 mmol) HATU und 179 mg (1.38 mmol) *N*,*N*-Diisopropylethylamin gegeben. Der Ansatz wurde 16 h bei 25°C gerührt. Dann wurden nochmals 60 mg (0.22 mmol) 3-Cyano-5-(pentafluor-λ⁶-sulfanyl)benzoesäure, 150 mg (0.41 mmol) HATU sowie 74 mg (0.57 mmol) *N*,*N*-Diisopropylethylamin hinzugegeben und das Gemisch mehrere Stunden auf 40°C erwärmt. Nach dem Abkühlen wurde das Reaktionsgemisch auf Wasser gegeben und mit Ethylacetat extrahiert. Die organische Phase wurde zweimal mit je 50 ml gesättigter Natriumhydrogencarbonat-Lösung und einmal mit gesättigter Natriumchlorid-Lösung gewaschen und über Natriumsulfat getrocknet. Nach Filtration wurde im Vakuum eingeengt und der so erhaltene Rückstand durch Chromatographie an einer Biotage-Anlage gereinigt (25 g Snap-Säule; Laufmittelgradient Ethylacetat/Hexan, beginnend mit 20% Ethylacetat, dann schnell auf 100% Ethylacetat ansteigend, danach Ethylacetat/ Methanol, von 0% Methanol bis 50% Methanol stetig ansteigend). Das so erhaltene Produkt wurde in Ethylacetat aufgenommen und dreimal mit je 25 ml Natriumhydrogencarbonat-Lösung und einmal mit gesättigter Natriumchlorid-Lösung gewaschen und über Natriumsulfat getrocknet. Nach Filtration wurde im Vakuum eingeengt. Auf diese Weise wurden 60 mg (15% d. Th.) der Titelverbindung erhalten.

¹H-NMR (300 MHz, DMSO-d₆, δ/ppm): 10.47 (s, 1H), 9.06 (d, 1H), 8.84 (s, 1H), 8.75 (s, 1H), 8.69 (s, 1H), 8.48 (dd, 1H), 8.19 (dt, 1H), 7.93 (d, 1H), 7.91 (d, 1H), 7.75 (d, 1H), 7.58 (dd, 1H), 7.39-7.48 (m, 2H), 6.85 (s, 1H), 2.27 (s, 3H).

LC/MS (Methode 5, ESIpos): Rₜ = 1.15 min, m/z = 545 [M+H]⁺.

### Beispiel 78

### 3-Brom-5-tert.-butyl-N-{2-methyl-5-[6-(pyridin-3-yl)-1H-imidazo[1,2-b]pyrazol-1-yl]phenyl}-3-(pentafluor-λ⁶-sulfanyl)benzamid

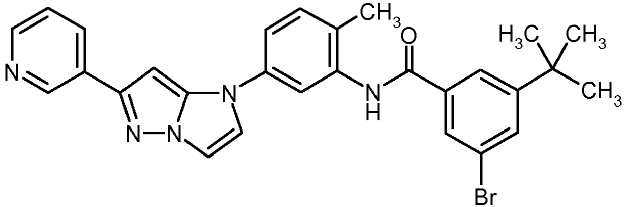

In Analogie zur Vorschrift von Beispiel 77 wurden aus 200 mg (0.69 mmol) der Verbindung aus Beispiel 14A und 248 mg (0.96 mmol) 3-Brom-5-*tert*.-butylbenzoesäure ein Rohprodukt erhalten, welches zunächst durch Chromatographie an einer Biotage-Anlage, wie zuvor beschrieben, gereinigt wurde. Man erhielt so 145 mg (40% d. Th.) der Titelverbindung, die ohne weitere Reinigung in der Folgereaktion eingesetzt wurden, sowie 60 mg an noch verunreinigtem Material, welches durch weitere Trennung mittels präparativer HPLC (Methode 16) gereinigt wurde. Auf diese Weise wurden weitere 14 mg (3.6% d. Th.) der Titelverbindung erhalten.

¹H-NMR (300 MHz, CDCl₃, δ/ppm): 9.13 (d, 1H), 8.55 (dd, 1H), 8.48 (d, 1H), 8.20 (dt, 1H), 7.90 (s, 1H), 7.79 (s, 1H), 7.74 (t, 2H), 7.51 (d, 1H), 7.30-7.40 (m, 3H), 7.25 (dd, 1H), 6.50 (s, 1H), 2.41 (s, 3H), 1.38 (s, 9H).

LC/MS (Methode 5, ESIpos): Rₜ = 1.32 min, m/z = 528/530 [M+H]⁺.

### Beispiel 79

### 3-Cyano-5-tert.-butyl-N-{2-methyl-5-[6-(pyridin-3-yl)-1H-imidazo[1,2-b]pyrazol-1-yl]phenyl}-3-(pentafluor-λ⁶-sulfanyl)benzamid

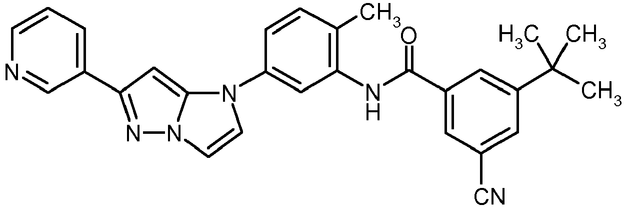

Zu einer Lösung von 145 mg (0.28 mmol) der Verbindung aus Beispiel 78 in 5.0 ml DMF (unter Argon entgast) wurden 35.5 mg (0.30 mmol) Zinkcyanid und 19 mg (0.016 mmol) Tetrakis(triphenylphosphin)palladium(0) gegeben und das Gemisch 2 h unter Rückfluss erhitzt. Das Reaktionsgemisch wurde nach dem Abkühlen mit Ethylacetat versetzt, und die organische Phase wurde nacheinander mit gesättigter Natriumhydrogencarbonat-Lösung und gesättigter Natriumchlorid-Lösung gewaschen. Nach dem Trocknen über Natriumsulfat und Filtration wurde im Vakuum eingeengt. Der so erhaltene Rückstand wurde durch Chromatographie an einer Biotage-Anlage gereinigt (10 g Snap-Säule; Laufmittelgradient Ethylacetat/Hexan, beginnend mit 20% Ethylacetat, dann schnell auf 100% Ethylacetat ansteigend, danach Ethylacetat/Methanol, von 0% Methanol bis 30% Methanol stetig ansteigend). Auf diese Weise wurden 44 mg (29% d. Th.) der Titelverbindung erhalten.

¹H-NMR (300 MHz, CDCl₃, δ/ppm): 9.12 (d, 1H), 8.55 (dd, 1H), 8.45 (d, 1H), 8.16-8.25 (m, 2H), 7.94 (s, 1H), 7.89 (s, 1H), 7.82 (s, 1H), 7.51 (d, 1H), 7.30-7.41 (m, 3H), 6.50 (s, 1H), 2.42 (s, 3H), 1.41 (s, 9H).

LC/MS (Methode 5, ESIpos): Rₜ = 1.18 min, m/z = 475 [M+H]⁺.

### Beispiel 80

### 3-Cyano-N-{2-methyl-3-[6-(pyridin-3-yl)-1H-imidazo[1,2-b]pyrazol-1-yl]phenyl}-5-(pentafluor-λ⁶-sulfanyl)benzamid

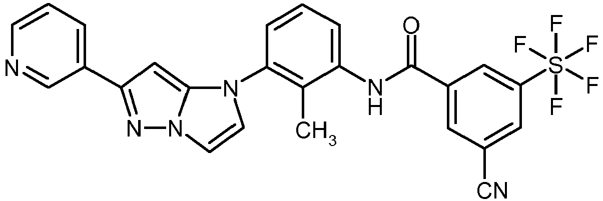

100 mg (0.346 mmol) der Verbindung aus Beispiel 69A und 111 mg (0.346 mmol, 85% Reinheit) der Verbindung aus Beispiel 23A wurden in 1.9 ml wasserfreiem DMF gelöst und nacheinander mit 158 mg (0.415 mmol) HATU und 72 µl (0.415 mmol) *N*,*N*-Diisopropylethylamin versetzt. Nachdem das Reaktionsgemisch über Nacht (ca. 15 h) bei RT gerührt worden war, wurde es in ca. 10 ml Wasser eingerührt. Der ausgefallene Niederschlag wurde abgesaugt, in Dichlormethan gelöst und nacheinander mit halbgesättigter wässriger Natriumhydrogencarbonat-Lösung und gesättigter Natriumchlorid-Lösung gewaschen. Nach Trocknen über wasserfreiem Magnesiumsulfat, Filtration und Entfernen des Lösungsmittels am Rotationsverdampfer wurde der so erhaltene Rückstand mittels präparativer HPLC gereinigt (Methode 33). Nach Eindampfen der Produktfraktionen wurde der Feststoff in Ethylacetat erneut gelöst und mit gesättigter wässriger Natriumhydrogencarbonat-Lösung gewaschen, um das Produkt in die freie Basenform zu überführen. Nach Trocknen der organischen Phase über wasserfreiem Magnesiumsulfat, Filtration und Eindampfen wurde das Produkt abschließend mit 3 ml Pentan, dem einige Tropfen Diisopropylether beigefügt waren, verrührt. Nach Absaugen und Trocknen des Feststoffs im Hochvakuum wurden so 66 mg (35% d. Th.) der Titelverbindung erhalten.

¹H-NMR (400 MHz, CDCl₃, δ/ppm): 9.00 (s, 1H), 8.69 (s, breit, 1H), 8.63 (s, 1H), 8.52 (d, 1H), 8.45 (s, 1H), 8.23 (s, 1H), 8.15 (dt, 1H), 7.70 (d, 1H), 7.52 (d, 1H), 7.44 (t, 1H), 7.37 (d, 1H), 7.33 (dd, 1H), 6.95 (d, 1H), 5.96 (s, 1H), 2.24 (s, 3H).

LC/MS (Methode 4, ESIpos): Rₜ = 0.97 min, m/z = 545 [M+H]⁺.

### Beispiel 81

### 3-Cyano-N-{2-fluor-3-[6-(pyridin-3-yl)-1H-imidazo[1,2-b]pyrazol-1-yl]phenyl}-5-(pentafluor-λ⁶-sulfanyl)benzamid

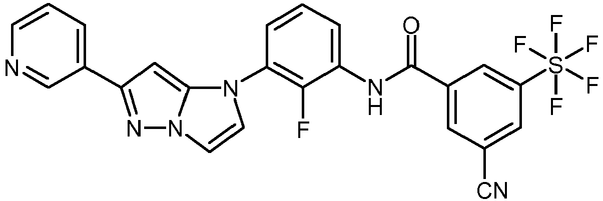

Analog zu dem unter Beispiel 96 beschriebenen Verfahren wurden aus 100 mg (0.341 mmol) der Verbindung aus Beispiel 70A und 110 mg (0.341 mmol, 85% Reinheit) der Verbindung aus Beispiel 23A 52 mg (28% d. Th.) der Titelverbindung erhalten. Die Reinigung erfolgte hier mittels präparativer HPLC nach Methode 33, und auf ein abschließendes Verrühren des Produktes konnte verzichtet werden.

¹H-NMR (400 MHz, CDCl₃, δ/ppm): 9.05 (d, 1H), 8.59 (breit, 1H), 9.58 (s, 1H), 8.55 (dd, 1H), 8.39 (s, 1H), 8.27-8.23 (m, 2H), 8.17 (dt, 1H), 7.55 (d, 1H), 7.45 (td, 1H), 7.39 (t, 1H), 7.35 (dd, 1H), 7.18 (t, 1H), 6.25 (s, 1H).

LC/MS (Methode 4, ESIpos): Rₜ = 1.00 min, m/z = 549 [M+H]⁺.

### Beispiel 82

### N-{2-Hydroxy-3-[6-(pyridin-3-yl)-1H-imidazo[1,2-b]pyrazol-1-yl]phenyl}-3-(pentafluor-λ⁶-sulfanyl)benzamid

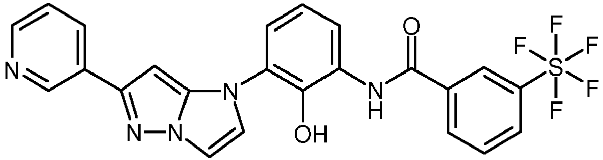

80 mg (0.192 mmol) der Verbindung aus Beispiel 71A und 48 mg (0.192 mmol) 3-(Pentafluor-λ⁶-sulfanyl)benzoesäure wurden in 1.5 ml wasserfreiem DMF gelöst und nacheinander mit 88 mg (0.231 mmol) HATU und 40 µl (0.231 mmol) *N,N*-Diisopropylethylamin versetzt. Nachdem das Reaktionsgemisch 30 min bei RT gerührt worden war, wurde es in ca. 10 ml Wasser eingerührt. Der ausgefallene Niederschlag wurde abgesaugt, in 3 ml Methanol gelöst und mit 385 µl (0.385 mmol) 1 M Natronlauge versetzt. Das Gemisch wurde 30 min bei RT gerührt und dann komplett mittels präparativer HPLC in seine Komponenten aufgetrennt (Methode 33). Die Produktfraktionen wurden vereinigt und vom Lösungsmittel befreit. Der Rückstand wurde mit einem Gemisch aus 5 ml Pentan und 1 ml Dichlormethan bei RT verrührt. Nach Absaugen und Trocknen des Feststoffs im Hochvakuum wurden 32 mg (32% d. Th.) der Titelverbindung erhalten.

¹H-NMR (400 MHz, DMSO-d₆, δ/ppm): 10.36 (s, breit, 1H), 9.78 (s, breit, 1H), 9.05 (d, 1H), 8.51 (s, 1H), 8.49 (dd, 1H), 8.33 (d, 1H), 8.20-8.15 (m, 2H), 7.87 (d, 1H), 7.82 (t, 1H), 7.57 (d, 1H), 7.47-7.41 (m, 3H), 7.07 (t, 1H), 6.40 (s, 1H).

LC/MS (Methode 3, ESIpos): Rₜ = 0.98 min, m/z = 522 [M+H]⁺.

### Beispiel 83

### 3-Cyano-N-{2-hydroxy-3-[6-(pyridin-3-yl)-1H-imidazo[1,2-b]pyrazol-1-yl]phenyl}-5-(pentafluor-λ⁶-sulfanyl)benzamid

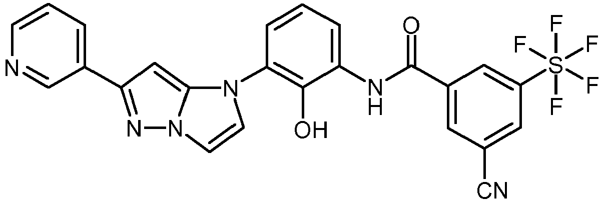

74 mg (0.178 mmol, 70% Reinheit) der Verbindung aus Beispiel 71A und 44 mg (0.160 mmol) der Verbindung aus Beispiel 23A wurden in 1 ml wasserfreiem DMF gelöst und nacheinander mit 81 mg (0.213 mmol) HATU und 37 µl (0.213 mmol) *N,N*-Diisopropylethylamin versetzt. Nachdem das Reaktionsgemisch 30 min bei RT gerührt worden war, wurde es in ca. 10 ml Wasser eingerührt. Der ausgefallene Niederschlag wurde abgesaugt, in ca. 3 ml Methanol gelöst und mittels präparativer HPLC in seine Komponenten aufgetrennt (Methode 33). Die Produktfraktionen wurden vereinigt und vom Lösungsmittel befreit. Der erhaltene Rückstand wurde mit wenig Dichlormethan bei RT verrührt. Nach Absaugen und Trocknen des Feststoffs im Hochvakuum wurden 36 mg (37% d. Th.) der Titelverbindung erhalten.

¹H-NMR (400 MHz, DMSO-d₆, δ/ppm): 10.49 (s, breit, 1H), 9.81 (s, breit, 1H), 9.05 (s, 1H), 8.86 (s, 1H), 8.76 (s, 1H), 8.75 (s, 1H), 8.49 (d, 1H), 8.19 (d, 1H), 7.88 (d, 1H), 7.57 (d, 1H), 7.48-7.41 (m, 3H), 7.07 (t, 1H), 6.41 (s, 1H).

LC/MS (Methode 3, ESIpos): Rₜ = 0.97 min, m/z = 547 [M+H]⁺.

### Beispiel 84

### N-{4-Methyl-3-[6-(pyrazin-2-yl)-1H-imidazo[1,2-b]pyrazol-1-yl]phenyl}-3-(pentafluor-λ⁶-sulfanyl)-benzamid

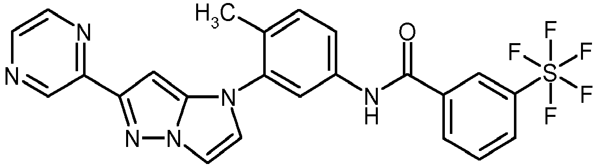

In Analogie zu Beispiel 90 wurden aus 80 mg (0.28 mmol) der Verbindung aus Beispiel 72A und 75 mg (0.30 mmol) 3-(Pentafluor-λ⁶-sulfanyl)benzoesäure 64 mg (45% d. Th.) der Titelverbindung erhalten.

¹H-NMR (400 MHz, DMSO-d₆, δ/ppm): 10.65 (s, 1H), 9.16 (d, 1H), 8.58 (dd, 1H), 8.50 (d, 1H), 8.37 (t, 1H), 8.24 (d, 1H), 8.12 (dd, 1H), 7.94 (t, 2H), 7.72-7.82 (m, 2H), 7.62 (d, 1H), 7.43 (d, 1H), 6.36 (d, 1H), 2.26 (s, 3H).

LC/MS (Methode 7, ESIpos): Rₜ = 1.29 min, m/z = 521 [M+H]⁺.

### Beispiel 85

### 3-Cyano-N-{4-methyl-3-[6-(1H-pyrazol-4-yl)-1H-imidazo[1,2-b]pyrazol-1-yl]phenyl}-5-(pentafluor-λ⁶-sulfanyl)benzamid

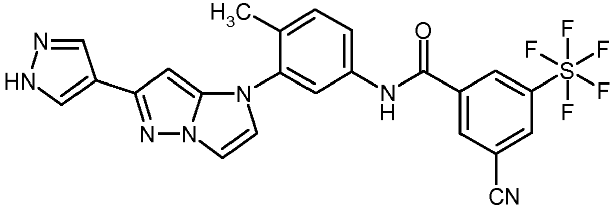

80 mg (0.10 mmol) der Verbindung aus Beispiel 75A wurden in 0.8 ml Trifluoressigsäure für 60 min bei 90°C gerührt. Der Ansatz wurde danach im Vakuum eingeengt und der Rückstand mittels präparativer HPLC (Methode 34) gereinigt. Es wurden 49 mg (90% d. Th.) der Titelverbindung erhalten.

¹H-NMR (400 MHz, DMSO-d₆, δ/ppm): 12.86 (breit, 1H), 10.78 (s, 1H), 8.86 (s, 1H), 8.74 (s, 1H), 8.66 (s, 1H), 8.02 (s, 1H), 7.92 (d, 1H), 7.82-7.74 (m, 3H), 7.48 (d, 1H), 7.42 (d, 1H), 5.92 (s, 1H), 2.28 (s, 3H).

LC/MS (Methode 3, ESIpos): Rₜ = 1.03 min, m/z = 534 [M+H]⁺.

### Beispiel 86

### 3-Cyano-N-{2,4-dimethyl-5-[6-(1H-pyrazol-4-yl)-1H-imidazo[1,2-b]pyrazol-1-yl]phenyl}-5-(pentafluor-λ⁶-sulfanyl)benzamid

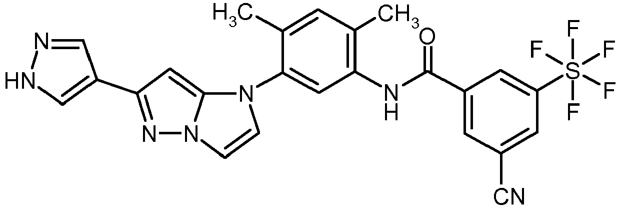

120 mg (0.19 mmol) der Verbindung aus Beispiel 76A wurden in Analogie zu Beispiel 12 umgesetzt und aufgearbeitet. Es wurden 61 mg (61% d. Th.) der Titelverbindung erhalten.

¹H-NMR (400 MHz, DMSO-d₆, δ/ppm): 12.84 (breit, 1H), 10.40 (s, 1H), 8.85 (s, 1H), 8.73 (s, 1H), 8.66 (s, 1H), 7.99 (s, 1H), 7.76 (d, 2H), 7.49 (s, 1H), 7.36 (d, 2H), 5.86 (s, 1H), 2.30 (s, 3H), 2.25 (s, 3H).

LC/MS (Methode 3, ESIpos): Rₜ = 1.03 min, m/z = 548 [M+H]⁺.

### Beispiel 87

### 3-Cyano-5-(pentafluor-λ⁶-sulfanyl)-N-{3-[6-(pyridin-3-yl)-1H-imidazo[1,2-b]pyrazol-1-yl]phenyl}-benzamid

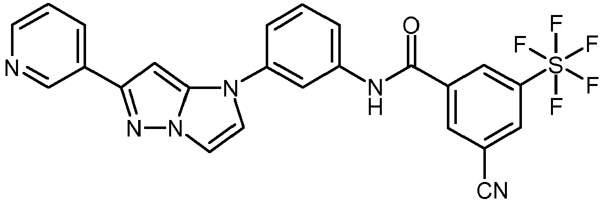

Analog zu dem unter Beispiel 26 beschriebenen Verfahren wurden aus 100 mg (0.363 mmol) der Verbindung aus Beispiel 62A und 117 mg (0.363 mmol) der Verbindung aus Beispiel 23A 62 mg (32% d. Th.) der Titelverbindung erhalten. Die Reaktionszeit betrug in diesem Fall ca. 15 h.

¹H-NMR (400 MHz, CDCl₃, δ/ppm): 9.57 (s, breit, 1H), 9.09 (d, 1H), 8.68 (s, 1H), 8.55-8.53 (m, 2H), 8.22-8.16 (m, 3H), 7.53-7.51 (m, 3H), 7.36 (dd, 1H), 7.32-7.27 (m, 2H), 6.47 (s, 1H).

LC/MS (Methode 4, ESIpos): Rₜ = 1.04 min, m/z = 531 [M+H]⁺.

### Beispiel 88

### 3-Fluor-N-{4-methyl-3-[6-(pyridin-3-yl)-1H-imidazo[1,2-b]pyrazol-1-yl]phenyl}-5-(pentafluor-λ⁶-sulfanyl)benzamid

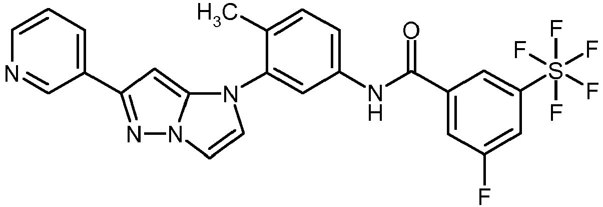

Analog zu dem unter Beispiel 26 beschriebenen Verfahren wurden aus 100 mg (0.346 mmol) der Verbindung aus Beispiel 6A und 97 mg (0.363 mmol) 3-Fluor-5-(pentafluor-λ⁶-sulfanyl)benzoesäure (JRD Fluorochemicals Ltd., Großbritannien) 165 mg (89% d. Th.) der Titelverbindung erhalten. Die Reaktionszeit betrug in diesem Fall ca. 15 h.

¹H-NMR (400 MHz, DMSO-d₆, δ/ppm): 10.72 (s, breit, 1H), 9.06 (d, 1H), 8.49 (dd, 1H), 8.30 (s, 1H), 8.25 (dt, 1H), 8.21-8.17 (m, 2H), 7.95 (d, 1H), 7.93 (d, 1H), 7.79 (dd, 1H), 7.56 (d, 1H), 7.49 (d, 1H), 6.42 (dd, 1H), 6.37 (s, 1H), 2.29 (s, 3H).

LC/MS (Methode 4, ESIpos): Rₜ = 1.08 min, m/z = 538 [M+H]⁺.

### Beispiel 89

### 3-Brom-N-{4-methyl-3-[6-(pyridin-3-yl)-1H-imidazo[1,2-b]pyrazol-1-yl]phenyl}-5-(pentafluor-λ⁶-sulfanyl)benzamid

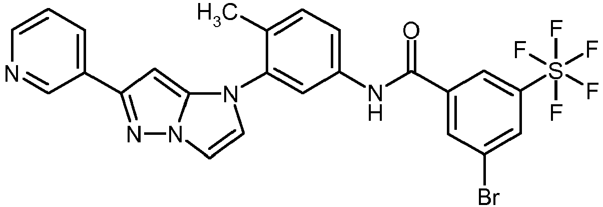

10.0g (34.6 mmol) der Verbindung aus Beispiel 6A und 11.3 g (34.6 mmol) der Verbindung aus Beispiel 15A wurden in 120 ml wasserfreiem DMF gelöst und nacheinander mit 15.8 g (41.5 mmol) HATU und 7.2 ml (41.5 mmol) *N,N-*Diisopropylethylamin versetzt. Nachdem das Reaktionsgemisch über Nacht (ca. 15 h) bei RT gerührt worden war, wurde es in 1.6 Liter Wasser eingerührt. Nach 40 Minuten wurde der ausgefallene Niederschlag abgesaugt, gründlich mit weiteren 0.5 Liter Wasser gewaschen und schließlich in 1.8 Liter Ethylacetat gelöst. Die organische Lösung wurde nacheinander mit Wasser und gesättigter Natriumchlorid-Lösung gewaschen. Nach Trocknen über wasserfreiem Magnesiumsulfat wurde filtriert und das Lösungsmittel am Rotationsverdampfer entfernt. Das so erhaltene Rohprodukt wurde mittels Saugfiltration gereinigt (ca. 330 g Kieselgel, Laufmittelgradient Cyclohexan/Ethylacetat 1:1 → 1:3). Es wurden 11.95 g (57% d. Th.) der Titelverbindung erhalten.

¹H-NMR (400 MHz, DMSO-d₆, δ/ppm): 10.73 (s, breit, 1H), 9.06 (d, 1H), 8.50 (s, 1H), 8.48 (dd, 1H), 8.43 (s, 1H), 8.40 (s, 1H), 8.19 (dt, 1H), 7.93 (m, 2H), 7.79 (dd, 1H), 7.55 (d, 1H), 7.49 (d, 1H), 7.42 (dd, 1H), 6.37 (s, 1H), 2.28 (s, 3H).

LC/MS (Methode 3, ESIpos): Rₜ = 1.12 min, m/z = 598/600 [M+H]⁺.

### Beispiel 90

### 2-Methoxy-N-{4-methyl-3-[6-(pyridin-3-yl)-1H-imidazo[1,2-b]pyrazol-1-yl]phenyl}-5-(pentafluor-λ⁶-sulfanyl)benzamid

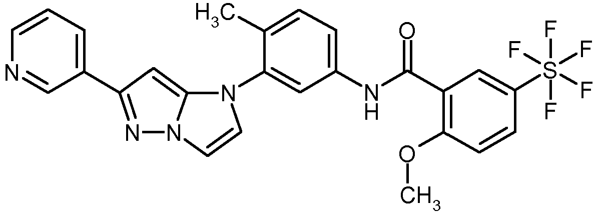

Zu einer Lösung von 200 mg (0.69 mmol) der Verbindung aus Beispiel 6A und 288 mg (1.04 mmol) 2-Methoxy-5-(pentafluor-λ⁶-sulfanyl)benzoesäure in 2.2 ml DMF wurden 394 mg (1.04 mmol) HATU und 127 mg (1.04 mmol) 4-*N,N*-Dimethylaminopyridin (DMAP) gegeben. Der Ansatz wurde 16 h bei 50°C gerührt. Anschließend wurde das Reaktionsgemisch direkt mittels präparativer HPLC gereinigt (Methode 16). Es wurden 222 mg (53% d. Th.) der Titelverbindung erhalten.

¹H-NMR (400 MHz, DMSO-d₆, δ/ppm): 10.40 (s, 1H), 9.01 (d, 1H), 8.45 (dd, 1H), 8.15 (dt, 1H), 7.96-8.05 (m, 2H), 7.87 (d, 2H), 7.68 (dd, 1H), 7.49 (d, 1H), 7.29-7.44 (m, 3H), 6.30 (s, 1H), 3.93 (s, 3H), 2.22 (s, 3H).

LC/MS (Methode 7, ESIpos): Rₜ = 1.32 min, m/z = 550 [M+H]⁺.

### Beispiel 91

### 2-Methyl-N-{4-methyl-3-[6-(pyridin-3-yl)-1H-imidazo[1,2-b]pyrazol-1-yl]phenyl}-5-(pentafluor-λ⁶-sulfanyl)benzamid

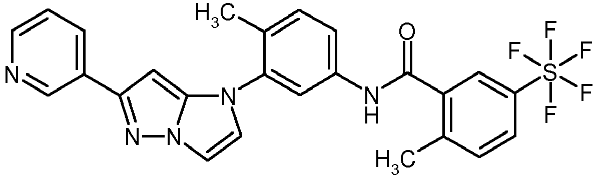

In Analogie zu Beispiel 90 wurden aus 200 mg (0.69 mmol) der Verbindung aus Beispiel 6A und 272 mg (1.04 mmol) 2-Methyl-5-(pentafluor-λ⁶-sulfanyl)benzoesäure 150 mg (39% d. Th.) der Titelverbindung erhalten.

¹H-NMR (400 MHz, DMSO-d₆, δ/ppm): 10.65 (s, 1H), 9.01 (d, 1H), 8.44 (dd, 1H), 8.14 (dt, 1H), 7.82-7.95 (m, 4H), 7.67 (dd, 1H), 7.53 (d, 1H), 7.49 (d, 1H), 7.42 (d, 1H), 7.38 (ddd, 1H), 6.30 (s, 1H), 2.41 (s, 3H), 2.23 (s, 3H).

LC/MS (Methode 7, ESIpos): Rₜ = 1.29 min, m/z = 534 [M+H]⁺.

### Beispiel 92

### 3-Cyano-5-(1-hydroxycyclobutyl)-N-{4-methyl-3-[6-(pyridin-3-yl)-1H-imidazo[1,2-b]pyrazol-1-yl]-phenyl}benzamid

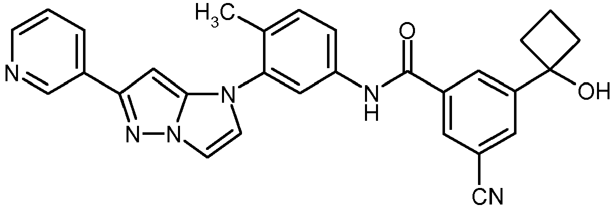

Analog zu dem unter Beispiel 26 beschriebenen Verfahren wurden aus 80 mg (0.276 mmol) der Verbindung aus Beispiel 6A und 60 mg (0.276 mmol) der Verbindung aus Beispiel 73A 115 mg (82% d. Th., 96% Reinheit) der Titelverbindung erhalten. Die Reaktionszeit betrug in diesem Fall 1 h. Das abschließende Verrühren des Produktes erfolgte in einem Gemisch aus 10 ml Pentan und 2 ml Diisopropylether.

¹H-NMR (400 MHz, DMSO-d₆, δ/ppm): 10.61 (s, breit, 1H), 9.06 (d, 1H), 8.48 (dd, 1H), 8.33 (dd, 1H), 8.30 (dd, 1H), 8.19 (dt, 1H), 8.12 (dd, 1H), 7.96 (d, 1H), 7.93 (d, 1H), 7.81 (dd, 1H), 7.56 (d, 1H), 7.48 (d, 1H), 7.42 (dd, 1H), 6.38 (s, 1H), 5.92 (s, 1H), 2.51-2.43 (m, 2H, teilweise überdeckt vom DMSO-Signal), 2.37-2.27 (m, 2H), 2.28 (s, 3H), 2.03-1.92 (m, 1H), 1.80-1.69 (m, 1H).

LC/MS (Methode 3, ESIpos): Rₜ = 0.88 min, m/z = 489 [M+H]⁺.

### Beispiel 93

### 2-tert.-Butyl-6-cyano-N-{4-methyl-3-[6-(pyridin-3-yl)-1H-imidazo[1,2-b]pyrazol-1-yl]phenyl}-isonicotinamid

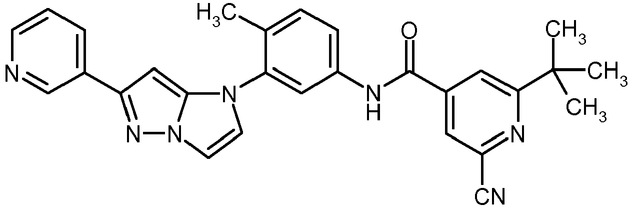

Eine Mischung aus 137 mg (0.28 mmol) der Verbindung aus Beispiel 65, 4.04 mg (0.012 mmol) Palladium(II)trifluoracetat, 9.91 mg (0.025 mmol) racemischem 2-Di-*tert*.-butylphosphino-1,1'-bi-naphthyl, 3.51 mg (0.054 mmol) Zink-Flocken und 18.6 mg (0.16 mmol) Zinkcyanid in 1.5 ml *N,N-*Dimethylacetamid wurde unter Argon 20 Stunden bei 95°C gerührt. Nach dem Abkühlen wurde mit 80 ml Ethylacetat verdünnt und das Gemisch je einmal mit 10 ml Wasser und 10 ml gesättigter Natriumchlorid-Lösung gewaschen. Die organische Phase wurde über Natriumsulfat getrocknet und nach Filtration im Vakuum eingeengt. Der so erhaltene Rückstand wurde mittels Chromatographie an einer Biotage-Anlage gereinigt (25 g Snap-Säule; Laufmittelgradient Ethylacetat/ Hexan, beginnend mit 70% Ethylacetat, dann schnell auf 100% Ethylacetat ansteigend). Eine weitere Reinigung erfolgte mittels präparativer Dickschicht-Chromatographie (2 mm Schichtdicke mit Konzentrierungszone, Laufmittel Ethylacetat, Elution mit Methylenchlorid/Methanol 7:3). Es wurden so 56 mg (38% d. Th.) der Titelverbindung erhalten.

¹H-NMR (400 MHz, DMSO-d₆, δ/ppm): 10.75 (s, 1H), 9.01 (d, 1H), 8.45 (dd, 1H), 8.29 (d, 1H), 8.11-8.19 (m, 2H), 7.90 (dd, 2H), 7.75 (dd, 1H), 7.51 (d, 1H), 7.46 (d, 1H), 7.38 (dd, 1H), 6.32 (s, 1H), 2.25 (s, 3H), 1.34 (s, 9H).

LC/MS (Methode 7, ESIpos): Rₜ = 1.21 min, m/z = 476 [M+H]⁺.

### Beispiel 94

### 3-Brom-5-(pentafluor-λ⁶-sulfanyl)-N-{3-[6-(pyridin-3-yl)-1H-imidazo[1,2-b]pyrazol-1-yl]-4-(trifluormethyl)phenyl} benzamid

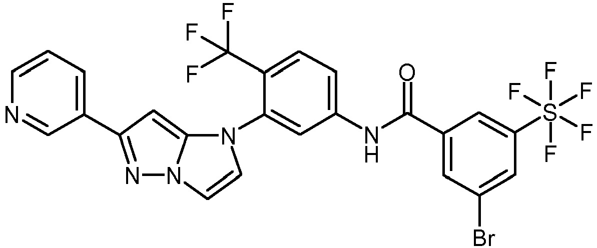

In Analogie zu Beispiel 90 wurden aus 660 mg (1.92 mmol) der Verbindung aus Beispiel 63A und 692 mg (2.12 mmol) der Verbindung aus Beispiel 15A 253 mg (70% Reinheit, 14% d. Th.) der Titelverbindung erhalten. Die Reaktionszeit betrug in diesem Fall insgesamt 80 h bei einer Temperatur von 60°C, wobei zwischenzeitlich erneut HATU und DMAP zugegeben wurden. Die Reinigung des Rohprodukts erfolgte mittels zweimaliger Chromatographie an einer Biotage-Anlage (25 g Snap-Säule; Laufmittelgradient zunächst Ethylacetat/Hexan mit schnell ansteigendem Ethylacetat-Anteil bis 100%, danach Ethylacetat/Methanol, von 0-20% Methanol langsam ansteigend).

LC/MS (Methode 7, ESIpos): Rₜ = 1.36 min, m/z = 652/654 [M+H]⁺.

### Beispiel 95

### 3-Cyano-5-(pentafluor-λ⁶-sulfanyl)-N-{3-[6-(pyridin-3-yl)-1H-imidazo[1,2-b]pyrazol-1-yl]-4-(trifluormethyl)phenyl} benzamid

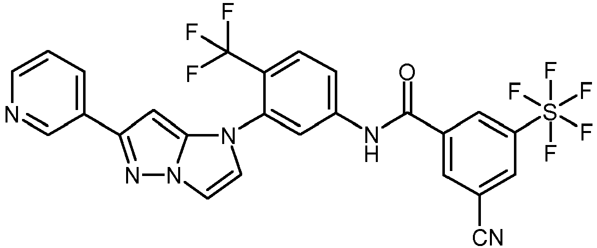

In Analogie zu Beispiel 79 wurden aus 253 mg (0.39 mmol) der Verbindung aus Beispiel 94 27 mg (11% d. Th.) der Titelverbindung erhalten. Die Reinigung des Rohprodukts erfolgte hier mittels präparativer HPLC (Methode 16).

¹H-NMR (400 MHz, DMSO-d₆, δ/ppm): 10.83 (s, 1H), 9.08 (d, 1H), 8.87 (t, 1H), 8.69 (s, 2H), 8.50 (dd, 1H), 8.20 (dt, 1H), 8.09 (d, 1H), 8.05 (d, 1H), 7.89-7.97 (m, 3H), 7.44 (ddd, 1H), 7.05 (s, 1H).

LC/MS (Methode 6, ESIpos): Rₜ = 1.23 min, m/z = 599 [M+H]⁺.

### Beispiel 96

### N-{4-Chlor-3-[6-(pyridin-3-yl)-1H-imidazo[1,2-b]pyrazol-1-yl]phenyl}-3-(pentafluor-λ⁶-sulfanyl)-benzamid

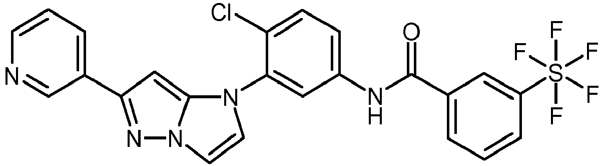

130 mg (0.420 mmol) der Verbindung aus Beispiel 64A und 104 mg (0.420 mmol) 3-(Pentafluor-λ⁶-sulfanyl)benzoesäure wurden in 2.5 ml wasserfreiem DMF gelöst und nacheinander mit 191 mg (0.504 mmol) HATU und 88 µl (0.504 mmol) *N*,*N*-Diisopropylethylamin versetzt. Nachdem das Reaktionsgemisch 15 h bei RT gerührt worden war, wurde es mit einigen ml Methanol verdünnt und anschließend komplett mittels präparativer HPLC (Methode 9) in seine Komponenten aufgetrennt. Die Produktfraktionen wurden vereinigt und am Rotationsverdampfer zur Trockene eingeengt. Hierdurch wurde die Titelverbindung in Form ihres Ameisensäure-Salzes erhalten. Um das Produkt in die salzfreie Form zu überführen, wurde gesättigte wässrige Natriumhydrogencarbonat-Lösung zugesetzt und mit Ethylacetat extrahiert. Der organische Extrakt wurde über wasserfreiem Magnesiumsulfat getrocknet, filtriert und am Rotationsverdampfer vom Lösungsmittel befreit. Der so erhaltene Rückstand wurde abschließend mit 3 ml Pentan, dem einige Tropfen Diisopropylether zugesetzt waren, verrührt. Nach Absaugen und Trocknen des Feststoffs im Hochvakuum wurden 53 mg (22% d. Th., 95% Reinheit) der Titelverbindung erhalten.

¹H-NMR (400 MHz, CDCl₃, δ/ppm): 9.10 (s, breit, 1H), 9.02 (s, 1H), 8.49 (d, 1H), 8.35 (s, 1H), 8.12-8.04 (m, 3H), 7.94 (d, 1H), 7.71 (d, 1H), 7.64-7.55 (m, 2H), 7.48 (s, 1H), 7.32 (dd, 1H), 7.17 (d, 1H), 6.09 (s, 1H).

LC/MS (Methode 3, ESIpos): Rₜ = 1.07 min, m/z = 540/542 [M+H]⁺.

### Beispiel 97

### N-{4-Chlor-3-[6-(pyridin-3-yl)-1H-imidazo[1,2-b]pyrazol-1-yl]phenyl}-3-cyano-5-(pentafluor-λ⁶-sulfanyl)benzamid

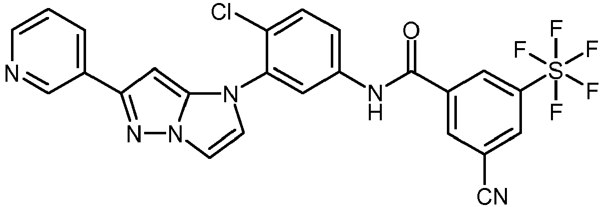

Analog zu dem unter Beispiel 96 beschriebenen Verfahren wurden aus 130 mg (0.420 mmol) der Verbindung aus Beispiel 64A und 135 mg (0.420 mmol, 85% Reinheit) der Verbindung aus Beispiel 23A 49 mg (19% d. Th., 95% Reinheit) der Titelverbindung erhalten.

¹H-NMR (400 MHz, CDCl₃, δ/ppm): 9.95 (s, breit, 1H), 9.02 (d, 1H), 8.67 (s, 1H), 8.53 (s, 1H), 8.51 (dd, 1H), 8.18 (s, 1H), 8.11 (d, 1H), 8.07 (d, 1H), 7.80 (dd, 1H), 7.58 (d, 1H), 7.50 (d, 1H), 7.34 (dd, 1H), 7.19 (d, 1H), 6.11 (s, 1H).

LC/MS (Methode 3, ESIpos): Rₜ = 1.07 min, m/z = 565/567 [M+H]⁺.

### Beispiel 98

### 3-Brom-N-{4-methoxy-3-[6-(pyridin-3-yl)-1H-imidazo[1,2-b]pyrazol-1-yl]phenyl}-5-(pentafluor-λ⁶-sulfanyl)benzamid

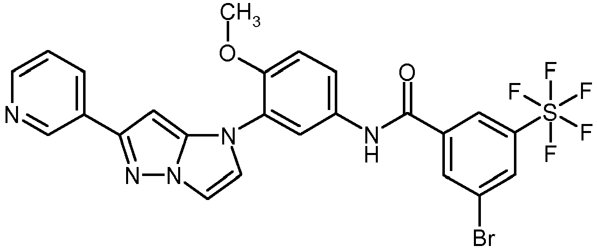

In Analogie zu Beispiel 50 wurden aus 400 mg (1.31 mmol) der Verbindung aus Beispiel 10A und 428 mg (1.31 mmol) der Verbindung aus Beispiel 15A nach einmaliger chromatographischer Reinigung an einer Biotage-Anlage 701 mg (78% d. Th.) der Titelverbindung erhalten.

¹H-NMR (400 MHz, DMSO-d₆, δ/ppm): 10.61 (s, 1H), 9.02 (d, 1H), 8.47 (s, 1H), 8.46 (dd, 1H), 8.36-8.40 (m, 2H), 8.15 (dt, 1H), 7.98 (d, 1H), 7.84 (d, 1H), 7.74 (dd, 1H), 7.52 (d, 1H), 7.39 (ddd, 1H), 7.31 (d, 1H), 6.38 (s, 1H), 3.87 (s, 3H).

LC/MS (Methode 7, ESIpos): Rₜ = 1.27 min, m/z = 614/616 [M+H]⁺.

### Beispiel 99

### 3-Cyano-N-{4-methoxy-3-[6-(pyridin-3-yl)-1H-imidazo[1,2-b]pyrazol-1-yl]phenyl}-5-(pentafluor-λ⁶-sulfanyl)benzamid

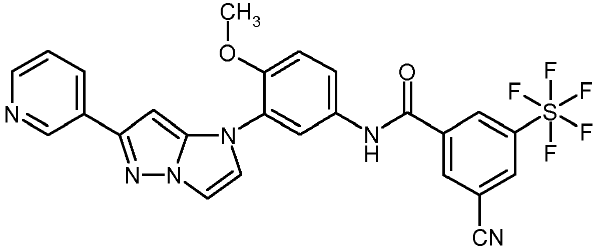

In Analogie zu Beispiel 79 wurden aus 700 mg (1.14 mmol) der Verbindung aus Beispiel 98 nach zweimaliger chromatographischer Reinigung an einer Biotage-Anlage und abschließender präparativer HPLC (Methode 16) 75 mg (11% d. Th.) der Titelverbindung erhalten.

¹H-NMR (400 MHz, DMSO-d₆, δ/ppm): 10.68 (s, 1H), 9.02 (d, 1H), 8.80 (dd, 1H), 8.71 (s, 1H), 8.61-8.66 (m, 1H), 8.47 (dd, 1H), 8.17 (dt, 1H), 7.99 (d, 1H), 7.85 (d, 1H), 7.73 (dd, 1H), 7.53 (d, 1H), 7.41 (dd, 1H), 7.32 (d, 1H), 6.38 (s, 1H), 3.87 (s, 3H).

LC/MS (Methode 7, ESIpos): Rₜ = 1.24 min, m/z = 561 [M+H]⁺.

### Beispiel 100

### N-{3,4-Dimethyl-5-[6-(pyridin-3-yl)-1H-imidazo[1,2-b]pyrazol-1-yl]phenyl}-3-(pentafluor-λ⁶-sulfanyl)benzamid

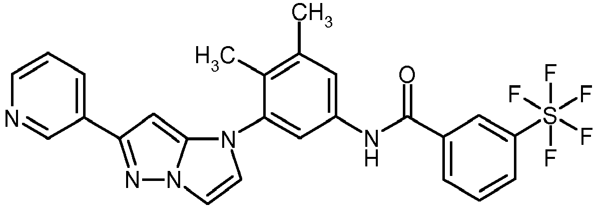

In Analogie zu Beispiel 90 wurden aus 150 mg (0.49 mmol) der Verbindung aus Beispiel 65A und 134 mg (0.54 mmol) 3-(Pentafluor-λ⁶-sulfanyl)benzoesäure 178 mg (66% d. Th.) der Titelverbindung erhalten.

¹H-NMR (400 MHz, DMSO-d₆, δ/ppm): 10.58 (s, 1H), 9.02 (s, 1H), 8.45 (d, 1H), 8.38 (t, 1H), 8.23 (d, 1H), 8.17 (dt, 1H), 8.12 (dd, 1H), 7.88 (d, 1H), 7.77 (dd, 2H), 7.68 (d, 1H), 7.46 (d, 1H), 7.39 (dd, 1H), 6.29 (s, 1H), 2.34 (s, 3H), 2.09 (s, 3H).

LC/MS (Methode 7, ESIpos): Rₜ = 1.21 min, m/z = 534 [M+H]⁺.

### Beispiel 101

### 3-Brom-N-{3,4-dimethyl-5-[6-(pyridin-3-yl)-1H-imidazo[1,2-b]pyrazol-1-yl]phenyl}-5-(pentafluor-λ⁶-sulfanyl)benzamid

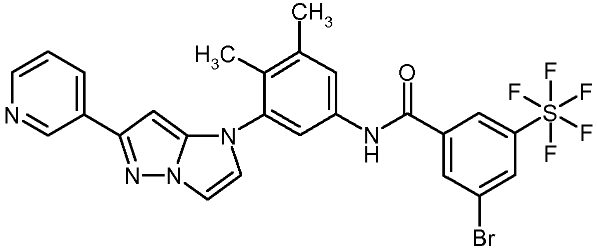

In Analogie zu Beispiel 90 wurde aus 370 mg (1.22 mmol) der Verbindung aus Beispiel 65A und 439 mg (1.34 mmol) der Verbindung aus Beispiel 15A ein Rohprodukt erhalten, welches, in Abweichung zu Beispiel 90, durch Chromatographie an einer Biotage-Anlage (25 g Snap-Säule; Laufmittelgradient Hexan/Ethylacetat, von 70% Ethylacetat bis 100% Ethylacetat stetig ansteigend) gereinigt wurde. Es wurden 977 mg (>100% d. Th.) der Titelverbindung erhalten, die nicht weiter aufgereinigt wurden.

LC/MS (Methode 7, ESIpos): Rₜ = 1.36 min, m/z = 612/614 [M+H]⁺.

### Beispiel 102

### 3-Cyano-N-{3,4-dimethyl-5-[6-(pyridin-3-yl)-1H-imidazo[1,2-b]pyrazol-1-yl]phenyl}-5-(pentafluor-λ⁶-sulfanyl)benzamid

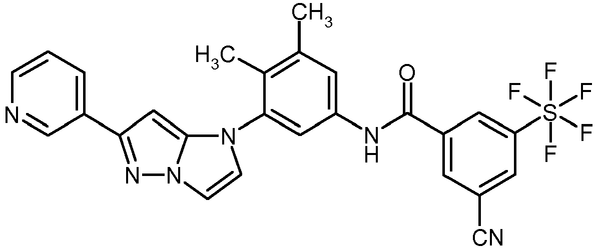

In Analogie zu Beispiel 79 wurden aus 970 mg (1.58 mmol) der Verbindung aus Beispiel 101 nach Reinigung mittels HPLC (Methode 16) 221 mg (24% d. Th.) der Titelverbindung erhalten.

¹H-NMR (400 MHz, DMSO-d₆, δ/ppm): 10.68 (s, 1H), 9.01 (d, 1H), 8.81 (d, 1H), 8.70 (s, 1H), 8.63 (d, 1H), 8.44 (dd, 1H), 8.14 (dt, 1H), 7.88 (d, 1H), 7.76 (d, 1H), 7.66 (d, 1H), 7.45 (d, 1H), 7.38 (ddd, 1H), 6.27 (s, 1H), 2.35 (s, 3H), 2.10 (s, 3H).

LC/MS (Methode 7, ESIpos): Rₜ = 1.20 min, m/z = 559 [M+H]⁺.

### Beispiel 103

### N-{3-Fluor-4-methyl-5-[6-(pyridin-3-yl)-1H-imidazo[1,2-b]pyrazol-1-yl]phenyl}-3-(pentafluor-λ⁶-sulfanyl)benzamid

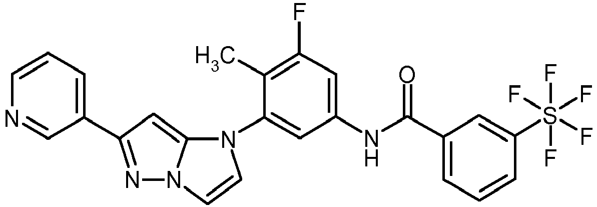

In Analogie zu Beispiel 90 wurden aus 150 mg (0.49 mmol) der Verbindung aus Beispiel 66A und 133 mg (0.54 mmol) 3-(Pentafluor-λ⁶-sulfanyl)benzoesäure 151 mg (58% d. Th.) der Titelverbindung erhalten.

¹H-NMR (400 MHz, DMSO-d₆, δ/ppm): 10.78 (s, 1H), 9.02 (d, 1H), 8.45 (dd, 1H), 8.37 (t, 1H), 8.24 (d, 1H), 8.11-8.18 (m, 2H), 7.92 (d, 1H), 7.71-7.85 (m, 3H), 7.55 (d, 1H), 7.39 (dd, 1H), 6.41 (s, 1H), 2.17 (d, 3H).

LC/MS (Methode 7, ESIpos): Rₜ = 1.24 min, m/z = 538 [M+H]⁺.

### Beispiel 104

### 3-Brom-N-{3-fluor-4-methyl-5-[6-(pyridin-3-yl)-1H-imidazo[1,2-b]pyrazol-1-yl]phenyl}-5-(pentafluor-λ⁶-sulfanyl)benzamid

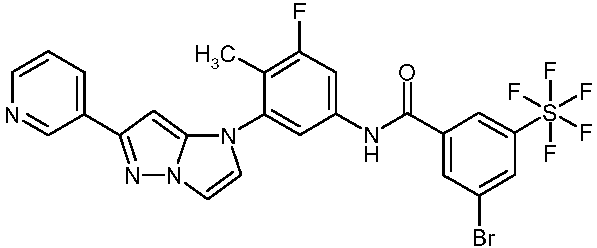

In Analogie zu Beispiel 90 wurde aus 400 mg (1.30 mmol) der Verbindung aus Beispiel 66A und 468 mg (1.43 mmol) der Verbindung aus Beispiel 15A ein Rohprodukt erhalten, welches durch Chromatographie an einer Biotage-Anlage (25 g Snap-Säule; Laufmittelgradient Hexan/Ethylacetat, von 70% Ethylacetat bis 100% Ethylacetat stetig ansteigend) gereinigt wurde. Es wurden 772 mg (94% d. Th.) der Titelverbindung erhalten.

¹H-NMR (400 MHz, DMSO-d₆, δ/ppm): 10.80 (s, 1H), 9.02 (d, 1H), 8.43-8.48 (m, 2H), 8.40 (t, 1H), 8.36 (t, 1H), 8.15 (dt, 1H), 7.92 (d, 2H), 7.79 (dd, 1H), 7.71 (s, 1H), 7.54 (d, 1H), 7.39 (ddd, 1H), 6.40 (d, 1H), 2.17 (d, 3H).

LC/MS (Methode 7, ESIpos): Rₜ = 1.38 min, m/z = 616/618 [M+H]⁺.

### Beispiel 105

### 3-Cyano-N-{3-fluor-4-methyl-5-[6-(pyridin-3-yl)-1H-imidazo[1,2-b]pyrazol-1-yl]phenyl}-5-(pentafluor-λ⁶-sulfanyl)benzamid

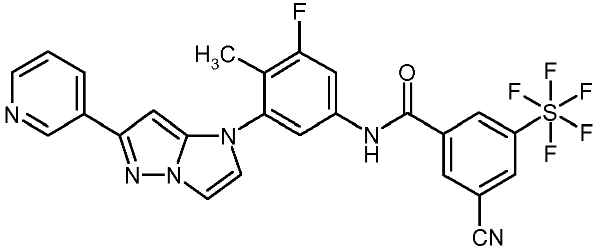

In Analogie zu Beispiel 79 wurden aus 755 mg (1.23 mmol) der Verbindung aus Beispiel 104 nach Reinigung mittels HPLC (Methode 16) 287 mg (41% d. Th.) der Titelverbindung erhalten.

¹H-NMR (400 MHz, DMSO-d₆, δ/ppm): 10.87 (s, 1H), 9.02 (dd, 1H), 8.83 (dd, 1H), 8.70 (s, 1H), 8.62 (dd, 1H), 8.46 (dd, 1H), 8.15 (dt, 1H), 7.92 (dd, 1H), 7.78 (dd, 1H), 7.71 (s, 1H), 7.55 (d, 1H), 7.39 (ddd, 1H), 6.39 (s, 1H), 2.17 (d, 3H).

LC/MS (Methode 5, ESIpos): Rₜ = 1.22 min, m/z = 563 [M+H]⁺.

### Beispiel 106

### N-{3-Chlor-4-methyl-5-[6-(pyridin-3-yl)-1H-imidazo[1,2-b]pyrazol-1-yl]phenyl}-3-(pentafluor-λ⁶-sulfanyl)benzamid

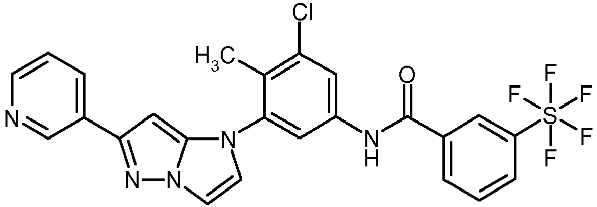

In Analogie zu Beispiel 90 wurden aus 240 mg (0.74 mmol) der Verbindung aus Beispiel 67A und 202 mg (0.82 mmol) 3-(Pentafluor-λ⁶-sulfanyl)benzoesäure 242 mg (59% d. Th.) der Titelverbindung erhalten.

¹H-NMR (400 MHz, DMSO-d₆, δ/ppm): 10.75 (s, 1H), 9.02 (d, 1H), 8.45 (dd, 1H), 8.39 (t, 1H), 8.24 (d, 1H), 8.11-8.18 (m, 2H), 8.06 (d, 1H), 7.92 (d, 1H), 7.88 (d, 1H), 7.79 (t, 1H), 7.54 (d, 1H), 7.38 (ddd, 1H), 6.39 (s, 1H), 2.26 (s, 3H).

LC/MS (Methode 7, ESIpos): Rₜ = 1.30 min, m/z = 554/556 [M+H]⁺.

### Beispiel 107

### N-{2,4-Dimethyl-5-[6-(pyridin-3-yl)-1H-imidazo[1,2-b]pyrazol-1-yl]phenyl}-3-fluor-5-(pentafluor-λ⁶-sulfanyl)benzamid

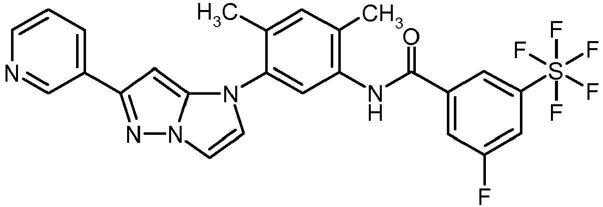

Analog zu dem unter Beispiel 26 beschriebenen Verfahren wurden aus 100 mg (0.330 mmol) der Verbindung aus Beispiel 11A und 88 mg (0.330 mmol) 3-Fluor-5-(pentafluor-λ⁶-sulfanyl)benzoesäure (JRD Fluorochemicals Ltd., Großbritannien) 103 mg (56% d. Th.) der Titelverbindung erhalten. Die Reaktionszeit betrug in diesem Fall 1 h. Auf die abschließende Neutralisation mittels einer Hydrogencarbonat-Kartusche konnte hier verzichtet werden, da das Produkt nach der HPLC-Reinigung bereits als freie Base angefallen war.

¹H-NMR (400 MHz, DMSO-d₆, δ/ppm): 10.36 (s, 1H), 9.05 (d, 1H), 8.49 (dd, 1H), 8.30 (s, 1H), 8.25 (dt, 1H), 8.22-8.15 (m, 2H), 7.90 (d, 1H), 7.50-7.49 (m, 2H), 7.44 (dd, 1H), 7.40 (s, 1H), 6.32 (s, 1H), 2.30 (s, 3H), 2.26 (s, 3H).

LC/MS (Methode 3, ESIpos): Rₜ = 1.06 min, m/z = 552 [M+H]⁺.

### Beispiel 108

### 3-Chlor-N-{2,4-dimethyl-5-[6-(pyridin-3-yl)-1H-imidazo[1,2-b]pyrazol-1-yl]phenyl}-5-(pentafluor-λ⁶-sulfanyl)benzamid

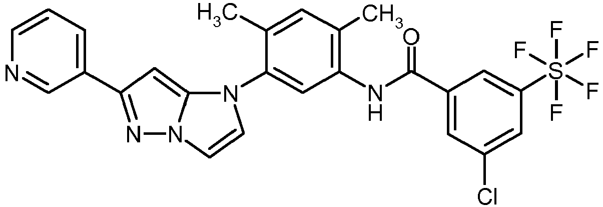

Analog zu dem unter Beispiel 107 beschriebenen Verfahren wurden aus 90 mg (0.297 mmol) der Verbindung aus Beispiel 11A und 84 mg (0.297 mmol) der Verbindung aus Beispiel 33A 119 mg (70% d. Th.) der Titelverbindung erhalten.

¹H-NMR (400 MHz, DMSO-d₆, δ/ppm): 10.38 (s, 1H), 9.05 (d, 1H), 8.49 (dd, 1H), 8.38-8.35 (m, 3H), 8.19 (dt, 1H), 7.90 (d, 1H), 7.51-7.49 (m, 2H), 7.43 (dd, 1H), 7.40 (s, 1H), 6.31 (s, 1H), 2.30 (s, 3H), 2.26 (s, 3H).

LC/MS (Methode 3, ESIpos): Rₜ = 1.10 min, m/z = 568/570 [M+H]⁺.

### Beispiel 109

### 3-Brom-N-{2,4-dimethyl-5-[6-(pyridin-3-yl)-1H-imidazo[1,2-b]pyrazol-1-yl]phenyl}-5-(pentafluor-λ⁶-sulfanyl)benzamid

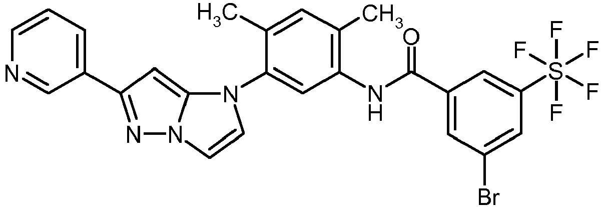

Analog zu dem unter Beispiel 89 beschriebenen Verfahren wurden aus 1.75 g (5.77 mmol) der Verbindung aus Beispiel 11A und 1.89 g (5.77 mmol) der Verbindung aus Beispiel 15A 3.02 g (85% d. Th.) der Titelverbindung erhalten. Das nach der chromatographischen Reinigung erhaltene Produkt wurde hier abschließend noch mit Pentan/Dichlormethan 10:1 verrührt.

¹H-NMR (400 MHz, DMSO-d₆, δ/ppm): 10.38 (s, 1H), 9.04 (s, 1H), 8.50-8.47 (m, 2H), 8.44 (s, 1H), 8.41 (s, 1H), 8.17 (d, 1H), 7.90 (d, 1H), 7.51-7.48 (m, 2H), 7.41 (dd, 1H), 7.39 (s, 1H), 6.31 (s, 1H), 2.30 (s, 3H), 2.26 (s, 3H).

LC/MS (Methode 3, ESIpos): Rₜ = 1.14 min, m/z = 612/614 [M+H]⁺.

### Beispiel 110

### 3-({2,4-Dimethyl-5-[6-(pyridin-3-yl)-1H-imidazo[1,2-b]pyrazol-1-yl]phenyl}carbamoyl)-5-(pentafluor-λ⁶-sulfanyl)benzoesäure

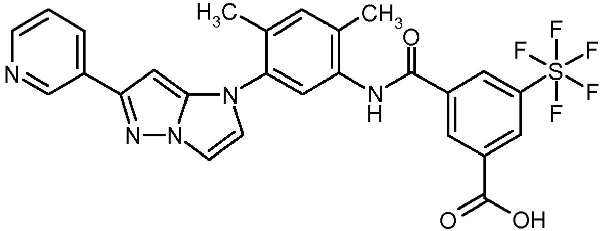

100 mg (0.142 mmol, 80% Reinheit) der Verbindung aus Beispiel 77A wurden in 2 ml DMSO gelöst und bei RT mit einer Lösung von 48 mg (0.350 mmol) Natriumdihydrogenphosphat-Hydrat in 0.7 ml Wasser versetzt. Dann wurde eine Lösung von 39 mg (0.342 mmol) Natriumchlorit in 0.3 ml Wasser zugetropft. Nachdem das Reaktionsgemisch 24 h bei RT gerührt worden war, wurde es mit 100 ml Wasser verdünnt und dreimal mit je ca. 100 ml Ethylacetat extrahiert. Die vereinigten organischen Extrakte wurden mit gesättigter Natriumchlorid-Lösung gewaschen, über wasserfreiem Magnesiumsulfat getrocknet, filtriert und am Rotationsverdampfer vom Lösungsmittel befreit. Das so erhaltene Rohprodukt wurde mittels präparativer HPLC gereinigt (Methode 32). Nach Eindampfen der Produktfraktionen wurde der Rückstand in einem Gemisch aus 2 ml Pentan, 0.5 ml Dichlormethan und 0.5 ml Diisopropylether 10 min lang verrührt. Nach Absaugen und Trocknen des Feststoffs im Hochvakuum wurden 43 mg (52% d. Th.) der Titelverbindung erhalten.

¹H-NMR (400 MHz, DMSO-d₆, δ/ppm): 14.04 (breit, 1H), 10.53 (s, 1H), 9.04 (s, 1H), 8.82 (s, 1H), 8.66 (s, 1H), 8.49-8.46 (m, 2H), 8.18 (dt, 1H), 7.90 (d, 1H), 7.50-7.49 (m, 2H), 7.41 (dd, 1H), 7.40 (s, 1H), 6.32 (s, 1H), 2.30 (s, 3H), 2.26 (s, 3H).

LC/MS (Methode 3, ESIpos): Rₜ = 1.01 min, m/z = 578 [M+H]⁺.

### Beispiel 111

### N-{2,4-Dimethyl-5-[6-(pyridin-3-yl)-1H-imidazo[1,2-b]pyrazol-1-yl]phenyl}-3-methyl-5-(pentafluor-λ⁶-sulfanyl)benzamid

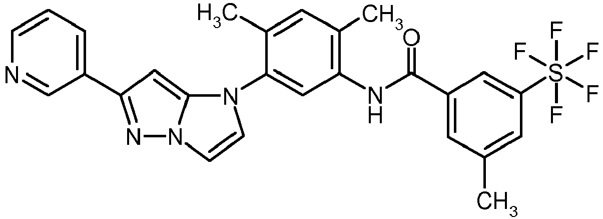

Analog zu dem unter Beispiel 107 beschriebenen Verfahren wurden aus 100 mg (0.330 mmol) der Verbindung aus Beispiel 11A und 86 mg (0.330 mmol) der Verbindung aus Beispiel 34A 105 mg (58% d. Th.) der Titelverbindung erhalten.

¹H-NMR (400 MHz, DMSO-d₆, δ/ppm): 10.27 (s, 1H), 9.05 (d, 1H), 8.49 (dd, 1H), 8.22 (s, 1H), 8.20 (dt, 1H), 8.11 (s, 1H), 8.00 (s, 1H), 7.90 (d, 1H), 7.49 (d, 1H), 7.48 (s, 1H), 7.43 (dd, 1H), 7.39 (s, 1H), 6.32 (s, 1H), 2.29 (s, 3H), 2.26 (s, 3H).

LC/MS (Methode 3, ESIpos): Rₜ = 1.17 min, m/z = 548 [M+H]⁺.

### Beispiel 112

### N-{2,4-Dimethyl-5-[6-(pyridin-3-yl)-1H-imidazo[1,2-b]pyrazol-1-yl]phenyl}-3-(methoxymethyl)-5-(pentafluor-λ⁶-sulfanyl)benzamid

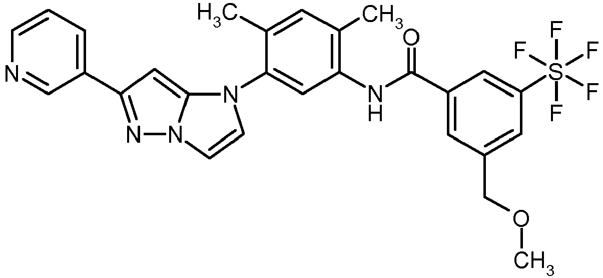

Eine Lösung von 51 mg (0.173 mmol) der Verbindung aus Beispiel 74A in 1 ml wasserfreiem DMF wurde nacheinander mit 75 mg (0.198 mmol) HATU, 24 mg (0.198 mmol) 4-*N*,*N*-Dimethylaminopyridin (DMAP) und 50 mg (0.165 mmol) der Verbindung aus Beispiel 11A versetzt. Nachdem das Reaktionsgemisch 1 h bei RT gerührt worden war, wurde es mit ca. 2 ml Methanol verdünnt und dann direkt mittels präparativer HPLC (Methode 32) in seine Komponenten aufgetrennt. Nach Eindampfen der Produktfraktionen und Trocknen des Rückstands im Hochvakuum wurden 60 mg (63% d. Th.) der Titelverbindung erhalten.

¹H-NMR (400 MHz, DMSO-d₆, δ/ppm): 10.36 (s, 1H), 9.06 (s, 1H), 8.50 (d, 1H), 8.36 (s, 1H), 8.24 (d, 1H), 8.22 (d, 1H), 8.06 (s, 1H), 7.90 (s, 1H), 7.50-7.48 (m, 2H), 7.45 (dd, 1H), 7.39 (s, 1H), 6.33 (s, 1H), 4.62 (s, 2H), 3.38 (s, 3H), 2.30 (s, 3H), 2.26 (s, 3H).

LC/MS (Methode 3, ESIpos): Rₜ = 1.06 min, m/z = 578 [M+H]⁺.

### Beispiel 113

### 3-Brom-5-tert.-butyl-N-{2,4-dimethyl-5-[6-(pyridin-3-yl)-1H-imidazo[1,2-b]pyrazol-1-yl]phenyl}-benzamid

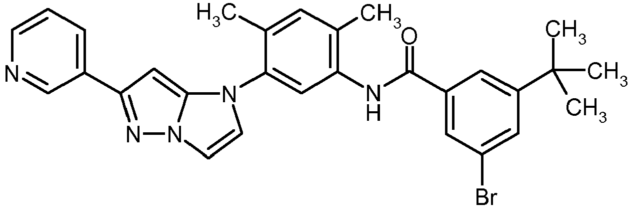

250 mg (0.82 mmol) der Verbindung aus Beispiel 11A und 212 mg (0.82 mmol) 3-Brom-5-*tert*.-butylbenzoesäure wurden in Analogie zu Beispiel 77 umgesetzt und aufgearbeitet. Das so erhaltene Rohprodukt wurde durch einmalige Chromatographie an einer Biotage-Anlage gereinigt (25 g Snap-Säule; Laufmittelgradient Ethylacetat/Hexan, beginnend mit 70% Ethylacetat, dann schnell auf 100% Ethylacetat ansteigend). Auf diese Weise wurden 352 mg (71% d. Th.) der Titelverbindung erhalten.

¹H-NMR (400 MHz, DMSO-d₆, δ/ppm): 10.05 (s, 1H), 9.00 (d, 1H), 8.44 (dd, 1H), 8.14 (dt, 1H), 7.91-7.97 (m, 2H), 7.85 (d, 1H), 7.74 (t, 1H), 7.28-7.46 (m, 4H), 6.26 (s, 1H), 2.25 (s, 3H), 2.21 (s, 3H), 1.29 (s, 9H).

LC/MS (Methode 7, ESIpos): Rₜ = 1.36 min, m/z = 542/544 [M+H]⁺.

### Beispiel 114

### 3-tert.-Butyl-5-cyano-N-{2,4-dimethyl-5-[6-(pyridin-3-yl)-1H-imidazo[1,2-b]pyrazol-1-yl]phenyl}-benzamid

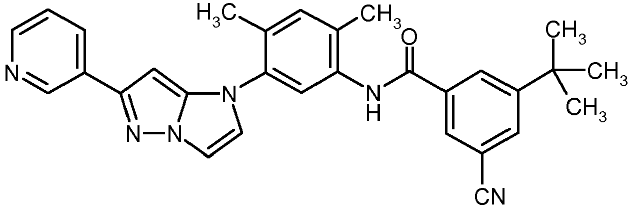

In Analogie zu Beispiel 79 wurde aus 347 mg (0.64 mmol) der Verbindung aus Beispiel 113 ein Rohprodukt erhalten, welches durch präparative HLPC (Methode 16) gereinigt wurde. Es wurden 82 mg (26% d. Th.) der Titelverbindung erhalten.

¹H-NMR (400 MHz, DMSO-d₆, δ/ppm): 10.11 (s, 1H), 9.03 (d, 1H), 8.48 (dd, 1H), 8.18-8.24 (m, 3H), 8.06 (t, 1H), 7.86 (d, 1H), 7.42-7.49 (m, 3H), 7.35 (s, 1H), 6.29 (s, 1H), 2.28 (s, 3H), 2.22 (s, 3H), 1.31 (s, 9H).

LC/MS (Methode 7, ESIpos): Rₜ = 1.20 min, m/z = 489 [M+H]⁺.

### Beispiel 115

### 2-tert.-Butyl-6-chlor-N-{2,4-dimethyl-5-[6-(pyridin-3-yl)-1H-imidazo[1,2-b]pyrazol-1-yl]phenyl}-isonicotinamid

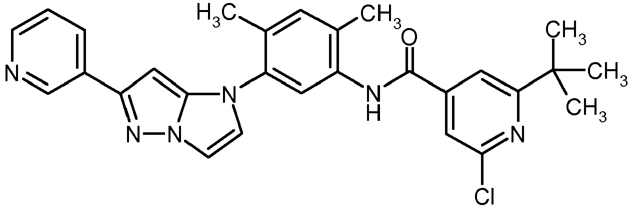

250 mg (0.82 mmol) der Verbindung aus Beispiel 11A und 176 mg (0.82 mmol) 2-*tert.*-Butyl-6-chlor-4-pyridincarbonsäure wurden in Analogie zu Beispiel 77 umgesetzt und aufgearbeitet. Das so erhaltene Rohprodukt wurde durch einmalige Chromatographie an einer Biotage-Anlage gereinigt (25 g Snap-Säule; Laufmittelgradient Ethylacetat/Hexan, beginnend mit 70% Ethylacetat, dann schnell auf 100% Ethylacetat ansteigend). Auf diese Weise wurden 341 mg (71% d. Th.) der Titelverbindung erhalten.

¹H-NMR (400 MHz, DMSO-d₆, δ/ppm): 10.27 (s, 1H), 9.00 (d, 1H), 8.44 (dd, 1H), 8.13 (dt, 1H), 7.81-7.87 (m, 2H), 7.77 (s, 1H), 7.42-7.48 (m, 2H), 7.32-7.41 (m, 2H), 6.26 (s, 1H), 2.26 (s, 3H), 2.22 (s, 3H), 1.31 (s, 9H).

LC/MS (Methode 7, ESIpos): Rₜ = 1.28 min, m/z = 499 [M+H]⁺.

### Beispiel 116

### 2-tert.-Butyl-6-cyano-N-{2,4-dimethyl-5-[6-(pyridin-3-yl)-1H-imidazo[1,2-b]pyrazol-1-yl]phenyl}-isonicotinamid

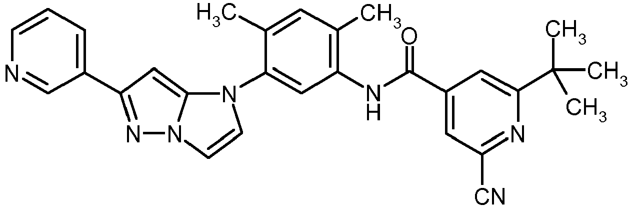

In Analogie zu Beispiel 79 wurde aus 337 mg (0.68 mmol) der Verbindung aus Beispiel 115 ein Rohprodukt erhalten, welches durch präparative HLPC (Methode 16) gereinigt wurde. Es wurden 105 mg (32% d. Th.) der Titelverbindung erhalten.

¹H-NMR (400 MHz, DMSO-d₆, δ/ppm): 10.33 (s, 1H), 9.01 (d, 1H), 8.45 (dd, 1H), 8.28 (d, 1H), 8.13-8.19 (m, 2H), 7.86 (d, 1H), 7.49 (s, 1H), 7.45 (d, 1H), 7.40 (dd, 1H), 7.37 (s, 1H), 6.26 (s, 1H), 2.28 (s, 3H), 2.23 (s, 3H), 1.34 (s, 9H).

LC/MS (Methode 7, ESIpos): Rₜ = 1.20 min, m/z = 490 [M+H]⁺.

### Beispiel 117

### 3-Brom-5-tert.-butyl-N-{2-fluor-4-methyl-5-[6-(pyridin-3-yl)-1H-imidazo[1,2-b]pyrazol-1-yl]-phenyl} benzamid

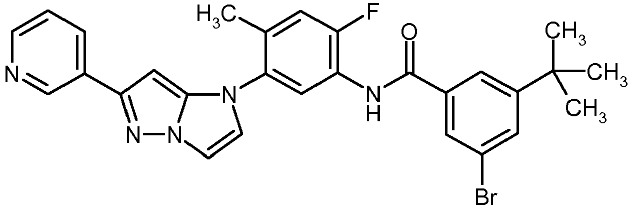

In Analogie zu Beispiel 50 wurden aus 235 mg (0.77 mmol) der Verbindung aus Beispiel 12A und 197 mg (0.77 mmol) 3-Brom-5-*tert*.-butylbenzoesäure nach einmaliger Chromatographie an einer Biotage-Anlage (25 g Snap-Säule; Laufmittelgradient Hexan/70-100% Ethylacetat) 180 mg (66% Reinheit, 29% d. Th.) der Titelverbindung erhalten.

LC/MS (Methode 7, ESIpos): Rₜ = 1.37 min, m/z = 546/548 [M+H]⁺.

### Beispiel 118

### 3-tert.-Butyl-5-cyano-N-{2-fluor-4-methyl-5-[6-(pyridin-3-yl)-1H-imidazo[1,2-b]pyrazol-1-yl]-phenyl} benzamid

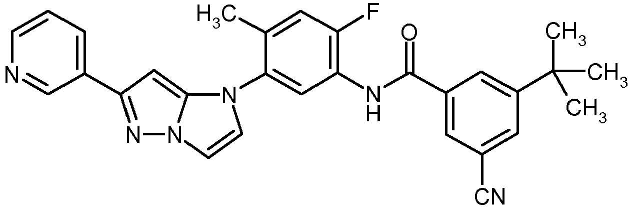

In Analogie zu Beispiel 79 wurden aus 180 mg (0.33 mmol) der Verbindung aus Beispiel 117 nach Reinigung mittels präparativer HPLC (Methode 16) 35 mg (22% d. Th.) der Titelverbindung erhalten.

¹H-NMR (400 MHz, DMSO-d₆, δ/ppm): 10.42 (s, 1H), 9.01 (dd, 1H), 8.44 (dd, 1H), 8.20 (dt, 2H), 8.14 (dt, 1H), 8.07 (t, 1H), 7.87 (dd, 1H), 7.76 (d, 1H), 7.42-7.50 (m, 2H), 7.37 (ddd, 1H), 6.30 (d, 1H), 2.24 (s, 3H), 1.31 (s, 9H).

LC/MS (Methode 7, ESIpos): Rₜ = 1.17 min, m/z = 493 [M+H]⁺.

### Beispiel 119

### N-{2,4-Dimethyl-3-[6-(pyridin-3-yl)-1H-imidazo[1,2-b]pyrazol-1-yl]phenyl}-3-(pentafluor-λ⁶-sulfanyl)benzamid

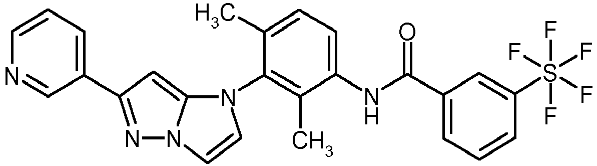

In Analogie zu Beispiel 50 wurde aus 125 mg (0.41 mmol) der Verbindung aus Beispiel 68A und 102 mg (0.41 mmol) 3-(Pentafluor-λ⁶-sulfanyl)benzoesäure ein Rohprodukt erhalten, welches nach dem ersten Lauf auf der Biotage-Anlage noch weiter durch präparative HPLC (Methode 16) gereinigt wurde. Man erhielt auf diese Weise 90 mg (41% d. Th.) der Titelverbindung.

¹H-NMR (400 MHz, DMSO-d₆, δ/ppm): 10.37 (s, 1H), 9.01 (d, 1H), 8.44 (dd, 1H), 8.41 (s, 1H), 8.27 (d, 1H), 8.09-8.19 (m, 2H), 7.89 (d, 1H), 7.78 (t, 1H), 7.43 (d, 1H), 7.29-7.40 (m, 3H), 6.13 (s, 1H), 2.07 (s, 3H), 1.91 (s, 3H).

LC/MS (Methode 7, ESIpos): Rₜ = 1.13 min, m/z = 534 [M+H]⁺.

### Beispiel 120

### 3-Brom-N-{2,4-dimethyl-3-[6-(pyridin-3-yl)-1H-imidazo[1,2-b]pyrazol-1-yl]phenyl}-5-(pentafluor-λ⁶-sulfanyl)benzamid

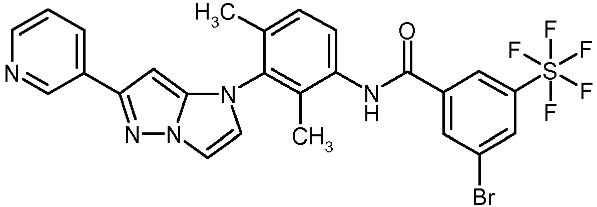

In Analogie zu Beispiel 50 wurde aus 370 mg (1.22 mmol) der Verbindung aus Beispiel 68A und 398 mg (1.22 mmol) der Verbindung aus Beispiel 15A ein Rohprodukt erhalten, welches durch einmalige Chromatographie an einer Biotage-Anlage (25 g Snap-Säule; Laufmittelgradient Hexan/ 70-100% Ethylacetat) gereinigt wurde. Es wurden 573 mg (65% d. Th.) der Titelverbindung erhalten.

¹H-NMR (400 MHz, DMSO-d₆, δ/ppm): 10.41 (s, 1H), 9.00 (dd, 1H), 8.48 (s, 1H), 8.43 (dd, 1H), 8.37-8.41 (m, 2H), 8.14 (dt, 1H), 7.88 (dd, 1H), 7.43 (d, 1H), 7.34-7.40 (m, 2H), 7.32 (d, 1H), 6.12 (s, 1H), 2.65 (s, 6H).

LC/MS (Methode 6, ESIpos): Rₜ = 1.27 min, m/z = 612/614 [M+H]⁺.

### Beispiel 121

### 3-Cyano-N-{2,4-dimethyl-3-[6-(pyridin-3-yl)-1H-imidazo[1,2-b]pyrazol-1-yl]phenyl}-5-(pentafluor-λ⁶-sulfanyl)benzamid

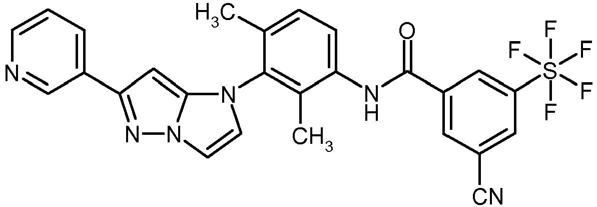

In Analogie zu Beispiel 79 wurden aus zwei getrennten Ansätzen mit 560 mg (0.91 mmol) bzw. 535 mg (0.87 mmol) der Verbindung aus Beispiel 120 nach Reinigung mittels präparativer HPLC (Methode 16) 47 mg (4.8% d. Th.) der Titelverbindung erhalten.

¹H-NMR (400 MHz, DMSO-d₆, δ/ppm): 10.48 (s, 1H), 9.00 (d, 1H), 8.82 (s, 1H), 8.71 (s, 1H), 8.65 (s, 1H), 8.43 (dd, 1H), 8.14 (dt, 1H), 7.89 (d, 1H), 7.45 (d, 1H), 7.35-7.41 (m, 2H), 7.33 (d, 1H), 6.13 (s, 1H), 2.07 (s, 3H), 1.93 (s, 3H).

LC/MS (Methode 6, ESIpos): Rₜ = 1.14 min, m/z = 559 [M+H]⁺.

### Beispiel 122

### N-{4-Methyl-3-[6-(pyridin-3-yl)-1H-imidazo[1,2-b]pyrazol-1-yl]phenyl}-3-sulfamoyl-5-(trifluormethyl)benzamid

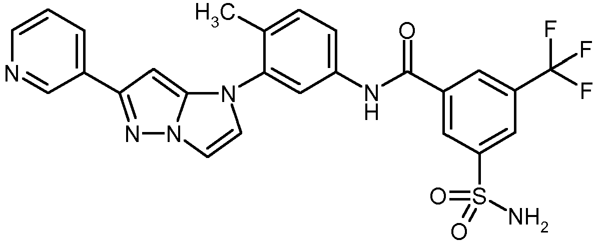

In Analogie zu Beispiel 90 wurden aus 150 mg (0.52 mmol) der Verbindung aus Beispiel 6A und 129 mg (0.47 mmol) 3-Sulfamoyl-5-(trifluormethyl)benzoesäure nach 3 h Rühren bei 50°C und HPLC-Reinigung (Methode 16) 68.5 mg (23% d. Th.) der Titelverbindung erhalten.

¹H-NMR (400 MHz, DMSO-d₆, δ/ppm): 10.83 (s, 1H), 9.08 (d, 1H), 8.63 (s, 1H), 8.51-8.55 (m, 2H), 8.35 (dt, 1H), 8.30 (s, 1H), 7.93 (d, 1H), 7.91 (d, 1H), 7.77 (dd, 1H), 6.42 (s, 1H), 7.69 (s, 2H), 7.53-7.59 (m, 2H), 7.47 (d, 1H), 2.25 (s, 3H).

LC/MS (Methode 7, ESIpos): Rₜ = 1.27 min, m/z = 451 [M+H]⁺.

### Beispiel 123

### 3-tert.-Butyl-N-{4-methyl-3-[6-(pyridin-3-yl)-1H-imidazo[1,2-b]pyrazol-1-yl]phenyl}benzamid

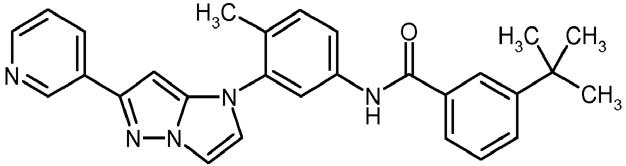

In Analogie zu Beispiel 90 wurden aus 200 mg (0.69 mmol) der Verbindung aus Beispiel 6A und 136 mg (0.76 mmol) 3-*tert*.-Butylbenzoesäure nach 16 h Rühren bei RT und HPLC-Reinigung (Methode 16) 165 mg (52% d. Th.) der Titelverbindung erhalten.

¹H-NMR (400 MHz, DMSO-d₆, δ/ppm): 10.32 (s, 1H), 9.02 (d, 1H), 8.44 (dd, 1H), 8.15 (dt, 1H), 7.94 (d, 1H), 7.89 (t, 1H), 7.87 (dd, 1H), 7.72-7.79 (m, 2H), 7.58-7.62 (m, 1H), 7.50 (d, 1H), 7.35-7.46 (m, 3H), 6.32 (s, 1H), 2.23 (s, 3H), 1.30 (s, 9H).

LC/MS (Methode 7, ESIpos): Rₜ = 1.22 min, m/z = 450 [M+H]⁺.

### Beispiel 124

### 3-Methyl-N-{4-methyl-3-[6-(pyridin-3-yl)-1H-imidazo[1,2-b]pyrazol-1-yl]phenyl}-5-sulfamoyl-benzamid

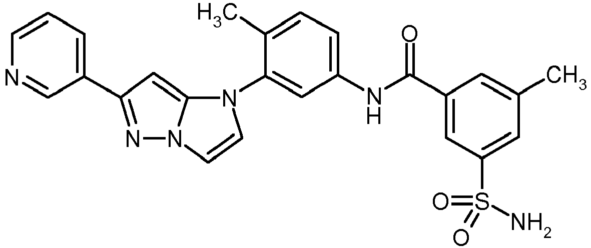

In Analogie zu Beispiel 90 wurden aus 150 mg (0.52 mmol) der Verbindung aus Beispiel 6A und 123 mg (0.57 mmol) 3-Methyl-5-sulfamoylbenzoesäure nach 3 h Rühren bei RT und HPLC-Reinigung (Methode 16) 92.7 mg (35% d. Th.) der Titelverbindung erhalten.

¹H-NMR (400 MHz, DMSO-d₆, δ/ppm): 10.59 (s, 1H), 9.08 (d, 1H), 8.53 (dd, 1H), 8.36 (dt, 1H), 8.15 (s, 1H), 7.97 (s, 1H), 7.95 (d, 1H), 7.91 (d, 1H), 7.82 (s, 1H), 7.77 (dd, 1H), 7.52-7.59 (m, 2H), 7.39-7.47 (m, 3H), 6.42 (s, 1H), 2.24 (s, 3H) [weiteres Signal im Lösungsmittel-Peak verborgen].

LC/MS (Methode 7, ESIpos): Rₜ = 0.85 min, m/z = 487 [M+H]⁺.

### Beispiel 125

### 4-tert.-Butyl-N-{4-methyl-3-[6-(pyridin-3-yl)-1H-imidazo[1,2-b]pyrazol-1-yl]phenyl}pyridin-2-carboxamid

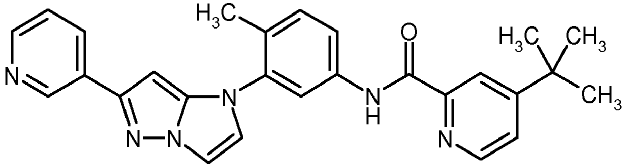

In Analogie zu Beispiel 90 wurden aus 200 mg (0.691 mmol) der Verbindung aus Beispiel 6A und 119 mg (0.63 mmol) 4-*tert*.-Butylpyridin-2-carbonsäure nach 3 h Rühren bei 50°C und HPLC-Reinigung (Methode 16) 172 mg (54% d. Th.) der Titelverbindung erhalten.

¹H-NMR (400 MHz, DMSO-d₆, δ/ppm): 10.82 (s, 1H), 9.02 (d, 1H), 8.61 (d, 1H), 8.45 (dd, 1H), 8.16 (dt, 1H), 8.10 (t, 2H), 7.86-7.94 (m, 2H), 7.67 (dd, 1H), 7.51 (d, 1H), 7.35-7.44 (m, 2H), 6.35 (s, 1H), 2.24 (s, 3H), 1.30 (s, 9H).

LC/MS (Methode 7, ESIpos): Rₜ = 1.22 min, m/z = 451 [M+H]⁺.

### Beispiel 126

### N-{2,4-Dimethyl-5-[6-(pyridin-3-yl)-1H-imidazo[1,2-b]pyrazol-1-yl]phenyl}-3-[(trifluormethyl)-sulfanyl]benzamid

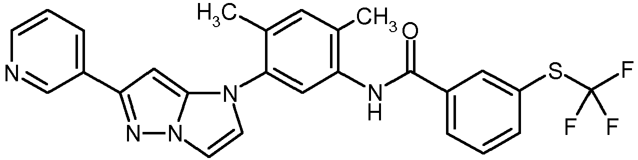

80 mg (0.264 mmol) der Verbindung aus Beispiel 11A und 62 mg (0.277 mmol) 3-[(Trifluormethyl)-sulfanyl]benzoesäure wurden in 2 ml DMF gelöst und nacheinander mit 120 mg (0.316 mmol) HATU und 60 µl (0.343 mmol) *N,N-*Diisopropylethylamin versetzt. Nach ca. 16 h Rühren bei RT wurde das Reaktionsgemisch mit 1 ml Acetonitril verdünnt und mittels präparativer HPLC in seine Komponenten aufgetrennt (Methode 36). Nach Vereinigen der Produktfraktionen, Eindampfen und Trocknen im Hochvakuum wurden 89 mg (95% Reinheit, 63% d. Th.) der Titelverbindung erhalten.

¹H-NMR (400 MHz, DMSO-d₆, δ/ppm): 10.18 (s, 1H), 9.06 (s, 1H), 8.50 (d, 1H), 8.29 (s, 1H), 8.23-8.19 (m, 2H), 7.96 (d, 1H), 7.90 (d, 1H), 7.73 (t, 1H), 7.51 (s, 1H), 7.49 (d, 1H), 7.45 (dd, 1H), 7.38 (s, 1H), 6.32 (s, 1H), 2.31 (s, 3H), 2.26 (s, 3H).

LC/MS (Methode 3, ESIpos): Rₜ = 1.05 min, m/z = 508 [M+H]⁺.

### Beispiel 127

### N-{2,4-Dimethyl-5-[6-(pyridin-3-yl)-1H-imidazo[1,2-b]pyrazol-1-yl]phenyl}-3-(trifluormethoxy)-benzamid

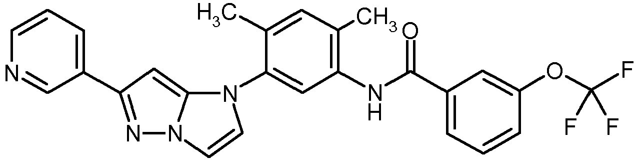

Analog zu dem unter Beispiel 126 beschriebenen Verfahren wurden aus 80 mg (0.264 mmol) der Verbindung aus Beispiel 11A und 57 mg (0.277 mmol) 3-(Trifluormethoxy)benzoesäure 85 mg (65% d. Th.) der Titelverbindung erhalten.

¹H-NMR (400 MHz, DMSO-d₆, δ/ppm): 10.14 (s, 1H), 9.05 (d, 1H), 8.49 (dd, 1H), 8.20 (dt, 1H), 8.04 (d, 1H), 7.91 (s, 1H), 7.89 (d, 1H), 7.70 (t, 1H), 7.62 (d, 1H), 7.50 (s, 1H), 7.48 (d, 1H), 7.43 (dd, 1H), 6.38 (s, 1H), 6.32 (s, 1H), 2.31 (s, 3H), 2.25 (s, 3H).

LC/MS (Methode 3, ESIpos): Rₜ = 1.02 min, m/z = 492 [M+H]⁺.

### Beispiel 128

### N-{2,4-Dimethyl-5-[6-(pyridin-3-yl)-1H-imidazo[1,2-b]pyrazol-1-yl]pheny}-3-(difluormethoxy)-benzamid

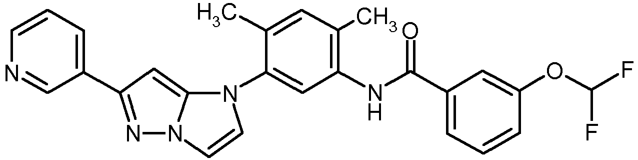

Analog zu dem unter Beispiel 126 beschriebenen Verfahren wurden aus 80 mg (0.264 mmol) der Verbindung aus Beispiel 11A und 52 mg (0.277 mmol) 3-(Difluormethoxy)benzoesäure 106 mg (84% d. Th.) der Titelverbindung erhalten.

¹H-NMR (400 MHz, DMSO-d₆, δ/ppm): 10.07 (s, 1H), 9.05 (d, 1H), 8.49 (dd, 1H), 8.21 (dt, 1H), 7.89 (d, 1H), 7.87 (d, 1H), 7.75 (s, 1H), 7.61 (t, 1H), 7.51-7.33 (m, 6H), 6.31 (s, 1H), 2.30 (s, 3H), 2.25 (s, 3H).

LC/MS (Methode 3, ESIpos): Rₜ = 0.93 min, m/z = 474 [M+H]⁺.

### Beispiel 129

### N-{2,4-Dimethyl-5-[6-(pyridin-3-yl)-1H-imidazo[1,2-b]pyrazol-1-yl]phenyl}-3-(1,1,2,2-tetrafluor-ethoxy)benzamid

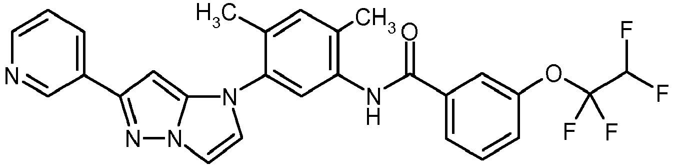

Analog zu dem unter Beispiel 126 beschriebenen Verfahren wurden aus 80 mg (0.264 mmol) der Verbindung aus Beispiel 11A und 66 mg (0.277 mmol) 3-(1,1,2,2-Tetrafluorethoxy)benzoesäure 87 mg (63% d. Th.) der Titelverbindung erhalten.

¹H-NMR (400 MHz, DMSO-d₆, δ/ppm): 10.14 (s, 1H), 9.06 (s, 1H), 8.50 (d, 1H), 8.23 (d, 1H), 8.00 (d, 1H), 7.89 (d, 1H), 7.86 (s, 1H), 7.66 (t, 1H), 7.53 (d, 1H), 7.50-7.45 (m, 3H), 7.38 (s, 1H), 6.86 (t, 1H), 6.33 (s, 1H), 2.30 (s, 3H), 2.25 (s, 3H).

LC/MS (Methode 3, ESIpos): Rₜ = 1.01 min, m/z = 524 [M+H]⁺.

### Beispiel 130

### N-{2,4-Dimethyl-5-[6-(pyridin-3-yl)-1H-imidazo[1,2-b]pyrazol-1-yl]phenyl}-3-(2,2,2-trifluor-ethoxy)benzamid

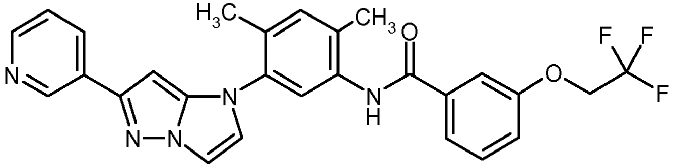

Analog zu dem unter Beispiel 126 beschriebenen Verfahren wurden aus 80 mg (0.264 mmol) der Verbindung aus Beispiel 11A und 61 mg (0.277 mmol) 3-(2,2,2-Trifluorethoxy)benzoesäure 95 mg (71% d. Th.) der Titelverbindung erhalten.

¹H-NMR (400 MHz, DMSO-d₆, δ/ppm): 9.96 (s, 1H), 9.07 (d, 1H), 8.50 (dd, 1H), 8.23 (dt, 1H), 7.89 (d, 1H), 7.67-7.64 (m, 2H), 7.53-7.45 (m, 4H), 7.37 (s, 1H), 7.30 (dd, 1H), 6.31 (s, 1H), 4.85 (quart, 2H), 2.30 (s, 3H), 2.25 (s, 3H).

LC/MS (Methode 3, ESIpos): Rₜ = 0.99 min, m/z = 506 [M+H]⁺.

### Beispiel 131

### N-{2,4-Dimethyl-5-[6-(pyridin-3-yl)-1H-imidazo[1,2-b]pyrazol-1-yl]phenyl}-3-methoxybenzamid

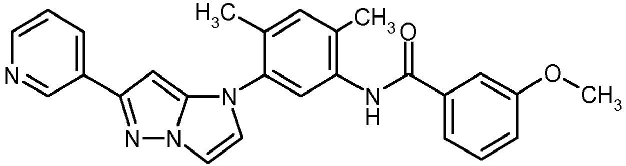

Analog zu dem unter Beispiel 126 beschriebenen Verfahren wurden aus 80 mg (0.264 mmol) der Verbindung aus Beispiel 11A und 42 mg (0.277 mmol) 3-Methoxybenzoesäure 83 mg (71% d. Th.) der Titelverbindung erhalten.

¹H-NMR (400 MHz, DMSO-d₆, δ/ppm): 9.93 (s, 1H), 9.10 (d, 1H), 8.56 (d, 1H), 8.37 (d, 1H), 7.90 (d, 1H), 7.60-7.55 (m, 2H), 7.52-7.50 (m, 3H), 7.45 (t, 1H), 7.37 (s, 1H), 7.16 (dd, 1H), 6.37 (s, 1H), 3.83 (s, 3H), 2.30 (s, 3H), 2.25 (s, 3H).

LC/MS (Methode 3, ESIpos): Rₜ = 0.88 min, m/z = 438 [M+H]⁺.

### Beispiel 132

### N-{2,4-Dimethyl-5-[6-(pyridin-3-yl)-1H-imidazo[1,2-b]pyrazol-1-yl]phenyl}-3-isopropoxybenzamid

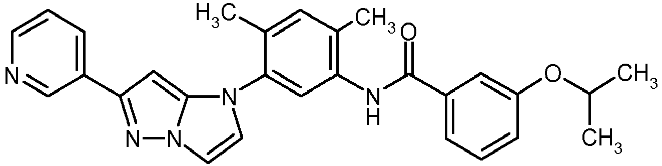

Analog zu dem unter Beispiel 126 beschriebenen Verfahren wurden aus 80 mg (0.264 mmol) der Verbindung aus Beispiel 11A und 50 mg (0.277 mmol) 3-Isopropoxybenzoesäure 88 mg (71% d. Th.) der Titelverbindung erhalten.

¹H-NMR (400 MHz, DMSO-d₆, δ/ppm): 9.90 (s, 1H), 9.05 (d, 1H), 8.50 (d, 1H), 8.22 (d, 1H), 7.89 (d, 1H), 7.53-7.43 (m, 5H), 7.42 (t, 1H), 7.36 (s, 1H), 7.14 (dd, 1H), 6.31 (s, 1H), 4.70 (sept, 1H), 2.30 (s, 3H), 2.24 (s, 3H), 1.29 (d, 6H).

LC/MS (Methode 3, ESIpos): Rₜ = 0.99 min, m/z = 466 [M+H]⁺.

### Beispiel 133

### N-{2,4-Dimethyl-5-[6-(pyridin-3-yl)-1H-imidazo[1,2-b]pyrazol-1-yl]phenyl}-3-isobutoxybenzamid

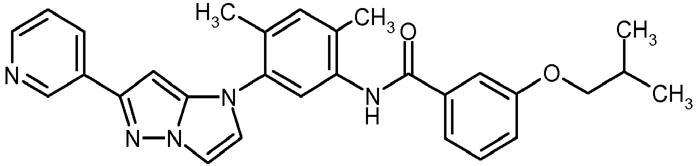

Analog zu dem unter Beispiel 126 beschriebenen Verfahren wurden aus 80 mg (0.264 mmol) der Verbindung aus Beispiel 11A und 54 mg (0.277 mmol) 3-Isobutoxybenzoesäure 84 mg (66% d. Th.) der Titelverbindung erhalten.

¹H-NMR (400 MHz, DMSO-d₆, δ/ppm): 9.93 (s, 1H), 9.05 (d, 1H), 8.49 (d, 1H), 8.20 (d, 1H), 7.89 (d, 1H), 7.55-7.41 (m, 6H), 7.36 (s, 1H), 7.16 (dd, 1H), 6.30 (s, 1H), 3.82 (d, 1H), 2.29 (s, 3H), 2.25 (s, 3H), 2.04 (m, 1H), 1.00 (d, 6H).

LC/MS (Methode 3, ESIpos): Rₜ = 1.10 min, m/z = 480 [M+H]⁺.

### Beispiel 134

### 3-tert.-Butoxy-N-{2,4-dimethyl-5-[6-(pyridin-3-yl)-1H-imidazo[1,2-b]pyrazol-1-yl]phenyl}benzamid

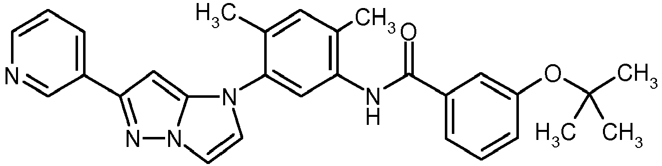

Analog zu dem unter Beispiel 126 beschriebenen Verfahren wurden aus 100 mg (0.330 mmol) der Verbindung aus Beispiel 11A und 74 mg (0.363 mmol, Gehalt 95%) 3-*tert*.-Butoxybenzoesäure 131 mg (83% d. Th.) der Titelverbindung erhalten.

¹H-NMR (400 MHz, DMSO-d₆, δ/ppm): 9.93 (s, 1H), 9.04 (d, 1H), 8.48 (d, 1H), 8.19 (d, 1H), 7.88 (d, 1H), 7.69 (d, 1H), 7.54-7.36 (m, 6H), 7.21 (d, 1H), 6.29 (s, 1H), 2.30 (s, 3H), 2.24 (s, 3H), 1.34 (s, 9H).

LC/MS (Methode 3, ESIpos): Rₜ = 1.02 min, m/z = 480 [M+H]⁺.

### Beispiel 135

### N-{2,4-Dimethyl-5-[6-(pyridin-3-yl)-1H-imidazo[1,2-b]pyrazol-1-yl]phenyl}-3-(2-ethoxyethoxy)-benzamid

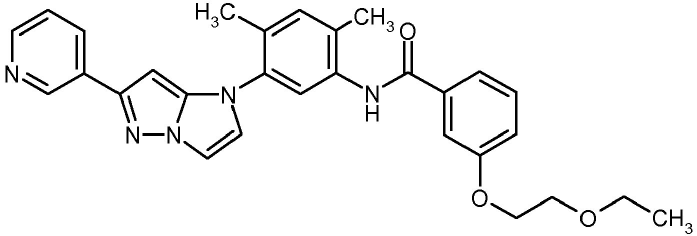

Analog zu dem unter Beispiel 126 beschriebenen Verfahren wurden aus 80 mg (0.264 mmol) der Verbindung aus Beispiel 11A und 58 mg (0.277 mmol) 3-(2-Ethoxyethoxy)benzoesäure 103 mg (78% d. Th.) der Titelverbindung erhalten.

¹H-NMR (400 MHz, DMSO-d₆, δ/ppm): 9.93 (s, 1H), 9.06 (d, 1H), 8.50 (d, 1H), 8.23 (dt, 1H), 7.89 (d, 1H), 7.59-7.53 (m, 2H), 7.50-7.42 (m, 4H), 7.37 (s, 1H), 7.18 (dd, 1H), 6.32 (s, 1H), 4.16 (dd, 2H), 3.72 (dd, 2H), 3.51 (quart, 2H), 2.30 (s, 3H), 2.25 (s, 3H), 1.13 (t, 3H).

LC/MS (Methode 3, ESIpos): Rₜ = 0.94 min, m/z = 496 [M+H]⁺.

### Beispiel 136

### N-{2,4-Dimethyl-5-[6-(pyridin-3-yl)-1H-imidazo[1,2-b]pyrazol-1-yl]phenyl}-3-(2-hydroxyethoxy)-benzamid

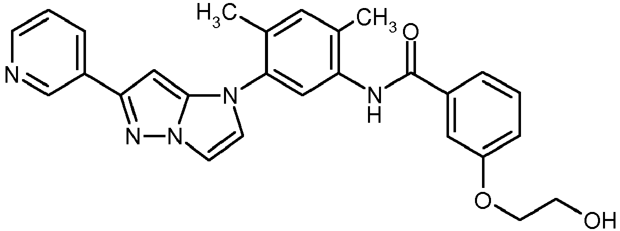

Analog zu dem unter Beispiel 126 beschriebenen Verfahren wurden aus 100 mg (0.330 mmol) der Verbindung aus Beispiel 11A und 63 mg (0.346 mmol) 3-(2-Hydroxyethoxy)benzoesäure 82 mg (51% d. Th., 97% Reinheit) der Titelverbindung erhalten. Für die präparative HPLC-Reinigung wurde in diesem Fall die Methode 38 verwendet.

¹H-NMR (400 MHz, DMSO-d₆, δ/ppm): 9.92 (s, 1H), 9.14 (d, 1H), 8.61 (d, 1H), 8.49 (d, 1H), 7.92 (d, 1H), 7.69 (dd, 1H), 7.56-7.50 (m, 4H), 7.44 (t, 1H), 7.38 (s, 1H), 7.17 (dd, 1H), 6.42 (s, 1H), 4.06 (t, 2H), 3.74 (t, 2H), 2.31 (s, 3H), 2.24 (s, 3H).

LC/MS (Methode 3, ESIpos): Rₜ = 0.78 min, m/z = 486 [M+H]⁺.

### Beispiel 137

### 3-[2-(Dimethylamino)ethoxy]-N-{2,4-dimethyl-5-[6-(pyridin-3-yl)-1H-imidazo[1,2-b]pyrazol-1-yl]-phenyl} benzamid

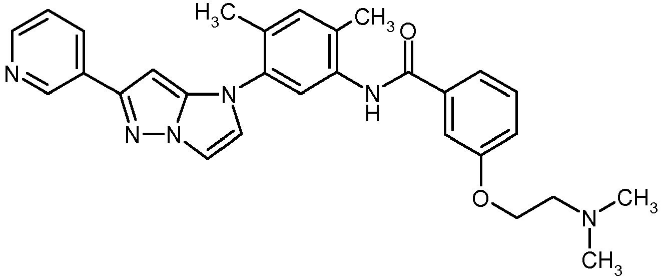

Analog zu dem unter Beispiel 126 beschriebenen Verfahren wurden aus 80 mg (0.264 mmol) der Verbindung aus Beispiel 11A und 68 mg (0.277 mmol) des Hydrochlorids von 3-[2-(Dimethyl-amino)ethoxy]benzoesäure [Lit.: US 6 069 149, Production Example 8] 72 mg (55% d. Th.) der Titelverbindung erhalten. Abweichend von Beispiel 126 wurden hier 2.5 Äquivalente (115 µl, 0.659 mmol) *N,N-*Diisopropylethylamin eingesetzt. Die Titelverbindung wurde nach der präparativen HPLC-Reinigung zunächst als Ameisensäure-Salz isoliert. Die Freisetzung der Base erfolgte durch Lösen des Formiats in einer kleinen Menge Methanol und anschließende Perkolation über eine Hydrogencarbonat-Kartusche (Fa. Polymerlabs, Stratospheres SPE, PL-HCO₃ MP SPE, Kapazität 0.9 mmol). Nach Eindampfen des Perkolats und Trocknen des Rückstands im Hochvakuum wurde die Titelverbindung in oben genannter Menge erhalten.

¹H-NMR (400 MHz, DMSO-d₆, δ/ppm): 9.92 (s, 1H), 9.04 (d, 1H), 8.48 (d, 1H), 8.17 (d, 1H), 7.88 (d, 1H), 7.56-7.39 (m, 6H), 7.36 (s, 1H), 7.17 (dd, 1H), 6.29 (s, 1H), 4.12 (t, 2H), 2.64 (t, 2H), 2.30 (s, 3H), 2.25 (s, 3H), 2.22 (s, 6H).

LC/MS (Methode 3, ESIpos): Rₜ = 0.69 min, m/z = 495 [M+H]⁺.

### Beispiel 138

### 3-(4,4-Difluorpiperidin-1-yl)-N-{2,4-dimethyl-5-[6-(pyridin-3-yl)-1H-imidazo[1,2-b]pyrazol-1-yl]-phenyl} benzamid

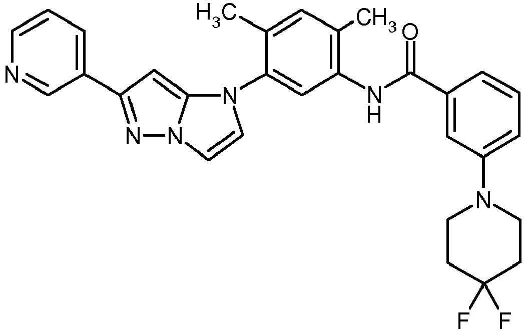

Analog zu dem unter Beispiel 126 beschriebenen Verfahren wurden aus 100 mg (0.330 mmol) der Verbindung aus Beispiel 11A und 87 mg (0.363 mmol) der Verbindung aus Beispiel 90A 110 mg (63% d. Th.) der Titelverbindung erhalten.

¹H-NMR (400 MHz, DMSO-d₆, δ/ppm): 9.88 (s, 1H), 9.05 (d, 1H), 8.49 (dd, 1H), 8.21 (dt, 1H), 7.89 (d, 1H), 7.54 (s, 1H), 7.48-7.35 (m, 6H), 7.23 (d, 1H), 6.30 (s, 1H), 3.41 (m, 4H), 2.30 (s, 3H), 2.25 (s, 3H), 2.07 (m, 4H).

LC/MS (Methode 3, ESIpos): Rₜ = 1.00 min, m/z = 527 [M+H]⁺.

### Beispiel 139

### N-{2,4-Dimethyl-5-[6-(pyridin-3-yl)-1H-imidazo[1,2-b]pyrazol-1-yl]phenyl}-3-(1,1,1-trifluor-2-methylpropan-2-yl)benzamid

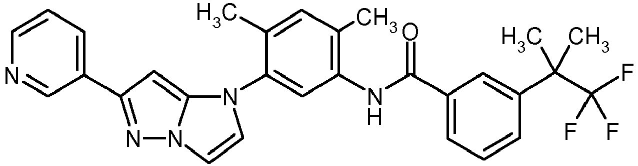

Analog zu dem unter Beispiel 126 beschriebenen Verfahren wurden aus 100 mg (0.330 mmol) der Verbindung aus Beispiel 11A und 80 mg (0.346 mmol) der Verbindung aus Beispiel 91A 115 mg (64% d. Th.) der Titelverbindung erhalten.

¹H-NMR (400 MHz, DMSO-d₆, δ/ppm): 10.05 (s, 1H), 9.06 (d, 1H), 8.50 (dd, 1H), 8.23 (d, 1H), 8.10 (s, 1H), 7.99 (d, 1H), 7.89 (dd, 1H), 7.78 (d, 1H), 7.57 (t, 1H), 7.50 (s, 1H), 7.49 (d, 1H), 7.45 (dd, 1H), 7.38 (s, 1H), 6.31 (s, 1H), 2.31 (s, 3H), 2.25 (s, 3H), 1.61 (s, 6H).

LC/MS (Methode 3, ESIpos): Rₜ = 1.05 min, m/z = 518 [M+H]⁺.

### Beispiel 140

### N-{2,4-Dimethyl-5-[6-(pyridin-3-yl)-1H-imidazo[1,2-b]pyrazol-1-yl]phenyl}-3-(3-methyloxetan-3-yl)benzamid

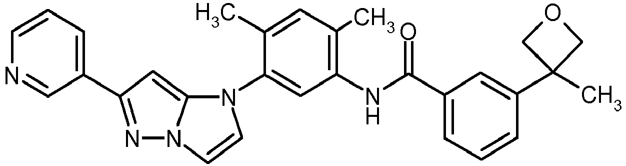

In einem Mikrowellenreaktionsgefäß wurde eine Mischung aus 200 mg (0.660 mmol) der Verbindung aus Beispiel 11A, 150 mg (0.660 mmol) der Verbindung aus Beispiel 99A, 209 mg (0.793 mmol) Molybdänhexacarbonyl, 19 mg (0.066 mmol) Tri-*tert*.-butylphosphoniumtetrafluoroborat und 62 mg (0.066 mmol) *trans*-Bis-(acetato)-bis-[*o*-(di-*o*-tolylphosphino)benzyl]-dipalladium(II) in 4.5 ml THF mit 302 mg (1.98 mmol) 1,8-Diazabicyclo[5.4.0]undec-7-en (DBU) versetzt. Nach der Zugabe des DBUs wurde das Reaktionsgefäß zügig mit einem Krampverschluss verschlossen. Dann wurde das Gemisch 30 min in einem Mikrowellenofen auf 140°C erhitzt (Biotage Initiator, mit dynamischer Steuerung der Einstrahlleistung). Nach dem Abkühlen auf RT wurde das Reaktionsgemisch mit ca. 15 ml Wasser versetzt und dreimal mit je ca. 15 ml Ethylacetat extrahiert. Die vereinigten organischen Extrakte wurden mit gesättigter wässriger Natriumchlorid-Lösung gewaschen und anschließend über Magnesiumsulfat getrocknet. Nach Filtration und Abdampfen des Lösungsmittels wurde der verbliebene Rückstand mittels präparativer HPLC in seine Komponenten aufgetrennt (Methode 33). Da die Produktfraktion noch verunreinigt war, wurde die präparative HPLC-Reinigung mit dieser Fraktion noch einmal nach gleicher Methode wiederholt. Es wurde so eine noch verunreinigte Fraktion von 15 mg erhalten sowie eine saubere Fraktion, die aus 6 mg (2% d. Th.) der Titelverbindung bestand.

¹H-NMR (400 MHz, DMSO-d₆, δ/ppm): 9.99 (s, 1H), 9.05 (s, 1H), 8.48 (s, 1H), 8.18 (d, 1H), 7.90-7.82 (m, 3H), 7.54-7.46 (m, 4H), 7.41 (dd, 1H), 7.37 (s, 1H), 6.30 (s, 1H), 4.87 (d, 2H), 4.58 (d, 2H), 2.30 (s, 3H), 2.26 (s, 3H), 1.67 (s, 3H).

LC/MS (Methode 3, ESIpos): Rₜ = 0.87 min, m/z = 478 [M+H]⁺.

### Beispiel 141

### N-{2,4-Dimethyl-5-[6-(pyridin-3-yl)-1H-imidazo[1,2-b]pyrazol-1-yl]phenyl}-3-(2-hydroxypropan-2-yl)benzamid

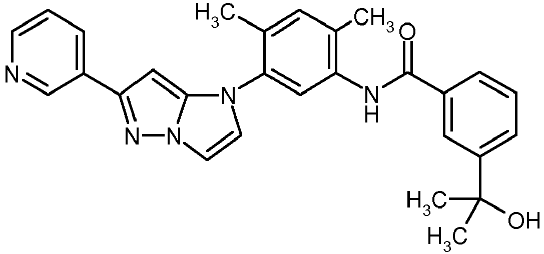

250 mg (0.824 mmol) der Verbindung aus Beispiel 11A und 156 mg (0.865 mmol) der Verbindung aus Beispiel 92A wurden in 6.3 ml DMF gelöst und nacheinander mit 376 mg (0.989 mmol) HATU und 215 µl (1.24 mmol) *N,N-*Diisopropylethylamin versetzt. Nach ca. 16 h Rühren bei RT wurde das Reaktionsgemisch mit ca. 3 ml Acetonitril verdünnt und anschließend in drei Portionen mittels präparativer HPLC in seine Komponenten aufgetrennt (Methode 36). Nach Vereinigen und Eindampfen der Produktfraktionen wurde der erhaltene Rückstand in einer kleinen Menge Methanol gelöst und über eine Hydrogencarbonat-Kartusche gegeben (Fa. Polymerlabs, Stratospheres SPE, PL-HCO₃ MP SPE, Kapazität 0.9 mmol), um aus dem Formiat-Salz aus der HPLC-Reinigung die freie Base herzustellen. Da das so erhaltene Produkt noch verunreinigt war, wurde es mittels MPLC weiter aufgereinigt (ca. 15 g Kieselgel, Laufmittel: Cyclohexan/Ethylacetat/ Methanol 50:50:0 → 0:100:0 → 0:91:9). Nach Eindampfen der Produktfraktionen und Trocknen des Rückstands im Hochvakuum wurden 238 mg (58% d. Th., 95% Reinheit) der Titelverbindung erhalten.

¹H-NMR (400 MHz, DMSO-d₆, δ/ppm): 9.94 (s, 1H), 9.04 (d, 1H), 8.48 (dd, 1H), 8.18 (dt, 1H), 8.07 (s, 1H), 7.88 (d, 1H), 7.82 (d, 1H), 7.68 (d, 1H), 7.50-7.39 (m, 4H), 7.37 (s, 1H), 6.30 (s, 1H), 5.13 (s, 1H), 2.30 (s, 3H), 2.25 (s, 3H), 1.47 (s, 6H).

LC/MS (Methode 3, ESIpos): Rₜ = 0.83 min, m/z = 466 [M+H]⁺.

### Beispiel 142

### N-{2,4-Dimethyl-5-[6-(pyridin-3-yl)-1H-imidazo[1,2-b]pyrazol-1-yl]phenyl}-3-(2-fluorpropan-2-yl)benzamid

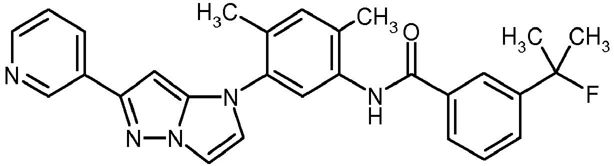

Eine Lösung von 100 mg (0.215 mmol) der Verbindung aus Beispiel 141 in 4.5 ml wasserfreiem Dichlormethan wurde bei -78°C tropfenweise mit einer Lösung von 34 µl (0.258 mmol) Diethylaminoschwefeltrifluorid (DAST) in 0.5 ml wasserfreiem Dichlormethan versetzt. Nachdem das Reaktionsgemisch 1 h bei -78°C gerührt worden war, wurde es mit ca. 5 ml gesättigter wässriger Natriumhydrogencarbonat-Lösung versetzt und auf RT erwärmt. Es wurde mit ca. 10 ml Wasser verdünnt und dreimal mit je ca. 10 ml Dichlormethan extrahiert. Die vereinigten organischen Extrakte wurden einmal mit gesättigter wässriger Natriumchlorid-Lösung gewaschen und über Magnesiumsulfat getrocknet. Nach Filtration und Eindampfen wurde der erhaltene Rückstand mittels präparativer HPLC (Methode 9) in seine Komponenten aufgetrennt. Nach Eindampfen der Produktfraktionen und Trocknen des Rückstands im Hochvakuum wurden 40 mg (39% d. Th.) der Titelverbindung erhalten.

¹H-NMR (400 MHz, DMSO-d₆, δ/ppm): 10.02 (s, 1H), 9.04 (d, 1H), 8.48 (dd, 1H), 8.17 (dt, 1H), 8.00 (s, 1H), 7.92 (d, 1H), 7.88 (d, 1H), 7.64 (d, 1H), 7.54 (t, 1H), 7.49 (s, 1H), 7.48 (d, 1H), 7.41 (dd, 1H), 7.37 (s, 1H), 6.29 (s, 1H), 2.30 (s, 3H), 2.25 (s, 3H), 1.70 (d, 6H).

LC/MS (Methode 3, ESIpos): Rₜ = 0.96 min, m/z = 468 [M+H]⁺.

### Beispiel 143

### N-{2,4-Dimethyl-5-[6-(pyridin-3-yl)-1H-imidazo[1,2-b]pyrazol-1-yl]phenyl}-3-(pentafluor-λ⁶-sulfanyl)-5-(piperidin-1-yl)benzamid

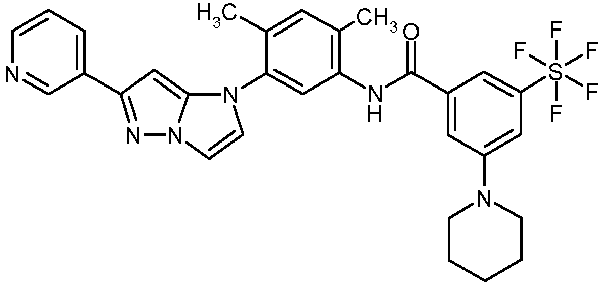

Analog zu dem unter Beispiel 126 beschriebenen Verfahren wurden aus 35 mg (0.115 mmol) der Verbindung aus Beispiel 11A und 40 mg (0.121 mmol) der Verbindung aus Beispiel 93A 53 mg (74% d. Th.) der Titelverbindung erhalten.

¹H-NMR (400 MHz, DMSO-d₆, δ/ppm): 10.20 (s, 1H), 9.05 (s, 1H), 8.48 (m, 1H), 8.19 (d, 1H), 7.89 (s, 1H), 7.75 (s, 1H), 7.70 (s, 1H), 7.49-7.39 (m, 5H), 6.30 (s, 1H), 3.32 (m, 4H, teilweise überdeckt vom Wassersignal), 2.28 (s, 3H), 2.25 (s, 3H), 1.66-1.55 (m, 6H).

LC/MS (Methode 3, ESIpos): Rₜ = 1.27 min, m/z = 617 [M+H]⁺.

### Beispiel 144

### 3-(4-Cyanopiperidin-1-yl)-N-{2,4-dimethyl-5-[6-(pyridin-3-yl)-1H-imidazo[1,2-b]pyrazol-1-yl]-phenyl}-5-(pentafluor-λ⁶-sulfanyl)benzamid

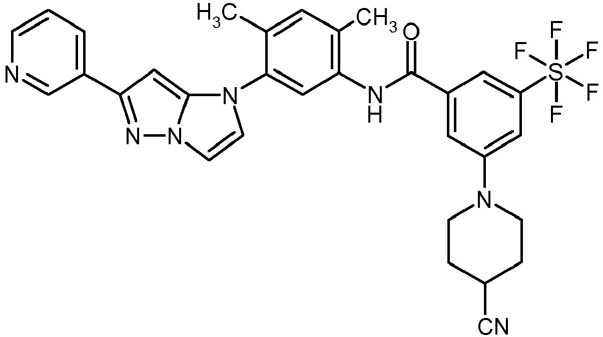

Analog zu dem unter Beispiel 126 beschriebenen Verfahren wurden aus 122 mg (0.401 mmol) der Verbindung aus Beispiel 11A und 150 mg (0.421 mmol) der Verbindung aus Beispiel 94A 120 mg (44% d. Th.) der Titelverbindung erhalten.

¹H-NMR (400 MHz, DMSO-d₆, δ/ppm): 10.21 (s, 1H), 9.04 (d, 1H), 8.48 (dd, 1H), 8.18 (dt, 1H), 7.90 (d, 1H), 7.77 (s, 1H), 7.75 (s, 1H), 7.55 (t, 1H), 7.48 (d, 1H), 7.46 (s, 1H), 7.41 (dd, 1H), 7.39 (s, 1H), 6.30 (s, 1H), 3.59-3.52 (m, 2H), 3.27-3.20 (m, 2H), 3.13-3.07 (m, 1H), 2.29 (s, 3H), 2.26 (s, 3H), 2.05-1.98 (m, 2H), 1.89-1.80 (m, 2H).

LC/MS (Methode 3, ESIpos): Rₜ = 1.08 min, m/z = 642 [M+H]⁺.

### Beispiel 145

### N-{2,4-Dimethyl-5-[6-(pyridin-3-yl)-1H-imidazo[1,2-b]pyrazol-1-yl]phenyl}-3-(4-methoxy-piperidin-1-yl)-5-(pentafluor-λ⁶-sulfanyl)benzamid

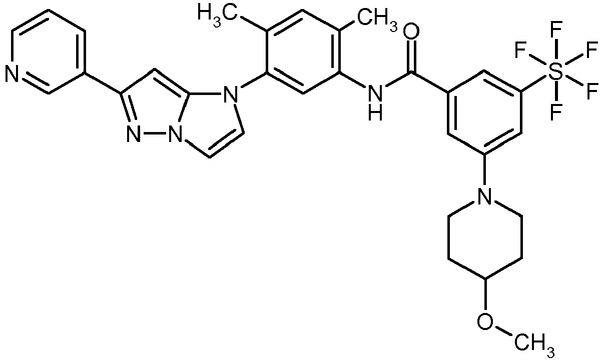

Analog zu dem unter Beispiel 126 beschriebenen Verfahren wurden aus 39 mg (0.127 mmol) der Verbindung aus Beispiel 11A und 48 mg (0.133 mmol) der Verbindung aus Beispiel 95A 54 mg (66% d. Th.) der Titelverbindung erhalten.

¹H-NMR (400 MHz, DMSO-d₆, δ/ppm): 10.21 (s, 1H), 9.04 (d, 1H), 8.48 (dd, 1H), 8.18 (dt, 1H), 7.89 (d, 1H), 7.76 (s, 1H), 7.71 (s, 1H), 7.50-7.48 (m, 2H), 7.46 (s, 1H), 7.41 (dd, 1H), 7.39 (s, 1H), 6.30 (s, 1H), 3.67-3.61 (m, 2H), 3.44-3.37 (m, 2H), 3.28 (s, 3H), 3.13-3.07 (m, 1H), 2.28 (s, 3H), 2.25 (s, 3H), 1.99-1.92 (m, 2H), 1.59-1.50 (m, 2H).

LC/MS (Methode 3, ESIpos): Rₜ = 1.14 min, m/z = 647 [M+H]⁺.

### Beispiel 146

### N-{2,4-Dimethyl-5-[6-(pyridin-3-yl)-1H-imidazo[1,2-b]pyrazol-1-yl]phenyl}-3-(3-methoxyazetidin-1-yl)-5-(pentafluor-λ⁶-sulfanyl)benzamid

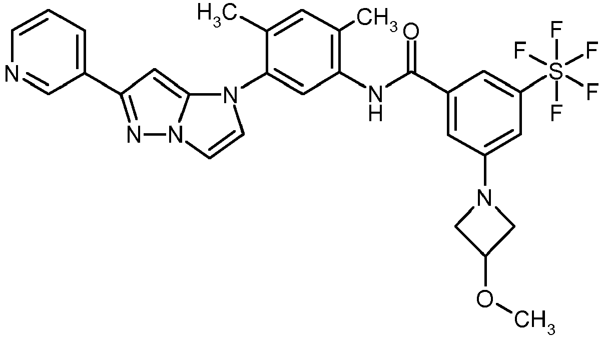

Analog zu dem unter Beispiel 126 beschriebenen Verfahren wurden aus 61 mg (0.200 mmol) der Verbindung aus Beispiel 11A und 70 mg (0.210 mmol) der Verbindung aus Beispiel 96A 75 mg (61% d. Th.) der Titelverbindung erhalten.

¹H-NMR (400 MHz, DMSO-d₆, δ/ppm): 10.16 (s, 1H), 9.04 (d, 1H), 8.48 (dd, 1H), 8.17 (dt, 1H), 7.88 (d, 1H), 7.66 (s, 1H), 7.47 (d, 1H), 7.45 (s, 1H), 7.41 (dd, 1H), 7.38 (s, 1H), 7.26 (s, 1H), 6.98 (t, 1H), 6.29 (s, 1H), 4.38-4.33 (m, 1H), 4.19 (dd, 2H), 3.78 (dd, 2H), 3.26 (s, 3H), 2.28 (s, 3H), 2.25 (s, 3H).

LC/MS (Methode 3, ESIpos): Rₜ = 1.09 min, m/z = 619 [M+H]⁺.

### Beispiel 147

### 3-Cyano-N-{2,4-dimethyl-5-[6-(pyridin-3-yl)-1H-imidazo[1,2-b]pyrazol-1-yl]phenyl}-5-(pentafluor-λ⁶-sulfanyl)benzamid-Dihydrochlorid

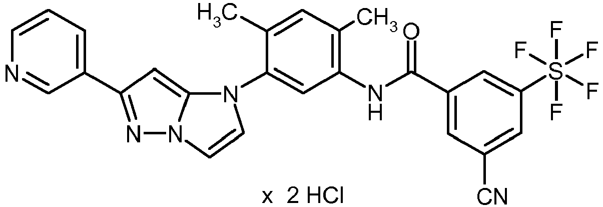

1.0 g (1.79 mmol) der Verbindung aus Beispiel 74 wurden in 9 ml Dioxan gelöst und mit 4.5 ml (17.9 mmol) einer 4 M Lösung von Chlorwasserstoff in Dioxan versetzt. Nachdem das Gemisch ca. 16 h bei RT gerührt worden war, wurde es am Rotationsverdampfer zur Trockene eingedampft. Der Rückstand wurde im Hochvakuum getrocknet. Es wurden 1.14 g (100% d. Th.) der Titelverbindung erhalten. 496 mg dieses Materials wurden durch Umkristallisation aus 23 ml Ethanol in einen kristallinen Zustand überführt.

¹H-NMR (400 MHz, DMSO-d₆, δ/ppm): 10.58 (s, 1H), 9.26 (d, 1H), 8.88 (d, 1H), 8.85 (s, 1H), 8.80 (s, 1H), 8.78 (d, 1H), 8.68 (s, 1H), 8.03 (dd, 1H), 7.98 (d, 1H), 7.60 (d, 1H), 7.54 (s, 1H), 7.42 (s, 1H), 6.62 (s, 1H), 2.33 (s, 3H), 2.26 (s, 3H).

LC/MS (Methode 3, ESIpos): Rₜ = 1.04 min, m/z = 559 [M+H]⁺.

### Beispiel 148

### N-{2,4-Dimethyl-5-[6-(pyridin-3-yl)-1H-imidazo[1,2-b]pyrazol-1-yl]phenyl}-3-(2-hydroxypropan-2-yl)-5-(pentafluor-λ⁶-sulfanyl)benzamid

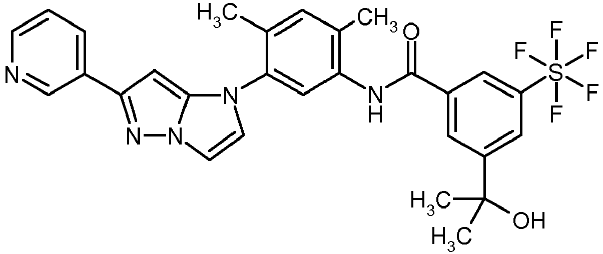

Analog zu dem unter Beispiel 126 beschriebenen Verfahren wurden aus 250 mg (0.824 mmol) der Verbindung aus Beispiel 11A und 252 mg (0.824 mmol) der Verbindung aus Beispiel 19A 281 mg (57% d. Th.) der Titelverbindung erhalten. Die Reinigung des Rohprodukts mittels präparativer HPLC erfolgte hier in zwei Portionen. Die Produktfraktionen wurden vereinigt, eingedampft, in wenig Methanol wieder aufgenommen und anschließend über eine Hydrogencarbonat-Kartusche gegeben (Fa. Polymerlabs, Stratospheres SPE, PL-HCO₃ MP SPE, Kapazität 0.9 mmol), um aus dem Formiat-Salz aus der HPLC-Reinigung die freie Base herzustellen.

¹H-NMR (400 MHz, DMSO-d₆, δ/ppm): 10.31 (s, 1H), 9.04 (d, 1H), 8.48 (dd, 1H), 8.32 (s, 1H), 8.28 (s, 1H), 8.19-8.16 (m, 2H), 7.89 (d, 1H), 7.49-7.47 (m, 2H), 7.41 (dd, 1H), 7.40 (s, 1H), 7.29 (s, 1H), 5.52 (s, 1H), 2.30 (s, 3H), 2.26 (s, 3H), 1.51 (s, 6H).

LC/MS (Methode 3, ESIpos): Rₜ = 1.03 min, m/z = 592 [M+H]⁺.

### Beispiel 149

### N-{2,4-Dimethyl-5-[6-(pyridin-3-yl)-1H-imidazo[1,2-b]pyrazol-1-yl]phenyl}-3-(2-fluorpropan-2-yl)-5-(pentafluor-λ⁶-sulfanyl)benzamid

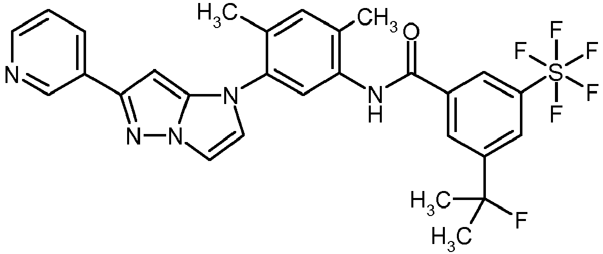

Eine Lösung von 100 mg (0.169 mmol) der Verbindung aus Beispiel 148 in 3.5 ml wasserfreiem Dichlormethan wurde bei -78°C tropfenweise mit einer Lösung von 27 µl (0.203 mmol) Diethylaminoschwefeltrifluorid (DAST) in 0.5 ml wasserfreiem Dichlormethan versetzt. Nachdem das Reaktionsgemisch 1 h bei -78°C gerührt worden war, wurde es mit 1 ml gesättigter wässriger Natriumhydrogencarbonat-Lösung versetzt und auf RT erwärmt. Das Gemisch wurde über eine Extrelut-Kartusche NT3 (Fa. Merck, Darmstadt) von Salzen und wässrigen Bestandteilen befreit. Nach Eindampfen wurde der erhaltene Rückstand mittels präparativer HPLC (Methode 36) in seine Komponenten aufgetrennt. Nach Eindampfen der Produktfraktionen und Trocknen des Rückstands im Hochvakuum wurden 50 mg (50% d. Th.) der Titelverbindung erhalten.

¹H-NMR (400 MHz, DMSO-d₆, δ/ppm): 10.37 (s, 1H), 9.04 (d, 1H), 8.48 (dd, 1H), 8.38 (s, 1H), 8.31 (s, 1H), 8.17 (dt, 1H), 8.07 (s, 1H), 7.89 (d, 1H), 7.49 (s, 1H), 7.47 (d, 1H), 7.41 (dd, 1H), 7.40 (s, 1H), 6.29 (s, 1H), 2.30 (s, 3H), 2.26 (s, 3H), 1.75 (d, 6H).

LC/MS (Methode 3, ESIpos): Rₜ = 1.15 min, m/z = 594 [M+H]⁺.

### Beispiel 150

### tert.-Butyl-[3-({2,4-dimethyl-5-[6-(pyridin-3-yl)-1H-imidazo[1,2-b]pyrazol-1-yl]phenyl}-carbamoyl)-5-(pentafluor-λ⁶-sulfanyl)phenyl]acetat

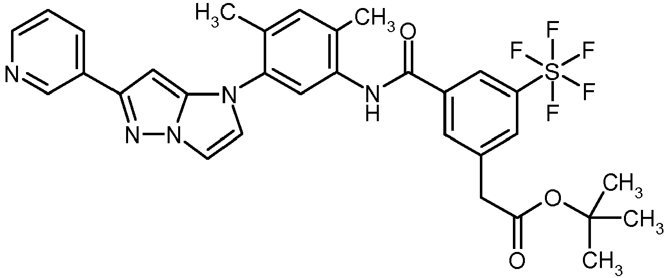

836 mg (2.31 mmol) der Verbindung aus Beispiel 97A und 1.05 g (2.77 mmol) HATU wurden in 14 ml DMF gelöst und langsam mit 338 mg (2.77 mmol) 4-*N*,*N-*Dimethylaminopyridin (DMAP) versetzt. Dann wurden 700 mg (2.31 mmol) der Verbindung aus Beispiel 11A hinzugefügt. Das Reaktionsgemisch wurde 3 h bei RT gerührt. Anschließend wurde es mit ca. 200 ml Wasser versetzt und dreimal mit je ca. 200 ml Ethylacetat extrahiert. Die vereinigten organischen Extrakte wurden mit gesättigter wässriger Natriumchlorid-Lösung gewaschen, über Magnesiumsulfat getrocknet, filtriert und zur Trockene eingedampft. Das so erhaltene Rohprodukt wurde mittels MPLC über ca. 100 g Kieselgel mit Cyclohexan/Ethylacetat 50:50 → 0:100 als Laufmittel gereinigt. Nach Eindampfen der Produktfraktionen und Trocknen des Rückstands im Hochvakuum wurden 966 mg (64% d. Th.) der Titelverbindung erhalten.

¹H-NMR (400 MHz, CDCl₃, δ/ppm): 9.04 (d, 1H), 8.52 (dd, 1H), 8.19-8.15 (m, 2H), 7.95-7.93 (m, 2H), 7.88 (s, 1H), 7.83 (s, 1H), 7.49 (d, 1H), 7.33 (dd, 1H), 6.94 (d, 1H), 6.01 (s, 1H), 3.69 (s, 2H), 2.39 (s, 3H), 2.28 (s, 3H), 1.46 (s, 9H) [weiteres Signal unter CHCl₃-Peak verborgen].

LC/MS (Methode 3, ESIpos): Rₜ = 1.21 min, m/z = 648 [M+H]⁺.

### Beispiel 151

### [3-({2,4-Dimethyl-5-[6-(pyridin-3-yl)-1H-imidazo[1,2-b]pyrazol-1-yl]phenyl}carbamoyl)-5-(pentafluor-λ⁶-sulfanyl)phenyl]essigsäure

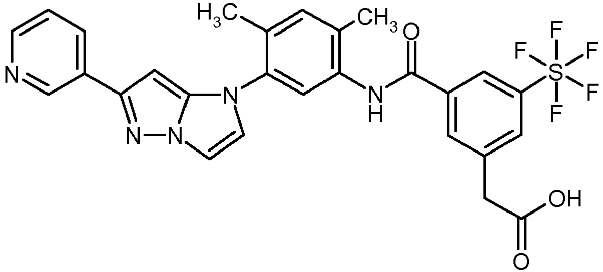

920 mg (1.42 mmol) der Verbindung aus Beispiel 150 wurden in 18 ml (71 mmol) einer 4 M Lösung von Chlorwasserstoff in Dioxan gelöst. Nachdem das Reaktionsgemisch 2 h bei RT gerührt worden war, wurden ca. 5 ml Methanol hinzugefügt und anschließend alles Flüchtige am Rotationsverdampfer entfernt. 85 mg des so erhaltenen Rohprodukts wurden mittels präparativer HPLC (Methode 36) gereinigt. Nach Vereinigung der Produktfraktionen, Eindampfen und Trocknen im Hochvakuum wurden 31 mg der Titelverbindung sowie 34 mg des korrespondierenden Methylesters (siehe Beispiel 152) erhalten. Das restliche Rohprodukt wurde mittels MPLC über ca. 30 g Kieselgel mit Ethylacetat als Laufmittel gereinigt. Hier wurden nach Vereinigung der Produktfraktionen, Eindampfen und Trocknen im Hochvakuum 301 mg der Titelverbindung sowie 387 mg des korrespondierenden Methylesters erhalten. Insgesamt wurden so 332 mg (39% d. Th.) der Titelverbindung gewonnen.

¹H-NMR (400 MHz, DMSO-d₆, δ/ppm): 12.62 (breit, 1H), 10.30 (s, 1H), 9.04 (s, 1H), 8.48 (d, 1H), 8.33 (s, 1H), 8.18-8.16 (m, 2H), 8.08 (s, 1H), 7.89 (d, 1H), 7.49-7.47 (m, 2H), 7.41 (dd, 1H), 7.39 (s, 1H), 6.30 (s, 1H), 3.87 (s, 2H), 2.30 (s, 3H), 2.26 (s, 3H).

LC/MS (Methode 3, ESIpos): Rₜ = 0.95 min, m/z = 592 [M+H]⁺.

### Beispiel 152

### Methyl-[3-({2,4-dimethyl-5-[6-(pyridin-3-yl)-1H-imidazo[1,2-b]pyrazol-1-yl]phenyl}carbamoyl)-5-(pentafluor-λ⁶-sulfanyl)phenyl]acetat

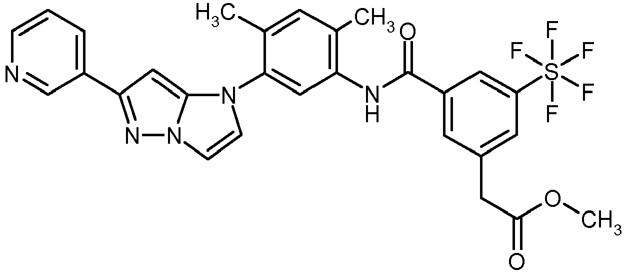

920 mg (1.42 mmol) der Verbindung aus Beispiel 150 wurden in 18 ml (71 mmol) einer 4 M Lösung von Chlorwasserstoff in Dioxan gelöst. Nachdem das Reaktionsgemisch 2 h bei RT gerührt worden war, wurden ca. 5 ml Methanol hinzugefügt und anschließend alles Flüchtige am Rotationsverdampfer entfernt. 85 mg des so erhaltenen Rohprodukts wurden mittels präparativer HPLC (Methode 36) gereinigt. Nach Vereinigung der Produktfraktionen, Eindampfen und Trocknen im Hochvakuum wurden 34 mg der Titelverbindung sowie 31 mg der korrespondierenden Carbonsäure (siehe Beispiel 151) erhalten. Das restliche Rohprodukt wurde mittels MPLC über ca. 30 g Kieselgel mit Ethylacetat als Laufmittel gereinigt. Hier wurden nach Vereinigung der Produktfraktionen, Eindampfen und Trocknen im Hochvakuum 387 mg der Titelverbindung sowie 301 mg der korrespondierenden Carbonsäure erhalten. Insgesamt wurden so 421 mg (48% d. Th.) der Titelverbindung gewonnen.

¹H-NMR (400 MHz, CDCl₃, δ/ppm): 9.03 (s, 1H), 9.52 (d, 1H), 8.20 (s, 1H), 8.17 (s, 1H), 8.15 (s, 1H), 7.97 (s, 1H), 7.93-7.86 (m, 3H), 7.49 (d, 1H), 7.32 (dd, 1H), 6.94 (d, 1H), 6.00 (s, 1H), 3.79 (s, 2H), 3.75 (s, 3H), 2.39 (s, 3H), 2.28 (s, 3H).

LC/MS (Methode 3, ESIpos): Rₜ = 1.05 min, m/z = 606 [M+H]⁺.

### Beispiel 153

### 3-(2-Amino-2-oxoethyl)-N-{2,4-dimethyl-5-[6-(pyridin-3-yl)-1H-imidazo[1,2-b]pyrazol-1-yl]-phenyl}-5-(pentafluor-λ⁶-sulfanyl)benzamid

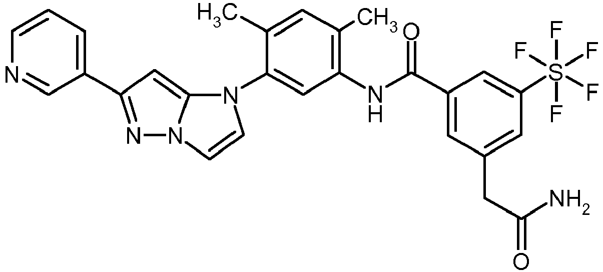

85 mg (0.128 mmol) der Verbindung aus Beispiel 151 und 58 mg (0.154 mmol) HATU wurden in 2 ml DMF vorgelegt und anschließend mit 67 µl (0.384 mmol) *N*,*N-*Diisopropylethylamin und 1.3 ml (0.640 mmol) einer 0.5 M Lösung von Ammoniak in THF versetzt. Nachdem das Reaktionsgemisch ca. 16 h bei RT gerührt worden war, wurde der komplette Ansatz mittels präparativer HPLC in seine Komponenten aufgetrennt (Methode 36). Die Produktfraktionen wurden vereinigt und am Rotationsverdampfer zur Trockene eingeengt. Zur weiteren Aufreinigung wurde das Produkt 10 min bei RT mit wenigen ml Pentan/Diisopropylether 4:1 verrührt. Der Feststoff wurde abgetrennt und im Hochvakuum getrocknet. Es wurden 9 mg (11% d. Th., 95% Reinheit) der Titelverbindung erhalten.

¹H-NMR (400 MHz, DMSO-d₆, δ/ppm): 10.33 (s, 1H), 9.04 (s, 1H), 8.48 (d, 1H), 8.33 (s, 1H), 8.18 (d, 1H), 8.14 (s, 1H), 8.04 (s, 1H), 7.89 (d, 1H), 7.63 (s, 1H), 7.49-7.48 (m, 2H), 7.41 (dd, 1H), 7.39 (s, 1H), 7.06 (s, 1H), 6.31 (s, 1H), 3.64 (s, 2H), 2.29 (s, 3H), 2.26 (s, 3H).

LC/MS (Methode 3, ESIpos): Rₜ = 0.88 min, m/z = 591 [M+H]⁺.

### Beispiel 154

### N-{2,4-Dimethyl-5-[6-(pyridin-3-yl)-1H-imidazo[1,2-b]pyrazol-1-yl]phenyl}-3-(2-hydroxy-2-methylpropyl)-5-(pentafluor-λ⁶-sulfanyl)benzamid

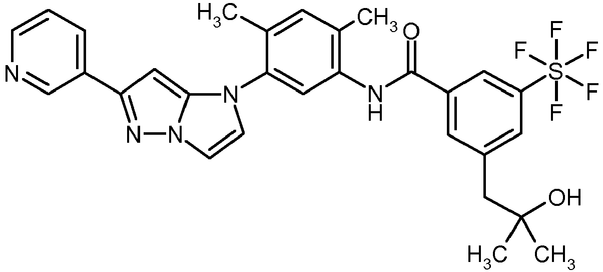

Unter Argon und bei 0°C wurde eine Lösung von 100 mg (0.165 mmol) der Verbindung aus Beispiel 152 in 3 ml wasserfreiem THF mit 661 µl (0.661 mmol) einer 1 M Lösung von Methylmagnesiumbromid in THF versetzt. Das Eis/Wasser-Bad wurde entfernt und das Rühren bei RT fortgesetzt. Nach ca. 16 h wurden 0.5 ml gesättigte wässrige Ammoniumchlorid-Lösung zum Reaktionsgemisch gegeben. Es wurde einige Minuten nachgerührt und dann mit ca. 20 ml Ethylacetat verdünnt. Unter Rühren wurde festes Magnesiumsulfat zugesetzt. Dann wurde filtriert und das Filtrat zur Trockene eingedampft. Der erhaltene Rückstand wurde in 5 ml Pentan aufgenommen und mit einigen Tropfen Diisopropylether versetzt. Der Feststoff wurde in dieser Mischung 10 min bei RT verrührt. Danach wurde abgesaugt, mit Pentan nachgewaschen und im Hochvakuum getrocknet. Es wurden 60 mg (57% d. Th., 95% Reinheit) der Titelverbindung erhalten.

¹H-NMR (400 MHz, CDCl₃, δ/ppm): 9.00 (s, 1H), 8.50 (d, 1H), 8.16 (s, 1H), 8.14 (dt, 1H), 8.02 (s, 1H), 7.94 (s, 1H), 7.91 (s, 1H), 7.82 (s, 1H), 7.47 (d, 1H), 7.31 (dd, 1H), 7.25 (s, 1H), 6.93 (d, 1H), 5.97 (s, 1H), 2.90 (s, 2H), 2.37 (s, 3H), 2.27 (s, 3H), 1.27 (s, 6H).

LC/MS (Methode 3, ESIpos): Rₜ = 1.04 min, m/z = 606 [M+H]⁺.

### Beispiel 155

### N-{2,4-Dimethyl-5-[6-(pyridin-3-yl)-1H-imidazo[1,2-b]pyrazol-1-yl]phenyl}-3-[(2-methoxyethoxy)-methyl]-5-(pentafluor-λ⁶-sulfanyl)benzamid

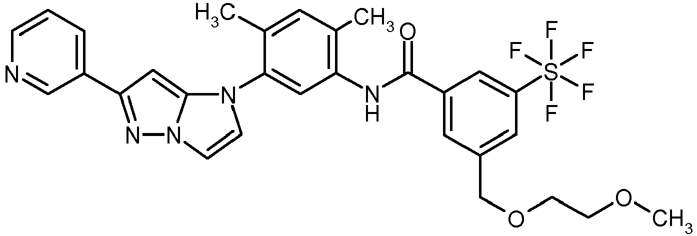

58 mg (0.173 mmol) der Verbindung aus Beispiel 98A und 75 mg (0.198 mmol) HATU wurden in 1 ml DMF gelöst und langsam mit 24 mg (0.198 mmol) 4*-N,N-*Dimethylaminopyridin (DMAP) versetzt. Danach wurden 50 mg (0165 mmol) der Verbindung aus Beispiel 11A hinzugefügt. Das Reaktionsgemisch wurde ca. 16 h bei RT gerührt und dann mittels präparativer HPLC in seine Komponenten aufgetrennt (Methode 36). Nach Vereinigen der Produktfraktionen, Eindampfen und Trocknen des Rückstands im Hochvakuum wurden 76 mg (75% d. Th.) der Titelverbindung erhalten.

¹H-NMR (400 MHz, DMSO-d₆, δ/ppm): 10.33 (s, 1H), 9.06 (d, 1H), 8.50 (dd, 1H), 8.35 (s, 1H), 8.24-8.21 (m, 2H), 8.07 (s, 1H), 7.89 (d, 1H), 7.50-7.48 (m, 2H), 7.46 (dd, 1H), 7.39 (s, 1H), 6.32 (s, 1H), 4.70 (s, 2H), 3.65 (dd, 2H), 3.52 (dd, 2H), 3.26 (s, 3H), 2.30 (s, 3H), 2.26 (s, 3H).

LC/MS (Methode 3, ESIpos): Rₜ = 1.07 min, m/z = 622 [M+H]⁺.

### Beispiel 156

### 3-Cyano-N-{2-hydroxy-4-methyl-3-[6-(pyridin-3-yl)-1H-imidazo[1,2-b]pyrazol-1-yl]phenyl}-5-(pentafluor-λ⁶-sulfanyl)benzamid

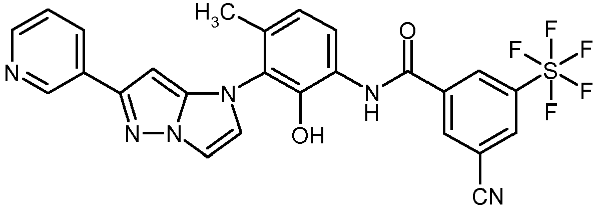

Analog zu dem unter Beispiel 155 beschriebenen Verfahren wurden 37 mg (0.109 mmol) der Verbindung aus Beispiel 82A und 28 mg (0.104 mmol) der Verbindung aus Beispiel 23A zu 9 mg (15% d. Th.) der Titelverbindung umgesetzt. Die präparative HPLC-Reinigung erfolgte hier nach Methode 37.

¹H-NMR (400 MHz, DMSO-d₆, δ/ppm): 10.39 (s, 1H), 9.62 (s, 1H), 9.10 (s, 1H), 8.83 (s, 1H), 8.73-8.72 (m, 2H), 8.56 (d, 1H), 8.41 (d, 1H), 7.86 (d, 1H), 7.62 (dd, 1H), 7.45 (d, 1H), 7.36 (d, 1H), 6.96 (d, 1H), 6.27 (s, 1H), 2.10 (s, 3H).

LC/MS (Methode 3, ESIpos): Rₜ = 1.00 min, m/z = 561 [M+H]⁺.

### Beispiel 157

### N-{4-Chlor-2-methyl-5-[6-(pyridin-3-yl)-1H-imidazo[1,2-b]pyrazol-1-yl]phenyl}-3-(pentafluor-λ⁶-sulfanyl)benzamid

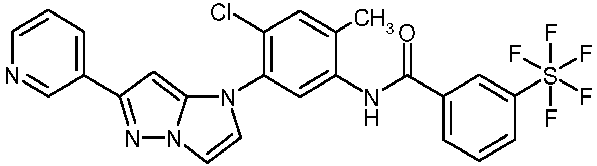

In Analogie zu Beispiel 90 wurden aus 330 mg (1.02 mmol) der Verbindung aus Beispiel 84A und 278 mg (0.63 mmol) 3-(Pentafluor-λ⁶-sulfanyl)benzoesäure nach 16 h Rühren bei 50°C und HPLC-Reinigung (Methode 16) 57.2 mg (10% d. Th.) der Titelverbindung erhalten.

¹H-NMR (400 MHz, DMSO-d₆, δ/ppm): 10.37 (s, 1H), 9.01 (d, 1H), 8.46 (dd, 1H), 8.38 (t, 1H), 8.25 (d, 1H), 8.11-8.18 (m, 3H), 7.90 (d, 1H), 7.79 (t, 1H), 7.74 (s, 1H), 7.68 (s, 1H), 7.51 (d, 1H), 7.39 (dd, 1H), 6.32 (s, 1H), 2.31 (s, 3H).

LC/MS (Methode 7, ESIpos): Rₜ = 1.30 min, m/z = 554/556 [M+H]⁺.

### Beispiel 158

### N-{3-[7-Fluor-6-(pyridin-3-yl)-1H-imidazo[1,2-b]pyrazol-1-yl]-4-methylphenyl}-3-(pentafluor-λ⁶-sulfanyl)benzamid

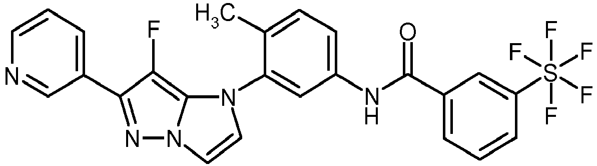

Analog zu dem unter Beispiel 126 beschriebenen Verfahren wurden aus 60 mg (0.195 mmol) der Verbindung aus Beispiel 83A und 48 mg (0.195 mmol) 3-(Pentafluor-λ⁶-sulfanyl)benzoesäure 35 mg (33% d. Th.) der Titelverbindung erhalten. Das aus der präparativen HPLC-Reinigung gewonnene Produkt wurde in wenig Methanol gelöst und über eine Hydrogencarbonat-Kartusche gegeben (Fa. Polymerlabs, Stratospheres SPE, PL-HCO₃ MP SPE, Kapazität 0.9 mmol). Nach Eindampfen des Eluats wurde der Rückstand im Hochvakuum getrocknet.

¹H-NMR (400 MHz, DMSO-d₆, δ/ppm): 8.98 (m, 1H), 8.55 (dd, 1H), 8.41 (m, 1H), 8.28 (d, 1H), 8.17-8.12 (m, 2H), 7.98 (m, 1H), 7.94 (d, 1H), 7.84-7.78 (m, 2H), 7.61 (d, 1H), 7.51-7.47 (m, 2H), 2.30 (s, 3H).

LC/MS (Methode 3, ESIpos): Rₜ = 1.12 min, m/z = 538 [M+H]⁺.

### Beispiel 159

### 3-Cyano-N-{3-[7-fluor-6-(pyridin-3-yl)-1H-imidazo[1,2-b]pyrazol-1-yl]-4-methylphenyl}-5-(pentafluor-λ⁶-sulfanyl)benzamid

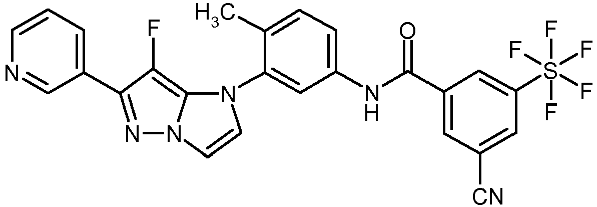

Analog zu dem unter Beispiel 126 beschriebenen Verfahren wurden aus 60 mg (0.195 mmol) der Verbindung aus Beispiel 83A und 53 mg (0.195 mmol) der Verbindung aus Beispiel 23A 61 mg (55% d. Th.) der Titelverbindung erhalten. Das aus der präparativen HPLC-Reinigung gewonnene Produkt wurde in wenig Methanol gelöst und über eine Hydrogencarbonat-Kartusche gegeben (Fa. Polymerlabs, Stratospheres SPE, PL-HCO₃ MP SPE, Kapazität 0.9 mmol). Nach Eindampfen des Eluats wurde der Rückstand im Hochvakuum getrocknet.

¹H-NMR (400 MHz, DMSO-d₆, δ/ppm): 10.79 (s, 1H), 8.99 (d, 1H), 8.84 (dd, 1H), 8.74 (s, 1H), 8.66 (t, 1H), 8.55 (dd, 1H), 8.13 (dt, 1H), 7.96 (d, 1H), 7.94 (d, 1H), 7.78 (dd, 1H), 7.61 (d, 1H), 7.51-7.48 (m, 2H), 2.31 (s, 3H).

LC/MS (Methode 3, ESIpos): Rₜ = 1.12 min, m/z = 563 [M+H]⁺.

### Beispiel 160

### N-{3-[7-Fluor-6-(pyridin-3-yl)-1H-imidazo[1,2-b]pyrazol-1-yl]-4-methylphenyl}-3-(2-hydroxypropan-2-yl)-5-(pentafluor-λ⁶-sulfanyl)benzamid

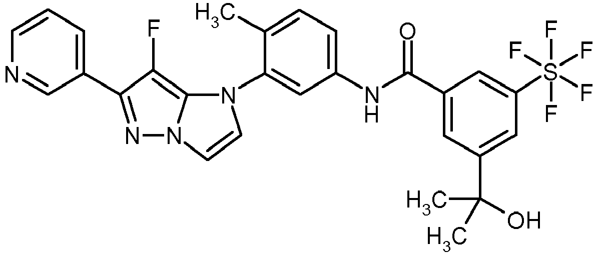

60 mg (0.195 mmol) der Verbindung aus Beispiel 83A und 60 mg (0.195 mmol) der Verbindung aus Beispiel 19A wurden in 2 ml DMF gelöst und nacheinander mit 89 mg (0.234 mmol) HATU und 41 µl (0.234 mmol) *N,N-*Diisopropylethylamin versetzt. Nach ca. 16 h Rühren bei RT wurde das Reaktionsgemisch mit 1 ml Acetonitril verdünnt und mittels präparativer HPLC in seine Komponenten aufgetrennt (Methode 36). Die Produktfraktionen wurden vereinigt und am Rotationsverdampfer vom Lösungsmittel befreit. Der Rückstand wurde in wenig Methanol gelöst und die Lösung über eine Hydrogencarbonat-Kartusche gegeben (Fa. Polymerlabs, Stratospheres SPE, PL-HCO₃ MP SPE, Kapazität 0.9 mmol), um das nach der präparativen HPLC erhaltene Formiat-Salz in die freie Base zu überführen. Nach Eindampfen und Trocknen im Hochvakuum wurden 42 mg (35% d. Th.) der Titelverbindung erhalten.

¹H-NMR (400 MHz, DMSO-d₆, δ/ppm): 10.66 (s, 1H), 8.99 (m, 1H), 8.55 (dd, 1H), 8.29 (s, 1H), 8.27 (t, 1H), 8.17 (t, 1H), 8.13 (dt, 1H), 7.97 (d, 1H), 7.94 (dd, 1H), 7.79 (dd, 1H), 7.61 (d, 1H), 7.51-7.47 (m, 2H), 5.52 (s, 1H), 2.30 (s, 3H).

LC/MS (Methode 3, ESIpos): Rₜ = 1.09 min, m/z = 596 [M+H]⁺.

### Beispiel 161

### 3-Brom-N-{4-methyl-3-[6-(pyrazin-2-yl)-1H-imidazo[1,2-b]pyrazol-1-yl]phenyl}-5-(pentafluor-λ⁶-sulfanyl)benzamid

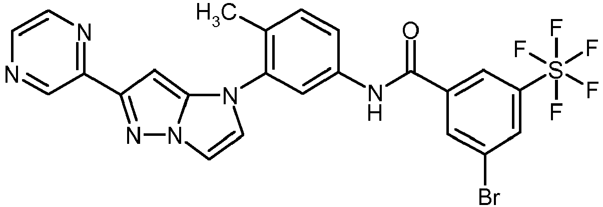

In Analogie zu Beispiel 90 wurde aus 170 mg (0.59 mmol) der Verbindung aus Beispiel 72A und 211 mg (0.64 mmol) der Verbindung aus Beispiel 15A nach 20 h bei RT ein Rohprodukt erhalten, welches nach Chromatographie mittels einer Biotage-Anlage (25 g Snap-Säule; Laufmittelgradient Ethylacetat/Methanol bis auf 8% Methanol steigend) 261 mg (65% d. Th.) der Titelverbindung lieferte.

¹H-NMR (400 MHz, DMSO-d₆, δ/ppm): 10.71 (s, 1H), 9.19 (d, 1H), 8.61 (dd, 1H), 8.53 (d, 1H), 8.49 (s, 1H), 8.37-8.44 (m, 2H), 7.96 (dd, 2H), 7.76 (dd, 1H), 7.64 (d, 1H), 7.47 (d, 1H), 6.39 (s, 1H), 2.29 (s, 3H).

LC/MS (Methode 7, ESIpos): Rₜ = 1.42 min, m/z = 599/601 [M+H]⁺.

### Beispiel 162

### 3-Cyano-N-{4-methyl-3-[6-(pyrazin-2-yl)-1H-imidazo[1,2-b]pyrazol-1-yl]phenyl}-5-(pentafluor-λ⁶-sulfanyl)benzamid

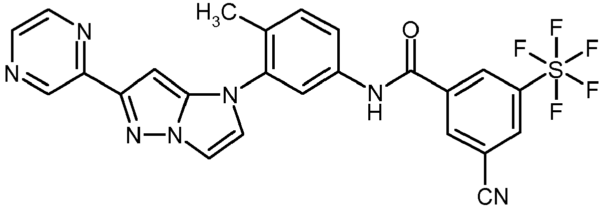

In Analogie zu Beispiel 79 wurden aus 241 mg (0.40 mmol) der Verbindung aus Beispiel 161 nach Aufreinigung mittels HPLC (Methode 16) 58.4 mg (25% d. Th.) der Titelverbindung erhalten.

¹H-NMR (400 MHz, DMSO-d₆, δ/ppm): 10.75 (s, 1H), 9.16 (d, 1H), 8.80 (dd, 1H), 8.71 (s, 1H), 8.61-8.63 (m, 1H), 8.59 (dd, 1H), 8.51 (d, 1H), 7.92-7.96 (m, 2H), 7.73 (dd, 1H), 7.62 (d, 1H), 7.46 (d, 1H), 6.36 (s, 1H), 2.28 (s, 3H).

LC/MS (Methode 7, ESIpos): Rₜ = 1.27 min, m/z = 546 [M+H]⁺.

### Beispiel 163

### N-{4-Methyl-3-[6-(pyrimidin-5-yl)-1H-imidazo[1,2-b]pyrazol-1-yl]phenyl}-3-(pentafluor-λ⁶-sulfanyl)benzamid

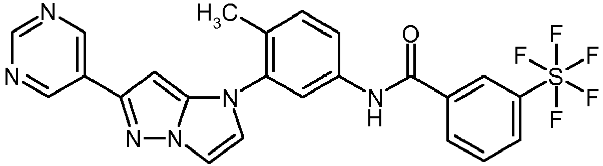

In Analogie zu Beispiel 90 wurde aus 107 mg (0.37 mmol) der Verbindung aus Beispiel 85A und 101 mg (0.41 mmol) 3-(Pentafluor-λ⁶-sulfanyl)benzoesäure nach 20 h bei RT ein Rohprodukt erhalten, welches nach HPLC-Reinigung (Methode 16) 108 mg (54% d. Th.) der Titelverbindung lieferte.

¹H-NMR (400 MHz, DMSO-d₆, δ/ppm): 10.65 (s, 1H), 9.18 (s, 2H), 9.06 (s, 1H), 8.38 (t, 1H), 8.24 (d, 1H), 8.12 (dd, 1H), 7.90-7.94 (m, 2H), 7.74-7.81 (m, 2H), 7.55 (d, 1H), 7.45 (d, 1H), 6.45 (s, 1H), 2.23 (s, 3H).

LC/MS (Methode 7, ESIpos): Rₜ = 1.26 min, m/z = 521 [M+H]⁺.

### Beispiel 164

### N-{2,4-Dimethyl-5-[6-(pyrimidin-5-yl)-1H-imidazo[1,2-b]pyrazol-1-yl]phenyl}-3-(pentafluor-λ⁶-sulfanyl)benzamid

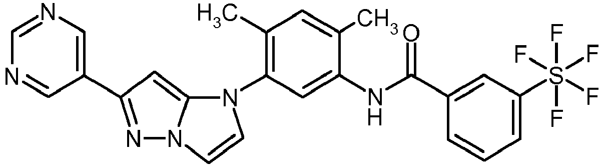

In Analogie zu Beispiel 90 wurden aus 80 mg (0.26 mmol) der Verbindung aus Beispiel 86A und 72 mg (0.29 mmol) 3-(Pentafluor-λ⁶-sulfanyl)benzoesäure nach 20 h Rühren bei RT und HPLC-Reinigung (Methode 16) 75.8 mg (54% d. Th.) der Titelverbindung erhalten.

¹H-NMR (400 MHz, DMSO-d₆, δ/ppm): 10.29 (s, 1H), 9.20 (s, 2H), 9.09 (s, 1H), 8.41 (s, 1H), 8.28 (d, 1H), 8.14 (dd, 1H), 7.92 (d, 1H), 7.80 (t, 1H), 7.47-7.52 (m, 2H), 7.39 (s, 1H), 6.41 (s, 1H), 2.29 (s, 3H), 2.24 (s, 3H).

LC/MS (Methode 7, ESIpos): Rₜ = 1.26 min, m/z = 535 [M+H]⁺.

### Beispiel 165

### 3-Brom-N-{2,4-dimethyl-5-[6-(pyrimidin-5-yl)-1H-imidazo[1,2-b]pyrazol-1-yl]phenyl}-5-(pentafluor-λ⁶-sulfanyl)benzamid

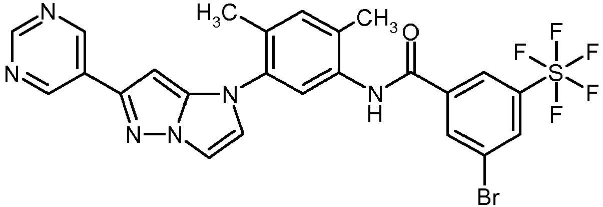

In Analogie zu Beispiel 90 wurde aus 210 mg (0.69 mmol) der Verbindung aus Beispiel 86A und 248 mg (0.76 mmol) der Verbindung aus Beispiel 15A nach 20 h bei RT ein Rohprodukt erhalten, welches nach Chromatographie mittels einer Biotage-Anlage (25 g Snap-Säule; Laufmittelgradient Ethylacetat/Methanol bis auf 8% Methanol steigend) 356 mg (72% d. Th.) der Titelverbindung lieferte.

¹H-NMR (400 MHz, DMSO-d₆, δ/ppm): 10.33 (s, 1H), 9.17 (s, 2H), 9.06 (s, 1H), 8.46 (s, 1H), 8.35-8.40 (m, 2H), 7.90 (d, 1H), 7.48 (d, 1H), 7.46 (s, 1H), 7.36 (s, 1H), 6.38 (s, 1H), 2.26 (s, 3H), 2.21 (s, 3H).

LC/MS (Methode 7, ESIpos): Rₜ = 1.38 min, m/z = 613/615 [M+H]⁺ (⁷⁹Br/⁸¹Br).

### Beispiel 166

### 3-Cyano-N-{2,4-dimethyl-5-[6-(pyrimidin-5-yl)-1H-imidazo[1,2-b]pyrazol-1-yl]phenyl}-5-(pentafluor-λ⁶-sulfanyl)benzamid

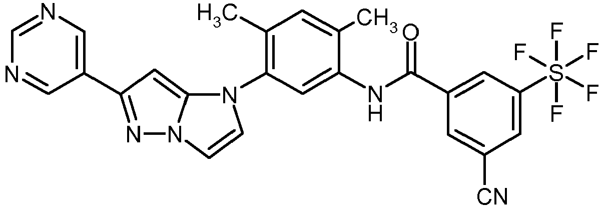

In Analogie zu Beispiel 79 wurden aus 340 mg (0.55 mmol) der Verbindung aus Beispiel 165 nach Aufreinigung mittels HPLC (Methode 16) 117 mg (36% d. Th.) der Titelverbindung erhalten.

¹H-NMR (400 MHz, DMSO-d₆, δ/ppm): 10.40 (s, 1H), 9.19 (s, 2H), 9.09 (s, 1H), 8.83 (t, 1H), 8.72 (s, 1H), 8.65 (s, 1H), 7.92 (d, 1H), 7.49-7.53 (m, 2H), 7.40 (s, 1H), 6.41 (s, 1H), 2.31 (s, 3H), 2.24 (s, 3H).

LC/MS (Methode 6, ESIpos): Rₜ = 1.25 min, m/z = 560 [M+H]⁺.

### Beispiel 167

### 3-Brom-N-{4-methyl-3-[6-(pyridazin-4-yl)-1H-imidazo[1,2-b]pyrazol-1-yl]phenyl}-5-(pentafluor-λ⁶-sulfanyl)benzamid

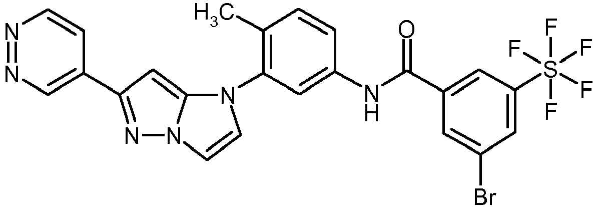

In Analogie zu Beispiel 90 wurde aus 250 mg (0.86 mmol) der Verbindung aus Beispiel 87A und 310 mg (0.95 mmol) der Verbindung aus Beispiel 15A nach 20 h bei RT ein Rohprodukt erhalten, welches nach Chromatographie mittels einer Biotage-Anlage (25 g Snap-Säule; Laufmittelgradient Ethylacetat/Methanol bis auf 8% Methanol steigend) 429 mg (79% d. Th.) der Titelverbindung lieferte.

¹H-NMR (400 MHz, DMSO-d₆, δ/ppm): 10.69 (s, 1H), 9.63 (dd, 1H), 9.16 (dd, 1H), 8.47 (s, 1H), 8.38 (dt, 2H), 7.94-7.99 (m, 2H), 7.90 (d, 1H), 7.77 (dd, 1H), 7.61 (d, 1H), 7.47 (d, 1H), 6.58-6.62 (m, 1H), 2.23 (s, 3H).

LC/MS (Methode 7, ESIpos): Rₜ = 1.34 min, m/z = 599/601 [M+H]⁺ (⁷⁹Br/⁸¹Br).

### Beispiel 168

### 3-Cyano-N-{4-methyl-3-[6-(pyridazin-4-yl)-1H-imidazo[1,2-b]pyrazol-1-yl]phenyl}-5-(pentafluor-λ⁶-sulfanyl)benzamid

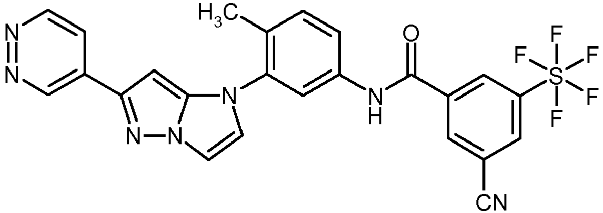

In Analogie zu Beispiel 79 wurden aus 413 mg (0.69 mmol) der Verbindung aus Beispiel 167 nach Aufreinigung mittels HPLC (Methode 16) 39 mg (10% d. Th.) der Titelverbindung erhalten.

¹H-NMR (400 MHz, DMSO-d₆, δ/ppm): 10.77 (s, 1H), 9.63 (dd, 1H), 9.16 (dd, 1H), 8.82 (t, 1H), 8.70 (s, 1H), 8.63 (t, 1H), 7.94-7.99 (m, 2H), 7.90 (d, 1H), 7.77 (dd, 1H), 7.62 (d, 1H), 7.48 (d, 1H), 6.61 (s, 1H), 2.24 (s, 3H).

LC/MS (Methode 7, ESIpos): Rₜ = 1.26 min, m/z = 546 [M+H]⁺.

### Beispiel 169

### N-{2,4-Dimethyl-5-[6-(pyridazin-3-yl)-1H-imidazo[1,2-b]pyrazol-1-yl]phenyl}-3-(pentanuor-λ⁶-sulfanyl)benzamid

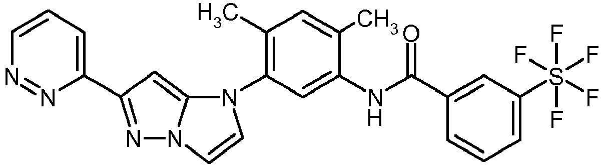

In Analogie zu Beispiel 90 wurden aus 50 mg (0.16 mmol) der Verbindung aus Beispiel 88A und 45 mg (0.18 mmol) 3-(Pentafluor-λ⁶-sulfanyl)benzoesäure nach 20 h Rühren bei RT und HPLC-Reinigung (Methode 16) 61.3 mg (70% d. Th.) der Titelverbindung erhalten.

¹H-NMR (400 MHz, DMSO-d₆, δ/ppm): 10.29 (s, 1H), 9.12 (dd, 1H), 8.41 (s, 1H), 8.28 (d, 1H), 8.10-8.18 (m, 2H), 7.94 (d, 1H), 7.80 (t, 1H), 7.71 (dd, 1H), 7.59 (d, 1H), 7.54 (s, 1H), 7.38 (s, 1H), 6.47 (s, 1H), 2.30 (s, 3H), 2.29 (s, 3H).

LC/MS (Methode 7, ESIpos): Rₜ = 1.24 min, m/z = 535 [M+H]⁺.

### Beispiel 170

### 3-Brom-N-{2,4-dimethyl-5-[6-(pyridazin-3-yl)-1H-imidazo[1,2-b]pyrazol-1-yl]phenyl}-5-(pentafluor-λ⁶-sulfanyl)benzamid

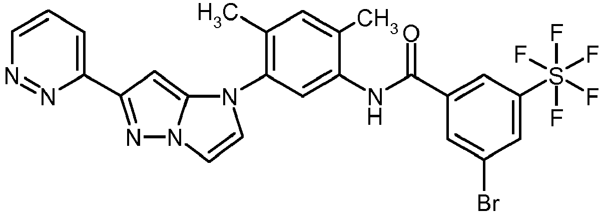

In Analogie zu Beispiel 90 wurde aus 115 mg (0.38 mmol) der Verbindung aus Beispiel 88A und 136 mg (0.42 mmol) der Verbindung aus Beispiel 15A nach 20 h bei RT ein Rohprodukt erhalten, welches nach Chromatographie mittels einer Biotage-Anlage (10 g Snap-Säule; Laufmittelgradient Ethylacetat/Methanol bis auf 8% Methanol steigend) 226 mg (78% d. Th.) der Titelverbindung lieferte.

¹H-NMR (400 MHz, DMSO-d₆, δ/ppm): 10.33 (s, 1H), 9.10 (dd, 1H), 8.46 (s, 1H), 8.37 (d, 2H), 8.12 (dd, 1H), 7.92 (d, 1H), 7.65-7.72 (m, 1H), 7.56 (d, 1H), 7.51 (s, 1H), 7.36 (s, 1H), 6.44 (s, 1H), 2.27 (s, 3H), 2.26 (s, 3H).

LC/MS (Methode 7, ESIpos): R = 1.36 min, m/z = 613/615 [M+H]⁺ (⁷⁹Br/⁸¹Br).

### Beispiel 171

### 3-Cyano-N-{2,4-dimethyl-5-[6-(pyridazin-3-yl)-1H-imidazo[1,2-b]pyrazol-1-yl]phenyl}-5-(pentafluor-λ⁶-sulfanyl)benzamid

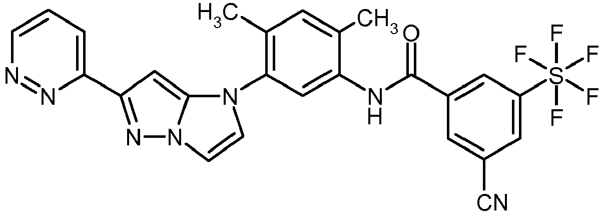

In Analogie zu Beispiel 79 wurden aus 215 mg (0.35 mmol) der Verbindung aus Beispiel 170 nach Aufreinigung mittels HPLC (Methode 16) 76 mg (39% d. Th.) der Titelverbindung erhalten.

¹H-NMR (400 MHz, DMSO-d₆, δ/ppm): 10.40 (s, 1H), 9.12 (dd, 1H), 8.82 (s, 1H), 8.73 (s, 1H), 8.66 (s, 1H), 8.15 (dd, 1H), 7.94 (d, 1H), 7.71 (dd, 1H), 7.59 (d, 1H), 7.57 (s, 1H), 7.39 (s, 1H), 6.47 (s, 1H), 2.31 (s, 3H), 2.29 (s, 3H).

LC/MS (Methode 7, ESIpos): Rₜ = 1.23 min, m/z = 560 [M+H]⁺.

### Beispiel 172

### N-{4-Methyl-3-[6-(1,3-thiazol-5-yl)-1H-imidazo[1,2-b]pyrazol-1-yl]phenyl}-3-(pentafluor-λ⁶-sulfanyl)benzamid

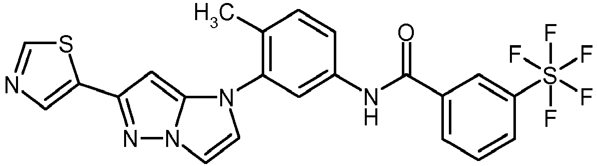

Zu einer Lösung von 100 mg (0.34 mmol) der Verbindung aus Beispiel 89A und 92 mg (0.37 mmol) 3-(Pentafluor-λ⁶-sulfanyl)benzoesäure in 2 ml DMF wurden 193 mg (0.51 mmol) HATU und 62 mg (0.51 mmol) 4-*N*,*N*-Dimethylaminopyridin (DMAP) gegeben. Der Ansatz wurde 20 h bei RT gerührt. Anschließend wurde das Reaktionsgemisch direkt mittels präparativer HPLC gereinigt (Methode 16). Es wurden 50.2 mg (27% d. Th.) der Titelverbindung erhalten.

¹H-NMR (400 MHz, DMSO-d₆, δ/ppm): 10.66 (s, 1H), 8.98 (s, 1H), 8.40 (s, 1H), 8.27 (d, 1H), 8.23 (s, 1H), 8.15 (dd, 1H), 7.93 (d, 1H), 7.87 (d, 1H), 7.78-7.84 (m, 2H), 7.53 (d, 1H), 7.46 (d, 1H), 6.25 (s, 1H), 2.26 (s, 3H).

LC/MS (Methode 7, ESIpos): Rₜ = 1.30 min, m/z = 526 [M+H]⁺.

### Beispiel 173

### 3-Fluor-N-{4-methyl-3-[6-(1,3-thiazol-5-yl)-1H-midazo[1,2-b]pyrazol-1-yl]phenyl}-5-(pentafluor-λ⁶-sulfanyl)benzamid

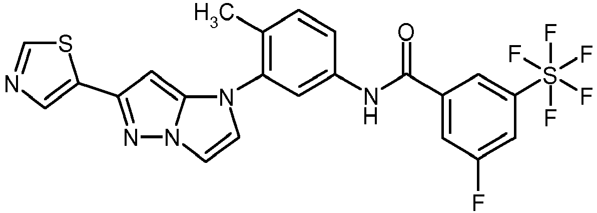

In Analogie zu Beispiel 90 wurden aus 100 mg (0.34 mmol) der Verbindung aus Beispiel 89A und 99 mg (0.37 mmol) 3-Fluor-5-(pentafluor-λ⁶-sulfanyl)benzoesäure nach 20 h Rühren bei RT und HPLC-Reinigung (Methode 16) 27.8 mg (15% d. Th.) der Titelverbindung erhalten.

¹H-NMR (400 MHz, DMSO-d₆, δ/ppm): 10.68 (s, 1H), 8.98 (s, 1H), 8.28 (s, 1H), 8.19-8.24 (m, 2H), 8.17 (d, 1H), 7.92 (d, 1H), 7.87 (d, 1H), 7.78 (dd, 1H), 7.52 (d, 1H), 7.47 (d, 1H), 6.25 (s, 1H), 2.26 (s, 3H).

LC/MS (Methode 7, ESIpos): Rₜ = 1.34 min, m/z = 544 [M+H]⁺.

### Beispiel 174

### 3-Chlor-N-{4-methyl-3-[6-(1,3-thiazol-5-yl)-1H-imidazo[1,2-b]pyrazol-1-yl]phenyl}-5-(pentafluor-λ⁶-sulfanyl)benzamid

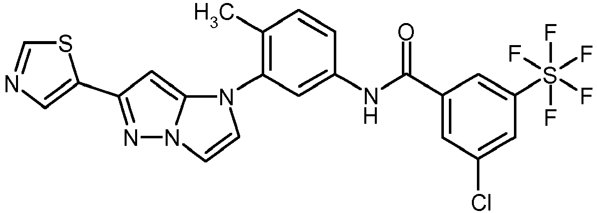

In Analogie zu Beispiel 90 wurden aus 100 mg (0.34 mmol) der Verbindung aus Beispiel 89A und 105 mg (0.37 mmol) der Verbindung aus Beispiel 33A nach 20 h Rühren bei RT und HPLC-Reinigung (Methode 16) 47.2 mg (25% d. Th.) der Titelverbindung erhalten.

¹H-NMR (400 MHz, DMSO-d₆, δ/ppm): 10.71 (s, 1H), 8.98 (s, 1H), 8.35-8.38 (m, 2H), 8.32-8.34 (m, 1H), 8.22 (s, 1H), 7.91 (d, 1H), 7.87 (d, 1H), 7.78 (dd, 1H), 7.53 (s, 1H), 7.47 (d, 1H), 6.25 (s, 1H), 2.26 (s, 3H).

LC/MS (Methode 7, ESIpos): Rₜ = 1.41 min, m/z = 560/562 [M+H]⁺ (³⁵Cl/³⁷Cl).

### Beispiel 175

### 3-Brom-N-{4-methyl-3-[6-(1,3-thiazol-5-yl)-1H-imidazo[1,2-b]pyrazol-1-yl]phenyl}-5-(pentafluor-λ⁶-sulfanyl)benzamid

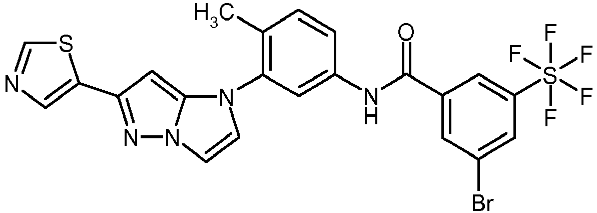

In Analogie zu Beispiel 90 wurde aus 250 mg (0.85 mmol) der Verbindung aus Beispiel 89A und 305 mg (0.93 mmol) der Verbindung aus Beispiel 15A nach 20 h bei RT ein Rohprodukt erhalten, welches nach Chromatographie mittels einer Biotage-Anlage (25 g Snap-Säule; Laufmittelgradient Ethylacetat/Methanol bis auf 8% Methanol steigend) 264 mg (49% d. Th.) der Titelverbindung lieferte.

¹H-NMR (400 MHz, DMSO-d₆, δ/ppm): 10.69 (s, 1H), 8.96 (s, 1H), 8.46 (s, 1H), 8.38-8.41 (m, 1H), 8.35-8.38 (m, 1H), 8.20 (s, 1H), 7.88 (d, 1H), 7.85 (d, 1H), 7.76 (dd, 1H), 7.51 (d, 1H), 7.45 (d, 1H), 6.23 (s, 1H), 2.23 (s, 3H).

LC/MS (Methode 7, ESIpos): Rₜ = 1.42 min, m/z = 604/606 [M+H]⁺ (⁷⁹Br/⁸¹Br).

### Beispiel 176

### 3-(Methylsulfonyl)-N-{4-methyl-3-[6-(1,3-thiazol-5-yl)-1H-imidazo[1,2-b]pyrazol-1-yl]phenyl}-5-(pentafluor-λ⁶-sulfanyl)benzamid

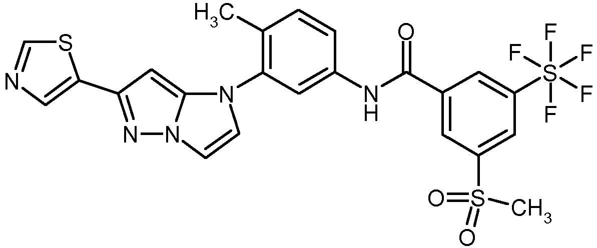

Zu einer Lösung von 100 mg (0.34 mmol) der Verbindung aus Beispiel 89A und 122 mg (0.37 mmol) der Verbindung aus Beispiel 32A in 2 ml DMF wurden 193 mg (0.51 mmol) HATU und 62 mg (0.51 mmol) 4-*N,N*-Dimethylaminopyridin (DMAP) gegeben. Der Ansatz wurde 20 h bei RT gerührt. Anschließend wurde das Reaktionsgemisch direkt mittels präparativer HPLC gereinigt (Methode 16). Es wurden 86.4 mg (42% d. Th.) der Titelverbindung erhalten.

¹H-NMR (400 MHz, DMSO-d₆, δ/ppm): 10.91 (s, 1H), 8.98 (d, 1H), 8.79 (s, 1H), 8.74 (t, 1H), 8.55 (t, 1H), 8.22 (d, 1H), 7.92 (d, 1H), 7.87 (dd, 1H), 7.80 (dd, 1H), 7.53 (d, 1H), 7.49 (d, 1H), 6.25 (s, 1H), 3.45 (s, 3H), 2.27 (s, 3H).

LC/MS (Methode 7, ESIpos): Rₜ = 1.23 min, m/z = 604 [M+H]⁺.

### Beispiel 177

### 3-Cyano-N-{4-methyl-3-[6-(1,3-thiazol-5-yl)-1H-imidazo[1,2-b]pyrazol-1-yl]phenyl}-5-(pentafluor-λ⁶-sulfanyl)benzamid

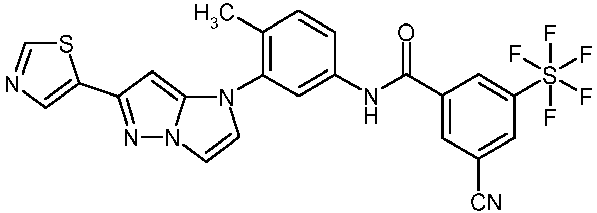

In Analogie zu Beispiel 79 wurden aus 260 mg (0.43 mmol) der Verbindung aus Beispiel 175 nach Aufreinigung mittels HPLC (Methode 16) 57.2 mg (23% d. Th.) der Titelverbindung erhalten.

¹H-NMR (400 MHz, DMSO-d₆, δ/ppm): 10.77 (s, 1H), 7.91 (d, 1H), 8.98 (s, 1H), 8.81-8.85 (m, 1H), 8.73 (s, 1H), 8.63-8.68 (m, 1H), 8.22 (s, 1H), 7.87 (d, 1H), 7.78 (dd, 1H), 7.53 (d, 1H), 7.49 (d, 1H), 6.24 (s, 1H), 2.26 (s, 3H).

LC/MS (Methode 7, ESIpos): Rₜ = 1.29 min, m/z = 551 [M+H]⁺.

### Beispiel 178

### 3-(2-Hydroxypropan-2-yl)-N-{4-methyl-3-[6-(1,3-thiazol-5-yl)-1H-imidazo[1,2-b]pyrazol-1-yl]-phenyl}-5-(pentafluor-λ⁶-sulfanyl)benzamid

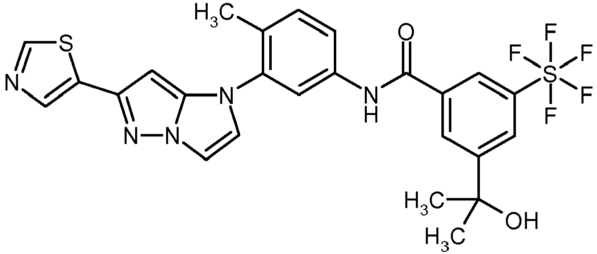

In Analogie zu Beispiel 90 wurden aus 100 mg (0.34 mmol) der Verbindung aus Beispiel 89A und 114 mg (0.37 mmol) der Verbindung aus Beispiel 19A nach 20 h Rühren bei RT und HPLC-Reinigung (Methode 16) 76.2 mg (35% d. Th.) der Titelverbindung erhalten.

¹H-NMR (400 MHz, DMSO-d₆, δ/ppm): 10.63 (s, 1H), 8.98 (s, 1H), 8.25-8.30 (m, 2H), 8.23 (s, 1H), 8.16 (t, 1H), 7.92 (d, 1H), 7.87 (d, 1H), 7.79 (dd, 1H), 7.52 (d, 1H), 7.47 (d, 1H), 6.25 (s, 1H), 5.49-5.52 (m, 1H), 2.25 (s, 3H), 1.50 (s, 6H).

LC/MS (Methode 7, ESIpos): Rₜ = 1.27 min, m/z = 584 [M+H]⁺.

### B. Bewertung der pharmakologischen Wirksamkeit

Die pharmakologische Aktivität der erfindungsgemäßen Verbindungen kann durch *in vitro-* und *in vivo*-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### Abkürzungen und Akronyme:

- Ahx: 6-Aminohexansäure
- ATP: Adenosintriphosphat
- BSA: bovines Serumalbumin
- DMSO: Dimethylsulfoxid
- EDTA: Ethylendiamin-*N,N,N',N*'-tetraessigsäure
- EGTA: Ethylenglykol-bis(aminoethylether)-*N,N,N'N'*-tetraessigsäure
- ELISA: enzyme-linked immunosorbent assay
- FCS: fötales Kälberserum
- GST: Glutathion-*S*-Transferase
- HEPES: 4-(2-Hydroxyethyl)piperazin-1-ethansulfonsäure
- HRP: Meerrettichperoxidase
- HUVEC: human umbilical vein endothelial cells
- PAGE: Polyacrylamid-Gelelektrophorese
- PBS: Phosphat-gepufferte Kochsalz-Lösung
- PEG: Polyethylenglykol
- PMSF: Phenylmethylsulfonylfluorid
- pTyr: Phosphotyrosin
- SDS: Natriumdodecylsulfat
- Tris: Tris(hydroxymethyl)aminomethan
- VEGF: vascular endothelial growth factor
- v/v: Volumen zu Volumen-Verhältnis (einer Lösung)
- w/v: Gewicht zu Volumen-Verhältnis (einer Lösung)

### B-1. Tie2-Kinase-Assay:

Die Tie2-inhibitorische Aktivität der erfindungsgemäßen Substanzen wurde mit Hilfe eines in den folgenden Abschnitten beschriebenen Tie2-TR-FRET-Assays bei einer ATP-Konzentration von 1 mM bestimmt (TR-FRET = *Time-Resolved Fluorescence Resonance Energy Transfer*):
Als Enzym wurde ein rekombinantes Fusionsprotein aus Glutathion-*S*-Transferase (GST) und der intrazellulären Domäne von humanem Tie2 (Aminosäuren 776-1124) verwendet, das in Baculovirusinfizierten Insektenzellen (Hi5) exprimiert und über Affinitätschromatographie an Glutathion-Sepharose gereinigt wurde. Alternativ kann auch kommerziell verfügbares GST-His6-Tie2-Fusionsprotein (ProQinase GmbH, Freiburg im Breisgau) verwendet werden. Als Substrat für die Kinasereaktion wurde das biotinylierte Peptid Biotin-Ahx-EPKDDAYPLYSDFG (C-Terminus in Amid-Form) verwendet, das kommerziell erhältlich ist (z.B. Firma Biosyntan, Berlin).

Für den Assay wurden 50 nl einer 100-fach konzentrierten Lösung der jeweiligen Testsubstanz in DMSO in eine schwarze *low-volume 384-well*-Mikrotiterplatte (Greiner Bio-One, Frickenhausen) pipettiert. Es wurden 2 µl einer Lösung des Tie2-Fusionsproteins in Assaypuffer [50 mM HEPES/ HCl pH 7, 10 mM MgCl₂, 2.5 mM MnCl₂, 1 mM Dithiothreitol, 0.01% (v/v) Nonidet P-40, 0.1% (w/v) bovines Serumalbumin (BSA), 1 x *Complete EDTA-free* Protease-Inhibitorengemisch (Roche)] hinzugegeben, und die Mischung wurde für 15 min inkubiert, um eine Vorbindung der Substanz an das Enzym vor der Kinasereaktion zu ermöglichen. Dann wurde die Kinasereaktion gestartet durch Zugabe von 3 µl einer Lösung von Adenosintriphosphat (ATP, 1.67 mM → Endkonzentration in 5 µl Assayvolumen = 1 mM) und Substrat (1.67 µM → Endkonzentration in 5 µl Assayvolumen = 1 µM) in Assaypuffer, und die resultierende Mischung wurde für eine Reaktionszeit von 60 min bei 22°C inkubiert. Die Konzentration des Tie2-Fusionsproteins wurde an die jeweilige Aktivität des Enzyms angepasst und so eingestellt, dass der Assay im linearen Bereich erfolgte. Typische Konzentrationen lagen im Bereich von 30 ng/ml.

Die Reaktion wurde gestoppt durch Zugabe von 5 µl einer Lösung von TR-FRET-Detektionsreagentien [200 nM Streptavidin-XL665 und 2 nM PT66-Eu-Chelat, ein Europiumchelat-markierter anti-Phosphotyrosin-Antikörper (Perkin-Elmer); alternativ kann auch PT66-Tb-Kryptat (Cisbio Bioassays, Codolet, Frankreich) verwendet werden] in wässriger EDTA-Lösung [90 mM EDTA, 0.28% (w/v) bovines Serumalbumin (BSA) in 50 mM HEPES pH 7.5]. Die resultierende Mischung wurde 1 h bei 22°C inkubiert, um die Bildung des Komplexes aus dem biotinylierten phosphorylierten Substrat und den Detektionsreagentien zu ermöglichen. Anschließend wurde die Menge des phosphorylierten Substrats bestimmt durch Messung des Resonanzenergietransfers vom PT66-Eu-Chelat zum Streptavidin-XL665. Hierzu wurden in einem TR-FRET-Messgerät (z.B. Viewlux, Perkin-Elmer) die Fluoreszenz-Emissionen bei 620 nm und 665 nm nach Anregung bei 350 nm gemessen. Das Verhältnis der Emissionen bei 665 nm und bei 620 nm wurde als Maß für die Menge des phosphorylierten Substrates genommen. Die so erhaltenen Daten wurden normalisiert (Enzymreaktion ohne Inhibitor = 0% Inhibition; alle anderen Assaykomponenten, jedoch kein Enzym = 100% Inhibition).

Üblicherweise wurde die jeweilige Testsubstanz auf derselben Mikrotiterplatte bei zehn verschiedenen Konzentrationen im Bereich von 20 µM bis 0.073 nM (z.B. bei 20 µM, 5.7 µM, 1.6 µM, 0.47 µM, 0.13 µM, 38 nM, 11 nM, 3.1 nM, 0.89 nM, 0.25 nM und 0.073 nM) in Doppelwerten für jede Konzentration getestet. Die Verdünnungsreihen wurden vor dem Assay auf der Ebene der 100-fach konzentrierten Lösung durch serielle Verdünnungen hergestellt (die exakten Konzentrationen können variieren in Abhängigkeit von den jeweils verwendeten Pipettoren). IC₅₀-Werte wurden mit einem 4-Parameter-Fit berechnet, wofür eine *inhouse*-Software verwendet wurde.

In der folgenden Tabelle 1 sind für individuelle Ausführungsbeispiele die IC₅₀-Werte aus diesem Assay aufgeführt (zum Teil als Mittelwerte aus mehreren unabhängigen Einzelbestimmungen):

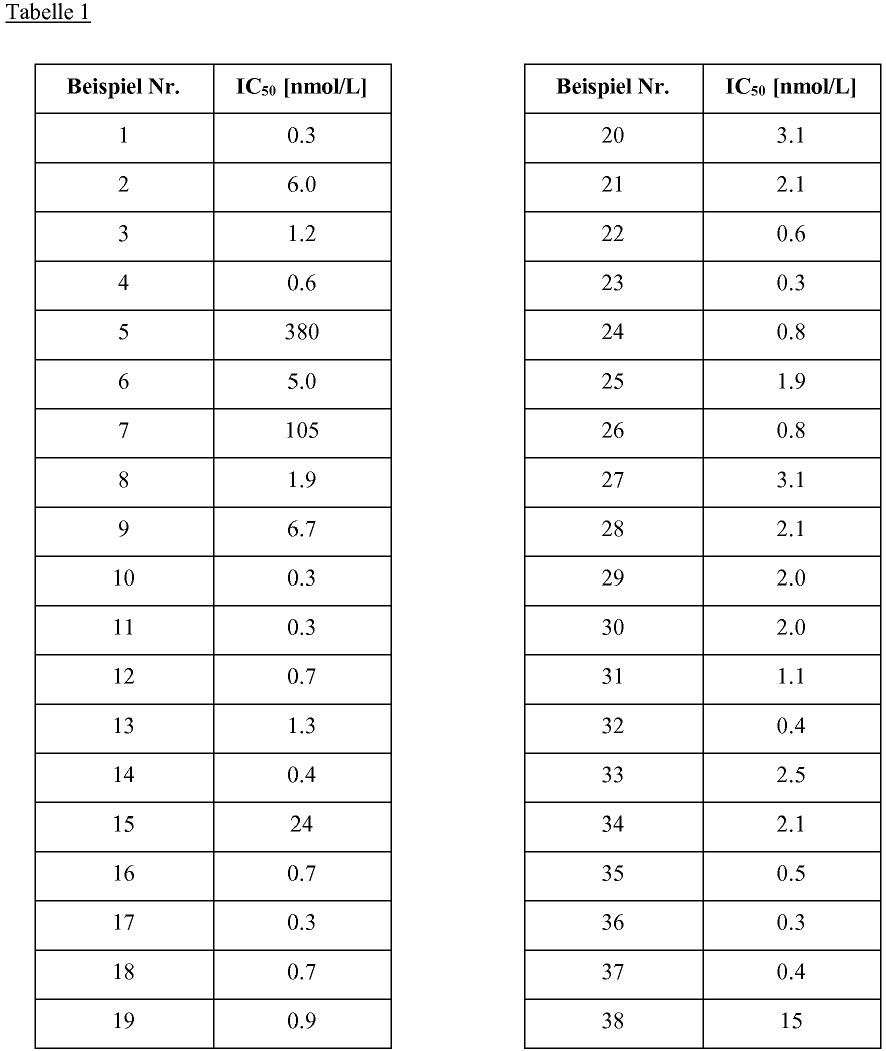

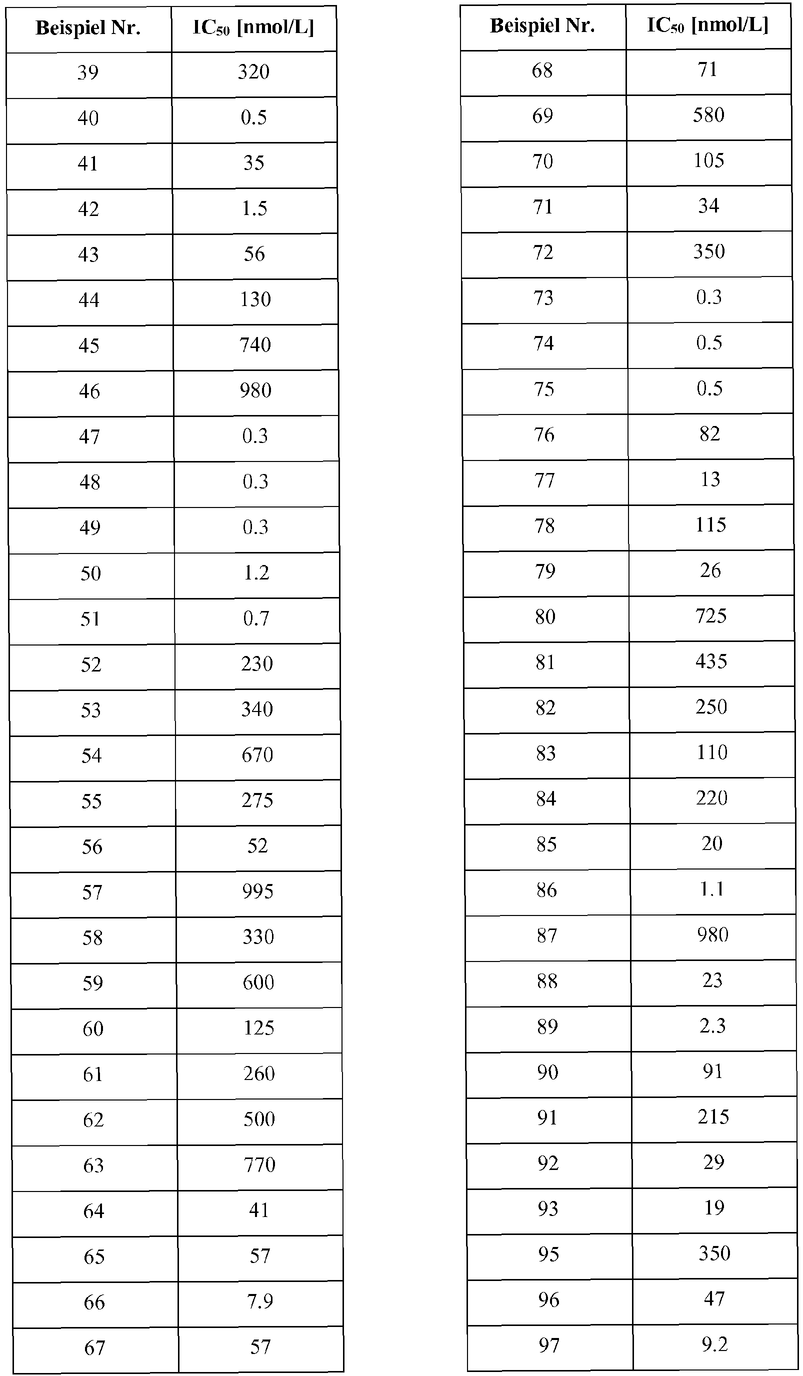

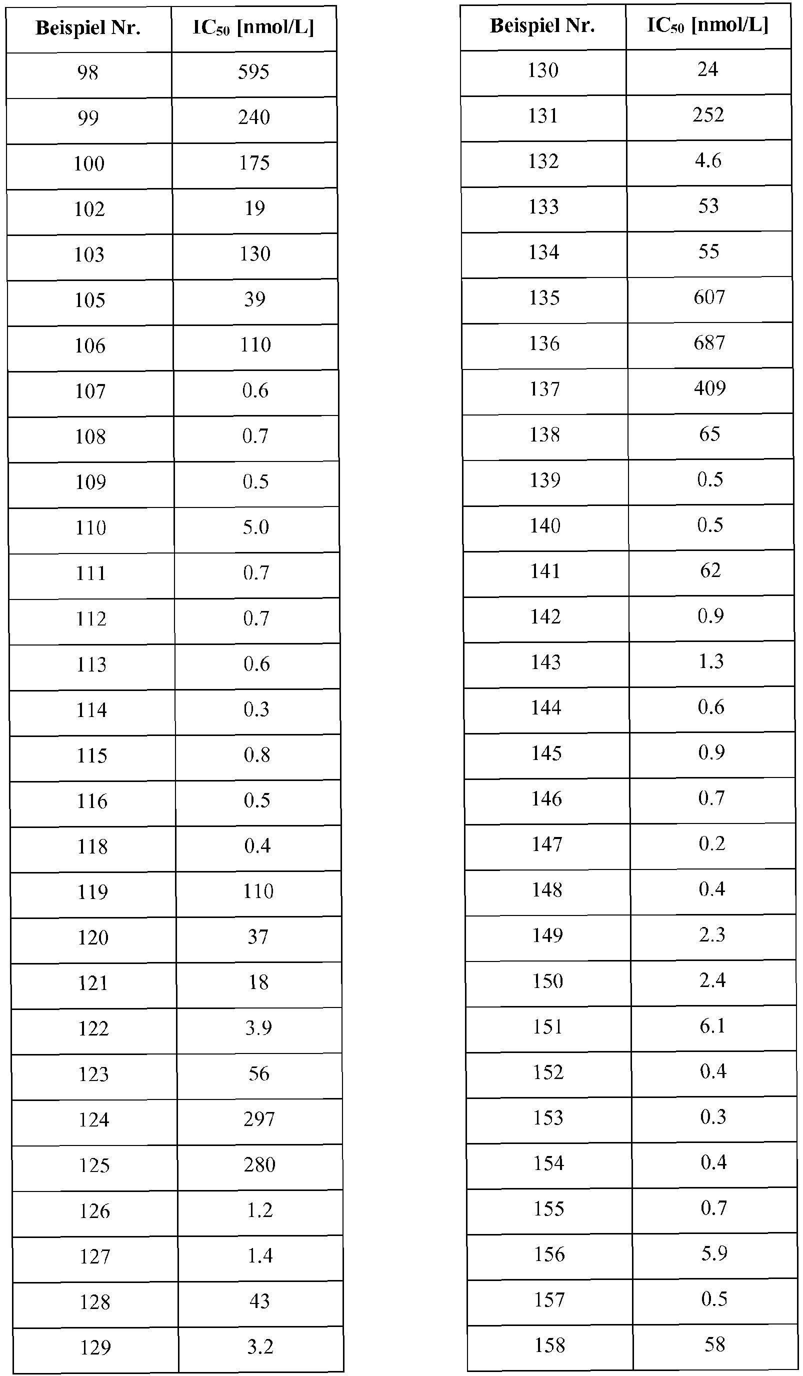

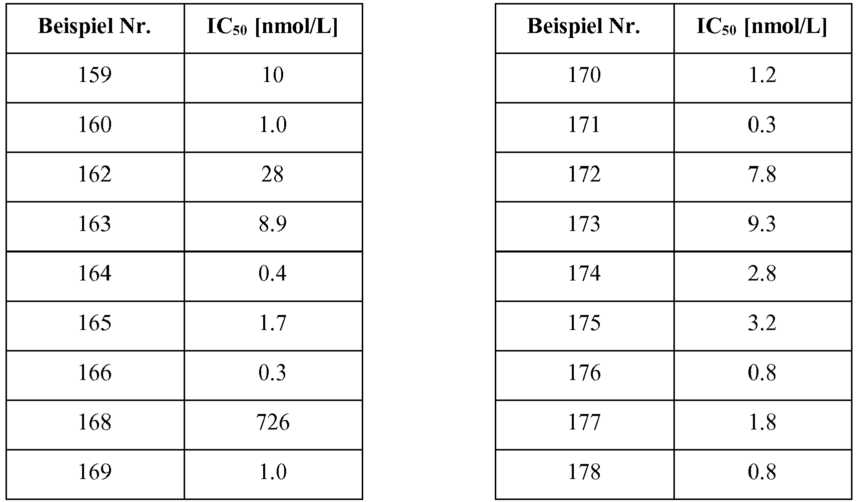

### B-2. Tie2-pTyr-ELISA:

Die zelluläre Aktivität der erfindungsgemäßen Verbindungen als Tie2-Kinaseinhibitoren wurde in humanen Endothelzellen (HUVEC) durch Messung der Hemmung der durch Behandlung mit Natriumorthovanadat erhöhten Autophosphorylierung des endogenen Tie2-Rezeptors mittels Tie2 / Phosphotyrosin-Sandwich-ELISA bestimmt.

### Zellkultur:

Humane Endothelzellen (human umbilical vein endothelial cells, HUVEC) wurden von der Firma Cellsystems (FC-0003) bezogen, in Vasculife VEGF-Vollmedium (Cellsystems, LL-1020) mit 2% fötalem Kälberserum (FCS) bei 37°C / 5% CO₂ kultiviert und für Tie2-ELISA-Messungen bis Passage 8 verwendet.

### Zellbehandlung:

HUVEC wurden in einem Kulturvolumen von 100 µl Vasculife VEGF-Vollmedium mit einer Zelldichte von 30 000 Zellen pro Vertiefung in transparenten, Collagen-beschichteten 96-well-Zellkulturplatten (Falcon, #353075) ausplattiert und über Nacht im Brutschrank bei 37°C / 5% CO₂ inkubiert. Von den in DMSO gelösten Testsubstanzen wurde jeweils eine Verdünnungsreihe in Vasculife VEGF-Magermedium [Basalmedium mit Lifefactors (Cellsystems, LM-0002), ohne FCS, mit 0.1% BSA] ohne Natriumorthovanadat und eine Verdünnungsreihe in Vasculife VEGF-Magermedium mit 8 mM Natriumorthovanadat in den gewünschten Konzentrationen im Bereich von 10 pM bis 10 µM hergestellt, wobei die finale DMSO-Konzentration 1% betrug. Nach Ausschlagen des Vollmediums wurden pro Plattenvertiefung je 100 µl der verdünnten Substanzen in Magermedium ohne Natriumorthovanadat auf die Zellen pipettiert und 10 min im Brutschrank bei 37°C / 5% CO₂ inkubiert. Dann wurden weitere 100 µl der gleichen Substanzverdünnung in Magermedium mit 8 mM Natriumorthovanadat in die jeweilige Plattenvertiefung hinzupipettiert und die Zellen in einem Volumen von nun 200 µl in Gegenwart von 4 mM Natriumorthovanadat bei der gewünschten Substanzkonzentration weitere 20 min im Brutschrank bei 37°C / 5% CO₂ inkubiert. Danach wurde der Zellkulturüberstand der Platten ausgeschlagen und die Zellen pro Vertiefung einmal mit 250 µl kaltem PBS, das 4 mM Natriumorthovanadat enthielt, gewaschen. Dann wurden pro Vertiefung 120 µl Duschl-Lysepuffer [50 mM HEPES pH 7.2, 150 mM NaCl, 1 mM MgCl2, 10% Glycerin, 1.5% Triton X-100, 4 mM Natriumorthovanadat, 250 µl S-PIC Phosphatase-Inhibitor-Cocktail 2 (Sigma, P5726) und 1 Tablette *Complete* Proteinase-Inhibitorengemisch (Roche, #1836145) pro 10 ml] auf die Zellen gegeben. Es wurde kurz geschüttelt, dann 20 min auf Eis inkubiert und anschließend die Lysate in den Zellkulturplatten bei -80°C für mindestens 30 min eingefroren.

### Sandwich-ELISA:

Für die Messung der Autophosphorylierung des endogenen Tie2-Rezeptors in den hergestellten Zelllysaten wurden ein selbst hergestellter, gegen den N-Terminus des Tie2-Rezeptors gerichteter anti-Tie2-Antikörper (1.09 mg/ml) und ein HRP-gekoppelter anti-Phosphotyrosin-Antikörper (Sigma, A4595, Klon pY-20) verwendet. Weiße 96-well-ELISA-Platten (Lumitrac600, Greiner, #655074) wurden pro Vertiefung mit 100 µl einer 1:1000-Verdünnung des anti-Tie2-Antikörpers in Beschichtungspuffer [15 mM Na₂CO₃, 35 mM NaHCO₃, pH 9.6] unter Schütteln bei 4°C über Nacht inkubiert. Die beschichteten ELISA-Platten wurden dreimal mit 250 µl PBST-Puffer [0.1% Tween-20 in PBS] gewaschen, die Vertiefungen mit 250 µl 3% BSA in PBST-Puffer 1-6 h unter Schütteln bei RT geblockt und wiederum dreimal mit 250 µl PBST-Puffer gewaschen. Aus den im Kühlschrank aufgetauten Zelllysatplatten wurden jeweils 100 µl Lysat in die beschichteten Vertiefungen der ELISA-Platten übertragen und über Nacht bei 4°C unter Schütteln inkubiert. Nach dreimaligem Waschen der Platten mit je 250 µl PBST-Puffer wurden 100 µl einer 1:5000-Verdünnung des anti-Phosphotyrosin-HRP-Antikörpers in 3% Prionex (Calbiochem, #529600) in PBST injede Vertiefung pipettiert und lichtgeschützt über Nacht bei 4°C unter Schütteln inkubiert. Nach dreimaligem Waschen der Platten mit je 250 µl PBST wurden 100 µl Chemilumineszenzsubstrat [BM Chemiluminescence ELISA Substrate (POD) Reagent A und B 1:100, Roche, #1582950] injede Vertiefung pipettiert, und nach 3 min wurde mit einem Lumineszenzmessgerät gemessen. Die Mittelwerte und Standardabweichungen einzelner Messungen wurden aus Dreifachbestimmungen mit Microsoft Excel berechnet. Das GraphPad Prism 5-Software-Paket wurde zur Datenanalyse und Ermittlung der IC₅₀-Werte verwendet.

In der folgenden Tabelle 2 sind für repräsentative Ausführungsbeispiele die aus 2-7 unabhängigen Messungen ermittelten IC₅₀-Werte aus diesem Assay aufgeführt:

**Tabelle 2**

| **Beispiel Nr.** | **IC₅₀ [nmol/L]** |
|---|---|
| 2 | 7.6 |
| 3 | 3.4 |
| 4 | 4.7 |
| 6 | 17 |
| 12 | 4.7 |
| 16 | 3.2 |
| 18 | 11 |
| 26 | 3.0 |
| 28 | 3.6 |
| 32 | 2.1 |
| 40 | 2.3 |
| 48 | 6.4 |
| 51 | 8.8 |
| 66 | 46 |
| 73 | 0.8 |
| 74 | 0.6 |
| 75 | 1.7 |
| 77 | 8.8 |
| 85 | 44 |
| 88 | 41 |
| 102 | 18 |
| 107 | 0.9 |
| 112 | 1.4 |
| 116 | 2.9 |
| 157 | 1.0 |
| 162 | 8.9 |

### B-3. Inhibition der Ang1-vermittelten Tie2-Phosphorylierung in vivo:

Zur Bewertung der Wirkung von ausgewählten erfindungsgemäßen Verbindungen auf das Zielprotein *in vivo* wurde in *ex* vivo-Analysen die Hemmung der Phosphorylierung von Tie2 in den Lungen von immundefizienten Mäusen untersucht.

Dazu wurde zum Zeitpunkt 0 je drei Tieren pro Gruppe 50 oder 100 mg/kg der jeweiligen Testsubstanz p.o. appliziert und die Tie2-Phosphorylierung nach 2 h 45 min durch i.v.-Applikation von 12.5 µg Angiopoietin-1 (R&D Systems, Best.-Nr. 923-AN/CF) pro Tier induziert. Nach 15 min wurden die Tiere getötet, die Lungen entnommen und sofort in flüssigem Stickstoff schockgefroren. Die Lungen wurden unter Trockeneiskühlung zerkleinert und mittels Ultra-Turrax (T10 basic, IKA-Werke) in Orthovanadat- und Proteaseinhibitor-haltigem Lysepuffer [50 mM Tris-Cl pH ∼ 8, 150 mM NaCl, 10% Glycerin, 1.5% Triton X-100, 1 mM EGTA, 50 mM NaF, 10 mM Na₄P₂O₇, 4 mM Na₃VO₄, 1% Phosphatase-Inhibitor-Cocktail 2 (Sigma, P5726), 1 mM PMSF, *Complete mini EDTA-free Protease Inhibitor Cocktail Tablet* (Roche, Best.-Nr. 1836170)] dispergiert. Nach einer 20-minütigen Inkubation auf Eis wurden die Lungenhomogenisate bei 4°C und 13 000 rpm 10 min lang zentrifugiert und die Proteinkonzentration der Überstände (Lungenlysate) mittels BCA-Assay (Pierce Thermo Scientific, Best.-Nr. 23225) bestimmt.

Für die Immunpräzipitation wurden 5-8 mg Lysatprotein mit 5 µg eines gegen den humanen Tie2-Rezeptor gerichteten monoklonalen Antikörpers (Anti-human Tie2 mouse monoclonal Ab, USBiological, #T5498-72) gemischt und bei 4°C auf einem Rotator überkopf-rotierend 1 h inkubiert. Dann wurden zur Lysat-Antikörper-Lösung 30 µl gepackte und in Lysepuffer gewaschene Protein G-Sepharose-Kügelchen (Protein G Sepharose 4 Fast Flow, GE Healthcare, Best.-Nr. 17-0618-01) gegeben und unter den gleichen Bedingungen über Nacht weiter inkubiert. Die Protein G-Sepharose-Kügelchen wurden durch Zentrifugation (30 sec) sedimentiert, der Überstand verworfen und die Kügelchen dreimal mit kaltem Lysepuffer gewaschen. Die Kügelchen wurden in je 40-70 µl reduzierendem SDS-PAGE-Probenpuffer [NuPAGE LDS Sample Buffer (4x), Invitrogen, #NP0007, NuPAGE Sample Reducing Agent, Invitrogen, #NP0004] 7 min lang bei 95°C erhitzt, und 15-25 µl jeder Probe pro Gelspur wurden auf einem 4-12% Criterion Gel (Criterion XT Precast Gel, BIO-RAD) aufgetrennt. Die Proteine wurden aus dem Polyacrylamid-Gel mittels einer Trans-Blot Semi-Dry-Apparatur (BIO-RAD Trans-Blot SD Semi-Dry Electrophoretic Transfer Cell, Kat.-Nr. 170-3940) auf eine Nitrozellulosemembran (BIO-RAD Trans-Blot Transfer Medium Pure Nitrocellulose Membrane, Kat.-Nr. 162-0114) transferiert. Zum Blockieren unspezifischer Bindungsstellen wurde die Membran 1 h unter Schütteln bei RT in TBST-Puffer [50 mM Tris-Cl pH 7.5, 150 mM NaCl, 0.05% Tween-20] mit 3% BSA inkubiert.

Zum Nachweis von phosphoryliertem Tie2 wurde die Membran über Nacht bei 4°C mit einer Lösung von 0.5 µg/ml eines Anti-Phospho(Y992)-Tie2-Antikörpers (R&D Systems, Best.-Nr. AF2720) in dem gleichen BSA-haltigen Puffer inkubiert, dreimal mit TBST gewaschen und 1 h bei RT mit einem entsprechenden HRP-gekoppelten Zweitantikörper (Dianova, Best.-Nr. 711-035-152) inkubiert. Nach drei weiteren Waschschritten wurden die Banden, die dem an Tyrosin-992 phosphorylierten Tie2-Protein entsprechen, über Chemilumineszenz (SuperSignal^{®} West Dura Extended Duration Substrate, Pierce Thermo Scientific, #34075) mit dem BIO-RAD Molecular Imager ChemiDoc XRS detektiert. Zum Nachweis von Tie2-Gesamtprotein auf der Blotmembran wurden zunächst die auf der Membran gebundenen Antikörper durch 30-minütiges Erhitzen im Wasserbad auf 50°C in einem Tris-HCl-Puffer (62.5 mM Tris-Cl pH 6.5) mit 2% SDS und 100 mM β-Mercaptoethanol und anschliessendem dreimaligen Waschen entfernt. Die Membranen wurden dann über Nacht bei 4°C mit einem das gesamte Tie2-Protein erkennenden Antikörper (Anti-mouse Tie2 goat polyclonal Ab, R&D Systems, #AF762, 0.2 µg/ml) inkubiert. Nach Waschen und Inkubation mit dem entsprechenden Zweitantikörper (Dianova, #705-035-147) wurden die Tie2-Proteinbanden wie oben beschrieben detektiert. Die Proteinbanden wurden mit der Software des BIO-RAD-Imagers densitometrisch ausgewertet und das Verhältnis von phosphoryliertem zu Gesamt-Tie2-Protein bestimmt.

### B-4. Hemmung des Tumorwachstums in humanen Tumor-Xenograftmodellen:

Zur Bewertung der Substanzwirkung *in vivo* wurden humane Tumor-Xenograftmodelle in immundefizienten Mäusen herangezogen. Dazu wurden Tumorzellen *in vitro* kultiviert und subkutan in nu/nu-Mäusen implantiert. Die Behandlung der Tiere erfolgte durch perorale Applikation der Testsubstanz nach der Etablierung des Tumors. Der Gesundheitszustand der Tiere wurde täglich überprüft, und die Behandlungen erfolgten entsprechend den Tierschutzbestimmungen. Die Tumorfläche wurde mit Schublehren gemessen (Länge L, Breite B = kleinere Ausdehnung). Das Tumorvolumen wurde nach der Formel (L x B²)/2 berechnet. Die Hemmung des Tumorwachstums wurde am Ende des Versuches als T/C-Verhältnis der Tumorflächen bzw. Tumorgewichte und als TGI-Wert (tumor growth inhibition, berechnet nach der Formel [1-(T/C)] x 100) bestimmt (T = Tumorgröße der behandelten Gruppe; C = Tumorgröße der unbehandelten Kontrollgruppe).

### B-5. Bestimmung pharmakokinetischer Kenngrößen nach intravenöser und peroraler Gabe:

Die zu untersuchende Substanz wurde Tieren (z.B. Mäusen oder Ratten) intravenös als Lösung appliziert (z.B. in entsprechendem Plasma mit geringem DMSO-Zusatz oder in einem PEG/ Ethanol/Wasser-Gemisch), die perorale Applikation erfolgte als Lösung (z.B. in Solutol/Ethanol/ Wasser- oder PEG/Ethanol/Wasser-Gemischen) oder als Suspension (z.B. in Tylose) jeweils über eine Schlundsonde. Nach Substanzgabe wurde den Tieren zu festgelegten Zeitpunkten Blut entnommen. Dieses wurde heparinisiert, anschließend wurde daraus durch Zentrifugation Plasma gewonnen. Die Substanz wurde im Plasma über LC-MS/MS analytisch quantifiziert. Aus den so ermittelten Plasmakonzentration-Zeit-Verläufen wurden unter Verwendung eines internen Standards und mit Hilfe eines validierten Rechenprogramms die pharmakokinetischen Kenngrößen berechnet, wie AUC (Fläche unter der Konzentration-Zeit-Kurve), Cₘₐₓ (maximale Plasmakonzentration), t_{1/2} (Halbwertszeit), Vss (Verteilungsvolumen) und CL (Clearance) sowie die absolute und die relative Bioverfügbarkeit F bzw. Fᵣₑₗ (i.v./p.o.-Vergleich bzw. Vergleich von Suspension zu Lösung nach p.o.-Gabe).

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 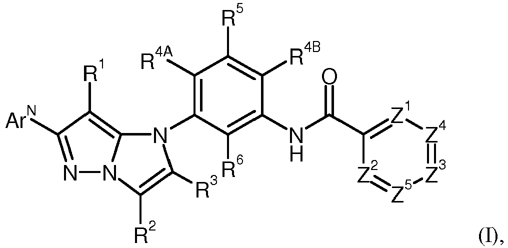 in welcher
Ar^{N} für 5- oder 6-gliedriges Aza-Heteroaryl steht, das ausgewählt ist aus der Gruppe bestehend aus 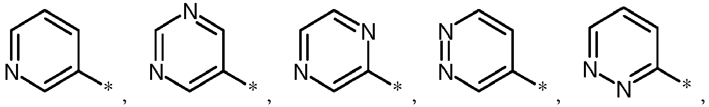 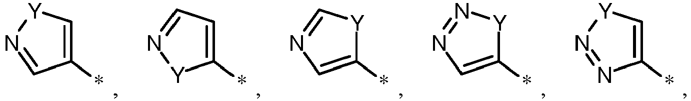 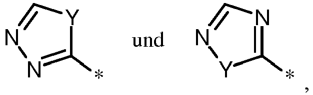 worin * die Verknüpfung zur Imidazopyrazol-Gruppierung markiert
und
Y O, S oder NH bedeutet,
R¹ für Wasserstoff oder Fluor steht,
R² für Wasserstoff oder (C₁-C₄)-Alkyl steht,
R³ für Wasserstoff steht,
R^{4A} und R^{4B} unabhängig voneinander für Wasserstoff, Fluor, Chlor, Methyl, Fluormethyl, Difluormethyl, Trifluormethyl, Hydroxymethyl, Methoxymethyl, Ethyl, Hydroxy, Methoxy oder Trifluormethoxy stehen,
R⁵ für Wasserstoff, Fluor, Chlor oder Methyl steht,
R⁶ für Wasserstoff, Fluor, Methyl oder Hydroxy steht,
Z¹ für C-R^{7A} oder N steht,
Z² für C-R^{7B} oder N steht,
Z³ für C-R⁸ oder N steht,
Z⁴ für C-R⁹ oder N steht
und
Z⁵ für C-R¹⁰ oder N steht,
wobei insgesamt maximal eines der Ringglieder Z¹, Z², Z³, Z⁴ und Z⁵ für N steht
und worin
R^{7A} und R^{7B} unabhängig voneinander Wasserstoff, Fluor, Chlor, Methyl, Hydroxy oder Methoxy bedeuten,
R⁸ Wasserstoff, Fluor, Chlor oder Methyl bedeutet,
R⁹ Wasserstoff, Pentafluorsulfanyl, (Trifluormethyl)sulfanyl, Trimethylsilyl, (C₁-C₆)-Alkyl, (C₁-C₆)-Alkoxy, (C₃-C₆)-Cycloalkyl, Oxetanyl oder Tetrahydropyranyl bedeutet,
wobei (C₁-C₆)-Alkyl und (C₁-C₆)-Alkoxy bis zu sechsfach mit Fluor substituiert sein können
und
(C₃-C₆)-Cycloalkyl, Oxetanyl und Tetrahydropyranyl bis zu zweifach, gleich oder verschieden, mit einem Rest ausgewählt aus der Reihe Fluor, Methyl, Trifluormethyl und Hydroxy substituiert sein können,
und
R¹⁰ Wasserstoff, Fluor, Chlor, Brom, Cyano, (C₁-C₆)-Alkyl, Hydroxy, (C₁-C₆)-Alkoxy, (C₁-C₄)-Alkylsulfonyl, (C₃-C₆)-Cycloalkyl, Phenyl, 5- oder 6-gliedriges Heteroaryl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₆)-Alkyl und (C₁-C₆)-Alkoxy mit einem Rest ausgewählt aus der Reihe Hydroxy, Methoxy, Ethoxy, 2-Hydroxyethoxy, 2-Methoxyethoxy, 2-Ethoxyethoxy, Amino, Methylamino und Dimethylamino oder bis zu sechsfach mit Fluor substituiert sein können
und
(C₃-C₆)-Cycloalkyl bis zu zweifach, gleich oder verschieden, mit einem Rest ausgewählt aus der Reihe Methyl, Hydroxy, Methoxy, Ethoxy, Amino, Methylamino und Dimethylamino substituiert sein kann
und
Phenyl und 5- oder 6-gliedriges Heteroaryl bis zu zweifach, gleich oder verschieden, mit einem Rest ausgewählt aus der Reihe Fluor, Chlor, Cyano, Methyl und Trifluormethyl substituiert sein können,
und worin
L¹ eine Bindung oder -CH₂- darstellt,
L² eine Bindung oder -CH₂- darstellt,
L³ eine Bindung oder -O- darstellt,
R¹¹ Wasserstoff oder (C₁-C₄)-Alkyl darstellt,
R^{12A}, R^{12B}, R^{13A} und R^{13B} unabhängig voneinander Wasserstoff oder (C₁-C₄)-Alkyl darstellen,
wobei (C₁-C₄)-Alkyl jeweils mit einem Rest ausgewählt aus der Reihe Hydroxy, Methoxy, Ethoxy, Amino, Methylamino und Dimethylamino substituiert sein kann,
oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N, O oder S enthalten kann und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann,
und
R¹⁴ einen über ein Ring-Kohlenstoffatom gebundenen 4- bis 6-gliedrigen Heterocyclus darstellt, der ein Ring-Heteroatom aus der Reihe N, O oder S enthält und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann,
wobei R¹⁰ nicht Wasserstoff, Fluor, Chlor oder Brom bedeutet, wenn Z⁴ für CH oder N steht, und Z⁵ nicht für N steht, wenn Z⁴ für CH steht,
sowie ihre Salze, Solvate und Solvate der Salze.

2. Verbindung der Formel (I) nach Anspruch 1, in welcher
Ar^{N} für 5- oder 6-gliedriges Aza-Heteroaryl steht, das ausgewählt ist aus der Gruppe bestehend aus 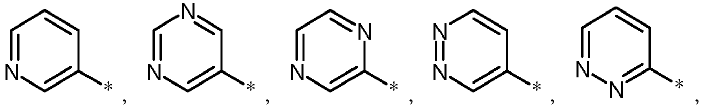 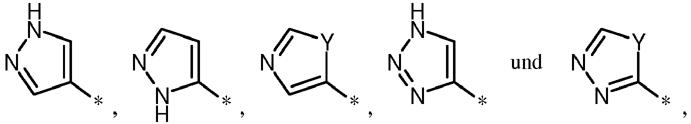 worin * die Verknüpfung zur Imidazopyrazol-Gruppierung markiert
und
Y S oder NH bedeutet,
R¹ für Wasserstoff oder Fluor steht,
R² für Wasserstoff oder (C₁-C₄)-Alkyl steht,
R³ für Wasserstoff steht,
R^{4A} und R^{4B} unabhängig voneinander für Wasserstoff, Fluor, Chlor, Methyl, Fluormethyl, Difluormethyl, Trifluormethyl, Ethyl oder Methoxy stehen,
R⁵ für Wasserstoff, Fluor, Chlor oder Methyl steht,
R⁶ für Wasserstoff, Fluor, Methyl oder Hydroxy steht,
Z¹ für C-R^{7A} oder N steht,
Z² für C-R^{7B} oder N steht,
Z³ für C-R⁸ oder N steht,
Z⁴ für C-R⁹ steht
und
Z⁵ für C-R¹⁰ oder N steht,
wobei insgesamt maximal eines der Ringglieder Z¹, Z², Z³ und Z⁵ für N steht
und worin
R^{7A} und R^{7B} unabhängig voneinander Wasserstoff oder Fluor bedeuten,
R⁸ Wasserstoff oder Fluor bedeutet,
R⁹ Wasserstoff, Pentafluorsulfanyl, (Trifluormethyl)sulfanyl, (C₁-C₄)-Alkyl, (C₁-C₄)-Alkoxy, Cyclopropyl, Cyclobutyl oder Oxetanyl bedeutet,
wobei (C₁-C₄)-Alkyl und (C₁-C₄)-Alkoxy bis zu sechsfach mit Fluor substituiert sein können
und
Cyclopropyl, Cyclobutyl und Oxetanyl bis zu zweifach, gleich oder verschieden, mit einem Rest ausgewählt aus der Reihe Fluor, Methyl, Trifluormethyl und Hydroxy substituiert sein können,
und
R¹⁰ Wasserstoff, Fluor, Chlor, Brom, Cyano, (C₁-C₄)-Alkyl, Hydroxy, (C₁-C₄)-Alkoxy, (C₁-C₄)-Alkylsulfonyl, 5-gliedriges Aza-Heteroaryl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
5-gliedriges Aza-Heteroaryl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A}, R^{12B}, R^{13A} und R^{13B} unabhängig voneinander Wasserstoff oder (C₁-C₄)-Alkyl darstellen
oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N oder O enthalten kann und der bis zu zweifach, gleich oder verschieden, mit einem Rest ausgewählt aus der Reihe Fluor, Cyano, Methyl, Ethyl, Hydroxy, Methoxy und Ethoxy substituiert sein kann,
und
R¹⁴ einen über ein Ring-Kohlenstoffatom gebundenen 4- bis 6-gliedrigen Heterocyclus darstellt, der ein Ring-Heteroatom aus der Reihe N oder O enthält und der bis zu zweifach, gleich oder verschieden, mit einem Rest ausgewählt aus der Reihe Fluor, Cyano, Methyl, Ethyl, Hydroxy, Methoxy und Ethoxy substituiert sein kann,
wobei R¹⁰ nicht Wasserstoff, Fluor, Chlor oder Brom bedeutet, wenn Z⁴ für CH steht, und Z⁵ nicht für N steht, wenn Z⁴ für CH steht,
sowie ihre Salze, Solvate und Solvate der Salze.

3. Verbindung der Formel (I) nach Anspruch 1 oder 2, in welcher
Ar^{N} für 5- oder 6-gliedriges Aza-Heteroaryl der Formel 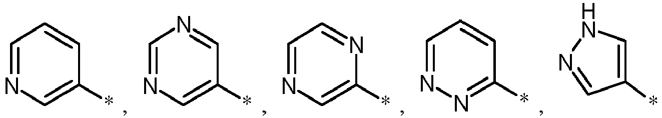 oder 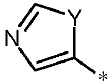 steht, worin * die Verknüpfung zur Imidazopyrazol-Gruppierung markiert
und
Y S oder NH bedeutet,
R¹ für Wasserstoff oder Fluor steht,
R² für Wasserstoff oder Methyl steht,
R³ für Wasserstoff steht,
R^{4A} für Chlor, Methyl oder Trifluormethyl steht,
R^{4B} für Wasserstoff, Fluor, Chlor oder Methyl steht,
R⁵ für Wasserstoff, Fluor, Chlor oder Methyl steht,
R⁶ für Wasserstoff, Fluor, Methyl oder Hydroxy steht,
Z¹ für CH steht,
Z² für CH steht,
Z³ für CH oder N steht,
Z⁴ für C-R⁹ steht, worin
R⁹ Pentafluorsulfanyl, (Trifluormethyl)sulfanyl, Trifluormethyl, Trifluormethoxy, (C₂-C₄)-Alkyl, (C₂-C₄)-Alkoxy, Cyclopropyl, Cyclobutyl oder Oxetan-3-yl bedeutet,
wobei (C₂-C₄)-Alkyl und (C₂-C₄)-Alkoxy bis zu fünffach mit Fluor substituiert sein können
und
Cyclopropyl, Cyclobutyl und Oxetan-3-yl mit einem Rest ausgewählt aus der Reihe Fluor, Methyl, Trifluormethyl und Hydroxy substituiert sein können,
und
Z⁵ für C-R¹⁰ steht, worin
R¹⁰ Wasserstoff, Fluor, Chlor, Brom, Cyano, (C₁-C₄)-Alkyl, Hydroxy, (C₁-C₄)-Alkoxy, Methylsulfonyl, 1*H*-Imidazol-1-yl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können
und
1*H*-Imidazol-1-yl bis zu zweifach mit Methyl substituiert sein kann,
und worin
L¹ eine Bindung oder -CH₂- darstellt,
L² eine Bindung darstellt,
L³ eine Bindung oder -O- darstellt,
R¹¹ Wasserstoff oder (C₁-C₄)-Alkyl darstellt,
R^{12A} und R^{12B} unabhängig voneinander Wasserstoff oder (C₁-C₄)-Alkyl darstellen
oder
R^{12A} und R^{12B} miteinander verknüpft sind und gemeinsam mit dem Stickstoffatom, an das sie gebunden sind, einen 4- bis 6-gliedrigen Heterocyclus bilden, der ein weiteres Ring-Heteroatom aus der Reihe N oder O enthalten kann und der mit einem Rest ausgewählt aus der Reihe Cyano, Methyl, Hydroxy und Methoxy oder bis zu zweifach mit Fluor substituiert sein kann,
R^{13A} und R^{13B} unabhängig voneinander Wasserstoff oder (C₁-C₄)-Alkyl darstellen,
und
R¹⁴ einen über ein Ring-Kohlenstoffatom gebundenen 4- bis 6-gliedrigen Heterocyclus darstellt, der als Ring-Heteroatom ein Stickstoffatom enthält und der mit einem Rest ausgewählt aus der Reihe Cyano, Methyl, Hydroxy und Methoxy oder bis zu zweifach mit Fluor substituiert sein kann,
sowie ihre Salze, Solvate und Solvate der Salze.

4. Verbindung der Formel (I) nach Anspruch 1, 2 oder 3, in welcher
Ar^{N} für 5- oder 6-gliedriges Aza-Heteroaryl der Formel 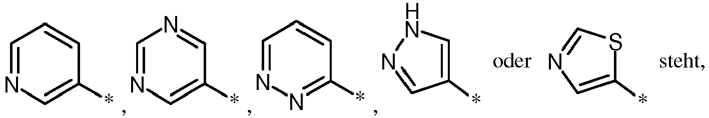
worin * die Verknüpfung zur Imidazopyrazol-Gruppierung markiert,
R¹ für Wasserstoff steht,
R² für Wasserstoff oder Methyl steht,
R³ für Wasserstoff steht,
R^{4A} für Chlor oder Methyl steht,
R^{4B} für Wasserstoff, Fluor, Chlor oder Methyl steht,
R⁵ für Wasserstoff steht,
R⁶ für Wasserstoff steht,
Z¹ für CH steht,
Z² für CH steht,
Z³ für CH oder N steht,
Z⁴ für C-R⁹ steht, worin
R⁹ Pentafluorsulfanyl, (Trifluormethyl)sulfanyl, Trifluormethyl, 2-Fluorpropan-2-yl, *tert.*-Butyl, 1,1,1-Trifluor-2-methylpropan-2-yl, Trifluormethoxy, 1,1,2,2-Tetrafluorethoxy oder 3-Methyloxetan-3-yl bedeutet,
und
Z⁵ für C-R¹⁰ steht, worin
R¹⁰ Wasserstoff, Fluor, Chlor, Cyano, (C₁-C₄)-Alkyl, Hydroxy, (C₁-C₄)-Alkoxy, Methylsulfonyl, 2-Methyl-1*H*-imidazol-1-yl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₄)-Alkyl und (C₁-C₄)-Alkoxy mit einem Rest ausgewählt aus der Reihe Hydroxy, Methoxy, Ethoxy und Amino oder bis zu dreifach mit Fluor substituiert sein können,
und worin
L¹ eine Bindung oder -CH₂- darstellt,
L² eine Bindung darstellt,
L³ eine Bindung oder -O- darstellt,
R¹¹ Wasserstoff darstellt,
R^{12A} und R^{12B} unabhängig voneinander Wasserstoff oder Methyl darstellen
oder
R^{12A} und R^{12B} miteinander verknüpft sind und gemeinsam mit dem Stickstoffatom, an das sie gebunden sind, einen Azetidin-1-yl-, Pyrrolidin-1-yl- oder Piperidin-1-yl-Ring, der jeweils mit einem Rest ausgewählt aus der Reihe Cyano, Hydroxy und Methoxy substituiert sein kann, oder einen Piperazin-1-yl-, 4-Methylpiperazin-1-yl- oder Morpholin-4-yl-Ring bilden,
R^{13A} und R^{13B} unabhängig voneinander Wasserstoff oder Methyl darstellen,
und
R¹⁴ einen Azetidin-3-yl-, Pyrrolidin-3-yl-, Piperidin-3-yl- oder Piperidin-4-yl-Ring, der jeweils mit Hydroxy substituiert sein kann, darstellt,
sowie ihre Salze, Solvate und Solvate der Salze.

5. Verfahren zur Herstellung von Verbindungen der Formel (I) 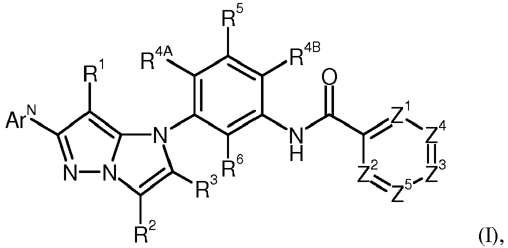 in welcher
Ar^{N} für 5- oder 6-gliedriges Aza-Heteroaryl steht, das ausgewählt ist aus der Gruppe bestehend aus 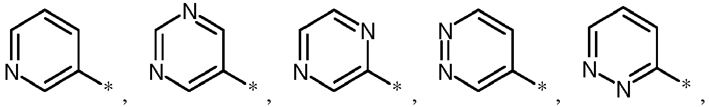 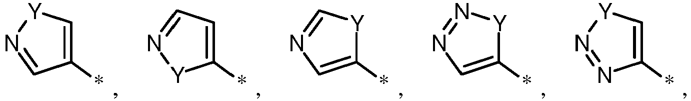 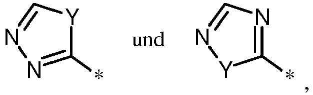 worin * die Verknüpfung zur Imidazopyrazol-Gruppierung markiert
und
Y O, S oder NH bedeutet,
R¹ für Wasserstoff oder Fluor steht,
R² für Wasserstoff oder (C₁-C₄)-Alkyl steht,
R³ für Wasserstoff steht,
R^{4A} und R^{4B} unabhängig voneinander für Wasserstoff, Fluor, Chlor, Methyl, Fluormethyl, Difluormethyl, Trifluormethyl, Hydroxymethyl, Methoxymethyl, Ethyl, Hydroxy, Methoxy oder Trifluormethoxy stehen,
R⁵ für Wasserstoff, Fluor, Chlor oder Methyl steht,
R⁶ für Wasserstoff, Fluor, Methyl oder Hydroxy steht,
Z¹ für C-R^{7A} oder N steht,
Z² für C-R^{7B} oder N steht,
Z³ für C-R⁸ oder N steht,
Z⁴ für C-R⁹ oder N steht und
Z⁵ für C-R¹⁰ oder N steht,
wobei insgesamt maximal eines der Ringglieder Z¹, Z², Z³, Z⁴ und Z⁵ für N steht
und worin
R^{7A} und R^{7B} unabhängig voneinander Wasserstoff, Fluor, Chlor, Methyl, Hydroxy oder Methoxy bedeuten,
R⁸ Wasserstoff, Fluor, Chlor oder Methyl bedeutet,
R⁹ Wasserstoff, Pentafluorsulfanyl, (Trifluormethyl)sulfanyl, Trimethylsilyl, (C₁-C₆)-Alkyl, (C₁-C₆)-Alkoxy, (C₃-C₆)-Cycloalkyl, Oxetanyl oder Tetrahydropyranyl bedeutet,
wobei (C₁-C₆)-Alkyl und (C₁-C₆)-Alkoxy bis zu sechsfach mit Fluor substituiert sein können
und
(C₃-C₆)-Cycloalkyl, Oxetanyl und Tetrahydropyranyl bis zu zweifach, gleich oder verschieden, mit einem Rest ausgewählt aus der Reihe Fluor, Methyl, Trifluormethyl und Hydroxy substituiert sein können,
und
R¹⁰ Wasserstoff, Fluor, Chlor, Brom, Cyano, (C₁-C₆)-Alkyl, Hydroxy, (C₁-C₆)-Alkoxy, (C₁-C₄)-Alkylsulfonyl, (C₃-C₆)-Cycloalkyl, Phenyl, 5- oder 6-gliedriges Heteroaryl oder eine Gruppe der Formel -L¹-C(=O)-OR¹¹, -L¹-NR^{12A}R^{12B}, -L¹-C(=O)-NR^{13A}R^{13B}, -L²-S(=O)₂-NR^{13A}R^{13B} oder -L³-R¹⁴ bedeutet,
wobei (C₁-C₆)-Alkyl und (C₁-C₆)-Alkoxy mit einem Rest ausgewählt aus der Reihe Hydroxy, Methoxy, Ethoxy, 2-Hydroxyethoxy, 2-Methoxyethoxy, 2-Ethoxyethoxy, Amino, Methylamino und Dimethylamino oder bis zu sechsfach mit Fluor substituiert sein können
und
(C₃-C₆)-Cycloalkyl bis zu zweifach, gleich oder verschieden, mit einem Rest ausgewählt aus der Reihe Methyl, Hydroxy, Methoxy, Ethoxy, Amino, Methylamino und Dimethylamino substituiert sein kann
und
Phenyl und 5- oder 6-gliedriges Heteroaryl bis zu zweifach, gleich oder verschieden, mit einem Rest ausgewählt aus der Reihe Fluor, Chlor, Cyano, Methyl und Trifluormethyl substituiert sein können,
und worin
L¹ eine Bindung oder -CH₂- darstellt,
L² eine Bindung oder -CH₂- darstellt,
L³ eine Bindung oder -O- darstellt,
R¹¹ Wasserstoff oder (C₁-C₄)-Alkyl darstellt,
R^{12A}, R^{12B}, R^{13A} und R^{13B} unabhängig voneinander Wasserstoff oder (C₁-C₄)-Alkyl darstellen,
wobei (C₁-C₄)-Alkyl jeweils mit einem Rest ausgewählt aus der Reihe Hydroxy, Methoxy, Ethoxy, Amino, Methylamino und Dimethylamino substituiert sein kann,
oder
R^{12A} und R^{12B} beziehungsweise R^{13A} und R^{13B} miteinander verknüpft sind und gemeinsam mit dem Stickstoffatom, an das sie jeweils gebunden sind, einen 4- bis 6-gliedrigen Heterocyclus bilden, der ein weiteres Ring-Heteroatom aus der Reihe N, O oder S enthalten kann und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann,
und
R¹⁴ einen über ein Ring-Kohlenstoffatom gebundenen 4- bis 6-gliedrigen Heterocyclus darstellt, der ein Ring-Heteroatom aus der
Reihe N, O oder S enthält und der bis zu zweifach, gleich oder verschieden, mit einem Rest ausgewählt aus der Reihe Fluor, Cyano, (C₁-C₄)-Alkyl, Hydroxy, (C₁-C₄)-Alkoxy und Oxo substituiert sein kann,
wobei R¹⁰ nicht Wasserstoff, Fluor, Chlor oder Brom bedeutet, wenn Z⁴ für CH oder N steht, und Z⁵ nicht für N steht, wenn Z⁴ für CH steht,
sowie ihre Salze, Solvate und Solvate der Salze **dadurch gekennzeichnet, dass** man entweder
[A] ein Anilin-Derivat der Formel (II) 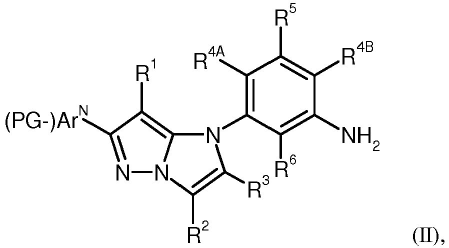 in welcher Ar^{N}, R¹ R², R³, R^{4A}, R^{4B}, R⁵ und R⁶ die in den Ansprüchen 1 bis 4 angegebenen Bedeutungen haben
und
(PG-) eine optionale Stickstoff-Schutzgruppe im Fall, dass Y in Ar^{N} für NH steht, bedeutet,
in einem inerten Lösungsmittel in Gegenwart eines Kondensationsmittels mit einer Carbonsäure der Formel (III) 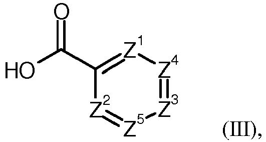 in welcher Z¹, Z², Z³, Z⁴ und Z⁵ die in den Ansprüchen 1 bis 4 angegebenen Bedeutungen haben,
zum Carbonsäureamid der Formel (IV) 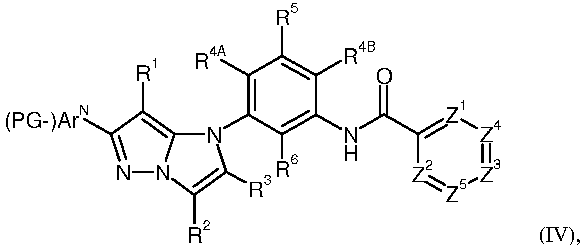 in welcher Ar^{N}, (PG-), R¹, R², R³, R^{4A}, R^{4B}, R⁵, R⁶, Z¹, Z², Z³, Z⁴ und Z⁵ die oben angegebenen Bedeutungen haben,
kuppelt und anschließend die Schutzgruppe PG, falls vorhanden, abspaltet, oder
[B] ein 1*H*-Imidazo[1,2-b]pyrazol-Derivat der Formel (V) 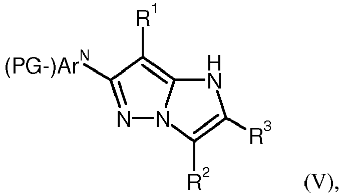 in welcher Ar^{N}, R¹, R² und R³ die in den Ansprüchen 1 bis 4 angegebenen Bedeutungen haben
und
(PG-) eine optionale Stickstoff-Schutzgruppe im Fall, dass Y in Ar^{N} für NH steht, bedeutet,
in einem inerten Lösungsmittel unter Kupfer(I)-Katalyse mit einem Phenylbromid der Formel (VI) 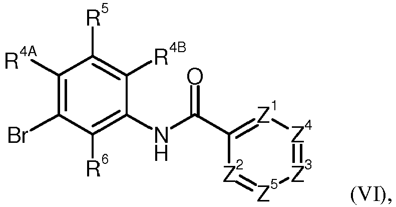 in welcher R^{4A}, R^{4B}, R⁵, R⁶, Z¹, Z², Z³, Z⁴ und Z⁵ die in den Ansprüchen 1 bis 4 angegebenen Bedeutungen haben,
zum 1-Phenyl-1*H*-imidazo[1,2-b]pyrazol-Derivat der Formel (IV) 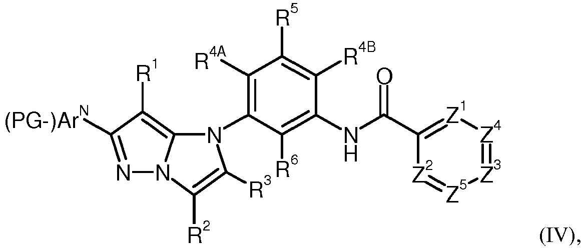 in welcher Ar^{N}, (PG-), R¹, R², R³, R^{4A}, R^{4B}, R⁵, R⁶, Z¹, Z², Z³, Z⁴ und Z⁵ die oben angegebenen Bedeutungen haben,
kuppelt und anschließend die Schutzgruppe PG, falls vorhanden, abspaltet,
oder
[C] ein Aminopyrazol-Derivat der Formel (VII) 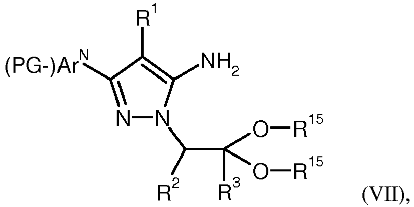 in welcher Ar^{N}, R¹, R² und R³ die in den Ansprüchen 1 bis 4 angegebenen Bedeutungen haben,
R¹⁵ für Methyl oder Ethyl steht,
und
(PG-) eine optionale Stickstoff-Schutzgruppe im Fall, dass Y in Ar^{N} für NH steht, bedeutet,
in einem inerten Lösungsmittel unter Palladium-Katalyse mit einem Phenylbromid der Formel (VI) 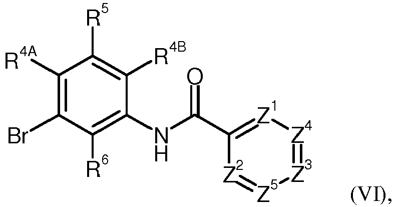 in welcher R^{4A}, R^{4B}, R⁵, R⁶, Z¹, Z², Z³, Z⁴ und Z⁵ die in den Ansprüchen 1 bis 4 angegebenen Bedeutungen haben,
zu einer Verbindung der Formel (VIII) 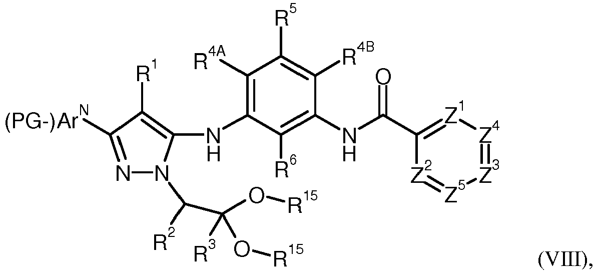 in welcher Ar^{N}, (PG-), R¹, R², R³, R^{4A}, R^{4B}, R⁵, R⁶, R¹⁵, Z¹, Z², Z³, Z⁴ und Z⁵ die oben angegebenen Bedeutungen haben,
kuppelt, die Verbindung der Formel (VIII) dann durch Säure-Behandlung zum 1-Phenyl-1*H*-imidazo[1,2-b]pyrazol-Derivat der Formel (IV) 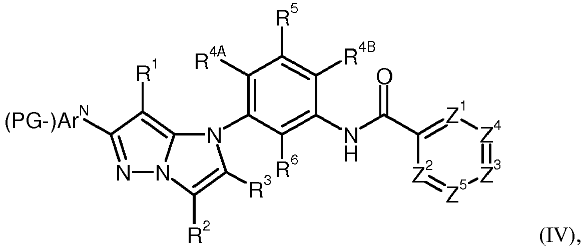 in welcher Ar^{N}, (PG-), R¹, R², R³, R^{4A}, R^{4B}, R⁵, R⁶, Z¹, Z², Z³, Z⁴ und Z⁵ die oben angegebenen Bedeutungen haben,
cyclisiert und anschließend die Schutzgruppe PG, falls vorhanden, abspaltet,
und gegebenenfalls die so erhaltenen Verbindungen der Formel (I) mit den entsprechenden (*i*) Lösungsmitteln und/oder (*ii*) Säuren oder Basen in ihre Solvate, Salze und/oder Solvate der Salze überführt.

6. Verbindung, wie in einem der Ansprüche 1 bis 4 definiert, zur Verwendung der Behandlung und/oder Prävention von Krankheiten.

7. Verbindung, wie in einem der Ansprüche 1 bis 4 definiert, zur Verwendung in einem Verfahren zur Behandlung und/oder Prävention von Krebs- und Tumorerkrankungen.

8. Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 4 definiert, in Kombination mit einem oder mehreren weiteren Wirkstoffen.

10. Arzneimittel nach Anspruch 8oder 9 zur Behandlung und/oder Prävention von Krebs- und Tumorerkrankungen.

## Claims

1. Compound of the formula (I) 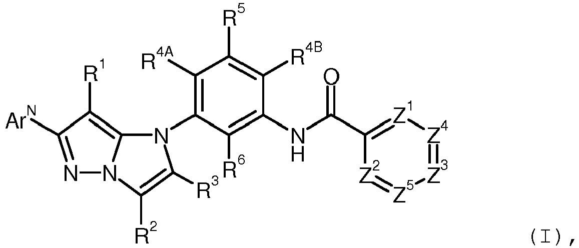 in which
Ar^{N} represents 5- or 6-membered azaheteroaryl selected from the group consisting of 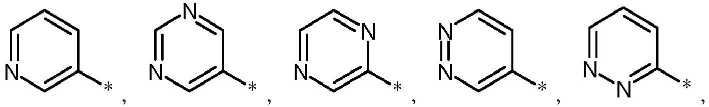 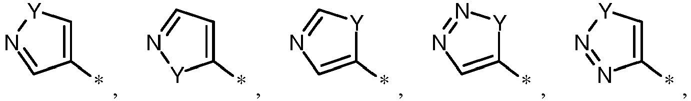 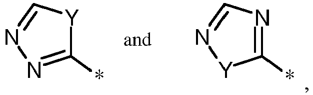 in which * marks the attachment to the imidazopyrazole grouping
and
Y represents 0, S or NH,
R¹ represents hydrogen or fluorine,
R² represents hydrogen or (C₁-C₄)-alkyl,
R³ represents hydrogen,
R^{4A} and R^{4B} independently of one another represent hydrogen, fluorine, chlorine, methyl, fluoromethyl, difluoromethyl, trifluoromethyl, hydroxymethyl, methoxymethyl, ethyl, hydroxy, methoxy or trifluoromethoxy,
R⁵ represents hydrogen, fluorine, chlorine or methyl,
R⁶ represents hydrogen, fluorine, methyl or hydroxy,
Z¹ represents C-R^{7A} or N,
Z² represents C-R^{7B} or N,
Z³ represents C-R⁸ or N,
Z⁴ represents C-R⁹ or N
and
Z⁵ represents C-R¹⁰ or N,
where in total at most one of the ring members Z¹, Z², Z³, Z⁴ and Z⁵ represents N and in which
R^{7A} and R^{7B} independently of one another represent hydrogen, fluorine, chlorine, methyl, hydroxy or methoxy,
R⁸ represents hydrogen, fluorine, chlorine or methyl,
R⁹ represents hydrogen, pentafluorosulphanyl, (trifluoromethyl)sulphanyl, trimethylsilyl, (C₁-C₆)-alkyl, (C₁-C₆)-alkoxy, (C₃-C₆)-cycloalkyl, oxetanyl or tetrahydropyranyl, where (C₁-C₆)-alkyl and (C₁-C₆)-alkoxy may be substituted up to six times by fluorine and
(C₃-C₆)-cycloalkyl, oxetanyl and tetrahydropyranyl may be substituted up to two times by identical or different radicals selected from the group consisting of fluorine, methyl, trifluoromethyl and hydroxy,
and
R¹⁰ represents hydrogen, fluorine, chlorine, bromine, cyano, (C₁-C₆)-alkyl, hydroxy, (C₁-C₆)-alkoxy, (C₁-C₄)-alkylsulphonyl, (C₃-C₆)-cycloalkyl, phenyl, 5- or 6-membered heteroaryl or a group of the formula -L¹-C(=O)-OR¹¹, -L¹-NR^{12A}R^{12B}, -L¹-C(=O)-NR^{13A}R^{13B}, -L²-S(=O)₂-NR^{13A}R^{13B} or - L³-R¹⁴,
where (C₁-C₆)-alkyl and (C₁-C₆)-alkoxy may be substituted by a radical selected from the group consisting of hydroxy, methoxy, ethoxy, 2-hydroxyethoxy, 2-methoxyethoxy, 2-ethoxyethoxy, amino, methylamino and dimethylamino or up to six times by fluorine and
(C₃-C₆)-cycloalkyl may be substituted up to two times by identical or different radicals selected from the group consisting of methyl, hydroxy, methoxy, ethoxy, amino, methylamino and dimethyl-amino
and
phenyl and 5- or 6-membered heteroaryl may be substituted up to two times by identical or different radicals selected from the group consisting of fluorine, chlorine, cyano, methyl and trifluoromethyl,
and in which
L¹ represents a bond or -CH₂-,
L² represents a bond or -CH₂-,
L³ represents a bond or -O-,
R¹¹ represents hydrogen or (C₁-C₄)-alkyl,
R^{12A}, R^{12B}, R^{13A} and R^{13B} independently of one another represent hydrogen or (C₁-C₄)-alkyl, where (C₁-C₄)-alkyl may in each case be substituted by a radical selected from the group consisting of hydroxy, methoxy, ethoxy, amino, methylamino and dimethylamino,
or
R^{12A} and R^{12B} and R^{13A} and R^{13B}, respectively, are attached to one another and together with the nitrogen atom to which they are respectively attached form a 4- to 6-membered heterocycle which may contain a further ring heteroatom from the group consisting of N, 0 and S and which may be substituted up to two times by identical or different radicals selected from the group consisting of fluorine, cyano, (C₁-C₄)-alkyl, hydroxy, (C₁-C₄)-alkoxy and oxo,
and
R¹⁴ represents a 4- to 6-membered heterocycle which is attached via a ring carbon atom and contains a ring heteroatom from the group consisting of N, 0 and S and which may be substituted up to two times by identical or different radicals selected from the group consisting of fluorine, cyano, (C₁-C₄)-alkyl, hydroxy, (C₁-C₄)-alkoxy and oxo,
where R¹⁰ does not represent hydrogen, fluorine, chlorine or bromine if Z⁴ represents CH or N, and Z⁵ does not represent N if Z⁴ represents CH,
and its salts, solvates and solvates of the salts.

2. Compound of the formula (I) according to Claim 1 in which
Ar^{N} represents 5- or 6-membered azaheteroaryl selected from the group consisting of 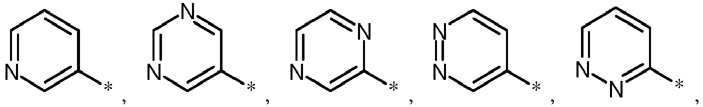 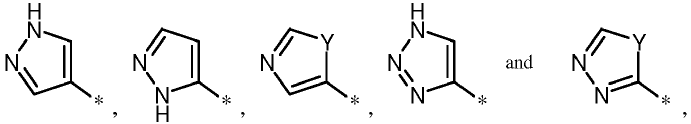 in which * marks the attachment to the imidazopyrazole grouping
and
Y represents S or NH,
R¹ represents hydrogen or fluorine,
R² represents hydrogen or (C₁-C₄)-alkyl,
R³ represents hydrogen,
R^{4A} and R^{4B} independently of one another represent hydrogen, fluorine, chlorine, methyl, fluoromethyl, difluoromethyl, trifluoromethyl, ethyl or methoxy,
R⁵ represents hydrogen, fluorine, chlorine or methyl,
R⁶ represents hydrogen, fluorine, methyl or hydroxy,
Z¹ represents C-R^{7A} or N,
Z² represents C-R^{7B} or N,
Z³ represents C-R⁸ or N,
Z⁴ represents C-R⁹
and
Z⁵ represents C-R¹⁰ or N,
where in total at most one of the ring members Z¹, Z², Z³, Z⁴ and Z⁵ represents N and in which
R^{7A} and R^{7B} independently of one another represent hydrogen or fluorine,
R⁸ represents hydrogen or fluorine,
R⁹ represents hydrogen, pentafluorosulphanyl, (trifluoromethyl)sulphanyl, (C₁-C₄)-alkyl, (C₁-C₄)-alkoxy, cyclopropyl, cyclobutyl or oxetanyl,
where (C₁-C₄)-alkyl and (C₁-C₄)-alkoxy may be substituted up to six times by fluorine
and
cyclopropyl, cyclobutyl and oxetanyl may be substituted up to two times by identical or different radicals selected from the group consisting of fluorine, methyl, trifluoromethyl and hydroxy,
and
R¹⁰ represents hydrogen, fluorine, chlorine, bromine, cyano, (C₁-C₄)-alkyl, hydroxy, (C₁-C₄)-alkoxy, (C₁-C₄)-alkylsulphonyl, 5-membered azaheteroaryl or a group of the formula -L¹-C(=O)-OR¹¹, -L¹-NR^{12A}R^{12B}, -L¹-C(=O)-NR^{13A}R^{13B}, -L²-S(=O)₂-NR^{13A}R^{13B} or -L³-R¹⁴,
where (C₁-C₄)-alkyl and (C₁-C₄)-alkoxy may be substituted by a radical selected from the group consisting of hydroxy, methoxy, ethoxy and amino or up to three times by fluorine
and
5-membered azaheteroaryl may be substituted up to two times by methyl, and in which
L¹ represents a bond or -CH₂-,
L² represents a bond,
L³ represents a bond or -O-,
R¹¹ represents hydrogen or (C₁-C₄)-alkyl,
R^{12A}, R^{12B}, R^{13A} and R^{13B} independently of one another represent hydrogen or (C₁-C₄)-alkyl or
R^{12A} and R^{12B} and R^{13A} and R^{13B}, respectively, are attached to one another and together with the nitrogen atom to which they are respectively attached form a 4- to 6-membered heterocycle which may contain a further ring heteroatom from the group consisting of N and O and which may be substituted up to two times by identical or different radicals selected from the group consisting of fluorine, cyano, methyl, ethyl, hydroxy, methoxy and ethoxy,
and
R¹⁴ represents a 4- to 6-membered heterocycle which is attached via a ring carbon atom and contains a ring heteroatom from the group consisting of N and 0 and which may be substituted up to two times by identical or different radicals selected from the group consisting of fluorine, cyano, methyl, ethyl, hydroxy, methoxy and ethoxy,
where R¹⁰ does not represent hydrogen, fluorine, chlorine or bromine if Z⁴ represents CH, and Z⁵ does not represent N if Z⁴ represents CH,
and its salts, solvates and solvates of the salts.

3. Compound of the formula (I) according to Claim 1 or 2 in which
Ar^{N} represents 5- or 6-membered azaheteroaryl of the formula 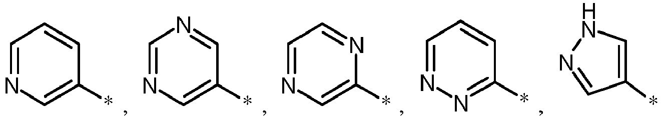 or 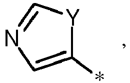 in which * marks the attachment to the imidazopyrazole grouping
and
Y represents S or NH,
R¹ represents hydrogen or fluorine,
R² represents hydrogen or methyl,
R³ represents hydrogen,
R^{4A} represents chlorine, methyl or trifluoromethyl,
R^{4B} represents hydrogen, fluorine, chlorine or methyl,
R⁵ represents hydrogen, fluorine, chlorine or methyl,
R⁶ represents hydrogen, fluorine, methyl or hydroxy,
Z¹ represents CH,
Z² represents CH,
Z³ represents CH or N,
Z⁴ represents C-R⁹, in which
R⁹ represents pentafluorosulphanyl, (trifluoromethyl)sulphanyl, trifluoromethyl, trifluoromethoxy, (C₂-C₄)-alkyl, (C₂-C₄)-alkoxy, cyclopropyl, cyclobutyl or oxetan-3-yl,
where (C₂-C₄)-alkyl and (C₂-C₄)-alkoxy may be substituted up to five times by fluorine
and
cyclopropyl, cyclobutyl and oxetan-3-yl may be substituted by a radical selected from the group consisting of fluorine, methyl, trifluoromethyl and hydroxy, and
Z⁵ represents C-R¹⁰, in which
R¹⁰ represents hydrogen, fluorine, chlorine, bromine, cyano, (C₁-C₄)-alkyl, hydroxy, (C₁-C₄)-alkoxy, methylsulphonyl, 1*H-*imidazol-1-yl or a group of the formula -L¹-C(=O)-OR¹¹, -L¹NR^{12A}R^{12B}, -L¹-C(-O)-NR^{13A}R^{13B}, -L²-S(=O)₂-NR^{13A}R^{13B} or -L³-R¹⁴, where (C₁-C₄)-alkyl and (C₁-C₄)-alkoxy may be substituted by a radical selected from the group consisting of hydroxy, methoxy, ethoxy and amino or up to three times by fluorine
and
1*H*-imidazol-1-yl may be substituted up to two times by methyl,
and in which
L¹ represents a bond or -CH₂-,
L² represents a bond,
L³ represents a bond or -O-,
R¹¹ represents hydrogen or (C₁-C₄)-alkyl,
R^{12A} and R^{12B} independently of one another represent hydrogen or (C₁-C₄)-alkyl or
R^{12A} and R^{12B} are attached to one another and together with the nitrogen atom to which they are attached form a 4- to 6-membered heterocycle which may contain a further ring hetero-atom from the group consisting of N and 0 and which may be substituted by a radical selected from the group consisting of cyano, methyl, hydroxy and methoxy or up to two times with fluorine,
R^{13A} and R^{13B} independently of one another represent hydrogen or (C₁-C₄)-alkyl, and
R¹⁴ represents a 4- to 6-membered heterocycle which is attached via a ring carbon atom and, as ring heteroatom, contains a nitrogen atom and which may be substituted by a radical selected from the group consisting of cyano, methyl, hydroxy and methoxy or up to two times with fluorine,
and its salts, solvates and solvates of the salts.

4. Compound of the formula (I) according to Claim 1, 2 or 3, in which
Ar^{N} represents 5- or 6-membered azaheteroaryl of the formula 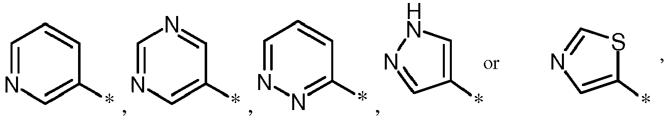 in which * marks the attachment to the imidazopyrazole grouping
R¹ represents hydrogen,
R² represents hydrogen or methyl,
R³ represents hydrogen,
R^{4A} represents chlorine or methyl,
R^{4B} represents hydrogen, fluorine, chlorine or methyl,
R⁵ represents hydrogen,
R⁶ represents hydrogen,
Z¹ represents CH,
Z² represents CH,
Z³ represents CH or N,
Z⁴ represents C-R⁹, in which
R⁹ represents pentafluorosulphanyl, (trifluoromethyl)sulphanyl, trifluoromethyl, 2-fluoropropan-2-yl, *tert*-butyl, 1,1,1-trifluoro-2-methylpropan-2-yl, trifluoromethoxy, 1,1,2,2-tetrafluoroethoxy or 3-methyloxetan-3-yl,
and
Z⁵ represents C-R¹⁰, in which
R¹⁰ represents hydrogen, fluorine, chlorine, cyano, (C₁-C₄)-alkyl, hydroxy, (C₁-C₄)-alkoxy, methylsulphonyl, 2-methyl-1*H-*imidazol-1-yl or a group of the formula -L¹-C(=O)-OR¹¹, -L¹-NR^{12A}R^{12B}, -L¹-C(=O)-NR^{13A}R^{13B}, -L²-S(=O)₂-NR^{13A}R^{13B} or -L³-R¹⁴, where (C₁-C₄)-alkyl and (C₁-C₄)-alkoxy may be substituted by a radical selected from the group consisting of hydroxy, methoxy, ethoxy and amino or up to three times by fluorine,
and in which
L¹ represents a bond or -CH₂-,
L² represents a bond,
L³ represents a bond or -O-,
R¹¹ represents hydrogen,
R^{12A} and R^{12B} independently of one another represent hydrogen or methyl
or
R^{12A} and R^{12B} are attached to one another and together with the nitrogen atom to which they are attached form an azetidin-1-yl, pyrrolidin-1-yl or piperidin-1-yl ring, each of which may be substituted by a radical selected from the group consisting of cyano, hydroxy and methoxy, or a piperazin-1-yl, 4-methylpiperazin-1-yl or morpholin-4-yl ring, R¹³
^{A} and R^{13B} represent independently of one another hydrogen or methyl, and
R¹⁴ represents an azetidin-3-yl, pyrrolidin-3-yl, piperidin-3-yl or piperidin-4-yl ring, each of which may be substituted by hydroxy,
and its salts, solvates and solvates of the salts.

5. Process for preparing compounds of the formula (I) 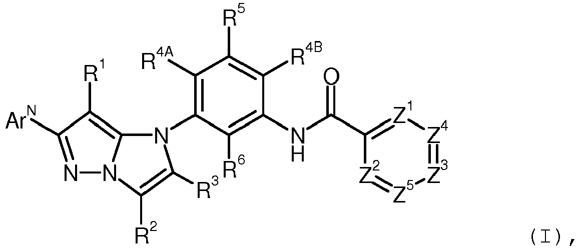 in which
Ar^{N} represents 5- or 6-membered azaheteroaryl selected from the group consisting of 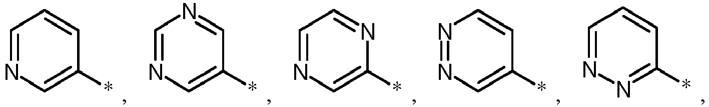 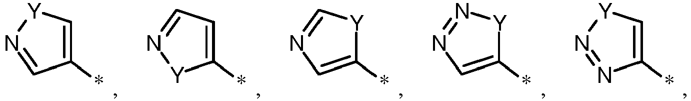 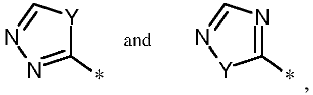 in which * marks the attachment to the imidazopyrazole grouping and
Y represents 0, S or NH,
R¹ represents hydrogen or fluorine,
R² represents hydrogen or (C₁-C₄)-alkyl,
R³ represents hydrogen,
R^{4A} and R^{4B} independently of one another represent hydrogen, fluorine, chlorine, methyl, fluoromethyl, difluoromethyl, trifluoromethyl, hydroxymethyl, methoxymethyl, ethyl, hydroxy, methoxy or trifluoromethoxy,
R⁵ represents hydrogen, fluorine, chlorine or methyl,
R⁶ represents hydrogen, fluorine, methyl or hydroxy,
Z¹ represents C-R^{7A} or N,
Z² represents C-R^{7B} or N,
Z³ represents C-R⁸ or N,
Z⁴ represents C-R⁹ or N
and
Z⁵ represents C-R¹⁰ or N,
where in total at most one of the ring members Z¹, Z², Z³, Z⁴ and Z⁵ represents N and in which
R^{7A} and R^{7B} independently of one another represent hydrogen, fluorine, chlorine, methyl, hydroxy or methoxy,
R⁸ represents hydrogen, fluorine, chlorine or methyl,
R⁹ represents hydrogen, pentafluorosulphanyl,
(trifluoromethyl)sulphanyl, trimethylsilyl, (C₁-C₆) -alkyl, (C₁-C₆) -alkoxy, (C₃-C₆)-cycloalkyl, oxetanyl or tetrahydropyranyl,
where (C₁-C₆)-alkyl and (C₁-C₆)-alkoxy may be substituted up to six times by fluorine
and
(C₃-C₆)-cycloalkyl, oxetanyl and tetrahydropyranyl may be substituted up to two times by identical or different radicals selected from the group consisting of fluorine, methyl, trifluoromethyl and hydroxy,
and
R¹⁰ represents hydrogen, fluorine, chlorine, bromine, cyano, (C₁-C₆)-alkyl, hydroxy, (C₁-C₆)-alkoxy, (C₁-C₄)-alkylsulphonyl, (C₃-C₆)-cycloalkyl, phenyl, 5- or 6-membered heteroaryl or a group of the formula -L¹-C(=O)-OR¹¹, -L¹-NR^{12A}R^{12B}, -L¹-C(=O)-NR^{13A}R^{13B}, -L²-S(=O)₂-NR^{13A}R^{13B} or - L³-R¹⁴,
where (C₁-C₆) -alkyl and (C₁-C₆) -alkoxy may be substituted by a radical selected from the group consisting of hydroxy, methoxy, ethoxy, 2-hydroxyethoxy, 2-methoxyethoxy, 2-ethoxyethoxy, amino, methylamino and dimethylamino or up to six times by fluorine
and
(C₃-C₆)-cycloalkyl may be substituted up to two times by identical or different radicals selected from the group consisting of methyl, hydroxy, methoxy, ethoxy, amino, methylamino and dimethylamino
and
phenyl and 5- or 6-membered heteroaryl may be substituted up to two times by identical or different radicals selected from the group consisting of fluorine, chlorine, cyano, methyl and trifluoromethyl,
and in which
L¹ represents a bond or -CH₂-,
L² represents a bond or -CH₂-,
L³ represents a bond or -O-,
R¹¹ represents hydrogen or (C₁-C₄)-alkyl,
R^{12A}, R^{12B}, R^{13A} and R^{13B} independently of one another represent hydrogen or (C₁-C₄)-alkyl,
where (C₁-C₄)-alkyl may in each case be substituted by a radical selected from the group consisting of hydroxy, methoxy, ethoxy, amino, methylamino and dimethylamino,
or
R^{12A} and R^{12B} and R^{13A} and R^{13B}, respectively, are attached to one another and together with the nitrogen atom to which they are respectively attached form a 4- to 6-membered heterocycle which may contain a further ring heteroatom from the group consisting of N, 0 and S and which may be substituted up to two times by identical or different radicals selected from the group consisting of fluorine, cyano, (C₁-C₄)-alkyl, hydroxy, (C₁-C₄)-alkoxy and oxo,
and
R¹⁴ represents a 4- to 6-membered heterocycle which is attached via a ring carbon atom and contains a ring heteroatom from the group consisting of N, 0 and S and which may be substituted up to two times by identical or different radicals selected from the group consisting of fluorine, cyano, (C₁-C₄)-alkyl, hydroxy, (C₁-C₄)-alkoxy and oxo,
where R¹⁰ does not represent hydrogen, fluorine, chlorine or bromine if Z⁴ represents CH or N, and Z⁵ does not represent N if Z⁴ represents CH,
and its salts, solvates and solvates of the salts, **characterized in that** either
[A] an aniline derivative of the formula (II) 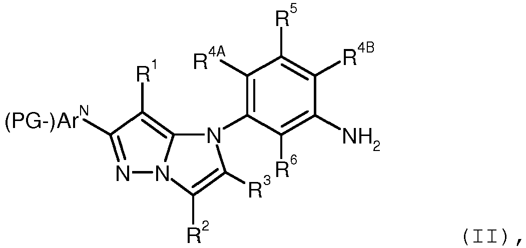
in which Ar^{N}, R¹, R², R³, R^{4A}, R^{4B}, R⁵ and R⁶ have the meanings given in any of Claims 1 to 4 and
(PG-) represents an optional nitrogen protective group in the case that Y in Ar^{N} represents NH,
is coupled in an inert solvent in the presence of a condensing agent with a carboxylic acid of the formula (III) 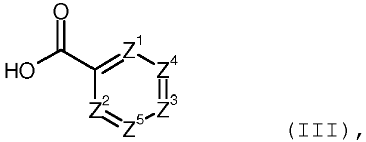
in which Z¹, Z², Z³, Z⁴ and Z⁵ have the meanings given in any of Claims 1 to 4,
to give the carboxamide of the formula (IV) 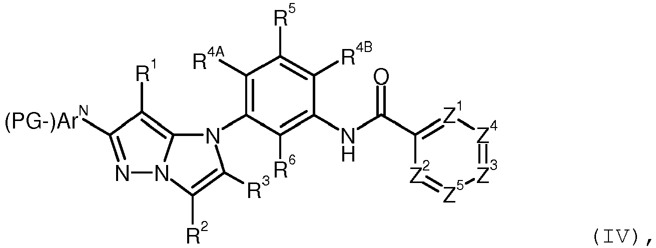
in which Ar^{N}, (PG-), R¹, R², R³, R^{4A}, R^{4B}, R⁵, R⁶, Z¹, Z², Z³, Z⁴ and Z⁵ have the meanings given above,
and the protective group PG, if present, is then removed, or
[B] a 1*H*-imidazo[1,2-b]pyrazole derivative of the formula (V) 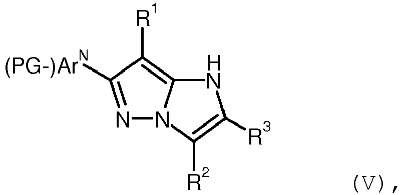
in which Ar^{N}, R¹, R² and R³ have the meanings given in any of Claims 1 to 4
and
(PG-) represents an optional nitrogen protective group in the case that Y in Ar^{N} represents NH,
is coupled in an inert solvent with copper(I) catalysis with a phenyl bromide of the formula (VI) 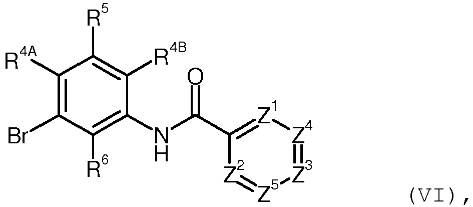
in which R^{4A}, R^{4B}, R⁵, R⁶, Z¹, Z², Z³, Z⁴ and Z⁵ have the meanings given in any of Claims 1 to 4,
to give the 1-phenyl-1*H*-imidazo[1,2-b]pyrazole derivative of the formula (IV) 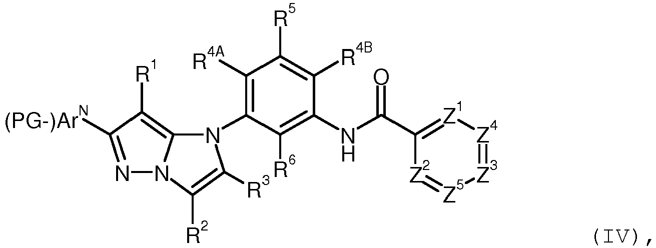
in which Ar^{N}, (PG-), R¹, R², R³, R^{4A}, R^{4B}, R⁵, R⁶, Z¹, Z², Z³, Z⁴ and Z⁵ have the meanings given above,
and the protective group PG, if present, is then removed,
or
[C] an aminopyrazole derivative of the formula (VII) 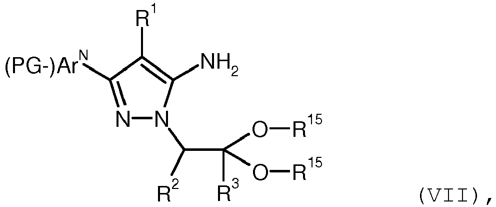
in which Ar^{N}, R¹, R² and R³ have the meanings given in any of Claims 1 to 4,
R¹⁵ represents methyl or ethyl,
and
(PG-) represents an optional nitrogen protective group in the case that Y in Ar^{N} represents NH,
is coupled in an inert solvent under palladium catalysis with a phenyl bromide of the formula (VI) 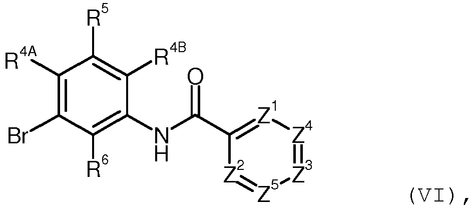
in which R^{4A}, R^{4B}, R⁵, R⁶, Z¹, Z², Z³, Z⁴ and Z⁵ have the meanings given in any of Claims 1 to 4,
to give a compound of the formula (VIII) 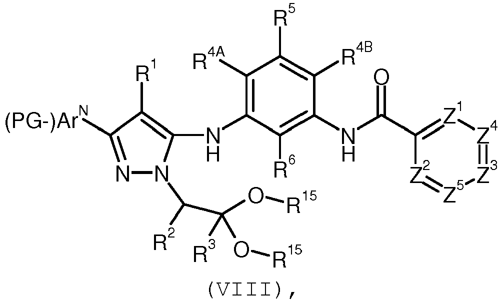
in which Ar^{N}, (PG-), R¹, R², R³, R^{4A}, R^{4B}, R⁵, R⁶, R¹⁵, Z¹, Z², Z³, Z⁴ and Z⁵ have the meanings given above,
the compound of the formula (VIII) is then cyclised by treatment with acid to give the 1-phenyl-1*H*-imidazo[1,2-b]pyrazole derivative of the formula (IV) 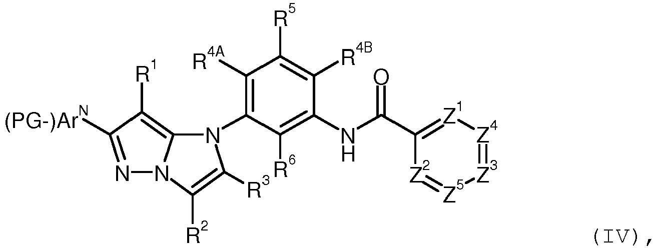
in which Ar^{N}, (PG-), R¹, R², R³, R^{4A}, R^{4B}, R⁵, R⁶, Z¹, Z², Z³, Z⁴ and Z⁵ have the meanings given above,
and the protective group PG, if present, is then removed,
and the compounds of the formula (I) obtained in this manner are optionally converted with the appropriate (i) solvents and/or (ii) acids or bases into their solvates, salts and/or solvates of the salts.

6. Compound as defined in any of Claims 1 to 4 for use of the treatment and/or prevention of diseases.

7. Compound as defined in any of Claims 1 to 4 for use in a method for the treatment and/or prevention of neoplastic disorders and tumour disorders.

8. Medicament, comprising a compound as defined in any of Claims 1 to 4 in combination with one or more inert, nontoxic, pharmaceutically suitable auxiliaries.

9. Medicament, comprising a compound as defined in any of Claims 1 to 4 in combination with one or more further active compounds.

10. Medicament according to Claim 8 or 9 for the treatment and/or prevention of neoplastic disorders and tumour disorders.

## Revendications

1. Composé de formule (I) 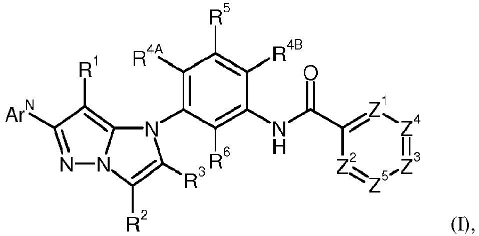 dans laquelle
Ar^{N} représente aza-hétéroaryle de 5 ou 6 chaînons, qui est choisi dans le groupe constitué par 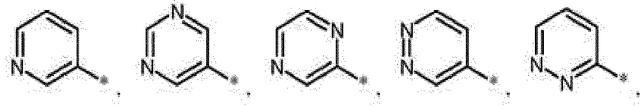 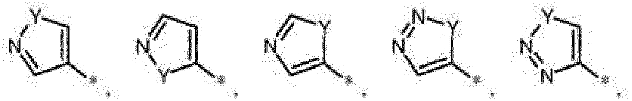 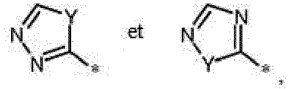
dans laquelle * marque la liaison au groupement imidazopyrazole et
Y signifie O, S ou NH,
R¹ représente hydrogène ou fluor,
R² représente hydrogène ou (C₁-C₄)-alkyle,
R³ représente hydrogène,
R^{4A} et R^{4B} représentent, indépendamment l'un de l'autre, hydrogène, fluor, chlore, méthyle, fluorométhyle, difluorométhyle, trifluorométhyle, hydroxyméthyle, méthoxyméthyle, éthyle, hydroxy, méthoxy ou trifluorométhoxy,
R⁵ représente hydrogène, fluor, chlore ou méthyle,
R⁶ représente hydrogène, fluor, méthyle ou hydroxy,
Z¹ représente C-R^{7A} ou N,
Z² représente C-R^{7B} ou N,
Z³ représente C-R⁸ ou N,
Z⁴ représente C-R⁹ ou N et
Z⁵ représente C-R¹⁰ ou N,
au total au maximum un des chaînons de cycle Z¹, Z², Z³, Z⁴ et Z⁵ représentant N et où
R^{7A} et R^{7B} signifient, indépendamment l'un de l'autre, hydrogène, fluor, chlore, méthyle, hydroxy ou méthoxy,
R⁸ signifie hydrogène, fluor, chlore ou méthyle,
R⁹ signifie hydrogène, pentafluorosulfanyle, (trifluorométhyl)sulfanyle, triméthylsilyle, (C₁-C₆) -alkyle, (C₁-C₆)-alcoxy, (C₃-C₆)-cycloalkyle, oxétanyle ou tétrahydropyrannyle,
(C₁-C₆)-alkyle et (C₁-C₆)-alcoxy pouvant être jusqu'à hexasubstitués par fluor et (C₃-C₆)-cycloalkyle, oxétanyle et tétrahydropyrannyle pouvant être jusqu'à disubstitués, de manière identique ou différente, par un radical choisi dans la série fluor, méthyle, trifluorométhyle et hydroxy et
R¹⁰ signifie hydrogène, fluor, chlore, brome, cyano, (C₁-C₆)-alkyle, hydroxy, (C₁-C₆)-alcoxy, (C₁-C₄)-alkylsulfonyle, (C₃-C₆)-cycloalkyle, phényle, hétéroaryle de 5 ou 6 chaînons ou un groupe de formule -L¹-C(=O)-OR¹¹, -L¹-NR^{12A}R^{12B}, -L¹-C(=O)-NR^{13A}R^{13B}, -L²-S(=O)₂-NR^{13A}R^{13B} ou -L³-R¹⁴,
(C₁-C₆)-alkyle et (C₁-C₆)-alcoxy pouvant être substitués par un radical choisi dans la série hydroxy, méthoxy, éthoxy, 2-hydroxyéthoxy, 2-méthoxyéthoxy, 2-éthoxyéthoxy, amino, méthylamino et diméthylamino ou jusqu'à hexasubstitués par fluor et
(C₃-C₆)-cycloalkyle pouvant être jusqu'à disubstitué, de manière identique ou différente, par un radical choisi dans la série méthyle, hydroxy, méthoxy, éthoxy, amino, méthylamino et diméthylamino et phényle et hétéroaryle de 5 ou 6 chaînons pouvant être jusqu'à disubstitués, de manière identique ou différente, par un radical choisi dans la série fluor, chlore, cyano, méthyle et trifluorométhyle,
et où
L¹ représente une liaison ou -CH₂-,
L² représente une liaison ou -CH₂-,
L³ représente une liaison ou -O-,
R¹¹ représente hydrogène ou (C₁-C₄)-alkyle,
R^{12A}, R^{12B}, R^{13A} et R^{13B} représentent,
indépendamment les uns des autres, hydrogène ou (C₁-C₄)-alkyle, (C₁-C₄)-alkyle pouvant être à chaque fois substitué par un radical choisi dans la série hydroxy, méthoxy, éthoxy, amino, méthylamino et diméthylamino ou
R^{12A} et R^{12B} ou R^{13A} et R^{13B} sont liés l'un à l'autre et forment, ensemble avec l'atome d'azote auquel ils sont à chaque fois liés, un hétérocycle de 4 à 6 chaînons, qui peut contenir un autre hétéroatome de cycle de la série N, O ou S et qui peut être jusqu'à disubstitué, de manière identique ou différente, par un radical choisi dans la série fluor, cyano, (C₁-C₄)-alkyle, hydroxy, (C₁-C₄)-alcoxy et oxo et
R¹⁴ représente un hétérocycle de 4 à 6 chaînons lié via un atome de carbone de cycle, qui contient un hétéroatome de cycle de la série N, O ou S et qui peut être jusqu'à disubstitué, de manière identique ou différente, par un radical choisi dans la série fluor, cyano, (C₁-C₄)-alkyle, hydroxy, (C₁-C₄)-alcoxy et oxo,
R¹⁰ ne représentant pas hydrogène, fluor, chlore ou brome lorsque Z⁴ représente CH ou N et Z⁵ ne représentant pas N lorsque Z⁴ représente CH,
ainsi que ses sels, solvates et solvates des sels.

2. Composé de formule (I) selon la revendication 1, dans laquelle
Ar^{N} représente aza-hétéroaryle de 5 ou 6 chaînons, qui est choisi dans le groupe constitué par 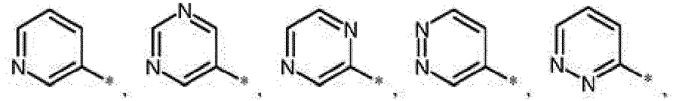 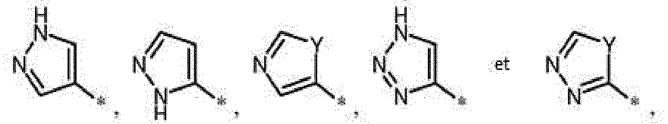 dans laquelle * marque la liaison au groupement imidazopyrazole et
Y signifie S ou NH,
R¹ représente hydrogène ou fluor,
R² représente hydrogène ou (C₁-C₄)-alkyle,
R³ représente hydrogène,
R^{4A} et R^{4B} représentent, indépendamment l'un de l'autre, hydrogène, fluor, chlore, méthyle, fluorométhyle, difluorométhyle, trifluorométhyle, éthyle ou méthoxy,
R⁵ représente hydrogène, fluor, chlore ou méthyle,
R⁶ représente hydrogène, fluor, méthyle ou hydroxy,
Z¹ représente C-R^{7A} ou N,
Z² représente C-R^{7B} ou N,
Z³ représente C-R⁸ ou N,
Z⁴ représente C-R⁹ et
Z⁵ représente C-R¹⁰ ou N,
au total au maximum un des chaînons de cycle Z¹, Z², Z³ et Z⁵ représentant N et où
R^{7A} et R^{7B} représentent, indépendamment l'un de l'autre, hydrogène ou fluor;
R⁸ signifie hydrogène ou fluor,
R⁹ signifie hydrogène, pentafluorosulfanyle, (trifluorométhyl)sulfanyle, (C₁-C₄)-alkyle, (C₁-C₄)-alcoxy, cyclopropyle, cyclobutyle ou oxétanyle,
(C₁-C₄)-alkyle et (C₁-C₄)-alcoxy pouvant être jusqu'à hexasubstitués par fluor et cyclopropyle, cyclobutyle et oxétanyle pouvant être jusqu'à disubstitués, de manière identique ou différente, par un radical choisi dans la série fluor, méthyle, trifluorométhyle et hydroxy et
R¹⁰ signifie hydrogène, fluor, chlore, brome, cyano, (C₁-C₄)-alkyle, hydroxy, (C₁-C₄)-alcoxy, (C₁-C₄)-alkylsulfonyle, aza-hétéroaryle de 5 chaînons ou un groupe de formule -L¹-C(=O)-OR¹¹, -L¹-NR^{12A}R^{12B}, -L¹-C(=O)-NR^{13A}R^{13B}, -L²-S(=O)₂-NR^{13A}R^{13B} ou -L³-R¹⁴, (C₁-C₄)-alkyle et (C₁-C₄)-alcoxy pouvant être substitués par un radical choisi dans la série hydroxy, méthoxy, éthoxy et amino ou jusqu'à trisubstitués par fluor et aza-hétéroaryle à 5 chaînons pouvant être jusqu'à disubstitué par méthyle, et où
L¹ représente une liaison ou -CH₂-,
L² représente une liaison,
L³ représente une liaison ou -O-,
R¹¹ représente hydrogène ou (C₁-C₄)-alkyle,
R^{12A}, R^{12B}, R^{13A} et R^{13B} représentent, indépendamment les uns des autres, hydrogène ou (C₁-C₄)-alkyle ou
R^{12A} et R^{12B} ou R^{13A} et R^{13B} sont liés l'un à l'autre et forment, ensemble avec l'atome d'azote auquel ils sont à chaque fois liés, un hétérocycle de 4 à 6 chaînons, qui peut contenir un autre hétéroatome de cycle de la série N ou O et qui peut être jusqu'à disubstitué, de manière identique ou différente, par un radical choisi dans la série fluor, cyano, méthyle, éthyle, hydroxy, méthoxy et éthoxy, et
R¹⁴ représente un hétérocycle de 4 à 6 chaînons lié via un atome de carbone de cycle, qui contient un hétéroatome de cycle de la série N ou O et qui peut être jusqu'à disubstitué, de manière identique ou différente, par un radical choisi dans la série fluor, cyano, méthyle, éthyle, hydroxy, méthoxy et éthoxy,
R¹⁰ ne représentant pas hydrogène, fluor, chlore ou brome lorsque Z⁴ représente CH ou N et Z⁵ ne représentant pas N lorsque Z⁴ représente CH,
ainsi que ses sels, solvates et solvates des sels.

3. Composé de formule (I) selon la revendication 1 ou 2, dans laquelle
Ar^{N} représente aza-hétéroaryle de 5 ou 6 chaînons de formule 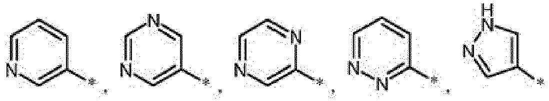 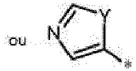 dans laquelle * marque la liaison au groupement imidazopyrazole et Y signifie S ou NH,
R¹ représente hydrogène ou fluor,
R² représente hydrogène ou méthyle,
R³ représente hydrogène,
R^{4A} représente chlore, méthyle ou trifluorométhyle,
R^{4B} représente hydrogène, fluor, chlore ou méthyle,
R⁵ représente hydrogène, fluor, chlore ou méthyle,
R⁶ représente hydrogène, fluor, méthyle ou hydroxy,
Z¹ représente CH,
Z² représente CH,
Z³ représente CH ou N,
Z⁴ représente C-R⁹, où
R⁹ signifie pentafluorosulfanyle, (trifluorométhyl)sulfanyle, trifluorométhyle, trifluorométhoxy, (C₂-C₄)-alkyle, (C₂-C₄)-alcoxy, cyclopropyle, cyclobutyle ou oxétan-3-yle,
(C₂-C₄)-alkyle et (C₂-C₄)-alcoxy pouvant être jusqu'à pentasubstitués par fluor et cyclopropyle, cyclobutyle et oxétan-3-yle pouvant être substitués par un radical choisi dans la série fluor, méthyle, trifluorométhyle et hydroxy,
et
Z⁵ représente C-R¹⁰, où
R¹⁰ signifie hydrogène, fluor, chlore, brome, cyano, (C₁-C₄)-alkyle, hydroxy, (C₁-C₄)-alcoxy, méthylsulfonyle, 1H-imidazol-1-yle ou un groupe de formule -L¹-C(=O)-OR¹¹, -L¹-NR^{12A}R^{12B}, -L¹-C(=O)-NR^{13A}R^{13B}, -L²-S(=O)₂-NR^{13A}R^{13B} ou -L³-R¹⁴,
(C₁-C₄)-alkyle et (C₁-C₄)-alcoxy pouvant être substitués par un radical choisi dans la série hydroxy, méthoxy, éthoxy et amino ou jusqu'à trisubstitués par fluor et 1H-imidazol-1-yle pouvant être jusqu'à disubstitué par méthyle et où
L¹ représente une liaison ou -CH₂-,
L² représente une liaison,
L³ représente une liaison ou -O-,
R¹¹ représente hydrogène ou (C₁-C₄)-alkyle,
R^{12A} et R^{12B} représentent, indépendamment l'un de l'autre, hydrogène ou (C₁-C₄)-alkyle ou
R^{12A} et R^{12B} sont liés l'un à l'autre et forment, ensemble avec l'atome d'azote auquel ils sont liés, un hétérocycle de 4 à 6 chaînons, qui peut contenir un autre hétéroatome de cycle de la série N ou O et qui peut être jusqu'à disubstitué par un radical choisi dans la série cyano, méthyle, hydroxy et méthoxy et jusqu'à disubstitué par fluor,
R^{13A} et R^{13B} représentent, indépendamment l'un de l'autre, hydrogène ou (C₁-C₄)-alkyle et
R¹⁴ représente un hétérocycle de 4 à 6 chaînons lié via un atome de carbone de cycle, qui contient comme hétéroatome de cycle un atome d'azote et qui peut être substitué par un radical choisi dans la série cyano, méthyle, hydroxy et méthoxy ou jusqu'à disubstitué par fluor,
ainsi que ses sels, solvates et solvates des sels.

4. Composé de formule (I) selon la revendication 1, 2 ou 3, dans laquelle
Ar^{N} représente aza-hétéroaryle de 5 ou 6 chaînons de formule 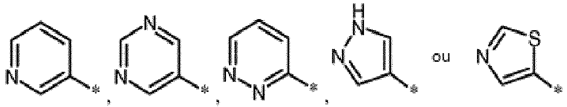 dans laquelle * marque la liaison au groupement imidazopyrazole,
R¹ représente hydrogène,
R² représente hydrogène ou méthyle,
R³ représente hydrogène,
R^{4A} représente chlore ou méthyle,
R^{4B} représente hydrogène, fluor, chlore ou méthyle,
R⁵ représente hydrogène,
R⁶ représente hydrogène,
Z¹ représente CH,
Z² représente CH,
Z³ représente CH ou N,
Z⁴ représente C-R⁹, où
R⁹ signifie pentafluorosulfanyle, (trifluorométhyl)sulfanyle, trifluorométhyle, 2-fluoropropan-2-yle, tert-butyle, 1,1,1-trifluoro-2-méthylpropan-2-yle, trifluorométhoxy, 1,1,2,2-tétrafluoroéthoxy ou 3-méthyloxétan-3-yle et
Z⁵ représente C-R¹⁰, où
R¹⁰ signifie hydrogène, fluor, chlore, cyano, (C₁-C₄)-alkyle, hydroxy, (C₁-C₄)-alcoxy, méthylsulfonyle, 2-méthyl-1H-imidazol-1-yle ou un groupe de formule -L¹-C(=O)-OR¹¹, -L¹-NR^{12A}R^{12B}, -L¹-C(=O)-NR^{13A}R^{13B}, -L²-S(=O)₂-NR^{13A}R^{13B} ou -L³-R¹⁴
(C₁-C₄)-alkyle et (C₁-C₄)-alcoxy pouvant être substitués par un radical choisi dans la série hydroxy, méthoxy, éthoxy et amino ou jusqu'à trisubstitués par fluor et
L¹ représente une liaison ou -CH₂-,
L² représente une liaison,
L³ représente une liaison ou -O-,
R¹¹ représente hydrogène,
R^{12A} et R^{12B} représentent, indépendamment l'un de l'autre, hydrogène ou méthyle ou
R^{12A} et R^{12B} sont liés l'un à l'autre et forment, ensemble avec l'atome d'azote auquel ils sont liés, un cycle azétidin-1-yle, pyrrolidin-1-yle ou pipéridin-1-yle qui peut à chaque fois être substitué par un radical choisi dans la série cyano, hydroxy et méthoxy, ou forment un cycle pipérazin-1-yle, 4-méthylpipérazin-1-yle ou morpholin-4-yle,
R^{13A} et R^{13B} représentent, indépendamment l'un de l'autre, hydrogène ou méthyle et
R¹⁴ représente un cycle azétidin-3-yle, pyrrolidin-3-yle, pipéridin-3-yle ou pipéridin-4-yle, qui peut à chaque fois être substitué par hydroxy,
ainsi que ses sels, solvates et solvates des sels.

5. Procédé pour la préparation de composés de formule (I) 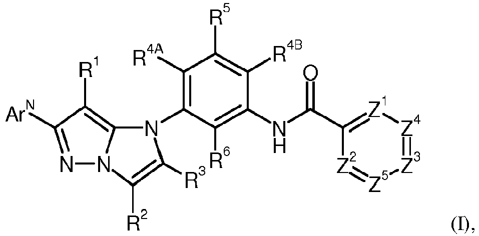 dans laquelle
Ar^{N} représente aza-hétéroaryle de 5 ou 6 chaînons, qui est choisi dans le groupe constitué par 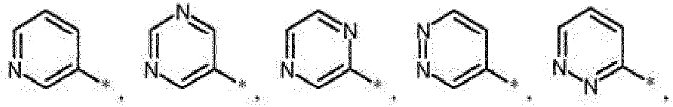 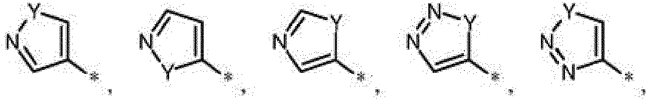 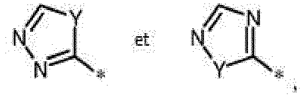 dans laquelle * marque la liaison au groupement imidazopyrazole et Y signifie O, S ou NH,
R¹ représente hydrogène ou fluor,
R² représente hydrogène ou (C₁-C₄)-alkyle,
R³ représente hydrogène,
R^{4A} et R^{4B} représentent, indépendamment l'un de l'autre, hydrogène, fluor, chlore, méthyle, fluorométhyle, difluorométhyle, trifluorométhyle, hydroxyméthyle, méthoxyméthyle, éthyle, hydroxy, méthoxy ou trifluorométhoxy,
R⁵ représente hydrogène, fluor, chlore ou méthyle,
R⁶ représente hydrogène, fluor, méthyle ou hydroxy,
Z¹ représente C-R^{7A} ou N,
Z² représente C-R^{7B} ou N,
Z³ représente C-R⁸ ou N,
Z⁴ représente C-R⁹ ou N et
Z⁵ représente C-R¹⁰ ou N,
au total au maximum un des chaînons de cycle Z¹, Z², Z³, Z⁴ et Z⁵ représentant N et où
R^{7A} et R^{7B} signifient, indépendamment l'un de l'autre, hydrogène, fluor, chlore, méthyle, hydroxy ou méthoxy,
R⁸ signifie hydrogène, fluor, chlore ou méthyle,
R⁹ signifie hydrogène, pentafluorosulfanyle, (trifluorométhyl)sulfanyle, triméthylsilyle, (C₁-C₆)-alkyle, (C₁-C₆)-alcoxy, (C₃-C₆)-cycloalkyle, oxétanyle ou tétrahydropyrannyle,
(C₁-C₆)-alkyle et (C₁-C₆)-alcoxy pouvant être jusqu'à hexasubstitués par fluor et (C₃-C₆)-cycloalkyle, oxétanyle et tétrahydropyrannyle pouvant être jusqu'à disubstitués, de manière identique ou différente, par un radical choisi dans la série fluor, méthyle, trifluorométhyle et hydroxy et
R¹⁰ signifie hydrogène, fluor, chlore, brome, cyano, (C₁-C₆)-alkyle, hydroxy, (C₁-C₆)-alcoxy, (C₁-C₄)-alkylsulfonyle, (C₃-C₆)-cycloalkyle, phényle, hétéroaryle de 5 ou 6 chaînons ou un groupe de formule -L¹-C(=O)-OR¹¹, -L¹-NR^{12A}R^{12B}, -L¹-C(=O)-NR^{13A}R^{13B}, -L²-S(=O)₂-NR^{13A}R^{13B} ou -L³-R¹⁴,
(C₁₋C₆)-alkyle et (C₁-C₆)-alcoxy pouvant être substitués par un radical choisi dans la série hydroxy, méthoxy, éthoxy, 2-hydroxyéthoxy, 2-méthoxyéthoxy, 2-éthoxyéthoxy, amino, méthylamino et diméthylamino ou jusqu'à hexasubstitués par fluor et
(C₃-C₆)-cycloalkyle pouvant être jusqu'à disubstitué, de manière identique ou différente, par un radical choisi dans la série méthyle, hydroxy, méthoxy, éthoxy, amino, méthylamino et diméthylamino et phényle et hétéroaryle de 5 ou 6 chaînons pouvant être jusqu'à disubstitués, de manière identique ou différente, par un radical choisi dans la série fluor, chlore, cyano, méthyle et trifluorométhyle et où
L¹ représente une liaison ou -CH₂-,
L² représente une liaison ou -CH₂-,
L³ représente une liaison ou -O-,
R¹¹ représente hydrogène ou (C₁-C₄)-alkyle,
R^{12A}, R^{12B}, R^{13A} et R^{13B} représentent, indépendamment les uns des autres, hydrogène ou (C₁-C₄)-alkyle,
(C₁-C₄)-alkyle pouvant être à chaque fois substitué par un radical choisi dans la série hydroxy, méthoxy, éthoxy, amino, méthylamino et diméthylamino ou
R^{12A} et R^{12B} ou R^{13A} et R^{13B} sont liés l'un à l'autre et forment, ensemble avec l'atome d'azote auquel ils sont à chaque fois liés, un hétérocycle de 4 à 6 chaînons, qui peut contenir un autre hétéroatome de cycle de la série N, O ou S et qui peut être jusqu'à disubstitué, de manière identique ou différente, par un radical choisi dans la série fluor, cyano, (C₁-C₄)-alkyle, hydroxy, (C₁-C₄)-alcoxy et oxo, et
R¹⁴ représente un hétérocycle de 4 à 6 chaînons lié via un atome de carbone de cycle, qui contient un hétéroatome de cycle de la série N, O ou S et qui peut être jusqu'à disubstitué, de manière identique ou différente, par un radical choisi dans la série fluor, cyano, (C₁-C₄)-alkyle, hydroxy, (C₁-C₄)-alcoxy et oxo,
R¹⁰ ne représentant pas hydrogène, fluor, chlore ou brome lorsque Z⁴ représente CH ou N et Z⁵ ne représentant pas N lorsque Z⁴ représente CH, ainsi que leurs sels, solvates et solvates des sels, **caractérisé en ce qu'**on couple
[A] soit un dérivé d'aniline de formule (II) 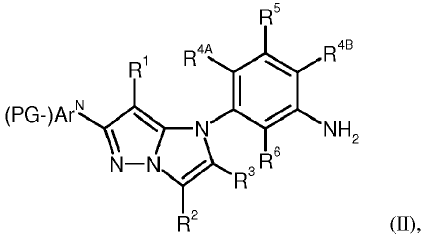
dans laquelle Ar^{N}, R¹, R², R³, R^{4A}, R^{4B}, R⁵ et R⁶ présentent les significations indiquées dans les revendications 1 à 4 et
(PG-) signifie un groupe de protection facultatif de l'azote dans le cas où Ar^{N} représente NH,
dans un solvant inerte en présence d'un agent de condensation, avec un acide carboxylique de formule (III), 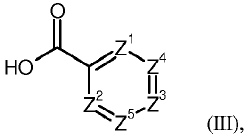
dans laquelle Z¹, Z², Z³, Z⁴ et Z⁵ présentent les significations indiquées dans les revendications 1 à 4,
en amide d'acide carboxylique de formule (IV) 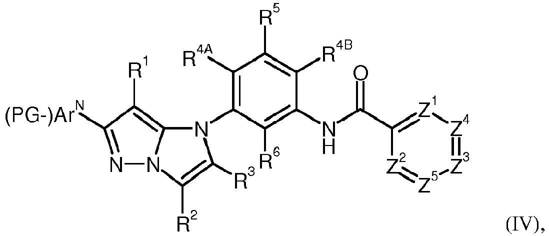
dans laquelle Ar^{N}, (PG-), R¹, R², R³, R^{4A}, R^{4B}, R⁵, R⁶, Z¹, Z², Z³, Z⁴ et Z⁵ présentent les significations indiquées ci-dessus,
puis on dissocie le groupe de protection PG, s'il est présent, soit
[B] un dérivé de 1H-imidazo[1,2-b]pyrazole de formule (V) 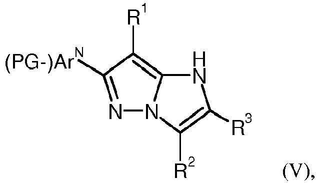
dans laquelle Ar^{N}, R¹, R² et R³ présentent les significations indiquées dans les revendications 1 à 4 et
(PG-) signifie un groupe de protection facultatif de l'azote dans le cas où Ar^{N} représente NH,
dans un solvant inerte sous catalyse au cuivre (I) avec un bromure de phényle de formule (VI) 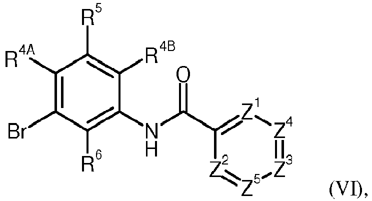
dans laquelle R^{4A}, R^{4B}, R⁵, R⁶, Z¹, Z², Z³, Z⁴ et Z⁵ présentent les significations indiquées dans les revendications 1 à 4,
en dérivé de 1-phényl-1H-imidazo[1,2-b]pyrazole de formule (IV) 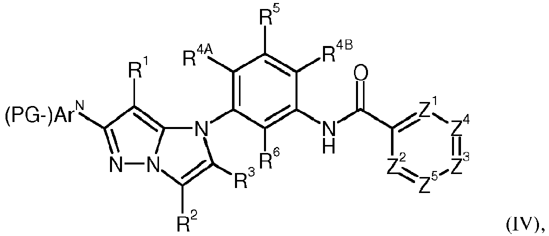
dans laquelle Ar^{N}, (PG-), R¹, R², R³, R^{4A}, R^{4B}, R⁵, R⁶, Z¹, Z², Z³, Z⁴ et Z⁵ présentent les significations indiquées ci-dessus,
puis on dissocie le groupe de protection PG, s'il est présent, soit
[C] un dérivé d'aminopyrazole de formule (VII) 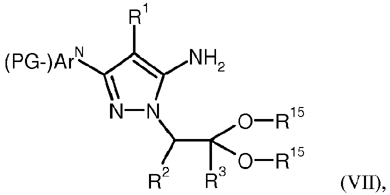
dans laquelle Ar^{N}, R¹, R² et R³ présentent les significations indiquées dans les revendications 1 à 4,
R¹⁵ représente méthyle ou éthyle et
(PG-) signifie un groupe de protection facultatif de l'azote dans le cas où Ar^{N} représente NH,
dans un solvant inerte sous catalyse au palladium avec un bromure de phényle de formule (VI) 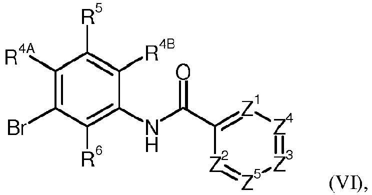
dans laquelle R^{4A}, R^{4B}, R⁵, R⁶, Z¹, Z², Z³, Z⁴ et Z⁵ présentent les significations indiquées dans les revendications 1 à 4,
en un composé de formule (VIII) 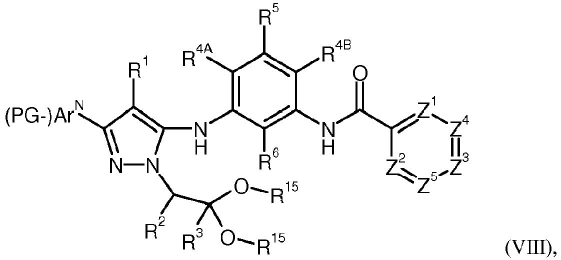
dans laquelle Ar^{N}, (PG-), R¹, R², R³, R^{4A}, R^{4B}, R⁵, R⁶, R¹⁵, Z¹, Z², Z³, Z⁴ et Z⁵ présentent les significations indiquées ci-dessus,
on cyclise ensuite le composé de formule (VIII) par traitement à l'acide en dérivé de 1-phényl-1H-imidazo[1,2-b]pyrazole de formule (IV) 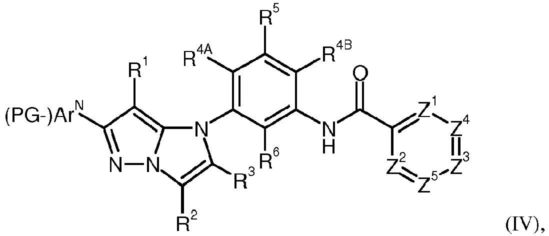
dans laquelle Ar^{N}, (PG-), R¹, R², R³, R^{4A}, R^{4B}, R⁵, R⁶, Z¹, Z², Z³, Z⁴ et Z⁵ présentent les significations indiquées ci-dessus,
et on dissocie ensuite le groupe de protection PG, s'il est présent,
et on transforme le cas échéant les composés de formule (I) ainsi obtenus avec (i) les solvants et/ou (ii) les bases ou acides correspondants en leurs solvates, sels et/ou solvates des sels.

6. Composé, tel que défini dans l'une quelconque des revendications 1 à 4, pour une utilisation dans le traitement et/ou la prévention de maladies.

7. Composé, tel que défini dans l'une quelconque des revendications 1 à 4, pour une utilisation dans un procédé pour le traitement et/ou la prévention de maladies cancéreuses et tumorales.

8. Médicament contenant un composé, tel que défini dans l'une quelconque des revendications 1 à 4, en combinaison avec un ou plusieurs adjuvants inertes, non toxiques, pharmaceutiquement appropriés.

9. Médicament contenant un composé tel que défini dans l'une quelconque des revendications 1 à 4 en combinaison avec une ou plusieurs autres substances actives.

10. Médicament selon la revendication 8 ou 9 pour le traitement et/ou la prévention de maladies cancéreuses et tumorales.
